(19) Europäisches Patentamt
European Patent Office
Office européen des brevets

(11) EP 1 430 898 A1

(12) **EUROPEAN PATENT APPLICATION**
published in accordance with Art. 158(3) EPC

(43) Date of publication:
**23.06.2004 Bulletin 2004/26**

(21) Application number: **02770216.6**

(22) Date of filing: **26.09.2002**

(51) Int Cl.[7]: **A61K 31/519**, A61K 31/5377, A61K 31/537, A61K 31/5365, A61K 31/527, A61K 31/5383, A61K 45/00, A61P 3/04, A61P 3/10, A61P 9/12, A61P 15/10, A61P 25/00, A61P 25/14, A61P 25/16, A61P 25/24, A61P 43/00, C07D 487/04, C07D 487/14, C07D 519/00, C07D 455/04

(86) International application number:
**PCT/JP2002/009911**

(87) International publication number:
**WO 2003/028732 (10.04.2003 Gazette 2003/15)**

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: **28.09.2001 JP 2001302375**
**31.01.2002 JP 2002023146**

(71) Applicant: **KYOWA HAKKO KOGYO CO., LTD.**
**Chiyoda-ku, Tokyo 100-8185 (JP)**

(72) Inventors:
- **UESAKA, Noriaki, c/o Pharma. Research Inst. Kyowa Nagaizumi-cho, Sunto-gun, Shizuoka 411-8 (JP)**
- **IMMA, Hironori, c/o Pharma. Research Inst. Kyowa Nagaizumi-cho, Sunto-gun, Shizuoka 411-8 (JP)**
- **KASHIMA, Hajime, c/o Pharma. Research Inst. Kyowa Nagaizumi-cho, Sunto-gun, Shizuoka 411-8 (JP)**
- **KUROKAWA, Masako, c/o Pharma. Research Inst. Kyowa Nagaizumi-cho, Sunto-gun, Shizuoka 411-8 (JP)**
- **NONAKA, Hiromi, c/o Pharma. Research Inst. Kyowa Nagaizumi-cho, Sunto-gun, Shizuoka 411-8 (JP)**
- **KANDA, Tomoyuki, c/o Pharma. Research Inst. Kyowa Nagaizumi-cho, Sunto-gun, Shizuoka 411-8 (JP)**
- **KUWANA, Yoshihisa, c/o Head Office, Kyowa Hakko Chiyoda-ku, Tokyo 100-8185 (JP)**
- **TOKI, Shinichiro, c/o Pharma. Research Inst. Kyowa Nagaizumi-cho, Sunto-gun, Shizuoka 411-8 (JP)**
- **SHIMADA, Junichi, c/o Pharm. Research Inst. Kyowa Nagaizumi-cho, Sunto-gun, Shizuoka 411-8 (JP)**

(74) Representative: **Tanner, James Percival et al**
**D. Young & Co,**
**21 New Fetter Lane**
**London EC4A 1DA (GB)**

# (54) RECEPTOR ANTAGONIST

(57) The present invention provides an $\alpha_{2c}$-adrenoceptor antagonist comprising, as an active ingredient, a condensed-ring-pyrimidine derivative represented by general formula (I) below or a pharmaceutically acceptable salt thereof useful for treating and/or preventing various diseases induced by hyperactivity of $\alpha_{2c}$-adrenoceptor (for example, Parkinson's disease, L-DOPA-induced dyskinesia, tardive dyskinesia and depression) and the like.

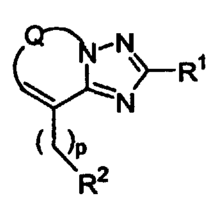

(I)

EP 1 430 898 A1

{wherein p represents an integer of 1 to 3;
$R^1$ represents a substituted or unsubstituted heterocyclic group, substituted or unsubstituted aryl, or the like;
$R^2$ represents $-N(-R^4)(-R^5)$ (wherein $R^4$ and $R^5$ are the same or different, and each represents a hydrogen atom,
substituted or unsubstituted aralkyl, or the like, or $R^4$ and $R^5$ form a substituted or unsubstituted heterocyclic group together with the adjacent nitrogen atom) or the like; and
-Q- represents $-N=C(-R^7)-$ [wherein $R^7$ represents $-N(-R^9)(-R^{10})$ (wherein $R^9$ and $R^{10}$ are the same or different, and each represents substituted or unsubstituted aralkyl, or the like, or $R^9$ and $R^{10}$ form a substituted or unsubstituted heterocyclic group together with the adjacent nitrogen atom) or the like] or the like}

## Description

### Technical Field

The present invention relates to condensed-ring-pyrimidine derivatives which show $\alpha_2$-adrenoceptor antagonism, particularly $\alpha_{2c}$-adrenoceptor antagonism and are useful for treating and/or preventing various diseases induced by hyperactivity of $\alpha_{2c}$-adrenoceptor (for example, Parkinson's disease, L-DOPA-induced dyskinesia, tardive dyskinesia, depression, hypertension, diabetes, obesity and erectile dysfunction), $\alpha_{2c}$-adrenoceptor antagonists comprising a condensed-ring-pyrimidine derivative as an active ingredient, etc.

### Background Art

Catecholamines (epinephrine, norepinephrine and dopamine) released from adrenergic nerve terminals diffuse through synaptic cleft to bind to adrenoceptors and show various physiological actions. Adrenoceptors, which in old times had been classified into α-receptor and β-receptor, were thereafter classified into 4 subtypes, $\alpha_1$, $\alpha_2$, $\beta_1$ and $\beta_2$, based on studies using specific agonists, antagonists and blockades. Furthermore, in recent years, plurality of molecular species have been confirmed for each of the subtypes as a result of molecular biology-based studies using cloning techniques. For example, for $\alpha_2$ receptors, the existence of 3 kinds, $\alpha_{2a}$, $\alpha_{2b}$ and $\alpha_{2c}$, is presently confirmed.

Catecholamines released from adrenergic nerve terminals have been known, for example, to control motor functions via $\alpha_2$ receptors [Neurology, Vol. 41, p. 986 (1991), Neuroscience, Vol. 41, p. 507 (1991)]; to participate in phallic erection via sympathetic nerves [Journal of Urology, Vol. 128, p. 45 (1982)]; to elevate blood pressure by constricting peripheral veins [Biochemical Pharmacology, Vol. 31, p. 467 (1982)]; and to promote the secretion of glucagon and suppress the secretion of insulin [Journal of Clinical Investigation, Vol. 63, p. 230 (1979)]. Furthermore, it has been known that $\alpha_2$ receptor antagonists suppress L-DOPA-induced dyskinesia [Naunyn-Schmiedeberg's Archives of Pharmacology, Vol. 361, p. 181 (2000)] and show anti-obesity action by promoting decomposition of fatty acids [American Journal of Clinical Nutrition, Vol. 55, p. 219S (1992)], that no depressive symptoms are observed in $\alpha_{2c}$ receptor-knockout mice (US Patent No. 5,902,807), and the like. Accordingly, antagonists having $\alpha_2$-adrenoceptor antagonism are expected as therapeutic agents, for example, for Parkinson's disease, L-DOPA-induced dyskinesia, tardive dyskinesia, depression, sexual dysfunction in males, dysfunction of phallic erection, hypertension, diabetes and obesity and/or as agents that show an effect of alleviating symptoms thereof.

On the other hand, [1,2,4]triazolo[1,5-c]pyrimidine derivatives are disclosed as compounds having diuretic action in Japanese Published Unexamined Patent Application No. 13792/85, as compounds having anti-asthmatic action in Japanese Published Unexamined Patent Application No. 56983/85 and as compounds having bronchodilatative action in Japanese Published Unexamined Patent Application No. 167592/84.

In WO98/42711, the following [1,2,4]triazolo[1,5-c]pyrimidine derivatives having adenosine receptor antagonism and action of alleviating various symptoms induced by hyperactivity of adenosine receptors are reported.

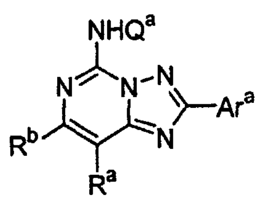

(E)

(wherein $Ar^a$ represents substituted or unsubstituted aryl, or a substituted or unsubstituted aromatic heterocyclic group; $R^a$ represents hydrogen, substituted or unsubstituted aryl, or a substituted or unsubstituted aromatic heterocyclic group; $R^b$ represents hydrogen, halogen, lower alkoxy or the like; and $Q^a$ represents hydrogen or 3,4-dimethoxybenzyl)

Also, in WO00/17201, the following [1,2,4]triazolo[1,5-c]pyrimidine derivatives having adenosine receptor antagonism and curative action on various diseases induced by hyperactivity of adenosine receptors are reported.

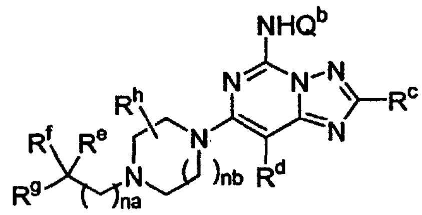

(F)

(wherein $R^c$ represents a substituted or unsubstituted aromatic heterocyclic group or the like; $R^d$ to $R^g$ each represent substituted or unsubstituted lower alkyl, a substituted or unsubstituted aromatic heterocyclic group or the like; $R^h$ represents hydrogen, substituted or unsubstituted lower alkyl, halogen, hydroxy or the like; na and nb each represent an integer of 0 to 4; and $Q^b$ represents hydrogen or 3,4-dimethoxybenzyl)

Disclosure of the Invention

An object of the present invention is to provide condensed-ring-pyrimidine derivatives or pharmaceutically acceptable salts thereof which show $\alpha_2$-adrenoceptor antagonism, particularly $\alpha_{2c}$-adrenoceptor antagonism and are useful for treating and/or preventing various diseases induced by hyperactivity of $\alpha_{2c}$-adrenoceptor (for example, Parkinson's disease, L-DOPA-induced dyskinesia, tardive dyskinesia, depression, hypertension, diabetes, obesity, and erectile dysfunction), $\alpha_{2c}$-adrenoceptor antagonists comprising a condensed-ring-pyrimidine derivative or a pharmaceutically acceptable salt thereof as an active ingredient, etc.

The present invention relates to the following (1) to (44).

(1) An $\alpha_{2c}$-adrenoceptor antagonist comprising, as an active ingredient, a condensed-ring-pyrimidine derivative represented by general formula (I):

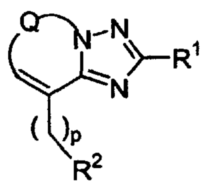

(I)

<wherein p represents an integer of 1 to 3;

$R^1$ represents a hydrogen atom, substituted or unsubstituted lower alkyl, substituted or unsubstituted cycloalkyl, substituted or unsubstituted aryl, substituted or unsubstituted aralkyl, a substituted or unsubstituted heterocyclic group, or substituted or unsubstituted heterocycle-lower alkyl;

$R^2$ represents $-N(-R^3)(-R^4)$ (wherein $R^3$ and $R^4$ are the same or different, and each represents a hydrogen atom, substituted or unsubstituted lower alkyl, substituted or unsubstituted cycloalkyl, substituted or unsubstituted aryl, substituted or unsubstituted aralkyl, a substituted or unsubstituted heterocyclic group, or substituted or unsubstituted heterocycle-lower alkyl, or $R^3$ and $R^4$ form a substituted or unsubstituted heterocyclic group together with the adjacent nitrogen atom) or general formula (A):

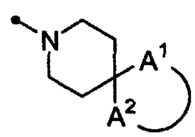

(A)

{wherein $-A^1-A^2-$ represents $-Y^1-C(=O)-Y^2-CH_2-CH_2-$ [wherein $Y^1$ and $Y^2$ are the same or different, and each represents an oxygen atom or $-N(-R^5)-$ (wherein $R^5$ represents a hydrogen atom, substituted or unsubstituted lower alkyl,

substituted or unsubstituted aryl, substituted or unsubstituted aralkyl, a substituted or unsubstituted heterocyclic group, or substituted or unsubstituted heterocycle-lower alkyl)] or $-Y^3-CH_2-Y^4-C(=O)-$ (wherein $Y^3$ and $Y^4$ have the same meanings as the above-described $Y^1$ and $Y^2$, respectively)}; and

- Q- represents (a) $-N=C(-R^7)-$ [wherein $R^7$ represents $-O(-R^8)$ (wherein $R^8$ represents a hydrogen atom, substituted or unsubstituted lower alkyl, substituted or unsubstituted lower alkanoyl, substituted or unsubstituted aroyl, substituted or unsubstituted aryl, substituted or unsubstituted aralkyl, a substituted or unsubstituted heterocyclic group, or substituted or unsubstituted heterocycle-lower alkyl), $-N(-R^9)(-R^{10})$ (wherein $R^9$ and $R^{10}$ are the same or different, and each represents a hydrogen atom, substituted or unsubstituted lower alkyl, substituted or unsubstituted cycloalkyl, substituted or unsubstituted lower alkenyl, substituted or unsubstituted lower alkynyl, substituted or unsubstituted aryl, substituted or unsubstituted lower alkanoyl, substituted or unsubstituted aroyl, substituted or unsubstituted aralkyl, a substituted or unsubstituted heterocyclic group, or substituted or unsubstituted heterocycle-lower alkyl, or $R^9$ and $R^{10}$ form a substituted or unsubstituted heterocyclic group together with the adjacent nitrogen atom) or $-S(-R^{11})$ (wherein $R^{11}$ represents a hydrogen atom, substituted or unsubstituted lower alkyl, substituted or unsubstituted aralkyl, or substituted or unsubstituted heterocycle-lower alkyl)],

(b) $-N(-R^{12})-C(=O)-$ (wherein $R^{12}$ represents a hydrogen atom, substituted or unsubstituted lower alkyl, substituted or unsubstituted aralkyl, or substituted or unsubstituted heterocycle-lower alkyl) or

(c) general formula (B):

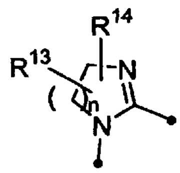

(B)

(wherein n represents an integer of 1 to 3; and $R^{13}$ and $R^{14}$ are the same or different, and each represents a hydrogen atom, substituted or unsubstituted lower alkyl, substituted or unsubstituted aryl, substituted or unsubstituted aralkyl, a substituted or unsubstituted heterocyclic group, or substituted or unsubstituted heterocycle-lower alkyl)> or a pharmaceutically acceptable salt thereof.

(2) The $\alpha_{2c}$-adrenoceptor antagonist according to the above (1), wherein $R^2$ is $-N(-R^3)$ $(-R^4)$ in which $R^3$ and $R^4$ form a substituted or unsubstituted heterocyclic group together with the adjacent nitrogen atom.

(3) The $\alpha_{2c}$-adrenoceptor antagonist according to the above (1), wherein $R^2$ is substituted or unsubstituted piperazinyl, substituted or unsubstituted piperidino, or substituted or unsubstituted tetrahydroisoquinolyl.

(4) The $\alpha_{2c}$-adrenoceptor antagonist according to any one of the above (1) to (3), wherein -Q- is $-N=C(-R^7)-$ in which $R^7$ is $-N(-R^9)(-R^{10})$.

(5) The $\alpha_{2c}$-adrenoceptor antagonist according to the above (4), wherein $R^9$ and $R^{10}$ each are a hydrogen atom.

(6) The $\alpha_{2c}$-adrenoceptor antagonist according to any one of the above (1) to (3), wherein -Q- is $-N=C(-R^7)-$ in which $R^7$ is $-S(-R^{11})$.

(7) The $\alpha_{2c}$-adrenoceptor antagonist according to any one of the above (1) to (3), wherein -Q- is $-N(-R^{12})-C(=O)-$.

(8) The $\alpha_{2c}$-adrenoceptor antagonist according to any one of the above (1) to (3), wherein -Q- is general formula (B).

(9) The $\alpha_{2c}$-adrenoceptor antagonist according to any one of the above (1) to (8), wherein $R^1$ is furyl.

(10) An agent for preventing and/or treating dyskinesia comprising, as an active ingredient, a condensed-ring-pyrimidine derivative represented by general formula (I):

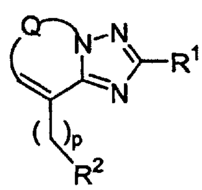

(I)

(wherein p, $R^1$, $R^2$ and -Q- each have the same meanings as defined above) or a pharmaceutically acceptable salt thereof.

(11) The agent for preventing and/or treating dyskinesia according to the above (10), wherein $R^2$ is $-N(-R^3)(-R^4)$ in which $R^3$ and $R^4$ form a substituted or unsubstituted heterocyclic group together with the adjacent nitrogen atom.

(12) The agent for preventing and/or treating dyskinesia according to the above (10), wherein $R^2$ is substituted or unsubstituted piperazinyl, substituted or unsubstituted piperidino, or substituted or unsubstituted tetrahydroisoquinolyl.

(13) The agent for preventing and/or treating dyskinesia according to any one of the above (10) to (12), wherein -Q- is $-N=C(-R^7)-$ in which $R^7$ is $-N(-R^9)(-R^{10})$.

(14) The agent for preventing and/or treating dyskinesia according to the above (13), wherein $R^9$ and $R^{10}$ each are a hydrogen atom.

(15) The agent for preventing and/or treating dyskinesia according to any one of the above (10) to (12), wherein -Q- is $-N=C(-R^7)-$ in which $R^7$ is $-S(-R^{11})$.

(16) The agent for preventing and/or treating dyskinesia according to any one of the above (10) to (12), wherein -Q- is $-N(-R^{12})-C(=O)-$.

(17) The agent for preventing and/or treating dyskinesia according to any one of the above (10) to (12), wherein -Q- is general formula (B).

(18) The agent for preventing and/or treating dyskinesia according to any one of the above (10) to (17), wherein $R^1$ is furyl.

(19) The agent for preventing and/or treating dyskinesia according to any one of the above (10) to (18), wherein dyskinesia is L-DOPA-induced dyskinesia.

(20) A condensed-ring-pyrimidine derivative represented by general formula (II):

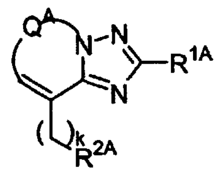

(II)

<wherein k represents an integer of 1 to 3;

$R^{1A}$ represents a hydrogen atom, substituted or unsubstituted lower alkyl, substituted or unsubstituted cycloalkyl, substituted or unsubstituted aryl, substituted or unsubstituted aralkyl, a substituted or unsubstituted heterocyclic group, or substituted or unsubstituted heterocycle-lower alkyl;

$R^{2A}$ represents $-N(-R^{3A})(-R^{4A})$ (wherein $R^{3A}$ and $R^{4A}$ are the same or different, and each represents a hydrogen atom, substituted or unsubstituted lower alkyl, substituted or unsubstituted cycloalkyl, substituted or unsubstituted aryl, substituted or unsubstituted aralkyl, a substituted or unsubstituted heterocyclic group, or substituted or unsubstituted heterocycle-lower alkyl, or $R^{3A}$ and $R^{4A}$ form a substituted or unsubstituted heterocyclic group together with the adjacent nitrogen atom) or general formula (C):

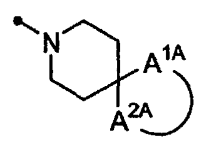

(C)

{wherein $-A^{1A}-A^{2A}-$ represents $-Y^{1A}-C(=O)-Y^{2A}-CH_2-CH_2-$ [wherein $Y^{1A}$ and $Y^{2A}$ are the same or different, and each represents an oxygen atom or $-N(-R^{5A})-$ (wherein $R^{5A}$ represents a hydrogen atom, substituted or unsubstituted lower alkyl, substituted or unsubstituted aryl, substituted or unsubstituted aralkyl, a substituted or unsubstituted heterocyclic group, or substituted or unsubstituted heterocycle-lower alkyl)] or $-Y^{3A}-CH_2-Y^{4A}-C(=O)-$ (wherein $Y^{3A}$ and $Y^{4A}$ have the same meanings as the above-described $Y^{1A}$ and $Y^{2A}$, respectively)}; and

$-Q^A-$ represents (d) $-N=C(-R^{7A})-$ [wherein $R^{7A}$ represents $-O(-R^{8A})$ (wherein $R^{8A}$ represents a hydrogen atom, substituted or unsubstituted lower alkyl, substituted or unsubstituted lower alkanoyl, substituted or unsubstituted aroyl, substituted or unsubstituted aryl, substituted or unsubstituted aralkyl, a substituted or unsubstituted heterocyclic group, or substituted or unsubstituted heterocycle-lower alkyl), $-N(-R^{9A})(-R^{10A})$ (wherein $R^{9A}$ and $R^{10A}$ are the same or different, and each represents a hydrogen atom, substituted or unsubstituted lower alkyl, substituted

or unsubstituted cycloalkyl, substituted or unsubstituted lower alkenyl, substituted or unsubstituted lower alkynyl, substituted or unsubstituted aryl, substituted or unsubstituted lower alkanoyl, substituted or unsubstituted aroyl, substituted or unsubstituted aralkyl, a substituted or unsubstituted heterocyclic group, or substituted or unsubstituted heterocycle-lower alkyl, or $R^{9A}$ and $R^{10A}$ form a substituted or unsubstituted heterocyclic group together with the adjacent nitrogen atom) or -S(-$R^{11A}$) (wherein $R^{11A}$ represents a hydrogen atom, substituted or unsubstituted lower alkyl, substituted or unsubstituted aralkyl, or substituted or unsubstituted heterocycle-lower alkyl)],
(e) -N(-$R^{12A}$)-C(=O)- (wherein $R^{12A}$ represents a hydrogen atom, substituted or unsubstituted lower alkyl, substituted or unsubstituted aralkyl, or substituted or unsubstituted heterocycle-lower alkyl) or
(f) general formula (D):

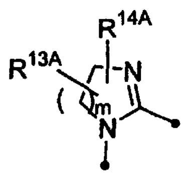

(D)

(wherein m represents an integer of 1 to 3; and $R^{13A}$ and $R^{14A}$ are the same or different, and each represents a hydrogen atom, substituted or unsubstituted lower alkyl, substituted or unsubstituted aryl, substituted or unsubstituted aralkyl, a substituted or unsubstituted heterocyclic group, or substituted or unsubstituted heterocycle-lower alkyl), provided that when $R^{1A}$ is furyl and -$Q^A$- is -N=C(-$R^{7A}$)- in which $R^{7A}$ is amino or 3,4-dimethoxybenzylamino, $R^{2A}$ is not morpholino, 1-piperazinyl, substituted 1-piperazinyl in which the 4-position is substituted by lower alkyl or aryl, phenylamino or substituted phenylamino in which the 4-position is substituted by halogen> or a pharmaceutically acceptable salt thereof.

(21) The condensed-ring-pyrimidine derivative according to the above (20), wherein the heterocyclic group in the substituted or unsubstituted heterocyclic group formed by $R^{3A}$ and $R^{4A}$ together with the adjacent nitrogen atom is not 1-piperazinyl, morpholino, thiomorpholino, piperidino or 1-homopiperazinyl when $R^{1A}$ is substituted or unsubstituted aryl, or a substituted or unsubstituted aromatic heterocyclic group, and -$Q^A$- is -N=C(-$R^{7A}$)- in which $R^{7A}$ is amino or 3,4-dimethoxyamino, or a pharmaceutically acceptable salt thereof.

(22) The condensed-ring-pyrimidine derivative according to the above (20) or (21), wherein $R^{1A}$ is substituted or unsubstituted lower alkyl, substituted or unsubstituted cycloalkyl, substituted or unsubstituted aralkyl, a substituted or unsubstituted alicyclic heterocyclic group, or substituted or unsubstituted heterocycle-lower alkyl, or a pharmaceutically acceptable salt thereof.

(23) The condensed-ring-pyrimidine derivative according to the above (20) or (21), wherein $R^{7A}$ is not amino or 3,4-dimethoxybenzylamino when $R^{1A}$ is substituted or unsubstituted aryl, or a substituted or unsubstituted aromatic heterocyclic group, or a pharmaceutically acceptable salt thereof.

(24) The condensed-ring-pyrimidine derivative according to any one of the above (20) to (23), wherein -$Q^A$- is -N=C(-$R^{7A}$)- in which $R^{7A}$ is -N(-$R^{9A}$)(-$R^{10A}$), or a pharmaceutically acceptable salt thereof.

(25) The condensed-ring-pyrimidine derivative according to any one of the above (20) to (23), wherein -$Q^A$- is -N=C(-$R^{7A}$)- in which $R^{7A}$ is -S(-$R^{11A}$), or a pharmaceutically acceptable salt thereof.

(26) The condensed-ring-pyrimidine derivative according to any one of the above (20) to (23), wherein -$Q^A$- is -N(-$R^{12A}$)-C(=O)-, or a pharmaceutically acceptable salt thereof.

(27) The condensed-ring-pyrimidine derivative according to any one of the above (20) to (23), wherein -$Q^A$- is general formula (D), or a pharmaceutically acceptable salt thereof.

(28) The condensed-ring-pyrimidine derivative according to any one of the above (20) to (27), wherein $R^{2A}$ is a group selected from the group consisting of pyridyl, tetrahydropyridyl, indolinyl, isoindolinyl, pyrrolidinyl, thiazolidinyl, oxazolidinyl, piperidino, homopiperidino, piperazinyl, homopiperazinyl, morpholino, thiomorpholino, tetrahydroquinolyl, tetrahydroisoquinolyl, octahydroquinolyl, benzimidazolyl, indazolyl, indolyl, isoindolyl, purinyl, dihydroindolyl, pyrrolyl, pyrazolyl, triazolyl, tetrazolyl, imidazolyl, octahydropyrido[1,2-a]pyrazinyl, octahydropyrrolo[1,2-a]pyrazinyl, 2-ketopiperazinyl, 1,8-diaza-4-oxabicyclo[4.4.0]decanyl, 2,5-diazabicyclo[2.2.1]heptyl, 2,3-dihydro-1H-benzo[de]isoquinolyl, 1,2,3,4-tetrahydrobenzofuro[2,3-c]pyridyl, 1,2,3,4-tetrahydro-9H-pyrido[3,4-b]indolyl, 5,6,7,8-tetrahydro-1,3-dioxolo[4,5-g]isoquinolyl and 1,2,4,5-tetrahydro-3H-benzo[d]azepinyl, or a pharmaceutically acceptable salt thereof.

(29) A pharmaceutical composition comprising the condensed-ring-pyrimidine derivative according to any one of the above (20) to (28) or a pharmaceutically acceptable salt thereof as an active ingredient.

(30) An $\alpha_{2c}$-adrenoceptor antagonist comprising the condensed-ring-pyrimidine derivative according to any one

of the above (20) to (28) or a pharmaceutically acceptable salt thereof as an active ingredient.

(31) An agent for preventing and/or treating Parkinson's disease comprising the condensed-ring-pyrimidine derivative according to any one of the above (20) to (28) or a pharmaceutically acceptable salt thereof as an active ingredient.

(32) An agent for preventing and/or treating dyskinesia comprising the condensed-ring-pyrimidine derivative according to any one of the above (20) to (28) or a pharmaceutically acceptable salt thereof as an active ingredient.

(33) An agent for preventing and/or treating dyskinesia comprising a compound having $\alpha_{2c}$-adrenoceptor antagonism or a pharmaceutically acceptable salt thereof as an active ingredient.

(34) The agent for preventing and/or treating dyskinesia according to the above (32) or (33), wherein dyskinesia is L-DOPA-induced dyskinesia.

(35) Use of the condensed-ring-pyrimidine derivative according to any one of the above (20) to (28) or a pharmaceutically acceptable salt thereof for the manufacture of an agent for preventing and/or treating diseases induced by hyperactivity of $\alpha_{2c}$-adrenoceptor.

(36) Use of the condensed-ring-pyrimidine derivative according to any one of the above (20) to (28) or a pharmaceutically acceptable salt thereof for the manufacture of an agent for preventing and/or treating Parkinson's disease.

(37) Use of the condensed-ring-pyrimidine derivative according to any one of the above (20) to (28) or a pharmaceutically acceptable salt thereof for the manufacture of an agent for preventing and/or treating dyskinesia.

(38) Use of a compound having $\alpha_{2c}$-adrenoceptor antagonism or a pharmaceutically acceptable salt thereof for the manufacture of an agent for preventing and/or treating dyskinesia.

(39) The use according to the above (27) or (28), wherein dyskinesia is L-DOPA-induced dyskinesia.

(40) A method for preventing and/or treating diseases induced by hyperactivity of $\alpha_{2c}$-adrenoceptor, which comprises administering an effective amount of the condensed-ring-pyrimidine derivative according to any one of the above (20) to (28) or a pharmaceutically acceptable salt thereof.

(41) A method for preventing and/or treating Parkinson's disease, which comprises administering an effective amount of the condensed-ring-pyrimidine derivative according to any one of the above (20) to (28) or a pharmaceutically acceptable salt thereof.

(42) A method for preventing and/or treating dyskinesia, which comprises administering an effective amount of the condensed-ring-pyrimidine derivative according to any one of the above (20) to (28) or a pharmaceutically acceptable salt thereof.

(43) A method for preventing and/or treating dyskinesia, which comprises administering an effective dose of a compound having $\alpha_{2c}$-adrenoceptor antagonism or a pharmaceutically acceptable salt thereof.

(44) The method for preventing and/or treating dyskinesia according to the above (42) or (43), wherein dyskinesia is L-DOPA-induced dyskinesia.

In the definition of each group in general formulae (I) and (II) :

(i) Examples of the lower alkyl include straight-chain or branched alkyl having 1 to 8 carbon atoms, such as methyl, ethyl, propyl, isopropyl, butyl, isobutyl, sec-butyl, tert-butyl, pentyl, isopentyl, hexyl, heptyl and octyl.

(ii) Examples of the cycloalkyl include those having 3 to 8 carbon atoms, such as cyclopropyl, cyclobutyl, cyclopentyl, cyclohexyl, cycloheptyl and cyclooctyl.

(iii) Examples of the lower alkenyl include straight-chain or branched alkenyl having 3 to 8 carbon atoms, such as allyl, propenyl, methacryl, 1-butenyl, crotyl, pentenyl, hexenyl, heptenyl and octenyl.

(iv) Examples of the lower alkynyl include straight-chain or branched alkynyl having 3 to 8 carbon atoms, such as propargyl, 1-propynyl, butynyl, pentynyl, hexynyl, heptynyl and octynyl.

(v) Examples of the lower alkanoyl include straight-chain or branched alkanoyl having 1 to 8 carbon atoms, such as formyl, acetyl, propionyl, butyryl, isobutyryl, valeryl, isovaleryl, pivaloyl, hexanoyl, heptanoyl and octanoyl.

(vi) The alkylene moiety of the aralkyl and the heterocycle-lower alkyl has the same meaning as the above-described lower alkyl (i) except one hydrogen atom is removed therefrom.

(vii) Examples of the aryl moiety of the aryl, aralkyl and aroyl include phenyl, naphthyl, 1,2,3,4-tetrahydronaphthyl, anthryl, indenyl and indanyl.

(viii) Examples of the aromatic heterocyclic group include 5- or 6-membered monocyclic aromatic heterocyclic groups containing at least one atom selected from a nitrogen atom, an oxygen atom and a sulfur atom, and bicyclic or tricyclic condensed-ring aromatic heterocyclic groups containing at least one atom selected from a nitrogen atom, an oxygen atom and a sulfur atom in which 3- to 8-membered rings are condensed, more specifically, pyridyl, pyrazinyl, pyrimidinyl, pyridazinyl, benzimidazolyl, 2-oxobenzimidazolyl, benzotriazolyl, purinyl, benzoxazolyl, benzothiazolyl, indazolyl, indolyl, isoindolyl, purinyl, quinolyl, isoquinolyl, phthalazinyl, naphthyridinyl, quinoxalinyl, pyrrolyl, pyrazolyl, quinazolinyl, cinnolinyl, triazolyl, tetrazolyl, imidazolyl, oxazolyl, thiazolyl, thienyl and furyl.

Examples of the alicyclic heterocyclic group include 5- or 6-membered monocyclic alicyclic heterocyclic groups containing at least one atom selected from a nitrogen atom, an oxygen atom and a sulfur atom, and bicyclic or tricyclic condensed-ring alicyclic heterocyclic groups containing at least one atom selected from a nitrogen atom, an oxygen atom and a sulfur atom in which 3- to 8-membered rings are condensed, more specifically, pyrrolidinyl, 2,5-dioxopyrrolidinyl, thiazolidinyl, oxazolidinyl, piperidyl, piperidino, piperazinyl, homopiperazinyl, homopiperidyl, homopiperidino, morpholinyl, morpholino, thiomorpholinyl, thiomorpholino, tetrahydropyranyl, tetrahydrofuranyl, tetrahydroquinolyl, tetrahydroisoquinolyl, octahydroquinolyl, indolinyl, 1,3,4,6,7,11b-hexahydro-2H-benzo[a]quinolizinyl and 1,4-benzodioxanyl.

Examples of the heterocyclic group and the heterocyclic group moiety of the heterocycle-lower alkyl include those mentioned above with respect to the aromatic heterocyclic group and alicyclic heterocyclic group.

(ix) Examples of the heterocyclic group formed together with the adjacent nitrogen atom include 5- to 8-membered monocyclic heterocyclic groups containing at least one nitrogen atom (the monocyclic heterocyclic groups may also contain another nitrogen atom, oxygen atom or sulfur atom), and bicyclic or tricyclic condensed-ring heterocyclic groups containing at least one nitrogen atom in which 3- to 8-membered rings are condensed (the condensed-ring heterocyclic groups may also contain another nitrogen atom, oxygen atom or sulfur atom), more specifically, pyridyl, tetrahydropyridyl, indolinyl, isoindolinyl, pyrrolidinyl, thiazolidinyl, oxazolidinyl, piperidino, homopiperidino, piperazinyl, homopiperazinyl, morpholino, thiomorpholino, perhydroazepinyl, perhydroazocinyl, tetrahydroquinolyl, tetrahydroisoquinolyl, octahydroquinolyl, benzimidazolyl, indazolyl, indolyl, isoindolyl, purinyl, dihydroindolyl, pyrrolyl, pyrazolyl, triazolyl, tetrazolyl, imidazolyl, octahydropyrido[1,2-a]pyrazinyl, octahydropyrrolo[1,2-a]pyrazinyl, 2-ketopiperazinyl, 1,8-diaza-4-oxabicyclo[4.4.0]decanyl, 2,5-diazabicyclo[2.2.1]heptyl, 2,3-dihydro-1H-benzo[de]isoquinolyl, 1,2,3,4-tetrahydrobenzofuro[2,3-c]pyridyl, 1,2,3,4-tetrahydro-9H-pyrido[3,4-b]indolyl, 5,6,7,8-tetrahydro-1,3-dioxolo[4,5-g]isoquinolyl and 1,2,4,5-tetrahydro-3H-benzo[d]azepinyl.

(x) The substituted lower alkyl, the substituted lower alkenyl, the substituted lower alkynyl, the substituted lower alkanoyl and the alkylene moiety of the substituted aralkyl have 1 to 3 substituents which are the same or different, such as cycloalkyl, aryl, substituted aryl [the substituted aryl has 1 to 3 substituents (xi) which are the same or different, such as lower alkyl, cycloalkyl, aryl, lower alkanoyl, a heterocyclic group, lower alkoxy, aryloxy, lower alkanoyloxy, lower alkoxycarbonyl, halogen, cyano, nitro, hydroxy, carboxy, amino, trihalogeno-lower alkyl, mono- or di-lower alkylamino or lower alkylsulfonyl], lower alkanoyl, substituted lower alkanoyl [the substituted lower alkanoyl has 1 to 3 substituents (xii) which are the same or different, such as cycloalkyl, aryl, lower alkanoyl, a heterocyclic group, lower alkoxy, aryloxy, lower alkanoyloxy, lower alkoxycarbonyl, halogen, cyano, nitro, hydroxy, carboxy, amino or mono- or di-lower alkylamino], lower alkoxy, substituted lower alkoxy [the substituent in the substituted lower alkoxy has the same meaning as the above-described substituent (xii) in the substituted lower alkanoyl], aryloxy, substituted aryloxy [the substituent in the substituted aryloxy has the same meaning as the above-described substituent (xi) in the substituted aryl], lower alkanoyloxy, lower alkoxycarbonyl, arylamino, substituted arylamino [the substituent in the substituted arylamino has the same meaning as the above-described substituent (xi) in the substituted aryl], mono- or di-lower alkylamino (the two lower alkyl moieties of the di-lower alkylamino may be the same or different), substituted mono- or di-lower alkylamino {the two lower alkyl moieties of the di-lower alkylamino may be the same or different, and examples of the substituent in the substituted mono- or di-lower alkylamino include aryl, substituted aryl [the substituent in the substituted aryl has the same meaning as the above-described substituent (xi) in the substituted aryl] and heterocyclic groups}, halogen, cyano, nitro, hydroxy or carboxy.

The lower alkyl moiety of the lower alkyl, lower alkoxycarbonyl, trihalogeno-lower alkyl, mono- or di-lower alkylamino, lower alkylsulfonyl and lower alkoxy; the cycloalkyl; the aryl moiety of the aryl, aryloxy and arylamino; the lower alkanoyl moiety of the lower alkanoyl and lower alkanoyloxy; and the heterocyclic group mentioned herein have the same meanings as the above-described lower alkyl (i), cycloalkyl (ii), aryl (vii), lower alkanoyl (v) and heterocyclic group (viii), respectively, and the halogen moiety (xiii) of the halogen and trihalogeno-lower alkyl means fluorine, chlorine, bromine and iodine atoms. The halogen in the definition of the groups in formula (II) also has the same meaning as the halogen moiety (xiii).

(xiv) Examples of the substituent in the substituted cycloalkyl and the alkylene mnoiety of heterocycle-lower alkyl include heterocyclic groups and substituted heterocyclic groups [the substituent in the substituted heterocyclic group has the same meaning as the above-described substituent (xi) in the substituted aryl] in addition to the groups mentioned in the definition of the substituent (x) in the alkylene moiety of the substituted lower alkyl and substituted aralkyl described above.

The heterocyclic group mentioned herein has the same meaning as the above-described heterocyclic group (viii).

(xv) Examples of the substituent in the aryl moiety of the substituted aryl, substituted aroyl and substituted aralkyl, in the heterocyclic group moiety of the substituted heterocyclic group, substituted aromatic heterocyclic group, substituted alicyclic heterocyclic group and substituted heterocycle-lower alkyl and in the substituted heterocyclic

group formed together with the adjacent nitrogen atom include lower alkyl, substituted lower alkyl [examples of the substituent in the substituted lower alkyl include -C(=O)-N(-$R^{X1}$)(-$R^{X2}$) (wherein $R^{X1}$ and $R^{X2}$ are the same or different, and each represents lower alkyl or aryl) in addition to the groups mentioned in the definition of the substituent (xii) in the substituted lower alkanoyl described above] and heterocycle-carbonyl in addition to the groups mentioned in the definition of the substituent (xiv) in the alkylene moiety of the substituted cycloalkyl and heterocycle-lower alkyl described above.

The lower alkyl and aryl mentioned herein have the same meanings as the above-described lower alkyl (i) and aryl (vii), respectively, and the heterocyclic group moiety of the heterocycle-carbonyl has the same meaning as the above-described heterocyclic group (viii).

Hereinafter, the compounds represented by general formulae (I) and (II) are referred to as Compounds (I) and (II), respectively. This applies to compounds of other formula numbers.

The pharmaceutically acceptable salts of Compounds (I) and (II) are preferably non-toxic and water soluble, and include acid addition salts including inorganic acid salts, such as a hydrochloride, a hydrobromide, a nitrate, a sulfate and a phosphate, and organic acid salts, such as a benzenesulfonate, a benzoate, a citrate, a fumarate, a gluconate, a lactate, a maleate, a malate, an oxalate, a methanesulfonate and a tartrate; alkali metal salts, such as a sodium salt and a potassium salt; alkaline earth metal salts, such as a magnesium salt and a calcium salt; ammonium salts, such as ammonium and tetramethylammonium; organic amine addition salts, such as an addition salt of morpholine or piperidine; amino acid addition salts, such as an addition salt of glycine, phenylalanine, lysine, asparatic acid or glutamic acid; and the like.

The production processes of Compounds (I) and (II) are explained below.

In the production processes described below, when the defined groups undergo changes under the reaction conditions or are not suitable to carry out the processes, production can be easily performed by applying means usually used in synthetic organic chemistry, such as protection of functional groups, removal of protecting groups, etc. [e.g. T. W. Greene, Protective Groups in Organic Synthesis, John Wiley & Sons Inc. (1981)].

Compound (I) can be produced through a series of reactions described below.

Compound (II) can be produced in a manner similar to that in the production of Compound (I) described below.

Production Process 1:

Compound (XI) which is the common starting compound in the synthesis of Compound (I) can be produced according to the following steps.

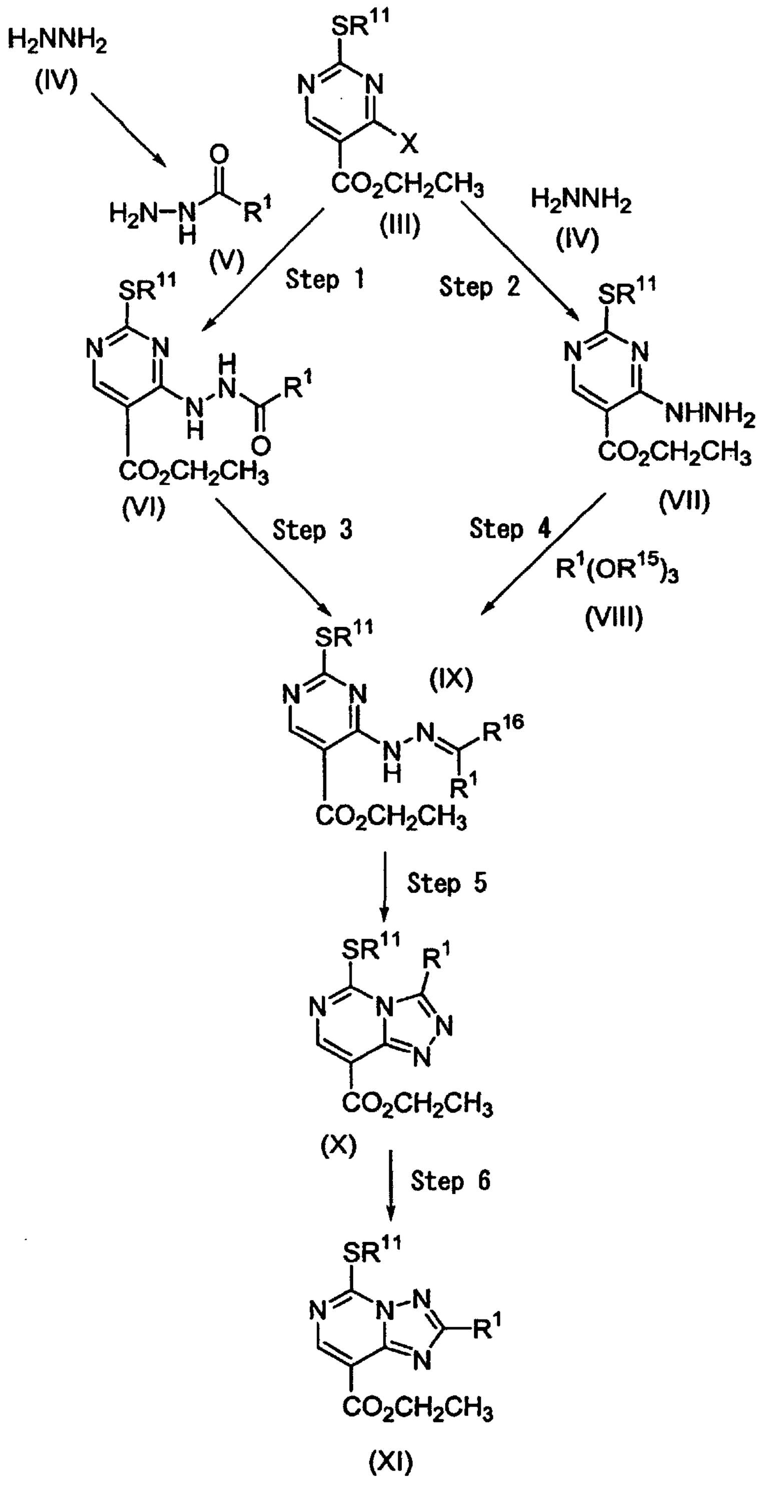


<wherein $R^1$ and $R^{11}$ each have the same meanings as defined above, $R^{15}$ represents lower alkyl [the lower alkyl has the same meaning as the above-described lower alkyl (i), among which methyl, ethyl, propyl and the like are preferred] or phenyl, $R^{16}$ represents halogen [the halogen has the same meaning as the above-described halogen moiety (xiii)], substituted or unsubstituted lower alkylsulfonyloxy {the lower alkyl moiety of the lower alkylsulfonyloxy has the same meaning as the above-described lower alkyl (i), and the substituted lower alkylsulfonyloxy has 1 to 3 substituents, such as halogen [the halogen has the same meaning as the above-described halogen moiety (xiii)]}, lower alkoxy [the lower alkyl moiety of the lower alkoxy has the same meaning as the above-described lower alkyl (i), among which methoxy,

ethoxy, propoxy and the like are preferred] or phenyloxy, and X represents halogen [the halogen has the same meaning as the above-described halogen moiety (xiii)]>

Step 1:

Compound (VI) can be obtained by reacting Compound (III) with 1 to 5 equivalents, preferably 1 to 2 equivalents of Compound (V) in a solvent in the presence of a base at a temperature between 0°C and 50°C, preferably between 0°C and room temperature for 0.5 to 5 hours, preferably for 0.5 to 2 hours.
Examples of the base include 1 to 10 equivalents, preferably 1 to 2 equivalents of triethylamine, diisopropylethylamine, pyridine, 1,8-diazabicyclo[5.4.0]undeca-7-ene (DBU), sodium methoxide, sodium ethoxide and potassium tert-butoxide, among which DBU or potassium tert-butoxide is preferred.
Examples of the solvent include diethyl ether, tetrahydrofuran (THF), dioxane, ethyl acetate, dichloromethane, chloroform, N,N-dimethylformamide (DMF) and dimethyl sulfoxide (DMSO), which may be used alone or as a mixture thereof. Among these, DMF or DMSO is preferred.
The starting Compound (III) is obtained in accordance with a known method [Chemical Abstracts, Vol. 54, 6732C (1960)], and the starting Compound (V) is obtained from hydrazine (IV) in accordance with a known method [Comprehensive Organic Transformation, VCH Publishers, Inc.: New York, p. 819 (1989)].

Step 2:

Compound (VII) can be obtained by reacting Compound (III) with 1 to 20 equivalents, preferably 1 to 5 equivalents of hydrazine (IV) in a solvent at a temperature between 0°C and the boiling point of the solvent used, preferably under reflux, for 0.5 to 5 hours, preferably for 0.5 to 2 hours.
Examples of the solvent include methanol, ethanol, THF, dichloromethane, chloroform and DMF, which may be used alone or as a mixture thereof. Among these, ethanol is preferred.

Step 3:

Compound (IX) in which $R^{16}$ is halogen or substituted or unsubstituted lower alkylsulfonyloxy can be obtained by reacting Compound (VI) obtained in Step 1 with 1 equivalent to a large excess, preferably 1 to 10 equivalents of phosphorus oxychloride, polyphosphoric acid, trimethylsilyl polyphosphate (PPSE), phosphorus pentachloride, phosphorus pentabromide, trifluoromethanesulfonic anhydride, methanesulfonic anhydride, or the like in a solvent at a temperature between room temperature and the boiling point of the solvent used, preferably at the boiling point of the solvent used.
Examples of the solvent include THF, ethyl acetate, dichloromethane, chloroform, DMF, N,N'-dimethylethylene urea, N,N'-dimethylpropylene urea, benzene, toluene and xylene, which may be used alone or as a mixture thereof.
When the solvents other than DMF are used, it is possible to allow 1 to 10 equivalents of DMF to be concomitantly present to promote the reaction.
PPSE is prepared from 1 equivalent to a large excess, preferably 1 to 10 equivalents of polyphosphoric acid and hexamethyldisiloxane.
Compound (IX) thus obtained is sometimes highly reactive and may be converted directly to Compound (X) and further to Compound (XI) under the reaction conditions of this step.

Step 4:

Compound (IX) in which $R^{16}$ is lower alkoxy or phenyloxy can also be obtained by reacting Compound (VII) obtained in Step 2 with 1 equivalent to a large excess, preferably a large excess of orthoester (VIII) without a solvent or in a solvent, preferably without a solvent at a temperature between room temperature and the boiling point of the solvent used, preferably under reflux.
Examples of the solvent include THF, ethyl acetate, dichloromethane, chloroform, DMF, benzene, toluene and xylene, which may be used alone or as a mixture thereof.
Compound (IX) thus obtained is sometimes highly reactive and may be converted directly to Compound (X) and further to Compound (XI) under the reaction conditions of this step.

Step 5:

Compound (X) can be obtained by allowing Compound (IX) obtained in Step 3 or 4 to cyclize in a solvent at a temperature between room temperature and the boiling point of the solvent used, preferably under reflux.

Examples of the solvent include benzene, toluene, xylene, chloroform, DMF, N,N'-dimethylethylene urea and N,N'-dimethylpropylene urea, which may be used alone or as a mixture thereof. Among these, xylene is preferred.
Compound (X) can also be obtained by allowing Compound (IX) to cyclize in a solvent in the presence of a base or a protonic acid at a temperature between room temperature and the boiling point of the solvent used.
Examples of the solvent include diethyl ether, THF, dichloromethane, chloroform, benzene, toluene and xylene, which may be used alone or as a mixture thereof.
Examples of the base include triethylamine, diisopropylethylamine, DBU, sodium hydroxide, potassium hydroxide and lithium hydroxide, and those of the protonic acid include formic acid, acetic acid, hydrochloric acid, sulfuric acid, p-toluenesulfonic acid and methanesulfonic acid.
Compound (X) thus obtained is sometimes highly reactive and may be converted directly to Compound (XI) under the reaction conditions of this step.

Step 6:

Compound (XI) can be obtained by treating Compound (X) obtained in Step 5 in a solvent at a temperature between room temperature and the boiling point of the solvent used, preferably under reflux.
Examples of the solvent include benzene, toluene, xylene, chloroform, DMF, N,N'-dimethylethylene urea and N,N'-dimethylpropylene urea, which may be used alone or as a mixture thereof. Among these, xylene is preferred.
Compound (XI) can also be obtained by treating Compound (X) in a solvent in the presence of a base or a protonic acid at a temperature between room temperature and the boiling point of the solvent used.
Examples of the solvent include diethyl ether, THF, dichloromethane, chloroform, benzene, toluene and xylene, which may be used alone or as a mixture thereof.
Examples of the base include triethylamine, diisopropylethylamine, DBU, sodium hydroxide, potassium hydroxide and lithium hydroxide, and those of the protonic acid include formic acid, acetic acid, hydrochloric acid, sulfuric acid, p-toluenesulfonic acid and methanesulfonic acid.

Production Process 2:

Compound (Ia-ia), i.e. Compound (I) in which -Q- is -N=C(-$R^{7B}$)- [wherein $R^{7B}$ represents -N(-$R^9$) (-$R^{10}$) (wherein $R^9$ and $R^{10}$ each have the same meanings as defined above)], and p is 1 can be produced according to the following steps.

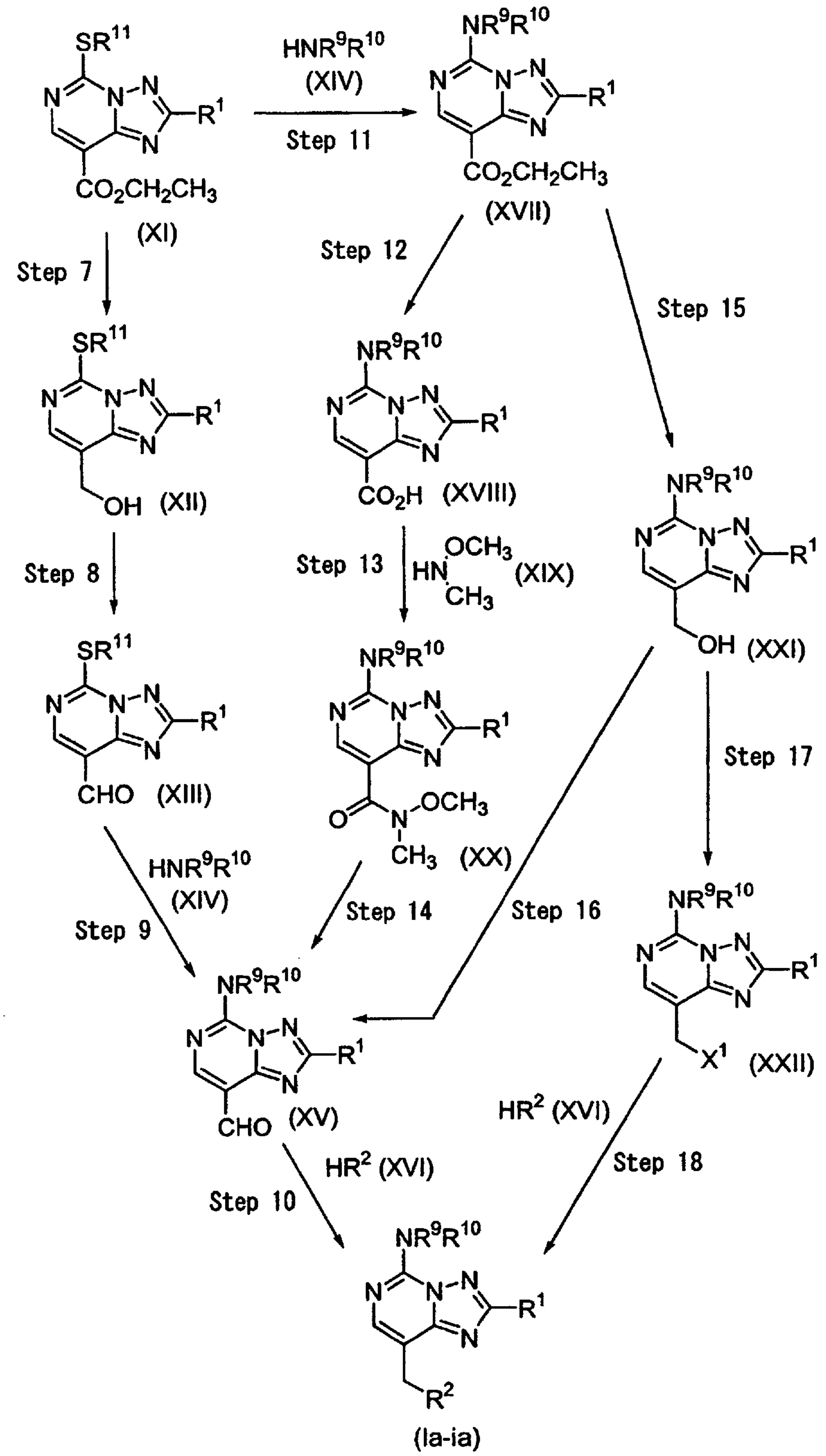


<wherein $R^1$, $R^2$, $R^9$, $R^{10}$ and $R^{11}$ each have the same meanings as defined above, and $X^1$ represents halogen [the halogen has the same meaning as the above-described halogen moiety (xiii)], substituted or unsubstituted lower alkylsulfonyloxy {the lower alkyl moiety of the lower alkylsulfonyloxy has the same meaning as the above-described lower alkyl (i), and the substituted lower alkylsulfonyloxy has 1 to 3 substituents, such as halogen [the halogen has the same meaning as the above-described halogen moiety (xiii)]} or substituted or unsubstituted arylsulfonyloxy {the aryl moiety of the arylsulfonyloxy has the same meaning as the above-described aryl (vii), and the substituted arylsulfonyloxy has

1 to 3 substituents, such as halogen [the halogen has the same meaning as the above-described halogen moiety (xiii)], hydroxy, nitro and lower alkyl [the lower alkyl has the same meaning as the above-described lower alkyl (i)]}>

Step 7:

Compound (XII) can be obtained by reducing Compound (XI) obtained in Step 6 in an inert solvent in the presence of 1 to 10 equivalents, preferably 1 to 3 equivalents of a reducing agent at a temperature between -78°C and the boiling point of the solvent used, preferably between 0°C and room temperature.
Examples of the inert solvent include diethyl ether, THF, dioxane and dichloromethane, which may be used alone or as a mixture thereof.
Examples of the reducing agent include lithium aluminum hydride and diisopropylaluminum hydride.

Step 8:

Compound (XIII) can be obtained by oxidizing Compound (XII) obtained in Step 7 according to a method generally used in organic synthesis.
For example, Compound (XIII) can be obtained by oxidizing Compound (XII) in a solvent in the presence of 1 equivalent to a large excess, preferably 1 to 5 equivalents of an oxidizing agent at a temperature between 0°C and the boiling point of the solvent used for 0.5 to 10 hours, preferably for 1 to 3 hours.
Examples of the solvent include petroleum ether, hexane, pentane, dichloromethane, dichloroethane, chloroform, toluene, benzene, THF and diethyl ether, which may be used alone or as a mixture thereof.
Examples of the oxidizing agent include manganese dioxide, chromium oxide, pyridinium chlorochromate (PCC), pyridinium dichromate (PDC), metachloroperbenzoic acid (mCPBA) and tert-butylhydroperoxide (TBHP). In addition to these, trifluoroacetic anhydride, acetic anhydride, dicyclohexylcarbodiimide (DCC), Dess-Martin reagent (1,1,1-triacetoxy-1,1-dihydro-1,2-benziodoxol-3(1H)-one; J. Am. Chem. Soc., Vol. 113, p. 7277 (1991)), DMSO activated by sulfur trioxide-pyridine complex ($SO_3 \cdot Py$), etc. can also be used.
It is also possible to obtain Compound (XIII) directly, not via Compound (XII), by treating Compound (XI) obtained in Step 6 in the presence of a reducing agent such as diisopropylaluminum hydride, etc. at a low temperature.

Step 9:

Compound (XV) can be obtained by reacting Compound (XIII) obtained in Step 8 with 1 to 10 equivalents, preferably 1 to 5 equivalents of Compound (XIV) in a solvent in the presence or absence of water at a temperature between room temperature and the boiling point of the solvent used, preferably under reflux, for 1 to 10 hours, preferably for 1 to 3 hours.
Examples of the solvent include methanol, ethanol, propanol, THF, chloroform, dichloroethane, DMF, N-methylpyrrolidone, 1,2-dimethoxyethane (DME) and diglyme, which may be used alone or as a mixture thereof.
It is also possible to carry out a similar reaction on a plate using a technique of combinatorial chemistry, and Compound (XV) can be obtained by properly adding a resin such as benzoylchloride polymer bound or poly(4-vinylpyridine) to the reaction system to remove excess Compound (XIV).

Step 10:

Compound (Ia-ia) can be obtained from Compound (XV) obtained in Step 9 and Compound (XVI) by the method of reductive amination, which is generally used in organic synthesis.
For example, Compound (Ia-ia) can be obtained by reacting Compound (XV) with 1 to 10 equivalents, preferably 1 to 3 equivalents of Compound (XVI) in an inert solvent in the presence of 1 to 10 equivalents, preferably 1 to 3 equivalents of a reducing agent, if necessary, with the addition of an acid such as hydrochloric acid or acetic acid.
Examples of the inert solvent include methanol, ethanol, dichloromethane, dichloroethane, THF, diethyl ether and DMF, which may be used alone or as a mixture thereof.
Examples of the reducing agent include sodium borohydride, sodium triacetoxyborohydride and sodium cyanoborohydride.
It is also possible to carry out a similar reaction on a plate using a technique of combinatorial chemistry, and Compound (Ia-ia) can be obtained by properly adding a resin such as benzoylchloride polymer bound or poly(4-vinylpyridine) to the reaction system to remove excess Compound (XVI).

Step 11:

Compound (XVII) can be obtained from Compound (XI) obtained in Step 6 and Compound (XIV) according to a method similar to Step 9.

Step 12:

Compound (XVIII) can be obtained by treating Compound (XVII) obtained in Step 11 with 1 to 50 equivalents, preferably 5 to 20 equivalents of a base in a solvent at a temperature between room temperature and the boiling point of the solvent used, preferably under reflux, for 1 to 10 hours, preferably for 1 to 2 hours.
Examples of the base include lithium hydroxide, potassium hydroxide, sodium hydroxide and calcium hydroxide.
Examples of the solvent include water, methanol, ethanol, DMF and THF, which may be used alone or as a mixture thereof.
This step can also be performed by the hydrolysis of esters commonly used.
For example, Compound (XVIII) can be obtained by hydrolyzing Compound (XVII) in a solvent suitable for each reaction under acidic conditions using, for example, hydrochloric acid, sulfuric acid, boron trichloride, acetic acid, formic acid, trifluoroacetic acid or p-toluenesulfonic acid, or under neutral conditions using, for example, lithium iodide, lithium bromide, alkylthiol, alkylselenol or trimethylsilane iodide.

Step 13:

Compound (XX) can be obtained by converting Compound (XVIII) obtained in Step 12 to the acid chloride thereof and reacting the obtained acid chloride with Compound (XIX).
For example, Compound (XVIII) is converted to the acid chloride thereof by treating the compound with thionyl chloride or phosphoryl chloride in the extent of 1 equivalent to an amount used as a solvent, or 1 to 20 equivalents of phosphorus pentachloride or oxalyl chloride, preferably using thionyl chloride in an amount used as a solvent, at a temperature between -15°C and the boiling point of the solvent used, preferably between 0°C and 50°C. Compound (XX) can be obtained by reacting the resulting acid chloride with 1 to 5 equivalents of Compound (XIX) in a solvent in the presence or absence of 1 equivalent to a large excess, preferably 1 to 10 equivalents of a base at a temperature between 0°C and the boiling point of the solvent used, preferably between 0°C and room temperature for 0.5 to 10 hours, preferably for 0.5 to 2 hours.
Examples of the solvent include dichloromethane, dichloroethane, chloroform, ethyl acetate, THF, benzene, toluene, DMF, acetonitrile and pyridine, which may be used alone or as a mixture thereof. Among these, dichloromethane or chloroform is preferred.
Examples of the base include triethylamime, diisopropylethylamine, pyridine and sodium hydroxide.
Compound (XX) can also be obtained by reacting Compound (XVIII) with 1 to 10 equivalents, preferably 2 to 3 equivalents of Compound (XIX) in the presence or absence of 1 to 10 equivalents, preferably 2 to 3 equivalents of hydroxybenzotriazol monohydrate using 1 to 10 equivalents, preferably 2 to 3 equivalents of a condensing agent such as dicyclohexylcarbodiimide or N-(3-dimethylaminopropyl)-N'-ethylcarbodiimide in a solvent such as dichloromethane, dichloroethane, THF or DMF at a temperature between -15°C and the boiling point of the solvent used, preferably at room temperature for 0.5 to 12 hours, preferably for 1 to 3 hours.

Step 14:

Compound (XV) can also be obtained from Compound (XX) obtained in Step 13 according to a method similar to Step 7.

Step 15:

Compound (XXI) can be obtained from Compound (XVII) obtained in Step 11 according to a method similar to Step 7.

Step 16:

Compound (XV) can also be obtained from Compound (XXI) obtained in Step 15 according to a method similar to Step 8.

Step 17:

Compound (XXII) can be obtained by reacting Compound (XXI) in a solvent or without a solvent with thionyl chloride, phosphoryl chloride, phosphorus pentoxide, methanesulfonic acid chloride, p-toluenesulfonic acid chloride, or the like in the extent of 1 equivalent to a large excess, preferably 1 to 5 equivalents, or in an amount used as a solvent in the case of no solvent, in the presence or absence of 1 equivalent to a large excess of a base.
Examples of the solvent include ethyl acetate, THF, dichloromethane, dichloroethane, chloroform, benzene, toluene, pyridine and DMF, which may be used alone or as a mixture thereof.
Examples of the base include triethylamine, diisopropylethylamine, pyridine, 2,6-lutidine, potassium carbonate, potassium hydrogencarbonate, sodium carbonate, sodium hydrogencarbonate and lithium chloride.

Step 18:

Compound (Ia-ia) can also be obtained by reacting Compound (XXII) obtained in Step 17 with 1 to 10 equivalents, preferably 1 to 5 equivalents of Compound (XVI) in a solvent in the presence or absence of a base at a temperature between 0°C and the boiling point of the solvent used for 1 to 24 hours.
In this case, the reaction can be promoted by allowing a salt such as sodium iodide or potassium iodide to be concomitantly present.
Examples of the solvent include diethyl ether, THF, ethyl acetate, acetonitrile, dioxane, dichloromethane, dichloroethane, chloroform and DMF, which may be used alone or as a mixture thereof.
Examples of the base include triethylamine, diisopropylethylamine, pyridine, potassium carbonate and sodium carbonate.

Production Process 3:

Compound (Ia-ib), i.e. Compound (I) in which -Q- is -N=C(-$R^{7B}$)- [wherein $R^{7B}$ represents -N(-$R^9$)(-$R^{10}$) (wherein $R^9$ and $R^{10}$ each have the same meanings as defined above)], and p is 2 or 3 can be produced according to the following steps.

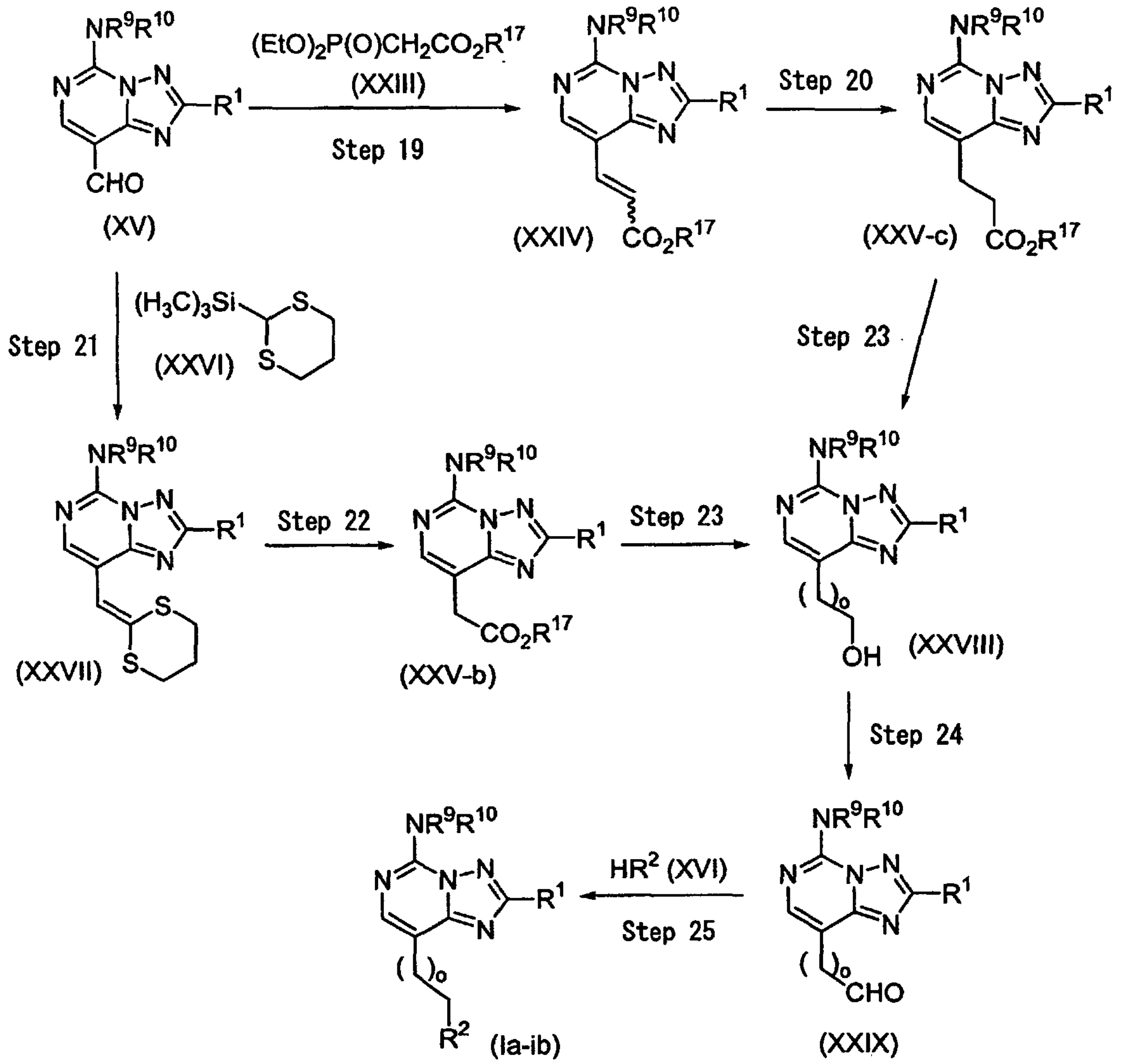


{wherein $R^1$, $R^2$, $R^9$ and $R^{10}$ each have the same meanings as defined above, Et represents ethyl, o represents 1 or 2, and $R^{17}$ represents lower alkyl [the lower alkyl has the same meaning as the above-described lower alkyl (i)]}

Step 19:

Compound (XXIV) can be obtained by reacting Compound (XV) obtained in Step 9, 14 or 16 with 1 to 5 equivalents, preferably 1 to 2 equivalents of Compound (XXIII) in a solvent in the presence of a base at a temperature between 0°C and the boiling point of the solvent used, preferably between 0°C and room temperature for 1 to 24 hours, preferably for 1 to 5 hours.
Examples of the solvent include THF, diethyl ether, dioxane, N,N'-dimethylethylene urea, N,N'-dimethylpropylene urea, benzene and toluene, which may be used alone or as a mixture thereof.
Examples of the base include sodium hydride, potassium tert-butoxide, lithium amide and lithium diisopropylamide.

Step 20:

Compound (XXV-c) can be obtained by treating Compound (XXIV) obtained in Step 19 in a solvent in a stream of hydrogen at atmospheric pressure to 100 Pa, preferably at atmospheric pressure in the presence of 0.01 to 5 times, preferably 0.05 to 0.5 times weight of a catalyst at a temperature between room temperature and the boiling point of the solvent used, preferably at room temperature for 0.5 to 24 hours, preferably for 1 to 5 hours.
Examples of the solvent include methanol, ethanol, ethyl acetate, THF, diethyl ether, dioxane, benzene and

DMF, which may be used alone or as a mixture thereof.

Examples of the catalyst include heterogeneous catalysts such as palladium-carbon, Adams' catalyst ($PtO_2$) and Raney nickel, and homogeneous catalysts such as Wilkinson's complex [chloro(triphenylphosphine)rhodium(I)].

Step 21:

Compound (XXVII) can be obtained by reacting Compound (XV) obtained in Step 9, 14 or 16 with 1 to 10 equivalents, preferably 1 to 2 equivalents of Compound (XXVI) in a solvent in the presence of a base at a temperature between -90°C and room temperature, preferably between -78°C and -40°C.

Examples of the solvent include THF, diethyl ether, dioxane, N,N'-dimethylethylene urea and N,N'-dimethylpropylene urea, which may be used alone or as a mixture thereof.

Examples of the base include sodium hydride, potassium tert-butoxide, n-butyl lithium, lithium amide and lithium diisopropylamide.

Step 22:

Compound (XXV-b) can be obtained by treating Compound (XXVII) obtained in Step 21 with mercury bichloride, mercury oxide, cadmium carbonate, N-chlorosuccinimide, N-bromosuccinimide, N-iodosuccinimide, silver nitrate, sodium perchlorate, hydrogen peroxide, 2,3-dichloro-5,6-dicyano-1,4-benzoquinone (DDQ), cerium (III) diammonium nitrate (CAN), thallium nitrate, bis(trifluoromethanesulfonyl)iodobenzene, bis(trifluoroacetoxy)iodobenzene, or the like in a solvent at a temperature between -78°C and the boiling point of the solvent used, preferably between -78°C and room temperature.

Examples of the solvent include water, acetonitrile, methanol, ethanol, dichloromethane and chloroform, which may be used alone or as a mixture thereof. Among these, a mixed solvent of methanol and water is preferred.

Step 23:

Compound (XXVIII) can be obtained from Compound (XXV-c) or Compound (XXV-b) obtained in Step 20 or 22 according to a method similar to Step 7.

Step 24:

Compound (XXIX) can be obtained from Compound (XXVIII) obtained in Step 23 according to a method similar to Step 8.

Step 25:

Compound (Ia-ib) can be obtained from Compound (XXIX) obtained in Step 24 according to a method similar to Step 10.

Production Process 4:

Compound (Ia-ii), i.e. Compound (I) in which -Q- is -N=C(-$R^{7C}$)- [wherein $R^{7C}$ represents -S(-$R^{11}$) (wherein $R^{11}$ has the same meaning as defined above)], Compound (Ib-i), i.e. Compound (I) in which -Q- is -NH-C(=O)-, or Compound (Ib-ii), i.e. Compound (I) in which -Q- is -N(-$R^{12C}$)-C(=O)- (wherein $R^{12C}$ has the same meaning as the above-described $R^{12}$ except one hydrogen atom is removed therefrom) can be produced according to the following steps.

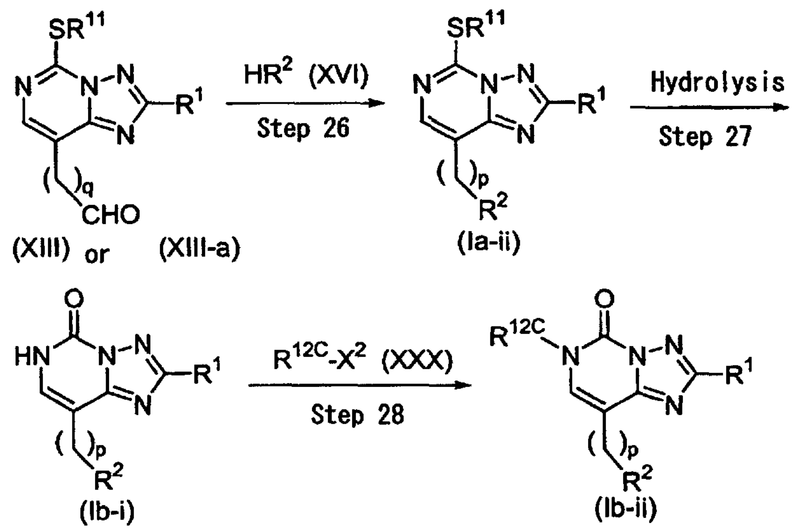


(XIII) or (XIII-a)

(Ia-ii)

(Ib-i)

(Ib-ii)

(wherein q represents p-1; p, $R^1$, $R^2$, $R^{11}$ and $R^{12C}$ each have the same meaning as defined above; and $X^2$ has the same meaning as the above-described $X^1$)

Step 26:

Compound (Ia-ii) can be obtained from Compound (XIII) obtained in Step 8 or Compound (XIII-a) obtained from Compound (XIII) according to methods similar to Steps 19 to 24 and Compound (XVI) according to a method similar to Step 10.

Step 27:

Compound (Ib-i) can be obtained by treating Compound (Ia-ii) obtained in Step 26 in a solvent or without a solvent in the presence of 1 equivalent to a large excess of aqueous ammonia, an acid or a base at a temperature between room temperature and the boiling point of the solvent used for 0.5 to 10 hours, preferably for 1 to 4 hours.

Examples of the solvent include water, diethyl ether, methanol, ethanol, THF and DMF, which may be used alone or as a mixture thereof.

Examples of the acid include acetic acid, hydrochloric acid and hydrobromic acid, and those of the base include lithium hydroxide, lithium hydroxide monohydrate, sodium hydroxide, potassium hydroxide, sodium methoxide and sodium ethoxide.

Step 28:

Compound (Ib-ii) can be obtained by reacting Compound (Ib-i) obtained in Step 27 with 1 to 5 equivalents, preferably 1 to 2 equivalents of Compound (XXX) in an inert solvent in the presence of 1 to 10 equivalents, preferably 1 to 2 equivalents of a base at a temperature between 0°C and the boiling point of the solvent used, preferably at room temperature for 1 to 24 hours, preferably for 2 to 5 hours.

Examples of the inert solvent include DMF, N,N'-dimethylethylene urea, THF and dioxane, which may be used alone or as a mixture thereof.

Examples of the base include sodium hydride, potassium tert-butoxide, lithium amide, potassium carbonate and sodium carbonate.

Compound (Ib-ii) can also be obtained by reacting Compound (Ib-i) with 1 to 5 equivalents, preferably 1 to 2 equivalents each of $R^{12}OH$ (wherein $R^{12}$ has the same meaning as defined above) and triphenylphosphine in a solvent in the presence of 1 to 5 equivalents, preferably 1 to 2 equivalents of a condensing agent at a temperature between -50°C and the boiling point of the solvent used, preferably between 0°C and room temperature for 0.5 to 24 hours, preferably for 1 to 5 hours.

Examples of the solvent include THF, dioxane, dichloromethane, chloroform, diethyl ether, DMF, N,N'-dimethylethylene urea, toluene and hexamethylphosphoric triamide, which may be used alone or as a mixture thereof. Among these, THF is preferred.

Examples of the condensing agent include azodicarboxylic acid diethyl ester, azodicarboxylic acid diisopropyl

ester and 4-methyl-1,2,4-triazolidine-3,5-dione, among which azodicarboxylic acid diethyl ester is preferred.

Production Process 5:

Some of the compounds obtained according to the above-described Production Processes 1 to 3 can be used as intermediates to prepare new Compound (I).

For example, Compound (Ia-ic), i.e. Compound (Ia-ia) or Compound (Ia-ib) in which $R^9$ and $R^{10}$ are a hydrogen atom and 3,4-dimethoxybenzyl, respectively, can be converted to Compound (Ia-id), i.e. Compound (I) in which -Q- is $-N=C(-NH_2)-$. Furthermore, Compound (Ia-ie), i.e. Compound (I) in which -Q- is $-N=C(-NH-R^{18})-$ (wherein $R^{18}$ represents substituted or unsubstituted lower alkanoyl or substituted or unsubstituted aroyl in the definition of the above-described $R^9$), can also be obtained from Compound (Ia-id).

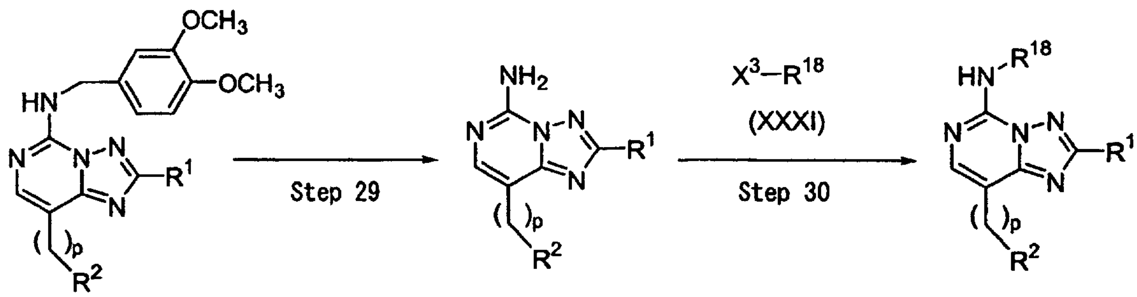


(wherein p, $R^1$, $R^2$ and $R^{18}$ each have the same meanings as defined above, and $X^3$ represents halogen [the halogen has the same meaning as the above-described halogen moiety (xiii)] or a hydroxyl group)

Step 29:

Compound (Ia-id) can be obtained by treating Compound (Ia-ic) obtained in Step 10, 18 or 25 in the presence of a catalytic amount to a large excess, preferably a catalytic amount to 5 equivalents of trifluoromethanesulfonic acid in the presence or absence of anisole in a solvent such as acetic acid, trifluoroacetic acid, dichloromethane or chloroform, preferably in trifluoroacetic acid at a temperature between room temperature and the boiling point of the solvent used, preferably between 30°C and 50°C for 1 to 24 hours, preferably for 1 to 6 hours.

This step can also be performed according to methods generally used in removing a benzyl group which is a protecting group generally used in organic synthesis, for example, hydrogenation reaction using palladium-carbon, nickel, platinum, or the like as a catalyst; the method utilizing the action of metallic sodium and using liquid ammonia as a solvent; the method using chloroformic acid vinyl ester, chloroformic acid 1-chloroethyl ester, or the like; and the method using an oxidizing agent such as DDQ, p-chloranyl (tetrahydro-1,4-benzoquinone) or mCPBA.

Step 30:

Compound (Ia-ie) can be obtained from Compound (Ia-id) obtained in Step 29 and Compound (XXXI) according to a method similar to Step 13.

Production Process 6:

Compound (Ic), i.e. Compound (I) in which -Q- is general formula (B)

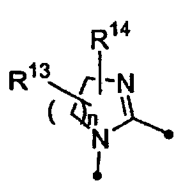

(B)

(wherein $R^{13}$, $R^{14}$ and n each have the same meanings as defined above) can be produced according to the following steps.

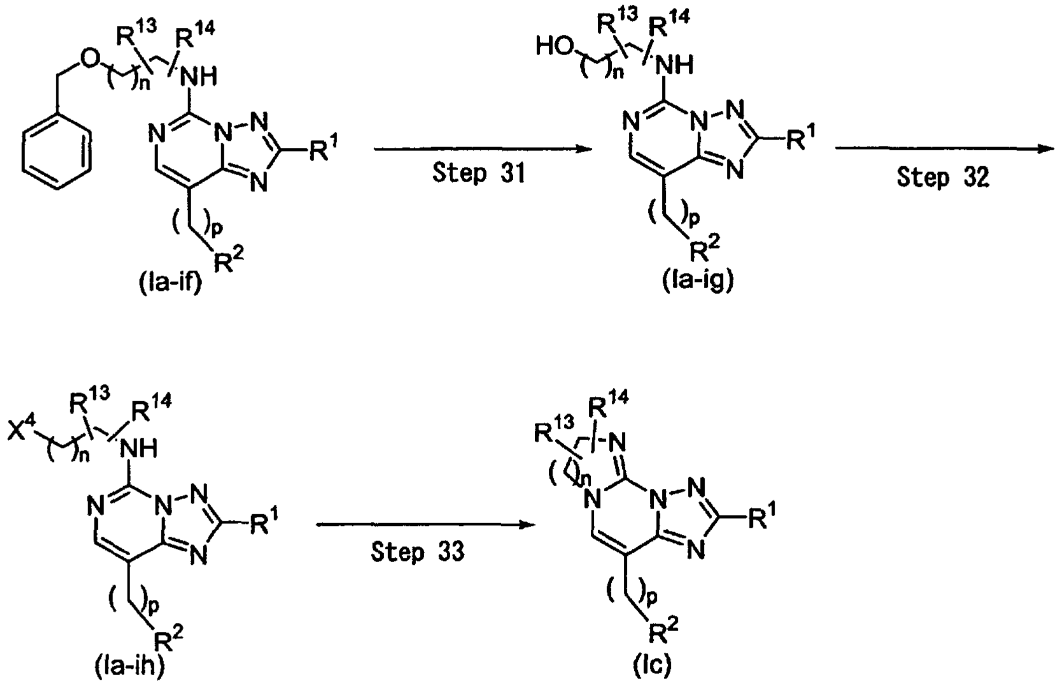


(Ia-if) (Ia-ig) (Ia-ih) (Ic)

(wherein p, $R^1$, $R^2$, $R^{13}$, $R^{14}$ and n each have the same meanings as defined above, and $X^4$ has the same meaning as the above-described $X^1$)

Step 31:

Compound (Ia-ig) can be obtained from Compound (Ia-if) obtained in Step 10, 18 or 25, for example, by hydrogenation reaction using palladium-carbon, nickel, platinum, or the like as a catalyst and by the method utilizing the action of metallic sodium and using aqueous ammonia as a solvent.

Compound (Ia-ig) can also be obtained by treating Compound (Ia-if) with a large excess of methanethiol or dimethyl sulfide and 1 to 50 equivalents, preferably 1 to 15 equivalents of boron trifluoride-diethyl ether complex in a solvent such as dichloromethane, dichloroethane or chloroform, preferably in dichloromethane at a temperature between 0°C and the boiling point of the solvent used, preferably at room temperature for 1 to 24 hours, preferably for 5 to 15 hours.

Step 32:

Compound (Ia-ih) can be obtained from Compound (Ia-ig) obtained in Step 31 according to a method similar to Step 17.

Compound (Ia-ih) thus obtained is sometimes highly reactive and may be converted directly to Compound (Ic) under the reaction conditions of this step.

Step 33:

Compound (Ic) can be obtained by treating Compound (Ia-ih) obtained in Step 32 with 1 to 10 equivalents, preferably 1 to 5 equivalents of a base in a solvent at a temperature between 0°C and the boiling point of the solvent used for 0.5 to 24 hours, preferably for 1 to 12 hours.

Examples of the solvent include dichloromethane, dichloroethane, chloroform, ethyl acetate, acetonitrile, THF, dioxane, DMF, benzene and toluene, which may be used alone or as a mixture thereof.

Production Process 7:

Compound (Ia-iii), i.e. Compound (I) in which -Q- is -N=C(-$R^{7D}$) - [wherein $R^{7D}$ represents -O(-$R^8$) (wherein $R^8$ has the same meaning as defined above)] can be produced according to the following step.

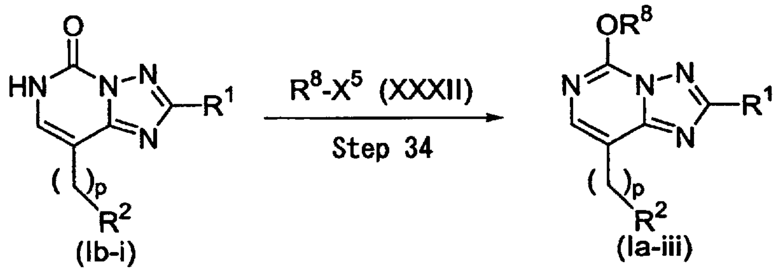


(Ib-i) (Ia-iii)

(wherein p, $R^1$, $R^2$ and $R^8$ each have the same meanings as defined above, and $X^5$ has the same meaning as the above-described $X^1$)

Step 34:

Compound (Ia-iii) can be obtained by reacting Compound (Ib-i) obtained in Step 27 with 1 to 5 equivalents, preferably 1 to 2 equivalents of Compound (XXXII) in an inert solvent in the presence of 1 to 10 equivalents, preferably 1 to 2 equivalents of a base at a temperature between 0°C and the boiling point of the solvent used, preferably at room temperature for 1 to 24 hours, preferably for 2 to 5 hours.

Examples of the inert solvent include DMF, N,N'-dimethylethylene urea, THF and dioxane, which may be used alone or as a mixture thereof.

Examples of the base include sodium hydride, potassium tert-butoxide, silver carbonate, lithium amide, potassium carbonate and sodium carbonate, among which silver carbonate [Heterocycles, Vol. 31, p. 818 (1990)] is preferred.

Compound (Ia-iii) can also be obtained by reacting Compound (Ib-i) with 1 to 5 equivalents, preferably 1 to 2 equivalents of Compound (XXXII) in a solvent in the presence of 1 to 5 equivalents, preferably 1 to 2 equivalents of a condensing agent at a temperature between -50°C and the boiling point of the solvent used, preferably between 0°C and room temperature for 0.5 to 24 hours, preferably for 1 to 5 hours.

Examples of the solvent include THF, dioxane, dichloromethane, chloroform, diethyl ether, DMF, N,N'-dimethylethylene urea, toluene and hexamethylphosphoric triamide, which may be used alone or as a mixture thereof. Among these, THF is preferred.

Examples of the condensing agent include azodicarboxylic acid diethyl ester, azodicarboxylic acid diisopropyl ester and 4-methyl-1,2,4-triazolidine-3,5-dione, among which azodicarboxylic acid diethyl ester is preferred.

The intermediates and the desired compounds in the above-described production processes can be isolated and purified by subjecting them to purification methods usually used in synthetic organic chemistry, for example, filtration, extraction, washing, drying, concentration, re-crystallization, various kinds of chromatography, such as high performance liquid chromatography, thin layer chromatography and silica gel chromatography. The intermediates can also be subjected to the subsequent reactions without purification.

For some of Compounds (I), there may exist regioisomers, geometrical isomers, optical isomers, tautomeric isomers and the like, and all possible isomers including them and mixtures thereof may be used for the receptor antagonist of the present invention.

When it is desired to obtain a salt of Compound (I), in the case where Compound (I) is produced in the form of the salt, it can be purified as it is, but where it is produced in its free form, it can be converted into a salt, after being dissolved or suspended in an appropriate solvent, by adding an appropriate acid or base and then isolated and purified.

Furthermore, Compound (I) or pharmaceutically acceptable salts thereof may exist in the form of adducts with water or various solvents, and these adducts are also used for the receptor antagonist of the present invention.

Examples of Compound (Ia) obtained by the above-described production processes are shown in Tables 1 to 3.

Table 1

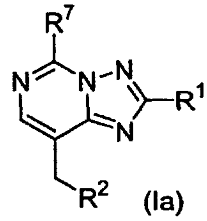

(Ia)

| Compd. No. (Ia) | $R^7$ | $R^2$ | $R^1$ |
|---|---|---|---|
| 1 | 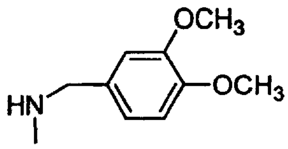 | 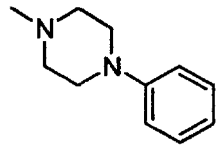 | 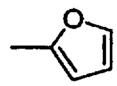 |
| 2 | 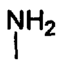 | 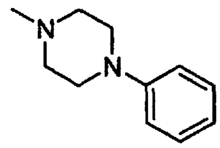 | 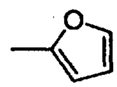 |
| 3 | 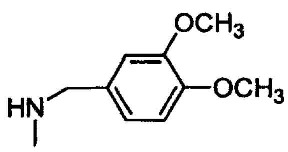 | 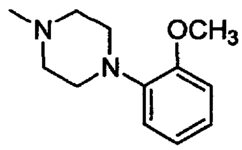 | 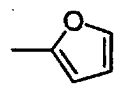 |
| 4 | 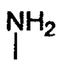 | 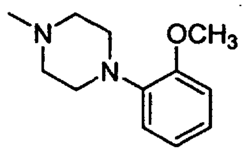 | 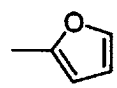 |
| 5 | 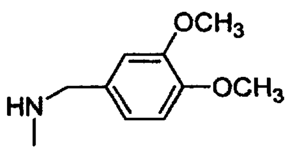 | 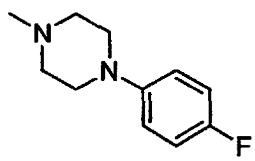 | 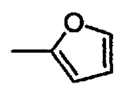 |
| 6 | 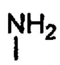 | 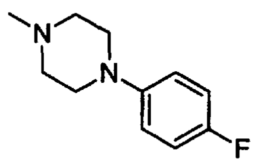 | 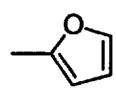 |
| 7 | 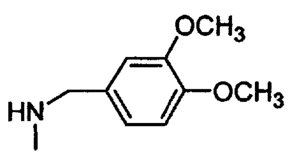 | 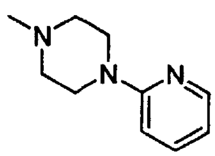 | 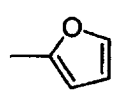 |

Contd.

| Compd. No. (Ia) | $R^7$ | $R^2$ | $R^1$ |
|---|---|---|---|
| 8 | 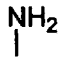 | 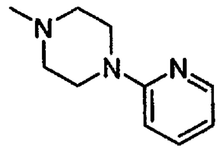 | 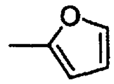 |
| 9 | 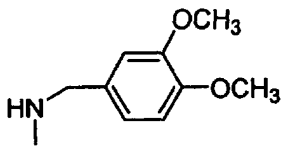 | 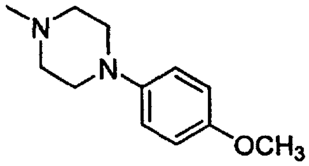 | 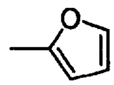 |
| 10 | 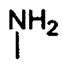 | 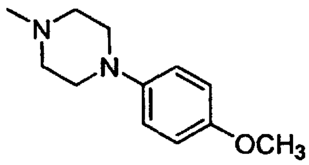 | 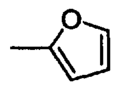 |
| 11 | 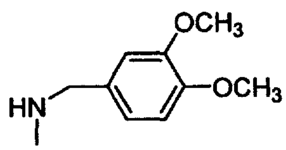 | 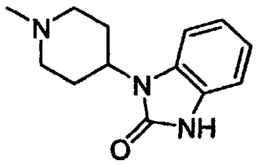 | 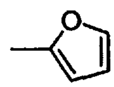 |
| 12 | 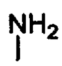 | 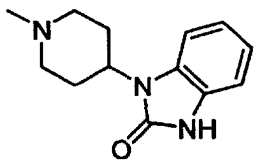 | 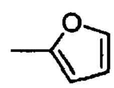 |
| 13 | 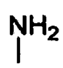 | 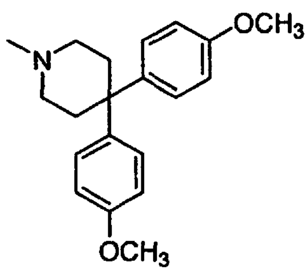 | 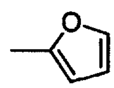 |
| 14 | 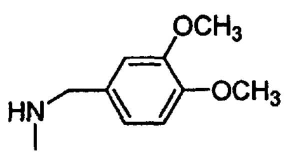 | 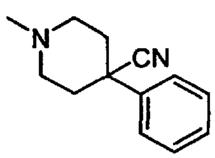 | 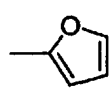 |
| 15 | 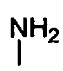 | 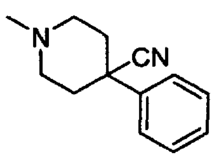 | 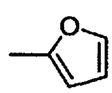 |

Contd.

| Compd. No. (Ia) | $R^7$ | $R^2$ | $R^1$ |
|---|---|---|---|
| 16 | 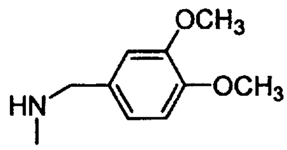 | 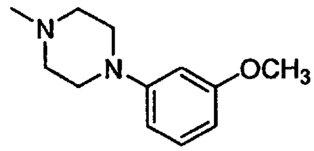 | 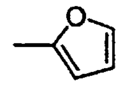 |
| 17 | 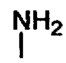 | 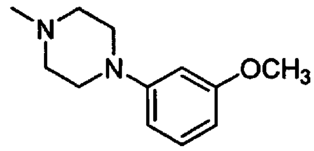 | 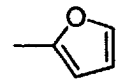 |
| 18 | 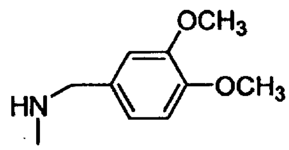 | 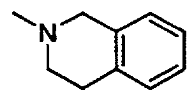 | 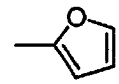 |
| 19 | 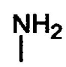 | 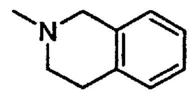 | 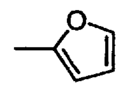 |
| 20 | 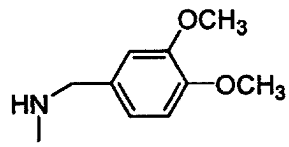 | 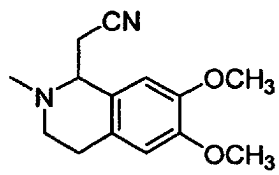 | 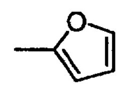 |
| 21 | 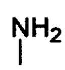 | 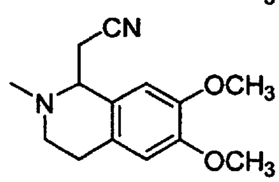 | 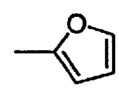 |
| 22 | 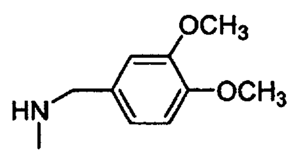 | 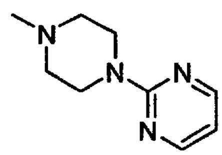 | 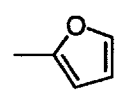 |
| 23 | 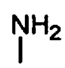 | 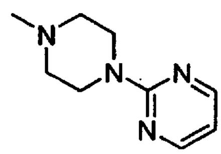 | 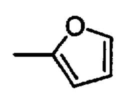 |

Contd.

| Compd. No. (Ia) | $R^7$ | $R^2$ | $R^1$ |
|---|---|---|---|
| 24 | 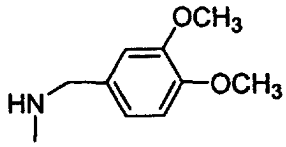 | 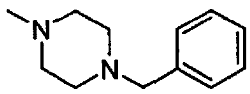 | 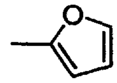 |
| 25 | 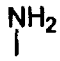 | 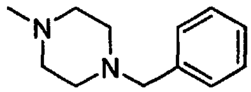 | 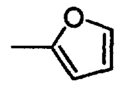 |
| 26 | 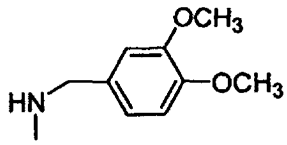 | 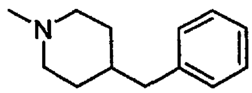 | 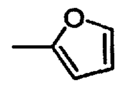 |
| 27 | 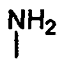 | 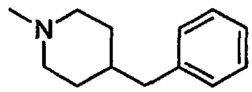 | 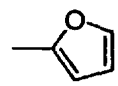 |
| 28 | 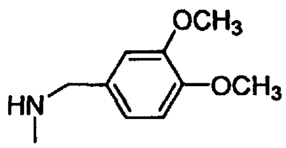 | 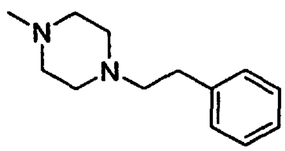 | 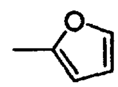 |
| 29 | 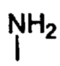 | 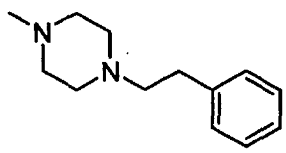 | 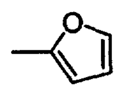 |
| 30 | 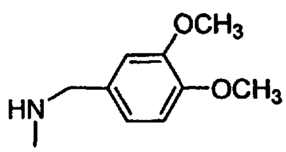 | 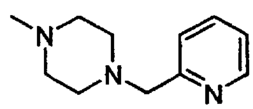 | 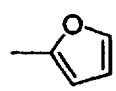 |
| 31 | 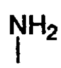 | 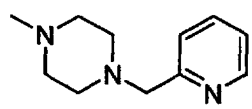 | 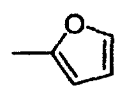 |

Contd.

| Compd. No. (Ia) | $R^7$ | $R^2$ | $R^1$ |
|---|---|---|---|
| 32 | 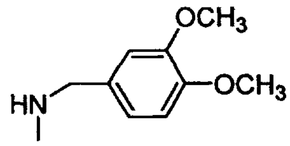 | 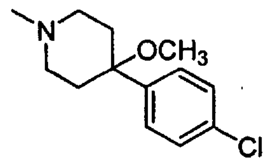 | 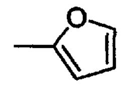 |
| 33 | 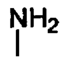 | 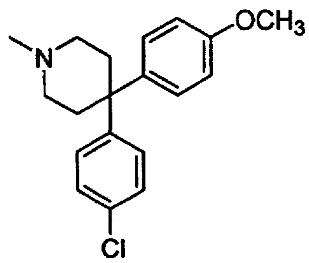 | 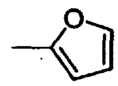 |
| 34 | 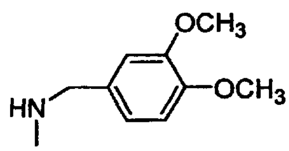 | 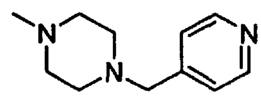 | 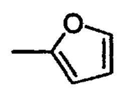 |
| 35 | 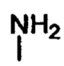 | 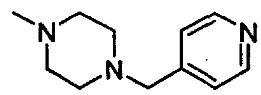 | 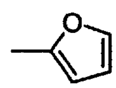 |
| 36 | 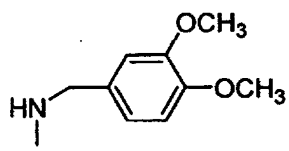 | 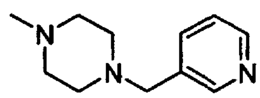 | 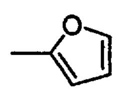 |
| 37 | 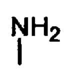 | 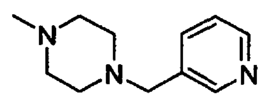 | 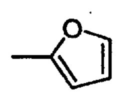 |
| 38 | 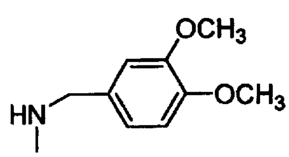 | 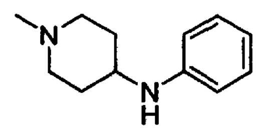 | 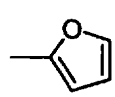 |

Contd.

| Compd. No. (1a) | $R^7$ | $R^2$ | $R^1$ |
|---|---|---|---|
| 39 | 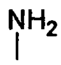 | 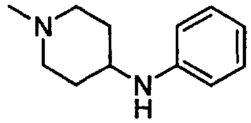 | 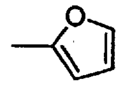 |
| 40 | 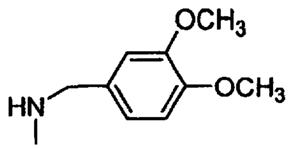 | 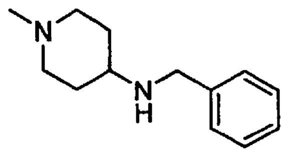 | 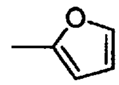 |
| 41 | 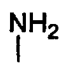 | 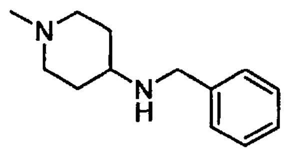 | 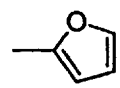 |
| 42 | 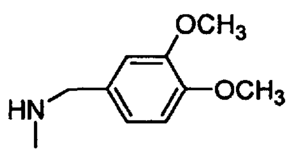 | 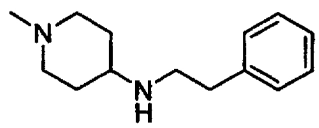 | 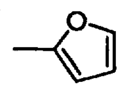 |
| 43 | 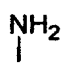 | 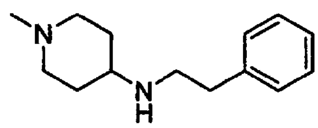 | 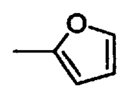 |
| 44 | 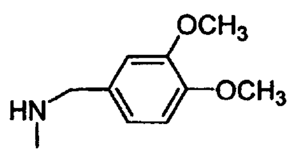 | 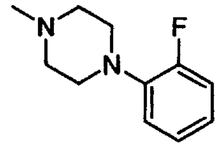 | 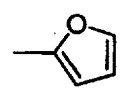 |
| 45 | 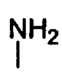 | 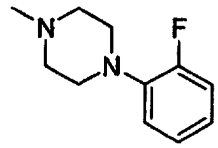 | 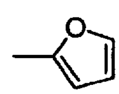 |

Contd.

| Compd. No. (Ia) | $R^7$ | $R^2$ | $R^1$ |
|---|---|---|---|
| 46 | 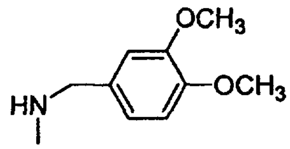 | 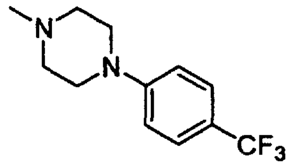 | 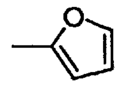 |
| 47 | 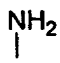 | 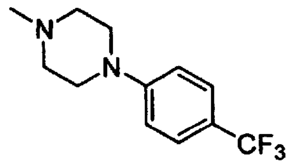 | 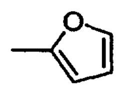 |
| 48 | 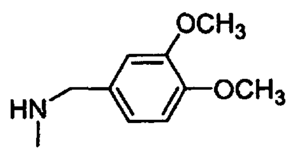 | 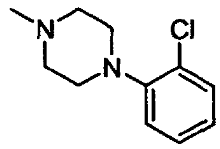 | 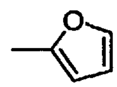 |
| 49 | 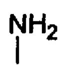 | 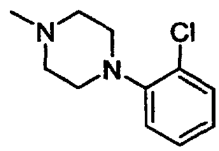 | 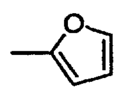 |
| 50 | 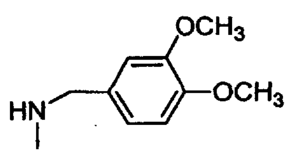 | 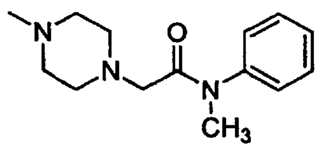 | 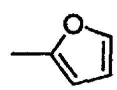 |
| 51 | 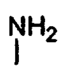 | 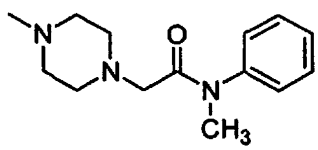 | 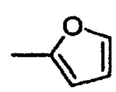 |
| 52 | 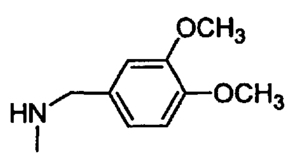 | 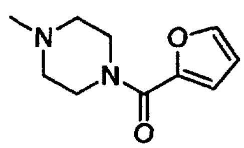 | 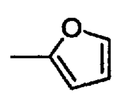 |
| 53 | 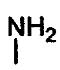 | 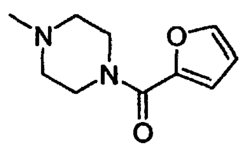 | 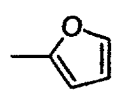 |

Contd.

| Compd. No. (Ia) | $R^7$ | $R^2$ | $R^1$ |
|---|---|---|---|
| 54 | 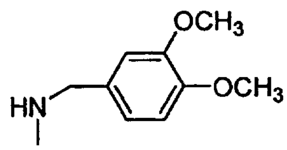 | 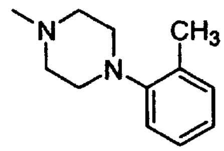 | 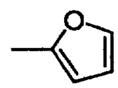 |
| 55 | 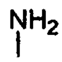 | 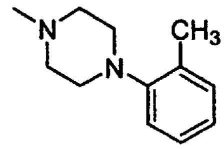 | 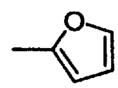 |
| 56 | 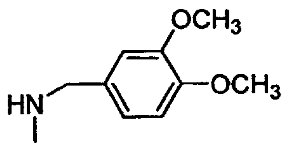 | 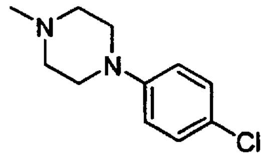 | 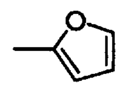 |
| 57 | 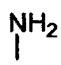 | 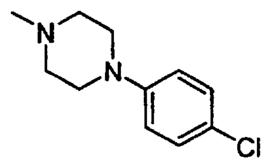 | 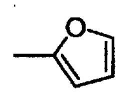 |
| 58 | 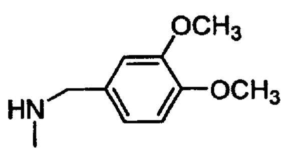 | 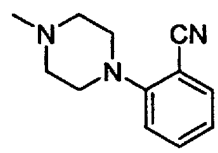 | 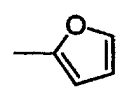 |
| 59 | 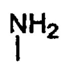 | 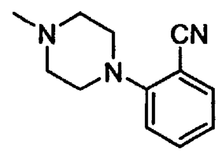 | 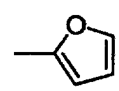 |
| 60 | 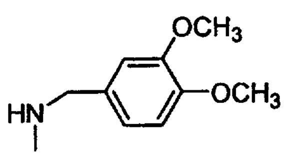 | 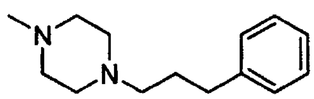 | 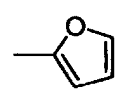 |
| 61 | 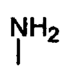 | 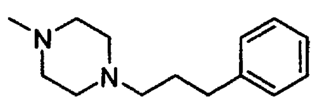 | 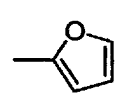 |

Contd.

| Compd. No. (Ia) | $R^7$ | $R^2$ | $R^1$ |
|---|---|---|---|
| 62 | 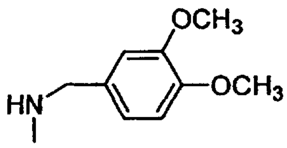 | 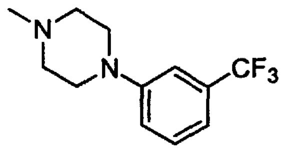 | 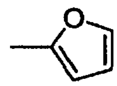 |
| 63 | 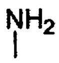 | 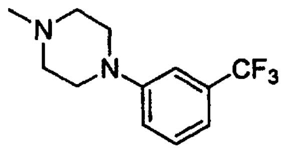 | 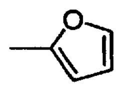 |
| 64 | 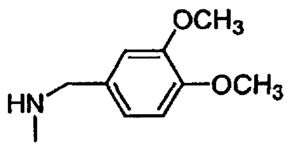 | 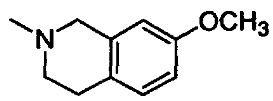 | 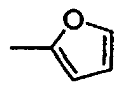 |
| 65 | 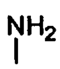 | 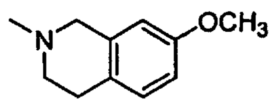 | 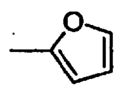 |
| 66 | 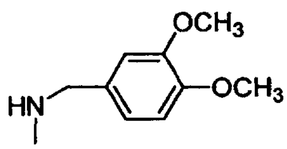 | 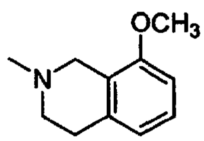 | 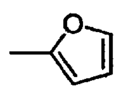 |
| 67 | 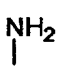 | 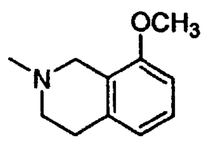 | 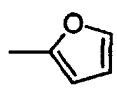 |
| 68 | 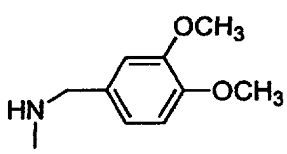 | 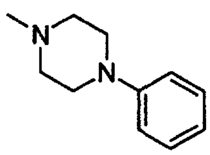 | 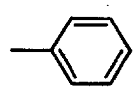 |
| 69 | 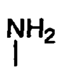 | 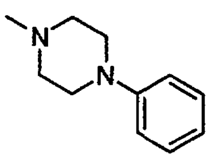 | 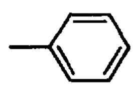 |

Contd.

| Compd. No. (Ia) | $R^7$ | $R^2$ | $R^1$ |
|---|---|---|---|
| 70 | 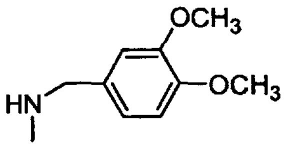 | 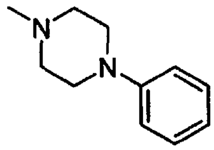 | 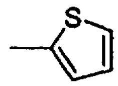 |
| 71 | 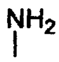 | 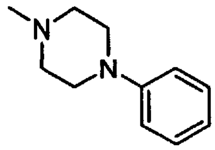 | 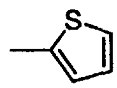 |
| 72 | 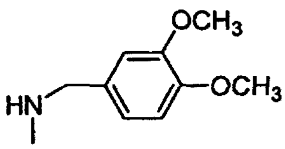 | 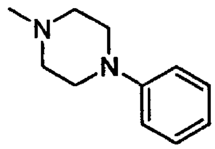 | 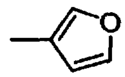 |
| 73 | 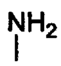 | 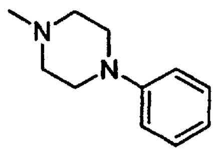 | 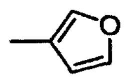 |
| 74 | 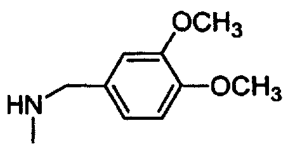 | 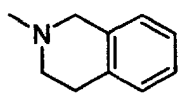 | 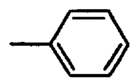 |
| 75 | 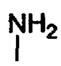 | 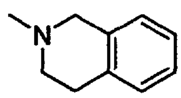 | 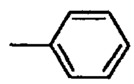 |
| 76 | 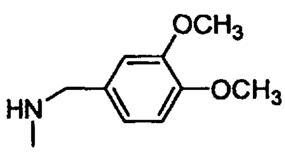 | 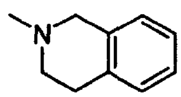 | 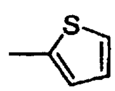 |
| 77 | 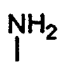 | 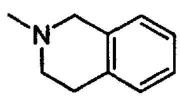 | 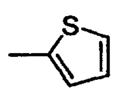 |

Contd.

| Compd. No. (Ia) | $R^7$ | $R^2$ | $R^1$ |
|---|---|---|---|
| 78 | 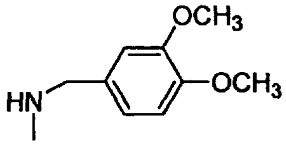 | 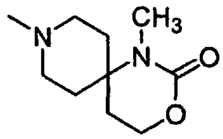 | 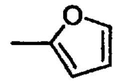 |
| 79 | 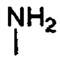 | 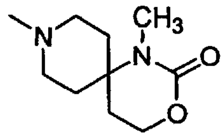 | 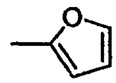 |
| 80 | 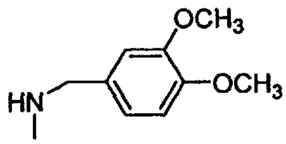 | 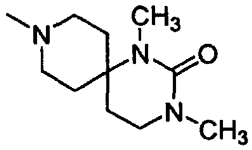 | 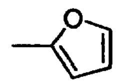 |
| 81 | 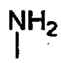 | 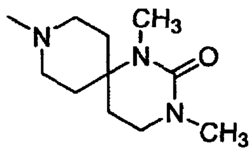 | 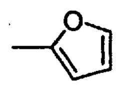 |
| 82 | 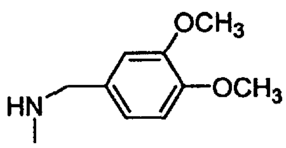 | 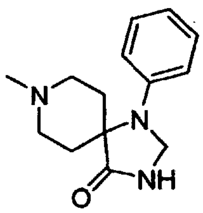 | 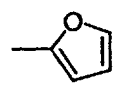 |
| 83 | 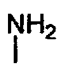 | 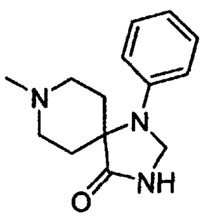 | 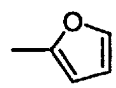 |
| 84 | 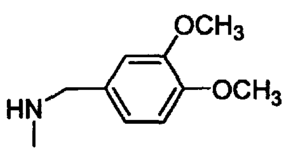 | 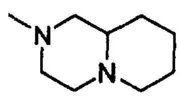 | 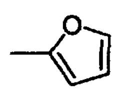 |
| 85 | 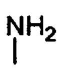 | 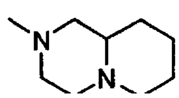 | 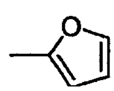 |

Contd.

| Compd. No. (Ia) | $R^7$ | $R^2$ | $R^1$ |
|---|---|---|---|
| 86 | 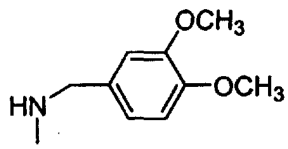 | 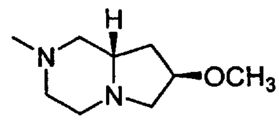 | 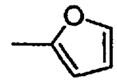 |
| 87 | 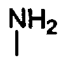 | 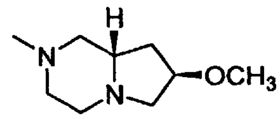 | 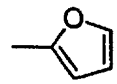 |
| 88 | 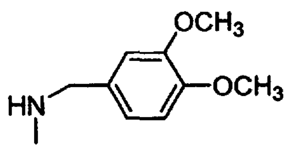 | 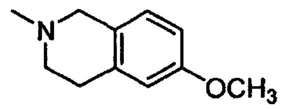 | 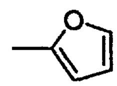 |
| 89 | 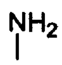 | 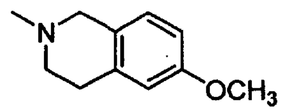 | 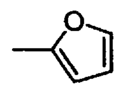 |
| 90 | 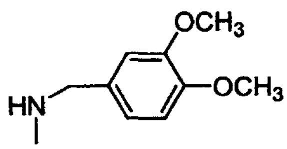 | 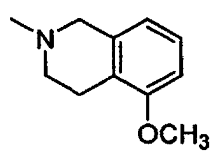 | 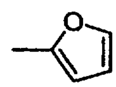 |
| 91 | 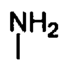 | 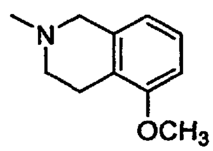 | 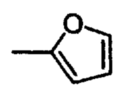 |
| 92 | 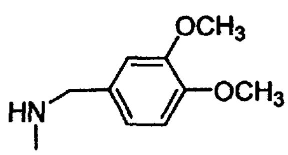 | 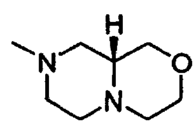 | 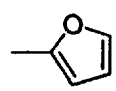 |
| 93 | 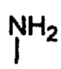 | 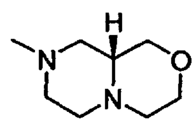 | 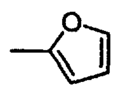 |

Contd.

| Compd. No. (Ia) | $R^7$ | $R^2$ | $R^1$ |
|---|---|---|---|
| 94 | 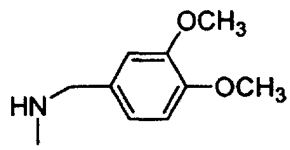 | 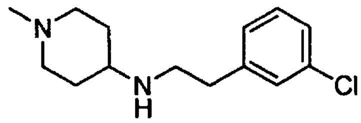 | 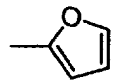 |
| 95 | 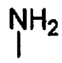 | 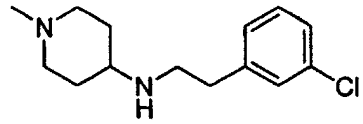 | 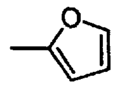 |
| 96 | 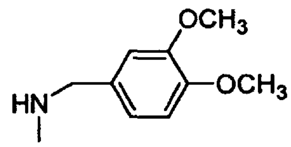 | 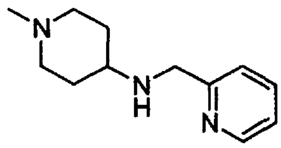 | 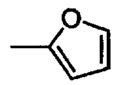 |
| 97 | 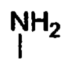 | 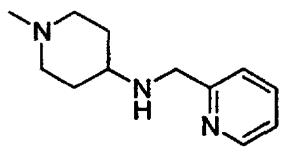 | 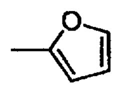 |
| 98 | 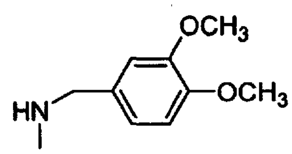 | 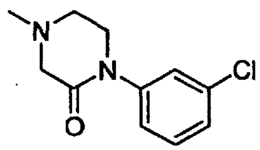 | 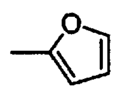 |
| 99 | 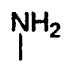 | 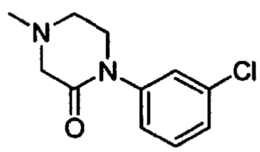 | 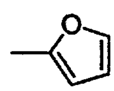 |
| 100 | 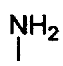 | 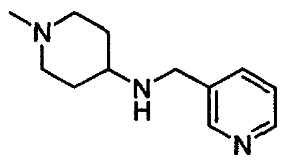 | 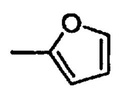 |
| 101 | 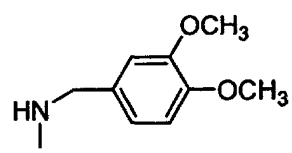 | 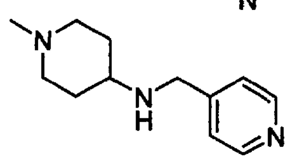 | 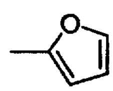 |
| 102 | 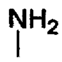 | 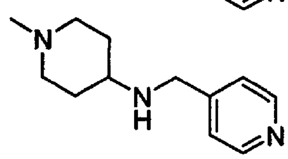 | 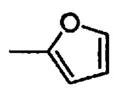 |

Contd.

| Compd. No. (Ia) | $R^7$ | $R^2$ | $R^1$ |
|---|---|---|---|
| 103 | 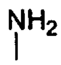 | 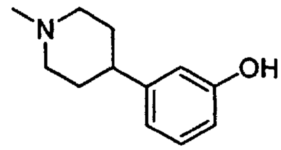 | 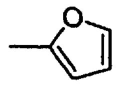 |
| 104 | 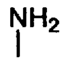 | 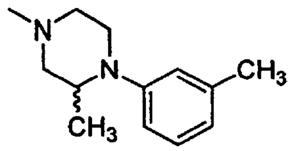 | 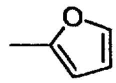 |
| 105 | 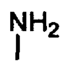 | 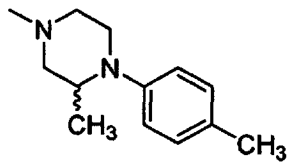 | 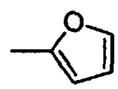 |
| 106 | 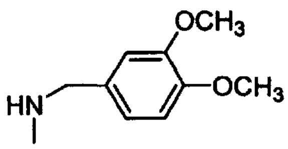 | 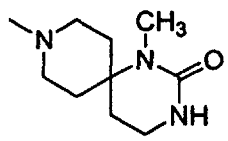 | 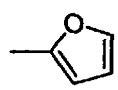 |
| 107 | 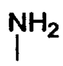 | 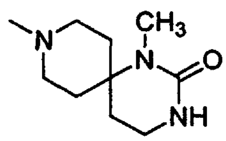 | 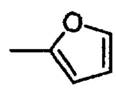 |
| 108 | 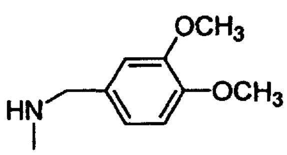 | 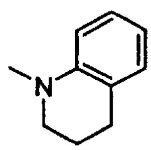 | 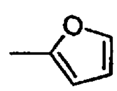 |
| 109 | 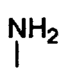 | 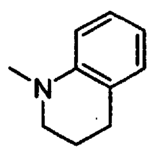 | 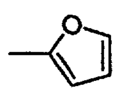 |

Contd.

| Compd. No. (Ia) | $R^7$ | $R^2$ | $R^1$ |
|---|---|---|---|
| 110 | 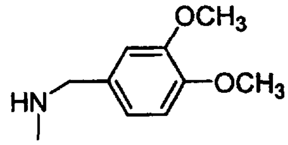 | 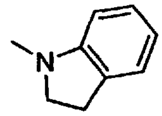 | 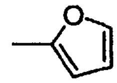 |
| 111 | 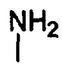 | 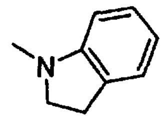 | 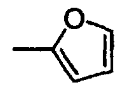 |
| 112 | 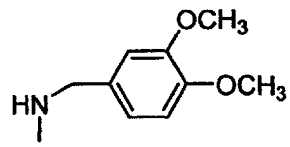 | 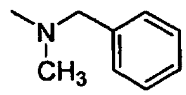 | 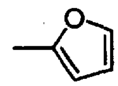 |
| 113 | 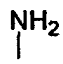 | 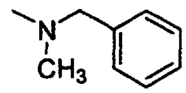 | 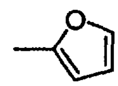 |
| 114 | 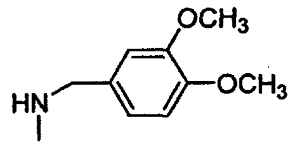 | 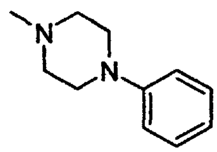 | $-CH_3$ |
| 115 | 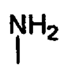 | 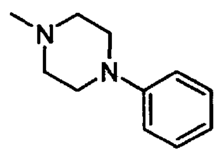 | $-CH_3$ |
| 116 | 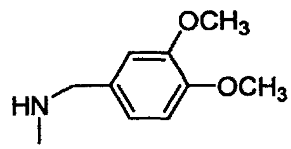 | 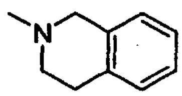 | $-CH_3$ |
| 117 | 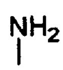 | 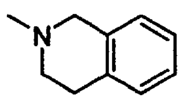 | $-CH_3$ |

Contd.

| Compd. No. (Ia) | $R^7$ | $R^2$ | $R^1$ |
|---|---|---|---|
| 118 | 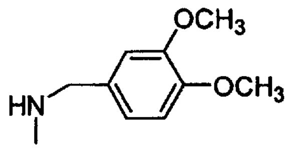 | 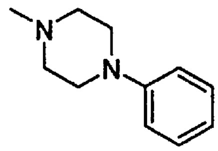 | —H |
| 119 | 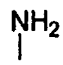 | 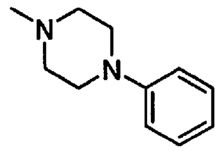 | —H |
| 120 | 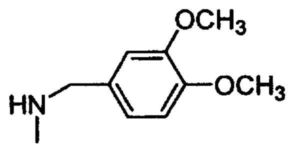 | 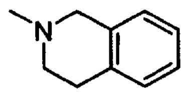 | —H |
| 121 | 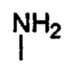 | 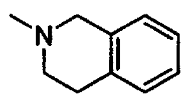 | —H |
| 122 | 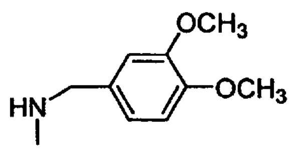 | 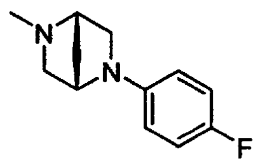 | 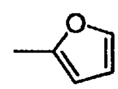 |
| 123 | 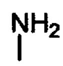 | 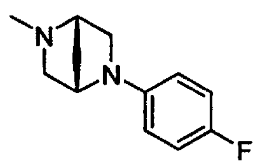 | 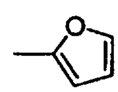 |
| 124 | 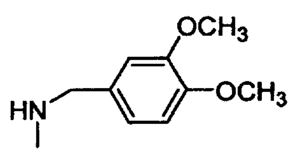 | 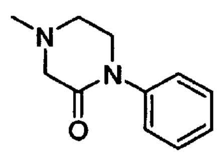 | 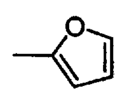 |
| 125 | 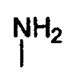 | 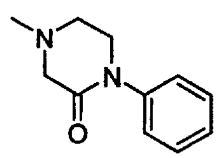 | 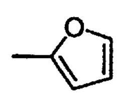 |

Contd.

| Compd. No. (Ia) | $R^7$ | $R^2$ | $R^1$ |
|---|---|---|---|
| 126 | 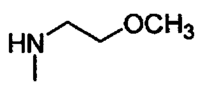 | 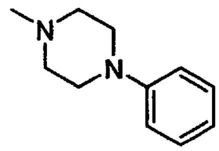 | 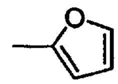 |
| 127 | 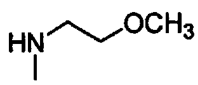 | 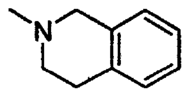 | 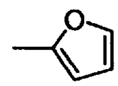 |
| 128 | 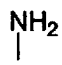 | 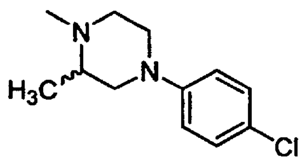 | 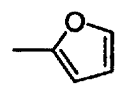 |
| 129 | 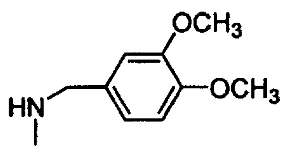 | 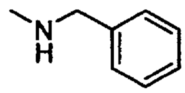 | 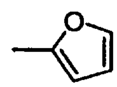 |
| 130 | 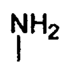 | 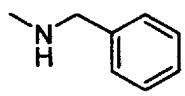 | 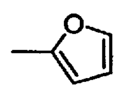 |
| 131 | 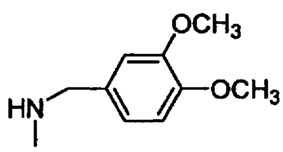 | 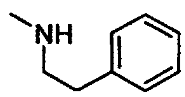 | 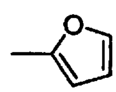 |
| 132 | 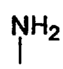 | 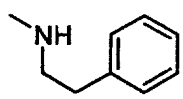 | 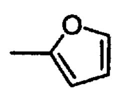 |

Contd.

| Compd. No. (Ia) | $R^7$ | $R^2$ | $R^1$ |
|---|---|---|---|
| 133 | 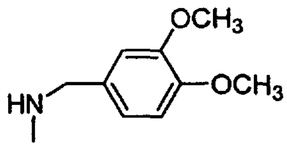 | 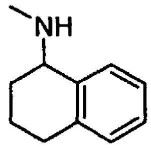 | 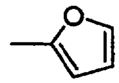 |
| 134 | 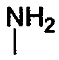 | 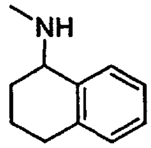 | 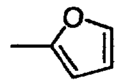 |
| 135 | 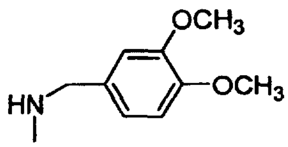 | 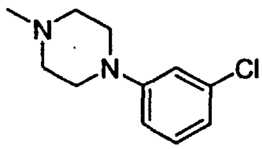 | 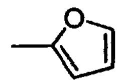 |
| 136 | 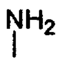 | 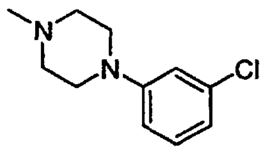 | 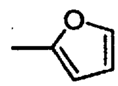 |
| 137 | 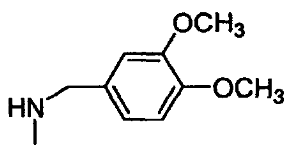 | 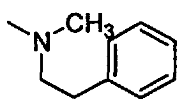 | 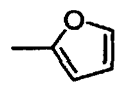 |
| 138 | 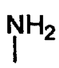 | 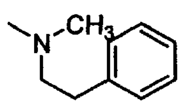 | 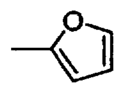 |
| 139 | 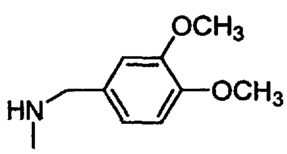 | 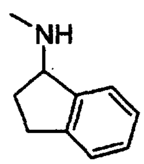 | 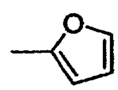 |
| 140 | 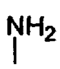 | 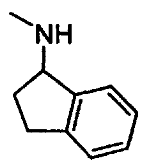 | 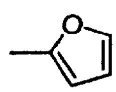 |

Contd.

| Compd. No. (Ia) | $R^7$ | $R^2$ | $R^1$ |
| --- | --- | --- | --- |
| 141 | 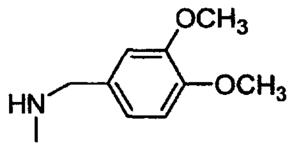 | 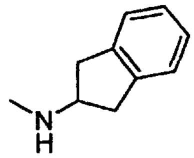 | 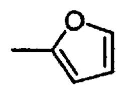 |
| 142 | 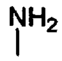 | 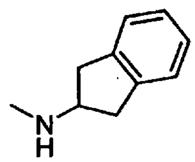 | 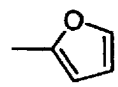 |
| 143 | 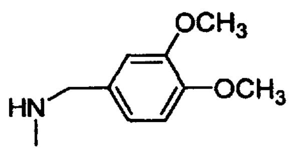 | 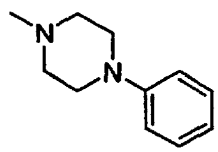 | 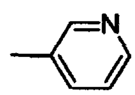 |
| 144 | 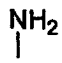 | 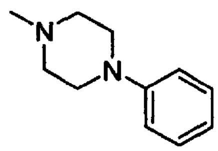 | 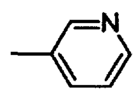 |
| 145 | 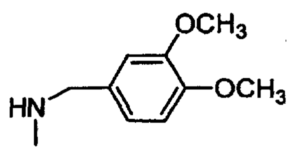 | 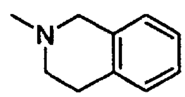 | 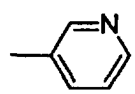 |
| 146 | 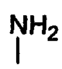 | 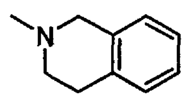 | 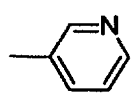 |
| 147 | 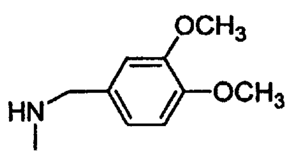 | 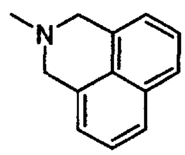 | 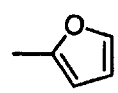 |
| 148 | 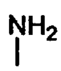 | 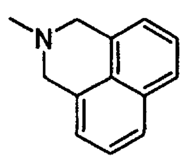 | 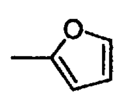 |

Contd.

| Compd. No. (Ia) | $R^7$ | $R^2$ | $R^1$ |
|---|---|---|---|
| 149 | 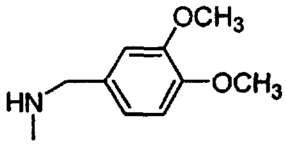 | 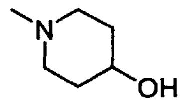 | 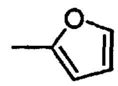 |
| 150 | 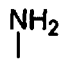 | 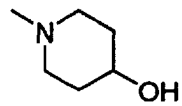 | 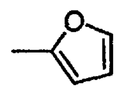 |
| 151 | 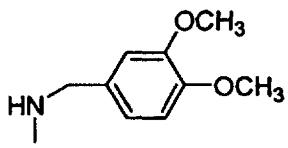 | 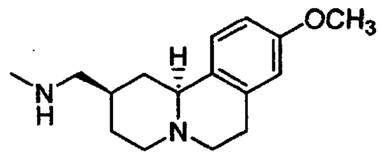 | 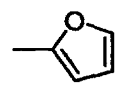 |
| 152 | 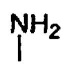 | 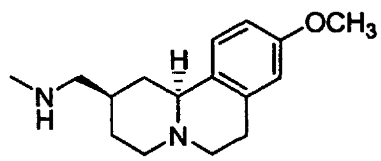 | 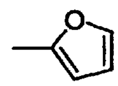 |
| 153 | 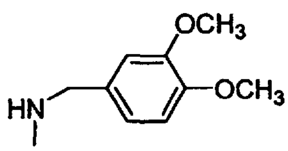 | 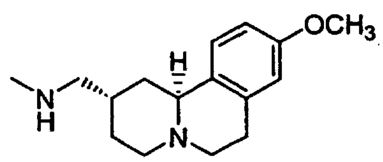 | 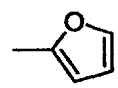 |
| 154 | 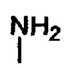 | 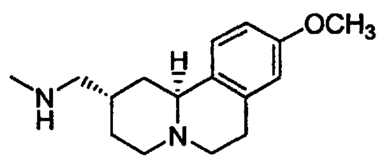 | 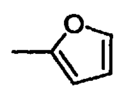 |
| 155 | 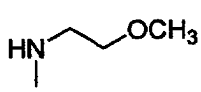 | 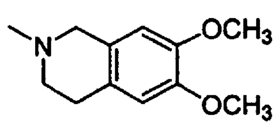 | 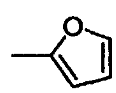 |

Contd.

| Compd. No. (Ia) | $R^7$ | $R^2$ | $R^1$ |
|---|---|---|---|
| 156 | 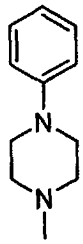 | 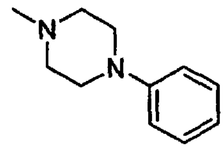 | 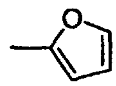 |
| 157 | 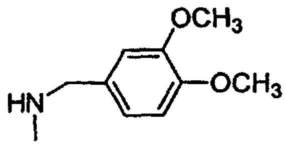 | 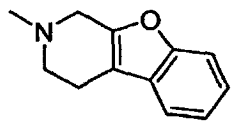 | 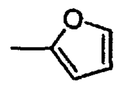 |
| 158 | 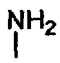 | 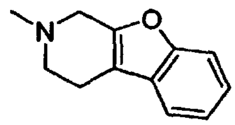 | 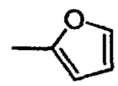 |
| 159 | 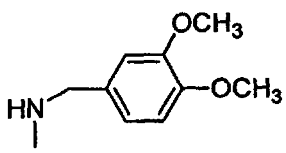 | 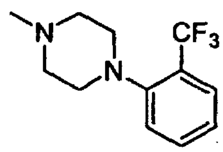 | 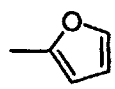 |
| 160 | 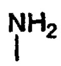 | 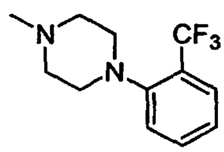 | 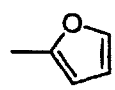 |
| 161 | 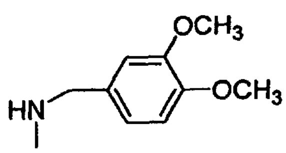 | 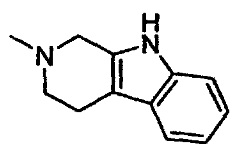 | 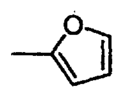 |
| 162 | 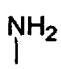 | 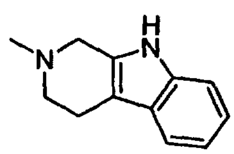 | 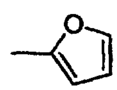 |

Contd.

| Compd. No. (Ia) | $R^7$ | $R^2$ | $R^1$ |
|---|---|---|---|
| 163 | 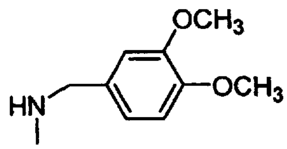 | 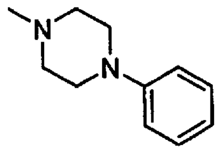 | 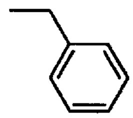 |
| 164 | 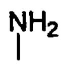 | 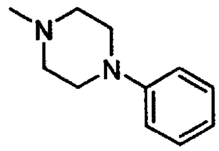 | 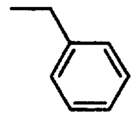 |
| 165 | 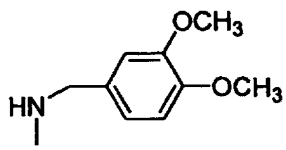 | 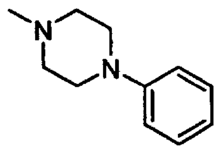 | 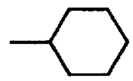 |
| 166 | 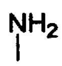 | 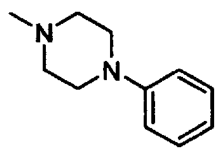 | 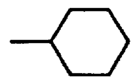 |
| 167 | 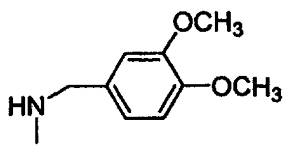 | 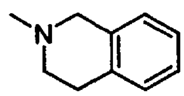 | 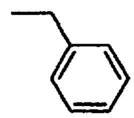 |
| 168 | 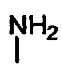 | 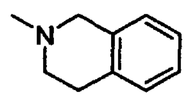 | 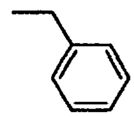 |
| 169 | 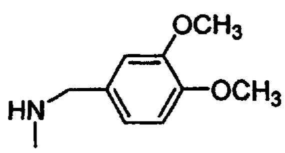 | 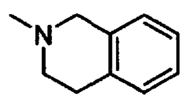 | 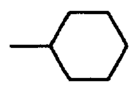 |
| 170 | 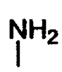 | 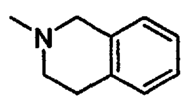 | 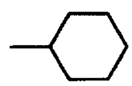 |

Contd.

| Compd. No. (Ia) | $R^7$ | $R^2$ | $R^1$ |
|---|---|---|---|
| 171 | 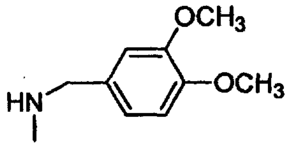 | 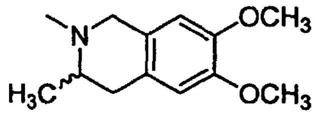 | 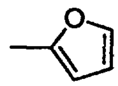 |
| 172 | 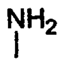 | 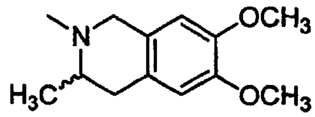 | 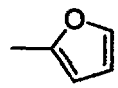 |
| 173 | 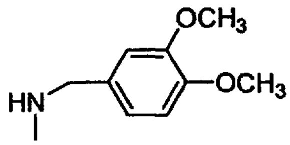 | 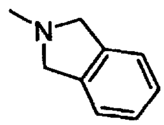 | 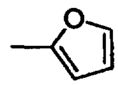 |
| 174 | 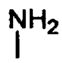 | 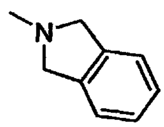 | 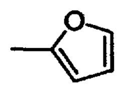 |
| 175 | 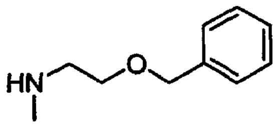 | 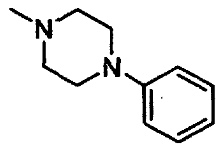 | 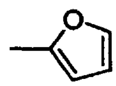 |
| 176 | 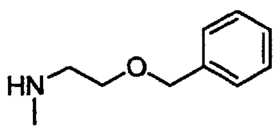 | 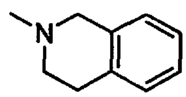 | 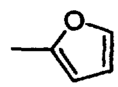 |
| 177 | 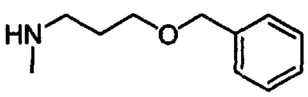 | 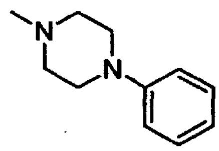 | 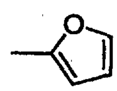 |
| 178 | 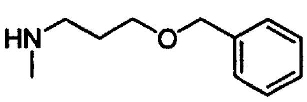 | 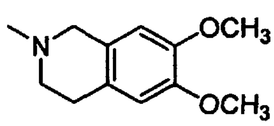 | 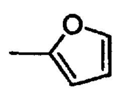 |

Contd.

| Compd. No. (Ia) | $R^7$ | $R^2$ | $R^1$ |
|---|---|---|---|
| 179 | 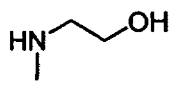 | 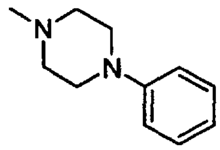 | 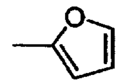 |
| 180 | 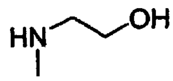 | 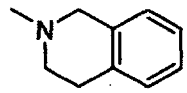 | 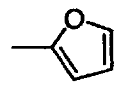 |
| 181 | 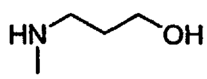 | 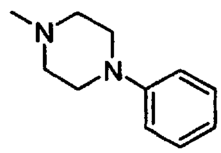 | 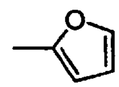 |
| 182 | 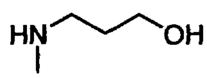 | 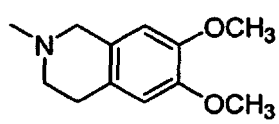 | 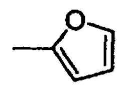 |
| 183 | 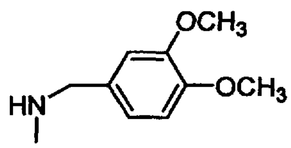 | 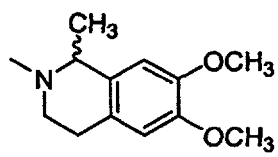 | 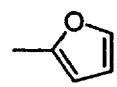 |
| 184 | 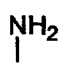 | 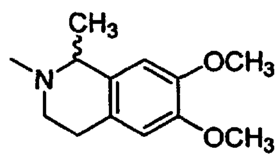 | 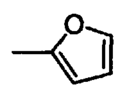 |
| 185 | 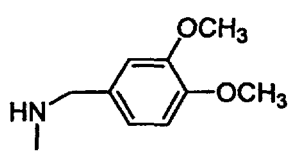 | 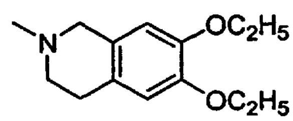 | 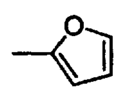 |
| 186 | 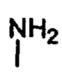 | 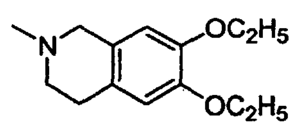 | 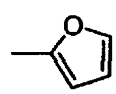 |

Contd.

| Compd. No. (Ia) | $R^7$ | $R^2$ | $R^1$ |
|---|---|---|---|
| 187 | 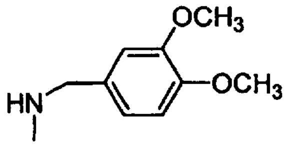 | 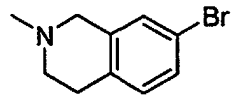 | 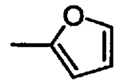 |
| 188 | $NH_2$ | 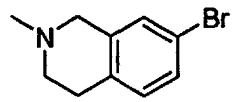 | 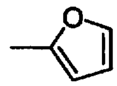 |
| 189 | $NH_2$ | 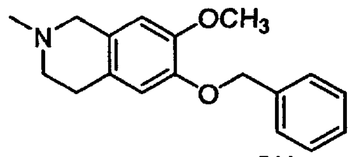 | 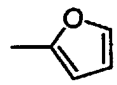 |
| 190 | 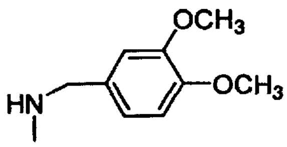 | 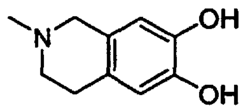 | 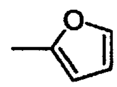 |
| 191 | $NH_2$ | 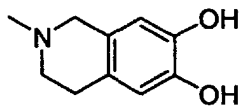 | 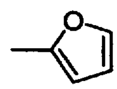 |
| 192 | 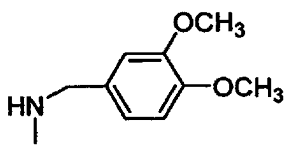 | 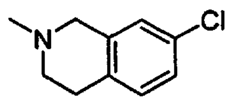 | 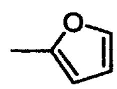 |
| 193 | $NH_2$ | 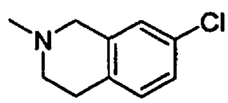 | 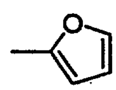 |
| 194 | 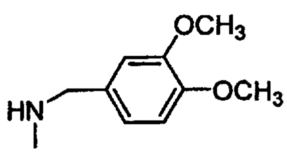 | 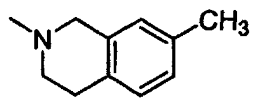 | 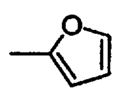 |
| 195 | $NH_2$ | 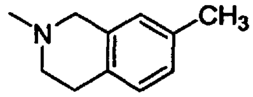 | 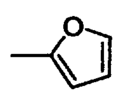 |

Contd.

| Compd. No. (Ia) | $R^7$ | $R^2$ | $R^1$ |
|---|---|---|---|
| 196 | 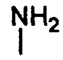 | 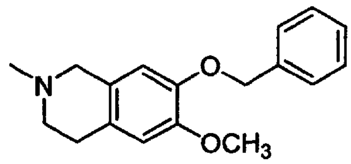 | 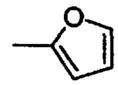 |
| 197 | 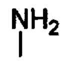 | 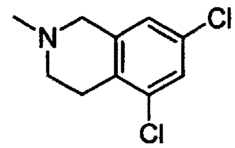 | 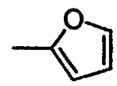 |
| 198 | 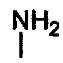 | 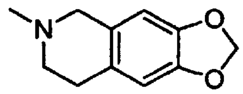 | 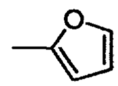 |
| 199 | 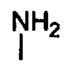 | 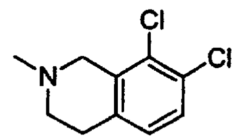 | 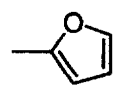 |
| 200 | 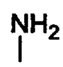 | 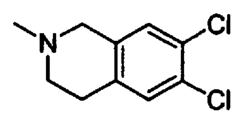 | 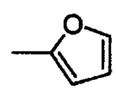 |
| 201 | 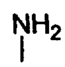 | 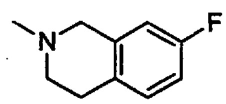 | 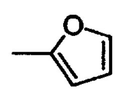 |
| 202 | 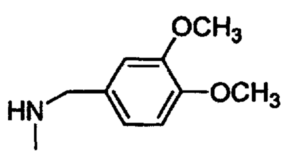 | 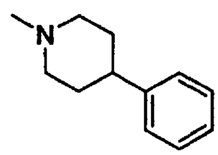 | 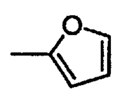 |
| 203 | 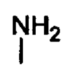 | 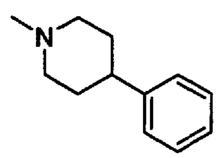 | 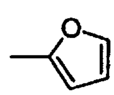 |

Contd.

| Compd. No. (Ia) | $R^7$ | $R^2$ | $R^1$ |
|---|---|---|---|
| 204 | 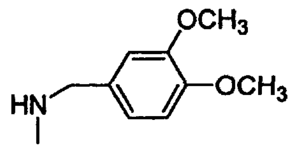 | 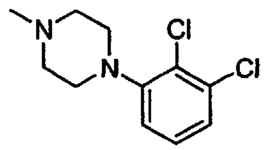 | 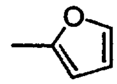 |
| 205 | 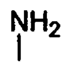 | 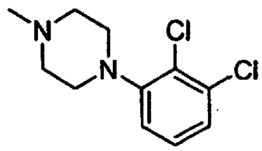 | 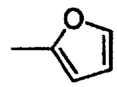 |
| 206 | 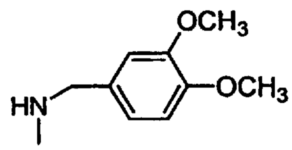 | 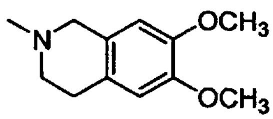 | 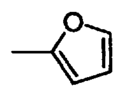 |
| 207 | 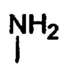 | 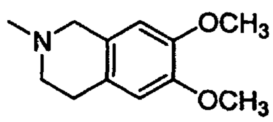 | 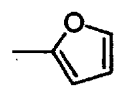 |
| 208 | 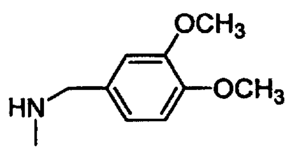 | 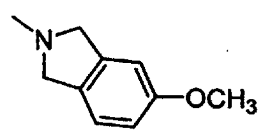 | 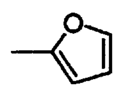 |
| 209 | 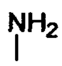 | 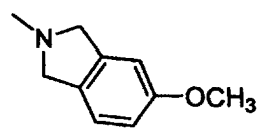 | 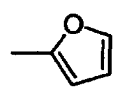 |
| 210 | 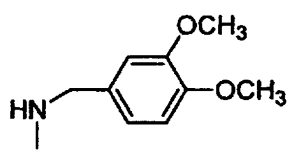 | 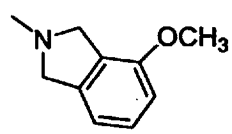 | 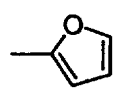 |
| 211 | 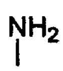 | 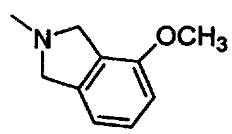 | 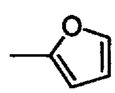 |

Contd.

| Compd. No. (Ia) | $R^7$ | $R^2$ | $R^1$ |
|---|---|---|---|
| 212 | 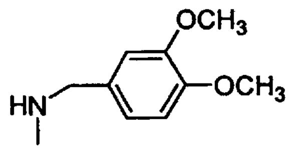 | 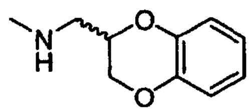 | 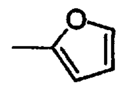 |
| 213 | 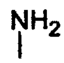 | 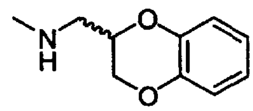 | 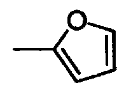 |
| 214 | 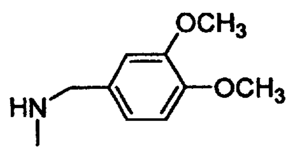 | 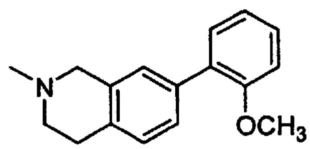 | 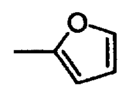 |
| 215 | 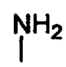 | 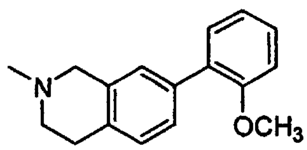 | 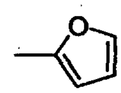 |
| 216 | 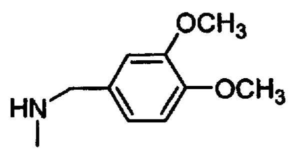 | 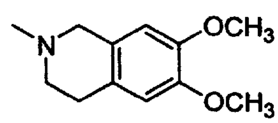 | 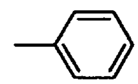 |
| 217 | 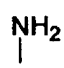 | 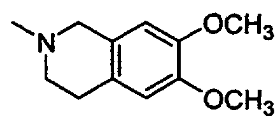 | 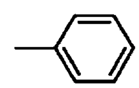 |
| 218 | 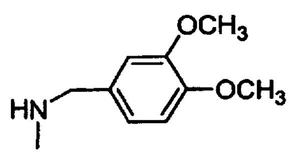 | 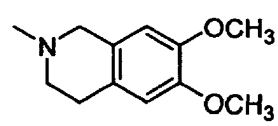 | 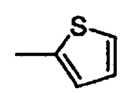 |
| 219 | 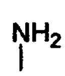 | 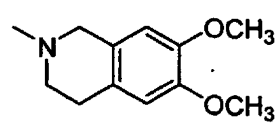 | 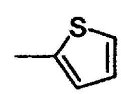 |

Contd.

| Compd. No. (Ia) | $R^7$ | $R^2$ | $R^1$ |
|---|---|---|---|
| 220 | 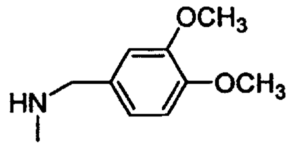 | 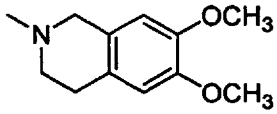 | 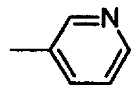 |
| 221 | 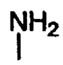 | 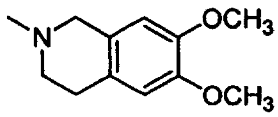 | 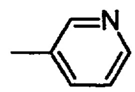 |
| 222 | 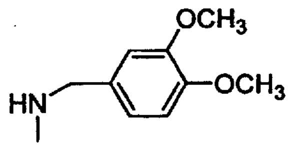 | 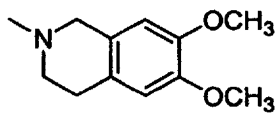 | 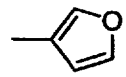 |
| 223 | 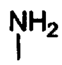 | 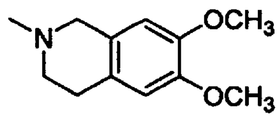 | 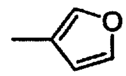 |
| 224 | 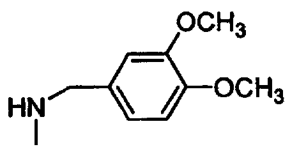 | 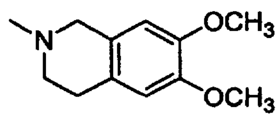 | 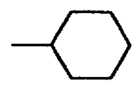 |
| 225 | 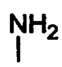 | 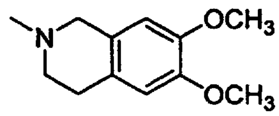 | 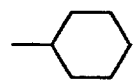 |
| 226 | 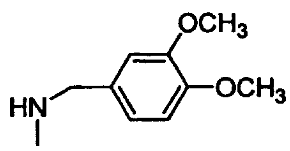 | 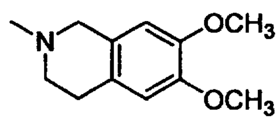 | 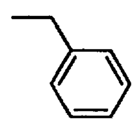 |
| 227 | 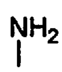 | 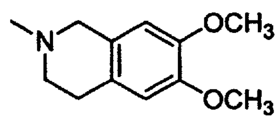 | 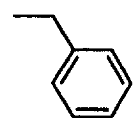 |

Contd.

| Compd. No. (Ia) | $R^7$ | $R^2$ | $R^1$ |
|---|---|---|---|
| 228 | 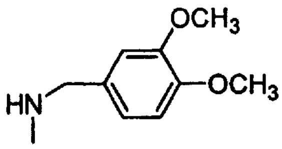 | 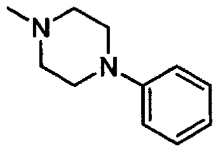 | 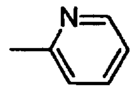 |
| 229 | 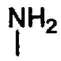 | 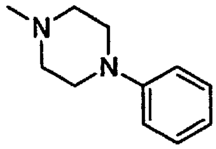 | 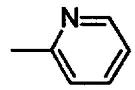 |
| 230 | 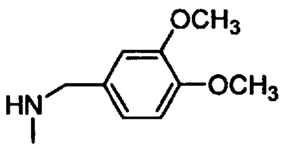 | 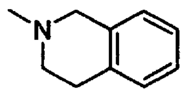 | 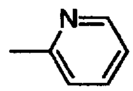 |
| 231 | 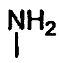 | 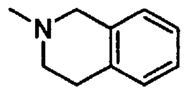 | 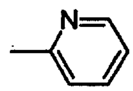 |
| 232 | 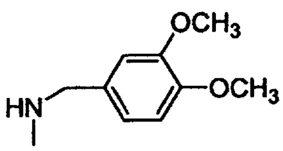 | 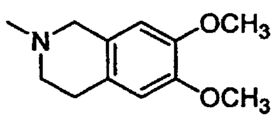 | 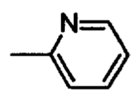 |
| 233 | 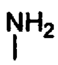 | 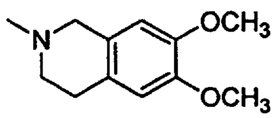 | 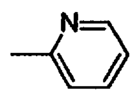 |
| 234 | 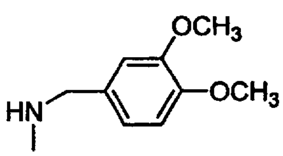 | 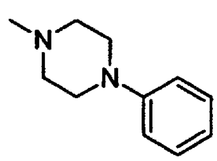 | 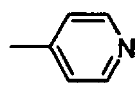 |
| 235 | 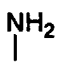 | 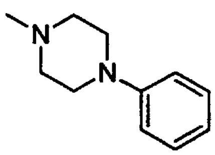 | 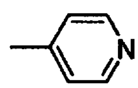 |

Contd.

| Compd. No. (Ia) | $R^7$ | $R^2$ | $R^1$ |
|---|---|---|---|
| 236 | 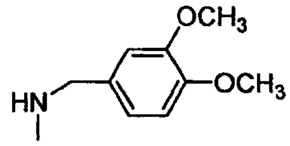 | 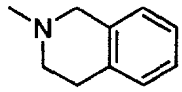 | 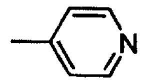 |
| 237 | 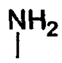 | 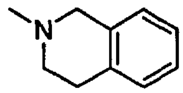 | 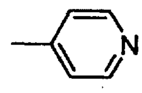 |
| 238 | 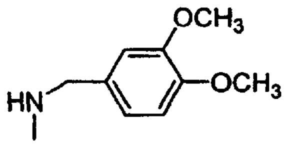 | 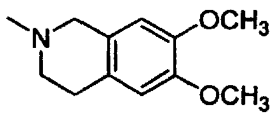 | 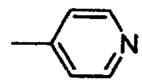 |
| 239 | 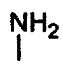 | 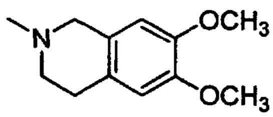 | 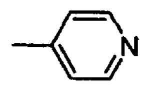 |
| 240 | 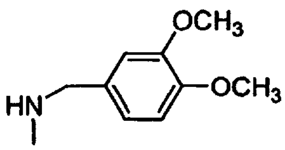 | 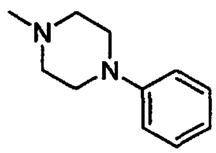 | 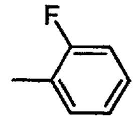 |
| 241 | 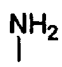 | 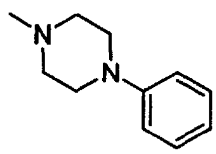 | 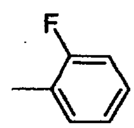 |
| 242 | 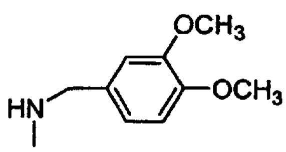 | 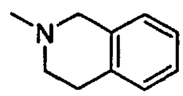 | 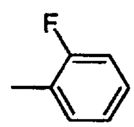 |
| 243 | 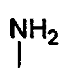 | 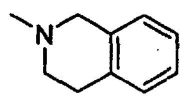 | 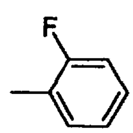 |

Contd.

| Compd. No. (Ia) | $R^7$ | $R^2$ | $R^1$ |
|---|---|---|---|
| 244 | 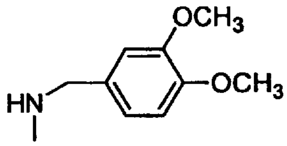 | 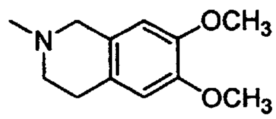 | 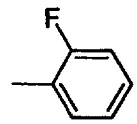 |
| 245 | 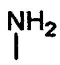 | 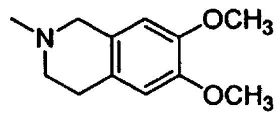 | 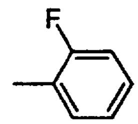 |
| 246 | 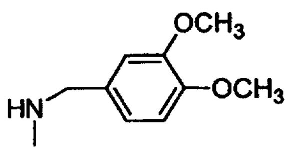 | 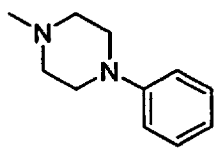 | 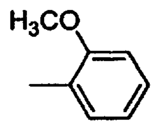 |
| 247 | 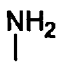 | 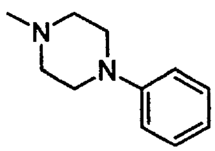 | 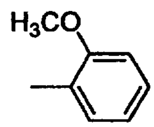 |
| 248 | 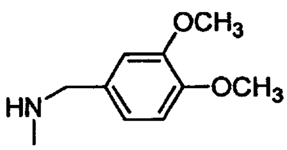 | 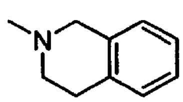 | 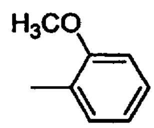 |
| 249 | 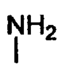 | 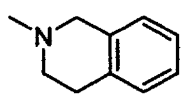 | 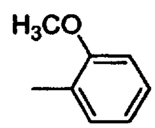 |
| 250 | 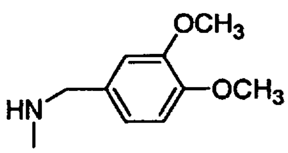 | 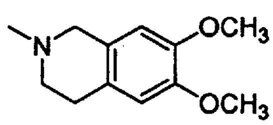 | 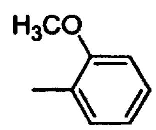 |

Contd.

| Compd. No. (Ia) | $R^7$ | $R^2$ | $R^1$ |
|---|---|---|---|
| 251 | 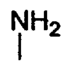 | 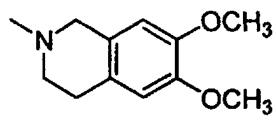 | 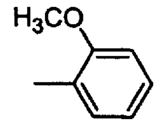 |
| 252 | 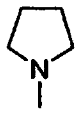 | 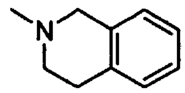 | 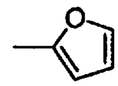 |
| 253 | 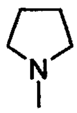 | 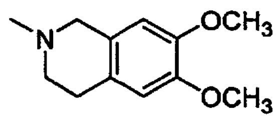 | 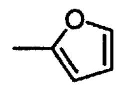 |
| 254 | 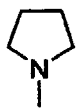 | 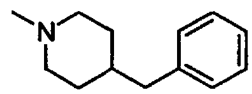 | 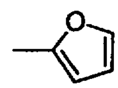 |
| 255 | 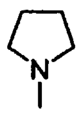 | 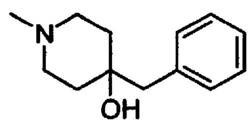 | 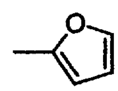 |
| 256 | 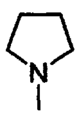 | 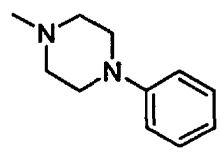 | 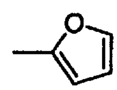 |
| 257 | 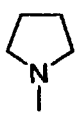 | 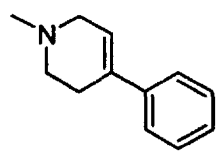 | 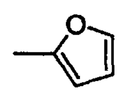 |

Contd.

| Compd. No. (Ia) | $R^7$ | $R^2$ | $R^1$ |
|---|---|---|---|
| 258 | 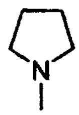 | 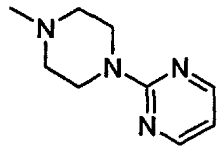 | 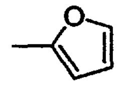 |
| 259 | 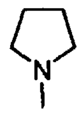 | 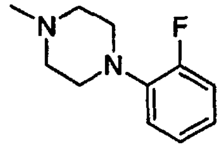 | 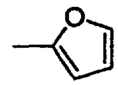 |
| 260 | 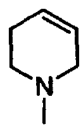 | 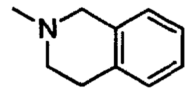 | 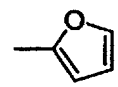 |
| 261 | 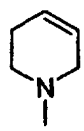 | 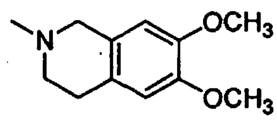 | 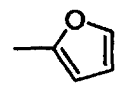 |
| 262 | 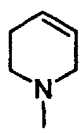 | 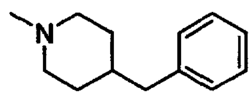 | 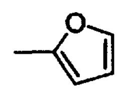 |
| 263 | 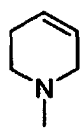 | 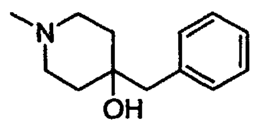 | 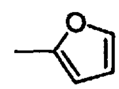 |
| 264 | 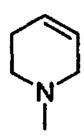 | 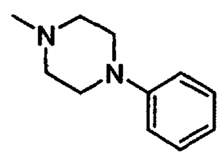 | 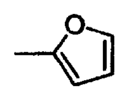 |
| 265 | 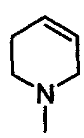 | 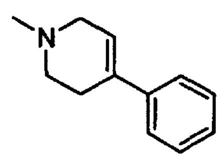 | 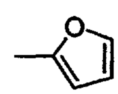 |

Contd.

| Compd. No. (Ia) | $R^7$ | $R^2$ | $R^1$ |
|---|---|---|---|
| 266 | 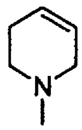 | 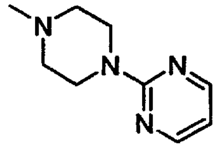 | 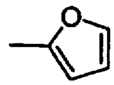 |
| 267 | 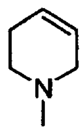 | 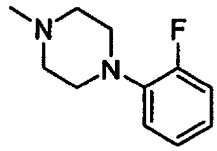 | 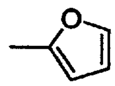 |
| 268 | 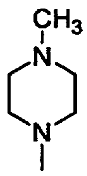 | 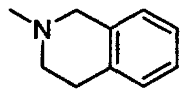 | 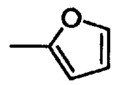 |
| 269 | 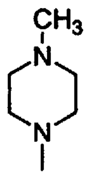 | 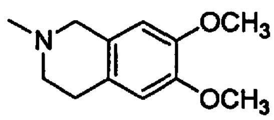 | 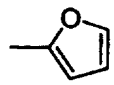 |
| 270 | 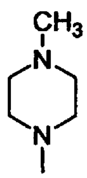 | 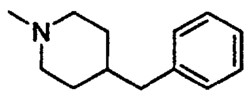 | 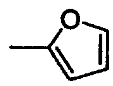 |
| 271 | 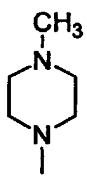 | 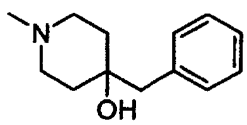 | 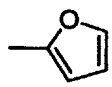 |
| 272 | 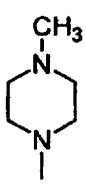 | 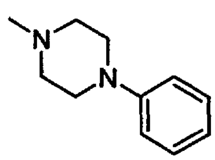 | 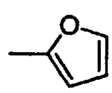 |

Contd.

| Compd. No. (Ia) | $R^7$ | $R^2$ | $R^1$ |
|---|---|---|---|
| 273 | 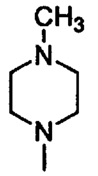 | 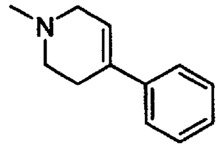 | 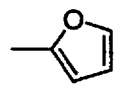 |
| 274 | 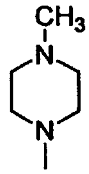 | 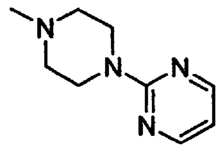 | 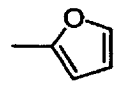 |
| 275 | 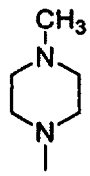 | 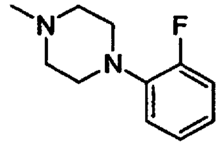 | 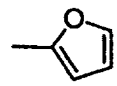 |
| 276 | 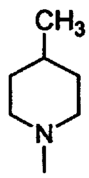 | 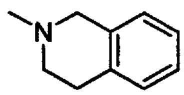 | 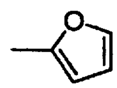 |
| 277 | 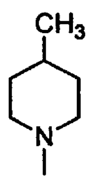 | 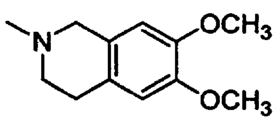 | 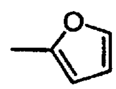 |
| 278 | 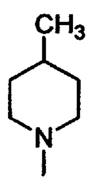 | 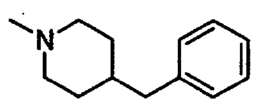 | 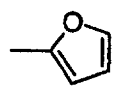 |
| 279 | 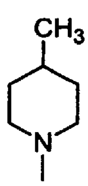 | 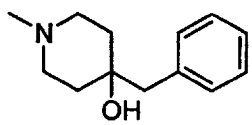 | 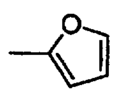 |

Contd.

| Compd. No. (Ia) | $R^7$ | $R^2$ | $R^1$ |
|---|---|---|---|
| 280 | 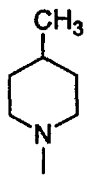 | 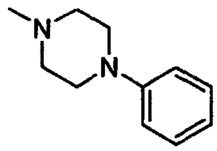 | 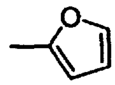 |
| 281 | 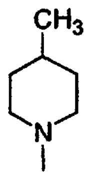 | 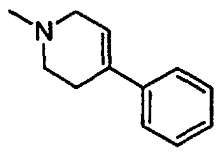 | 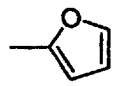 |
| 282 | 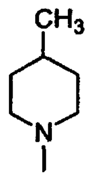 | 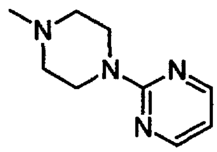 | 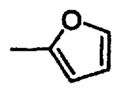 |
| 283 | 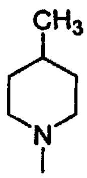 | 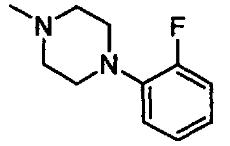 | 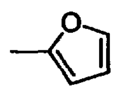 |
| 284 | 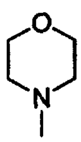 | 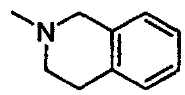 | 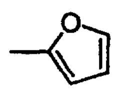 |
| 285 | 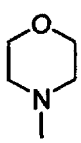 | 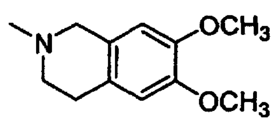 | 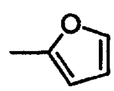 |
| 286 | 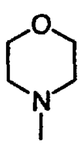 | 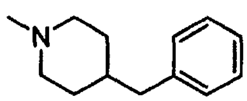 | 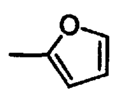 |

Contd.

| Compd. No. (Ia) | $R^7$ | $R^2$ | $R^1$ |
|---|---|---|---|
| 287 | 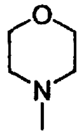 | 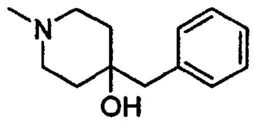 | 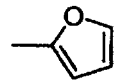 |
| 288 | 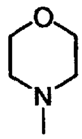 | 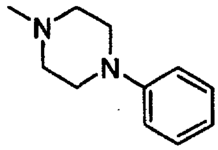 | 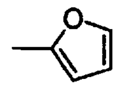 |
| 289 | 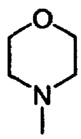 | 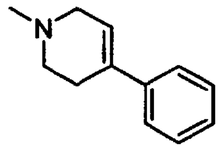 | 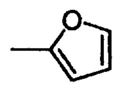 |
| 290 | 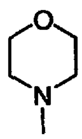 | 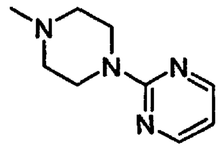 | 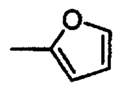 |
| 291 | 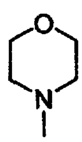 | 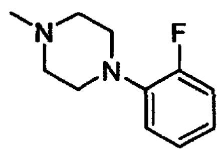 | 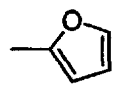 |
| 292 | 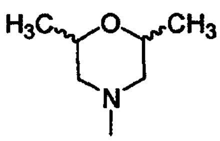 | 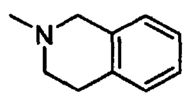 | 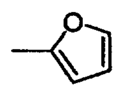 |
| 293 | 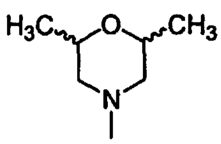 | 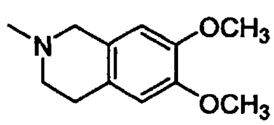 | 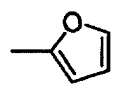 |
| 294 | 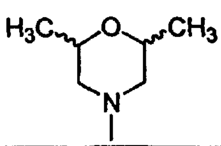 | 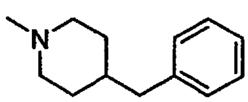 | 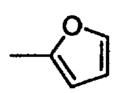 |

Contd.

| Compd. No. (Ia) | $R^7$ | $R^2$ | $R^1$ |
|---|---|---|---|
| 295 | 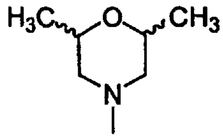 | 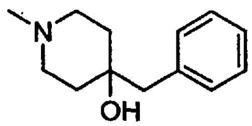 | 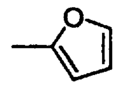 |
| 296 | 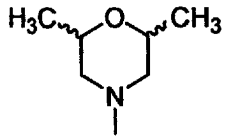 | 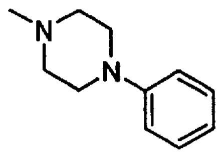 | 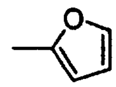 |
| 297 | 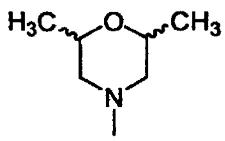 | 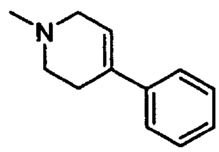 | 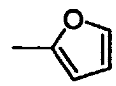 |
| 298 | 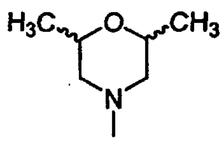 | 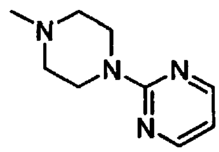 | 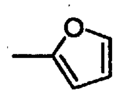 |
| 299 | 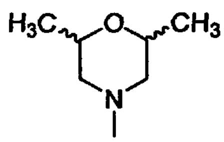 | 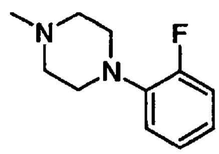 | 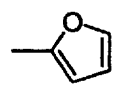 |
| 300 | 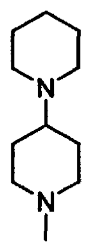 | 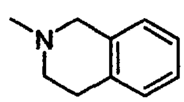 | 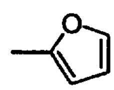 |

Contd.

| Compd. No. (Ia) | $R^7$ | $R^2$ | $R^1$ |
|---|---|---|---|
| 301 | 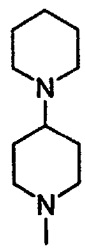 | 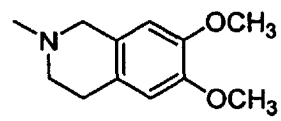 | 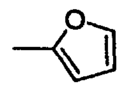 |
| 302 | 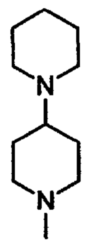 | 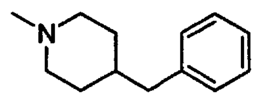 | 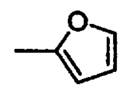 |
| 303 | 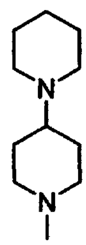 | 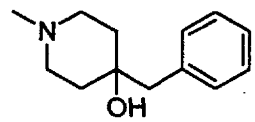 | 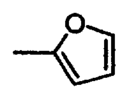 |
| 304 | 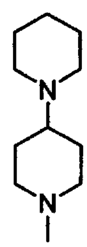 | 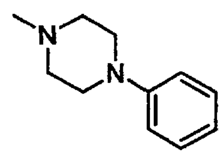 | 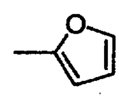 |
| 305 | 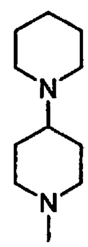 | 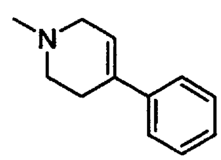 | 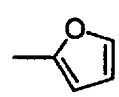 |

Contd.

| Compd. No. (Ia) | $R^7$ | $R^2$ | $R^1$ |
|---|---|---|---|
| 306 | 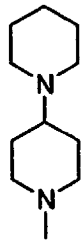 | 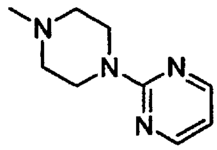 | 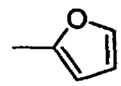 |
| 307 | 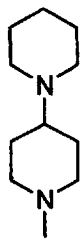 | 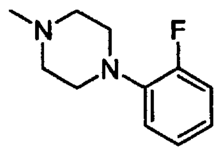 | 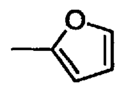 |
| 308 | 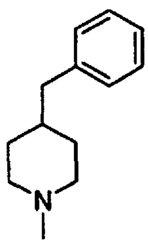 | 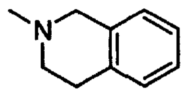 | 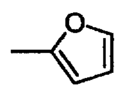 |
| 309 | 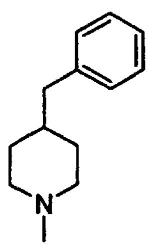 | 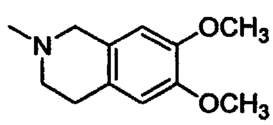 | 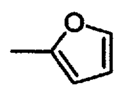 |
| 310 | 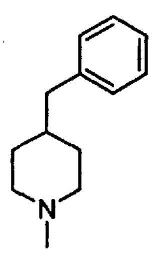 | 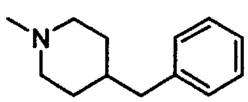 | 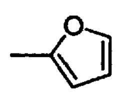 |

Contd.

| Compd. No. (Ia) | $R^7$ | $R^2$ | $R^1$ |
|---|---|---|---|
| 311 | 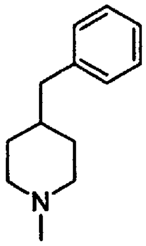 | 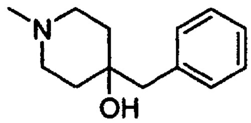 | 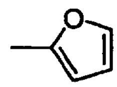 |
| 312 | 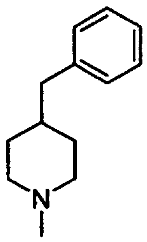 | 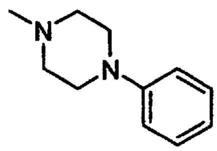 | 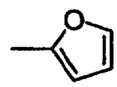 |
| 313 | 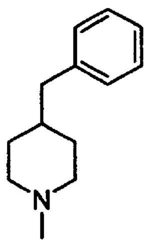 | 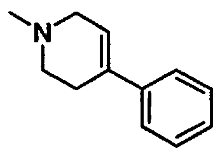 | 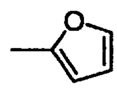 |
| 314 | 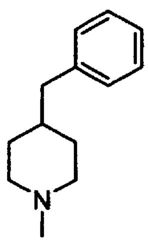 | 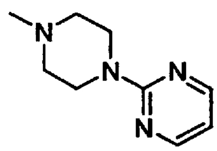 | 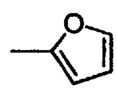 |
| 315 | 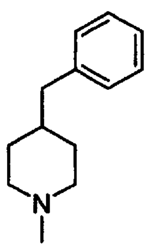 | 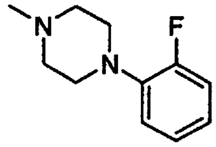 | 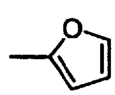 |

Contd.

| Compd. No. (Ia) | $R^7$ | $R^2$ | $R^1$ |
|---|---|---|---|
| 316 | 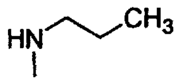 | 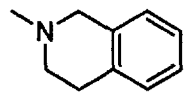 | 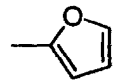 |
| 317 | 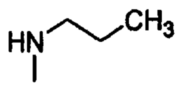 | 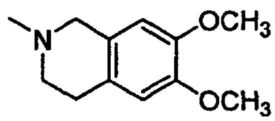 | 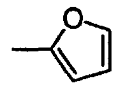 |
| 318 | 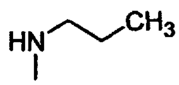 | 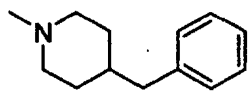 | 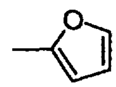 |
| 319 | 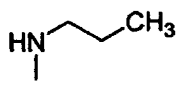 | 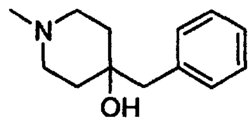 | 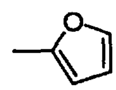 |
| 320 | 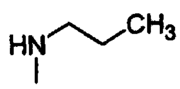 | 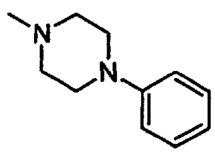 | 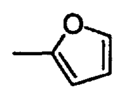 |
| 321 | 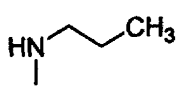 | 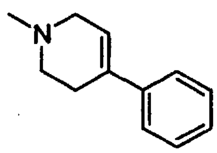 | 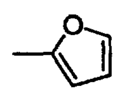 |
| 322 | 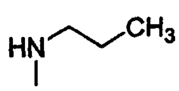 | 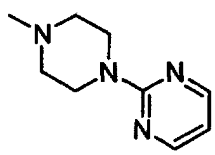 | 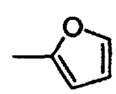 |
| 323 | 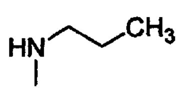 | 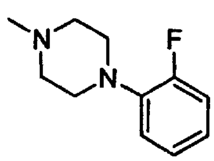 | 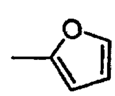 |

Contd.

| Compd. No. (Ia) | $R^7$ | $R^2$ | $R^1$ |
|---|---|---|---|
| 324 | 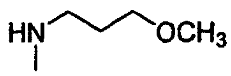 | 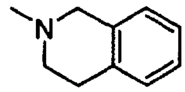 | 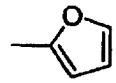 |
| 325 | 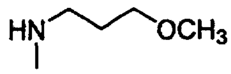 | 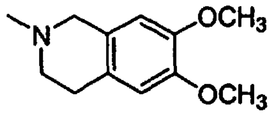 | 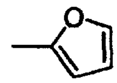 |
| 326 | 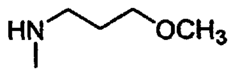 | 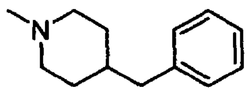 | 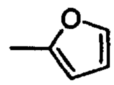 |
| 327 | 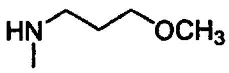 | 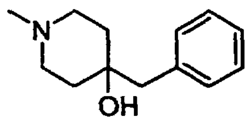 | 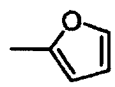 |
| 328 | 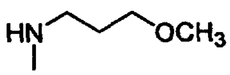 | 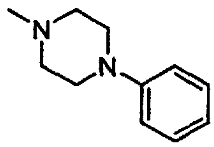 | 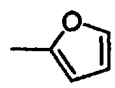 |
| 329 | 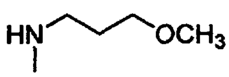 | 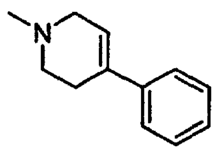 | 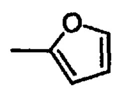 |
| 330 | 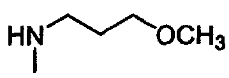 | 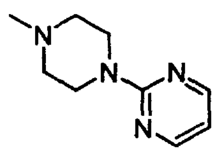 | 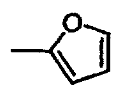 |
| 331 | 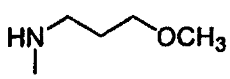 | 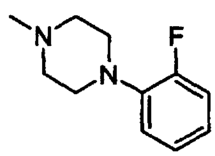 | 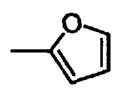 |

Contd.

| Compd. No. (Ia) | $R^7$ | $R^2$ | $R^1$ |
|---|---|---|---|
| 332 | 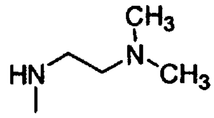 | 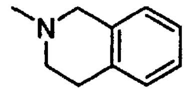 | 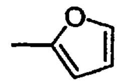 |
| 333 | 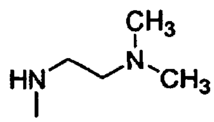 | 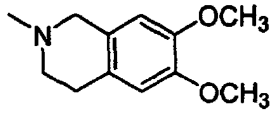 | 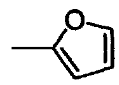 |
| 334 | 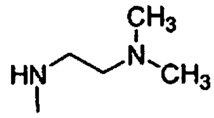 | 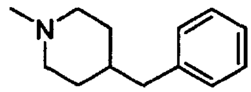 | 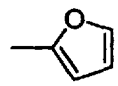 |
| 335 | 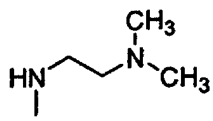 | 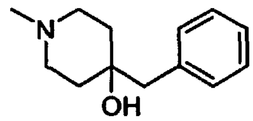 | 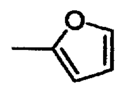 |
| 336 | 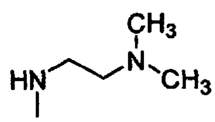 | 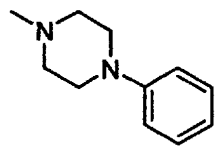 | 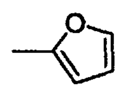 |
| 337 | 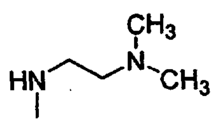 | 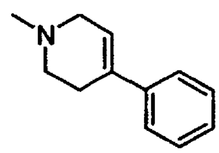 | 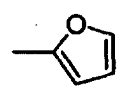 |
| 338 | 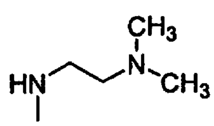 | 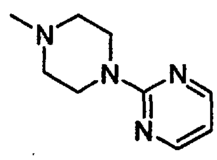 | 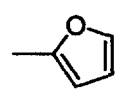 |
| 339 | 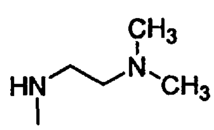 | 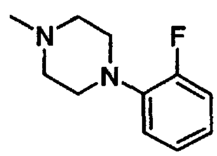 | 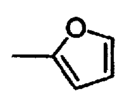 |

Contd.

| Compd. No. (Ia) | $R^7$ | $R^2$ | $R^1$ |
|---|---|---|---|
| 340 | 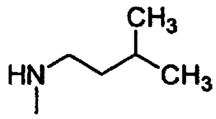 | 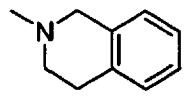 | 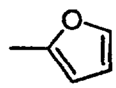 |
| 341 | 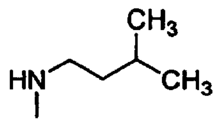 | 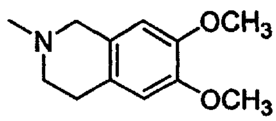 | 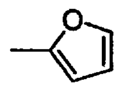 |
| 342 | 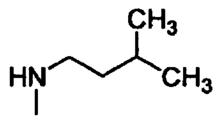 | 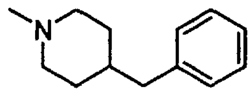 | 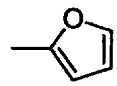 |
| 343 | 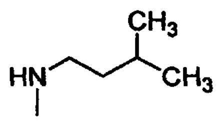 | 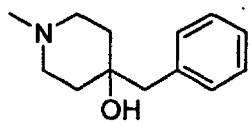 | 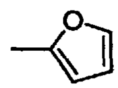 |
| 344 | 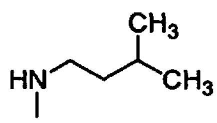 | 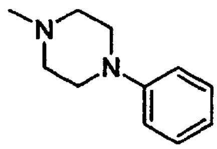 | 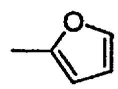 |
| 345 | 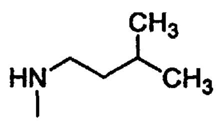 | 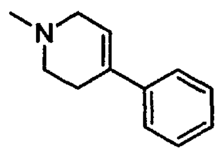 | 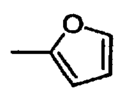 |
| 346 | 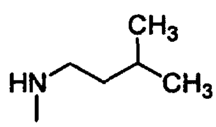 | 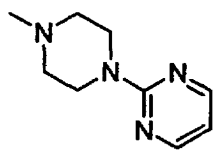 | 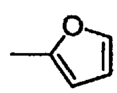 |

Contd.

| Compd. No. (Ia) | $R^7$ | $R^2$ | $R^1$ |
|---|---|---|---|
| 347 | 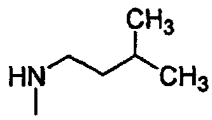 | 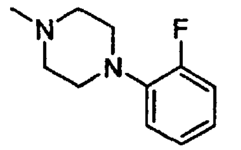 | 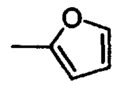 |
| 348 | 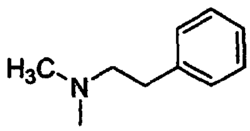 | 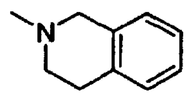 | 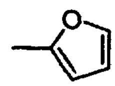 |
| 349 | 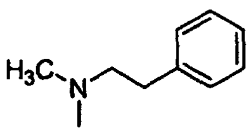 | 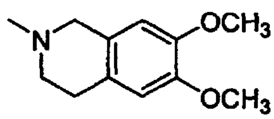 | 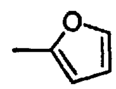 |
| 350 | 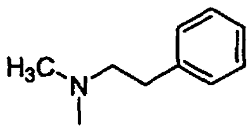 | 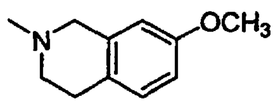 | 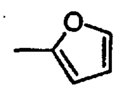 |
| 351 | 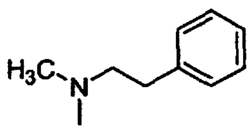 | 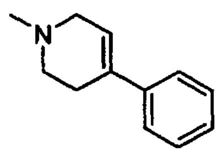 | 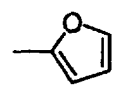 |
| 352 | 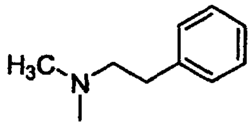 | 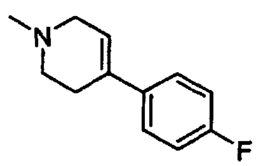 | 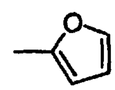 |
| 353 | 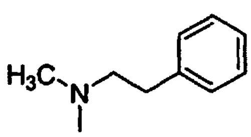 | 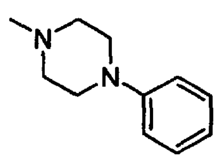 | 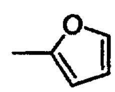 |

Contd.

| Compd. No. (Ia) | $R^7$ | $R^2$ | $R^1$ |
|---|---|---|---|
| 354 | 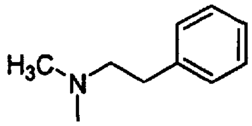 | 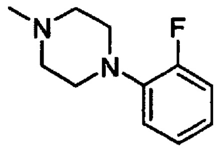 | 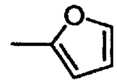 |
| 355 | 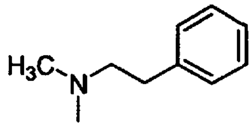 | 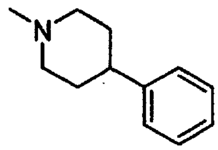 | 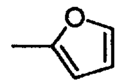 |
| 356 | 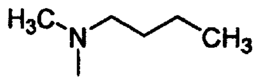 | 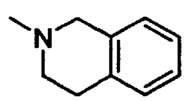 | 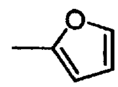 |
| 357 | 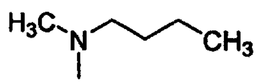 | 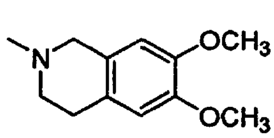 | 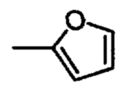 |
| 358 | 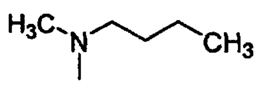 | 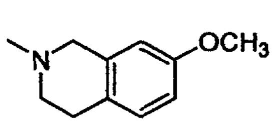 | 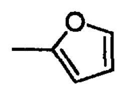 |
| 359 | 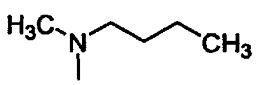 | 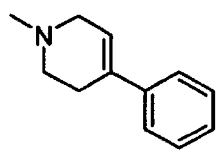 | 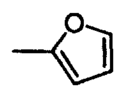 |
| 360 | 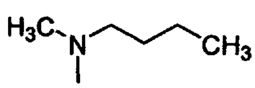 | 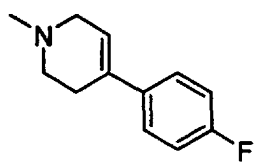 | 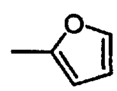 |
| 361 | 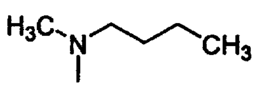 | 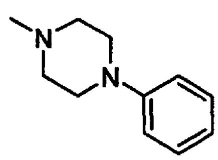 | 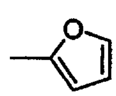 |

Contd.

| Compd. No. (Ia) | $R^7$ | $R^2$ | $R^1$ |
|---|---|---|---|
| 362 | 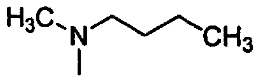 | 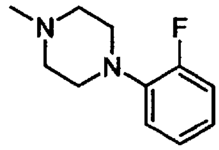 | 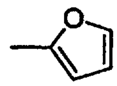 |
| 363 | 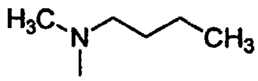 | 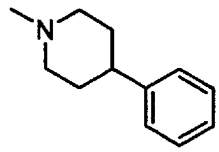 | 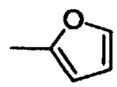 |
| 364 | 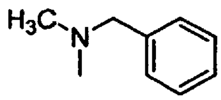 | 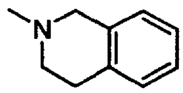 | 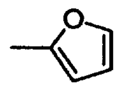 |
| 365 | 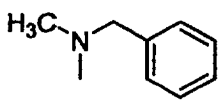 | 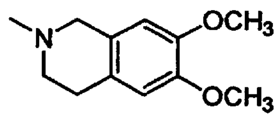 | 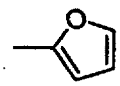 |
| 366 | 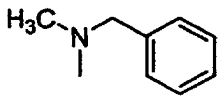 | 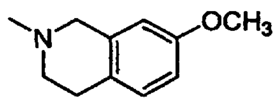 | 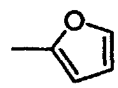 |
| 367 | 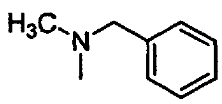 | 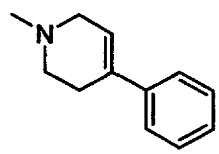 | 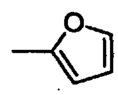 |
| 368 | 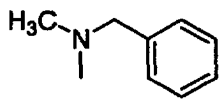 | 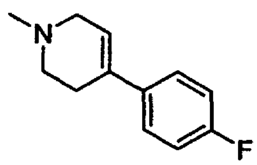 | 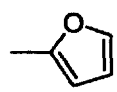 |
| 369 | 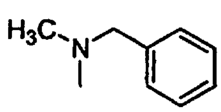 | 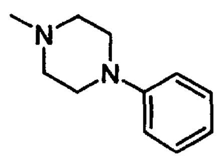 | 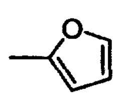 |

Contd.

| Compd. No. (Ia) | $R^7$ | $R^2$ | $R^1$ |
|---|---|---|---|
| 370 | 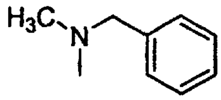 | 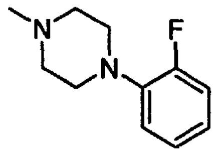 | 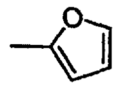 |
| 371 | 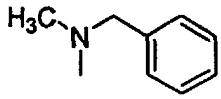 | 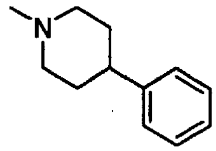 | 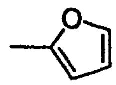 |
| 372 | 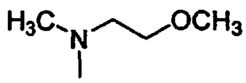 | 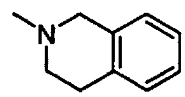 | 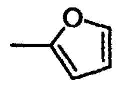 |
| 373 | 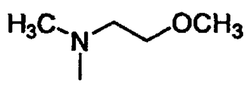 | 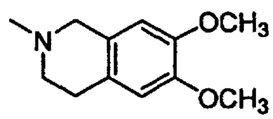 | 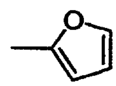 |
| 374 | 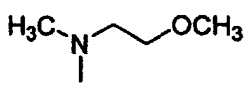 | 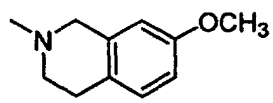 | 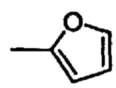 |
| 375 | 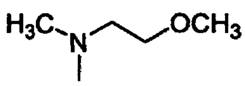 | 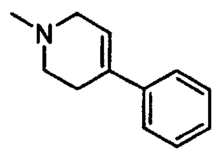 | 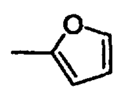 |
| 376 | 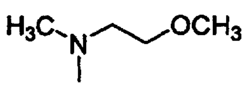 | 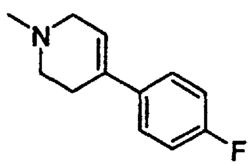 | 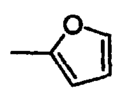 |
| 377 | 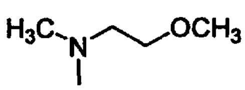 | 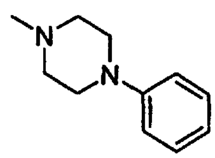 | 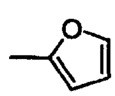 |

Contd.

| Compd. No. (Ia) | $R^7$ | $R^2$ | $R^1$ |
|---|---|---|---|
| 378 | 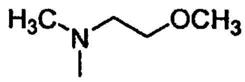 | 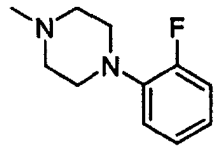 | 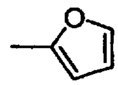 |
| 379 | 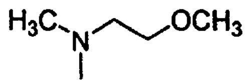 | 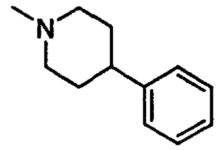 | 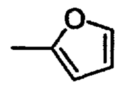 |
| 380 | 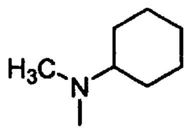 | 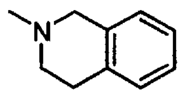 | 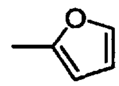 |
| 381 | 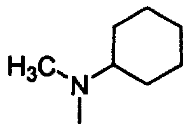 | 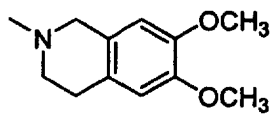 | 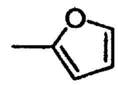 |
| 382 | 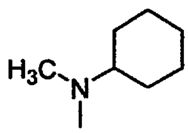 | 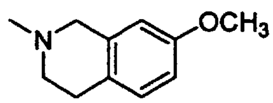 | 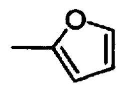 |
| 383 | 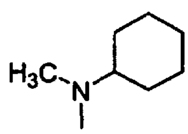 | 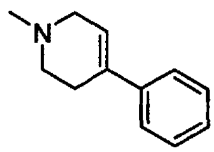 | 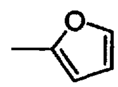 |
| 384 | 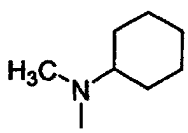 | 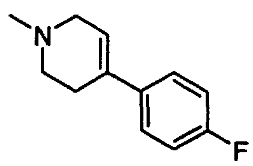 | 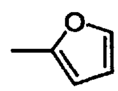 |
| 385 | 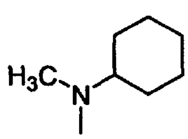 | 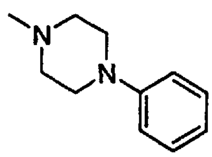 | 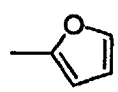 |

Contd.

| Compd. No. (Ia) | $R^7$ | $R^2$ | $R^1$ |
|---|---|---|---|
| 386 | 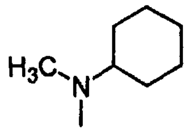 | 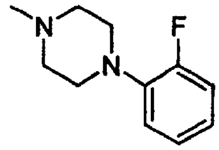 | 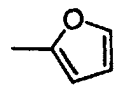 |
| 387 | 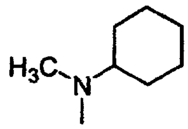 | 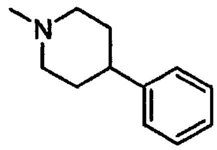 | 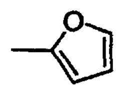 |
| 388 | 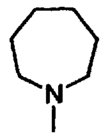 | 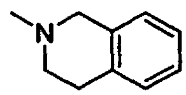 | 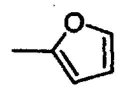 |
| 389 | 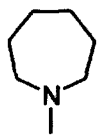 | 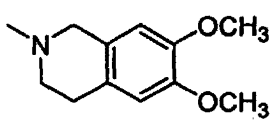 | 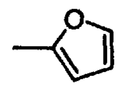 |
| 390 | 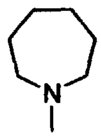 | 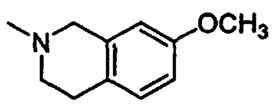 | 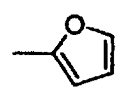 |
| 391 | 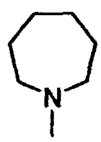 | 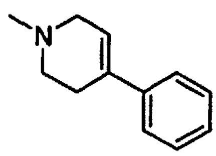 | 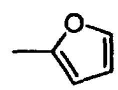 |
| 392 | 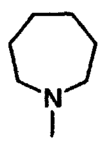 | 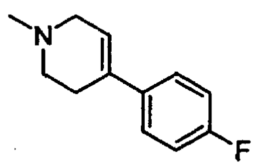 | 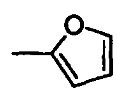 |

Contd.

| Compd. No. (Ia) | $R^7$ | $R^2$ | $R^1$ |
|---|---|---|---|
| 393 | 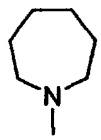 | 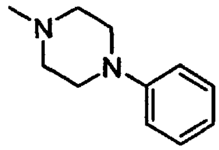 | 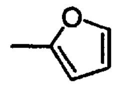 |
| 394 | 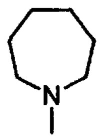 | 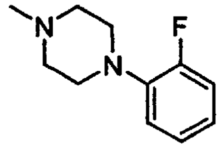 | 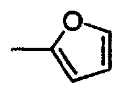 |
| 395 | 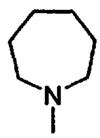 | 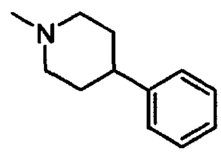 | 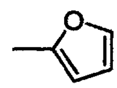 |
| 396 | 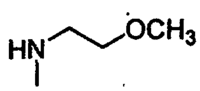 | 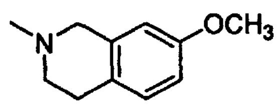 | 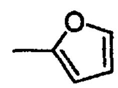 |
| 397 | 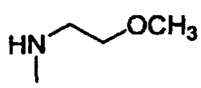 | 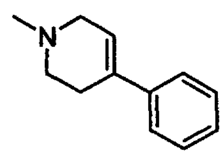 | 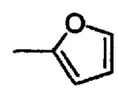 |
| 398 | 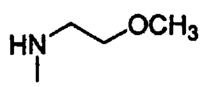 | 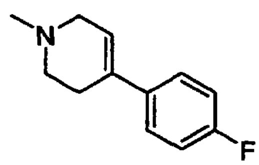 | 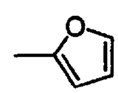 |
| 399 | 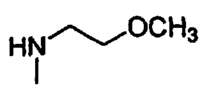 | 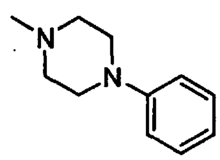 | 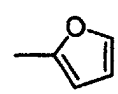 |
| 400 | 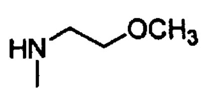 | 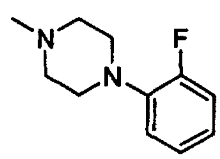 | 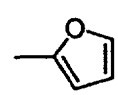 |

Contd.

| Compd. No. (Ia) | $R^7$ | $R^2$ | $R^1$ |
|---|---|---|---|
| 401 | 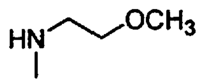 | 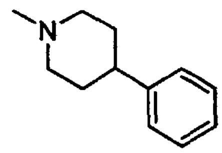 | 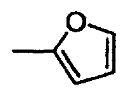 |
| 402 | 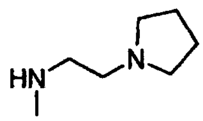 | 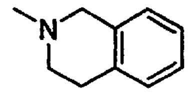 | 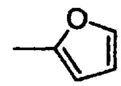 |
| 403 | 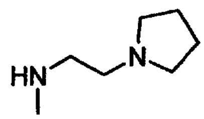 | 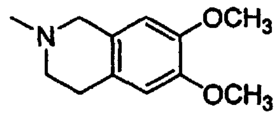 | 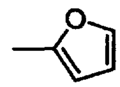 |
| 404 | 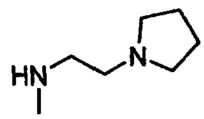 | 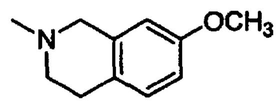 | 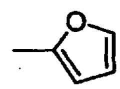 |
| 405 | 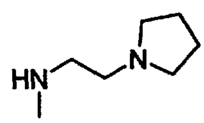 | 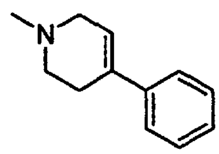 | 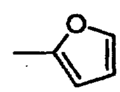 |
| 406 | 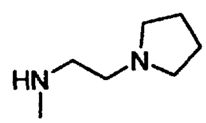 | 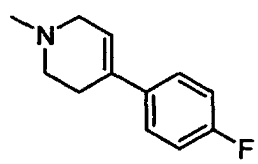 | 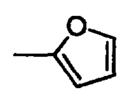 |
| 407 | 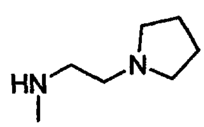 | 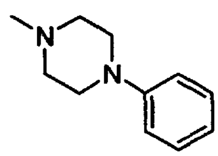 | 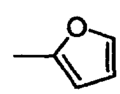 |
| 408 | 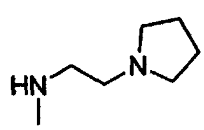 | 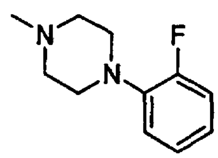 | 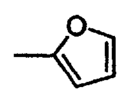 |

Contd.

| Compd. No. (Ia) | $R^7$ | $R^2$ | $R^1$ |
|---|---|---|---|
| 409 | 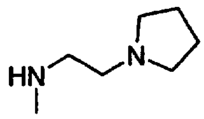 | 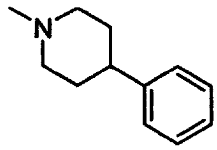 | 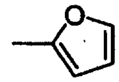 |
| 410 | 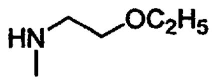 | 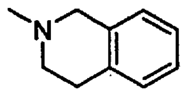 | 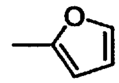 |
| 411 | 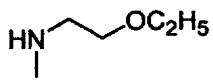 | 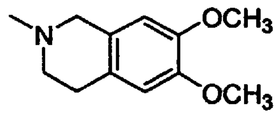 | 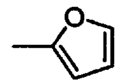 |
| 412 | 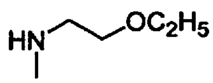 | 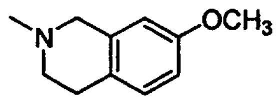 | 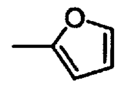 |
| 413 | 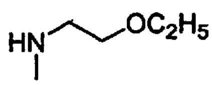 | 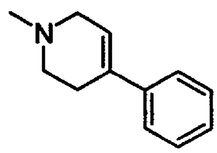 | 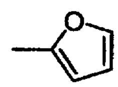 |
| 414 | 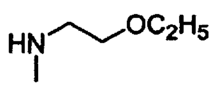 | 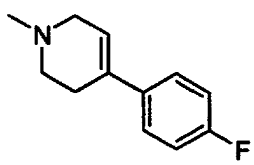 | 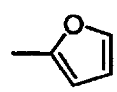 |
| 415 | 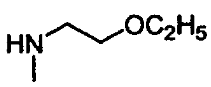 | 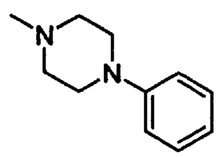 | 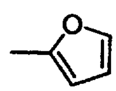 |
| 416 | 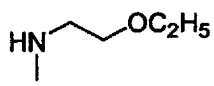 | 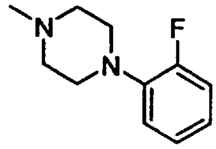 | 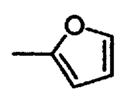 |

Contd.

| Compd. No. (Ia) | $R^7$ | $R^2$ | $R^1$ |
|---|---|---|---|
| 417 | 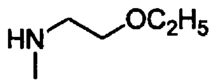 | 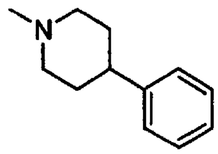 | 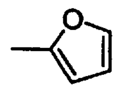 |
| 418 | 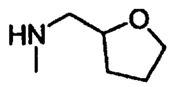 | 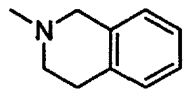 | 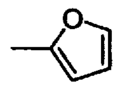 |
| 419 | 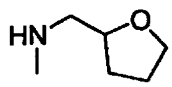 | 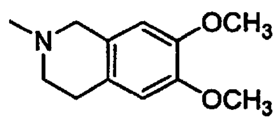 | 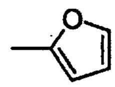 |
| 420 | 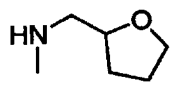 | 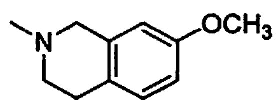 | 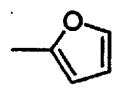 |
| 421 | 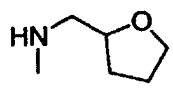 | 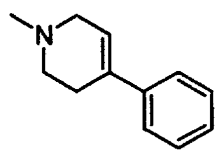 | 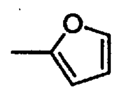 |
| 422 | 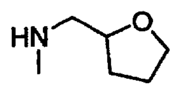 | 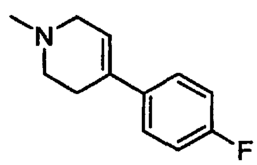 | 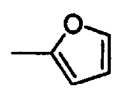 |
| 423 | 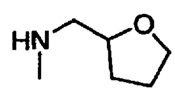 | 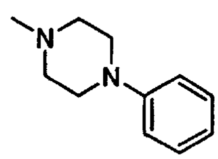 | 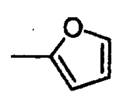 |

Contd.

| Compd. No. (Ia) | $R^7$ | $R^2$ | $R^1$ |
|---|---|---|---|
| 424 | 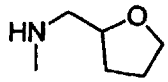 | 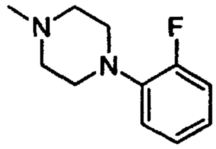 | 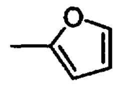 |
| 425 | 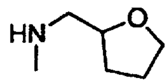 | 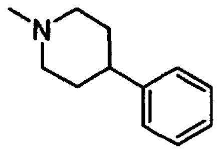 | 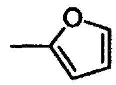 |
| 426 | 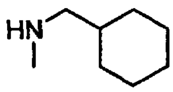 | 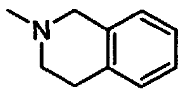 | 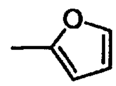 |
| 427 | 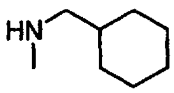 | 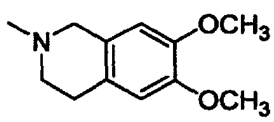 | 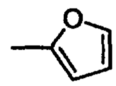 |
| 428 | 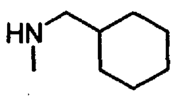 | 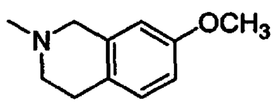 | 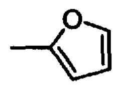 |
| 429 | 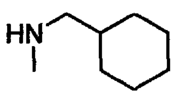 | 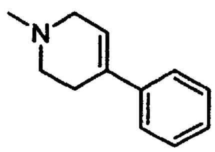 | 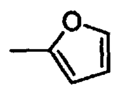 |
| 430 | 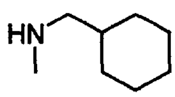 | 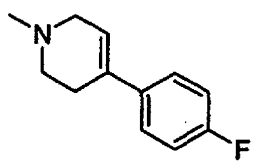 | 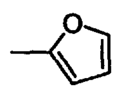 |

Contd.

| Compd. No. (Ia) | $R^7$ | $R^2$ | $R^1$ |
|---|---|---|---|
| 431 | 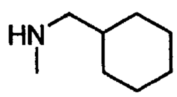 | 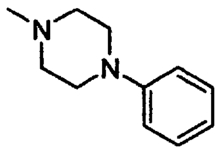 | 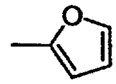 |
| 432 | 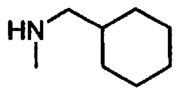 | 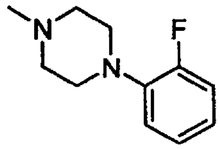 | 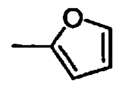 |
| 433 | 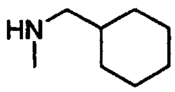 | 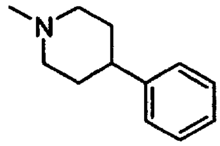 | 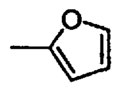 |
| 434 | 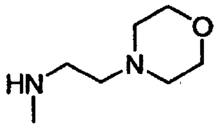 | 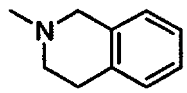 | 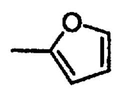 |
| 435 | 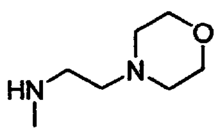 | 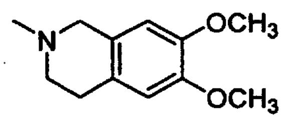 | 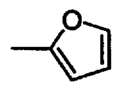 |
| 436 | 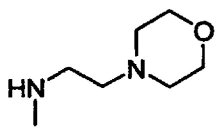 | 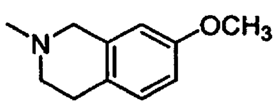 | 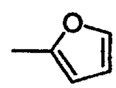 |
| 437 | 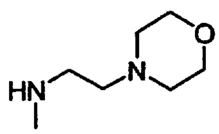 | 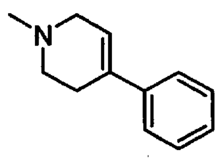 | 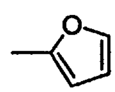 |

Contd.

| Compd. No. (Ia) | $R^7$ | $R^2$ | $R^1$ |
|---|---|---|---|
| 438 | 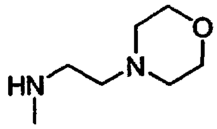 | 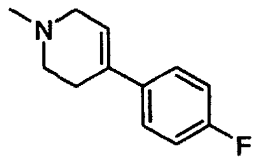 | 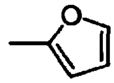 |
| 439 | 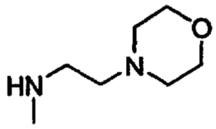 | 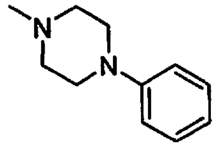 | 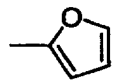 |
| 440 | 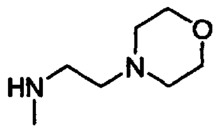 | 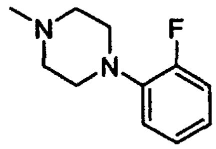 | 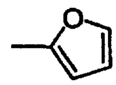 |
| 441 | 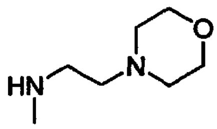 | 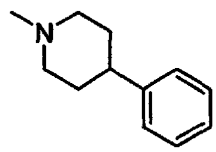 | 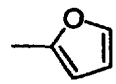 |
| 442 | 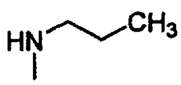 | 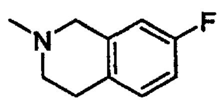 | 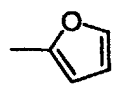 |
| 443 | 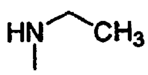 | 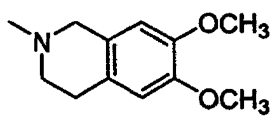 | 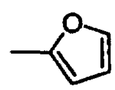 |
| 444 | 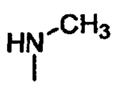 | 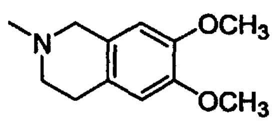 | 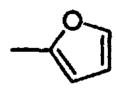 |
| 445 | 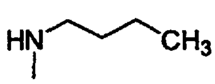 | 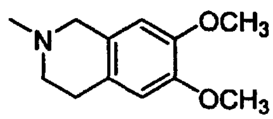 | 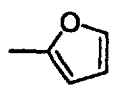 |

Contd.

| Compd. No. (Ia) | $R^7$ | $R^2$ | $R^1$ |
|---|---|---|---|
| 446 | 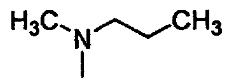 | 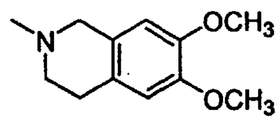 | 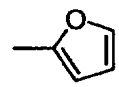 |
| 447 | 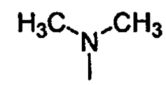 | 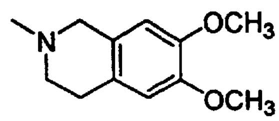 | 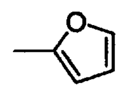 |
| 448 | 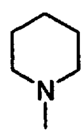 | 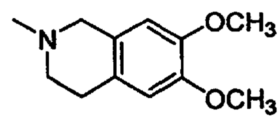 | 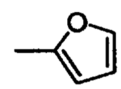 |
| 449 | 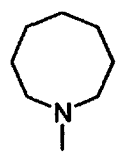 | 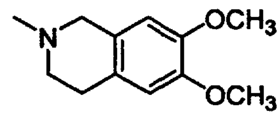 | 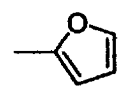 |
| 450 | 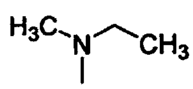 | 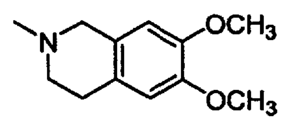 | 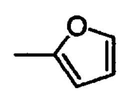 |
| 451 | 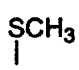 | 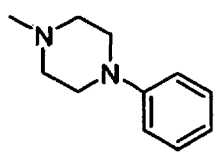 | 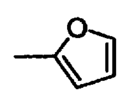 |
| 452 | 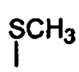 | 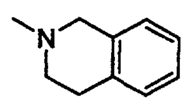 | 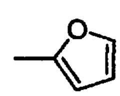 |
| 453 | 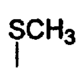 | 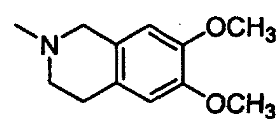 | 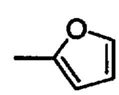 |

Contd.

| Compd. No. (Ia) | $R^7$ | $R^2$ | $R^1$ |
|---|---|---|---|
| 454 | 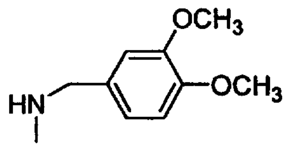 | 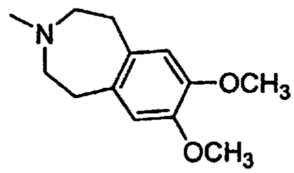 | 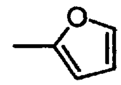 |
| 455 | 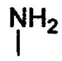 | 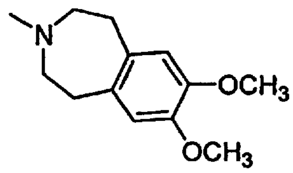 | 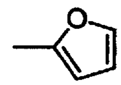 |
| 456 | 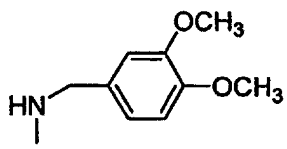 | 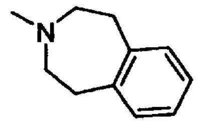 | 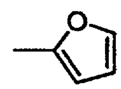 |
| 457 | 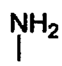 | 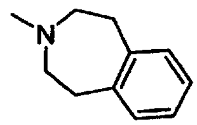 | 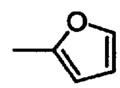 |
| 458 | 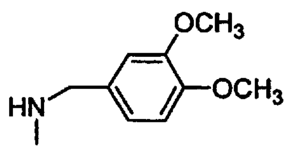 | 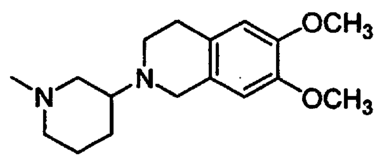 | 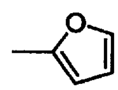 |
| 459 | 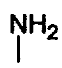 | 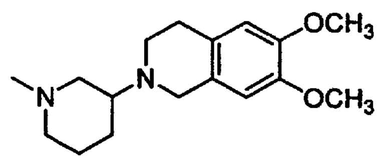 | 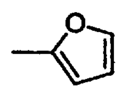 |
| 460 | 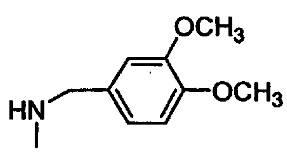 | 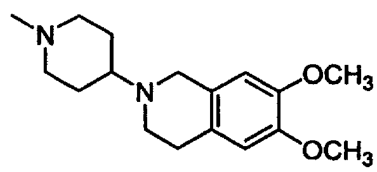 | 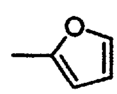 |
| 461 | 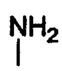 | 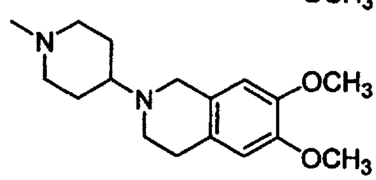 | 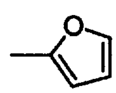 |

Contd.

| Compd. No. (Ia) | $R^7$ | $R^2$ | $R^1$ |
|---|---|---|---|
| 462 | 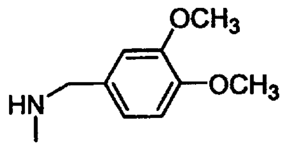 | 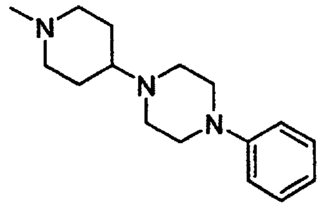 | 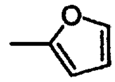 |
| 463 | 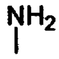 | 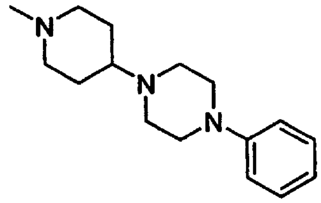 | 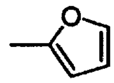 |
| 464 | 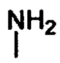 | 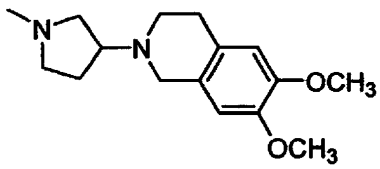 | 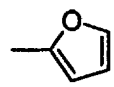 |
| 465 | 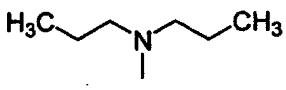 | 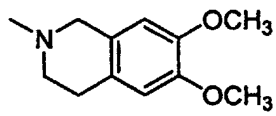 | 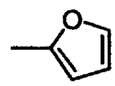 |
| 466 | 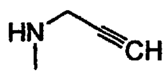 | 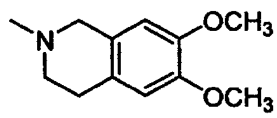 | 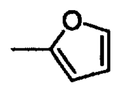 |
| 467 | 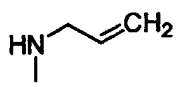 | 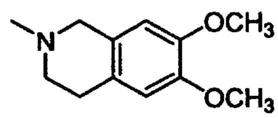 | 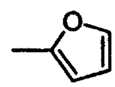 |
| 468 | 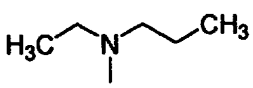 | 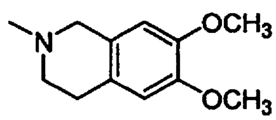 | 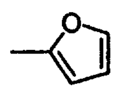 |
| 469 | 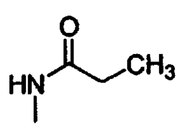 | 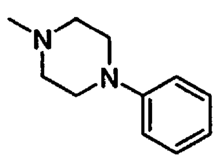 | 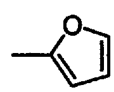 |
| 470 | 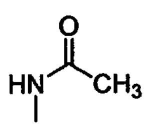 | 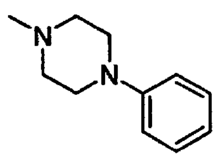 | 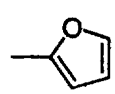 |

Table 2

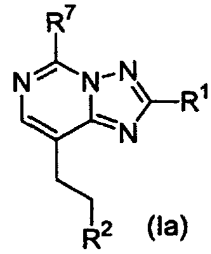

(Ia)

| Compd. No. (Ia) | $R^7$ | $R^2$ | $R^1$ |
|---|---|---|---|
| 471 | 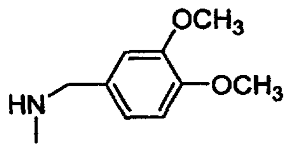 | 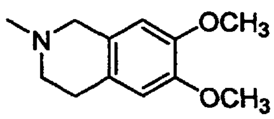 | 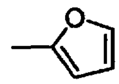 |
| 472 | 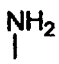 | 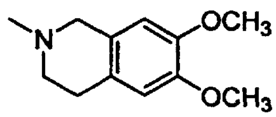 | 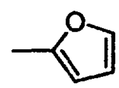 |
| 473 | 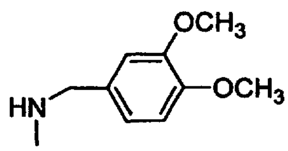 | 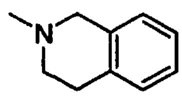 | 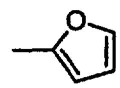 |
| 474 | 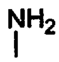 | 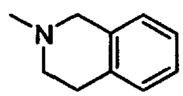 | 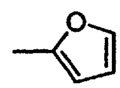 |
| 475 | 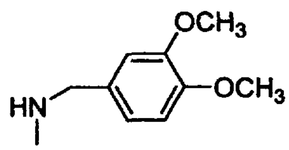 | 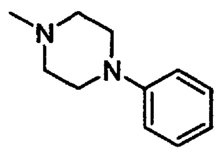 | 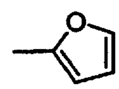 |
| 476 | 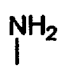 | 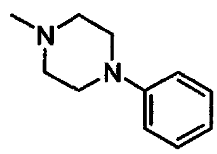 | 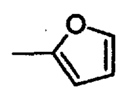 |

Table 3

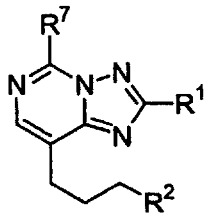

(Ia)

| Compd. No. (Ia) | $R^7$ | $R^2$ | $R^1$ |
|---|---|---|---|
| 477 | 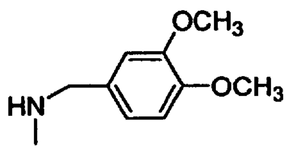 | 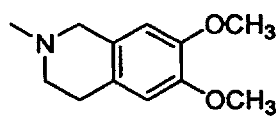 | 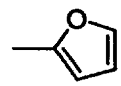 |
| 478 | 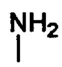 | 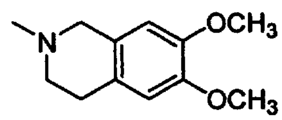 | 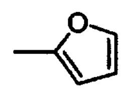 |
| 479 | 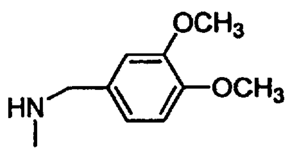 | 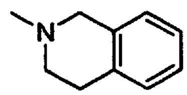 | 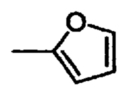 |
| 480 | 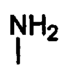 | 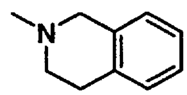 | 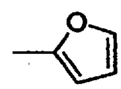 |
| 481 | 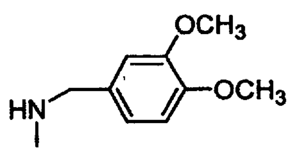 | 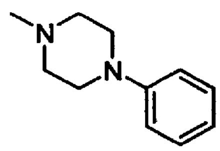 | 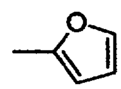 |
| 482 | 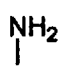 | 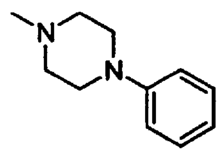 | 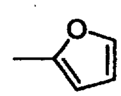 |

Examples of Compound (Ib) obtained by the above-described production process are shown in Table 4.

Table 4

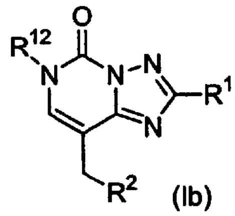

(Ib)

| Compd. No. (Ib) | $R^{12}$ | $R^2$ | $R^1$ |
|---|---|---|---|
| 483 | 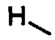 | 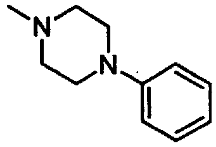 | 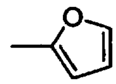 |
| 484 | 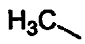 | 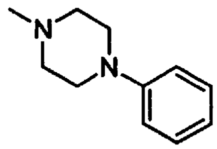 | 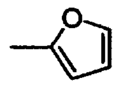 |
| 485 | 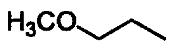 | 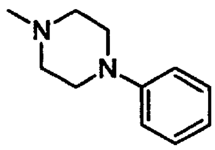 | 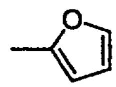 |
| 486 | 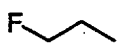 | 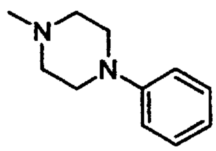 | 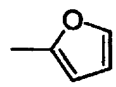 |
| 487 | 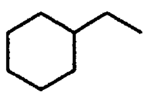 | 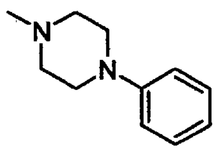 | 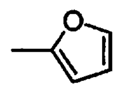 |
| 488 | 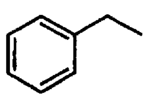 | 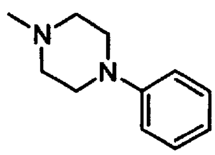 | 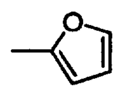 |
| 489 | 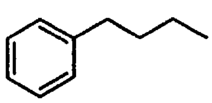 | 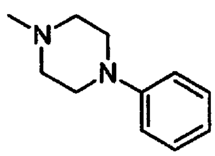 | 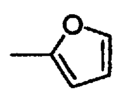 |

Examples of Compound (Ic) obtained by the above-described production process are shown in Table 5.

Table 5

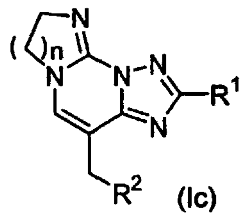

(Ic)

| Compd. No. (Ic) | n | $R^2$ | $R^1$ |
|---|---|---|---|
| 490 | 1 | 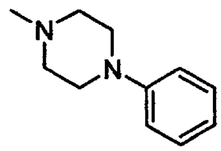 | 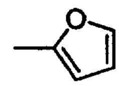 |
| 491 | 1 | 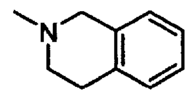 | 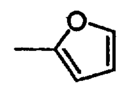 |
| 492 | 2 | 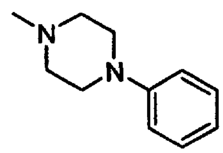 | 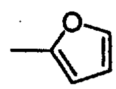 |
| 493 | 2 | 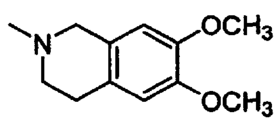 | 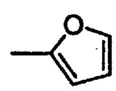 |

The pharmacological activities of Compounds (I) and (II) are illustrated below referring to test examples.

Test Example 1

Adrenoceptor binding activity ($\alpha_{2C}$-adrenoceptor binding test)

This test was carried out according to the method of Schaak et al. [Journal of Pharmacological Experimental Therapeutics, Vol. 281, p. 983 (1997)] as slightly modified.

Human hepatocyto HepG2 cells were cultured in DMEM medium (GIBCO BRL) containing 10% fetal bovine serum (FBS), 100 units/ml penicillin and 100 μg/ml streptomycin, and the membrane fraction of the cultured cells was used. The cultured cells were washed with phosphate-buffered saline (PBS), then scraped with a scraper and recovered by centrifugation at 800 rpm for 5 minutes. To the cells was added a buffer [50 mmol/l tris(hydroxymethyl)aminomethane: Tris, 5 mmol/l ethylenediamine tetraacetic acid (EDTA), pH 7.5], and the cells were suspended using Polytron Homogenizer (Kinematica), followed by centrifugation at 39,000 x g for 10 minutes. The precipitate thus obtained was re-suspended in the same amount of the buffer, and the cell suspension was obtained by centrifugation and removing the supernatant in a similar manner.

To 100 μl of the cell suspension were added 80 μl of tritium-labeled MK-912 (methyl-$^3$H: 2.89 TBq/mmol; New England Nuclear)(final concentration: 1.0 nmol/l) and 20 μl of a test compound. The mixture was allowed to stand at 25°C for 30 minutes and then rapidly filtered by suction through a glass fiber filter treated with 0.3% polyethyleneimine (GF/C; Whatman). The filter was immediately washed 3 times with 200 μl of an ice-cooled buffer and transferred to a vial. A liquid scintillator (ULTIMA GOLD; Packard) was added thereto and the radioactivity was measured with a liquid scintillation counter (Packard).

The inhibition ratio of the test compound against the $\alpha_{2C}$-adrenoceptor binding ($^3$H-MK-912 binding) was

calculated using the following equation.

$$\text{Inhibition ratio (\%)} = \{1-(\text{amount of the binding in the presence of the test compound - amount of the nonspecific binding})/(\text{amount of the total binding - amount of the nonspecific binding})\} \times 100$$

(Note) Amount of the total binding means the amount of radioactivity of $^3$H-MK-912 bound in the absence of the test compound. Amount of the nonspecific binding means the amount of radioactivity of $^3$H-MK-912 bound in the presence of 10 μmol/l Yohimbine (Sigma). Amount of the binding in the presence of the test compound means the amount of radioactivity of $^3$H-MK-912 bound in the presence of varied concentrations of the test compound.

The results are shown in Table 6.

Table 6

| Compound No. (I) | Inhibition ratio (%) against $\alpha_{2C}$-adrenoceptor binding |
|---|---|
| | $10^{-7}$ mol/l |
| 2 | 80 |
| 19 | 96 |
| 23 | 70 |
| 45 | 88 |
| 49 | 91 |
| 55 | 92 |
| 59 | 65 |
| 89 | 86 |
| 127 | 68 |
| 144 | 75 |
| 166 | 84 |
| 182 | 72 |
| 195 | 87 |
| 203 | 83 |
| 213 | 96 |
| 370 | 91 |
| 452 | 58 |
| 483 | 60 |
| 491 | 74 |

Test Example 2

Anti-dyskinesia activity

Parkinson's disease is characterized by degeneration and neuronal cell death of the nigrostriatal dopaminergic neurons. In primates, treatment with a dopamine neurotoxin, 1-methyl-4-phenyl-1,2,3,6-tetrahydropyridine (hereinafter referred to as "MPTP"), causes selective degeneration and loss of the nigrostriatal dopaminergic neurons, showing akinesia, rigidity, etc. These MPTP-treated primates are known as a model of Parkinson's disease [Proceedings of the National Academy of Science USA, Vol. 80, p. 4546 (1983)]. Common marmoset belongs to Anthropoidea and is known to show Parkinsonian symptoms caused by MPTP treatment as in the case of other Anthropoidea [Neuroscience Letter, Vol. 57, p. 37 (1985)]. On the other hand, current treatment for Parkinson's disease is based primarily on dopamine replacement therapy by administration of L-DOPA. The administration of L-DOPA is effective treatment of symptoms associated with Parkinson's disease (anti-Parkinson activity). However, the long-term therapy using L-DOPA is known to cause loss of medicinal efficacy (wearing off and on-off), dyskinesia, psychosis, etc. Particularly, L-DOPA-induced

dyskinesia (hereinafter referred to as "LID") has become an issue due to the frequency of its development and the pain of the patients. The above-described MPTP-treated common marmoset shows dyskinesia similar to that in patients with Parkinson's disease, by repeated administration of L-DOPA, so this chronically exposure model is known as a model of LID [Movement Disorder, Vol. 10, p. 731 (1995)].

This test was carried out using 4 animals per group of female and male common marmosets of 5 to 7 years of age (300 to 375 g in body weight, CLEA Japan). MPTP (RBI) was dissolved in physiological saline for injection (Otsuka Pharmaceutical Co., Ltd.) and subcutaneously administered to each common marmoset once a day for 5 days in a dose of 2.0 mg/kg. More than six weeks after the administration, animals showing chronic symptoms of Parkinson's disease were used to prepare a model of dyskinesia. A suspension of 4:1 combination of L-DOPA (Kyowa Hakko) and benserazide hydrochloride (Kyowa Hakko) in an aqueous solution of 0.5% methylcellulose 400cP (MC400) and 10% sucrose was repeatedly administered to the animals twice a day at an interval of 5 to 6 hours. A model animal developing serious dyskinesia is established around 3 weeks after the start of the treatment with L-DOPA. Model animals developing serious dyskinesia were used for the test. Each of the test compounds was suspended in an aqueous solution of 0.5% MC400 and 10% sucrose for use. More than one hour before the administration of the test compound, the animals were housed in an experimental cage (equipped with a locomotor counter) to acclimate them to the experimental condition. At day 1 of the test, the basal locomotor activity was measured, and it was confirmed that the animals did not make a movement without L-DOPA treatment. At day 2, the animals were treated with a vehicle and L-DOPA, and the measurement of spontaneous locomotor count and also the LID scoring were carried out. At day 3, the test compound plus L-DOPA were administered to the animals, the measurement of locomotor activity and also the LID scoring were carried out, and the LID score was compared with that at the time of the treatment with a vehicle. The LID scoring was carried out twice a day by observing the state of LID from a one-way mirror at 10-minute intervals for 3 hours after the administration of the test compound plus L-DOPA or of L-DOPA alone together with a solvent. The locomotor activity was measured at 10-minute intervals for 12 hours by a computer-controlled automatic measuring apparatus. The LID score judged based on the criteria of judgment shown below was used as the score of individual animals.

Criteria of judgment:

Scoring was carried out based on the presence or absence of dystonia, chorea, athetosis and stereotypy in the trunk and four limbs of the animals by applying the state to the following categories [Movement Disorder, Vol. 10, p. 731 (1995)]. The criteria of judgment are shown in Table 7.

Table 7

| Score | Seriousness | State of dyskinesia |
|---|---|---|
| 0 | No development | No development |
| 1 | Gentle | Dyskinesia-like posture or movement is momentarily or seldom shown during the period of observation |
| 2 | Modest | Dyskinesia-like abnormal actions are clearly observed but do not inhibit normal actions |
| 3 | Remarkable | Frequent and continuous dyskinesia is observed and affects normal actions |
| 4 | Serious | Dyskinesia is continuously observed and normal actions are almost impossible |

The state with the highest score was regarded as the score during the relevant time of observation.

Judgment of the results was made by comparing the averages of the highest LID scores of 4 animals in one group in the presence or absence of a test compound administered (statistical analysis: Wilcoxon Rank Sum test).

The test compounds showing high affinity to $\alpha_{2C}$-adrenoceptor showed anti-dyskinesia activity in the MPTP-treated common marmoset model of Parkinson's disease. In this case, because no strong influence was observed on locomotor activity, it is not considered that the compounds suppressed the normal movement improved by the anti-Parkinson action of L-DOPA. Furthermore, among known $\alpha_2$-adrenoceptor antagonists, ARC239 and idazoxan having an affinity to $\alpha_{2C}$-adrenoceptor showed anti-dyskinesia activity, but BRL44408 having a low affinity to $\alpha_{2C}$-adrenoceptor did not show anti-dyskinesia activity. This indicated that $\alpha_{2C}$-adrenoceptor antagonism is important for the manifestation of anti-dyskinesia activity. The affinity of ARC239 and BRL44408 to $\alpha_{2C}$-adrenoceptor is described in Journal of Pharmacology and Experimental Therapeutics, Vol. 271, p. 1558 (1994), and the affinity of idazoxan thereto is described in Proceedings of the National Academy of Science USA, Vol. 85, p. 6301 (1988).

As described above, $\alpha_{2C}$-adrenoceptor antagonism or anti-dyskinesia activity of Compounds (I) and (II) has been shown by Test Examples 1 and 2.

Compounds (I) and (II) and pharmaceutically acceptable salts thereof show excellent $\alpha_2$-adrenoceptor antagonism, particularly $\alpha_{2C}$-adrenoceptor antagonism. Accordingly, it was suggested that a pharmaceutical comprising Compound (I) or (II) as an active ingredient is effective for various diseases induced by hyperactivity of $\alpha_2$-adrenoceptor, particularly $\alpha_{2c}$-adrenoceptor [for example, Parkinson's disease, tremor, dyskinesia (L-DOPA-induced dyskinesia, tardive dyskinesia, myoclonus, tic, Tourette's syndrome), Huntington's disease, dystonia, anxiety disorders (panic attack and panic disorder, phobia, obsessive-compulsive disorder, post-traumatic stress disorder, acute stress disorder, generalized anxiety disorder, anxiety stemming from physical disability or substances), mood disorders (depression, dysthymic disorder, bipolar disorder, cyclothymic disorder), hypertension, diabetes, obesity, glaucoma (primary open-angle glaucoma, angle-closure glaucoma), genital insufficiency (erectile dysfunction), kidney diseases (acute renal failure, chronic renal failure, glomerulonephritis), peripheral vascular diseases (peripheral arterial occlusion, thromboangiitis obliterans, Raynaud's disease and Raynaud's phenomenon, acrocyanosis, erythromelalgia), urinary incontinence (transient incontinence, constant incontinence), pains (acute postoperative pain, cancer pain, neuropathic pain, psychogenic pain syndrome), cerebrovascular diseases (transient cerebral ischemic attack (TIA), ischemic attack, intracranial hemorrhage, subarachnoidal hemorrhage) and head injury].

Although Compounds (I) and (II) and pharmaceutically acceptable salts thereof can be administered as such, it is generally preferred to offer them in the form of various pharmaceutical preparations. Such pharmaceutical preparations are to be used in animals or humans.

The pharmaceutical preparations of the present invention can contain Compound (I) or (II) or a pharmaceutical salt thereof as the active ingredient alone or in combination with any other active ingredients for the treatment of different diseases. These pharmaceutical preparations are produced by any methods well known in the technical field of pharmaceutics by mixing the active ingredient with one or more pharmaceutically acceptable carriers.

It is desirable to select a route of administration that is most effective in the treatment, examples thereof being oral administration and intravenous and other parenteral administrations.

Examples of the dosage form include tablets and injections.

Examples of the carriers for the pharmaceutical preparations include lactose, mannitol, glucose, hydroxypropylcellulose, starch, magnesium stearate, sorbitan fatty acid ester, glyceric acid ester, distilled water for injection, physiological saline, propylene glycol, polyethylene glycol and ethanol. The pharmaceutical preparations of the present invention may also contain various excipients, lubricants, binders, disintegrators, isotonizing agents, emulsifiers and the like.

When used for the above-described purposes, Compound (I) or (II) or a pharmaceutically acceptable salt thereof is generally administered either systemically or locally by oral or parenteral administration. The dose and the frequency of administration vary depending on the mode of administration, the age and body weight of the patient and the properties and seriousness of the symptoms to be treated, but they are generally administered in a dose of from 1 to 100 mg per dose per adult person orally or parenterally once to several times a day, or intravenously by continuous administration for the period of 1 to 24 hours per day. However, these doses and frequency of administration vary depending on the various conditions described above.

## Best Modes for Carrying out the Invention

The present invention is described in detail below referring to reference examples and examples.

Unless otherwise stated, proton nuclear magnetic resonance spectrum ($^1$H-NMR) used in reference examples and examples was measured at 270 MHz. Furthermore, in proton nuclear magnetic resonance spectrum, exchangeable hydrogen may not be clearly observed depending upon the compound and measuring conditions, and hydrogen atom on quaternary nitrogen atom may be observed in the case of hydrochloride form of a compound. A symbol "br" means a broad signal.

### Reference Example 1

4-(N'-Benzoylhydrazino)-5-ethoxycarbonyl-2-methylthiopyrimidine

4-Chloro-5-ethoxycarbonyl-2-methylthiopyrimidine (25.0 g, 107 mmol) and benzoylhydrazine (17.8 g, 131 mmol) were dissolved in DMSO (300 ml), and potassium tert-butoxide (26.9 g, 240 mmol) was slowly added thereto under ice-cooling, followed by stirring for about 2 hours at room temperature. After the completion of reaction, the pH was adjusted to about 4.0 by adding water and acetic acid to the reaction mixture under ice-cooling, and the deposited crystals were recovered by filtration under reduced pressure. The obtained crystals were washed with water and dried under reduced pressure to obtain the subject compound (34.9 g, 96%) as a white powder.
$^1$H NMR ($CDCl_3$, $\delta$, ppm): 1.41 (t, J = 7.3 Hz, 3H), 2.47 (s, 3H), 4.39 (q, J = 7.3 Hz, 2H), 7.46-7.60 (m, 3H), 7.86 (d, J = 6.9Hz, 2H), 8.71 (s, 1H), 8.89 (brs, 1H), 10.33 (brs, 1H)

Reference Example 2

8-Ethoxycarbonyl-5-methylthio-3-phenyl[1,2,4]triazolo[4,3-c]pyrimidine

Diphosphorus pentoxide (49.0 g, 345 mmol) was suspended in xylene (230 ml), and hexamethyldisiloxane (77.2 ml, 345 mmol) was added thereto, followed by stirring at 90°C for about one hour. To the reaction system was added 4-(N'-benzoylhydrazino)-5-ethoxycarbonyl-2-methylthiopyrimidine (22.9 g, 69.1 mmol) obtained in Reference Example 1 and the mixture was stirred at 140°C for about 2 hours. After the completion of reaction, the reaction mixture was neutralized by adding water and a saturated aqueous ammonia solution and subjected to extraction with ethyl acetate. After the organic layer was dried over magnesium sulfate, the solvent was distilled away under reduced pressure. The resulting residue was washed with ethyl acetate to obtain the subject compound (17.0 g, 77%) as a white powder.
$^1$H NMR ($CDCl_3$, δ, ppm): 1.48 (t, J = 7.3 Hz, 3H), 2.57 (s, 3H), 4.56 (q, J = 7.3 Hz, 2H), 7.47-7.64 (m, 5H), 8.50 (s, 1H)

Reference Example 3

8-Ethoxycarbonyl-5-methylthio-2-phenyl[1,2,4]triazolo[1,5-c]pyrimidine

8-Ethoxycarbonyl-5-methylthio-3-phenyl[1,2,4]triazolo[4,3-c]pyrimidine (17.0 g, 54.1 mmol) obtained in Reference Example 2 was dissolved in THF (200 ml), and DBU (11.6 ml, 81.2 mmol) was added thereto under ice-cooling, followed by stirring at room temperature for about one hour. The deposited crystals were recovered by filtration and washed with diethyl ether to obtain the subject compound (11.3 g, 66%) as a white powder.
$^1$H NMR ($CDCl_3$, δ , ppm): 1.50 (t, J = 7.3 Hz, 3H), 2.81 (s, 3H), 4.53 (q, J = 7.3 Hz, 2H), 7.49-7.51 (m, 3H), 8.39-8.43 (m, 2H), 8.80 (s, 1H)

Reference Example 4

5-(3,4-Dimethoxybenzylamino)-8-ethoxycarbonyl-2-phenyl[1,2,4]triazolo[1,5-c]pyrimidine

8-Ethoxycarbonyl-5-methylthio-2-phenyl[1,2,4]triazolo[1,5-c]pyrimidine (11.3 g, 35.8 mmol) obtained in Reference Example 3 was dissolved in ethanol (200 ml), and 3,4-dimethoxybenzylamine (16.2 ml, 107 mmol) was added thereto, followed by stirring at 100°C for about one hour. After the completion of reaction, the solvent was distilled away from the reaction mixture under reduced pressure, and the resulting residue was washed with diethyl ether to obtain the subject compound (14.6 g, 97%) as a white powder.
$^1$H NMR ($CDCl_3$, δ, ppm): 1.47 (t, J = 7.3 Hz, 3H), 3.89 (s, 6H), 4.59 (q, J = 7.3 Hz, 2H), 4.85 (m, 2H), 6.85-7.00 (m, 4H), 7.46-7.48 (m, 3H), 8.31-8.34 (m, 2H), 8.73 (s, 1H)

Reference Example 5

8-Carboxy-5-(3,4-dimethoxybenzylamino)-2-phenyl[1,2,4]triazolo[1,5-c]pyrimidine

5-(3,4-Dimethoxybenzylamino)-8-ethoxycarbonyl-2-phenyl[1,2,4]triazolo[1,5-c]pyrimidine (14.6 g, 34.8 mmol) obtained in Reference Example 4 was dissolved in a mixed solvent of ethanol (218 ml) and water (7.25 ml), and lithium hydroxide (14.7 g, 348 mmol) was added thereto, followed by stirring at 100°C for about 2 hours. After the completion of reaction, the pH of the reaction mixture was adjusted to acidic range by dropwise addition of concentrated hydrochloric acid under ice-cooling, and the deposited crystals were recovered by filtration. The obtained crystals were washed with ice water to obtain the subject compound (14.5 g, quantitative) as a white powder.
$^1$H NMR ($CDCl_3$, δ, ppm): 3.89 (s, 6H), 4.88 (d, J = 5.9 Hz, 2H), 6.86-7.01 (m, 4H), 7.48-7.52 (m, 2H), 8.21-8.25 (m, 2H), 8.80 (s, 1H)

Reference Example 6

5-(3,4-Dimethoxybenzylamino)-N-methoxy-N-methyl-2-phenyl[1,2,4]triazolo[1,5-c]pyrimidine-8-carboxamide

8-Carboxy-5-(3,4-dimethoxybenzylamino)-2-phenyl[1,2,4]triazolo[1,5-c]pyrimidine (14.5 g, substantial content: 34.8 mmol) obtained in Reference Example 5 was dissolved in thionyl chloride (186 ml) and the solution was stirred at room temperature for about 2 hours. After the completion of reaction, thionyl chloride was distilled away from the reaction mixture under reduced pressure, and the resulting residue was dissolved in dichloromethane. N,O-dimethylhydroxylamine hydrochloride (7.25 g, 74.3 mmol) and triethylamine (51.8 ml, 371 mmol) were added thereto, and the mixture was stirred at room temperature for about 1.5 hours. After the completion of reaction, the reaction mixture was subjected to extraction by adding water and ethyl acetate thereto, and the organic layer was dried over magnesium sulfate. The solvent was distilled away under reduced pressure to obtain the subject compound (16.5 g, quantitative) as a yellow amorphous matter.
$^1$H NMR ($CDCl_3$, δ, ppm): 3.37 (m, 3H), 3.74-3.88 (m, 9H), 4.78-4.82 (m, 2H), 6.77-6.96 (m, 4H), 7.42-7.51 (m, 3H), 8.18-8.25 (m, 3H)

Reference Example 7

5-(3,4-Dimethoxybenzylamino)-8-formyl-2-phenyl[1,2,4]triazolo[1,5-c]pyrimidine

5-(3,4-Dimethoxybenzylamino)-N-methoxy-N-methyl-2-phenyl[1,2,4]triazolo[1,5-c]pyrimidine-8-carboxamide (14.8 g, 34.0 mmol) obtained in Reference Example 6 was dissolved in THF (340 ml), and lithium aluminum hydride (1.29 g, 34.0 mmol) was added thereto under ice-cooling, followed by stirring at 0°C for about 2 hours. After the completion of reaction, diethyl ether (340 ml) was added to the reaction mixture, to which a saturated aqueous sodium sulfate solution was added dropwise until the foaming subsided, followed by liquid separation. After the organic layer was dried over anhydrous sodium sulfate, the solvent was distilled away under reduced pressure, and the resulting residue was purified by silica gel column chromatography (eluted with 1% methanol-chloroform) to obtain the subject compound (4.89 g, 38%) as a white powder.
$^1$H NMR ($CDCl_3$, δ, ppm): 3.88 (s, 6H), 4.86 (d, J = 5.9 Hz, 2H), 6.83-6.93 (m, 3H), 7.12 (t, J = 5.9 Hz, 1H), 7.46-7.48 (m, 3H), 8.27-8.31 (m, 2H), 8.55 (s, 1H), 10.31 (s, 1H)

Reference Example 8

5-Ethoxycarbonyl-2-methylthio-4-[N'-(2-thiophenecarbonyl)hydrazino]pyrimidine

The subject compound (quantitative) was obtained as a white powder from 4-chloro-5-ethoxycarbonyl-2-methylthiopyrimidine and 2-thiophenecarboxylic acid hydrazide in a manner similar to that in Reference Example 1.
$^1$H NMR ($CDCl_3$, δ, ppm): 1.40 (t, J = 7.3 Hz, 3H), 2.43 (s, 3H), 4.37 (q, J = 7.3 Hz, 2H), 7.09 (m, 1H), 7.53 (m, 1H), 7.70 (m, 1H), 8.69 (s, 1H), 9.00 (brs, 1H), 10.06 (brs, 1H)

Reference Example 9

8-Ethoxycarbonyl-5-methylthio-3-(2-thienyl)[1,2,4]triazolo[4,3-c]pyrimidine

In a manner similar to that in Reference Example 2, the subject compound (45%) was obtained as a white powder from 5-ethoxycarbonyl-2-methylthio-4-[N'-(2-thiophenecarbonyl)hydrazino]pyrimidine obtained in Reference Example 8.
$^1$H NMR ($CDCl_3$, δ, ppm): 1.45 (t, J = 7.3 Hz, 3H), 2.54 (s, 3H), 4.51 (q, J = 7.3 Hz, 2H), 6.75 (dd, J = 3.8, 5.1 Hz, 1H), 7.44 (dd, J = 1.3, 5.1 Hz, 1H), 7.54 (dd, J = 1.3, 3.8 Hz, 1H), 8.44 (s, 1H)

Reference Example 10

8-Ethoxycarbonyl-5-methylthio-2-(2-thienyl) [1,2,4]triazolo[1,5-c]pyrimidine

In a manner similar to that in Reference Example 3, the subject compound (29%) was obtained as a white powder from 8-ethoxycarbonyl-5-methylthio-3-(2-thienyl)[1,2,4]triazolo[4,3-c] obtained in Reference Example 9.
$^1$H NMR ($CDCl_3$, δ, ppm): 1.47 (t, J = 7.3 Hz, 3H), 2.78 (s, 3H), 4.51 (q, J = 7.3 Hz, 2H), 7.16 (dd, J = 3.8, 5.1 Hz, 1H), 7.50 (dd, J = 1.3, 5.1 Hz, 1H), 8.04 (dd, J = 1.3, 3.8 Hz, 1H), 8.76 (s, 1H)

Reference Example 11

8-Carboxy-5-(3,4-dimethoxybenzylamino)-2-(2-thienyl) [1,2,4]triazolo[1,5-c]pyrimidine

8-Ethoxycarbonyl-5-methylthio-2-(2-thienyl) [1,2,4]triazolo[1,5-c]pyrimidine (12.3 g, 38.5 mmol) obtained in Reference Example 10 was dissolved in ethanol (385 ml). To the solution was added 3,4-dimethoxybenzylamine (19.3 g, 116 mmol), and the mixture was refluxed at 100°C for about 2 hours. After the completion of reaction, the reaction

mixture was subjected to extraction by adding water and ethyl acetate thereto. The organic layer was washed with 0.5 mol/l hydrochloric acid (250 ml) and dried over magnesium sulfate. The solvent was distilled away, and the resulting residue was dissolved in a mixed solvent of ethanol (240 ml) and water (8 ml). To the solution was added lithium hydroxide (16.0 g, 380 mmol), and the mixture was refluxed for about 2 hours. After the completion of reaction, the pH was adjusted to acidic range by dropwise addition of concentrated hydrochloric acid to the reaction mixture under ice-cooling, and the deposited crystals were recovered by filtration. The resulting crystals were washed with ice water to obtain the subject compound (6.25 g, 40%) as a white powder.
$^1$H NMR ($CDCl_3$, δ, ppm): 3.88 (s, 3H), 3.89 (s, 3H), 4.84 (s, 2H), 6.85-6.89 (m, 1H), 6.98-7.02 (m, 2H), 7.16 (dd, J = 3.8, 5.1 Hz, 1H), 7.50 (dd, J = 1.3, 5.1 Hz, 1H), 8.00 (dd, J = 1.3, 3.8 Hz, 1H), 8.50 (s, 1H)

Reference Example 12

5-(3,4-Dimethoxybenzylamino)-8-formyl-2-(2-thienyl) [1,2,4]triazolo[1,5-c]pyrimidine

8-Carboxy-5-(3,4-dimethoxybenzylamino)-2-(2-thienyl) [1,2,4]triazolo[1,5-c]pyrimidine (7.40 g, 18.8 mmol) obtained in Reference Example 11 was dissolved in thionyl chloride (95 ml), followed by stirring at room temperature for about 2 hours. After the completion of reaction, thionyl chloride was distilled away under reduced pressure, and the resulting residue was dissolved in dichloromethane. To the solution were added N,O-dimethylhydroxylamine hydrochloride (3.67 g, 37.6 mmol) and triethylamine (26.2 ml, 188 mmol), followed by stirring at room temperature for about 1.5 hours. After the completion of reaction, the reaction mixture was subjected to extraction by adding water and ethyl acetate thereto, and the organic layer was dried over magnesium sulfate. The solvent was distilled away under reduced pressure, and the resulting residue was dissolved in THF (120 ml). To the solution was added lithium aluminum hydride (714 mg, 18.8 mmol) under ice-cooling, followed by stirring at 0°C for about 2 hours. After the completion of reaction, diethyl ether (120 ml) was added to the reaction mixture, to which a saturated aqueous sodium sulfate solution was added dropwise until the foaming subsided, followed by liquid separation. The organic layer was dried over anhydrous sodium sulfate, the solvent was distilled away under reduced pressure, and the resulting residue was purified by silica gel column chromatography (eluted with 1% methanol-chloroform) to obtain the subject compound (2.06 g, 29%) as a white powder.
$^1$H NMR ($CDCl_3$, δ, ppm): 3.89 (s, 6H), 4.87 (d, J = 5.9 Hz, 2H), 6.85-7.14 (m, 4H), 7.15 (dd, J = 3.8, 5.1 Hz, 1H), 7.49 (dd, J = 1.3, 5.1 Hz, 1H), 7.97 (dd, J = 1.3, 3.8 Hz, 1H), 8.57 (s, 1H), 10.30 (s, 1H)

Reference Example 13

5-Ethoxycarbonyl-4-[N'-(3-furoyl)hydrazino]-2-methylthiopyrimidine

The subject compound (67%) was obtained as a white powder from 4-chloro-5-ethoxycarbonyl-2-methylthiopyrimidine and 3-furoic hydrazide in a manner similar to that in Reference Example 1.
$^1$H NMR ($CDCl_3$, δ, ppm) : 1.40 (t, J = 7.3 Hz, 3H), 2.46 (s, 3H), 4.40 (q, J = 7.3 Hz, 2H), 6.72 (dd, J = 0.8, 1.8 Hz, 1H), 7.47 (dd, J = 1.7, 1.8 Hz, 1H), 8.04 (dd, J = 0.8, 1.7 Hz, 1H), 8.60 (brs, 1H), 8.71 (s, 1H), 10.10 (brs, 1H)

Reference Example 14

8-Ethoxycarbonyl-3-(3-furyl)-5-methylthio[1,2,4]triazolo[4,3-c]pyrimidine

In a manner similar to that in Reference Example 2, the subject compound (89%) was obtained as a white powder from 5-ethoxycarbonyl-4-[N'-(3-furoyl)hydrazino]-2-methylthiopyrimidine obtained in Reference Example 13.
$^1$H NMR ($CDCl_3$, δ, ppm): 1.47 (t, J = 7.3 Hz, 3H), 2.65 (s, 3H), 4.54 (q, J = 7.3 Hz, 2H), 6.72 (dd, J = 0.8, 1.8 Hz, 1H), 7.59 (dd, J = 1.7, 1.8 Hz, 1H), 7.83 (dd, J = 0.8, 1.7 Hz, 1H), 8.49 (s, 1H)

Reference Example 15

8-Ethoxycarbonyl-2-(3-furyl)-5-methylthio[1,2,4]triazolo[1,5-c]pyrimidine

In a manner similar to that in Reference Example 3, the subject compound (91%) was obtained as a white powder from 8-ethoxycarbonyl-3-(3-furyl)-5-methylthio[1,2,4]triazolo[4,3-c]pyrimidine obtained in Reference Example 14.
$^1$H NMR ($CDCl_3$, δ, ppm): 1.47 (t, J = 7.3 Hz, 3H), 2.80 (s, 3H), 4.53 (q, J = 7.3 Hz, 2H), 7.10 (dd, J = 0.8, 1.8 Hz, 1H), 7.53 (dd, J = 1.7, 1.8 Hz, 1H), 8.34 (dd, J = 0.8, 1.7 Hz, 1H), 8.78 (s, 1H)

Reference Example 16

5-(3,4-Dimethoxybenzylamino)-8-ethoxycarbonyl-2-(3-furyl)[1,2,4]triazolo[1,5-c]pyrimidine

In a manner similar to that in Reference Example 4, the subject compound (90%) was obtained as a pink powder from 8-ethoxycarbonyl-2-(3-furyl)-5-methylthio[1,2,4]triazolo[1,5-c]pyrimidine obtained in Reference Example 15.
$^{1}$H NMR ($CDCl_3$, δ, ppm): 1.45 (t, J = 7.3 Hz, 3H), 3.88 (s, 6H), 4.49 (q, J = 7.3 Hz, 2H), 4.83 (d, J = 5.8 Hz, 2H), 6.81-7.12 (m, 5H), 7.50 (dd, J = 1.7, 1.8 Hz, 1H), 8.27 (dd, J = 0.8, 1.7 Hz, 1H), 8.71 (s, 1H)

Reference Example 17

8-Carboxy-5-(3,4-dimethoxybenzylamino)-2-(3-furyl)[1,2,4]triazolo[1,5-c]pyrimidine

In a manner similar to that in Reference Example 5, the subject compound (97%) was obtained as a white powder from 5-(3,4-dimethoxybenzylamino)-8-ethoxycarbonyl-2-(3-furyl)[1,2,4]triazolo[1,5-c]pyrimidine obtained in Reference Example 16.
$^{1}$H NMR ($CDCl_3$, δ, ppm): 3.88 (s, 6H) , 4.84 (s, 2H), 6.80-7.12 (m, 4H), 7.53 (dd, J = 1.7, 1.8 Hz, 1H), 8.32 (dd, J = 0.8, 1.7 Hz, 1H), 8.79 (s, 1H)

Reference Example 18

5-(3,4-Dimethoxybenzylamino)-N-methoxy-N-methyl-2-(3-furyl)[1,2,4]triazolo[1,5-c]pyrimidine-8-carboxamide

In a manner similar to that in Reference Example 6, the subject compound (94%) was obtained as a white powder from 8-carboxy-5-(3,4-dimethoxybenzylamino)-2-(3-furyl) [1,2,4]triazolo[1,5-c]pyrimidine obtained in Reference Example 17.
$^{1}$H NMR ($CDCl_3$, δ, ppm): 3.41 (s, 3H), 3.74 (s, 3H), 3.89 (s, 6H), 4.81 (d, J = 5.8 Hz, 2H), 6.63 (t, J = 5.3 Hz, 1H), 6.85-6.99 (m, 4H), 7.49 (dd, J = 1.7, 1.8 Hz, 1H), 8.21 (dd, J = 0.8, 1.7 Hz, 1H), 8.23 (s, 1H)

Reference Example 19

5-(3,4-Dimethoxybenzylamino)-8-formyl-2-(3-furyl)[1,2,4]triazolo[1,5-c]pyrimidine

In a manner similar to that in Reference Example 7, the subject compound (51%) was obtained as a white powder from 5-(3,4-dimethoxybenzylamino)-N-methoxy-N-methyl-2-(3-furyl)[1,2,4]triazolo[1,5-c]pyrimidine-8-carboxamide obtained in Reference Example 18.
$^{1}$H NMR ($CDCl_3$, δ , ppm): 3.89 (s, 6H), 4.87 (d, J = 5.9 Hz, 2H), 6.83-7.00 (m, 5H), 7.26 (dd, J = 1.7, 1.8 Hz, 1H), 8.27 (dd, J = 0.8, 1.7 Hz, 1H), 8.55 (s, 1H), 10.24 (s, 1H)

Reference Example 20

5-Ethoxycarbonyl-2-methylthio-4-(N'-nicotinoylhydrazino)pyrimidine

The subject compound (82%) was obtained as a pale yellow powder from 4-chloro-5-ethoxycarbonyl-2-methylthiopyrimidine and nicotinic acid hydrazide in a manner similar to that in Reference Example 1.
$^{1}$H NMR ($CDCl_3$, δ, ppm): 1.41 (t, J = 7.3 Hz, 3H), 2.48 (s, 3H), 4.40 (q, J = 7.3 Hz, 2H), 7.44 (ddd, J = 1.0, 5.0, 7.9 Hz, 1H), 8.20 (ddd, J = 2.0, 2.0, 7.9 Hz, 1H), 8.73 (s, 1H), 8.77 (dd, J = 2.0, 5.0 Hz, 1H), 9.00-9.21 (m, 2H), 10.31 (brs, 1H)

Reference Example 21

5-(3,4-Dimethoxybenzylamino)-8-ethoxycarbonyl-2-(3-pyridyl) [1,2,4]triazolo[1,5-c]pyrimidine

5-Ethoxycarbonyl-2-methylthio-4-(N'-nicotinoylhydrazino)pyrimidine (12.0 g, 36.0 mmol) obtained in Reference Example 20 was dissolved in phosphorus oxychloride (80 ml), and the solution was refluxed at 120°C for about one hour. Phosphorus oxychloride was distilled away under reduced pressure, and the resulting residue was dissolved in THF (100 ml). DBU (20 ml, 145 mmol) was added thereto, and the mixture was stirred at room temperature for about 30 minutes. Subsequently, 3,4-dimethoxybenzylamine (18.1 g, 108 mmol) was added thereto, and the mixture was

refluxed at 100°C for about 2 hours. After the completion of reaction, the reaction mixture was subjected to extraction by adding water and ethyl acetate thereto, and the organic layer was dried over magnesium sulfate. The solvent was distilled away under reduced pressure, and the resulting residue was washed with ethyl acetate to obtain the subject compound (12.3 g, 79%) as a white powder.
$^1$H NMR ($CDCl_3$, δ, ppm): 1.47 (t, J = 7.3 Hz, 3H), 3.89 (s, 6H), 4.49 (q, J = 7.3 Hz, 2H), 4.88 (d, J = 5.8 Hz, 2H), 6.88-7.03 (m, 4H), 7.42 (ddd, J = 1.0, 5.0, 7.9 Hz, 1H), 8.62 (ddd, J = 2.0, 2.0, 7.9 Hz, 1H), 8.71 (dd, J = 2.0, 5.0 Hz, 1H), 8.76 (s, 1H), 9.50 (dd, J = 1.0, 2.0 Hz, 1H)

Reference Example 22

5-(3,4-Dimethoxybenzylamino)-8-hydroxymethyl-2-(3-pyridyl)[1,2,4]triazolo[1,5-c]pyrimidine

5-(3,4-Dimethoxybenzylamino)-8-ethoxycarbonyl-2-(3-pyridyl)[1,2,4]triazolo[1,5-c]pyrimidine (5.60 g, 12.9 mmol) obtained in Reference Example 21 was dissolved in dichloromethane (100 ml) and cooled to -78°C. Thereafter, a solution of 0.93 mol/l diisobutylaluminum hydride in toluene (41.6 ml, 38.7 mmol) was added dropwise thereto. The temperature of the reaction mixture was raised to room temperature, and the mixture was stirred for about 30 minutes. After the completion of reaction, diethyl ether (100 ml) was added to the reaction mixture, to which saturated aqueous sodium sulfate solution was added dropwise until the foaming subsided, followed by liquid separation. The organic layer was dried over anhydrous sodium sulfate, and the solvent was distilled away under reduced pressure to obtain the subject compound (2.83 g, 56%) as a white powder.
$^1$H NMR ($CDCl_3$, δ, ppm): 3.87 (s, 6H), 4.78 (d, J = 5.3 Hz, 2H), 4.93 (s, 2H), 6.54 (t, J = 5.3 Hz, 1H) , 6.79-7.01 (m, 3H), 7.39 (ddd, J = 1.0, 5.0, 7.9 Hz, 1H), 7.92 (s, 1H), 8.49 (ddd, J = 2.0, 2.0, 7.9 Hz, 1H), 8.69 (dd, J = 2.0, 5.0 Hz, 1H), 9.43 (dd, J = 1.0, 2.0 Hz, 1H)

Reference Example 23

5-(3,4-Dimethoxybenzylamino)-8-formyl-2-(3-pyridyl)[1,2,4]triazolo[1,5-c]pyrimidine

5-(3,4-Dimethoxybenzylamino)-8-hydroxymethyl-2-(3-pyridyl)[1,2,4]triazolo[1,5-c]pyrimidine (2.00 g, 5.10 mmol) obtained in Reference Example 22 was dissolved in 1,2-dichloroethane (50 ml). To the solution was added manganese dioxide (4.43 g, 51.0 mmol) , and the mixture was refluxed for about 2 hours. After the completion of reaction, manganese dioxide was removed by filtration, and the solvent was distilled away under reduced pressure to obtain the subject compound (62%) as a white powder.
$^1$H NMR ($CDCl_3$, δ, ppm): 3.86 (s, 6H), 4.92 (d, J = 5.9 Hz, 2H), 6.85 (d, J = 7.8 Hz, 1H), 6.97 (s, 1H), 6.99 (d, J = 7.8 Hz, 1H), 7.44 (ddd, J = 1.0, 5.0, 7.9 Hz, 1H), 7.75 (t, J = 5.9 Hz, 1H), 8.55 (ddd, J = 2.0, 2.0, 7.9 Hz, 1H), 8.58 (s, 1H), 8.67 (dd, J = 2.0, 5.0 Hz, 1H), 9.43 (dd, J = 1.0, 2.0 Hz, 1H), 10.27 (s, 1H)

Reference Example 24

4-(N'-Acetylhydrazino)-5-ethoxycarbonyl-2-methylthiopyrimidine

The subject compound (84%) was obtained as a white powder from 4-chloro-5-ethoxycarbonyl-2-methylthiopyrimidine and acetylhydrazine in a manner similar to that in Reference Example 1.
$^1$H NMR ($CDCl_3$, δ, ppm) : 1.38 (t, J = 7.3 Hz, 3H), 2.13 (s, 3H), 2.52 (s, 3H), 4.36 (q, J = 7.3 Hz, 2H), 8.28 (brs, 1H), 8.69 (s, 1H), 9.96 (brs, 1H)

Reference Example 25

8-Ethoxycarbonyl-3-methyl-5-methylthio[1,2,4]triazolo[4,3-c]pyrimidine

In a manner similar to that in Reference Example 2, the subject compound (78%) was obtained as a white powder from 4-(N'-acetylhydrazino)-5-ethoxycarbonyl-2-methylthiopyrimidine obtained in Reference Example 24.
$^1$H NMR ($CDCl_3$, δ, ppm): 1.37 (t, J = 7.3 Hz, 3H), 2.45 (s, 3H), 4.33 (q, J = 7.3 Hz, 2H), 4.46 (s, 3H), 8.65 (s, 1H)

Reference Example 26

5-(3,4-Dimethoxybenzylamino)-8-ethoxycarbonyl-2-methyl[1,2,4]triazolo[1,5-c]pyrimidine

8-Ethoxycarbonyl-3-methyl-5-methylthio[1,2,4]triazolo[4,3-c]pyrimidine (14.8 g, 58.6 mmol) obtained in Reference Example 25 was dissolved in THF (150 ml), and DBU (13.4 g, 87.9 mmol) was added thereto, followed by stirring at room temperature for about 20 minutes. Subsequently, 3,4-dimethoxybenzylamine (14.7 g, 87.9 mmol) was added to the reaction mixture, followed by stirring at 60°C for about 2 hours. After the completion of reaction, the reaction mixture was subjected to extraction by adding water and chloroform thereto, and the organic layer was dried over magnesium sulfate. The solvent was distilled away under reduced pressure, and the resulting residue was washed with ethyl acetate to obtain the subject compound (17.3 g, 80%) as a white powder.
$^1$H NMR ($CDCl_3$, δ, ppm): 1.43 (t, J = 7.3 Hz, 3H), 2.60 (s, 3H), 3.88 (s, 6H), 4.46 (q, J = 7.3 Hz, 2H), 4.80 (d, J = 5.8 Hz, 2H), 6.86 (t, J = 5.8 Hz, 1H), 6.90-6.97 (m, 3H), 8.69 (s, 1H)

Reference Example 27

5-(3,4-Dimethoxybenzylamino)-8-hydroxymethyl-2-methyl[1,2,4]triazolo[1,5-c]pyrimidine

In a manner similar to that in Reference Example 22, the subject compound (88%) was obtained as a pale yellow oily matter from 5-(3,4-dimethoxybenzylamino)-8-ethoxycarbonyl-2-methyl[1,2,4]triazolo[1,5-c]pyrimidine obtained in Reference Example 26.
$^1$H NMR ($CDCl_3$, δ, ppm): 2.56 (s, 3H), 2.72 (s, 1H), 3.87 (s, 3H), 3.88 (s, 3H), 4.74 (d, J = 5.8 Hz, 2H), 4.86 (s, 2H), 6.28 (t, J = 5.8 Hz, 1H), 6.84 (d, J = 7.8 Hz, 1H), 6.93 (s, 1H), 6.94 (d, J = 7.8 Hz, 1H), 7.85 (s, 1H)

Reference Example 28

5-(3,4-Dimethoxybenzylamino)-8-formyl-2-methyl[1,2,4]triazolo[1,5-c]pyrimidine

In a manner similar to that in Reference Example 23, the subject compound (75%) was obtained as a white powder from 5-(3,4-dimethoxybenzylamino)-8-hydroxymethyl-2-methyl[1,2,4]triazolo[1,5-c]pyrimidine obtained in Reference Example 27.
$^1$H NMR ($CDCl_3$, δ, ppm) : 2.61 (s, 3H) , 3.88 (s, 3H), 3.88 (s, 3H), 4.85 (d, J = 5.8 Hz, 2H), 6.87 (d, J = 8.2 Hz, 1H), 6.92 (s, 1H) , 6.94 (d, J = 8.2 Hz, 1H), 6.98 (t, J = 5.8 Hz, 1H), 8.51 (s, 1H), 10.17 (s, 1H)

Reference Example 29

5-Ethoxycarbonyl-4-hydrazino-2-methylthiopyrimidine

4-Chloro-5-ethoxycarbonyl-2-methylthiopyrimidine (26.5 g, 114 mmol) was dissolved in ethanol (250 ml), and hydrazine monohydrate (28.5 g, 570 mmol) was added thereto under ice-cooling, followed by stirring at room temperature for about 2 hours. After the completion of reaction, the solvent was distilled away under reduced pressure. The resulting residue was washed with water to obtain the subject compound (24.5 g, 94%) as a white powder.
$^1$H NMR ($CDCl_3$, δ, ppm): 1.37 (t, J = 7.3 Hz, 3H), 2.56 (s, 3H), 4.15 (brs, 2H), 4.33 (q, J = 7.3 Hz, 2H), 8.65 (s, 1H), 9.00 (brs, 1H)

Reference Example 30

8-Ethoxycarbonyl-5-methylthio[1,2,4]triazolo[1,5-c]pyrimidine

5-Ethoxycarbonyl-4-hydrazino-2-methylthiopyrimidine (19.2 g, 84.1 mmol) obtained in Reference Example 29 was dissolved in trimethyl orthoformate (200 ml), and the solution was refluxed at 130°C for about 2 hours. Trimethyl orthoformate was distilled away under reduced pressure, and the resulting residue was dissolved in xylene (200 ml). To the solution was added pyridinium p-toluenesulfonate (1.0 g, 5 wt%), and the mixture was refluxed at 150°C for about 2 hours. After the completion of reaction, the reaction mixture was subjected to extraction by adding water and ethyl acetate thereto, and the organic layer was dried over magnesium sulfate. The solvent was distilled away under reduced pressure to obtain the subject compound (20.0 g, quantitative) as a yellow solid.
$^1$H NMR ($CDCl_3$, δ , ppm): 1.47 (t, J = 7.3 Hz, 3H), 2.81 (s, 3H), 4.53 (q, J = 7.3 Hz, 2H), 8.51 (s, 1H), 8.83 (brs, 1H)

Reference Example 31

5-(3,4-Dimethoxybenzylamino)-8-ethoxycarbonyl[1,2,4]triazolo[1,5-c]pyrimidine

In a manner similar to that in Reference Example 4, the subject compound (82%) was obtained as a white powder from 8-ethoxycarbonyl-5-methylthio[1,2,4]triazolo[1,5-c]pyrimidine obtained in Reference Example 30.
$^1$H NMR ($CDCl_3$, δ, ppm): 1.42 (t, J = 7.3 Hz, 3H), 3.88 (s, 6H), 4.49 (q, J = 7.3 Hz, 2H), 4.84 (d, J = 5.8 Hz, 2H), 6.85-6.97 (m, 4H), 8.34 (s, 1H), 8.76 (s, 1H)

Reference Example 32

5-(3,4-Dimethoxybenzylamino)-8-hydroxymethyl[1,2,4]triazolo[1,5-c]pyrimidine

In a manner similar to that in Reference Example 22, the subject compound (70%) was obtained as a pale yellow oily matter from 5-(3,4-dimethoxybenzylamino)-8-ethoxycarbonyl[1,2,4]triazolo[1,5-c]pyrimidine obtained in Reference Example 31.
$^1$H NMR ($CDCl_3$, δ, ppm): 2.83 (s, 1H), 3.88 (s, 3H), 3.88 (s, 3H), 4.77 (d, J = 5.8 Hz, 2H), 4.90 (s, 2H), 6.40 (t, J = 5.8 Hz, 1H), 6.85 (d, J = 7.6 Hz, 1H), 6.94 (s, 1H), 6.95 (d, J = 7.6 Hz, 1H), 7.91 (s, 1H), 8.25 (s, 1H)

Reference Example 33

5-(3,4-Dimethoxybenzylamino)-8-formyl[1,2,4]triazolo[1,5-c]pyrimidine

In a manner similar to that in Reference Example 23, the subject compound (94%) was obtained as white crystals from 5-(3,4-dimethoxybenzylamino)-8-hydroxymethyl[1,2,4]triazolo[1,5-c]pyrimidine obtained in Reference Example 32.
$^1$H NMR ($CDCl_3$, δ, ppm): 3.88 (s, 3H), 3.88 (s, 3H), 4.88 (d, J = 5.8 Hz, 2H), 6.87 (d, J = 7.9 Hz, 1H), 6.94 (s, 1H), 6.95 (d, J = 7.9 Hz, 1H), 6.98 (t, J = 5.8 Hz, 1H), 8.35 (s, 1H), 8.57 (s, 1H), 10.24 (s, 1H)

Reference Example 34

5-Ethoxycarbonyl-2-methylthio-4-(N'-phenylacetylhydrazino)pyrimidine

The subject compound (80%) was obtained as a white powder from 4-chloro-5-ethoxycarbonyl-2-methylthiopyrimidine and phenylacetylhydrazine in a manner similar to that in Reference Example 1.
$^1$H NMR ($CDCl_3$, δ, ppm): 1.37 (t, J = 7.3 Hz, 3H), 2.28 (s, 3H), 3.74 (s, 2H), 4.36 (q, J = 7.3 Hz, 2H), 7.30-7.41 (m, 5H), 8.13 (brs, 1H), 8.64 (s, 1H), 10.18 (brs, 1H)

Reference Example 35

3-Benzyl-8-ethoxycarbonyl-5-methylthio[1,2,4]triazolo[4,3-c]pyrimidine

In a manner similar to that in Reference Example 2, the subject compound (79%) was obtained as pale orange crystals from 5-ethoxycarbonyl-2-methylthio-4-(N'-phenylacetylhydrazino)pyrimidine obtained in Reference Example 34.
$^1$H NMR ($CDCl_3$, δ, ppm): 1.46 (t, J = 7.3 Hz, 3H), 2.69 (s, 3H), 4.52 (q, J = 7.3 Hz, 2H), 4.91 (s, 2H), 7.13-7.32 (m, 5H), 8.39 (s, 1H)

Reference Example 36

2-Benzyl-8-ethoxycarbonyl-5-methylthio[2,2,4]triazolo[1,5-c] pyrimidine

In a manner similar to that in Reference Example 3, the subject compound (73%) was obtained as a white powder from 3-benzyl-8-ethoxycarbonyl-5-methylthio[1,2,4]triazolo[4,3-c]pyrimidine obtained in Reference Example 35.
$^1$H NMR ($CDCl_3$, δ, ppm) : 1.43 (t, J = 7.3 Hz, 3H) , 2.76 (s, 3H), 4.36 (s, 2H), 4.47 (q, J = 7.3 Hz, 2H), 7.17-7.33 (m, 3H), 7.41-7.46 (m, 2H), 8.74 (s, 1H)

Reference Example 37

2-Benzyl-5-(3,4-dimethoxybenzylamino)-8-ethoxycarbonyl[1,2,4]triazolo[1,5-c]pyrimidine

In a manner similar to that in Reference Example 4, the subject compound (90%) was obtained as a yellow amorphous matter from 2-benzyl-8-ethoxycarbonyl-5-methylthio[1,2,4]triazolo[1,5-c]pyrimidine obtained in Reference Example 36.
$^1$H NMR ($CDCl_3$, δ , ppm): 1.42 (t, J = 7.3 Hz, 3H), 3.85 (s, 3H), 3.86 (s, 3H), 4.27 (s, 2H), 4.44 (q, J = 7.3 Hz, 2H), 4.74 (d, J = 5.9 Hz, 2H), 6.78-6.93 (m, 4H), 7.13-7.38 (m, 5H), 8.67 (s, 1H)

Reference Example 38

2-Benzyl-5-(3,4-dimethoxybenzylamino)-8-hydroxymethyl[1,2,4]triazolo[1,5-c]pyrimidine

In a manner similar to that in Reference Example 22, the subject compound (65%) was obtained as a pale yellow oily matter from 2-benzyl-5-(3,4-dimethoxybenzylamino)-8-ethoxycarbonyl[1,2,4]triazolo[1,5-c]pyrimidine obtained in Reference Example 37.
$^1$H NMR ($CDCl_3$, δ, ppm): 3.85 (s, 3H), 3.86 (s, 3H), 4.19 (s, 2H), 4.70 (d, J = 5.9 Hz, 2H), 4.81 (d, J = 5.7 Hz, 2H), 6.38 (t, J = 5.9 Hz, 1H), 6.78-6.95 (m, 3H), 7.15-7.35 (m, 5H), 7.84 (s, 1H)

Reference Example 39

2-Benzyl-5-(3,4-dimethoxybenzylamino)-8-formyl[1,2,4]triazolo[1,5-c]pyrimidine

In a manner similar to that in Reference Example 23, the subject compound (50%) was obtained as white crystals from 2-benzyl-5-(3,4-dimethoxybenzylamino)-8-hydroxymethyl[1,2,4]triazolo[1,5-c]pyrimidine obtained in Reference Example 38.
$^1$H NMR ($CDCl_3$, δ, ppm): 3.87 (s, 3H), 3.88 (s, 3H), 4.27 (s, 2H), 4.81 (d, J = 5.8 Hz, 2H), 6.81-6.96 (m, 4H), 7.16-7.38 (m, 5H), 8.51 (s, 1H), 10.19 (s, 1H)

Reference Example 40

4-(N'-Cyclohexylcarbonylhydrazino)-5-ethoxycarbonyl-2-methylthiopyrimidine

The subject compound (93%) was obtained as a white powder from 4-chloro-5-ethoxycarbonyl-2-methylthiopyrimidine and cyclohexylcarbonylhydrazine in a manner similar to that in Reference Example 1.
$^1$H NMR ($CDCl_3$, δ, ppm): 1.23-1.98 (m, 13H), 2.27 (tt, J = 3.6, 11.5 Hz, 1H), 2.52 (s, 3H), 4.35 (q, J = 7.3 Hz, 2H), 8.16 (brs, 1H), 8.68 (s, 1H), 10.03 (brs, 1H)

Reference Example 41

3-Cyclohexyl-8-ethoxycarbonyl-5-methylthio[1,2,4]triazolo[4,3-c]pyrimidine

In a manner similar to that in Reference Example 2, the subject compound (91%) was obtained as a pale yellow powder from 4-(N'-cyclohexylcarbonylhydrazino)-5-ethoxycarbonyl-2-methylthiopyrimidine obtained in Reference Example 40.
$^1$H NMR ($CDCl_3$, δ, ppm): 1.34-1.55 (m, 6H), 1.79-1.95 (m, 5H), 2.16-2.22 (m, 2H), 2.79 (s, 3H), 3.68 (tt, J = 3.6, 11.5 Hz, 1H), 4.49 (q, J = 7.3 Hz, 2H), 8.38 (s, 1H)

Reference Example 42

2-Cyclohexyl-8-ethoxycarbonyl-5-methylthio[1,2,4]triazolo[1,5-c]pyrimidine

In a manner similar to that in Reference Example 3, the subject compound (quantitative) was obtained as a pale yellow solid from 3-cyclohexyl-8-ethoxycarbonyl-5-methylthio[1,2,4]triazolo[4,3-c] obtained in Reference Example 41.
$^1$H NMR ($CDCl_3$, δ, ppm): 1.25-1.50 (m, 6H), 1.66-1.89 (m, 5H), 2.02-2.18 (m, 2H), 2.77 (s, 3H), 3.07 (tt, J = 3.6, 11.5 Hz, 1H), 4.48 (q, J = 7.3 Hz, 2H), 8.73 (s, 1H)

Reference Example 43

2-Cyclohexyl-5-(3,4-dimethoxybenzylamino)-8-ethoxycarbonyl[1,2,4]triazolo[1,5-c]pyrimidine

In a manner similar to that in Reference Example 4, the subject compound (82%) was obtained as a white powder from 2-cyclohexyl-8-ethoxycarbonyl-5-methylthio[1,2,4]triazolo[1,5-c]pyrimidine obtained in Reference Example 42.
$^1$H NMR ($CDCl_3$, $\delta$, ppm): 1.25-1.46 (m, 6H), 1.55-1.90 (m, 5H), 2.02-2.15 (m, 2H), 2.97 (tt, J = 3.6, 11.5 Hz, 1H), 3.88 (s, 6H), 4.44 (q, J = 7.3 Hz, 2H), 4.81 (d, J = 5.8 Hz, 2H), 6.77 (t, J = 5.8 Hz, 1H), 6.83-6.98 (m, 3H), 8.67 (s, 1H)

Reference Example 44

2-Cyclohexyl-5-(3,4-dimethoxybenzylamino)-8-hydroxymethyl[1,2,4]triazolo[1,5-c]pyrimidine

In a manner similar to that in Reference Example 22, the subject compound (77%) was obtained as a pale yellow oily matter from 2-cyclohexyl-5-(3,4-dimethoxybenzylamino)-8-ethoxycarbonyl[1,2,4]triazolo[1,5-c]pyrimidine obtained in Reference Example 43.
$^1$H NMR ($CDCl_3$, $\delta$, ppm): 1.23-1.40 (m, 3H), 1.44-1.78 (m, 5H), 1.99-2.05 (m, 2H), 2.85 (tt, J = 3.6, 11.5 Hz, 1H), 3.87 (s, 6H), 4.13 (t, J = 5.6 Hz, 1H), 4.73 (d, J = 5.9 Hz, 2H), 4.87 (d, J = 5.6 Hz, 2H), 6.38 (t, J = 5.9 Hz, 1H), 6.80-6.95 (m, 3H), 7.86 (s, 1H)

Reference Example 45

2-Cyclohexyl-5-(3,4-dimethoxybenzylamino)-8-formyl[1,2,4]triazolo[1,5-c]pyrimidine

In a manner similar to that in Reference Example 23, the subject compound (63%) was obtained as a white powder from 2-cyclohexyl-5-(3,4-dimethoxybenzylamino)-8-hydroxymethyl[1,2,4]triazolo[1,5-c]pyrimidine obtained in Reference Example 44.
$^1$H NMR ($CDCl_3$, $\delta$, ppm): 1.27-1.49 (m, 3H), 1.55-1.87 (m, 5H), 2.02-2.12 (m, 2H), 2.96 (tt, J = 3.6, 11.5 Hz, 1H), 3.89 (s, 6H), 4.86 (d, J = 5.8 Hz, 2H), 6.84-7.00 (m, 4H), 8.51 (s, 1H), 10.23 (s, 1H)

Reference Example 46

2-(2-Furyl)-8-hydroxymethyl-5-methylthio[1,2,4]triazolo[1,5-c]pyrimidine

The subject compound (48%) was obtained as pale yellow crystals from a known compound (WO98/42711), 8-ethoxycarbonyl-2-(2-furyl)-5-methylthio[1,2,4]triazolo[1,5-c]pyrimidine, in a manner similar to that in Reference Example 22.
$^1$H NMR ($CDCl_3$, $\delta$, ppm): 1.45-1.78 (m, 1H), 2.84 (s, 3H), 5.02 (s, 2H), 6.59 (dd, J = 1.8, 3.6 Hz, 1H), 7.29 (d, J = 3.6 Hz, 1H), 7.65 (d, J = 1.8 Hz, 1H), 8.12 (s, 1H)

Reference Example 47

8-Formyl-2-(2-furyl)-5-methylthio[1,2,4]triazolo[1,5-c]pyrimidine

In a manner similar to that in Reference Example 23, the subject compound (80%) was obtained as white crystals from 2-(2-furyl)-8-hydroxymethyl-5-methylthio[1,2,4]triazolo[1,5-c]pyrimidine obtained in Reference Example 46.
$^1$H NMR (DMSO-$d_6$, $\delta$, ppm): 2.82 (s, 3H), 6.78 (dd, J = 1.8, 3.5 Hz, 1H), 7.38 (d, J = 3.5 Hz, 1H), 8.00 (d, J = 1.8 Hz, 1H), 8.80 (s, 1H), 10.29 (s, 1H)

Reference Example 48

Methyl(1-benzyl-4-methylaminopiperidin-4-yl)acetate and N-methyl-(1-benzyl-4-methylaminopiperidin-4-yl)acetamide

A known compound (Japanese Published Unexamined Patent Application No. 87567/98), methyl(1-benzylpiperidin-4-ylidene) acetate (712 mg, 2.85 mmol), was dissolved in 40% methylamine (methanol solution, 6 ml), and the solution was stirred in a sealed tube at 50°C for 3 hours. After the reaction mixture was allowed to cool to room

temperature, the solvent was distilled away under reduced pressure, and the resulting residue was purified by silica gel column chromatography (eluted with ethyl acetate/methanol/triethylamine = 10/1/0.5) to obtain methyl(1-benzyl-4-methylaminopiperidin-4-yl)acetate (376 mg, 48%) and N-methyl-(1-benzyl-4-methylaminopiperidin-4-yl)acetamide (226 mg, 29%), respectively as a pale yellow oily matter.
Methyl(1-benzyl-4-methylaminopiperidin-4-yl)acetate
$^1$H NMR ($CDCl_3$, δ , ppm): 1.49-1.73 (m, 5H), 2.27 (s, 3H), 2.38-2.52 (m, 6H), 3.50 (s, 2H), 3.66 (s, 3H), 7.18-7.36 (m, 5H)
N-methyl-(1-benzyl-4-methylaminopiperidin-4-yl)acetamide
$^1$H NMR ($CDCl_3$, δ, ppm): 1.53-1.69 (m, 6H), 2.24-2.56 (m, 5H), 2.28 (s, 3H), 2.31 (s, 2H), 7.76 (d, J = 4.9 Hz, 3H), 3.50 (s, 2H), 7.18-7.37 (m, 5H), 8.36-8.52 (m, 1H)

Reference Example 49

1-Benzyl-4-(2-hydroxyethyl)-4-methylaminopiperidine

Lithium aluminum hydride (367 mg, 9.69 mmol) was suspended in THF (10 ml) in a stream of argon, and a solution of methyl(1-benzyl-4-methylaminopiperidin-4-yl)acetate (891 mg, 3.23 mmol) obtained in Reference Example 48 in THF (5 ml) was added thereto under ice-cooling, followed by stirring at room temperature for 2 hours. After the addition of methanol under ice-cooling until the foaming subsided, a 20% aqueous solution of potassium sodium tartrate (8 ml) was added thereto, and the mixture was further stirred at room temperature for one hour. The reaction mixture was diluted with ethyl acetate, washed with water and saturated saline and dried over anhydrous magnesium sulfate. The solvent was distilled away under reduced pressure to obtain the subject compound (751 mg, 94%) as a pale brown oily matter.
$^1$H NMR ($CDCl_3$, δ, ppm): 1.44-1.80 (m, 4H), 1.62 (t, J = 5.6 Hz, 2H), 2.24-2.63 (m, 5H), 2.31 (s, 3H), 3.46-3.97 (m, 1H), 3.49 (s, 1H), 3.80 (t, J = 5.6 Hz, 2H), 7.22-7.41 (m, 5H)

Reference Example 50

9-Benzyl-1-methyl-3-oxa-1,9-diazaspiro[5.5]undecan-2-one

1-Benzyl-4-(2-hydroxyethyl)-4-methylaminopiperidine (751 mg, 3.21 mmol) obtained in Reference Example 49 was dissolved in toluene (100 ml), and carbonyldiimidazole (2.08 g, 12.8 mmol) was added thereto, followed by stirring at 50°C for 11 hours. After cooling to room temperature, the solvent was distilled away under reduced pressure, and the resulting residue was purified by silica gel column chromatography (eluted with chloroform/methanol = 20/1) to obtain the subject compound (508 mg, 58%) as a pale brown oily matter.
$^1$H NMR ($CDCl_3$, δ, ppm): 1.42-1.53 (m, 2H), 1.98-2.27 (m, 6H), 2.79-2.90 (m, 2H), 2.94 (s, 3H), 3.52 (s, 2H), 4.10 - 4.19 (m, 2H), 7.22-7.38 (m, 5H)

Reference Example 51

1-Methyl-3-oxa-1,9-diazaspiro[5.5]undecan-2-one

9-Benzyl-1-methyl-3-oxa-1,9-diazaspiro[5.5]undecan-2-one (500 mg, 1.82 mmol) obtained in Reference Example 50 and 10% palladium-carbon (250 mg) were suspended in ethanol (10 ml), and the suspension was stirred in a hydrogen stream at room temperature for 22 hours. After palladium-carbon was removed from the reaction mixture by filtration through Celite, the solvent was distilled away under reduced pressure to obtain the subject compound (330 mg, quantitative) as a pale brown oily matter.
$^1$H NMR ($CDCl_3$, δ , ppm): 1.60-1.74 (m, 2H), 1.96-2.32 (m, 4H), 2.41-2.59 (m, 2H), 2.75-3.04 (m, 1H), 2.97 (s, 3H), 3.31-3.45 (m, 2H), 4.07-4.28 (m, 2H)

Reference Example 52

1-Benzyl-4-methylamino-4-(2-methylaminoethyl)piperidine

In a manner similar to that in Reference Example 49, the subject compound (quantitative) was obtained as a pale brown oily matter from N-methyl-(1-benzyl-4-methylaminopiperidin-4-yl)acetamide obtained in Reference Example 48.
$^1$H NMR ($CDCl_3$, δ, ppm): 1.50-1.72 (m, 6H) , 2.21-2.62 (m, 6H), 2.25 (s, 3H), 2.42 (s, 3H), 3.40 (s, 2H), 3.46-3.58 (m, 2H), 3.64-3.82 (m, 2H), 7.29-7.40 (m, 5H)

Reference Example 53

9-Benzyl-1,3-dimethyl-1,3,9-triazaspiro[5.5]undecan-2-one

In a manner similar to that in Reference Example 50, the subject compound (82%) was obtained as a pale brown oily matter from 1-benzyl-4-methylamino-4-(2-methylaminoethyl)piperidine obtained in Reference Example 52.
$^1$H NMR ($CDCl_3$, δ, ppm): 1.39-1.48 (m, 2H), 1.91-2.00 (m, 2H), 2.07-2.22 (m 4H) , 2.73-2.83 (m, 2H), 2.91 (s, 3H), 2.92 (s, 3H), 3.10-3.18 (m, 2H), 3.51 (s, 2H), 7.22-7.38 (m, 5H)

Reference Example 54

1,3-Dimethyl-1,3,9-triazaspiro[5.5]undecan-2-one

In a manner similar to that in Reference Example 51, the subject compound (quantitative) was obtained as a pale brown oily matter from 9-benzyl-1,3-dimethyl-1,3,9-triazaspiro[5.5]undecan-2-one obtained in Reference Example 53.
$^1$H NMR ($CDCl_3$, δ, ppm): 1.43-1.55 (m, 2H), 1.89-2.07 (m, 4H), 2.68-2.83 (m, 2H), 2.92 (s, 3H), 2.94 (s, 3H), 2.95 - 3.07 (m, 2H), 3.13-3.22 (m, 2H), 7.62-7.70 (m, 1H)

Reference Example 55

1-Benzyl-4-cyanomethyl-4-methylaminopiperidine

The subject compound (90%) was obtained as pale yellow crystals from a known compound [Journal of Medicinal Chemistry, Vol. 42, p. 730 (1999)], (1-benzylpiperidin-4-ylidene)acetonitrile, in a manner similar to that in Reference Example 48.
$^1$H NMR ($CDCl_3$, δ, ppm): 1.30-1.49 (m, 1H), 1.57-1.79 (m, 4H), 2.31 (s, 3H), 2.34-2.57 (m, 4H), 2.47 (s, 2H), 3.51 (s, 2H), 7.19-7.38 (m, 5H)

Reference Example 56

4-(2-Aminoethyl)-1-benzyl-4-methylaminopiperidine

In a manner similar to that in Reference Example 49, the subject compound (quantitative) was obtained as a pale brown oily matter from 1-benzyl-4-cyanomethyl-4-methylaminopiperidine obtained in Reference Example 55.
$^1$H NMR ($CDCl_3$, δ, ppm) : 1.42-2.60 (m, 15H) , 2.26 (s, 3H), 3.49 (s, 2H), 7.12-7.40 (m, 5H)

Reference Example 57

9-Benzyl-1-methyl-1,3,9-triazaspiro[5.5]undecan-2-one

In a manner similar to that in Reference Example 50, the subject compound (34%) was obtained from 4-(2-aminoethyl)-1-benzyl-4-methylaminopiperidine obtained in Reference Example 56.
$^1$H NMR ($CDCl_3$, δ, ppm): 1.44-1.53 (m, 2H), 1.90-2.00 (m, 2H), 2.10-2.20 (m, 4H), 2.75-2.84 (m, 2H), 2.92 (s, 3H), 3.16-3.25 (m, 2H) , 3.52 (s, 2H) , 3.66-3.75 (m, 1H), 7.20 -7.39 (m, 5H)

Reference Example 58

1-Methyl-1,3,9-triazaspiro[5.5]undecan-2-one

In a manner similar to that in Reference Example 51, the subject compound (quantitative) was obtained as pale yellow crystals from 9-benzyl-1-methyl-1,3,9-triazaspiro[5.5]undecan-2-one obtained in Reference Example 57.
$^1$H NMR ($CDCl_3$, δ, ppm): 1.43-1.59 (m, 2H), 1.89-2.09 (m, 5H), 2.72-2.85 (m, 2H), 2.90 (s, 3H), 2.96-3.06 (m, 2H), 3.08-3.29 (m, 2H), 4.89-5.08 (m, 1H)

Reference Example 59

8-Formyl-2-(2-furyl)-5-(4-phenylpiperazin-1-yl)[1,2,4]triazolo[1,5-c]pyrimidine

The subject compound (62%) was obtained as white crystals from 8-formyl-2-(2-furyl)-5-methylthio[1,2,4]triazolo[1,5-c]pyrimidine obtained in Reference Example 47 and 1-phenylpiperazine in a manner similar to that in Reference Example 4.
$^1$H NMR ($CDCl_3$, δ, ppm): 3.36-3.48 (m, 4H), 4.56-4.81 (m, 4H), 6.60 (dd, J = 1.5, 3.5 Hz, 1H), 6.89-6.73 (m, 3H), 7.25-7.37 (m, 3H), 7.65 (d, J = 1.5 Hz, 1H), 8.54 (s, 1H), 10.36 (s, 1H)

Reference Example 60

2-(2-Furyl)-8-ethoxycarbonyl-5-(2-methoxyethylamino)[1,2,4]triazolo[1,5-c]pyrimidine

The subject compound (85%) was obtained as white crystals from a known compound (WO98/42711), 2-(2-furyl)-8-ethoxycarbonyl-5-methylthio[1,2,4]triazolo[1,5-c]pyrimidine, and 2-methoxyethylamine in a manner similar to that in Reference Example 4.
$^1$H NMR ($CDCl_3$, δ, ppm): 1.45 (t, J = 7.3 Hz, 3H), 3.42 (s, 3H), 3.67 (t, J = 5.1 Hz, 2H), 3.93 (dt, J = 5.1, 5.1 Hz, 2H), 4.47 (q, J = 7.3 Hz, 2H), 6.59 (dd, J = 1.6, 3.8 Hz, 1H), 6.96 (t, J = 5.1 Hz, 1H), 7.37 (dd, J = 0.5, 3.8 Hz, 1H), 7.62 (dd, J = 0.5, 1.6 Hz, 1H), 8.69 (s, 1H)

Reference Example 61

8-Carboxy-2-(2-furyl)-5-(2-methoxyethylamino)[1,2,4]triazolo[1,5-c]pyrimidine

In a manner similar to that in Reference Example 5, the subject compound (82%) was obtained as white crystals from 2-(2-furyl)-8-ethoxycarbonyl-5-(2-methoxyethylamino)[1,2,4]triazolo[1,5-c]pyrimidine obtained in Reference Example 60.
$^1$H NMR (DMSO-$d_6$, δ, ppm): 3.28 (s, 3H), 3.60 (t, J = 5.1 Hz, 2H), 3.77 (dt, J = 5.1, 5.1 Hz, 2H), 6.71-6.82 (m, 1H), 7.23-7.30 (m, 1H), 7.97 (s, 1H) , 8.56 (s, 1H) , 8.88 (t, J = 5.1 Hz, 1H)

Reference Example 62

2-(2-Furyl)-N-methoxy-5-(2-methoxyethylamino)-N-methyl[1,2,4]triazolo[1,5-c]pyrimidine-8-carboxamide

In a manner similar to that in Reference Example 6, the subject compound (87%) was obtained as white crystals from 8-carboxy-2-(2-furyl)-5-(2-methoxyethylamino)[1,2,4]triazolo[1,5-c]pyrimidine obtained in Reference Example 61.
$^1$H NMR ($CDCl_3$, δ, ppm): 3.40 (s, 3H), 3.42 (s, 3H), 3.66 (t, J = 5.4 Hz, 2H), 3.72 (s, 3H), 3.89 (dt, J = 5.4, 5.4 Hz, 2H), 6.58 (dd, J = 1.6, 3.5 Hz, 1H), 6.75 (t, J = 5.4 Hz, 1H), 7.28 (dd, J = 0.8, 3.5 Hz, 1H), 7.62 (dd, J = 0.8, 1.6 Hz, 1H), 8.24 (s, 1H)

Reference Example 63

8-Formyl-2-(2-furyl)-5-(2-methoxyethylamino)[1,2,4]triazolo[1,5-c]pyrimidine

In a manner similar to that in Reference Example 7, the subject compound (36%) was obtained as yellow crystals from 2-(2-furyl)-N-methoxy-5-(2-methoxyethylamino)-N-methyl[1,2,4]triazolo[1,5-c]pyrimidine-8-carboxamide obtained in Reference Example 62.
$^1$H NMR ($CDCl_3$, δ, ppm): 3.43 (s, 3H), 3.69 (t, J = 9.2 Hz, 2H), 3.97 (t, J = 9.2 Hz, 2H), 6.61 (s, 1H), 7.35 (s, 1H), 7.64 (s, 1H), 8.52 (s, 1H), 10.28 (s, 1H)

Reference Example 64

5-(3,4-Dimethoxybenzylamino)-8-(1,4-dioxa-8-azaspiro[4.5]decan-8-ylmethyl)-2-(2-furyl)[1,2,4]triazolo[1,5-c]pyrimidine

A known compound (WO98/42711), 5-(3,4-dimethoxybenzylamino)-8-formyl-2-(2-furyl) [1,2,4]triazolo[2,5-c]pyrimidine (1.0 g, 2.64 mmol), was suspended in dichloroethane (48 ml), and 1,4-dioxa-8-azaspiro[4.5]decane (1.01 ml, 7.91 mmol) was added thereto, followed by stirring at room temperature for 0.5 hour. Sodium triacetoxyborohydride (1.68 g, 7.91 mmol) was added to the reaction mixture under ice-cooling, and the mixture was stirred at room temperature for 13 hours. The reaction mixture was diluted with ethyl acetate, washed with water and saturated saline and dried over anhydrous magnesium sulfate. The solvent was distilled away under reduced pressure, and the resulting residue was purified by silica gel column chromatography (eluted with ethyl acetate/hexane = 4/1) to obtain the subject compound (1.32 g, 99%) as a pale brown oily matter.
$^1$H NMR ($CDCl_3$, δ, ppm): 1.78 (t, J = 5.7 Hz, 4H), 2.68 (t, J = 5.7 Hz, 4H), 3.88 (s, 3H), 3.88 (s, 3H), 3.88 (s, 2H), 3.94 (s, 4H), 4.75 (d, J = 5.7 Hz, 2H), 6.46 (t, J = 5.7 Hz, 1H), 6.56 (dd, J = 1.6, 3.5 Hz, 1H), 6.85 (d, J = 7.8 Hz, 1H), 6.95 (s, 1H), 6.97 (d, J = 7.8 Hz, 1H), 7.21 (dd, J = 0.8, 3.5 Hz, 1H), 7.59 (dd, J = 0.8, 1.6 Hz, 1H), 7.92 (s, 1H)

Reference Example 65

5-(3-Benzyloxypropylamino)-8-formyl-2-(2-furyl)[1,2,4]triazolo[1,5-c]pyrimidine

8-Formyl-2-(2-furyl)-5-methylthio[1,2,4]triazolo[1,5-c]pyrimidine (2.0 g, 7.68 mmol) obtained in Reference Example 47, 3-benzyloxypropylamine (1.51 g, 9.22 mmol) synthesized according to a known method and water (3.1 ml) were stirred in 1,2-dimethoxyethane (30 ml) at 85°C for 16 hours. After cooling to room temperature, the solvent was distilled away under reduced pressure, and the resulting residue was purified by silica gel column chromatography (eluted with hexane/ethyl acetate = 1/1) to obtain the subject compound (84%) as a pale brown oily matter.
$^1$H NMR ($CDCl_3$, δ, ppm) : 2.00-2.13 (m, 2H), 3.70 (t, J = 5.6 Hz, 2H), 3.75-3.97 (m, 2H), 4.58 (s, 2H), 6.58 (dd, J = 1.7, 3.5 Hz, 1H), 7.26-7.45 (m, 7H), 7.62 (d, J = 1.7 Hz, 1H), 8.53 (s, 1H), 10.28 (s, 1H)

Reference Example 66

5-(2-Benzyloxyethylamino)-8-formyl-2-(2-furyl)[1,2,4]triazolo[1,5-c]pyrimidine

The subject compound (33%) was obtained as a pale brown oily matter from 8-formyl-2-(2-furyl)-5-methylthio [1,2,4]triazolo[1,5-c]pyrimidine obtained in Reference Example 47 and O-benzylethanolamine in a manner similar to that in Reference Example 65.
$^1$H NMR ($CDCl_3$, δ , ppm) : 3.72-3.81 (m, 2H) , 3.89-4.03 (m, 2H), 4.59 (s, 2H), 6.20 (dd, J = 1.8, 3.5 Hz, 1H), 7.03-7.20 (m, 1H), 7.20-7.43 (m, 6H), 7.65 (d, J = 1.8 Hz, 1H), 8.51 (s, 1H), 10.29 (s, 1H)

Reference Example 67

5-(3,4-Dimethoxybenzylamino)-2-(2-furyl)-8-(4-axopiperidinomethyl)[1,2,4]triazolo[1,5-c]pyrimidine

5-(3,4-Dimethoxybenzylamino)-8-(1,4-dioxa-8-azaspiro[4.5]decan-8-ylmethyl)-2-(2-furyl) [1,2,4]triazolo [1,5-c]pyrimidine (1.22 g, 2.47 mmol) obtained in Reference Example 64 was dissolved in tetrahydrofuran (25 ml), and 2 mol/l hydrochloric acid (12 ml) was added thereto, followed by stirring at 100°C for 13 hours. A saturated aqueous sodium hydrogen carbonate solution was added to the reaction mixture, and the mixture was subjected to extraction with chloroform. The organic layer was washed with saturated saline and dried over anhydrous magnesium sulfate. The solvent was distilled away under reduced pressure to obtain the subject compound (1.10 g, quantitative) as a pale brown oily matter.
$^1$H NMR ($CDCl_3$, δ, ppm): 2.49 (t, J = 6.0 Hz, 4H), 2.90 (t, J = 6.0 Hz, 4H), 3.88 (s, 3H), 3.88 (s, 3H), 3.95 (s, 2H), 4.76 (d, J = 5.7 Hz, 2H), 6.49 (t, J = 5.7 Hz, 1H), 6.57 (dd, J = 1.6, 3.2 Hz, 1H), 6.85 (d, J = 7.8 Hz, 1H), 6.95 (s, 1H), 6.98 (d, J = 7.8 Hz, 1H), 7.22 (d, J = 3.2 Hz, 1H), 7.60 (d, J = 1.6 Hz, 1H), 7.97 (s, 1H)

Reference Example 68

5-Ethoxycarbonyl-2-methylthio-4-[N'-(pyridine-2-carbonyl)hydrazino]pyrimidine

The subject compound (80%) was obtained as a pale yellow powder from 4-chloro-5-ethoxycarbonyl-2-methylthiopyrimidine and pyridine-2-carboxylic acid hydrazide in a manner similar to that in Reference Example 1.
$^1$H NMR ($CDCl_3$, δ , ppm): 1.41 (t, J = 7.1 Hz, 3H), 2.47 (s, 3H), 4.40 (q, J = 7.1 Hz, 2H), 7.49 (ddd, J = 1.3, 4.8, 7.6 Hz, 1H), 7.89 (ddd, J = 1.8, 7.6, 7.9 Hz, 1H), 8.19 (ddd, J = 0.8, 1.3, 7.9 Hz, 1H), 8.62 (ddd, J = 0.8, 1.8, 4.8 Hz, 1H), 8.71 (s, 1H), 10.37 (d, J = 5.9 Hz, 1H), 10.53 (d, J = 5.9 Hz, 1H)

Reference Example 69

5-(3,4-Dimethoxybenzylamino)-8-ethoxycarbonyl-2-(2-pyridyl) [1,2,4] triazolo [1, 5-c] pyrimidine

In a manner similar to that in Reference Example 21, the subject compound (58%) was obtained as a white powder from 5-ethoxycarbonyl-2-methylthio-4-[N'-(pyridine-2-carbonyl)hydrazino]pyrimidine obtained in Reference Example 68.
$^1$H NMR ($CDCl_3$, δ, ppm) : 1.47 (t, J = 7.1 Hz, 3H), 3.89 (s, 6H), 4.49 (q, J = 7.1 Hz, 2H), 4.83 (d, J = 5.8 Hz, 2H), 6.82-6.97 (m, 3H), 7.22 (t, J = 5.8 Hz, 1H), 7.49 (ddd, J = 1.3, 4.8, 7.6 Hz, 1H), 7.90 (ddd, J = 1.8, 7.6, 7.9 Hz, 1H), 8.54 (ddd, J = 0.8, 1.3, 7.9 Hz, 1H), 8.77 (ddd, J = 0.8, 1.8, 4.8 Hz, 1H), 8.79 (s, 1H)

Reference Example 70

8-Carboxy-5-(3,4-dimethoxybenzylamino)-2-(2-pyridyl) [1,2,4]triazolo[1,5-c]pyrimidine

In a manner similar to that in Reference Example 5, the subject compound (83%) was obtained as a white powder from 5-(3,4-dimethoxybenzylamino)-8-ethoxycarbonyl-2-(2-pyridyl)[1,2,4]triazolo[1,5-c]pyrimidine obtained in Reference Example 69.
$^1$H NMR (DMSO-$d_6$, δ, ppm) : 3.72 (s, 3H), 3.75 (s, 3H), 4.74 (d, J = 5.9 Hz, 2H), 6.87-6.97 (m, 3H), 7.61 (ddd, J = 1.3, 4.8, 7.6 Hz, 1H), 8.07 (ddd, J = 1.8, 7.6, 7.9 Hz, 1H), 8.35 (ddd, J = 0.8, 1.3, 7.9 Hz, 1H), 8.59 (s, 1H), 8.78 (ddd, J = 0.8, 1.8, 4.8 Hz, 1H), 9.58 (t, J = 5.9 Hz, 1H)

Reference Example 71

5-(3,4-Dimethoxybenzylamino)-8-formyl-2-(2-pyridyl)[1,2,4]triazolo[1,5-c]pyrimidine

In a manner similar to that in Reference Example 12, the subject compound (25%) was obtained as a white powder from 8-carboxy-5-(3,4-dimethoxybenzylamino)-2-(2-pyridyl)[1,2,4]triazolo[1,5-c]pyrimidine obtained in Reference Example 70.
$^1$H NMR ($CDCl_3$, δ, ppm): 3.88 (s, 6H), 4.86 (d, J = 5.8 Hz, 2H), 6.83-7.03 (m, 4H), 7.42 (ddd, J = 1.3, 4.8, 7.6 Hz, 1H), 7.89 (ddd, J = 1.8, 7.6, 7.9 Hz, 1H), 8.35 (ddd, J = 0.8, 1.3, 7.9 Hz, 1H), 8.61 (s, 1H), 8.78 (ddd, J = 0.8, 1.8, 4.8 Hz, 1H), 10.34 (s, 1H)

Reference Example 72

5-Ethoxycarbonyl-4-(N'-isonicotinoylhydrazino)-2-methylthiopyrimidine

The subject compound (88%) was obtained as a white powder from commercially available 4-chloro-5-ethoxycarbonyl-2-methylthiopyrimidine and isonicotinic acid hydrazide in a manner similar to that in Reference Example 1.
$^1$H NMR ($CDCl_3$, δ, ppm): 1.41 (t, J = 7.1 Hz, 3H), 2.49 (s, 3H), 4.37 (q, J = 7.1 Hz, 2H), 7.70 (d, J = 6.2 Hz, 2H), 8.74 (s, 1H), 8.80 (d, J = 6.2 Hz, 2H), 9.08 (brs, 1H), 10.34 (brs, 1H)

Reference Example 73

5-(3,4-Dimethoxybenzylamino)-8-ethoxycarbonyl-2-(4-pyridyl)[1,2,4]triazolo[1,5-c]pyrimidine

In a manner similar to that in Reference Example 21, the subject compound (33%) was obtained as a white powder from 5-ethoxycarbonyl-4-(N'-isonicotinoylhydrazino)-2-methylthiopyrimidine obtained in Reference Example 72.
$^1$H NMR ($CDCl_3$, δ, ppm): 1.47 (t, J = 7.1 Hz, 3H), 3.90 (s, 6H), 4.49 (q, J = 7.1 Hz, 2H), 4.88 (d, J = 5.4 Hz, 2H), 6.86-6.99 (m, 4H), 8.18 (d, J = 6.2 Hz, 2H), 8.76 (d, J = 6.2 Hz, 2H), 8.77 (s, 1H)

Reference Example 74

5-(3,4-Dimethoxybenzylamino)-8-hydroxymethyl-2-(4-pyridyl)[1,2,4]triazolo[1,5-c]pyrimidine

In a manner similar to that in Reference Example 22, the subject compound (85%) was obtained as a yellow solid from 5-(3,4-dimethoxybenzylamino)-8-ethoxycarbonyl-2-(4-pyridyl)[1,2,4]triazolo[1,5-c]pyrimidine obtained in

Reference Example 73.
$^1H$ NMR ($CDCl_3$, δ, ppm): 3.89 (s, 6H), 4.78 (d, J = 5.4 Hz, 2H), 4.94 (s, 2H), 6.48 (t, J = 5.4 Hz, 1H), 6.85-7.00 (m, 3H), 7.94 (s, 1H), 8.09 (d, J = 6.2 Hz, 2H), 8.74 (d, J = 6.2 Hz, 2H)

Reference Example 75

5-(3,4-Dimethoxybenzylamino)-8-formyl-2-(4-pyridyl)[1,2,4]triazolo[1,5-c]pyrimidine

In a manner similar to that in Reference Example 23, the subject compound (92%) was obtained as a white powder from 5-(3,4-dimethoxybenzylamino)-8-hydroxymethyl-2-(4-pyridyl)[1,2,4]triazolo[1,5-c]pyrimidine obtained in Reference Example 74.
$^1H$ NMR ($CDCl_3$, δ, ppm): 3.89 (s, 6H), 4.91 (d, J = 5.4 Hz, 2H), 6.86-7.00 (m, 3H), 7.25 (t, J = 5.4 Hz, 1H), 8.13 (d, J = 6.2 Hz, 2H), 8.60 (s, 1H), 8.75 (d, J = 6.2 Hz, 2H), 10.29 (s, 1H)

Reference Example 76

5-Ethoxycarbonyl-4-[N'-(2-fluorobenzoyl)hydrazino]-2-methylthiopyrimidine

The subject compound (59%) was obtained as a white powder from 4-chloro-5-ethoxycarbonyl-2-methylthiopyrimidine and 2-fluorobenzoylhydrazine in a manner similar to that in Reference Example 1.
$^1H$ NMR (DMSO-$d_6$, δ, ppm): 1.34 (t, J = 7.0 Hz, 3H), 2.44 (s, 3H), 4.34 (q, J = 7.0 Hz, 2H), 7.30-7.40 (m, 2H), 7.50-7.55 (m, 1H), 7.70 (dt, J = 1.4, 7.6 Hz, 1H), 8.68 (s, 1H), 9.83 (s, 1H), 10.64 (s, 1H)

Reference Example 77

8-Ethoxycarbonyl-3-(2-fluorophenyl)-5-methylthio[1,2,4] triazolo [4,3-c] pyrimidine

In a manner similar to that in Reference Example 2, the subject compound (86%) was obtained as a white powder from 5-ethoxycarbonyl-4-[N'-(2-fluorobenzoyl)hydrazino]-2-methylthiopyrimidine obtained in Reference Example 76.
$^1H$ NMR ($CDCl_3$, δ, ppm): 1.48 (t, J = 7.0 Hz, 3H), 2.60 (s, 3H), 4.55 (q, J = 7.0 Hz, 2H), 7.05-7.35 (m, 2H), 7.56-7.67 (m, 2H), 8.52 (s, 1H)

Reference Example 78

5-(3,4-Dimethoxybenzylamino)-8-ethoxycarbonyl-2-(2-fluorophenyl) [1,2,4]triazolo[1,5-c]pyrimidine

In a manner similar to that in Reference Example 26, the subject compound (58%) was obtained as a white powder from 8-ethoxycarbonyl-3-(2-fluorophenyl)-5-methylthio[1,2,4]triazolo[4,3-c]pyrimidine obtained in Reference Example 77.
$^1H$ NMR ($CDCl_3$, δ, ppm): 1.46 (t, J = 7.0 Hz, 3H), 3.88 (s, 6H), 4.48 (q, J = 7.0 Hz, 2H), 4.86 (d, J = 5.4 Hz, 2H), 6.84-7.02 (m, 4H), 7.16-7.30 (m, 2H), 7.42-7.47 (m, 1H), 8.36 (dt, J = 1.4, 7.6 Hz, 1H), 8.75 (s, 1H)

Reference Example 79

5-(3,4-Dimethoxybenzylamino)-2-(2-fluorophenyl)-8-hydroxymethyl[1,2,4]triazolo[1,5-c]pyrimidine

In a manner similar to that in Reference Example 22, the subject compound (quantitative) was obtained as a white amorphous matter from 5-(3,4-dimethoxybenzylamino)-8-ethoxycarbonyl-2-(2-fluorophenyl)[1,2,4]triazolo[1,5-c]pyrimidine obtained in Reference Example 78.
$^1H$ NMR ($CDCl_3$, δ, ppm): 3.88 (s, 6H), 4.77 (d, J = 5.4 Hz, 2H), 4.93 (s, 2H), 6.55 (t, J = 5.4 Hz, 1H), 6.82-6.98 (m, 3H), 7.16-7.32 (m, 2H), 7.41-7.45 (m, 1H), 8.20 (dt, J = 1.4, 7.6 Hz, 1H)

Reference Example 80

5-(3,4-Dimethoxybenzylamino)-2-(2-fluorophenyl)-8-formyl [1,2,4]triazolo[1,5-c]pyrimidine

In a manner similar to that in Reference Example 23, the subject compound (92%) was obtained as a white

powder from 5-(3,4-dimethoxybenzylamino)-2-(2-fluorophenyl)-8-hydroxymethyl[1,2,4]triazolo[1,5-c]pyrimidine obtained in Reference Example 79.
$^1$H NMR ($CDCl_3$, δ, ppm): 3.89 (s, 6H), 4.89 (d, J = 5.4 Hz, 2H), 6.84-6.99 (m, 3H), 7.11-7.32 (m, 3H), 7.44-7.52 (m, 1H), 8.32 (dt, J = 1.4, 7.6 Hz, 1H), 8.59 (s, 1H), 10.32 (s, 1H)

Reference Example 81

5-Ethoxycarbonyl-4-[N'-(2-methoxybenzoyl)hydrazino]-2-methylthiopyrimidine

The subject compound (87%) was obtained as a white powder from 4-chloro-5-ethoxycarbonyl-2-methylthiopyrimidine and 2-methoxybenzoylhydrazine in a manner similar to that in Reference Example 1.
$^1$H NMR (DMSO-$d_6$, δ, ppm): 1.34 (t, J = 7.0 Hz, 3H), 2.46 (s, 3H), 3.95 (s, 3H), 4.35 (q, J = 7.0 Hz, 2H), 7.10 (t, J = 7.6 Hz, 1H), 7.22 (d, J = 8.6 Hz, 1H), 7.56 (ddd, J = 1.7, 7.6, 8.6 Hz, 1H), 7.83 (dd, J = 1.7, 7.6 Hz, 1H), 8.67 (s, 1H), 10.11 (d, J = 3.6 Hz, 1H), 10.57 (d, J = 3.6 Hz, 1H)

Reference Example 82

8-Ethoxycarbonyl-3-(2-methoxyphenyl)-5-methylthio[1,2,4]triazolo[4,3-c]pyrimidine

In a manner similar to that in Reference Example 2, the subject compound (88%) was obtained as a white powder from 5-ethoxycarbonyl-4-[N'-(2-methoxybenzoyl)hydrazino]-2-methylthiopyrimidine obtained in Reference Example 81.
$^1$H NMR ($CDCl_3$, δ, ppm): 1.48 (t, J = 7.0 Hz, 3H), 2.55 (s, 3H), 3.72 (s, 3H), 4.55 (q, J = 7.0 Hz, 2H), 6.98 (t, J = 8.6 Hz, 1H), 7.09 (d, J = 7.6 Hz, 1H), 7.47 (dd, J = 1.7, 7.6 Hz, 1H), 7.56 (ddd, J = 1.7, 7.6, 8.6 Hz, 1H), 8.49 (s, 1H)

Reference Example 83

5-(3,4-Dimethoxybenzylamino)-8-ethoxycarbonyl-2-(2-methoxyphenyl) [1,2,4]triazolo[1,5-c]pyrimidine

In a manner similar to that in Reference Example 26, the subject compound (95%) was obtained as a white powder from 8-ethoxycarbonyl-3-(2-methoxyphenyl)-5-methylthio[1,2,4]triazolo[4,3-c]pyrimidine obtained in Reference Example 82.
$^1$H NMR ($CDCl_3$, δ, ppm): 1.46 (t, J = 7.0 Hz, 3H), 3.87 (s, 6H), 3.92 (s, 3H), 4.47 (q, J = 7.0 Hz, 2H), 4.81 (d, J = 5.4 Hz, 2H), 6.80-6.94 (m, 4H), 7.03 (t, J = 8.6 Hz, 1H), 7.10 (d, J = 7.6 Hz, 1H), 7.45 (ddd, J = 1.7, 7.6, 8.6 Hz, 1H), 8.20 (dd, J = 1.7, 7.6 Hz, 1H), 8.73 (s, 1H)

Reference Example 84

5-(3,4-Dimethoxybenzylamino)-8-hydroxymethyl-2-(2-methoxyphenyl)[1,2,4]triazolo[1,5-c]pyrimidine

In a manner similar to that in Reference Example 22, the subject compound (47%) was obtained as a white powder from 5-(3,4-dimethoxybenzylamino)-8-ethoxycarbonyl-2-(2-methoxyphenyl)[1,2,4]triazolo[1,5-c]pyrimidine obtained in Reference Example 83.
$^1$H NMR ($CDCl_3$, δ, ppm): 3.86 (s, 3H), 3.88 (s, 6H) , 4.71 (d, J = 5.4 Hz, 2H), 4.91 (s, 2H), 6.66 (t, J = 5.4 Hz, 1H), 6.78-6.93 (m, 3H), 7.00-7.10 (m, 2H), 7.44 (ddd, J = 1.7, 7.6, 8.6 Hz, 1H), 7.87 (s, 1H), 7.98 (dd, J = 1.7, 7.6 Hz, 1H)

Reference Example 85

5-(3,4-Dimethoxybenzylamino)-8-formyl-2-(2-methoxyphenyl)[1,2,4]triazolo[1,5-c]pyrimidine

In a manner similar to that in Reference Example 23, the subject compound (91%) was obtained as a white powder from 5-(3,4-dimethoxybenzylamino)-8-hydroxymethyl-2-(2-methoxyphenyl)[1,2,4]triazolo[1,5-c]pyrimidine obtained in Reference Example 84.
$^1$H NMR ($CDCl_3$, δ, ppm): 3.86 (s, 6H), 3.90 (s, 3H), 4.80 (d, J = 5.4 Hz, 2H) , 6.76-6.88 (m, 3H) , 7.02-7.03 (m, 2H) , 7.47-7.50 (m, 2H), 8.20 (dd, J = 1.7, 7.6 Hz, 1H), 8.57 (s, 1H), 10.34 (s, 1H)

Reference Example 86

5-(3,4-Dimethoxybenzylamino)-8-(1,3-dithian-2-ylidenemethyl) -2- (2-furyl) [1,2,4]triazolo[1,5-c]pyrimidine

To a solution of 2-(trimethysilyl)-1,3-dithiane (0.52 ml, 2.7 mmol) in THF (1.8 ml) was added n-butyl lithium (1.59 ml, 1.59 mol/l hexane solution, 2.53 mmol) slowly under ice-cooling, and the mixture was stirred for about 0.3 hour under ice-cooling, followed by cooling to -78°C. A known compound (WO98/42711), 5-(3,4-dimethoxybenzylamino)-8-formyl-2-(2-furyl)[1,2,4]triazolo[1,5-c]pyrimidine (424 mg, 1.05 mmol), was suspended in THF (2.7 ml), and the suspension was gradually added to the reaction mixture at -78°C, followed by stirring at -78°C for 3 hours. Water was added to the reaction mixture, which was then subjected to extraction with chloroform. The organic layer was washed with water and saturated saline and dried over anhydrous magnesium sulfate. The solvent was distilled away under reduced pressure, and the resulting residue was purified by silica gel column chromatography (eluted with ethyl acetate/hexane = 4/1) to obtain the subject compound (352.8 mg, 65%) as white crystals.
$^1$H NMR ($CDCl_3$, δ, ppm): 2.18-2.29 (m, 2H), 2.93-3.06 (m, 4H), 3.88 (s, 3H), 3.88 (s, 3H), 4.77 (d, J = 5.9 Hz, 2H), 6.46 (t, J = 5.9 Hz, 1H), 6.56 (dd, J = 1.6, 3.2 Hz, 1H), 6.84 (d, J = 8.6 Hz, 1H), 6.94 (s, 1H), 6.96 (d, J = 8.6 Hz, 1H), 7.21 (s, 1H), 7.22 (d, J = 3.2 Hz, 1H), 7.59 (d, J = 1.6 Hz, 1H), 8.31 (s, 1H)

Reference Example 87

5-(3,4-Dimethoxybenzylamino)-2-(2-furyl)-8-(methoxycarbonylmethyl) [1,2,4]triazolo[1,5-c]pyrimidine

5-(3,4-Dimethoxybenzylamino)-8-(1,3-dithian-2-ylidenemethyl) -2- (2-furyl) [1,2,4] triazolo [1, 5-c] pyrimidine (352.8 mg, 0.73 mmol) obtained in Reference Example 86 was dissolved in a mixed solvent of methanol (24.4 ml) and water (2.8 ml). Mercuric chloride (441.2 mg, 1.61 mmol) was added thereto, and the mixture was refluxed for about 3 hours. After filtration of the reaction mixture, the solvent was distilled away under reduced pressure. The resulting residue was diluted with chloroform, washed with a saturated aqueous sodium bicarbonate solution and saturated saline and then dried over anhydrous magnesium sulfate. The solvent was distilled away under reduced pressure to obtain the subject compound (305.4 mg, 98%) as a white solid.
$^1$H NMR ($CDCl_3$, δ, ppm): 3.74 (s, 3H), 3.88 (s, 3H), 3.88 (s, 3H), 3.89 (s, 2H), 4.75 (d, J = 5.9 Hz, 2H) , 6.42 (t, J = 5.9 Hz, 1H), 6.56 (dd, J = 1.9, 3.5 Hz, 1H), 6.85 (d, J = 7.8 Hz, 1H), 6.95 (s, 1H), 6.96 (d, J = 7.8 Hz, 1H), 7.20 (dd, J = 0.8, 3.5 Hz, 1H), 7.59 (dd, J = 0.8, 1.9 Hz, 1H), 7.84 (s, 1H)

Reference Example 88

5-(3,4-Dimethoxybenzylamino)-2-(2-furyl)-8-(2-hydroxyethyl)[1,2,4]triazolo[1,5-c]pyrimidine

In a manner similar to that in Reference Example 22, the subject compound (84%) was obtained as a white solid from 5-(3,4-dimethoxybenzylamino)-2-(2-furyl)-8-(methoxycarbonylmethyl)[1,2,4]triazolo[1,5-c]pyrimidine obtained in Reference Example 87.
$^1$H NMR ($CDCl_3$, δ, ppm): 3.09 (t, J = 5.1 Hz, 2H), 3.88 (s, 3H), 3.88 (s, 3H), 3.93-4.05 (m, 2H), 4.73 (d, J = 5.7 Hz, 2H), 6.41 (t, J = 5.7 Hz, 1H), 6.56 (dd, J = 1.6, 3.2 Hz, 1H), 6.84 (d, J = 8.1 Hz, 1H), 6.94 (s, 1H), 6.95 (d, J = 8.1 Hz, 1H), 7.19 (dd, J = 0.5, 3.2 Hz, 1H), 7.59 (dd, J = 0.5, 1.6 Hz, 1H), 7.77 (s, 1H)

Reference Example 89

5-(3,4-Dimethoxybenzylamino)-8-formylmethyl-2-(2-furyl)[1,2,4]triazolo[1,5-c]pyrimidine

5-(3,4-Dimethoxybenzylamino)-2-(2-furyl)-8-(2-hydroxyethyl)[1,2,4]triazolo[1,5-c]pyrimidine (100.6 mg, 0.253 mmol) obtained in Reference Example 88 was dissolved in dichloromethane (2.5 ml), and 1,1,1-triacetoxy-1,1-dihydro-1,2-benziodoxol-3(1H)-one (432.4 mg, 1.01 mmol) was added thereto, followed by stirring at room temperature for 0.5 hour. After the completion of reaction, the reaction mixture was poured into a mixed solution of a saturated aqueous sodium bicarbonate solution and a saturated aqueous sodium thiosulfate solution, followed by extraction with ethyl acetate. The organic layer was washed with saturated saline and then dried over anhydrous magnesium sulfate. The solvent was distilled away under reduced pressure to obtain the subject compound (99.2 mg, 99%) as a pale brown oily matter.
$^1$H NMR ($CDCl_3$, δ, ppm): 3.88 (s, 3H), 3.88 (s, 3H), 3.94-3.96 (m, 2H), 4.75 (d, J = 5.9 Hz, 2H), 6.47 (t, J = 5.9 Hz, 1H), 6.56 (dd, J = 1.1, 3.5 Hz, 1H), 6.85 (d, J = 8.1 Hz, 1H), 6.94 (s, 1H), 6.96 (d, J = 8.1 Hz, 1H), 7.19 (d, J = 3.5 Hz, 1H), 7.59 (d, J = 1.1 Hz, 1H), 7.79 (s, 1H), 9.87-9.92 (m, 1H)

Reference Example 90

5-[N-(tert-Butoxycarbonyl)-N-(3,4-dimethoxybenzyl)amino]-8-formyl-2-(2-furyl)[1,2,4]triazolo[1,5-c]pyrimidine

A known compound (WO98/42711), 5-(3,4-dimethoxybenzylamino)-8-formyl-2-(2-furyl)[1,2,4]triazolo[1,5-c] pyrimidine (9.04 g, 23.9 mmol), was dissolved in dichloromethane (96 ml). To the solution were added triethylamine (7.23 g, 71.5 mmol), di(tert-butyl)dicarbonate (10.4 g, 47.7 mmol) and dimethylaminopyridine (2.91 g, 23.8 mmol) under ice-cooling, and the mixture was stirred at room temperature for 2 hours. The reaction mixture was diluted with ethyl acetate, washed with 10% hydrochloric acid, a saturated aqueous sodium bicarbonate solution and saturated saline and then dried over anhydrous magnesium sulfate. The solvent was distilled away under reduced pressure, and the resulting residue was purified by silica gel column chromatography (eluted with ethyl acetate/hexane = 1/1) to obtain the subject compound (10.9 g, 95%) as white crystals.
$^{1}$H NMR ($CDCl_3$, δ, ppm): 1.39 (s, 9H), 3.80 (s, 3H), 3.81 (s, 3H), 5.22 (s, 2H), 6.64 (dd, J = 1.8, 3.5 Hz, 1H), 6.73 (d, J = 8.1 Hz, 1H), 6.92 (dd, J = 2.0, 8.1 Hz, 1H), 7.08 (d, J = 2.0 Hz, 1H), 7.34 (d, J = 3.5 Hz, 1H), 7.69 (d, J = 1.8 Hz, 1H), 8.67 (s, 1H), 10.57 (s, 1H)

Reference Example 91

5-[N-(tert-Butoxycarbonyl)-N-(3,4-dimethoxybenzyl)amino]-8-(2-ethoxycarbonylethen-1-yl)-2-(2-furyl) [1,2,4]triazolo [1,5-c]pyrimidine

To THF (250 ml) was added 60% sodium hydride (6.54 g, 164 mmol) slowly under ice-cooling, and after the completion of heat generation, ethyl diethylphosphonoacetate (36.7 g, 164 mmol) was gradually added dropwise thereto, followed by stirring under ice-cooling for 0.5 hour. 5-[N-(tert-Butoxycarbonyl)-N-(3,4-dimethoxybenzyl)amino]-8-formyl-2-(2-furyl)[1,2,4]triazolo[1,5-c]pyrimidine (26.1 g, 54.5 mmol) obtained in Reference Example 90 was suspended in THF (295 ml), and the suspension was gradually added to the reaction mixture under ice-cooling, followed by stirring at room temperature for 2 hours. Water was added to the reaction mixture under ice-cooling, and the mixture was subjected to extraction with ethyl acetate. Thereafter, the organic layer was washed with water and saturated saline and dried over anhydrous magnesium sulfate. The solvent was distilled away under reduced pressure, and the resulting residue was suspended in ethyl acetate and re-slurried with diisopropyl ether. The obtained crystals were recovered by filtration and dried to obtain the subject compound (21.5 g, 72%) as white crystals.
Melting point: 124-129°C
$^{1}$H NMR ($CDCl_3$, δ, ppm): 1.36 (s, 9H), 1.37 (t, J = 7.1 Hz, 3H), 3.77 (s, 3H), 3.78 (s, 3H), 4.31 (q, J = 7.1 Hz, 2H), 5.16 (s, 2H), 6.61 (dd, J = 1.8, 3.6 Hz, 1H), 6.70 (d, J = 8.1 Hz, 1H), 6.87 (dd, J = 2.0, 8.1 Hz, 1H), 7.09 (d, J = 2.0 Hz, 1H), 7.31 (d, J = 3.6 Hz, 1H), 7.60 (d, J = 15.8 Hz, 1H), 7.66 (d, J = 1.8 Hz, 1H), 7.72 (d, J = 15.8 Hz, 1H), 8.17 (s, 1H)

Reference Example 92

5-[N-(tert-Butoxycarbonyl)-N-(3,4-dimethoxybenzyl)amino]-8-(2-ethoxycarbonylethyl)-2-(2-furyl)[1,2,4]triazolo[1,5-c] pyrimidine

5-[N-(tert-Butoxycarbonyl)-N-(3,4-dimethoxybenzyl)amino]-8-(2-ethoxycarbonylethen-1-yl)-2-(2-furyl)[1,2,4] triazolo[1,5-c]pyrimidine obtained in Reference Example 91 was dissolved in ethanol (203 ml), and 10% palladium-carbon (5.75 g) was added thereto in a stream of argon, followed by stirring at 30°C for one hour with hydrogenation. Palladium-carbon was removed by filtration through Celite, and the filter cake was washed with methanol. The obtained filtrate and washings were combined, and the solvent was distilled away under reduced pressure. The resulting residue was purified by silica gel column chromatography (eluted with ethyl acetate/hexane = 1/1) to obtain the subject compound (9.44 g, 82%) as white crystals.
Melting point: 100-103°C
$^{1}$H NMR ($CDCl_3$, δ, ppm): 1.27 (t, J = 7.3 Hz, 3H), 1.36 (s, 9H), 2.87 (t, J = 7.4 Hz, 2H), 3.27 (t, J = 7.4 Hz, 2H), 3.78 (s, 3H), 3.80 (s, 3H), 4.12 (q, J = 7.3 Hz, 2H), 5.12 (s, 2H), 6.60 (dd, J = 1.8, 3.4 Hz, 1H), 6.71 (d, J = 8.3 Hz, 1H), 6.87 (dd, J = 2.0, 8.3 Hz, 1H), 7.12 (d, J = 2.0 Hz, 1H), 7.22 (dd, J = 1.0, 3.4 Hz, 1H), 7.65 (dd, J = 1.0, 1.8 Hz, 1H), 7.96 (s, 1H)

Reference Example 93

5- [N- (tert-Butoxycarbonyl) -N- (3,4-dimethoxybenzyl)amino]-2-(2-furyl)-8-(3-hydroxypropyl)[1,2,4]triazolo[1,5-c]r pyrimidine

To ice-cooled THF (18 ml) were successively added lithium aluminum hydride (69.0 mg, 1.80 mmol) and 5-[N-(tert-butoxycarbonyl)-N-(3,4-dimethoxybenzyl)amino]-8-(2-ethoxycarbonylethyl)-2-(2-furyl)[1,2,4]triazolo[1,5-c] pyrimidine (1.00 g, 1.80 mmol) obtained in Reference Example 92 gradually, and the mixture was stirred in a stream of argon for one hour under ice-cooling. Diethyl ether (18 ml) and a saturated aqueous sodium sulfate solution (1.4 ml) were slowly added thereto under ice cooling, and the mixture was stirred at room temperature for one hour, followed by liquid separation. After the organic layer was dried over anhydrous sodium sulfate, the solvent was distilled away under reduced pressure, and the resulting residue was purified by silica gel column chromatography (eluted with ethyl acetate/hexane = 4/1) to obtain the subject compound (871 mg, 94%) as white crystals.
$^1$H NMR ($CDCl_3$, δ, ppm): 1.36 (s, 9H), 2.01 (t, J = 6.7 Hz, 2H), 3.09 (t, J = 6.7 Hz, 2H), 3.32-3.35 (m, 1H), 3.57-3.59 (m, 2H), 3.79 (s, 3H), 3.80 (s, 3H), 5.13 (s, 2H), 6.59 (dd, J = 1.7, 3.3 Hz, 1H), 6.71 (d, J = 8.3 Hz, 1H), 6.87 (dd, J = 2.0, 8.3 Hz, 1H), 7.11 (d, J = 2.0 Hz, 1H), 7.22 (d, J = 3.3 Hz, 1H), 7.64 (d, J = 1.7 Hz, 1H), 7.95 (s, 1H)

Reference Example 94

5-[N-(tert-Butoxycarbonyl)-N-(3,4-dimethoxybenzyl)amino]-8-(2-formylethyl)-2-(2-furyl)[1,2,4]triazolo[1,5-c] pyrimidine

5-[N-(tert-Butoxycarbonyl)-N-(3,4-dimethoxybenzyl)amino]-2-(2-furyl)-8-(3-hydroxypropyl)[1,2,4]triazolo [1,5-c] pyrimidine (3.07 g, 6.00 mmol) obtained in Reference Example 93 was dissolved in dichloromethane (60 ml), and PCC (2.60 g, 12.0 mmol) and 3A Molecular Sieves (6.02 g) were added thereto, followed by stirring at room temperature for one hour. After filtration of the reaction mixture, the solvent was distilled away from the filtrate under reduced pressure, and the resulting residue was purified by silica gel column chromatography (eluted with ethyl acetate/hexane = 4/1) to obtain the subject compound (2.43 g, 80%) as white crystals.
$^1$H NMR ($CDCl_3$, δ , ppm): 1.36 (s, 9H), 3.06 (t, J = 7.0 Hz, 2H), 3.25 (t, J = 7.0 Hz, 2H), 3.77 (s, 3H), 3.79 (s, 3H), 5.11 (s, 2H), 6.59 (dd, J = 1.6, 3.2 Hz, 1H), 6.70 (d, J = 8.4 Hz, 1H), 6.86 (dd, J = 2.0, 8.4 Hz, 1H), 7.10 (d, J = 2.2 Hz, 1H), 7.20 (d, J = 3.2 Hz, 1H), 7.64 (d, J = 1.6 Hz, 1H), 7.96 (s, 1H), 9.84 (s, 1H)

Reference Example 95

5-(3,4-Dimethoxybenzylamino)-8-(2-formylethyl)-2-(2-furyl) [1,2,4]triazolo[1,5-c] pyrimidne

5-[N-(tert-Butoxycarbonyl)-N-(3,4-dimethoxybenzyl)amino]-8-(2-formylethyl)-2-(2-furyl)[1,2,4]triazolo[1,5-c] pyrimidine (920 mg, 1.81 mmol) obtained in Reference Example 94 was stirred in trifluoroacetic acid (10.2 ml) at room temperature for 2 hours. The reaction mixture was poured into water. After the deposited crystals were recovered by filtration, they were dried to obtain the subject compound (620 mg, 84%) as white crystals.
$^1$H NMR ($CDCl_3$, δ, ppm): 3.01 (t, J = 7.3 Hz, 2H), 3.17 (t, J = 7.3 Hz, 2H), 3.88 (s, 3H), 3.88 (s, 3H), 4.74 (d, J = 5.4 Hz, 2H), 6.36 (t, J = 5.4 Hz, 1H), 6.57 (dd, J = 1.9, 3.5 Hz, 1H), 6.85 (d, J = 7.6 Hz, 1H), 6.95 (s, 1H), 6.98 (d, J = 7.6 Hz, 1H), 7.21 (dd, J = 0.8, 3.5 Hz, 1H), 7.61 (dd, J = 0.8, 1.9 Hz, 1H), 7.80 (s, 1H), 9.89 (t, J = 1.4 Hz, 1H)

Example 1

5-(3,4-Dimethoxybenzylamino)-2-(2-furyl)-8-(4-phenylpiperazin-1-ylmethyl)[1,2,4]triazolo[1,5-c]pyrimidine (Compound 1)

A known compound (WO98/42711), 5-(3,4-dimethoxybenzylamino)-8-formyl-2-(2-furyl)[1,2,4]triazolo[1,5-c] pyrimidine (3.81 g, 10.0 mmol), was suspended in dichloroethane (182 ml), and 1-phenylpiperazine (1.71 g, 10.5 mmol) was added thereto, followed by stirring at room temperature for 0.5 hour. Sodium triacetoxyborohydride (6.38 g, 30.1 mmol) was added to the reaction mixture under ice-cooling, followed by stirring at room temperature for 2 hours. The reaction mixture was diluted with ethyl acetate, then washed with water and saturated saline and dried over anhydrous magnesium sulfate. The solvent was distilled away under reduced pressure, and the resulting residue was purified by silica gel column chromatography (eluted with ethyl acetate/hexane = 4/1) to obtain the subject compound (4.78 g, 91%) as a pale brown oily matter.
$^1$H NMR ($CDCl_3$, δ, ppm): 2.76 (t, J = 4.8 Hz, 4H), 3.23 (t, J = 4.8 Hz, 4H), 3.88 (s, 3H), 3.88 (s, 3H), 3.91 (s, 2H), 4.76

(d, J = 5.6 Hz, 2H), 6.42 (t, J = 5.6 Hz, 1H), 6.57 (dd, J = 1.8, 3.5 Hz, 1H), 6.81-6.99 (m, 8H), 7.22 (d, J = 3.5 Hz, 1H), 7.60 (d, J = 1.8 Hz, 1H), 7.97 (s, 1H)

Example 2

5-Amino-2-(2-furyl)-8-(4-phenylpiperazin-1-ylmethyl) [1,2,4]triazolo[1,5-c]pyrimidine (Compound 2)

5-(3,4-Dimethoxybenzylamino)-2-(2-furyl)-8-(4-phenylpiperazin-1-ylmethyl)[1,2,4]triazolo[1,5-c]pyrimidine (4.78 g, 9.1 mmol) obtained in Example 1 was stirred in trifluoroacetic acid (26 ml) in the presence of anisole (4.95 ml, 45.5 mmol) and trifluoromethanesulfonic acid (4.03 ml, 45.5 mmol) at 50°C for one hour. After cooling to room temperature, the reaction mixture was poured into water (50 ml), and the deposited crystals were recovered by filtration. The crystals were purified by silica gel column chromatography (eluted with chloroform/methanol = 10/1) to obtain the subject compound (2.70 g, 79%) as white crystals.
Melting point: 185-187°C
$^1$H NMR ($CDCl_3$, δ, ppm): 2.76 (t, J = 4.9 Hz, 4H), 3.23 (t, J = 4.9 Hz, 4H), 3.91 (s, 2H), 5.87 (s, 2H), 6.59 (dd, J = 1.8, 3.5 Hz, 1H), 6.84 (dd, J = 7.3, 7.3 Hz, 1H), 6.92 (d, J = 7.3 Hz, 2H), 7.25 (dd, J = 7.3, 7.3 Hz, 2H), 7.26 (dd, J = 0.8, 3.5 Hz, 1H), 7.63 (dd, J = 0.8, 1.8 Hz, 1H), 7.92 (s, 1H)

Example 3

5-(3,4-Dimethoxybenzylamino)-2-(2-furyl)-8-[4-(2-methoxyphenyl)piperazin-1-ylmethyl][1,2,4]triazolo[1,5-c]pyrimidine (Compound 3)

The subject compound (98%) was obtained as a pale brown oily matter from 5-(3,4-dimethoxybenzylamino)-8-formyl-2-(2-furyl)[1,2,4]triazolo[1,5-c]pyrimidine and 1-(2-methoxyphenyl)piperazine in a manner similar to that in Example 1.
$^1$H NMR ($CDCl_3$, δ, ppm): 2.80-2.81 (m, 4H), 3.11-3.13 (m, 4H), 3.84 (s, 3H), 3.89 (s, 3H), 3.89 (s, 3H), 3.95 (s, 2H), 4.76 (d, J = 5.6 Hz, 2H), 6.42 (t, J = 5.6 Hz, 1H), 6.57 (dd, J = 1.8, 3.5 Hz, 1H), 6.85-6.99 (m, 7H), 7.22 (d, J = 3.5 Hz, 1H), 7.60 (d, J = 1.8 Hz, 1H), 7.96 (s, 1H)

Example 4

5-Amino-2-(2-furyl)-8-[4-(2-methoxyphenyl)piperazin-1-ylmethyl][1,2,4]triazolo[1,5-c]pyrimidine (Compound 4)

The subject compound (65%) was obtained as white crystals from 5-(3,4-dimethoxybenzylamino)-2-(2-furyl)-8-[4-(2-methoxyphenyl)piperazin-1-ylmethyl][1,2,4]triazolo[1,5-c]pyrimidine obtained in Example 3 in a manner similar to that in Example 2.
Melting point: 220-221°C
$^1$H NMR ($CDCl_3$, δ, ppm): 2.81 (t, J = 4.9 Hz, 4H), 3.12 (t, J = 4.9 Hz, 4H), 3.85 (s, 3H), 3.94 (s, 2H), 5.83 (s, 2H), 6.59 (dd, J = 1.8, 3.5 Hz, 1H), 6.83-7.01 (m, 4H), 7.25 (d, J = 3.5 Hz, 1H), 7.63 (d, J = 1.8 Hz, 1H), 7.91 (s, 1H)

Example 5

5-(3,4-Dimethoxybenzylamino)-8-[4-(4-fluorophenyl)piperazin-1-ylmethyl]-2-(2-furyl) [1,2,4]triazolo[1,5-c]pyrimidine (Compound 5)

The subject compound (quantitative) was obtained as a pale brown oily matter from 5-(3,4-dimethoxybenzylamino)-8-formyl-2-(2-furyl)[1,2,4]triazolo[1,5-c]pyrimidine and 1-(4-fluorophenyl)piperazine in a manner similar to that in Example 1.
$^1$H NMR ($CDCl_3$, δ , ppm): 2.77 (t, J = 4.9 Hz, 4H), 3.15 (t, J = 4.9 Hz, 4H), 3.88 (s, 3H), 3.88 (s, 3H), 3.91 (s, 2H), 4.76 (d, J = 5.6 Hz, 2H), 6.42 (t, J = 5.6 Hz, 1H), 6.57 (dd, J = 1.8, 3.5 Hz, 1H), 6.83-6.99 (m, 7H), 7.22 (d, J = 3.5 Hz, 1H), 7.60 (d, J = 1.8 Hz, 1H), 7.96 (s, 1H)

Example 6

5-Amino-8-[4-(4-fluorophenyl)piperazin-1-ylmethyl]-2-(2-furyl)[1,2,4]triazolo[1,5-c]pyrimidine (Compound 6)

In a manner similar to that in Example 2, the subject compound (52%) was obtained as white crystals from

5-(3,4-dimethoxybenzylamino-8-[4-(4-fluorophenyl)piperazin-1-ylmethyl]-2-(2-furyl) [1,2,4]triazolo[1,5-c]pyrimidine obtained in Example 5.
Melting point: 256-260°C
$^1$H NMR ($CDCl_3$, δ, ppm) : 2.76 (t, J = 5.0 Hz, 4H), 3.15 (t, J = 5.0 Hz, 4H), 3.91 (s, 2H), 5.87 (s, 2H), 6.59 (dd, J = 1.8, 3.5 Hz, 1H), 6.85 (dd, J = 8.1, 8.1 Hz, 2H), 6.92 (dd, J = 8.1, 8.1 Hz, 2H), 7.25 (d, J = 3.5 Hz, 1H), 7.63 (d, J = 1.8 Hz, 1H), 7.91 (s, 1H)

Example 7

5-(3,4-Dimethoxybenzylamino)-2-(2-furyl)-8-[4-(2-pyridyl)piperazin-1-ylmethyl][1,2,4]triazolo[1,5-c]pyrimidine (Compound 7)

The subject compound (76%) was obtained as a pale brown oily matter from 5-(3,4-dimethoxybenzylamino)-8-formyl-2-(2-furyl)[1,2,4]triazolo[1,5-c]pyrimidine and 1-(2-pyridyl)piperazine in a manner similar to that in Example 1.
$^1$H NMR ($CDCl_3$, δ, ppm): 2.71 (t, J = 4.9 Hz, 4H) , 3.58 (t, J = 4.9 Hz, 4H), 3.88 (s, 3H), 3.88 (s, 3H), 3.90 (s, 2H), 4.76 (d, J = 5.7 Hz, 2H), 6.41 (t, J = 5.7 Hz, 2H), 6.57 (dd, J = 1.8, 3.5 Hz, 1H), 6.60-6.64 (m, 2H), 6.85 (d, J = 5.8 Hz, 1H), 6.95 (s, 1H), 6.97 (d, J = 5.8 Hz, 1H), 7.21 (d, J = 3.5 Hz, 1H), 7.42-7.49 (m, 1H), 7.59 (d, J = 1.8 Hz, 1H) , 8.17-8.18 (m, 1H)

Example 8

5-Amino-2-(2-furyl)-8-[4-(2-pyridyl)piperazin-1-ylmethyl][1,2,4]triazalo[1,5-c]pyrimidine (Compound 8)

In a manner similar to that in Example 2, the subject compound (80%) was obtained as white crystals from 5-(3,4-dimethoxybenzylamino)-2-(2-furyl)-8-[4-(2-pyridyl)piperazin-1-ylmethyl][1,2,4]triazolo[1,5-c]pyrimidine obtained in Example 7.
Melting point: 257-258°C
$^1$H NMR (DMSO-$d_6$, δ , ppm) : 2.54-2.56 (m, 4H) , 3.46-3.48 (m, 4H), 3.71 (s, 2H), 6.61 (dd, J = 5.3, 8.2 Hz, 1H), 6.71 (dd, J = 1.7, 3.3 Hz, 1H), 6.78-6.80 (m, 1H), 7.21 (d, J = 3.3 Hz, 1H), 7.50 (dd, J = 5.3, 8.2 Hz, 1H), 7.82 (d, J = 1.7 Hz, 1H), 7.87 (s, 2H), 7.93 (s, 1H), 8.07-8.09 (m, 1H)

Example 9

5-(3,4-Dimethoxybenzylamino)-2-(2-furyl)-8-[4-(4-methoxyphenyl)piperazin-1-ylmethyl] [1,2,4]triazolo[1,5-c] pyrimidine (Compound 9)

The subject compound (53%) was obtained as a pale brown oily matter from 5-(3,4-dimethoxybenzylamino)-8-formyl-2-(2-furyl)[1,2,4]triazolo[1,5-c]pyrimidine and 1-(4-methoxyphenyl)piperazine in a manner similar to that in Example 1.
$^1$H NMR ($CDCl_3$, δ, ppm): 2.77 (t, J = 4.8 Hz, 4H), 3.12 (t, J = 4.8 Hz, 4H), 3.72 (s, 3H), 3.86 (s, 3H), 3.87 (s, 3H), 3.87 (s, 2H), 4.73 (d, J = 5.6 Hz, 2H), 6.51 (t, J = 5.6 Hz, 1H), 6.54 (dd, J = 1.8, 3.3 Hz, 1H), 6.81 (d, J = 7.9 Hz, 2H), 6.84 (d, J = 7.9 Hz, 2H), 6.85 (d, J = 5.4 Hz, 1H), 6.92 (s, 1H), 6.93 (d, J = 5.4 Hz, 1H), 7.20 (dd, J = 1.0, 3.3 Hz, 1H), 7.58 (dd, J = 1.0, 1.8 Hz, 1H), 7.91 (s, 1H)

Example 10

5-Amino-2-(2-furyl)-8-[4-(4-methoxyphenyl)piperazin-1-ylmethyl][1,2,4]triazolo[1,5-c]pyrimidine (Compound 10)

In a manner similar to that in Example 2, the subject compound (52%) was obtained as white crystals from 5-(3,4-dimethoxybenzylamino)-2-(2-furyl)-8-[4-(4-methoxyphenyl)piperazin-1-ylmethyl][1,2,4]triazolo[1,5-c]pyrimidine obtained in Example 9.
Melting point: 199-200°C
$^1$H NMR (DMSO-$d_6$, δ, ppm): 2.59-2.61 (m, 4H), 3.00-3.02 (m, 4H), 3.67 (s, 3H), 3.72 (s, 2H), 6.75 (dd, J = 1.7, 3.3 Hz, 1H), 6.78 (d, J = 9.3 Hz, 2H), 6.87 (d, J = 9.3 Hz, 2H), 7.21 (d, J = 3.3 Hz, 1H), 7.81 (d, J = 1.7 Hz, 1H), 7.87 (s, 2H), 7.93 (s, 1H)

Example 11

5-(3,4-Dimethoxybenzylamino) -2- (2-furyl) -8- [4- (2-oxobenzimidazol-1-yl)piperidinomethyl][1,2,4]triazolo[1,5-c] pyrimidine (Compound 11)

The subject compound (64%) was obtained as a pale brown oily matter from 5-(3,4-dimethoxybenzylamino)-8-formyl-2-(2-furyl) [1,2,4]triazolo[1,5-c]pyrimidine and 4-(2-oxobenzimidazol-1-yl)piperidine in a manner similar to that in Example 1.
$^1$H NMR ($CDCl_3$, δ, ppm): 1.81-1.85 (m, 2H), 2.30-2.39 (m, 2H), 2.45-2.58 (m, 2H), 3.16-3.20 (m, 2H), 3.89 (s, 3H), 3.89 (s, 3H), 3.91 (s, 2H), 4.33-4.42 (m, 1H), 4.77 (d, J = 5.8 Hz, 2H), 6.44 (t, J = 5.8 Hz, 1H), 6.58 (dd, J = 1.8, 3.5 Hz, 1H), 6.86 (d, J = 8.9 Hz, 1H), 6.96 (s, 1H), 6.97 (d, J = 8.9 Hz, 1H), 7.01-7.09 (m, 4H), 7.23 (d, J = 3.5 Hz, 1H), 7.61 (d, J = 1.8 Hz, 1H), 8.02 (s, 1H), 8.50 (s, 1H)

Example 12

5-Amino-2-(2-furyl)-8-[4-(2-oxobenzimidazol-1-yl)piperidinomethyl][1,2,4]triazolo[1,5-c]pyrimidine (Compound 12)

In a manner similar to that in Example 2, the subject compound (54%) was obtained as white crystals from 5-(3,4-dimethoxybenzylamino)-2-(2-furyl)-8-[4-(2-oxobenzimidazol-1-yl)piperidinomethyl][1,2,4]triazolo[1,5-c]pyrimidine obtained in Example 11.
Melting point: 283-284°C
$^1$H NMR (DMSO-$d_6$, δ, ppm) : 1.61-1.66 (m, 2H), 2.15-2.23 (m, 2H), 2.30-2.39 (m, 2H), 3.07-3.15 (m, 2H), 3.74 (s, 2H), 4.09-4.13 (m, 1H), 6.72 (dd, J = 1.7, 3.3 Hz, 1H), 6.95-6.96 (m, 3H), 7.20 (d, J = 3.3 Hz, 1H), 7.21-7.22 (m, 1H), 7.84 (s, 1H), 7.87 (s, 2H), 7.94 (d, J = 1.7 Hz, 1H), 10.80 (s, 1H)

Example 13

5-Amino-8-[4,4-bis(4-methoxyphenyl)piperidinomethyl]-2-(2-furyl) [1,2,4]triazolo[1,5-c]pyrimidine (Compound 13)

In a manner similar to that in Example 2, the subject compound (20%) was obtained as white crystals from 5-(3,4-dimethoxybenzylamino)-8-(1,4-dioxa-8-azaspiro[4.5]decan-8-ylmethyl)-2-(2-furyl)[1,2,4]triazolo[1,5-c]pyrimidine obtained in Reference Example 64.
Melting point: 123-127°C
$^1$H NMR ($CDCl_3$, δ, ppm) : 2.30-2.47 (m, 4H) , 2.55-2.72 (m, 4H), 3.71 (s, 2H), 3.77 (s, 6H), 5.98 (brs, 2H, $NH_2$), 6.57 (dd, J = 1.6, 3.2 Hz, 1H), 6.80 (d, J = 8.6 Hz, 4H), 7.14 (d, J = 8.6 Hz, 4H), 7.23 (d, J = 3.2 Hz, 1H), 7.61 (d, J = 1.6 Hz, 1H), 7.86 (s, 1H)

Example 14

8-(4-Cyano-4-phenylpiperidinomethyl)-5-(3,4-dimethoxybenzylamino)-2-(2-furyl)[1,2,4]triazolo[1,5-c]pyrimidine (Compound 14)

The subject compound (34%) was obtained as a pale brown oily matter from 5-(3,4-dimethoxybenzylamino)-8-formyl-2-(2-furyl)[1,2,4]triazolo[1,5-c]pyrimidine and 4-cyano-4-phenylpiperidine hydrochloride in a manner similar to that in Example 1.
$^1$H NMR ($CDCl_3$, δ, ppm): 2.04-2.25 (m, 4H), 2.58-2.78 (m, 2H), 3.08-3.22 (m, 2H), 3.88 (s, 3H), 3.88 (s, 3H), 3.94 (s, 2H), 4.76 (d, J = 5.9 Hz, 2H), 6.45 (t, J = 5.9 Hz, 1H, NH), 6.57 (dd, J = 1.6, 3.2 Hz, 1H), 6.85 (d, J = 8.4 Hz, 1H), 6.96 (s, 1H), 6.98 (d, J = 8.4 Hz, 1H), 7.23 (d, J = 3.2 Hz, 1H), 7.28-7.43 (m, 3H) , 7.46-7.53 (m, 2H) , 7.61 (d, J = 1.6 Hz, 1H), 7.94 (s, 1H)

Example 15

5-Amino-8-(4-cyano-4-phenylpiperidinomethyl)-2-(2-furyl) [1,2,4]triazolo[1,5-c]pyrimidine (Compound 15)

In a manner similar to that in Example 2, the subject compound (71%) was obtained as white crystals from 8-(4-cyano-4-phenylpiperidinomethyl)-5-(3,4-dimethoxybenzylamino)-2-(2-furyl) [1,2,4]triazolo[1,5-c]pyrimidine obtained in Example 14.
Melting point: 248-253°C

$^1H$ NMR ($CDCl_3$, δ, ppm): 2.07-2.23 (m, 4H), 2.60-2.76 (m, 2H), 3.08-3.18 (m, 2H), 3.93 (s, 2H), 5.97 (s, 2H, $NH_2$), 6.59 (dd, J = 1.6, 3.5 Hz, 1H), 7.27 (dd, J = 1.1, 3.5 Hz, 1H), 7.30-7.56 (m, 5H), 7.63 (dd, J = 1.1, 1.6 Hz, 1H) , 7.88 (s,1H)

Example 16

5-(3,4-Dimethoxybenzylamino)-2-(2-furyl)-8-[4-(3-methoxyphenyl)piperazin-1-ylmethyl][1,2,4]triazolo[1,5-c] pyrimidine (Compound 16)

The subject compound (57%) was obtained as a pale brown oily matter from 5-(3,4-dimethoxybenzylamino)-8-formyl-2-(2-furyl)[1,2,4]triazolo[1,5-c]pyrimidine and 1-(3-methoxyphenyl)piperazine in a manner similar to that in Example 1.
$^1H$ NMR ($CDCl_3$, δ, ppm): 2.76 (t, J = 4.9 Hz, 4H), 3.23 (t, J = 4.9 Hz, 4H), 3.78 (s, 3H), 3.88 (s, 3H), 3.88 (s, 3H), 3.88 (s, 2H), 4.76 (d, J = 5.6 Hz, 2H), 6.39-6.45 (m, 4H), 6.55 (dd, J = 1.8, 3.3 Hz, 1H), 6.86 (d, J = 8.9 Hz, 1H), 6.94 (s, 1H), 6.95 (d, J = 8.9 Hz, 1H), 7.16 (t, J = 5.6 Hz, 1H), 7.21 (d, J = 3.3 Hz, 1H), 7.60 (d, J = 1.8 Hz, 1H), 7.91 (s, 1H)

Example 17

5-Amino-2-(2-furyl)-8-[4-(3-methoxyphenyl)piperazin-1-ylmethyl][1,2,4]triazolo[1,5-c]pyrimidine (Compound 17)

In a manner similar to that in Example 2, the subject compound (24%) was obtained as white crystals from 5-(3,4-dimethoxybenzylamino)-2-(2-furyl)-8-[4-(3-methoxyphenyl)piperazin-1-ylmethyl][1,2,4]triazolo[1,5-c]pyrimidine obtained in Example 16.
Melting point: 182-184°C
$^1H$ NMR (DMSO-$d_6$, δ, ppm): 2. 59 (t, J = 4. 9 Hz, 4H) , 3.12 (t, J = 4.9 Hz, 4H), 3.69 (s, 3H), 3.72 (s, 2H), 6.34 (dd, J = 2.4, 8.0 Hz, 1H), 6.42 (s, 1H), 6.50 (dd, J = 2.4, 8.0 Hz, 1H), 6.71 (dd, J = 1.8, 3.5 Hz, 1H), 7.08 (dd, J = 8.0, 8.0 Hz, 1H), 7.20 (d, J = 3.5 Hz, 1H), 7.81 (s, 1H), 7.87 (s, 2H), 7.93 (d, J = 1.8 Hz, 1H)

Example 18

5-(3,4-Dimethoxybenzylamino)-2-(2-furyl)-8-(1,2,3,4-tetrahydroisoquinolin-2-ylmethyl)[1,2,4]triazolo[1,5-c]pyrimidine (Compound 18)

The subject compound (85%) was obtained as a pale brown oily matter from 5-(3,4-dimethoxybenzylamino)-8-formyl-2-(2-furyl)[1,2,4]triazolo[1,5-c]pyrimidine and 1,2,3,4-tetrahydroisoquinoline in a manner similar to that in Example 1.
$^1H$ NMR ($CDCl_3$, δ, ppm) : 2.91-2.93 (m, 4H), 3.79-3.81 (m, 2H), 3.88 (s, 3H), 3.88 (s, 3H), 4.01 (s, 2H), 4.76 (d, J = 5.6 Hz, 2H), 6.42 (t, J = 5.6 Hz, 1H), 6.57 (dd, J = 1.8, 3.5 Hz, 1H), 6.85 (d, J = 7.9 Hz, 1H), 6.96 (s, 1H), 6.97 (d, J = 7.9 Hz, 1H), 7.10-7.11 (m, 4H), 7.23 (d, J = 3.5 Hz, 1H), 7.60 (d, J = 1.8 Hz, 1H), 8.02 (s, 1H)

Example 19

5-Amino-2-(2-furyl)-8-(1,2,3,4-tetrahydroisoquinolin-2-ylmethyl) [1,2,4]triazolo[1,5-c]pyrimidine (Compound 19)

In a manner similar to that in Example 2, the subject compound (72%) was obtained as white crystals from 5-(3,4-dimethoxybenzylamino)-2-(2-furyl)-8-(1,2,3,4-tetrahydroisoquinolin-2-ylmethyl)[1,2,4]triazolo[1,5-c]pyrimidine obtained in Example 18.
Melting point: 189-190°C
$^1H$ NMR (DMSO-$d_6$, δ, ppm): 2.75-2.80 (m, 4H), 3.63 (s, 2H), 3.82 (s, 2H), 6.71 (dd, J = 1.8, 3.5 Hz, 1H), 7.00-7.09 (m, 4H), 7.21 (d, J = 3.5 Hz, 1H), 7.84 (s, 1H), 7.86 (s, 2H), 7.92 (d, J = 1.8 Hz, 1H)

Example 20

8-(1-Cyanomethyl-6,7-dimethoxy-1,2,3,4-tetrahydroisoquinolin-2-ylmethyl)-5-(3,4-dimethoxybenzylamino)-2-(2-furyl) [1,2,4]triazolo[1,5-c]pyrimidine (Compound 20)

The subject compound (86%) was obtained as a pale brown oily matter from 5-(3,4-dimethoxybenzylamino)-8-formyl-2-(2-furyl) [1,2,4]triazolo[1,5-c]pyrimidine and 6,7-dimethoxy-1,2,3,4-tetrahydro-1-isoquinolinylacetonitrile in a manner similar to that in Example 1.

$^1H$ NMR ($CDCl_3$, δ, ppm): 2.68-2.72 (m, 1H), 2.88-2.96 (m, 4H), 3.14-3.19 (m, 1H), 3.84 (s, 3H), 3.87 (s, 3H), 3.89 (s, 3H), 3.89 (s, 3H), 4.09 (s, 2H), 4.12-4.14 (m, 1H), 4.76 (d, J = 5.6 Hz, 2H), 6.41 (t, J = 5.6 Hz, 1H), 6.57 (dd, J = 1.8, 3.5 Hz, 1H), 6.61 (s, 1H), 6.63 (s, 1H), 6.86 (d, J = 8.0 Hz, 1H), 6.96 (s, 1H), 6.97 (d, J = 8.0 Hz, 1H), 7.21 (d, J = 3.5 Hz, 1H), 7.59 (d, J = 1.8 Hz, 1H), 8.07 (s, 1H)

Example 21

5-Amino-8-(1-cyanomethyl-6,7-dimethoxy-1,2,3,4-tetrahydroisoquinolin-2-ylmethyl)-2-(2-furyl)[1,2,4]triazolo[1,5-c]pyrimidine (Compound 21)

In a manner similar to that in Example 2, the subject compound (64%) was obtained as white crystals from 8-(1-cyanomethyl-6,7-dimethoxy-1,2,3,4-tetrahydroisoquinolin-2-ylmethyl)-5-(3,4-dimethoxybenzylamino)-2-(2-furyl)[1,2,4]triazolo[1,5-c]pyrimidine obtained in Example 20.
Melting point: 213-214°C
$^1H$ NMR (DMSO-$d_6$, δ, ppm) : 2.50-2.53 (m, 1H), 2.61-2.82 (m, 4H), 3.11-3.14 (m, 1H), 3.69 (s, 3H), 3.72 (s, 3H), 3.95 (s, 2H), 4.02-4.06 (m, 1H), 6.69 (s, 1H), 6.71 (dd, J = 1.8, 3.5 Hz, 1H), 6.80 (s, 1H), 7.19 (d, J = 3.5 Hz, 1H), 7.85 (s, 2H), 7.91 (d, J = 1.8 Hz, 1H), 7.95 (s, 1H)

Example 22

5-(3,4-Dimethoxybenzylamino)-2-(2-furyl)-8-[4-(2-pyrimidinyl)piperazin-1-ylmethyl][1,2,4]triazolo[1,5-c]pyrimidine (Compound 22)

The subject compound (73%) was obtained as a pale brown oily matter from 5-(3,4-dimethoxybenzylamino)-8-formyl-2-(2-furyl)[1,2,4]triazolo[1,5-c]pyrimidine and 1-(2-pyrimidinyl)piperazine in a manner similar to that in Example 1.
$^1H$ NMR ($CDCl_3$, δ, ppm): 2.66 (t, J = 5.0 Hz, 4H), 3.84 (t, J = 5.0 Hz, 4H), 3.88 (s, 3H), 3.88 (s, 3H), 3.89 (s, 2H), 4.76 (d, J = 5.6 Hz, 2H), 6.41 (t, J = 5.6 Hz, 1H), 6.46 (dd, J = 4.7, 4.7 Hz, 1H), 6.56 (dd, J = 1.8, 3.5 Hz, 1H), 6.84 (d, J = 7.9 Hz, 1H), 6.95 (s, 1H), 6.96 (d, J = 7.9 Hz, 1H), 7.22 (d, J = 3.5 Hz, 1H), 7.59 (d, J = 1.8 Hz, 1H), 7.98 (s, 1H), 8.29 (d, J = 4.7 Hz, 2H)

Example 23

5-Amino-2-(2-furyl)-8-[4-(2-pyrimidinyl)piperazin-1-ylmethyl][1,2,4]triazolo[1,5-c]pyrimidine (Compound 23)

In a manner similar to that in Example 2, the subject compound (10%) was obtained as white crystals from 5-(3,4-dimethoxybenzylamino)-2-(2-furyl)-8-[4-(2-pyrimidinyl)piperazin-1-ylmethyl][1,2,4]triazolo[1,5-c]pyrimidine obtained in Example 22.
Melting point: 253-254°C
$^1H$ NMR (DMSO-$d_6$, δ, ppm) : 3.70-3.74 (m, 10H) , 6.60 (dd, J = 4.7, 4.7 Hz, 1H), 6.71 (dd, J = 1.8, 3.5 Hz, 1H), 7.20 (d, J = 3.5 Hz, 1H), 7.82 (s, 1H), 7.86 (s, 2H), 7.93 (d, J = 1.8 Hz, 1H), 8.32 (d, J = 4.7 Hz, 2H)

Example 24

8-(4-Benzylpiperazin-1-ylmethyl)-5-(3,4-dimethoxybenzylamino)-2-(2-furyl)[1,2,4]triazolo[1,5-c]pyrimidine (Compound 24)

The subject compound (82%) was obtained as a pale brown oily matter from 5-(3,4-dimethoxybenzylamino)-8-formyl-2-(2-furyl)[1,2,4]triazolo[1,5-c]pyrimidine and 1-benzylpiperazine in a manner similar to that in Example 1.
$^1H$ NMR ($CDCl_3$, δ, ppm) : 2.51-2.53 (m, 4H) , 2.63-2.65 (m, 4H), 3.51 (s, 2H), 3.86 (s, 2H), 3.88 (s, 3H), 3.88 (s, 3H), 4.74 (d, J = 5.6 Hz, 2H), 6.40 (t, J = 5.6 Hz, 1H), 6.56 (dd, J = 1.8, 3.5 Hz, 1H), 6.85 (d, J = 8.0 Hz, 1H), 6.95 (s, 1H), 6.96 (d, J = 8.0 Hz, 1H), 7.20 (d, J = 3.5 Hz, 1H), 7.29-7.31 (m, 5H), 7.59 (d, J = 1.8 Hz, 1H), 7.91 (s, 1H)

Example 25

5-Amino-8-(4-benzylpiperazin-1-ylmethyl)-2-(2-furyl)[1,2,4]triazolo[1,5-c]pyrimidine (Compound 25)

In a manner similar to that in Example 2, the subject compound (88%) was obtained as white crystals from

8-(4-benzylpiperazin-1-ylmethyl)-5-(3,4-dimethoxybenzylamino)-2-(2-furyl)[1,2,4]triazolo[1,5-c]pyrimidine obtained in Example 24.
Melting point: 182-183°C
$^1$H NMR ($CDCl_3$, δ, ppm) : 2.51-2.53 (m, 4H), 2.62-2.64 (m, 4H), 3.51 (s, 2H), 3.85 (s, 2H), 5.97 (s, 2H), 6.57 (dd, J = 1.7, 3.3 Hz, 1H), 7.23 (dd, J = 1.0, 3.3 Hz, 1H), 7.26-7.31 (m, 5H), 7.62 (dd, J = 1.0, 1.7 Hz, 1H), 7.85 (s, 1H)

Example 26

8-(4-Benzylpiperidinomethyl)-5-(3,4-dimethoxybenzylamino)-2-(2-furyl)[1,2,4]triazolo[1,5-c]pyrimidine (Compound 26)

The subject compound (84%) was obtained as a pale brown oily matter from 5-(3,4-dimethoxybenzylamino)-8-formyl-2-(2-furyl) [1,2,4]triazolo[1,5-c]pyrimidine and 4-benzylpiperidine in a manner similar to that in Example 1.
$^1$H NMR ($CDCl_3$, δ, ppm): 1.34-1.42 (m, 1H) , 1.54-1.62 (m, 1H), 1.79 (brs, 4H), 2.07-2.15 (m, 1H), 2.52-2.54 (m, 2H), 3.00-3.05 (m, 2H) , 3.83 (s, 2H), 3.88 (s, 3H) , 3.88 (s, 3H), 4.75 (d, J = 5.6 Hz, 2H), 6.40 (t, J = 5.6 Hz, 1H), 6.56 (dd, J = 1.8, 3.5 Hz, 1H), 6.84 (d, J = 8.1 Hz, 1H), 6.95 (s, 1H), 6.96 (d, J = 8.1 Hz, 1H), 7.11-7.15 (m, 3H), 7.20 (dd, J = 1.0, 3.5 Hz, 1H), 7.21-7.23 (m, 2H), 7.59 (dd, J = 1.0, 1.8 Hz, 1H), 7.94 (s, 1H)

Example 27

5-Amino-8-(4-benzylpiperidinomethyl)-2-(2-furyl) [1,2,4]triazolo[1,5-c]pyrimidine (Compound 27)

In a manner similar to that in Example 2, the subject compound (78%) was obtained as white crystals from 8-(4-benzylpiperidinomethyl)-5-(3,4-dimethoxybenzylamino)-2-(2-furyl)[1,2,4]triazolo[1,5-c]pyrimidine obtained in Example 26.
Melting point: 169-170°C
$^1$H NMR ($CDCl_3$, δ, ppm) : 1.23-1.48 (m, 2H) , 1.50-1.56 (m, 1H), 1.61-1.66 (m, 2H), 2.03-2.11 (m, 2H), 2.52-2.54 (m, 2H), 2.96-3.01 (m, 2H), 3.80 (s, 2H), 5.94 (s, 2H), 6.57 (dd, J = 1.7, 3.3 Hz, 1H), 7.11-7.19 (m, 5H), 7.23 (dd, J = 1.0, 3.3 Hz, 1H), 7.62 (dd, J = 1.0, 1.7 Hz, 1H), 7.86 (s, 1H)

Example 28

5-(3,4-Dimethoxybenzylamino)-2-(2-furyl)-8-[4-phenethylpiperazin-1-ylmethyl][1,2,4]triazolo[1,5-c]pyrimidine (Compound 28)

The subject compound (88%) was obtained as a pale brown oily matter from 5-(3,4-dimethoxybenzylamino)-8-formyl-2-(2-furyl)[1,2,4]triazolo[1,5-c]pyrimidine and 1-(2-phenylethyl)piperazine in a manner similar to that in Example 1.
$^1$H NMR ($CDCl_3$, δ , ppm): 2.58-2.79 (m, 10H), 2.80-2.83 (m, 2H), 3.87 (s, 2H), 3.89 (s, 3H), 3.89 (s, 3H), 4.75 (d, J = 5.8 Hz, 2H), 6.41 (t, J = 5.8 Hz, 1H), 6.56 (dd, J = 1.7, 3.3 Hz, 1H), 6.85 (d, J = 7.9 Hz, 1H), 6.95 (s, 1H), 6.97 (d, J = 7.9 Hz, 1H) , 7.16-7.21 (m, 4H), 7.22 (d, J = 3.3 Hz, 1H), 7.25-7.27 (m, 1H), 7.60 (d, J = 1.7 Hz, 1H), 7.93 (s, 1H)

Example 29

5-Amino-2-(2-furyl)-8-[4-phenethylpiperazin-1-ylmethyl][1,2,4]triazolo[1,5-c]pyrimidine (Compound 29)

In a manner similar to that in Example 2, the subject compound (94%) was obtained as white crystals from 5-(3,4-dimethoxybenzylamino)-2-(2-furyl)-8-[4-phenethylpiperazin-1-ylmethyl] [1,2,4]triazolo[1,5-c]pyrimidine obtained in Example 28.
Melting point: 174-176°C
$^1$H NMR ($CDCl_3$, δ, ppm): 2.58-2.78 (m, 10H), 2.80-2.83 (m, 2H), 3.86 (s, 2H), 5.96 (s, 2H), 6.58 (dd, J = 1.8, 3.5 Hz, 1H), 7.15-7.20 (m, 5H), 7.24 (d, J = 3.5 Hz, 1H), 7.63 (d, J = 1.8 Hz, 1H), 7.87 (s, 1H)

Example 30

5-(3,4-Dimethoxybenzylamino)-2-(2-furyl)-8-[4-(2-pyridylmethyl)piperazin-1-ylmethyl][1,2,4]triazolo[1,5-c]pyrimidine (Compound 30)

The subject compound (quantitative) was obtained as a pale brown oily matter from 5-(3,4-dimethoxybenzylamino)-8-formyl-2-(2-furyl)[1,2,4]triazolo[1,5-c]pyrimidine and 1-(2-pyridylmethyl)piperazine in a manner similar to that in Example 1.
$^1$H NMR ($CDCl_3$, δ, ppm): 2.57-2.59 (m, 4H), 2.66-2.68 (m, 4H), 3.67 (s, 2H), 3.87 (s, 2H), 3.88 (s, 3H), 3.88 (s, 3H), 4.75 (d, J = 5.8 Hz, 2H), 6.41 (t, J = 5.8 Hz, 1H), 6.56 (dd, J = 1.8, 3.5 Hz, 1H), 6.84 (d, J = 7.9 Hz, 1H), 6.95 (s, 1H), 6.96 (d, J = 7.9 Hz, 1H), 7.12-7.17 (m, 1H), 7.21 (dd, J = 1.0, 3.5 Hz, 1H), 7.37-7.40 (m, 1H), 7.59 (dd, J = 1.0, 1.8 Hz, 1H), 7.60-7.66 (m, 1H), 7.92 (s, 1H), 8.54-8.56 (m, 1H)

Example 31

5-Amino-2-(2-furyl)-8-[4-(2-pyridylmethyl)piperazin-1-ylmethyl][1,2,4]triazolo[1,5-c]pyrimidine (Compound 31)

In a manner similar to that in Example 2, the subject compound (80%) was obtained as white crystals from 5-(3,4-dimethoxybenzylamino)-2-(2-furyl)-8-[4-(2-pyridylmethyl)piperazin-1-ylmethyl][1,2,4]triazolo[1,5-c]pyrimidine obtained in Example 30.
Melting point: 181-182°C
$^1$H NMR ($CDCl_3$, δ, ppm): 2.61-2.63 (m, 4H), 2.68-2.70 (m, 4H), 3.80 (s, 2H), 3.88 (s, 2H), 5.96 (s, 2H), 6.58 (dd, J = 1.8, 3.5 Hz, 1H), 7.13-7.18 (m, 1H), 7.23 (dd, J = 1.0, 3.5 Hz, 1H), 7.38-7.41 (m, 1H), 7.60-7.67 (m, 1H) , 7.62 (dd, J = 1.0, 1.8 Hz, 1H), 7.88 (s, 1H) , 8.54-8.56 (m, 1H)

Example 32

8-[4-(4-Chlorophenyl)-4-methoxypiperidinomethyl]-5-(3,4-dimethoxybenzylamino)-2-(2-furyl)[1,2,4]triazolo[1,5-c]pyrimidine (Compound 32)

The subject compound (92%) was obtained as white crystals from 3-(3,4-dimethoxybenzylamino)-8-formyl-2-(2-furyl)[1,2,4]triazolo[1,5-c]pyrimidine and 4-(4-chlorophenyl)-4-methoxypiperidine in a manner similar to that in Example 1.
Melting point: 178-180°C
$^1$H NMR ($CDCl_3$, δ, ppm): 1.95-2.04 (m, 4H), 2.50-2.64 (m, 2H), 2.83-2.92 (m, 2H), 2.94 (s, 3H), 3.88 (s, 3H), 3.88 (s, 3H), 3.88 (s, 2H), 4.76 (d, J = 5.7 Hz, 2H), 6.42 (t, J = 5.7 Hz, 1H), 6.56 (dd, J = 1.6, 3.5 Hz, 1H), 6.85 (d, J = 7.8 Hz, 1H), 6.96 (s, 1H), 6.98 (d, J = 7.8 Hz, 1H), 7.22 (dd, J = 0.8, 3.5 Hz, 1H), 7.32 (s, 4H), 7.60 (dd, J = 0.8, 1.6 Hz, 1H), 7.94 (s, 1H)

Example 33

5-Amino-8-[4-(4-chlorophenyl)-4-(4-methoxyphenyl)piperidinomethyl]-2-(2-furyl) [1,2,4]triazolo[1,5-c]pyrimidine (Compound 33)

In a manner similar to that in Example 2, the subject compound (84%) was obtained as white crystals from 8-[4-(4-chlorophenyl)-4-methoxypiperidinomethyl]-5-(3,4-dimethoxybenzylamino)-2-(2-furyl)[1,2,4]triazolo[1,5-c]pyrimidine obtained in Example 32.
Melting point: 179-181°C
$^1$H NMR ($CDCl_3$, δ, ppm): 2.32-2.50 (m, 4H), 2.52-2.72 (m, 4H), 3.72 (s, 2H), 3.77 (s, 3H), 5.91 (brs, 2H), 6.57 (dd, J = 1.6, 3.8 Hz, 1H), 6.81 (d, J = 8.9 Hz, 2H), 7.13 (d, J = 8.9 Hz, 2H), 7.16 (d, J = 8.4 Hz, 2H), 7.22 (d, J = 8.4 Hz, 2H), 7.23 (d, J = 3.8 Hz, 1H), 7.61 (d, J = 1.6 Hz, 1H), 7.86 (s, 1H)

Example 34

5-(3,4-Dimethoxybenzylamino)-2-(2-furyl)-8-[4-(4-pyridylmethyl)piperazin-1-ylmethyl][1,2,4]triazolo[1,5-c]pyrimidine (Compound 34)

The subject compound (97%) was obtained as a pale brown oily matter from 5-(3,4-dimethoxybenzylamino)-8-formyl-2-(2-furyl)[1,2,4]triazolo[1,5-c]pyrimidine and 1-(4-pyridylmethyl)piperazine in a manner similar to that in Example 1.
$^1$H NMR ($CDCl_3$, δ, ppm): 2.50-2.52 (m, 4H), 2.62-2.64 (m, 4H), 3.50 (s, 2H), 3.86 (s, 2H), 3.88 (s, 3H), 3.88 (s, 3H), 4.75 (d, J = 5.6 Hz, 2H), 6.41 (t, J = 5.6 Hz, 1H), 6.56 (dd, J = 1.7, 3.3 Hz, 1H), 6.85 (d, J = 7.9 Hz, 1H), 6.95 (s, 1H), 6.96 (d, J = 7.9 Hz, 1H), 7.20 (dd, J = 1.0, 3.3 Hz, 1H), 7.24 (dd, J = 1.6, 4.4 Hz, 2H), 7.60 (dd, J = 1.0, 1.7 Hz, 1H), 7.92 (s, 1H), 8.52 (dd, J = 1.6, 4.4 Hz, 2H)

Example 35

5-Amino-2- (2-furyl) -8- [4- (4-pyridylmethyl)piperazin-1-ylmethyl][1,2,4]triazolo[1,5-c]pyrimidine (Compound 35)

In a manner similar to that in Example 2, the subject compound (92%) was obtained as white crystals from 5-(3,4-dimethoxybenzylamino)-2-(2-furyl)-8-[4-(4-pyridylmethyl)piperazin-1-ylmethyl][1,2,4]triazolo[1,5-c]pyrimidine obtained in Example 34.
Melting point: 198-200°C
$^1$H NMR ($CDCl_3$, δ, ppm): 2.51-2.53 (m, 4H), 2.64-2.66 (m, 4H), 3.51 (s, 2H), 3.87 (s, 2H), 5.96 (s, 2H), 6.58 (dd, J = 1.8, 3.5 Hz, 1H), 7.24 (d, J = 3.5 Hz, 1H), 7.25 (dd, J = 1.7, 4.3 Hz, 2H), 7.62 (d, J = 1.8 Hz, 1H), 7.87 (s, 1H), 8.51 (dd, J = 1.7, 4.3 Hz, 2H)

Example 36

5-(3,4-Dimethoxybenzylamino)-2-(2-furyl)-8-[4-(3-pyridylmethyl)piperazin-1-ylmethyl][1,2,4]triazolo[1,5-c]pyrimidine (Compound 36)

The subject compound (71%) was obtained as a pale brown oily matter from 5-(3,4-dimethoxybenzylamino)-8-formyl-2-(2-furyl)[1,2,4]triazolo[1,5-c]pyrimidine and 1-(3-pyridylmethyl)piperazine in a manner similar to that in Example 1.
Melting point: 118-119°C
$^1$H NMR ($CDCl_3$, δ, ppm): 2.50-2.52 (m, 4H), 2.62-2.64 (m, 4H), 3.51 (s, 2H), 3.85 (s, 2H), 3.88 (s, 3H), 3.88 (s, 3H), 4.74 (d, J = 5.6 Hz, 2H), 6.42 (t, J = 5.6 Hz, 1H), 6.56 (dd, J = 1.7, 3.3 Hz, 1H), 6.84 (d, J = 8.1 Hz, 1H), 6.95 (s, 1H), 6.96 (d, J = 8.1 Hz, 1H), 7.21 (d, J = 3.3 Hz, 1H), 7.23-7.24 (m, 1H), 7.59 (d, J = 1.7 Hz, 1H), 7.64-7.66 (m, 1H), 7.91 (s, 1H), 8.47-8.52 (m, 2H)

Example 37

5-Amino-2-(2-furyl)-8-[4-(3-pyridylmethyl)piperazin-1-ylmethyl] [1,2,4]triazolo[1,5-c]pyrimidine (Compound 37)

In a manner similar to that in Example 2, the subject compound (quantitative) was obtained as white crystals from 5-(3,4-dimethoxybenzylamino)-2-(2-furyl)-8-[4-(3-pyridylmethyl)piperazin-1-ylmethyl][1,2,4]triazolo[1,5-c]pyrimidine obtained in Example 36.
Melting point: 159-161°C
$^1$H NMR (DMSO-$d_6$, δ, ppm): 2.08-2.49 (m, 4H), 3.24-3.27 (m, 4H), 3.51 (s, 2H), 3.71 (s, 2H), 6.71 (dd, J = 1.8, 3.6 Hz, 1H), 7.19 (d, J = 3.6 Hz, 1H), 7.31-7.36 (m, 1H), 7.67-7.70 (m, 1H), 7.78 (s, 1H), 7.87 (s, 2H), 7.92 (d, J = 1.8 Hz, 1H), 8.45-8.46 (m, 2H)

Example 38

8-(4-Anilinopiperidinomethyl)-5-(3,4-dimethoxybenzylamino)-2-(2-furyl)[1,2,4]triazolo[1,5-c]pyrimidine (Compound 38)

The subject compound (73%) was obtained as a pale brown oily matter from 5-(3,4-dimethoxybenzylamino)-2-(2-furyl)-8-(4-oxopiperidinomethyl) [1,2,4]triazolo[1,5-c]pyrimidine obtained in Reference Example 67 and aniline in a manner similar to that in Example 1.
$^1$H NMR ($CDCl_3$, δ , ppm): 1.43-1.60 (m, 2H), 2.00-2.12 (m, 2H) , 2.24-2.60 (m, 2H) , 2.92-3.05 (m, 2H), 3.25-3.39 (m, 1H), 3.85 (s, 2H), 3.88 (s, 3H), 3.88 (s, 3H), 4.76 (d, J = 6.8 Hz, 2H), 6.43 (t, J = 6.8 Hz, 1H), 6.55-6.62 (m, 3H), 6.67 (t, J = 7.6 Hz, 1H), 6.85 (d, J = 7.8 Hz, 1H), 6.96 (s, 1H), 6.98 (d, J = 7.8 Hz, 1H), 7.15 (dd, J = 7.6, 7.6 Hz, 2H), 7.20-7.23 (m, 1H), 7.58-7.62 (m, 1H), 7.92 (s, 1H)

Example 39

5-Amino-8-(4-anilinopiperidinomethyl)-2-(2-furyl)[1,2,4]triazolo[1,5-c]pyrimidine (Compound 39)

In a manner similar to that in Example 2, the subject compound (91%) was obtained as white crystals from 8-(4-anilinopiperidinomethyl)-5-(3,4-dimethoxybenzylamino)-2-(2-furyl)[1,2,4]triazolo[1,5-c]pyrimidine obtained in Example 38.
Melting point: 177-180°C
$^1$H NMR ($CDCl_3$, δ, ppm): 1.42-1.61 (m, 2H), 2.00-2.16 (m, 2H), 2.23-2.50 (m, 2H), 2.93-3.04 (m, 2H), 3.25-3.42 (m, 1H), 3.85 (s, 2H), 6.14 (brs, 2H), 6.55-6.62 (m, 3H), 6.67 (t, J = 7.6 Hz, 1H), 7.15 (dd, J = 7.6, 7.6 Hz, 2H), 7.23-7.27 (m, 1H), 7.60-7.65 (m, 1H), 7.87 (s, 1H)

Example 40

8-(4-Benzylaminopiperidinomethyl)-5-(3,4-dimethoxybenzylamino)-2-(2-furyl)[1,2,4]triazolo[1,5-c]pyrimidine (Compound 40)

The subject compound (92%) was obtained as a pale brown oily matter from 5-(3,4-dimethoxybenzylamino)-2-(2-furyl)-8-(4-oxopiperidinomethyl)[1,2,4]triazolo[1,5-c]pyrimidine obtained in Reference Example 67 and benzylamine in a manner similar to that in Example 1.
$^1$H NMR ($CDCl_3$, δ, ppm) : 1.45-1.62 (m, 2H) , 1.85-2.00 (m, 2H), 2.10-2.25 (m, 2H) , 2.50-2.62 (m, 1H), 2.92-3.05 (m, 2H), 3.82 (s, 2H), 3.82 (s, 2H), 3.88 (s, 3H), 3.88 (s, 3H), 4.75 (d, J = 5.4 Hz, 2H), 6.45 (t, J = 5.4 Hz, 1H), 6.56 (dd, J = 1.6, 3.2 Hz, 1H), 6.84 (d, J = 7.8 Hz, 1H), 6.95 (s, 1H), 6.98 (d, J = 7.8 Hz, 1H), 7.21 (d, J = 3.2 Hz, 1H), 7.22-7.40 (m, 5H), 7.59 (d, J = 1.6 Hz, 1H), 7.93 (s, 1H)

Example 41

5-Amino-8-(4-benzylaminopiperidinomethyl)-2-(2-furyl)[1,2,4]triazolo[1,5-c]pyrimidine (Compound 41)

In a manner similar to that in Example 2, the subject compound (60%) was obtained as white crystals from 8-(4-benzylaminopiperidinomethyl)-5-(3,4-dimethoxybenzylamino)-2-(2-furyl)[1,2,4]triazolo[1,5-c]pyrimidine obtained in Example 40.
Melting point: 174-176°C
$^1$H NMR ($CDCl_3$, δ, ppm): 1.38-1.58 (m, 2H), 1.85-1.97 (m, 2H), 2.10-2.25 (m, 2H), 2.47-2.61 (m, 1H), 2.88-3.02 (m, 2H), 3.81 (s, 2H), 3.82 (s, 2H), 6.11 (brs, 2H), 6.58 (dd, J = 1.6, 3.2 Hz, 1H), 7.16-7.34 (m, 6H), 7.62 (d, J = 1.6 Hz, 1H), 7.87 (s, 1H)

Example 42

5-(3,4-Dimethoxybenzylamino)-2-(2-furyl)-8-(4-phenethylaminopiperidinomethyl)[1,2,4]triazolo[1,5-c]pyrimidine (Compound 42)

The subject compound (85%) was obtained as a pale brown oily matter from 5-(3,4-dimethoxybenzylamino)-2-(2-furyl)-8-(4-oxopiperidinomethyl)[1,2,4]triazola[1,5-c]pyrimidine obtained in Reference Example 67 and phenethylamine in a manner similar to that in Example 1.
$^1$H NMR ($CDCl_3$, δ, ppm): 1.30-1.53 (m, 2H), 1.78-1.92 (m, 2H), 2.09-2.22 (m, 2H), 2.42-2.58 (m, 1H), 2.76-3.03 (m, 6H), 3.80 (s, 2H), 3.88 (s, 3H), 3.88 (s, 3H), 4.75 (d, J = 5.7 Hz, 2H), 6.44 (t, J = 5.7 Hz, 1H), 6.56 (dd, J = 1.6, 3.5 Hz, 1H), 6.85 (d, J = 7.8 Hz, 1H), 6.95 (s, 1H), 6.97 (d, J = 7.8 Hz, 1H), 7.13-7.35 (m, 6H), 7.59 (dd, J = 0.8, 1.6 Hz, 1H), 7.92 (s, 1H)

Example 43

5-Amino-2-(2-furyl)-8-(4-phenethylaminopiperidinomethyl)[1,2,4]triazolo[1,5-c]pyrimidine (Compound 43)

In a manner similar to that in Example 2, the subject compound (57%) was obtained as white crystals from 5-(3,4-dimethoxybenzylamino)-2-(2-furyl)-8-(4-phenethylaminopiperidinomethyl)[1,2,4]triazolo[1,5-c]pyrimidine obtained in Example 42.
Melting point: 144-147°C

$^1$H NMR ($CDCl_3$, δ, ppm): 1.30-1.51 (m, 2H), 1.80-1.93 (m, 2H), 2.08-2.23 (m, 2H), 2.42-2.56 (m, 1H), 2.72-3.03 (m, 6H), 3.81 (s, 2H), 6.08 (brs, 2H), 6.58 (dd, J = 2.2, 3.8 Hz, 1H), 7.12-7.34 (m, 6H), 7.62 (d, J = 2.2 Hz, 1H), 7.86 (s, 1H)

Example 44

5-(3,4-Dimethoxybenzylamino-8-[4-(2-fluorophenyl)piperazin-1-ylmethyl]-2-(2-furyl)[1,2,4]triazolo[1,5-c]pyrimidine (Compound 44)

The subject compound (92%) was obtained as a pale brown oily matter from 5-(3,4-dimethoxybenzylamino)-8-formyl-2-(2-furyl)[1,2,4]triazolo[1,5-c]pyrimidine and 1-(2-fluorophenyl)piperazine in a manner similar to that in Example 1.
$^1$H NMR ($CDCl_3$, δ, ppm): 2.79 (t, J = 4.7 Hz, 4H), 3.14 (t, J = 4.7 Hz, 4H), 3.89 (s, 3H), 3.89 (s, 3H), 3.92 (s, 2H), 4.76 (d, J = 5.6 Hz, 2H), 6.43 (t, J = 5.6 Hz, 1H), 6.57 (dd, J = 1.8, 3.6 Hz, 1H), 6.84-7.07 (m, 7H), 7.23 (dd, J = 0.9, 3.6 Hz, 1H), 7.60 (dd, J = 0.9, 1.8 Hz, 1H), 7.96 (s, 1H)

Example 45

5-Amino-8-[4-(2-fluorophenyl)piperazin-1-ylmethyl]-2-(2-furyl)[1,2,4]triazolo[1,5-c]pyrimidine (Compound 45)

In a manner similar to that in Example 2, the subject compound (94%) was obtained as white crystals from 5-(3,4-dimethoxybenzylamino-8-[4-(2-fluorophenyl)piperazin-1-ylmethyl]-2-(2-furyl)[1,2,4]triazolo[1,5-c]pyrimidine obtained in Example 44.
Melting point: 212-213°C
$^1$H NMR (DMSO-$d_6$, δ, ppm): 2.61-2.63 (m, 4H), 3.00-3.02 (m, 4H), 3.73 (s, 2H), 6.72 (dd, J = 1.8, 3.6 Hz, 1H), 6.94-7.14 (m, 4H), 7.21 (dd, J = 1.0, 3.6 Hz, 1H), 7.81 (s, 1H), 7.87 (s, 2H), 7.93 (dd, J = 1.0, 1.8 Hz, 1H)

Example 46

5-(3,4-Dimethoxybenzylamino)-2- (2-furyl) -8- [4- (4-trifluoromethylphenyl)piperazin-1-ylmethyl][1,2,4]triazolo[1,5-c] pyrimidine (Compound 46)

The subject compound (quantitative) was obtained as a pale brown oily matter from 5-(3,4-dimethoxybenzylamino)-8-formyl-2-(2-furyl)[1,2,4]triazolo[1,5-c]pyrimidine and 1-(4-trifluoromethylphenyl)piperazine in a manner similar to that in Example 1.
$^1$H NMR ($CDCl_3$, δ, ppm): 2.75 (t, J = 5.0 Hz, 4H), 3.31 (t, J = 5.0 Hz, 4H), 3.88 (s, 3H), 3.88 (s, 3H), 3.90 (s, 2H), 4.76 (d, J = 5.6 Hz, 2H), 6.44 (t, J = 5.6 Hz, 1H), 6.57 (dd, J = 1.7, 3.3 Hz, 1H), 6.85 (d, J = 8.0 Hz, 1H), 6.90 (d, J = 8.9 Hz, 2H), 6.95 (s, 1H), 6.96 (d, J = 8.0 Hz, 1H), 7.22 (d, J = 3.3 Hz, 1H), 7.45 (d, J = 8.9 Hz, 2H), 7.60 (d, J = 1.7 Hz, 1H), 7.96 (s, 1H)

Example 47

5-Amino-2-(2-furyl)-8-[4-(4-trifluoromethylphenyl)piperazin-1-ylmethyl][1,2,4]triazolo[1,5-c]pyrimidine (Compound 47)

In a manner similar to that in Example 2, the subject compound (53%) was obtained as white crystals from 5-(3,4-dimethoxybenzylamino)-2-(2-furyl)-8-[4-(4-trifluoromethylphenyl)piperazin-1-ylmethyl] [1,2,4]triazolo[1,5-c]pyrimidine obtained in Example 46.
Melting point: 261-263°C
$^1$H NMR (DMSO-$d_6$, δ, ppm): 2.59-2.61 (m, 4H), 3.03-3.05 (m, 4H), 3.73 (s, 2H), 6.72 (dd, J = 1.8, 3.6 Hz, 1H), 7.02 (d, J = 8.6 Hz, 2H), 7.21 (d, J = 3.6 Hz, 1H), 7.47 (d, J = 8.6 Hz, 2H), 7.82 (s, 1H), 7.86 (s, 2H), 7.92 (d, J = 1.8 Hz, 1H)

Example 48

8-[4-(2-Chlorophenyl)piperazin-1-ylmethyl]-5-(3,4-dimethoxybenzylamino)-2-(2-furyl)[1,2,4]triazolo[1,5-c]pyrimidine (Compound 48)

The subject compound (32%) was obtained as a pale brown oily matter from 5-(3,4-dimethoxybenzylamino)-8-formyl-2-(2-furyl) [1,2,4]triazolo[1,5-c]pyrimidine and 1-(2-chlorophenyl)piperazine in a manner similar to that in Example 1.

$^1$H NMR ($CDCl_3$, δ, ppm): 2.79-2.81 (m, 4H), 3.09-3.11 (m, 4H), 3.88 (s, 3H), 3.88 (s, 3H), 3.93 (s, 2H), 4.76 (d, J = 5.6 Hz, 2H), 6.45 (t, J = 5.6 Hz, 1H), 6.57 (dd, J = 1.8, 3.5 Hz, 1H), 6.85 (d, J = 8.0 Hz, 1H), 6.93 (s, 1H), 6.94 (d, J = 8.0 Hz, 1H), 7.04 (dd, J = 1.6, 8.0 Hz, 2H), 7.23 (d, J = 3.5 Hz, 1H), 7.34 (dd, J = 1.6, 8.0 Hz, 2H), 7.61 (d, J = 1.8 Hz, 1H), 7.96 (s, 1H)

Example 49

5-Amino-8-[4-(2-chlorophenyl)piperazin-1-ylmethyl]-2-(2-furyl)[1,2,4]triazolo[1,5-c]pyrimidine (Compound 49)

In a manner similar to that in Example 2, the subject compound (41%) was obtained as white crystals from 8-[4-(2-chlorophenyl)piperazin-1-ylmethyl]-5-(3,4-dimethoxybenzylamino)-2-(2-furyl)[1,2,4]triazolo[1,5-c]pyrimidine obtained in Example 48.
Melting point: 227-228°C
$^1$H NMR (DMSO-$d_6$, δ, ppm): 2.62-2.64 (m, 4H), 2.96-2.98 (m, 4H), 3.74 (s, 2H), 6.72 (dd, J = 1.7, 3.3 Hz, 1H), 6.99-7.05 (m, 1H), 7.12-7.14 (m, 1H), 7.21 (d, J = 3.3 Hz, 1H), 7.22-7.27 (m, 1H), 7.36-7.39 (m, 1H), 7.82 (s, 1H), 7.87 (s, 2H), 7.93 (d, J = 1.7 Hz, 1H)

Example 50

2-{4-[5-(3,4-Dimethoxybenzylamino)-2-(2-furyl)[1,2,4]triazolo[1,5-c]pyrimidin-8-ylmethyl]piperazin-1-yl}-N-methylacetoanilide (Compound 50)

The subject compound (quantitative) was obtained as a pale brown oily matter from 5-(3,4-dimethoxybenzylamino)-8-formyl-2-(2-furyl)[1,2,4]triazolo[1,5-c]pyrimidine and N-methyl-N-[2-(piperazin-1-yl)acetyl]aniline in a manner similar to that in Example 1.
$^1$H NMR ($CDCl_3$, δ, ppm): 2.48-2.50 (m, 4H), 2.59-2.61 (m, 4H), 2.92 (s, 2H), 3.26 (s, 3H), 3.81 (s, 2H), 3.88 (s, 3H), 3.88 (s, 3H), 4.74 (d, J = 5.6 Hz, 2H), 6.40 (t, J = 5.6 Hz, 1H), 6.56 (dd, J = 1.8, 3.5 Hz, 1H), 6.85 (d, J = 8.1 Hz, 1H), 6.94 (s, 1H), 6.95 (d, J = 8.1 Hz, 1H), 7.17 -7.20 (m, 1H), 7.21 (d, J = 3.5 Hz, 1H) , 7.29-7.42 (m, 4H), 7.59 (d, J = 1.8 Hz, 1H), 7.88 (s, 1H)

Example 51

2-{4-[5-Amino-2-(2-furyl)[1,2,4]triazolo[1,5-c]pyrimidin-8-ylmethyl]piperazin-1-yl}-N-methylacetoanilide (Compound 51)

In a manner similar to that in Example 2, the subject compound (78%) was obtained as white crystals from 2-{4-[5-(3,4-dimethoxybenzylamino)-2-(2-furyl)[1,2,4]triazolo[1,5-c]pyrimidin-8-ylmethyl]piperazin-1-yl}-N-methylacetoanilide obtained in Example 50.
Melting point: 171-173°C
$^1$H NMR (DMSO-$d_6$, δ, ppm) : 2.35-2.37 (m, 4H) , 2.88-2.90 (m, 4H), 3.16 (s, 2H), 3.31 (s, 3H), 3.60 (s, 2H), 6.71 (dd, J = 1.8, 3.5 Hz, 1H), 7.20 (d, J = 3.5 Hz, 1H), 7.32-7.44 (m, 5H), 7.73 (s, 1H), 7.84 (s, 2H), 7.93 (d, J = 1.8 Hz, 1H)

Example 52

5-(3,4-Dimethoxybenzylamino-8-[4-(2-furoyl)piperazin-1-ylmethyl]-2-(2-furyl)[1,2,4]triazolo[1,5-c]pyrimidine (Compound 52)

The subject compound (quantitative) was obtained as a pale brown oily matter from 5-(3,4-dimethoxybenzylamino)-8-formyl-2-(2-furyl)[1,2,4]triazolo[1,5-c]pyrimidine and 1-(2-furoyl)piperazine in a manner similar to that in Example 1.
$^1$H NMR ($CDCl_3$, δ, ppm): 2.65 (t, J = 5.0 Hz, 4H), 3.83 (t, J = 5.0 Hz, 4H), 3.88 (s, 3H), 3.88 (s, 3H), 3.92 (s, 2H), 4.75 (d, J = 5.6 Hz, 2H), 6.44 (t, J = 5.6 Hz, 1H), 6.46 (dd, J = 1.8. 3.5 Hz, 1H), 6.57 (dd, J = 1.7, 3.3 Hz, 1H), 6.85 (d, J = 7.9 Hz, 1H), 6.95 (s, 1H), 6.96 (d, J = 7.9 Hz, 1H), 6.98 (dd, J = 1.0, 3.3 Hz, 1H), 7.21 (dd, J = 1.0, 3.5 Hz, 1H), 7.46 (dd, J = 1.0, 1.7 Hz, 1H), 7.60 (dd, J = 1.0, 1.8 Hz, 1H), 7.92 (s, 1H)

Example 53

5-Amino-8-[4-(2-furoyl)piperazin-1-ylmethyl]-2-(2-furyl) [1,2,4]triazolo[1,5-c]pyrimidine (Compound 53)

In a manner similar to that in Example 2, the subject compound (77%) was obtained as white crystals from 5-(3,4-dimethoxybenzylamino-8-[4-(2-furoyl)piperazin-1-ylmethyl]-2-(2-furyl)[1,2,4]triazolo[1,5-c]pyrimidine obtained in Example 52.
Melting point: 185-187°C
$^1$H NMR ($CDCl_3$, δ, ppm): 2.65 (t, J = 5.1 Hz, 4H), 3.83 (t, J = 5.1 Hz, 4H), 3.92 (s, 2H), 5.94 (s, 2H), 6.46 (dd, J = 1.8, 3.5 Hz, 1H), 6.59 (dd, J = 1.7, 3.3 Hz, 1H), 6.97 (dd, J = 1.0, 3.5 Hz, 1H), 7.24 (d, J = 3.3 Hz, 1H), 7.46 (dd, J = 1.0, 1.8 Hz, 1H), 7.63 (d, J = 1.7 Hz, 1H), 7.87 (s, 1H)

Example 54

5-(3,4-Dimethoxybenzylamino)-2-(2-furyl)-8-[4-(2-methylphenyl)piperazin-1-ylmethyl][1,2,4]triazolo[1,5-c]pyrimidine (Compound 54)

The subject compound (quantitative) was obtained as a pale brown oily matter from 5-(3,4-dimethoxybenzylamino)-8-formyl-2-(2-furyl)[1,2,4]triazolo[1,5-c]pyrimidine and 1-(2-methylphenyl)piperazine in a manner similar to that in Example 1.
$^1$H NMR ($CDCl_3$, δ, ppm): 2.29 (s, 3H), 2.77 (t, J = 4.7 Hz, 4H), 2.97 (t, J = 4.7 Hz, 4H), 3.89 (s, 3H), 3.89 (s, 3H), 3.92 (s, 2H), 4.76 (d, J = 5.6 Hz, 2H), 6.43 (t, J = 5.6 Hz, 1H), 6.57 (dd, J = 1.7, 3.3Hz, 1H), 6.85 (d, J = 8.1 Hz, 1H), 6.96 (s, 1H), 6.97 (d, J = 8.1 Hz, 1H), 6.99-7.03 (m, 2H), 7.13-7.18 (m, 2H), 7.23 (dd, J = 1.0, 3.3 Hz, 1H), 7.60 (dd, J = 1.0, 1.7 Hz, 1H), 7.98 (s, 1H)

Example 55

5-Amino-2-(2-furyl)-8-[4-(2-methylphenyl)piperazin-1-ylmethyl][1,2,4]triazolo[1,5-c]pyrimidine (Compound 55)

In a manner similar to that in Example 2, the subject compound (81%) was obtained as white crystals from 5-(3,4-dimethoxybenzylamino)-2-(2-furyl)-8-[4-(2-methylphenyl)piperazin-1-ylmethyl][1,2,4]triazolo[1,5-c]pyrimidine obtained in Example 54.
Melting point: 204-206°C
$^1$H NMR ($CDCl_3$, δ, ppm) : 2.29 (s, 3H) , 2.76 (t, J = 4.6 Hz, 4H), 2.97 (t, J = 4.6 Hz, 4H), 3.92 (s, 2H), 6.05 (s, 2H), 6.58 (dd, J = 1.7, 3.3 Hz, 1H), 6.93-7.04 (m, 2H), 7.12-7.18 (m, 2H), 7.27 (d, J = 3.3 Hz, 1H), 7.63 (d, J = 1.7 Hz, 1H), 7.92 (s, 1H)

Example 56

8-[4-(4-Chlorophenyl)piperazin-1-ylmethyl]-5-(3,4-dimethoxybenzylamino)-2-(2-furyl)[1,2,4]triazolo[1,5-c]pyrimidine (Compound 56)

The subject compound (quantitative) was obtained as a pale brown oily matter from 5-(3,4-dimethoxybenzylamino)-8-formyl-2-(2-furyl)[1,2,4]triazolo[1,5-c]pyrimidine and 1-(4-chlorophenyl)piperazine in a manner similar to that in Example 1.
$^1$H NMR ($CDCl_3$, δ , ppm) : 2.76 (t, J = 4.8 Hz, 4H), 3.19 (t, J = 4.8 Hz, 4H), 3.88 (s, 3H), 3.88 (s, 3H), 3.90 (s, 2H), 4.76 (d, J = 5.6 Hz, 2H), 6.43 (t, J = 5.6 Hz, 1H), 6.57 (dd, J = 1.7, 3.3 Hz, 1H), 6.81-6.87 (m, 5H) , 6.94-7.00 (m, 2H), 7.23 (d, J = 3.3 Hz, 1H), 7.60 (d, J = 1.7 Hz, 1H), 7.96 (s, 1H)

Example 57

5-Amino-8-[4-(4-chlorophenyl)piperazin-1-ylmethyl)-2-(2-furyl) [1,2,4]triazolo[1,5-c]pyrimidine (Compound 57)

In a manner similar to that in Example 2, the subject compound (62%) was obtained as white crystals from 8-[4-(4-chlorophenyl)piperazin-1-ylmethyl]-5-(3,4-dimethoxybenzylamino)-2-(2-furyl)[1,2,4]triazolo[1,5-c]pyrimidine obtained in Example 56.
Melting point: 239-240°C
$^1$H NMR ($CDCl_3$, δ, ppm): 2.75 (t, J = 4.9 Hz, 4H), 3.19 (t, J = 4.9 Hz, 4H), 3.90 (s, 2H), 5.86 (s, 2H), 6.58 (dd, J = 1.8,

3.6 Hz, 1H), 6.82 (s, J = 9.1 Hz, 2H), 7.18 (d, J = 9.1 Hz, 2H), 7.25 (d, J = 3.6 Hz, 1H), 7.63 (d, J = 1.8 Hz, 1H), 7.91 (s, 1H)

Example 58

8-[4-(2-Cyanophenyl)piperazin-1-ylmethyl]-5-(3,4-dimethoxybenzylamino)-2-(2-furyl) [1,2,4]triazolo[1,5-c]pyrimidine (Compound 58)

The subject compound (quantitative) was obtained as a pale brown oily matter from 5-(3,4-dimethoxybenzylamino)-8-formyl-2-(2-furyl)[1,2,4]triazolo[1,5-c]pyrimidine and 1-(2-cyanophenyl)piperazine in a manner similar to that in Example 1.
$^1$H NMR ($CDCl_3$, $\delta$, ppm): 2.82 (t, J = 4.6 Hz, 4H), 3.27 (t, J = 4.6 Hz, 4H), 3.89 (s, 3H), 3.89 (s, 3H), 3.93 (s, 2H), 4.76 (d, J = 5.6 Hz, 2H), 6.43 (t, J = 5.6 Hz, 1H), 6.57 (dd, J = 1.7, 3.3 Hz, 1H), 6.85 (d, J = 8.1 Hz, 1H), 6.96 (s, 1H), 6.97 (d, J = 8.1 Hz, 1H), 7.24 (d, J = 3.3 Hz, 1H), 7.44-7.57 (m, 4H), 7.60 (d, J = 1.7 Hz, 1H), 7.95 (s, 1H)

Example 59

5-Amino-8-[4-(2-cyanophenyl)piperazin-1-ylmethyl]-2-(2-furyl) [1,2,4]triazolo[1,5-c]pyrimidine (Compound 59)

In a manner similar to that in Example 2, the subject compound (77%) was obtained as white crystals from 8-[4-(2-cyanophenyl)piperazin-1-ylmethyl]-5-(3,4-dimethoxybenzylamino)-2-(2-furyl) [1,2,4]triazolo[1,5-c]pyrimidine obtained in Example 58.
Melting point: 245-246°C
$^1$H NMR ($CDCl_3$, $\delta$, ppm): 2.82 (t, J = 4.8 Hz, 4H), 3.27 (t, J = 4.8 Hz, 4H), 3.92 (s, 2H), 5.90 (s, 2H), 6.59 (dd, J = 1.7, 3.3 Hz, 1H), 6.96-7.02 (m, 2H), 7.27 (d, J = 3.3 Hz, 1H), 7.43-7.57 (m, 2H), 7.63 (d, J = 1.7 Hz, 1H), 7.89 (s, 1H)

Example 60

5-(3,4-Dimethoxybenzylamino)-2-(2-furyl)-8-[4-(3-phenylpropyl)piperazin-1-ylmethyl] [1,2,4]triazolo[1,5-c]pyrimidine (Compound 60)

The subject compound (90%) was obtained as a pale brown oily matter from 5-(3,4-dimethoxybenzylamino)-8-formyl-2-(2-furyl)[1,2,4]triazolo[1,5-c]pyrimidine and 1-(3-phenylpropyl)piperazine in a manner similar to that in Example 1.
$^1$H NMR ($CDCl_3$, $\delta$, ppm): 1.75-1.87 (m, 2H), 2.35-2.40 (m, 2H), 2.51 (s, 2H), 2.59-2.65 (m, 8H), 3.85 (s, 2H), 3.88 (s, 3H), 3.88 (s, 3H), 4.75 (d, J = 5.6 Hz, 2H), 6.40 (t, J = 5.6 Hz, 1H), 6.56 (dd, J = 1.7, 3.3 Hz, 1H), 6.85 (d, J = 8.1 Hz, 1H), 6.95 (s, 1H), 6.96 (d, J = 8.1 Hz, 1H), 7.16-7.19 (m, 3H), 7.21 (d, J = 3.3 Hz, 1H) , 7.22-7.24 (m, 2H), 7.60 (d, J = 1.7 Hz, 1H), 7.92 (s, 1H)

Example 61

5-Amino-2-(2-furyl)-8-[4-(3-phenylpropyl)piperazin-1-ylmethyl][1,2,4]triazolo[1,5-c]pyrimidine (Compound 61)

In a manner similar to that in Example 2, the subject compound (88%) was obtained as white crystals from 5-(3,4-dimethoxybenzylamino)-2-(2-furyl)-8-[4-(3-phenylpropyl)piperazin-1-ylmethyl] [1,2,4]triazolo[1,5-c]pyrimidine obtained in Example 60.
Melting point: 150-151°C
$^1$H NMR ($CDCl_3$, $\delta$ , ppm): 1.78-1.87 (m, 2H), 2.35-2.40 (m, 2H), 2.48-2.50 (m, 4H) , 2.59-2.65 (m, 6H), 3.85 (s, 2H), 3.92 (s, 2H), 6.58 (dd, J = 1.7, 3.5 Hz, 1H), 7.16-7.26 (m, 5H), 7.29 (d, J = 3.5 Hz, 1H), 7.62 (d, J = 1.7 Hz, 1H), 7.86 (s, 1H)

Example 62

5-(3,4-Dimethoxybenzylamino)-2-(2-furyl)-8-[4-(3-trifluoromethylphenyl)piperazin-1-ylmethyl][1,2,4]triazolo[1,5-c] pyrimidine (Compound 62)

The subject compound (quantitative) was obtained as a pale brown oily matter from 5-(3,4-dimethoxybenzylamino)-8-formyl-2-(2-furyl)[1,2,4]triazolo[1,5-c]pyrimidine and 1-(3-trifluoromethylphenyl)piperazine in a manner similar to that in Example 1.
$^1$H NMR ($CDCl_3$, $\delta$, ppm): 2.77 (t, J = 5.0 Hz, 4H), 3.27 (t, J = 5.0 Hz, 4H), 3.89 (s, 3H), 3.89 (s, 3H), 3.91 (s, 2H), 4.76

(d, J = 5.6 Hz, 2H), 6.44 (t, J = 5.6 Hz, 1H), 6.57 (dd, J = 1.8, 3.5 Hz, 1H), 6.85 (d, J = 8.1 Hz, 1H), 6.96 (s, 1H), 6.97 (d, J = 8.1 Hz, 1H), 7.03-7.10 (m, 3H), 7.23 (d, J = 3.5 Hz, 1H), 7.30-7.36 (m, 1H), 7.61 (d, J = 1.8 Hz, 1H), 7.97 (s, 1H)

Example 63

5-Amino-2-(2-furyl)-8-[4-(3-trifluoromethylphenyl)piperazin-1-ylmethyl][1,2,4]triazolo[1,5-c]pyrimidine (Compound 63)

In a manner similar to that in Example 2, the subject compound (94%) was obtained as white crystals from 5-(3,4-dimethoxybenzylamino)-2-(2-furyl)-8-[4-(3-trifluoromethylphenyl)piperazin-1-ylmethyl][1,2,4]triazolo[1,5-c]pyrimidine obtained in Example 62.
Melting point: 205-206°C
$^1$H NMR ($CDCl_3$, δ , ppm) : 2.76 (t, J = 4.9 Hz, 4H), 3.27 (t, J = 4.9 Hz, 4H), 3.91 (s, 2H), 5.85 (s, 2H), 6.59 (dd, J = 1.8, 3.6 Hz, 1H), 7.03-7.10 (m, 3H), 7.25 (d, J = 3.6 Hz, 1H), 7.27-7.36 (m, 1H) , 7.63 (d, J = 1.8 Hz, 1H), 7.91 (s, 1H)

Example 64

5-(3,4-Dimethoxybenzylamino)-2-(2-furyl)-8-(7-methoxy-1,2,3,4-tetrahydroisoquinolin-2-ylmethyl)[1,2,4]triazolo[1,5-c]pyrimidine (Compound 64)

The subject compound (98%) was obtained as a pale brown oily matter from 5-(3,4-dimethoxybenzylamino)-8-formyl-2-(2-furyl)[1,2,4]triazolo[1,5-c]pyrimidine and 7-methoxy-1,2,3,4-tetrahydroisoquinoline in a manner similar to that in Example 1.
$^1$H NMR ($CDCl_3$, δ, ppm): 2.87-2.88 (m, 4H), 3.75 (s, 3H), 3.76 (s, 2H), 3.88 (s, 3H), 3.88 (s, 3H), 4.00 (s, 2H), 4.76 (d, J = 5.6 Hz, 2H), 6.41 (t, J = 5.6 Hz, 1H), 6.55 (dd, J = 1.8, 3.6 Hz, 1H), 6.70 (d, J = 8.2 Hz, 1H), 6.85 (d, J = 8.1 Hz, 1H), 6.94 (s, 1H), 6.95 (s, 1H), 6.96 (d, J = 8.1 Hz, 1H), 7.00 (d, J = 8.2 Hz, 1H), 7.22 (d, J = 3.6 Hz, 1H), 7.60 (d, J = 1.8 Hz, 1H), 8.02 (s, 1H)

Example 65

5-Amino-2-(2-furyl)-8-(7-methoxy-1,2,3,4-tetrahydroisoquinolin-2-ylmethyl)[1,2,4]triazolo[1,5-c]pyrimidine (Compound 65)

In a manner similar to that in Example 2, the subject compound (81%) was obtained as white crystals from 5-(3,4-dimethoxybenzylamino)-2-(2-furyl)-8-(7-methoxy-1,2,3,4-tetrahydroisoquinolin-2-ylmethyl)[1,2,4]triazolo[1,5-c]pyrimidine obtained in Example 64.
Melting point: 168-170°C
$^1$H NMR ($CDCl_3$, δ, ppm): 2.85-2.87 (m, 4H), 3.75 (s, 3H), 3.75 (s, 2H), 4.00 (s, 2H), 5.95 (s, 2H), 6.55 (s, 1H), 6.59 (dd, J = 1.8, 3.5 Hz, 1H), 6.72 (d, J = 8.4 Hz, 1H), 7.01 (d, J = 8.4 Hz, 1H), 7.25 (d, J = 3.5 Hz, 1H), 7.63 (d, J = 1.8 Hz, 1H), 7.96 (s, 1H)

Example 66

5-(3,4-Dimethoxybenzylamino)-2-(2-furyl)-8-(8-methoxy-1,2,3,4-tetrahydroisoquinolin-2-ylmethyl) [1,2,4]triazolo[1,5-c]pyrimidine (Compound 66)

The subject compound (quantitative) was obtained as a pale brown oily matter from 5-(3,4-dimethoxybenzylamino)-8-formyl-2- (2-furyl) [1,2,4]triazolo[1,5-c]pyrimidine and 8-methoxy-1,2,3,4-tetrahydroisoquinoline in a manner similar to that in Example 1.
$^1$H NMR ($CDCl_3$, δ, ppm): 2.72-2.90 (m, 4H), 3.75 (s, 2H), 3.79 (s, 3H), 3.88 (s, 3H), 3.88 (s, 3H), 4.03 (s, 2H), 4.76 (d, J = 5.6 Hz, 2H), 6.40 (t, J = 5.6 Hz, 1H), 6.57 (dd, J = 1.8, 3.6 Hz, 1H), 6.65 (d, J = 8.2 Hz, 1H), 6.72 (d, J = 8.2 Hz, 1H), 6.85 (d, J = 8.1 Hz, 1H), 6.96 (s, 1H), 6.97 (d, J = 8.1 Hz, 1H), 7.10 (dd, J = 8.2, 8.2 Hz, 1H), 7.22 (d, J = 3.6 Hz, 1H), 7.59 (d, J = 1.8 Hz, 1H), 8.03 (s, 1H)

Example 67

5-Amino-2-(2-furyl)-8-(8-methoxy-1,2,3,4-tetrahydroisoquinolin-2-ylmethyl)[1,2,4]triazolo[1,5-c]pyrimidine (Compound 67)

In a manner similar to that in Example 2, the subject compound (81%) was obtained as white crystals from 5-(3,4-dimethoxybenzylamino)-2-(2-furyl)-8-(8-methoxy-1,2,3,4-tetrahydroisoquinolin-2-ylmethyl)[1,2,4]triazolo[1,5-c]pyrimidine obtained in Example 66.
Melting point: 210-212°C
$^1$H NMR ($CDCl_3$, δ, ppm): 2.82-2.91 (m, 4H), 3.74 (s, 2H), 3.78 (s, 3H), 4.01 (s, 2H), 5.99 (s, 2H), 6.58 (dd, J = 1.7, 3.5 Hz, 1H), 6.65 (d, J = 8.1 Hz, 1H), 6.72 (d, J = 8.1 Hz, 1H), 7.10 (dd, J = 8.1, 8.1 Hz, 1H), 7.25 (d, J = 3.5 Hz, 1H), 7.62 (d, J = 1.7 Hz, 1H), 7.98 (s, 1H)

Example 68

5-(3,4-Dimethoxybenzylamino)-2-phenyl-8-(4-phenylpiperazin-1-ylmethyl) [1,2,4]triazolo[1,5-c]pyrimidine (Compound 68)

The subject compound (75%) was obtained as a white powder from 5-(3,4-dimethoxybenzylamino)-8-formyl-2-phenyl[1,2,4]triazolo[1,5-c]pyrimidine obtained in Reference Example 7 and 1-phenylpiperazine in a manner similar to that in Example 1.
Melting point: 155-156°C
$^1$H NMR (DMSO-$d_6$, δ, ppm): 2.62 (m, 4H), 3.12 (m, 4H), 3.32 (s, 6H), 3.74 (s, 2H), 4.67 (d, J = 5.8 Hz, 2H), 6.75 (t, J = 5.8 Hz, 1H), 6.87-6.96 (m, 4H), 7.08-7.18 (m, 3H), 7.54-7.58 (m, 3H), 7.88 (s, 1H), 8.24-8.27 (m, 2H), 8.58 (t, J = 7.3 Hz, 1H)

Example 69

5-Amino-2-phenyl-8-(4-phenylpiperazin-1-ylmethyl)[1,2,4]triazolo[1,5-c]pyrimidine (Compound 69)

In a manner similar to that in Example 2, the subject compound (83%) was obtained as a white powder from 5-(3,4-dimethoxybenzylamino)-2-phenyl-8-(4-phenylpiperazin-1-ylmethyl) [1,2,4]triazolo(1,5-c]pyrimidine obtained in Example 68.
Melting point: 185-187°C
$^1$H NMR ($CDCl_3$, δ , ppm): 2.79 (t, J = 4.9 Hz, 4H), 3.24 (t, J = 4.9 Hz, 4H) , 3.97 (s, 2H), 6.14 (brs, 2H), 6.77-7.00 (m, 3H), 7.21-7.28 (m, 2H), 7.47-7.53 (m, 3H), 7.88 (s, 1H), 8.27-8.32 (m, 2H)

Example 70

5-(3,4-Dimethoxybenzylamino)-8-(4-phenylpiperazin-1-ylmethyl)-2-(2-thienyl)[1,2,4]triazolo[1,5-c]pyrimidine (Compound 70)

The subject compound (79%) was obtained as a white powder from 5-(3,4-dimethoxybenzylamino)-8-formyl-2-(2-thienyl) [1,2,4]triazolo[1,5-c]pyrimidine obtained in Reference Example 12 and 1-phenylpiperazine in a manner similar to that in Example 1.
Melting point: 186-187°C
$^1$H NMR ($CDCl_3$, δ, ppm): 2.77 (t, J = 4.9 Hz, 4H), 3.23 (t, J = 4.9 Hz, 4H), 3.88 (s, 6H), 3.90 (s, 2H), 4.77 (d, J = 5.9 Hz, 2H), 6.38 (t, J = 5.9 Hz, 1H), 6.88-7.00 (m, 6H), 7.14 (dd, J = 3.8, 5.1 Hz, 1H), 7.25 (t, J = 8.1 Hz, 2H), 7.44 (dd, J = 1.3, 5.1 Hz, 1H), 7.89 (dd, J = 1.3, 3.8 Hz, 1H), 7.90 (s, 1H)

Example 71

5-Amino-8-(4-phenylpiperazin-1-ylmethyl)-2-(2-thienyl)[1,2,4]triazolo[1,5-c]pyrimidine (Compound 71)

In a manner similar to that in Example 2, the subject compound (60%) was obtained as a white powder from 5-(3,4-dimethoxybenzylamino)-8-(4-phenylpiperazin-1-ylmethyl)-2-(2-thienyl)[1,2,4]triazolo[1,5-c]pyrimidine obtained in Example 70.
Melting point: 203-204°C

$^1H$ NMR (DMSO-$d_6$, δ, ppm): 2.80-3.18 (m, 4H), 3.60-3.98 (m, 4H), 4.40-4.62 (m, 2H), 6.85 (t, J = 7.3 Hz, 1H), 6.99 (d, J = 8.1 Hz, 2H), 7.22-7.24 (m, 2H), 7.27 (dd, J = 3.8, 5.1 Hz, 1H), 7.80 (dd, J = 1.3, 5.1 Hz, 1H), 7.88 (dd, J = 1.3, 3.8 Hz, 1H), 8.04 (s, 1H), 8.24 (brs, 2H)

Example 72

5-(3,4-Dimethoxybenzylamino)-2-(3-furyl)-8-(4-phenylpiperazin-1-ylmethyl) [1,2,4]triazolo[1,5-c]pyrimidine (Compound 72)

The subject compound (70%) was obtained as a white powder from 5-(3,4-dimethoxybenzylamino)-8-formyl-2-(3-furyl)[1,2,4]triazolo[1,5-c]pyrimidine obtained in Reference Example 19 and 1-phenylpiperazine in a manner similar to that in Example 1.
Melting point: 167-168°C
$^1H$ NMR ($CDCl_3$, δ, ppm): 2.77 (t, J = 4.9 Hz, 4H), 3.24 (t, J = 4.9 Hz, 4H), 3.87-3.89 (m, 8H), 4.77 (d, J = 5.8 Hz, 2H), 6.34 (t, J = 5.8 Hz, 1H), 6.82-7.02 (m, 7H), 7.12-7.21 (m, 2H), 7.51 (dd, J = 1.7, 1.8 Hz, 1H), 8.19 (s, 1H), 8.20 (dd, J = 0.8, 1.7 Hz, 1H)

Example 73

5-Amino-2-(3-furyl)-8-(4-phenylpiperazin-1-ylmethyl)[1,2,4]triazolo[1,5-c]pyrimidine (Compound 73)

In a manner similar to that in Example 2, the subject compound (32%) was obtained as a white powder from 5-(3,4-dimethoxybenzylamino)-2-(3-furyl)-8-(4-phenylpiperazin-1-ylmethyl)[1,2,4]triazolo[1,5-c]pyrimidine obtained in Example 72.
Melting point: 209-210°C
$^1H$ NMR ($CDCl_3$, δ, ppm) : 2.58-2.62 (m, 4H), 3.28-3.33 (m, 4H), 3.73 (s, 2H), 6.75 (t, J = 7.2 Hz, 1H), 6.90 (d, J = 7.9 Hz, 2H), 7.00 (dd, J = 0.8, 1.8 Hz, 1H), 7.15-7.22 (m, 2H), 7.79 (brs, 2H), 7.80 (s, 1H), 7.86 (dd, J = 1.7, 1.8 Hz, 1H), 8.38 (dd, J = 0.8, 1.7 Hz, 1H)

Example 74

5-(3,4-Dimethoxybenzylamino)-2-phenyl-8-(1,2,3,4-tetrahydroisoquinolin-2-ylmethyl) [1,2,4]triazolo[1,5-c]pyrimidine (Compound 74)

The subject compound (66%) was obtained as a white powder from 5-(3,4-dimethoxybenzylamino)-8-formyl-2-phenyl[1,2,4]triazolo[1,5-c]pyrimidine obtained in Reference Example 7 and 1,2,3,4-tetrahydroisoquinoline in a manner similar to that in Example 1.
Melting point: 142-143°C
$^1H$ NMR ($CDCl_3$, δ, ppm): 2.92-2.96 (m, 4H), 3.83 (s, 2H), 3.89 (s, 6H), 4.04 (s, 2H), 4.79 (d, J = 5.8 Hz, 2H), 6.41 (t, J = 5.8 Hz, 1H), 6.87 (d, J = 8.2 Hz, 1H), 6.97-7.02 (m, 3H), 7.10-7.12 (m, 3H), 7.44-7.50 (m, 3H), 8.01 (s, 1H), 8.25-8.30 (m, 2H)

Example 75

5-Amino-2-phenyl-8-(1,2,3,4-tetrahydroisoquinolin-2-ylmethyl) [1,2,4]triazolo[1,5-c]pyrimidine (Compound 75)

In a manner similar to that in Example 2, the subject compound (39%) was obtained as a white powder from 5-(3,4-dimethoxybenzylamino)-2-phenyl-8-(1,2,3,4-tetrahydroisoquinolin-2-ylmethyl) [1,2,4]triazolo[1,5-c]pyrimidine obtained in Example 74.
Melting point: 186-187°C
$^1H$ NMR (DMSO-$d_6$, δ , ppm) : 2.76-2.82 (m, 4H) , 3.65 (s, 2H), 3.84 (s, 2H), 7.00-7.12 (m, 5H), 7.24 (dd, J = 3.3, 4.9 Hz, 1H), 7.77 (dd, J = 1.1, 4.9 Hz, 1H), 7.78 (brs, 2H), 7.81-7.86 (m, 2H), 7.84 (s, 1H)

Example 76

5-(3,4-Dimethoxybenzylamino)-8-(1,2,3,4-tetrahydroisoquinolin-2-ylmethyl)-2-(2-thienyl)[1,2,4]triazolo[1,5-c]pyrimidine (Compound 76)

The subject compound (85%) was obtained as a white powder from 5-(3,4-dimethoxybenzylamino)-8-formyl-2-(2-thienyl) [1,2,4]triazolo[1,5-c]pyrimidine obtained in Reference Example 12 and 1,2,3,4-tetrahydroisoquinoline in a manner similar to that in Example 1.
Melting point: 139-140°C
$^1$H NMR ($CDCl_3$, δ, ppm): 2.85-2.94 (m, 4H), 3.81 (s, 2H), 3.88 (s, 6H), 4.00 (s, 2H), 4.77 (d, J = 5.8 Hz, 2H), 6.37 (t, J = 5.8 Hz, 1H), 6.85-6.90 (m, 1H), 6.97-7.02 (m, 3H), 7.05-7.16 (m, 4H), 7.44 (dd, J = 1.3, 5.1 Hz, 1H), 7.89 (dd, J = 1.3, 3.8 Hz, 1H), 7.99 (s, 1H)

Example 77

5-Amino-8-(1,2,3,4-tetrahydroisoquinolin-2-ylmethyl)-2-(2-thienyl)[1,2,4]triazolo[1,5-c]pyrimidine (Compound 77)

In a manner similar to that in Example 2, the subject compound (40%) was obtained as a white powder from 5-(3,4-dimethoxybenzylamino)-8-(1,2,3,4-tetrahydroisoquinolin-2-ylmethyl)-2-(2-thienyl)[1,2,4]triazolo[1,5-c]pyrimidine obtained in Example 76.
Melting point: 192-193°C
$^1$H NMR (DMSO-$d_6$, δ, ppm) : 2.77-2.79 (m, 4H), 3.67 (s, 2H), 3.87 (s, 2H), 7.01-7.10 (m, 2H), 7.52-7.58 (m, 2H), 7.82 -7.86 (m, 3H), 8.20-8.27 (m, 2H)

Example 78

9-[5-(3,4-Dimethoxybenzylamino)-2-(2-furyl) [1,2,4]triazolo[1,5-c]pyrimidin-8-ylmethyl]-1-methyl-3-oxa-1,9-diazaspiro [5.5]undecan-2-one (Compound 78)

In a manner similar to that in Example 1, the subject compound (35%) was obtained from 5-(3,4-dimethoxybenzylamino)-8-formyl-2-(2-furyl)[1,2,4]triazolo[1,5-c]pyrimidine and 1-methyl-3-oxa-1,9-diazaspiro[5.5]undecan-2-one obtained in Reference Example 51.
$^1$H NMR ($CDCl_3$, δ, ppm): 1.42-1.53 (m, 2H), 1.99-2.07 (m, 2H), 2.18-2.35 (m, 4H), 2.89-3.03 (m, 2H), 2.92 (s, 3H), 3.84 (s, 2H), 3.89 (s, 3H), 3.89 (s, 3H), 4.11-4.19 (m, 2H), 4.76 (d, J = 5.8 Hz, 2H), 6.44 (t, J = 5.8 Hz, 1H), 6.57 (dd, J = 1.7, 3.4 Hz, 1H), 6.86 (d, J = 8.1 Hz, 1H), 6.95 (d, J = 1.7 Hz, 1H), 6.97 (dd, J = 1.7, 8.1 Hz, 1H), 7.21 (d, J = 3.4 Hz, 1H), 7.61 (d, J = 1.7 Hz, 1H), 7.92 (s, 1H)

Example 79

9-[5-Amino-2-(2-furyl)[1,2,4]triazolo[1,5-c]pyrimidin-8-ylmethyl]-1-methyl-3-oxa-1,9-diazaspiro[5.5]undecan-2-one (Compound 79)

In a manner similar to that in Example 2, the subject compound (58%) was obtained as a white powder from 9-[5-(3,4-dimethoxybenzylamino)-2- (2-furyl) [1,2,4]triazolo [1, 5-c]pyrimidin-8-ylmethyl]-1-methyl-3-oxa-1,9-diazaspiro[5.5]undecan-2-one obtained in Example 78.
Melting point: 148.4-152.6°C
$^1$H NMR (DMSO-$d_6$, δ , ppm): 1.39-1.52 (m, 2H), 1.91-2.27 (m, 6H), 2.72-2.87 (m, 2H), 2.75 (s, 3H), 3.71 (s, 2H), 4.06 (t, J = 4.9 Hz, 2H), 6.72 (dd, J = 1.7, 3.5 Hz, 1H), 7.19 (d, J = 3.5 Hz, 1H) , 7.79 (d, J = 1.7 Hz, 1H), 7.87 (brs, 2H), 7.93 (s, 1H)

Example 80

9-[5-(3,4-Dimethoxybenzylamino)-2-(2-furyl) [1,2,4]triazolo[1,5-c]pyrimidin-8-ylmethyl]-1,3-dimethyl-1,3,9-triazaspiro [5.5]undecan-2-one (Compound 80)

In a manner similar to that in Example 1, the subject compound (98%) was obtained as a pale brown oily matter from 5-(3,4-dimethoxybenzylamino)-8-formyl-2-(2-furyl)[1,2,4]triazolo[1,5-c]pyrimidine and 1,3-dimethyl-1,3,9-triazaspiro[5.5]undecan-2-one obtained in Reference Example 54.

$^1H$ NMR ($CDCl_3$, δ, ppm): 1.39-1.57 (m, 2H), 1.76-2.42 (m, 6H), 2.77-3.08 (m, 2H), 2.92 (s, 3H), 2.93 (s, 3H), 3.08 - 3.28 (m, 2H), 3.83 (s, 2H), 3.88 (s, 3H), 3.88 (s, 3H), 4.70-4.85 (m, 2H), 6.39-6.52 (m, 1H), 6.52-6.64 (m, 1H), 6.78-7.07 (m, 3H), 7.14-7.36 (m, 1H), 7.53-7.69 (m, 1H), 7.86-8.04 (m, 1H)

Example 81

9-[5-Amino-2-(2-furyl)[1,2,4]triazolo[1,5-c]pyrimidin-8-ylmethyl]-1,3-dimethyl-1,3,9-triazaspiro[5.5]undecan-2-one (Compound 81)

In a manner similar to that in Example 2, the subject compound (46%) was obtained as pale brown crystals from 9-[5-(3,4-dimethoxybenzylamino)-2-(2-furyl)[1,2,4]triazolo[1,5-c]pyrimidin-8-ylmethyl]-1,3-dimethyl-1,3,9-triazaspiro[5.5]undecan-2-one obtained in Example 80.
$^1H$ NMR (DMSO-$d_6$, δ , ppm): 1.33-1.48 (m, 2H), 1.81-2.07 (m, 4H), 2.23-2.31 (m, 2H), 2.65-2.91 (m, 2H), 2.73 (s, 3H), 2.76 (s, 3H), 3.03-3.18 (m, 2H), 3.69 (s, 2H), 6.72 (d, J = 1.7, 3.4 Hz, 1H), 7.19 (d, J = 3.4 Hz, 1H), 7.78 (d, J = 1.7 Hz, 1H), 7.85 (brs, 2H), 7.93 (s, 1H)

Example 82

5-(3,4-Dimethoxybenzylamino)-2-(2-furyl)-8-(1-phenyl-1,3,8-triazaspiro[4.5]decan-4-on-8-ylmethyl) [1,2,4]triazaspiro [1,5-c]pyrimidine (Compound 82)

The subject compound (70%) was obtained as a pale brown oily matter from 5-(3,4-dimethoxybenzylamino)-8-formyl-2-(2-furyl) [1,2,4]triazolo[1,5-c]pyrimidine and 1-phenyl-1,3,8-triazaspiro[4,5]decan-4-one in a manner similar to that in Example 1.
$^1H$ NMR ($CDCl_3$, δ , ppm) : 1.74 (d, J = 13.5 Hz, 2H) , 2.72-2.77 (m, 4H), 2.94-2.99 (m, 4H), 3.88 (s, 3H), 3.88 (s, 3H), 3.92 (s, 2H), 4.76 (d, J = 5.6 Hz, 2H), 6.40 (t, J = 5.6 Hz, 1H), 6.57 (dd, J = 1.8, 3.5 Hz, 1H), 6.84 (t, J = 13.5 Hz, 1H), 6.87-6.99 (m, 8H), 7.23 (d, J = 3.5 Hz, 1H), 7.59 (d, J = 1.8 Hz, 1H), 8.00 (s, 1H)

Example 83

5-Amino-2-(2-furyl)-8-(1-phenyl-1,3,8-triazaspiro[4.5]decan-4-on-8-ylmethyl)[1,2,4]triazolo[1,5-c]pyrimidine (Compound 83)

In a manner similar to that in Example 2, the subject compound (87%) was obtained as white crystals from 5-(3,4-dimethoxybenzylamino)-2-(2-furyl)-8-(1-phenyl-1,3,8-triazaspiro[4.5]decan-4-on-8-ylmethyl)[1,2,4]triazolo [1,5-c]pyrimidine obtained in Example 82.
Melting point: 267-269°C
$^1H$ NMR (DMSO-$d_6$, δ, ppm): 1.57 (d, J = 13.5 Hz, 2H), 2.50-2.51 (m, 4H), 2.80-2.83 (m, 4H), 3.75 (s, 2H), 4.57 (s, 2H), 6.72-6.77 (m, 2H), 6.84-6.87 (m, 2H), 7.20 (t, J = 13.5 Hz, 1H), 7.21-7.23 (m, 1H), 7.84 (s, 2H), 7.93 (s, 1H), 7.95 (s, 1H)

Example 84

5-(3,4-Dimethoxybenzylamino)-2-(2-furyl)-8-(1,3,4,6,7,8,9,9a-octahydropyrido[1,2-a]pyrazin-2-ylmethyl)[1,2,4] triazolo[1,5-c]pyrimidine (Compound 84)

The subject compound (92%) was obtained as a pale brown oily matter from 5-(3,4-dimethoxybenzylamino)-8-formyl-2-(2-furyl)[1,2,4]triazolo[1,5-c]pyrimidine and 1,3,4,6,7,8,9,9a-octahydropyrido[1,2-a]pyrazine in a manner similar to that in Example 1.
$^1H$ NMR ($CDCl_3$, δ , ppm) : 1.24-1.26 (m, 2H), 1.48-1.51 (m, 2H), 1.70-1.73 (m, 2H), 2.06-2.09 (m, 4H), 2.35-2.39 (m, 1H), 2.78-2.96 (m, 4H), 3.83 (s, 2H), 3.89 (s, 3H), 3.89 (s, 3H), 4.76 (d, J = 5.6 Hz, 2H), 6.40 (t, J = 5.6 Hz, 1H), 6.56 (dd, J = 1.8, 3.6 Hz, 1H), 6.85 (d, J = 8.1 Hz, 1H), 6.95 (s, 1H), 6.97 (d, J = 8.1 Hz, 1H), 7.21 (d, J = 3.5 Hz, 1H), 7.59 (d, J = 1.8 Hz, 1H), 7.92 (s, 1H)

Example 85

5-Amino-2-(2-furyl)-8-(1,3,4,6,7,8,9,9a-octahydropyrido[1,2-a]pyrazin-2-ylmethyl) [1,2,4]triazolo[1,5-c]pyrimidine (Compound 85)

In a manner similar to that in Example 2, the subject compound (82%) was obtained as white crystals from 5-(3,4-dimethoxybenzylamino)-2-(2-furyl)-8-(1,3,4,6,7,8,9,9a-octahydropyrido[1,2-a]pyrazin-2-ylmethyl)[1,2,4]triazolo[1,5-c]pyrimidine obtained in Example 84.
Melting point: 189-191°C
$^1$H NMR ($CDCl_3$, δ, ppm): 1.24-1.36 (m, 2H), 1.50-1.52 (m, 1H), 1.62-1.72 (m, 2H), 2.01-2.11 (m, 4H), 2.30-2.49 (m, 2H), 2.73-2.94 (m, 4H), 3.82 (s, 2H), 5.95 (s, 2H), 6.57 (dd, J = 1.8, 3.5 Hz, 1H), 7.24 (d, J = 3.5 Hz, 1H), 7.63 (d, J = 1.8 Hz, 1H), 7.86 (s, 1H)

Example 86

5-(3,4-Dimethoxybenzylamino)-2-(2-furyl)-8-([7R,8aS]-7-methoxy-1,2,3,4,6,7,8,8a-octahydropyrrolo[1,2-a]pyrazin-2-ylmethyl) [1,2,4]triazolo[1,5-c]pyrimidine (Compound 86)

The subject compound (91%) was obtained as a pale brown oily matter from 5-(3,4-dimethoxybenzylamino)-8-formyl-2-(2-furyl)[1,2,4]triazolo[1,5-c]pyrimidine and [7R,8aS]-7-methoxy-1,2,3,4,6,7,8,8a-octahydropyrrolo[1,2-a]pyrazine in a manner similar to that in Example 1.
$^1$H NMR ($CDCl_3$, δ, ppm): 1.74-1.82 (m, 1H), 1.95-2.02 (m, 1H), 2.14-2.19 (m, 1H), 2.37-2.46 (m, 4H), 2.93-2.96 (m, 2H), 3.06-3.10 (m, 1H), 3.27 (s, 3H), 3.41-3.47 (m, 1H), 3.89 (s, 3H), 3.89 (s, 3H), 3.90 (s, 2H), 3.93-4.00 (m, 1H), 4.75 (d, J = 5.6 Hz, 2H), 6.42 (t, J = 5.6 Hz, 1H), 6.57 (dd, J = 1.8, 3.5 Hz, 1H), 6.85 (d, J = 8.0 Hz, 1H), 6.95 (s, 1H), 6.97 (d, J = 8.0 Hz, 1H), 7.21 (d, J = 3.5 Hz, 1H), 7.59 (d, J = 1.8 Hz, 1H), 7.93 (s, 1H)

Example 87

5-Amino-2-(2-furyl)-8-([7R,8aS]-7-methoxy-1,2,3,4,6,7,8,8a-octahydropyrrolo[1,2-a]pyrazin-2-ylmethyl)[1,2,4]triazolo [1,5-c]pyrimidine (Compound 87)

In a manner similar to that in Example 2, the subject compound (81%) was obtained as white crystals from 5-(3,4-dimethoxybenzylamino)-2-(2-furyl)-8-([7R,8aS]-7-methoxy-1,2,3,4,6,7,8,8a-octahydropyrrolo[1,2-a]pyrazin-2-ylmethyl)[1,2,4]triazolo[1,5-c]pyrimidine obtained in Example 86.
Melting point: 180-181°C
$^1$H NMR ($CDCl_3$, δ , ppm): 1.54-1.66 (m, 1H), 1.74-1.80 (m, 1H), 1.95-2.02 (m, 1H), 2.14-2.20 (m, 1H), 2.33-2.49 (m, 3H), 2.89-2.96 (m, 2H), 3.05-3.09 (m, 1H), 3.27 (s, 3H), 3.42-3.52 (m, 1H), 3.89 (s, 2H), 3.93-4.00 (m, 1H), 6.06 (s, 2H), 6.58 (dd, J = 1.8, 3.5 Hz, 1H), 7.23 (d, J = 3.5 Hz, 1H), 7.63 (d, J = 1.8 Hz, 1H), 7.87 (s, 1H)

Example 88

5-(3,4-Dimethoxybenzylamino)-2-(2-furyl)-8-(6-methoxy-1,2,3,4-tetrahydroisoquinolin-2-ylmethyl)[1,2,4]triazolo [1,5-c]pyrimidine (Compound 88)

The subject compound (94%) was obtained as a pale brown oily matter from 5-(3,4-dimethoxybenzylamino)-8-formyl-2-(2-furyl)[1,2,4]triazolo[1,5-c]pyrimidine and 6-methoxy-1,2,3,4-tetrahydroisoquinoline in a manner similar to that in Example 1.
$^1$H NMR ($CDCl_3$, δ, ppm) : 2.85-2.89 (m, 4H) , 3.72 (s, 2H) , 3.77 (s, 3H), 3.88 (s, 3H), 3.88 (s, 3H), 3.99 (s, 2H), 4.76 (d, J = 5.6 Hz, 2H), 6.41 (t, J = 5.6 Hz, 1H), 6.56 (dd, J = 1.7, 3.4 Hz, 1H), 6.63-6.70 (m, 2H), 6.85 (d, J = 8.0 Hz, 1H), 6.90-6.94 (m, 1H), 6.96 (s, 1H), 6.97 (d, J = 8.0 Hz, 1H), 7.22 (d, J = 3.4 Hz, 1H), 7.60 (d, J = 1.7 Hz, 1H), 8.01 (s, 1H)

Example 89

5-Amino-2-(2-furyl)-8-(6-methoxy-1,2,3,4-tetrahydroisoquinolin-2-ylmethyl)[1,2,4]triazolo[1,5-c]pyrimidine (Compound 89)

In a manner similar to that in Example 2, the subject compound (76%) was obtained as white crystals from 5-(3,4-dimethoxybenzylamino)-2-(2-furyl)-8-(6-methoxy-1,2,3,4-tetrahydroisoquinolin-2-ylmethyl)[1,2,4]triazolo[1,5-c]pyrimidine obtained in Example 88.
Melting point: 171-173°C
$^1$H NMR ($CDCl_3$, δ, ppm): 2.85-2.92 (m, 4H), 3.72 (s, 2H), 3.76 (s, 3H), 4.00 (s, 2H), 5.95 (s, 2H), 6.58 (dd, J = 1.8, 3.5 Hz, 1H), 6.64 (d, J = 2.6 Hz, 1H), 6.68 (dd, J = 2.6, 8.3 Hz, 1H), 6.92 (d, J = 8.3 Hz, 1H), 7.25 (d, J = 3.5 Hz, 1H), 7.63 (d, J = 1.8 Hz, 1H), 7.96 (s, 1H)

Example 90

5-(3,4-Dimethoxybenzylamino)-2-(2-furyl)-8-(5-methoxy-1,2,3,4-tetrahydroisoquinolin-2-ylmethyl)[1,2,4]triazolo [1,5-c]pyrimidine (Compound 90)

The subject compound (98%) was obtained as a pale brown oily matter from 5-(3,4-dimethoxybenzylamino)-8-formyl-2-(2-furyl) [1,2,4]triazolo[1,5-c]pyrimidine and 5-methoxy-1,2,3,4-tetrahydroisoquinoline in a manner similar to that in Example 1.
$^1$H NMR ($CDCl_3$, δ, ppm): 2.79-2.82 (m, 2H), 2.87-2.92 (m, 2H), 3.76 (s, 2H), 3.81 (s, 3H), 3.88 (s, 3H), 3.88 (s, 3H), 3.99 (s, 2H), 4.76 (d, J = 5.6 Hz, 2H), 6.41 (t, J = 5.6 Hz, 1H), 6.56 (dd, J = 1.7, 3.3 Hz, 1H), 6.65 (dd, J = 8.7, 8.7 Hz, 2H), 6.85 (d, J = 8.0 Hz, 1H), 6.96 (s, 1H), 6.98 (d, J = 8.0 Hz, 1H), 7.08 (dd, J = 8.7, 8.7 Hz, 1H), 7.22 (d, J = 3.3 Hz, 1H), 7.60 (d, J = 1.7 Hz, 1H), 8.01 (s, 1H)

Example 91

5-Amino-2-(2-furyl)-8-(5-methoxy-1,2,3,4-tetrahydroisoquinolin-2-ylmethyl)[1,2,4]triazolo[1,5-c]pyrimidine (Compound 91)

In a manner similar to that in Example 2, the subject compound (87%) was obtained as white crystals from 5-(3,4-dimethoxybenzylamino)-2-(2-furyl)-8-(5-methoxy-1,2,3,4-tetrahydroisoquinolin-2-ylmethyl)[1,2,4]triazolo[1,5-c] pyrimidine obtained in Example 90.
Melting point: 198-200°C
$^1$H NMR ($CDCl_3$, δ, ppm): 2.80 (t, J = 5.6 Hz, 2H), 2.89 (t, J = 5.6 Hz, 2H), 3.76 (s, 2H), 3.81 (s, 2H), 3.99 (s, 3H), 5.98 (s, 2H), 6.58 (dd, J = 1.8, 3.5 Hz, 1H), 6.65 (dd, J = 8.4, 8.4 Hz, 2H), 7.08 (dd, J = 8.4, 8.4 Hz, 1H), 7.25 (d, J = 3.5 Hz, 1H), 7.63 (d, J = 1.8 Hz, 1H), 7.96 (s, 1H)

Example 92

8-[(6R)-1,8-Diaza-4-oxabicyclo-[4.4.0]decan-8-ylmethyl]-5-(3,4-dimethoxybenzylamino)-2-(2-furyl)[1,2,4]triazolo [1,5-c]pyrimidine (Compound 92)

The subject compound (82%) was obtained as a pale brown oily matter from 5-(3,4-dimethoxybenzylamino)-8-formyl-2- (2-furyl) [1,2,4]triazolo[1,5-c]pyrimidine and (6R)-1,8-diaza-4-oxabicyclo[4.4.0]decane in a manner similar to that in Example 1.
$^1$H NMR ($CDCl_3$, δ, ppm) : 1.90-1.97 (m, 1H), 2.34-2.44 (m, 4H), 2.63-2.67 (m, 1H) , 2.73-2.76 (m, 2H), 2.95-2.98 (m, 1H), 3.19-3.27 (m, 1H) , 3.62-3.76 (m, 3H) , 3.84 (s, 2H), 3.89 (s, 3H), 3.89 (s, 3H), 4.76 (d, J = 5.6 Hz, 2H), 6.41 (t, J = 5.6 Hz, 1H), 6.56 (dd, J = 1.8, 3.5 Hz, 1H), 6.85 (d, J = 8.0 Hz, 1H), 6.95 (s, 1H), 6.97 (d, J = 8.0 Hz, 1H), 7.21 (d, J = 3.5 Hz, 1H), 7.60 (d, J = 1.8 Hz, 1H), 7.91 (s, 1H)

Example 93

5-Amino-8-[(6R)-1,8-diaza-4-oxabicyclo[4.4.0]decan-8-ylmethyl]-2-(2-furyl)[1,2,4]triazolo[1,5-c]pyrimidine (Compound 93)

In a manner similar to that in Example 2, the subject compound (56%) was obtained as white crystals from 8-[(6R)-1,8-diaza-4-oxabicyclo[4.4.0]decan-8-ylmethyl]-5-(3,4-dimethoxybenzylamino)-2-(2-furyl)[1,2,4]triazolo [1,5-c]pyrimidine obtained in Example 92.
Melting point: 200-202°C
$^1$H NMR ($CDCl_3$, δ, ppm): 1.90-1.97 (m, 1H), 2.34-2.45 (m, 5H), 2.63-2.76 (m, 3H), 2.94-2.97 (m, 1H), 3.23-3.27 (m, 1H), 3.62-3.72 (m, 2H), 3.83 (s, 2H), 5.92 (s, 2H), 6.58 (dd, J = 1.8, 3.5 Hz, 1H), 7.23 (d, J = 3.5 Hz, 1H), 7.63 (d, J = 1.8 Hz, 1H), 7.86 (s, 1H)

Example 94

8-{4-[2-(3-Chlorophenyl)ethylamino]piperidinomethyl}-5-(3,4-dimethoxybenzylamino)-2-(2-furyl) [1,2,4]triazolo[1,5-c] pyrimidine (Compound 94)

The subject compound (87%) was obtained as a pale brown oily matter from 5-(3,4-dimethoxybenzylamino)-2-(2-furyl)-8-(4-oxopiperidinomethyl)[1,2,4]triazolo[1,5-c]pyrimidine obtained in Reference Example 67 and 2-(3-chlorophenyl)ethylamine in a manner similar to that in Example 1.
$^1$H NMR ($CDCl_3$, $\delta$, ppm): 1.32-1.52 (m, 2H), 1.79-1.91 (m,2H), 2.08-2.21 (m, 2H), 2.41-2.57 (m, 1H), 2.72-3.05 (m, 6H), 3.79 (s, 2H), 3.88 (s, 3H), 3.88 (s, 3H), 4.75 (d, J = 5.7 Hz, 2H), 6.49 (t, J = 5.7 Hz, 1H), 6.56 (dd, J = 1.6, 3.5 Hz, 1H), 6.84 (d, J = 7.8 Hz, 1H), 6.95 (s, 1H), 6.96 (d, J = 7.8 Hz, 1H), 7.03-7.30 (m, 5H), 7.59 (d, J = 1.6 Hz, 1H), 7.91 (s, 1H)

Example 95

5-Amino-8-{4-[2-(3-chlorophenyl)ethylamino] piperidinomethyl}-2-(2-furyl)[1,2,4]triazolo[1,5-c]pyrimidine (Compound 95)

In a manner similar to that in Example 2, the subject compound (49%) was obtained as white crystals from 8-{4-[2-(3-chlorophenyl)ethylamino]piperidinomethyl}-5-(3,4-dimethoxybenzylamino)-2-(2-furyl)[1,2,4]triazolo[1,5-c] pyrimidine obtained in Example 94.
Melting point: 160-162°C
$^1$H NMR ($CDCl_3$, $\delta$, ppm): 1.30-1.50 (m, 2H), 1.78-1.88 (m, 2H), 2.10-2.25 (m, 2H), 2.40-2.62 (m, 1H), 2.64-3.00 (m, 6H), 3.82 (s, 2H), 5.97 (brs, 2H), 6.58 (dd, J = 1.6, 3.2 Hz, 1H), 7.04-7.22 (m, 4H), 7.24 (d, J = 3.2 Hz, 1H), 7.62 (d, J = 1.6 Hz, 1H), 7.87 (s, 1H)

Example 96

5-(3,4-Dimethoxybenzylamino)-2-(2-furyl)-8-[4-(2-pyridylmethylamino)piperidinomethyl][1,2,4]triazolo[1,5-c] pyrimidine (Compound 96)

The subject compound (67%) was obtained as a pale brown oily matter from 5-(3,4-dimethoxybenzylamino)-2-(2-furyl)-8-(4-oxopiperidinomethyl) [1,2,4]triazolo[1,5-c]pyrimidine obtained in Reference Example 67 and 2-aminomethylpyridine in a manner similar to that in Example 1.
$^1$H NMR ($CDCl_3$, $\delta$, ppm): 1.42-1.60 (m, 2H) , 1.83-1. 98 (m, 2H), 2.11-2.25 (m, 2H), 2.47-2.52 (m, 1H), 2.92-3.07 (m, 2H), 3.82 (s, 2H), 3.88 (s, 3H), 3.88 (s, 3H), 3.92 (s, 2H), 4.75 (d, J = 6.8 Hz, 2H), 6.48 (t, J = 6.8 Hz, 1H), 6.58 (dd, J = 1.6, 3.5 Hz, 1H), 6.84 (d, J = 7.8 Hz, 1H), 6.95 (s, 1H) , 6.97 (d, J = 7.8 Hz, 1H), 7.11-7.18 (m, 1H), 7.21 (d, J = 3.5 Hz, 1H), 7.30 (d, J = 7.8 Hz, 1H), 7.56-7.68 (m, 2H), 7.92 (s, 1H), 8.50-8.57 (m, 1H)

Example 97

5-Amino-2-(2-furyl)-8-[4-(2-pyridylmethylamino)piperidinomethyl][1,2,4]triazolo[1,5-c]pyrimidine (Compound 97)

In a manner similar to that in Example 2, the subject compound (47%) was obtained as white crystals from 5-(3,4-dimethoxybenzylamino)-2-(2-furyl)-8-[4-(2-pyridylmethylamino)piperidinomethyl][1,2,4]triazolo[1,5-c]pyrimidine obtained in Example 96.
Melting point: 149-151°C
$^1$H NMR ($CDCl_3$, $\delta$, ppm): 1.45-1.61 (m, 2H), 1.87-2.00 (m, 2H), 2.13-2.25 (m, 2H), 2.47-2.62 (m, 1H), 2.92-3.04 (m, 2H), 3.83 (s, 2H), 3.93 (s, 2H), 5.98 (brs, 2H), 6.58 (dd, J = 1.6, 3.5 Hz, 1H), 7.10-7.20 (m, 1H), 7.24 (dd, J = 0.8, 3.5 Hz, 1H), 7.30 (d, J = 7.8 Hz, 1H), 7.62 (dd, J = 0.8, 1.6 Hz, 1H), 7.58-7.71 (m, 1H), 7.88 (s, 1H), 8.50-8.60 (m, 1H)

Example 98

8-[4-(3-Chlorophenyl)-3-oxopiperazin-1-ylmethyl]-5-(3,4-dimethoxybenzylamino)-2-(2-furyl)[1,2,4]triazolo[1,5-c] pyrimidine (Compound 98)

The subject compound (quantitative) was obtained as a pale brown oily matter from 3-(3,4-dimethoxybenzylamino)-8-formyl-2-(2-furyl) [1,2,4]triazolo[1,5-c]pyrimidine and a known compound (WO00/01691), 1-(3-chlorophenyl)piperazin-2-one, in a manner similar to that in Example 1.

$^1H$ NMR ($CDCl_3$, δ , ppm) : 2.98 (t, J = 3.5 Hz, 2H) , 3.48 (s, 2H), 3.70 (t, J = 3.5 Hz, 2H), 3.88 (s, 3H), 3.89 (s, 3H), 3.93 (s, 2H), 4.77 (d, J = 5.4 Hz, 2H), 6.53 (t, J = 5.4 Hz, 1H), 6.58 (dd, J = 1.9, 3.5 Hz, 1H), 6.80-7.02 (m, 3H), 7.15-7.37 (m, 5H), 7.60 (d, J = 1.9 Hz, 1H), 7.93 (s, 1H)

Example 99

5-Amino-8-[4-(3-chlorophenyl)-3-oxopiperazin-1-ylmethyl]-2-(2-furyl) [1,2,4]triazolo[1,5-c]pyrimidine (Compound 99)

In a manner similar to that in Example 2, the subject compound (52%) was obtained as white crystals from 8-[4-(3-chlorophenyl)-3-oxopiperazin-1-ylmethyl]-5-(3,4-dimethoxybenzylamino)-2-(2-furyl)[1,2,4]triazolo[1,5-c]pyrimidine obtained in Example 98.
Melting point: 218-220°C
$^1H$ NMR ($CDCl_3$, δ, ppm): 2.98 (t, J = 3.5 Hz, 2H), 3.46 (s, 2H), 3.71 (t, J = 3.5 Hz, 2H), 3.96 (s, 2H), 6.02 (brs, 2H), 6.60 (dd, J = 1.9, 3.5 Hz, 1H), 7.15-7.37 (m, 5H), 7.64 (d, J = 1.9 Hz, 1H), 7.89 (s, 1H)

Example 100

5-Amino-2-(2-furyl)-8-[4-(3-pyridylmethylamino)piperidinomethyl][1,2,4]triazolo[1,5-c]pyrimidine (Compound 100)

The subject compound (50%) was obtained as white crystals by preparing 5-(3,4-dimethoxybenzylamino)-2-(2-furyl)-8-[4-(3-pyridylmethylamino)piperidinomethyl] [1,2,4]triazolo[1,5-c]pyrimidine from 5-(3,4-dimethoxybenzylamino)-2-(2-furyl)-8-(4-oxopiperidinomethyl)[1,2,4]triazolo[1,5-c]pyrimidine obtained in Reference Example 67 and 3-aminomethylpyridine in a manner similar to that in Example 1 and subsequently treating the obtained compound without purification in a manner similar to that in Example 2.
Melting point: 149-151°C
$^1H$ NMR ($CDCl_3$, δ, ppm) : 1.40-1.57 (m, 2H) , 1.85-1.97 (m, 2H), 2.12-2.26 (m, 2H), 2.45-2.60 (m, 1H) , 2.92-3.04 (m, 2H), 3.83 (s, 2H), 3.83 (s, 2H), 5.97 (brs, 2H), 6.58 (dd, J = 1.6, 3.2 Hz, 1H), 7.25 (dd, J = 4.6, 7.8 Hz, 1H), 7.25 (d, J = 3.2 Hz, 1H), 7.63 (d, J = 1.6 Hz, 1H), 7.68 (brt, J = 7.8 Hz, 1H), 7.88 (s, 1H), 8.49 (dd, J = 1.6, 4.6 Hz, 1H), 8.55 (d, J = 1.6 Hz, 1H)

Example 101

5-(3,4-Dimethoxybenzylamino)-2-(2-furyl)-8-[4-(4-pyridylmethylamino)piperidinomethyl][1,2,4]triazolo[1,5-c] pyrimidine (Compound 101)

The subject compound (68%) was obtained as a pale brown oily matter from 5-(3,4-dimethoxybenzylamino)-2-(2-furyl)-8-(4-oxopiperidinomethyl) [1,2,4]triazolo[1,5-c]pyrimidine obtained in Reference Example 67 and 4-aminomethylpyridine in a manner similar to that in Example 1.
$^1H$ NMR ($CDCl_3$, δ , ppm): 1.38-1.56 (m, 2H), 1.87-1.96 (m, 2H), 2.10-2.25 (m, 2H), 2.43-2.56 (m, 1H), 2.90-3.04 (m, 2H), 3.82 (s, 2H), 3.84 (s, 2H), 3.90 (s, 3H), 3.90 (s, 3H), 4.75 (d, J = 5.4 Hz, 2H), 6.46 (t, J = 5.4 Hz, 1H), 6.56 (dd, J = 1.9, 3.5 Hz, 1H), 6.84 (d, J = 7.8 Hz, 1H), 6.95 (s, 1H), 6.98 (d, J = 7.8 Hz, 1H), 7.16-7.28 (m, 3H), 7.59 (dd, J = 0.8, 1.9 Hz, 1H), 7.92 (s, 1H), 8.50-8.56 (m, 2H)

Example 102

5-Amino-2-(2-furyl)-8-[4-(4-pyridylmethylamino)piperidinomethyl][1,2,4]triazolo[1,5-c]pyrimidine (Compound 102)

In a manner similar to that in Example 2, the subject compound (70%) was obtained as white crystals from 5-(3,4-dimethoxybenzylamino)-2-(2-furyl)-8-[4-(4-pyridylmethylamino)piperidinomethyl][1,2,4]triazolo[1,5-c]pyrimidine obtained in Example 101.
Melting point: 166-167°C
$^1H$ NMR ($CDCl_3$, δ , ppm) : 1.39-1.57 (m, 2H), 1.83-1.96 (m, 2H), 2.12-2.25 (m, 2H), 2.43-2.58 (m, 1H), 2.93-3.03 (m, 2H), 3.83 (s, 2H), 3.83 (s, 2H), 5.94 (brs, 2H), 6.58 (dd, J = 1.9, 3.5 Hz, 1H), 7.18-7.32 (m, 3H), 7.63 (dd, J = 0.8, 1.9 Hz, 1H), 7.88 (s, 1H), 8.50-8.57 (m, 2H)

Example 103

5-Amino-2-(2-furyl)-8-[4-(3-hydroxyphenyl)piperidinomethyl][1,2,4]triazolo[1,5-c]pyrimidine (Compound 103)

The subject compound (8%) was obtained as white crystals by preparing 5-(3,4-dimethoxybenzylamino)-2-(2-furyl)-8-[4-(3-hydroxyphenyl)piperidinomethyl][1,2,4]triazolo[1,5-c]pyrimidine as a pale brown oily matter from 5-(3,4-dimethoxybenzylamino)-8-formyl-2-(2-furyl)[1,2,4]triazolo[1,5-c]pyrimidine and 4-(3-hydroxyphenyl)piperidine in a manner similar to that in Example 1 and subsequently treating the obtained compound without purification in a manner similar to that in Example 2.
Melting point: 239-243°C
$^1$H NMR (DMSO-$d_6$, δ , ppm): 1.49-1.80 (m, 4H), 2.03-2.18 (m, 2H), 2.25-2.50 (m, 1H), 2.92-3.07 (m, 2H), 3.69 (s, 2H), 6.50-6.67 (m,3H), 6.72 (dd, J = 1.9, 3.2 Hz, 1H), 7.05 (t, J = 7.6 Hz, 1H), 7.21 (dd, J = 0.8, 3.2 Hz, 1H), 7.80 (s, 1H), 7.85 (brs, 2H), 7.93 (dd, J = 0.8, 1.9 Hz, 1H), 9.20 (s, 1H)

Example 104

5-Amino-2-(2-furyl)-8-[3-methyl-4-(3-methylphenyl)piperazin-1-ylmethyl] [1,2,4]triazolo[1,5-c]pyrimidine (Compound 104)

The subject compound (67%) was obtained as white crystals by preparing 5-(3,4-dimethoxybenzylamino)-2-(2-furyl)-8-[3-methyl-4-(3-methylphenyl)piperazin-1-ylmethyl][1,2,4]triazolo[1,5-c]pyrimidine as a pale brown oily matter from 5-(3,4-dimethoxybenzylamino)-8-formyl-2-(2-furyl) [1,2,4]triazolo[1,5-c]pyrimidine and 2-methyl-1-(3-methylphenyl)piperazine in a manner similar to that in Example 1 and subsequently treating the obtained compound without purification in a manner similar to that in Example 2.
Melting point: 198-200°C
$^1$H NMR ($CDCl_3$, δ, ppm) : 1.09 (d, J = 6.8 Hz, 3H), 2.30 (s, 3H), 2.47-2.60 (m, 1H), 2.60-2.77 (m, 2H) , 2.85-2.96 (m, 1H), 3.08-3.29 (m, 2H), 3.79-3.90 (m, 3H), 5.88 (brs, 2H), 6.59 (dd, J = 1.6, 3.2 Hz, 1H), 6.63-6.77 (m, 2H), 6.74 (s, 1H) , 7.10-7.18 (m, 1H), 7.26 (d, J = 3.2 Hz, 1H), 7.63 (d, J = 1.6 Hz, 1H), 7.96 (s, 1H)

Example 105

5-Amino-2-(2-furyl)-8-[3-methyl-4-(4-methylphenyl)piperazin-1-ylmethyl][1,2,4]triazolo[1,5-c]pyrimidine (Compound 105)

The subject compound (48%) was obtained as white crystals by preparing 5-(3,4-dimethoxybenzylamino)-2-(2-furyl)-8-[3-methyl-4-(4-methylphenyl)piperazin-1-ylmethyl][1,2,4]triazolo[1,5-c]pyrimidine from 5-(3,4-dimethoxybenzylamino)-8-formyl-2-(2-furyl)[1,2,4]triazolo[1,5-c]pyrimidine and 2-methyl-1-(4-methylphenyl)piperazine in a manner similar to that in Example 1 and subsequently treating the obtained compound without purification in a manner similar to that in Example 2.
Melting point: 205-207°C
$^1$H NMR ($CDCl_3$, □δ, ppm): 1.04 (d, J = 6.5 Hz, 3H), 2.27 (s, 3H), 2.56-2.92 (m, 4H), 3.10-3.17 (m, 2H), 3.65-3.77 (m, 1H), 3.88 (s, 2H), 5.89 (s, 2H), 6.59 (dd, J = 1.6, 3.2 Hz, 1H), 6.85 (d, J = 8.6, 8.6 Hz, 2H), 7.06 (d, J = 8.6 Hz, 2H), 7.26 (d, J = 3.2 Hz, 1H), 7.63 (d, J = 1.6 Hz, 1H), 7.95 (s, 1H)

Example 106

9-[5-(3,4-Dimethoxybenzylamino)-2-(2-furyl) [1,2,4]triazolo[1,5-c]pyrimidin-8-ylmethyl]-1-methyl-1,3,9-triazaspiro[5.5] undecan-2-one (Compound 106)

In a manner similar to that in Example 1, the subject compound (87%) was obtained as pale yellow crystals from 5-(3,4-dimethoxybenzylamino)-8-formyl-2-(2-furyl)[1,2,4]triazolo[1,5-c]pyrimidine and 1-methyl-1,3,9-triazaspiro [5.5]undecan-2-one obtained in Reference Example 58.
$^1$H NMR ($CDCl_3$, δ, ppm): 1.46-1.57 (m, 2H), 1.91-2.02 (m, 2H), 2.10-2.39 (m, 4H), 2.86-3.01 (m, 2H), 2.92 (s, 3H), 3.16-3.28 (m, 2H), 3.86 (s, 2H), 3.89 (s, 3H), 3.89 (s, 3H), 4.63-4.73 (m, 1H), 4.76 (d, J = 5.8 Hz, 1H), 6.44 (d, J = 5.8 Hz, 1H), 6.57 (dd, J = 1.6, 3.3 Hz, 1H), 6.86 (d, J = 7.9 Hz, 1H), 6.96 (s, 1H), 6.98 (d, J = 7.9 Hz, 1H), 7.21 (d, J = 3.3 Hz, 1H), 7.60 (d, J = 1.6 Hz, 1H), 7.94 (s, 1H)

Example 107

9-[5-Amino-2-(2-furyl)[1,2,4]triazolo[1,5-c]pyrimidin-8-ylmethyl]-1-methyl-1,3,9-triazaspiro[5.5]undecan-2-one (Compound 107)

In a manner similar to that in Example 2, the subject compound (91%) was obtained from 9-[5-(3,4-dimethoxybenzylamino)-2-(2-furyl)[1,2,4]triazolo[1,5-c]pyrimidin-8-ylmethyl]-1-methyl-1,3,9-triazaspiro[5.5]undecan-2-one obtained in Example 106.
Melting point: 167.1-167.8°C
$^1$H NMR (DMSO-$d_6$, δ, ppm): 1.35-1.51 (m, 2H), 1.76-1.89 (m, 2H), 1.91-2.08 (m, 2H), 2.14-2.30 (m, 2H), 2.68-2.89 (m, 2H), 2.78 (s, 3H), 2.96-3.10 (m, 2H), 3.69 (s, 2H), 6.18 -6.26 (m, 1H), 6.71 (dd, J = 1.7, 3.3 Hz, 1H), 7.20 (d, J = 3.3 Hz, 1H), 7.78 (s, 1H), 7.84 (brs, 2H), 7.93 (d, J = 1.7 Hz, 1H)

Example 108

5-(3,4-Dimethoxybenzylamino)-2-(2-furyl)-8-(1,2,3,4-tetrahydroquinolin-1-ylmethyl)[1,2,4]triazolo[1,5-c]pyrimidine (Compound 108)

The subject compound (55%) was obtained as a pale brown oily matter from 5-(3,4-dimethoxybenzylamino)-8-formyl-2-(2-furyl) [1,2,4]triazolo[1,5-c]pyrimidine and 1,2,3,4-tetrahydroquinoline in a manner similar to that in Example 1.
$^1$H NMR ($CDCl_3$, δ , ppm): 2.02-2.07 (m, 2H), 2.80-2.85 (m, 2H), 3.48-3.53 (m, 2H), 3.86 (s, 3H), 3.87 (s, 3H), 3.89 (s, 2H), 4.72 (d, J = 5.6 Hz, 2H), 6.38 (t, J = 5.6 Hz, 1H), 6.57 (dd, J = 1.8, 3.5 Hz, 1H), 6.59-6.62 (m, 2H), 6.82 (d, J = 8.1 Hz, 1H), 6.85 (s, 1H), 6.87 (d, J = 8.1 Hz, 1H), 6.98-7.01 (m, 2H), 7.23 (d, J = 3.5 Hz, 1H), 7.61 (d, J = 1.8 Hz, 1H), 7.79 (s, 1H)

Example 109

5-Amino-2-(2-furyl)-8-(1,2,3,4-tetrahydroquinolin-1-ylmethyl)[1,2,4]triazolo[1,5-c]pyrimidine (Compound 109)

In a manner similar to that in Example 2, the subject compound (67%) was obtained as white crystals from 5-(3,4-dimethoxybenzylamino)-2-(2-furyl)-8-(1,2,3,4-tetrahydroquinolin-1-ylmethyl) [1,2,4]triazolo[1,5-c]pyrimidine obtained in Example 108.
Melting point: 189-191°C
$^1$H NMR ($CDCl_3$, δ , ppm): 2.00-2.17 (m, 2H), 2.83 (t, J = 6.3 Hz, 2H), 3.49 (t, J = 6.3 Hz, 2H), 4.73 (s, 2H), 5.91 (s, 2H), 6.57 (dd, J = 1.8, 3.5 Hz, 1H), 6.59-6.62 (m, 2H), 6.98-7.01 (m, 2H), 7.25 (d, J = 3.5 Hz, 1H), 7.64 (d, J = 1.8 Hz, 1H), 7.71 (s, 1H)

Example 110

5-(3,4-Dimethoxybenzylamino)-2-(2-furyl)-8-(1-indolinylmethyl) [1,2,4]triazolo[1,5-c]pyrimidine (Compound 110)

The subject compound (quantitative) was obtained as a pale brown oily matter from 5-(3,4-dimethoxybenzylamino)-8-formyl-2-(2-furyl)[1,2,4)triazolo[1,5-c]pyrimidine and indoline in a manner similar to that in Example 1.
$^1$H NMR ($CDCl_3$, δ , ppm) : 2.97-3.06 (m, 2H), 3.48-3.58 (m, 2H), 3.87 (s, 3H), 3.88 (s, 3H), 4.54 (s, 2H), 4.74 (d, J = 5.6 Hz, 2H), 6.41 (t, J = 5.6 Hz, 1H), 6.57 (dd, J = 1.8, 3.5 Hz, 1H), 6.63-6.73 (m, 2H), 6.84 (d, J = 8.1 Hz, 1H), 6.95 (s, 1H), 6.97 (d, J = 8.1 Hz, 1H), 7.04-7.13 (m, 2H), 7.23 (d, J = 3.5 Hz, 1H), 7.60 (d, J = 1.8 Hz, 1H), 7.95 (s, 1H)

Example 111

5-Amino-2-(2-furyl)-8-(1-indolinylmethyl)[1,2,4]triazolo[1,5-c]pyrimidine (Compound 111)

In a manner similar to that in Example 2, the subject compound (89%) was obtained as white crystals from 5-(3,4-dimethoxybenzylamino)-2-(2-furyl)-8-(1-indolinylmethyl)[1,2,4]triazolo[1,5-c]pyrimidine obtained in Example 110.
Melting point: 190-192°C
$^1$H NMR ($CDCl_3$, δ , ppm): 3.01 (t, J = 8.3 Hz, 2H), 3.50 (t, J = 8.3 Hz, 2H), 4.54 (s, 2H), 5.99 (s, 2H), 6.59 (dd, J = 1.8, 3.5 Hz, 1H), 6.61-6.71 (m, 2H), 7.04-7.11 (m, 2H), 7.24 (d, J = 3.5 Hz, 1H), 7.63 (d, J = 1.8 Hz, 1H), 7.89 (s, 1H)

Example 112

5-(3,4-Dimethoxybenzylamino)-2-(2-furyl)-8-(N-methylbenzylaminomethyl) [1,2,4]triazolo[1,5-c]pyrimidine (Compound 112)

The subject compound (93%) was obtained as a pale brown oily matter from 5-(3,4-dimethoxybenzylamino)-8-formyl-2-(2-furyl) [1,2,4]triazolo[1,5-c]pyrimidine and N-methylbenzylamine in a manner similar to that in Example 1.
$^1$H NMR ($CDCl_3$, δ, ppm): 2.30 (s, 3H), 3.67 (s, 2H), 3.86 (s, 2H), 3.88 (s, 3H), 3.88 (s, 3H), 4.76 (d, J = 5.6 Hz, 2H), 6.39 (t, J = 5.6 Hz, 1H), 6.57 (dd, J = 1.8, 3.5 Hz, 1H), 6.85 (d, J = 7.9 Hz, 1H), 6.95 (s, 1H), 6.97 (d, J = 7.9 Hz, 1H), 7.22 (d, J = 3.5 Hz, 1H), 7.24-7.40 (m, 5H), 7.60 (d, J = 1.8 Hz, 1H), 8.01 (s, 1H)

Example 113

5-Amino-2-(2-furyl)-8-(N-methylbenzylaminomethyl)[1,2,4]triazolo[1,5-c]pyrimidine (Compound 113)

In a manner similar to that in Example 2, the subject compound (68%) was obtained as white crystals from 5-(3,4-dimethoxybenzylamino)-2-(2-furyl)-8-(N-methylbenzylaminomethyl) [1,2,4]triazolo[1,5-c]pyrimidine obtained in Example 112.
Melting point: 123-125°C
$^1$H NMR ($CDCl_3$, δ , ppm): 2.30 (s, 3H), 3.67 (s, 2H), 3.86 (s, 2H), 5.93 (s, 2H), 6.59 (dd, J = 1.7, 3.4 Hz, 1H), 7.25 (d, J = 3.4 Hz, 1H), 7.29-7.41 (m, 5H), 7.63 (d, J = 1.7 Hz, 1H), 7.94 (s, 1H)

Example 114

5-(3,4-Dimethoxybenzylamino)-2-methyl-8-(4-phenylpiperazin-1-ylmethyl) [1,2,4]triazolo[1,5-c]pyrimidine (Compound 114)

The subject compound (97%) was obtained as a white powder from 5-(3,4-dimethoxybenzylamino)-8-formyl-2-methyl[1,2,4]triazolo[1,5-c]pyrimidine obtained in Reference Example 28 and 1-phenylpiperazine in a manner similar to that in Example 1.
Melting point: 123-124°C
$^1$H NMR ($CDCl_3$, δ, ppm): 2.57 (s, 3H), 2.73 (t, J = 5.0 Hz, 4H), 3.22 (t, J = 5.0 Hz, 4H), 3.82 (s, 2H), 3.88 (s, 3H), 3.88 (s, 3H), 4.79 (d, J = 5.8 Hz, 2H), 6.26 (t, J = 5.8 Hz, 1H), 6.81-6.98 (m, 6H), 7.22-7.29 (m, 2H), 7.89 (s, 1H)

Example 115

5-Amino-2-methyl-8-(4-phenylpiperazin-1-ylmethyl)[1,2,4]triazolo[1,5-c]pyrimidine (Compound 115)

In a manner similar to that in Example 2, the subject compound (83%) was obtained as a white powder from 5-(3,4-dimethoxybenzylamino)-2-methyl-8-(4-phenylpiperazin-1-ylmethyl) [1,2,4]triazolo[1,5-c]pyrimidine obtained in Example 114.
Melting point: 250-252°C
$^1$H NMR ($CDCl_3$, δ , ppm) : 2.60 (s, 3H), 2.72 (t, J = 4.9 Hz, 4H), 3.22 (t, J = 4.9 Hz, 4H), 3.82 (s, 2H), 5.88 (s, 2H), 6.84 (dd, J = 7.5, 7.5 Hz, 1H), 6.90 (d, J = 7.5 Hz, 2H), 7.23 (d, J = 7.5 Hz, 1H), 7.26 (d, J = 7.5 Hz, 1H), 7.83 (s, 1H)

Example 116

5-(3,4-Dimethoxybenzylamino)-2-methyl-8-(1,2,3,4-tetrahydroisoquinolin-2-ylmethyl)[1,2,4]triazolo[1,5-c]pyrimidine (Compound 116)

The subject compound (90%) was obtained as a white powder from 5-(3,4-dimethoxybenzylamino)-8-formyl-2-methyl[1,2,4]triazolo[1,5-c]pyrimidine obtained in Reference Example 28 and 1,2,3,4-tetrahydroisoquinoline in a manner similar to that in Example 1.
Melting point: 132-134°C
$^1$H NMR ($CDCl_3$, δ, ppm): 2.57 (s, 3H), 2.87-2.93 (m, 4H), 3.76 (s, 2H), 3.88 (s, 3H), 3.88 (s, 3H), 3.92 (s, 2H), 4.74 (d, J = 5.8 Hz, 2H), 6.26 (t, J = 5.8 Hz, 1H), 6.85 (d, J = 8.2 Hz, 1H), 6.94 (s, 1H), 6.95 (d, J = 8.2 Hz, 1H), 6.98-7.07 (m, 1H), 7.09-7.15 (m, 3H), 7.94 (s, 1H)

Example 117

5-Amino-2-methyl-8-(1,2,3,4-tetrahydroisoquinolin-2-ylmethyl) [1,2,4]triazolo[1,5-c]pyrimidine (Compound 117)

In a manner similar to that in Example 2, the subject compound (79%) was obtained as a white powder from 5-(3,4-dimethoxybenzylamino)-2-methyl-8-(1,2,3,4-tetrahydroisoquinolin-2-ylmethyl) [1,2,4]triazolo[1,5-c]pyrimidine obtained in Example 116.
Melting point: 198-200°C
$^1$H NMR ($CDCl_3$, δ, ppm): 2.60 (s, 3H), 2.86-2.92 (m, 4H), 3.75 (s, 2H), 3.92 (s, 2H), 5.82 (s, 2H), 6.99-7.01 (m, 1H), 7.09-7.10 (m, 3H), 7.88 (s, 1H)

Example 118

5-(3,4-Dimethoxybenzylamino)-8-(4-phenylpiperazin-1-ylmethyl) [1,2,4]triazolo[1,5-c]pyrimidine (Compound 118)

The subject compound (63%) was obtained as a white powder from 5-(3,4-dimethoxybenzylamino)-8-formyl [1,2,4]triazolo[1.5-c]pyrimidine obtained in Reference Example 33 and 1-phenylpiperazine in a manner similar to that in Example 1.
Melting point: 120-122°C
$^1$H NMR ($CDCl_3$, δ , ppm) : 2.73 (t, J = 4.9 Hz, 4H), 3.22 (t, J = 4.9 Hz, 4H), 3.85 (s, 2H), 3.88 (s, 3H), 3.88 (s, 3H), 4.76 (d, J = 5.8 Hz, 2H), 6.37 (t, J = 5.8 Hz, 1H), 6.81-6.98 (m, 6H), 7.22-7.28 (m, 2H), 7.93 (s, 1H), 8.25 (s, 1H)

Example 119

5-Amino-8-(4-phenylpiperazin-1-ylmethyl)[1,2,4]triazolo[1,5-c]pyrimidine (Compound 119)

In a manner similar to that in Example 2, the subject compound (79%) was obtained as white crystals from 5-(3,4-dimethoxybenzylamino)-8-(4-phenylpiperazin-1-ylmethyl) [1,2,4]triazolo[1,5-c]pyrimidine obtained in Example 118.
Melting point: 229-230°C
$^1$H NMR (DMSO-$d_6$, δ, ppm) : 2.58 (t, J = 4.8 Hz, 4H), 3.10 (t, J = 4.8 Hz, 4H), 3.71 (s, 2H), 6.75 (dd, J = 7.5, 7.5 Hz, 1H), 6.89 (d, J = 7.5 Hz, 2H), 7.17 (d, J = 7.5 Hz, 1H), 7.19 (d, J = 7.5 Hz, 1H), 7.79 (s, 1H), 7.83 (s, 2H), 8.46 (s, 1H)

Example 120

5-(3,4-Dimethoxybenzylamino)-8-(1,2,3,4-tetrahydroisoquinolin-2-ylmethyl)[1,2,4]triazolo[1,5-c]pyrimidine (Compound 120)

The subject compound (91%) was obtained as a white powder from 5-(3,4-dimethoxybenzylamino)-8-formyl [1,2,4]triazolo[1,5-c]pyrimidine obtained in Reference Example 33 and 1,2,3,4-tetrahydroisoquinoline in a manner similar to that in Example 1.
Melting point: 157-159°C
$^1$H NMR ($CDCl_3$, δ , ppm): 2.85-2.93 (m, 4H), 3.76 (s, 2H), 3.88 (s, 3H), 3.88 (s, 3H), 3.96 (s, 2H), 4.76 (d, J = 5.8 Hz, 2H), 6.36 (t, J = 5.8 Hz, 1H), 6.86 (d, J = 7.7 Hz, 1H), 6.95 (s, 1H), 6.97 (d, J = 7.7 Hz, 1H), 6.98-7.00 (m, 1H), 7.09-7.10 (m, 3H), 7.98 (s, 1H), 8.25 (s, 1H)

Example 121

5-Amino-8-(1,2,3,4-tetrahydroisoquinolin-2-ylmethyl)[1,2,4]triazolo[1,5-c]pyrimidine (Compound 121)

In a manner similar to that in Example 2, the subject compound (78%) was obtained as white crystals from 5-(3,4-dimethoxybenzylamino)-8-(1,2,3,4-tetrahydroisoquinolin-2-ylmethyl)[1,2,4]triazolo[1,5-c]pyrimidine obtained in Example 120.
Melting point: 181-183°C
$^1$H NMR (DMSO-$d_6$, δ, ppm): 2.73-2.79 (m, 2H), 3.32 (s, 2H), 3.61 (s, 2H), 3.81 (s, 2H), 6.99-7.03 (m, 1H), 7.07-7.12 (m, 3H), 7.82 (s, 1H), 7.82 (s, 2H), 8.52 (s, 1H)

Example 122

5-(3,4-Dimethoxybenzylamino)-8-[(1S,4S)-2-(4-fluorophenyl)-2,5-diazabicyclo[2.2.1]heptan-5-ylmethyl]-2-(2-furyl) [1,2,4]triazolo[1,5-c]pyrimidine (Compound 122)

The subject compound (95%) was obtained as a pale brown oily matter from 5-(3,4-dimethoxybenzylamino)-8-formyl-2-(2-furyl) [1,2,4]triazolo[1,5-c]pyrimidine and (1S,4S)-2-(4-fluorophenyl)-2,5-diazabicyclo[2.2.1]heptane in a manner similar to that in Example 1.
$^1$H NMR ($CDCl_3$, δ , ppm): 1.93 (d, J = 9.6 Hz, 1H), 2.04 (d, J = 9.6 Hz, 1H), 2.83 (d, J = 9.7 Hz, 1H), 3.02 (d, J = 9.7 Hz, 1H), 3.38 (d, J = 8.6 Hz, 1H), 3.47 (d, J = 8.6 Hz, 1H), 3.68 (s, 1H), 3.87 (s, 3H), 3.87 (s, 3H), 3.97 (s, 2H), 4.20 (s, 1H), 4.74 (d, J = 5.4 Hz, 2H), 6.38 (t, J = 5.4 Hz, 1H), 6.43-6.53 (m, 2H), 6.56 (dd, J = 1.6, 3.2 Hz, 1H), 6.80-7.00 (m, 5H), 7.18 (d, J = 3.2 Hz, 1H), 7.59 (d, J = 1.6 Hz, 1H), 7.95 (s, 1H)

Example 123

5-Amino-8-[(1S,4S)-2-(4-fluorophenyl)-2,5-diazabicyclo[2.2.1]heptan-5-ylmethyl]-2-(2-furyl)[1,2,4]triazolo[1,5-c] pyrimidine (Compound 123)

In a manner similar to that in Example 2, the subject compound (85%) was obtained as white crystals from 5-(3,4-dimethoxybenzylamino)-8-[(1S,4S)-2-(4-fluorophenyl)-2,5-diazabicyclo[2.2.1]heptan-5-ylmethyl]-2-(2-furyl) [1,2,4]triazolo[1,5-c]pyrimidine obtained in Example 122.
Melting point: 194-196°C
$^1$H NMR ($CDCl_3$, δ, ppm): 1.94 (d, J = 8.9 Hz, 1H), 2.05 (d, J = 8.9 Hz, 1H), 2.81 (d, J = 9.2 Hz, 1H), 3.03 (d, J = 9.2 Hz, 1H), 3.38 (d, J = 8.9 Hz, 1H), 3.47 (d, J = 8.9 Hz, 1H), 3.67 (s, 1H), 3.98 (s, 2H), 4.20 (s, 1H), 6.01 (brs, 2H), 6.45-6.55 (m, 2H), 6.59 (brd, J = 2.7 Hz, 1H), 6.87 -7.00 (m, 2H), 7.21 (d, J = 2.7 Hz, 1H), 7.62 (s, 1H), 7.88 (s, 1H)

Example 124

5-(3,4-Dimethoxybenzylamino)-2-(2-furyl)-8-(3-oxo-4-phenylpiperazin-1-ylmethyl)[1,2,4]triazolo[1,5-c]pyrimidine (Compound 124)

The subject compound (quantitative) was obtained as a pale brown oily matter from 5-(3,4-dimethoxybenzylamino)-8-formyl-2-(2-furyl)[1,2,4]triazolo[1,5-c]pyrimidine and a known compound (WO00/01691), 1-phenylpiperazin-2-one, in a manner similar to that in Example 1.
$^1$H NMR ($CDCl_3$, δ, ppm): 2.98 (t, J = 5.4 Hz, 2H), 3.46 (s, 2H), 3.73 (t, J = 5.4 Hz, 2H), 3.88 (s, 3H), 3.88 (s, 3H), 3.95 (s, 2H), 4.77 (d, J = 5.7 Hz, 2H), 6.49 (t, J = 5.7 Hz, 1H), 6.58 (dd, J = 1.9, 3.5 Hz, 1H), 6.86 (d, J = 7.8 Hz, 1H), 6.93-7.02 (m, 2H), 7.20-7.45 (m, 6H), 7.61 (d, J = 1.9 Hz, 1H), 7.96 (s, 1H)

Example 125

5-Amino-2-(2-furyl)-8-(3-oxo-4-phenylpiperazin-1-ylmethyl)[1,2,4]triazolo[1,5-c]pyrimidine (Compound 125)

In a manner similar to that in Example 2, the subject compound (71%) was obtained as white crystals from 5-(3,4-dimethoxybenzylamino)-2-(2-furyl)-8-(3-oxo-4-phenylpiperazin-1-ylmethyl)[1,2,4]triazolo[1,5-c]pyrimidine obtained in Example 124.
Melting point: 251-254°C
$^1$H NMR ($CDCl_3$, δ , ppm): 2.98 (t, J = 5.4 Hz, 2H), 3.47 (s, 2H), 3.72 (t, J = 5.4 Hz, 2H), 3.96 (s, 2H), 5.95 (brs, 2H), 6.59 (dd, J = 1.6, 3.2 Hz, 1H), 7.20-7.47 (m, 6H), 7.63 (dd, J = 0.5, 1.6 Hz, 1H), 7.91 (s, 1H)

Example 126

2-(2-Furyl)-5-(2-methoxyethylamino)-8-(4-phenylpiperazin-1-ylmethyl) [1,2,4]triazolo[1,5-c]pyrimidine (Compound 126)

The subject compound (85%) was obtained as white crystals from 8-formyl-2-(2-furyl)-5-(2-methoxyethylamino)[1,2,4]triazolo[1,5-c]pyrimidine obtained in Reference Example 63 and 1-phenylpiperazine in a manner similar to that in Example 1.
Melting point: 217-220°C

$^1H$ NMR (DMSO-$d_6$, δ , ppm): 2.98-3.90 (m, 12H) , 3.34 (s, 3H), 4.55 (s, 2H), 6.72-6.80 (m, 1H), 6.84 (t, J = 7.3 Hz, 1H), 6.97 (d, J = 8.1 Hz, 2H) , 7.15-7.30 (m, 3H), 7.97 (s, 1H), 8.26 (s, 1H), 8.50 (brs, 1H)

Example 127

2-(2-Furyl)-5-(2-methoxyethylamino)-8-(1,2,3,4-tetrahydroisoquinolin-2-ylmethyl)[1,2,4]triazolo[1,5-c]pyrimidine (Compound 127)

The subject compound (80%) was obtained as white crystals from 8-formyl-2-(2-furyl)-5-(2-methoxyethylamino)[1,2,4]triazolo[1,5-c]pyrimidine obtained in Reference Example 63 and 1,2,3,4-tetrahydroisoquinoline in a manner similar to that in Example 1.
Melting point: 135-136°C
$^1H$ NMR ($CDCl_3$, δ, ppm): 2.80-3.00 (m, 4H), 3.42 (s, 3H), 3.66 (t, J = 5.1 Hz, 2H), 3.78 (s, 2H), 3.85 (dt, J = 4.9, 5.1 Hz, 2H), 3.99 (s, 2H), 6.45 (t, J = 4.9 Hz, 1H), 6.58 (brd, J = 3.2 Hz, 1H), 6.95-7.17 (m, 4H), 7.24 (d, J = 3.2 Hz, 1H), 7.62 (s, 1H), 7.97 (s, 1H)

Example 128

5-Amino-8-[4-(4-chlorophenyl)-2-methylpiperazin-1-ylmethyl]-2-(2-furyl)[1,2,4]triazolo[1,5-c]pyrimidine (Compound 128)

The subject compound (49%) was obtained as white crystals by preparing 8-[4-(4-chlorophenyl)-2-methylpiperazin-1-ylmethyl]-5-(3,4-dimethoxybenzylamino)-2-(2-furyl)[1,2,4]triazolo[1,5-c]pyrimidinefrom 5-(3,4-dimethoxybenzylamino)-8-formyl-2-(2-furyl)[1,2,4]triazalo[1,5-c]pyrimidine and a known compound [Journal of Organometallic Chemistry, Vol. 576, p. 125 (1999)], 1-(4-chlorophenyl)-3-methylpiperazine, in a manner similar to that in Example 1 and subsequently treating the thus obtained compound without purification in a manner similar to that in Example 2.
Melting point: 202-205°C
$^1H$ NMR ($CDCl_3$, δ , ppm) : 1.31 (d, J = 5.7 Hz, 3H) , 2.47-2.57 (m, 1H), 2.63-2.85 (m, 2H), 2.86-3.07 (m, 2H), 3.27 - 3.43 (m, 2H), 3.78 (d, J = 14.9 Hz, 1H), 4.23 (d, J = 14.9 Hz, 1H), 5.84 (brs, 2H), 6.59 (dd, J = 1.6, 3.2 Hz, 1H), 6.81 (d, J = 8.9 Hz, 2H), 7.18 (d, J = 8.9 Hz, 2H), 7.24 (dd, J = 0.5, 3.2 Hz, 1H), 7.63 (dd, J = 0.5, 1.6 Hz, 1H), 7.94 (s, 1H)

Example 129

8-Benzylaminomethyl-5-(3,4-dimethoxybenzylamino)-2-(2-furyl)[1,2,4]triazolo[1,5-c]pyrimidine (Compound 129)

The subject compound (97%) was obtained as a pale brown oily matter from 5-(3,4-dimethoxybenzylamino)-8-formyl-2-(2-furyl)[1,2,4]triazolo[1,5-c]pyrimidine and benzylamine in a manner similar to that in Example 1.
$^1H$ NMR ($CDCl_3$, δ , ppm): 3.85-3.87 (m, 2H), 3.88 (s, 3H), 3.88 (s, 3H), 4.03-4.05 (m, 2H), 4.75 (d, J = 5.6 Hz, 2H), 6.56 (t, J = 5.6 Hz, 1H), 6.57 (dd, J = 1.8, 3.5 Hz, 1H), 6.85 (d, J = 7.7 Hz, 1H), 6.95 (s, 1H), 6.97 (d, J = 7.7 Hz, 1H), 7.18-7.20 (m, 1H), 7.20 (d, J = 3.5 Hz, 1H), 7.22-7.38 (m, 5H), 7.59 (d, J = 1.8 Hz, 1H), 7.86 (s, 1H)

Example 130

5-Amino-8-benzylaminomethyl-2-(2-furyl)[1,2,4]triazolo[1,5-c]pyrimidine (Compound 130)

In a manner similar to that in Example 2, the subject compound (66%) was obtained as white crystals from 8-benzylaminomethyl-5-(3,4-dimethoxybenzylamino)-2-(2-furyl)[1,2,4]triazolo[1,5-c]pyrimidine obtained in Example 129.
Melting point: 240-242°C
$^1H$ NMR ($CDCl_3$, δ , ppm): 3.86 (s, 2H), 4.05 (s, 2H), 5.92 (s, 2H), 6.59 (dd, J = 1.8, 3.5 Hz, 1H), 7.24 (d, J = 3.5 Hz, 1H), 7.25-7.38 (m, 6H), 7.63 (d, J = 1.8 Hz, 1H), 7.79 (s, 1H)

Example 131

5-(3,4-Dimethoxybenzylamino)-2-(2-furyl)-8-phenethylaminomethyl[1,2,4]triazolo[1,5-c]pyrimidine (Compound 131)

The subject compound (96%) was obtained as a pale brown oily matter from 5-(3,4-dimethoxybenzylamino)-8-formyl-2-(2-furyl) [1,2,4]triazolo[1,5-c]pyrimidine and phenethylamine in a manner similar to that in Example 1.

$^1H$ NMR ($CDCl_3$, δ , ppm): 2.80-2.90 (m, 2H), 2.91-2.97 (m, 2H), 3.87 (s, 3H), 3.88 (s, 3H), 4.04 (s, 2H), 4.74 (d, J = 5.6 Hz, 2H), 6.39 (t, J = 5.6 Hz, 1H), 6.56 (dd, J = 1.8, 3.6 Hz, 1H), 6.84 (d, J = 7.9 Hz, 1H), 6.94 (s, 1H), 6.95 (d, J = 7.9 Hz, 1H), 7.17 (d, J = 3.6 Hz, 1H), 7.19-7.25 (m, 6H), 7.60 (d, J = 1.8 Hz, 1H) , 7.83 (s, 1H)

Example 132

5-Amino-2-(2-furyl)-8-phenethylaminomethyl)[1,2,4]triazolo[1,5-c]pyrimidine (Compound 132)

In a manner similar to that in Example 2, the subject compound (64%) was obtained as white crystals from 5-(3,4-dimethoxybenzylamino)-2-(2-furyl)-8-phenethylaminomethyl[1,2,4]triazolo[1,5-c]pyrimidine obtained in Example 131.
Melting point: 155-156°C
$^1H$ NMR ($CDCl_3$, δ, ppm): 2.81-2.87 (m, 2H), 2.91-2.97 (m, 2H), 4.04 (s, 2H), 5.89 (s, 2H), 6.59 (dd, J = 1.8, 3.6 Hz, 1H), 7.20 (d, J = 3.6 Hz, 1H), 7.21-7.28 (m, 6H), 7.63 (d, J = 1.8 Hz, 1H), 7.76 (s, 1H)

Example 133

5-(3,4-Dimethoxybenzylamino)-2-(2-furyl)-8-(1,2,3,4-tetrahydro-1-naphthylaminomethyl)[1,2,4]triazolo[1,5-c]pyrimidine (Compound 133)

The subject compound (quantitative) was obtained as a pale brown oily matter from 5-(3,4-dimethoxybenzylamino)-8-formyl-2-(2-furyl) [1,2,4]triazolo[1,5-c]pyrimidine and 1,2,3,4-tetrahydro-1-naphthylamine in a manner similar to that in Example 1.
$^1H$ NMR ($CDCl_3$, δ , ppm) : 1.71-1.79 (m, 2H), 1.89-1.91 (m, 1H), 1.93-2.02 (m, 2H) , 2.72-2.88 (m, 2H), 3.88 (s, 3H), 3.88 (s, 3H), 4.05-4.29 (m, 2H), 4.75 (d, J = 5.6 Hz, 2H), 6.28 (t, J = 5.6 Hz, 1H), 6.31-6.40 (m, 1H), 6.58 (dd, J = 1.7, 3.5 Hz, 1H), 6.84 (d, J = 8.0 Hz, 1H), 6.94 (s, 1H), 6.96 (d, J = 8.0 Hz, 1H), 7.04-7.14 (m, 3H), 7.22 (d, J = 3.5 Hz, 1H), 7.38-7.41 (m, 1H), 7.60 (d, J = 1.7 Hz, 1H), 7.96 (s, 1H)

Example 134

5-Amino-2-(2-furyl)-8-(1,2,3,4-tetrahydro-1-naphthylaminomethyl)[1,2,4]triazolo[1,5-c]pyrimidine (Compound 134)

In a manner similar to that in Example 2, the subject compound (82%) was obtained as white crystals from 5-(3,4-dimethoxybenzylamino)-2-(2-furyl)-8-(1,2,3,4-tetrahydro-1-naphthylaminomethyl) [1,2,4]triazolo[1,5-c]pyrimidine obtained in Example 133.
Melting point: 164-166°C
$^1H$ NMR ($CDCl_3$, δ , ppm): 1.91-2.04 (m, 4H), 2.67-2.88 (m, 2H), 3.80-3.89 (m, 1H) , 4.06-4.22 (m, 2H), 5.97 (s, 2H), 6.60 (dd, J = 1.8, 3.6 Hz, 1H), 7.08-7.15 (m, 3H), 7.20 (d, J = 3.6 Hz, 1H), 7.37-7.40 (m, 2H), 7.64 (d, J = 1.8 Hz, 1H), 7.89 (s, 1H)

Example 135

8-[4-(3-Chlorophenyl)piperazin-1-ylmethyl]-5-(3,4-dimethoxybenzylamino)-2-(2-furyl)[1,2,4]triazolo[1,5-c]pyrimidine (Compound 135)

The subject compound (quantitative) was obtained as a pale brown oily matter from 5-(3,4-dimethoxybenzylamino)-8-formyl-2-(2-furyl) [1,2,4]triazolo[1,5-c]pyrimidine and 1-(3-chlorophenyl)piperazine in a manner similar to that in Example 1.
$^1H$ NMR ($CDCl_3$, δ, ppm): 2.74-2.76 (m, 4H), 3.22-3.24 (m, 4H), 3.86 (s, 2H), 3.89 (s, 3H), 3.89 (s, 3H), 4.76 (d, J = 5.6 Hz, 2H), 6.42 (t, J = 5.6 Hz, 1H), 6.57 (dd, J = 1.8, 3.6 Hz, 1H), 6.75-6.99 (m, 6H), 7.11-7.17 (m, 1H), 7.22 (d, J = 3.6 Hz, 1H), 7.60 (d, J = 1.8 Hz, 1H), 7.96 (s, 1H)

Example 136

5-Amino-8-[4-(3-chlorophenyl)piperazin-1-ylmethyl]-2-(2-furyl)[1,2,4]triazolo[1,5-c]pyrimidine (Compound 136)

In a manner similar to that in Example 2, the subject compound (71%) was obtained as white crystals from 8-[4-(3-chlorophenyl)piperazin-1-ylmethyl]-5-(3,4-dimethoxybenzylamino)-2-(2-furyl)[1,2,4]triazolo[1,5-c]pyrimidine

obtained in Example 135.
Melting point: 203-206°C
$^1$H NMR ($CDCl_3$, δ, ppm): 2.74 (t, J = 5.0 Hz, 4H), 3.23 (t, J = 5.0 Hz, 4H), 3.90 (s, 2H), 6.01 (s, 2H), 6.59 (dd, J = 1.8, 3.5 Hz, 1H), 6.75-6.80 (m, 2H), 6.83-6.89 (m, 1H), 7.11-7.17 (m, 1H), 7.25 (d, J = 3.5 Hz, 1H), 7.63 (d, J = 1.8 Hz, 1H), 7.90 (s, 1H)

Example 137

5-(3,4-Dimethoxybenzylamino)-2-(2-furyl)-8-(N-methylphenethylaminomethyl) [1,2,4]triazolo[1,5-c]pyrimidine (Compound 137)

The subject compound (quantitative) was obtained as a pale brown oily matter from 5-(3,4-dimethoxybenzylamino)-8-formyl-2-(2-furyl) [1,2,4]triazolo[1,5-c]pyrimidine and N-methylphenethylamine in a manner similar to that in Example 1.
$^1$H NMR ($CDCl_3$, δ, ppm) : 2.40 (s, 3H) , 2.75-2.80 (m, 2H), 2.90-2.95 (m, 2H), 3.84 (s, 2H), 3.88 (s, 3H), 3.88 (s, 3H), 4.75 (d, J = 5.6 Hz, 2H), 6.40 (t, J = 5.6 Hz, 1H), 6.56 (dd, J = 1.8, 3.6 Hz, 1H), 6.85 (d, J = 7.9 Hz, 1H), 6.95 (s, 1H), 6.97 (d, J = 7.9 Hz, 1H), 7.22 (d, J = 3.6 Hz, 1H), 7.23-7.29 (m, 5H), 7.60 (d, J = 1.8 Hz, 1H), 7.89 (s, 1H)

Example 138

5-Amino-2-(2-furyl)-8-(N-methylphenethylaminomethyl) [1,2,4]triazolo[1,5-c]pyrimidine (Compound 138)

In a manner similar to that in Example 2, the subject compound (81%) was obtained as white crystals from 5-(3,4-dimethoxybenzylamino)-2-(2-furyl)-8-(N-methylphenethylaminomethyl)[1,2,4]triazolo[1,5-c]pyrimidine obtained in Example 137.
Melting point: 87-90°C
$^1$H NMR ($CDCl_3$, δ, ppm): 2.40 (s, 3H), 2.76-2.80 (m, 2H), 2.89-2.94 (m, 2H), 3.88 (s, 2H), 6.06 (s, 2H), 6.57 (dd, J = 1.7, 3.5 Hz, 1H), 7.15-7.21 (m, 2H), 7.23 (d, J = 3.5 Hz, 1H), 7.25-7.30 (m, 3H), 7.62 (d, J = 1.7 Hz, 1H), 7.80 (s, 1H)

Example 139

5-(3,4-Dimethoxybenzylamino)-2-(2-furyl)-8-(1-indanylaminomethyl)[1,2,4]triazolo[1,5-c]pyrimidine (Compound 139)

The subject compound (quantitative) was obtained as a pale brown oily matter from 5-(3,4-dimethoxybenzylamino)-8-formyl-2-(2-furyl) [1,2,4]triazolo[1,5-c]pyrimidine and 1-aminoindane in a manner similar to that in Example 1.
$^1$H NMR ($CDCl_3$, δ, ppm): 2.83-2.91 (m, 2H), 3.16-3.24 (m, 2H), 3.68-3.73 (m, 1H), 3.87 (s, 3H), 3.88 (s, 3H), 4.09 - 4.12 (m, 2H), 4.32-4.35 (m, 1H), 4.74 (d, J = 5.6 Hz, 2H), 6.39 (t, J = 5.6 Hz, 1H), 6.57 (dd, J = 1.8, 3.5 Hz, 1H), 6.83 (d, J = 8.0 Hz, 1H), 6.94 (s, 1H), 6.95 (d, J = 8.0 Hz, 1H), 7.11-7.18 (m, 4H), 7.21 (d, J = 3.5 Hz, 1H), 7.60 (d, J = 1.8 Hz, 1H), 7.90 (s, 1H)

Example 140

5-Amino-2-(2-furyl)-8-(1-indanylaminomethyl) [1,2,4]triazolo[1,5-c]pyrimidine (Compound 140)

In a manner similar to that in Example 2, the subject compound (82%) was obtained as white crystals from 5-(3,4-dimethoxybenzylamino)-2-(2-furyl)-8-(1-indanylaminomethyl)[1,2,4]triazolo[1,5-c]pyrimidine obtained in Example 139.
Melting point: 164-165°C
$^1$H NMR ($CDCl_3$, δ, ppm): 1.89-2.02 (m, 1H), 2.38-2.50 (m, 1H), 2.78-2.89 (m, 1H), 3.00-3.11 (m, 1H), 4.15 (d, J = 7.1 Hz, 2H), 4.35-4.37 (m, 1H), 5.91 (s, 2H), 6.59 (dd, J = 1.8, 3.6 Hz, 1H), 7.16-7.21 (m, 4H), 7.23 (d, J = 3.6 Hz, 1H), 7.42 (t, J = 7.1 Hz, 1H), 7.63 (d, J = 1.8 Hz, 1H), 7.89 (s, 1H)

Example 141

5-(3,4-Dimethoxybenzylamino)-2-(2-furyl)-8-(2-indanylaminomethyl) [1,2,4]triazolo[1,5-c]pyrimidine (Compound 141)

The subject compound (98%) was obtained as a pale brown oily matter from 5-(3,4-dimethoxybenzylamino)-8-formyl-2-(2-furyl)[1,2,4]triazolo[1,5-c]pyrimidine and 2-aminoindane in a manner similar to that in Example 1.
$^1$H NMR ($CDCl_3$, δ, ppm): 1.90-2.02 (m, 1H), 2.38-2.50 (m, 1H), 2.77-2.90 (m, 1H), 3.00-3.11 (m, 1H), 3.88 (s, 3H), 3.88 (s, 3H), 4.08-4.21 (m, 2H), 4.32-4.37 (m, 1H), 4.75 (d, J = 5.6 Hz, 2H), 6.38 (t, J = 5.6 Hz, 1H), 6.57 (dd, J = 1.8, 3.5 Hz, 1H) , 6.83 (d, J = 7.9 Hz, 1H), 6.94 (s, 1H), 6.96 (d, J = 7.9 Hz, 1H), 7.04-7.21 (m, 4H), 7.22 (d, J = 3.5 Hz, 1H), 7.39-7.42 (m, 1H), 7.60 (d, J = 1.8 Hz, 1H), 7.95 (s, 1H)

Example 142

5-Amino-2-(2-furyl)-8-(2-indanylaminomethyl) [1,2,4]triazolo[1,5-c]pyrimidine (Compound 142)

In a manner similar to that in Example 2, the subject compound (quantitative) was obtained as white crystals from 5-(3,4-dimethoxybenzylamino)-2-(2-furyl)-8-(2-indanylaminomethyl) [1,2,4]triazolo[1,5-c]pyrimidine obtained in Example 141.
Melting point: 177-179°C
$^1$H NMR ($CDCl_3$, δ , ppm): 2.82-2.91 (m, 2H), 3.16-3.24 (m, 2H), 3.66-3.76 (m, 1H), 4.09-4.12 (m, 2H), 4.31-4.34 (m, 1H), 5.87 (s, 2H), 6.59 (dd, J = 1.8, 3.5 Hz, 1H), 7.11-7.21 (m, 4H), 7.24 (d, J = 3.5 Hz, 1H), 7.63 (d, J = 1.8 Hz, 1H), 7.84 (s, 1H)

Example 143

5-(3,4-Dimethoxybenzylamino)-8-(4-phenylpiperazin-1-ylmethyl)-2-(3-pyridyl) [1,2,4]triazolo[1,5-c]pyrimidine (Compound 143)

The subject compound (81%) was obtained as a white powder from 5-(3,4-dimethoxybenzylamino)-8-formyl-2-(3-pyridyl)[1,2,4]triazolo[1,5-c]pyrimidine obtained in Reference Example 23 and 1-phenylpiperazine in a manner similar to that in Example 1.
Melting point: 161-162°C
$^1$H NMR ($CDCl_3$, δ , ppm): 2.79 (t, J = 4.9 Hz, 4H), 3.24 (t, J = 4.9 Hz, 4H), 3.89 (s, 6H), 3.93 (s, 2H), 4.80(d, J = 5.9 Hz, 2H), 6.42 (t, J = 5.9 Hz, 1H), 6.82-7.05 (m, 6H), 7.21-7.32 (m, 2H), 7.39 (ddd, J = 1.0, 5.0, 7.9 Hz, 1H) , 7.98 (s, 1H), 8.53 (ddd, J = 2.0, 2.0, 7.9 Hz, 1H), 8.69 (dd, J = 2.0, 5.0 Hz, 1H), 9.48 (dd, J = 1.0, 2.0 Hz, 1H)

Example 144

5-Amino-8-(4-phenylpiperazin-1-ylmethyl)-2-(3-pyridyl)[1,2,4]triazolo[1,5-c]pyrimidine (Compound 144)

In a manner similar to that in Example 2, the subject compound (74%) was obtained as a white powder from 5-(3,4-dimethoxybenzylamino)-8-(4-phenylpiperazin-1-ylmethyl)-2-(3-pyridyl) [1,2,4]triazolo[1,5-c]pyrimidine obtained in Example 143.
Melting point: 239-241°C
$^1$H NMR ($CDCl_3$, δ , ppm): 2.63 (t, J = 4.9 Hz, 4H), 3.13 (t, J = 4.9 Hz, 4H), 3.78 (s, 2H), 6.75 (t, J = 7.3 Hz, 1H), 6.90 (d, J = 7.9 Hz, 2H), 7.14-7.21 (m, 2H), 7.61 (ddd, J = 1.0, 5.0, 7.9 Hz, 1H), 7.85 (s, 1H), 7.93 (brs, 2H), 8.52 (ddd, J = 2.0, 2.0, 7.9 Hz, 1H), 8.73 (dd, J = 2.0, 5.0 Hz, 1H), 9.38 (dd, J = 1.0, 2.0 Hz, 1H)

Example 145

5-(3,4-Dimethoxybenzylamino)-2-(3-pyridyl)-8-(1,2,3,4-tetrahydroisoquinolin-2-ylmethyl)[1,2,4]triazolo[1,5-c] pyrimidine (Compound 145)

The subject compound (78%) was obtained as a white powder from 5-(3,4-dimethoxybenzylamino)-8-formyl-2-(3-pyridyl) [1,2,4]triazolo[1,5-c]pyrimidine obtained in Reference Example 23 and 1,2,3,4-tetrahydroisoquinoline in a manner similar to that in Example 1.
Melting point: 156-157°C
$^1$H NMR ($CDCl_3$, δ, ppm): 2.87-2.98 (m, 4H), 3.82 (s, 2H), 3.89 (s, 6H), 4.02 (s, 2H), 4.80 (d, J = 5.8 Hz, 2H), 6.43 (t, J = 5.8 Hz, 1H), 6.83-7.16 (m, 7H), 7.40 (ddd, J = 1.0, 5.0, 7.9 Hz, 1H), 8.01 (s, 1H), 8.54 (ddd, J = 2.0, 2.0, 7.9 Hz, 1H), 8.69 (dd, J = 2.0, 5.0 Hz, 1H), 9.48 (dd, J = 1.0, 2.0 Hz, 1H)

Example 146

5-Amino-2-(3-pyridyl)-8-(1,2,3,4-tetrahydroisoquinolin-2-ylmethyl) [1,2,4]triazolo[1,5-c]pyrimidine (Compound 146)

In a manner similar to that in Example 2, the subject compound (68%) was obtained as a white powder from 5-(3,4-dimethoxybenzylamino)-2-(3-pyridyl)-8-(1,2,3,4-tetrahydroisoquinolin-2-ylmethyl) [1,2,4]triazolo[1,5-c]pyrimidine obtained in Example 145.
Melting point: 214-216°C
$^1$H NMR ($CDCl_3$, δ , ppm): 2.75-2.83 (m, 4H), 3.67 (s, 2H), 3.88 (s, 2H), 7.01-7.15 (m, 4H), 7.60 (ddd, J = 1.0, 5.0, 7.9 Hz, 1H), 7.88 (s, 1H), 7.93 (brs, 2H), 8.53 (ddd, J = 2.0, 2.0, 7.9 Hz, 1H), 8.73 (dd, J = 2.0, 5.0 Hz, 1H), 9.38 (dd, J = 1.0, 2.0 Hz, 1H)

Example 147

8-(2,3-Dihydro-1H-benzo[de]isoquinolin-2-ylmethyl)-5-(3,4-dimethoxybenzylamino)-2-(2-furyl) [2,2,4]triazolo[1,5-c] pyrimidine (Compound 147)

The subject compound (91%) was obtained as a pale brown oily matter from 5-(3,4-dimethoxybenzylamino)-8-formyl-2-(2-furyl)[1,2,4]triazolo[1,5-c]pyrimidine and 2,3-dihydro-1H-benzo[de]isoquinoline in a manner similar to that in Example 1.
$^1$H NMR ($CDCl_3$, δ, ppm): 3.88 (s, 3H), 3.88 (s, 3H), 4.09 (s, 2H), 4.17 (s, 4H), 4.76 (d, J = 5.7 Hz, 2H), 6.44 (t, J = 5.7 Hz, 1H), 6.56 (dd, J = 1.9, 3.5 Hz, 1H), 6.85 (d, J = 8.4 Hz, 1H), 6.95 (s, 1H), 6.97 (d, J = 8.4 Hz, 1H), 7.19 (d, J = 7.3 Hz, 2H), 7.23 (d, J = 3.5 Hz, 1H), 7.40 (dd, J = 7.3, 7.8 Hz, 2H), 7.60 (d, J = 1.9 Hz, 1H), 7.70 (d, J = 7.8 Hz, 2H), 7.97 (s, 1H)

Example 148

5-Amino-8-(2,3-dihydro-1H-benzo[de]isoquinolin-2-ylmethyl)-2-(2-furyl)[1,2,4]triazolo[1,5-c]pyrimidine (Compound 148)

In a manner similar to that in Example 2, the subject compound (73%) was obtained as white crystals from 8-(2,3-dihydro-1H-benzo[de]isoquinolin-2-ylmethyl)-5-(3,4-dimethoxybenzylamino)-2-(2-furyl) [1,2,4]triazolo[1,5-c]pyrimidine obtained in Example 147.
Melting point: 250-254°C
$^1$H NMR (DMSO-$d_6$, δ , ppm): 3.95 (s, 2H), 3.99 (s, 4H), 6.70 (dd, J = 1.9, 3.2 Hz, 1H), 7.19 (d, J = 3.2 Hz, 1H), 7.25 (d, J = 7.0 Hz, 2H), 7.41 (dd, J = 7.0, 7.8 Hz, 2H), 7.74 (d, J = 7.8 Hz, 2H), 7.86 (s, 1H), 7.91 (d, J = 1.9 Hz, 1H)

Example 149

5-(3,4-Dimethoxybenzylamino)-2-(2-furyl)-8-(4-hydroxypiperidinomethyl) [1,2,4]triazolo[1,5-c]pyrimidine (Compound 149)

The subject compound (55%) was obtained as a pale brown oily matter from 5-(3,4-dimethoxybenzylamino)-2-(2-furyl)-8-(4-oxopiperidinomethyl)[1,2,4]triazolo[1,5-c]pyrimidine obtained in Reference Example 67 and 2-ethylpiperidine in a manner similar to that in Example 1.
$^1$H NMR ($CDCl_3$, δ , ppm): 1.55-1.70 (m, 2H), 1.85-1.96 (m, 2H), 2.32 (t, J = 9.3 Hz, 2H), 2.90 (dt, J = 5.4, 9.3 Hz, 2H), 3.65-3.80 (m, 1H), 3.83 (s, 2H), 3.88 (s, 3H), 3.88 (s, 3H), 4.76 (d, J = 5.4 Hz, 2H), 6.42 (t, J = 5.4 Hz, 1H), 6.56 (dd, J = 1.6, 3.5 Hz, 1H), 6.85 (d, J = 8.1 Hz, 1H), 6.95 (s, 1H), 6.97 (d, J = 8.1Hz, 1H), 7.21 (dd, J = 0.8, 3.5 Hz, 1H), 7.60 (dd, J = 0.8, 1.6 Hz, 1H), 7.94 (s, 1H)

Example 150

5-Amino-2-(2-furyl)-8-(4-hydroxypiperidinomethyl)[1,2,4]triazolo[1,5-c]pyrimidine (Compound 150)

In a manner similar to that in Example 2, the subject compound (30%) was obtained as white crystals from 5-(3,4-dimethoxybenzylamino) -2- (2-furyl) -8- (4-hydroxypiperidinomethyl) [1,2,4]triazolo[1,5-c]pyrimidine obtained in Example 149.
Melting point: 233-236°C

$^1H$ NMR (DMSO-$d_6$, δ, ppm): 1.29-1.36 (m, 2H), 1.63-1.76 (m, 2H), 2.03-2.17 (m, 2H), 2.65-2.80 (m, 2H), 3.32-3.50 (m, 1H), 3.62 (s, 2H), 6.71 (dd, J = 1.6, 3.5 Hz, 1H), 7.19 (d, J = 3.5 Hz, 1H), 7.75 (s, 1H), 7.84 (brs, 2H), 7.93 (d, J = 1.6 Hz, 1H)

Example 151

5-(3,4-Dimethoxybenzylamino)-2-(2-furyl)-8-[(2RS,11bSR)-9-methoxy-1,3,4,6,7,11b-hexahydro-2H-benzo[a]quinolizin-2-ylmethylaminomethyl] [1,2,4]triazolo[1,5-c]pyrimidine (Compound 151)

The subject compound (quantitative) was obtained as a pale brown oily matter from 5-(3,4-dimethoxybenzylamino)-8-formyl-2-(2-furyl) [1,2,4]triazolo[1,5-c]pyrimidine and a known compound (WO99/21856), (2RS,11bSR)-9-methoxy-1,3,4,6,7,11b-hexahydro-2H-benzo[a]quinolizin-2-ylmethylamine, in a manner similar to that in Example 1.
$^1H$ NMR ($CDCl_3$, δ, ppm): 1.00-1.18 (m, 1H), 1.28-1.49 (m, 1H), 1.62-1.92 (m, 2H), 2.26-2.75 (m, 6H), 2.88-3.25 (m, 4H), 3.73 (s, 3H), 3.88 (s, 3H), 3.88 (s, 3H), 4.04 (d, J = 2.4 Hz, 2H), 4.75 (d, J = 5.7 Hz, 2H), 6.43 (t, J = 5.7 Hz, 1H), 6.57 (dd, J = 1.9, 3.5 Hz, 1H), 6.60 (d, J = 2.7 Hz, 1H), 6.63 (dd, J = 2.7, 8.6 Hz, 1H), 6.84 (d, J = 8.1 Hz, 1H), 6.94 (s, 1H), 6.96 (d, J = 8.1 Hz, 1H), 7.11 (d, J = 8.6 Hz, 1H), 7.21 (dd, J = 0.5, 3.5 Hz, 1H), 7.60 (dd, J = 0.5, 1.9 Hz, 1H), 7.87 (s, 1H)

Example 152

5-Amino-2-(2-furyl)-8-[(2RS,11bSR)-9-methoxy-1,3,4,6,7,11b-hexahydro-2H-benzo[a]quinolizin-2-ylmethylaminomethyl] [1,2,4]triazolo[1,5-c]pyrimidine (Compound 152)

In a manner similar to that in Example 2, the subject compound (69%) was obtained as white crystals from 5-(3,4-dimethoxybenzylamino)-2-(2-furyl)-8-[(2RS,11bSR)-9-methoxy-1,3,4,6,7,11b-hexahydro-2H-benzo[a]quinolizin-2-ylmethylaminomethyl] [1,2,4]triazolo[1,5-c]pyrimidine obtained in Example 151.
Melting point: 112-115°C
$^1H$ NMR ($CDCl_3$, δ, ppm): 1.00-1.17 (m, 1H), 1.30-1.47 (m, 1H), 1.70-1.95 (m, 2H), 2.25-2.75 (m, 6H), 2.85-3.22 (m, 4H), 3.76 (s, 3H), 4.04 (d, J = 1.6 Hz, 2H), 6.07 (brs, 2H), 6.58 (dd, J = 1.6, 3.2 Hz, 1H), 6.59 (d, J = 2.7 Hz, 1H), 6.65 (dd, J = 2.7, 8.7 Hz, 1H), 7.12 (d, J = 8.7 Hz, 1H), 7.24 (d, J = 3.2 Hz, 1H), 7.63 (d, J = 1.6 Hz, 1H), 7.81 (s, 1H)

Example 153

5-(3,4-Dimethoxybenzylamino)-2-(2-furyl)-8-[(2SR,11bSR)-9-methoxy-1,3,4,6,7,11b-hexahydro-2H-benzo[a]quinolizin-2-ylmethylaminomethyl] [1,2,4]triazolo[1,5-c]pyrimidine (Compound 153)

The subject compound (92%) was obtained as a pale brown oily matter from 5-(3,4-dimethoxybenzylamino)-8-formyl-2-(2-furyl) [1,2,4]triazolo[1,5-c]pyrimidine and a known compound (WO99/21856), (2SR,11bSR)-9-methoxy-1,3,4,6,7,11b-hexahydro-2H-benzo[a]quinolizin-2-ylmethylamine, in a manner similar to that in Example 1.
$^1H$ NMR ($CDCl_3$, δ , ppm): 1.55-1.69 (m, 1H), 1.72-2.05 (m, 3H), 2.10-2.20 (m, 1H), 2.40-2.85 (m, 6H), 2.90-3.23 (m, 2H), 3.35-3.45 (m, 1H), 3.75 (s, 3H), 3.88 (s, 3H), 3.88 (s, 3H), 4.05 (d, J = 6.2 Hz, 2H), 4.75 (d, J = 5.9 Hz, 2H), 6.43 (t, J = 5.9 Hz, 1H), 6.57 (dd, J = 1.6, 3.5 Hz, 1H), 6.59 (d, J = 2.7 Hz, 1H), 6.63 (dd, J = 2.7, 8.6 Hz, 1H), 6.84 (d, J = 8.4 Hz, 1H), 6.95 (s, 1H), 6.97 (d, J = 8.4 Hz, 1H), 7.09 (d, J = 8.6 Hz, 1H), 7.20 (dd, J = 0.8, 3.5 Hz, 1H), 7.60 (dd, J = 0.8, 1.6 Hz, 1H), 7.88 (s, 1H)

Example 154

5-Amino-2-(2-furyl)-8-[(2SR,11bSR)-9-methoxy-1,3,4,6,7,11b-hexahydro-2H-benzo[a]quinolizin-2-ylmethylaminomethyl] [1,2,4]triazolo[1,5-c]pyrimidine (Compound 154)

In a manner similar to that in Example 2, the subject compound (46%) was obtained as white crystals from 5-(3,4-dimethoxybenzylamino)-2-(2-furyl)-8-[(2SR,11bSR)-9-methoxy-1,3,4,6,7,11b-hexahydro-2H-benzo[a]quinolizin-2-ylmethylaminomethyl] [1,2,4]triazolo[1,5-c]pyrimidine obtained in Example 153.
Melting point: 223-225°C
$^1H$ NMR ($CDCl_3$, δ, ppm): 1.54-1.71 (m, 1H), 1.76-2.05 (m, 3H), 2.10-2.24 (m, 1H), 2.36-2.88 (m, 6H), 2.91-3.22 (m, 2H), 3.33-3.46 (m, 1H), 3.75 (s, 3H), 4.02 (d, J = 14.0 Hz, 1H), 4.09 (d, J = 14.0 Hz, 1H), 6.17 (brs, 2H), 6.58 (dd, J = 1.4, 3.5 Hz, 1H), 6.59 (d, J = 2.7 Hz, 1H), 6.64 (dd, J = 2.7, 8.4 Hz, 1H), 7.09 (d, J = 8.4 Hz, 1H), 7.23 (dd, J = 0.5, 3.5 Hz, 1H), 7.63 (dd, J = 0.5, 1.4 Hz, 1H), 7.82 (s, 1H)

Example 155

8-(6,7-Dimethoxy-1,2,3,4-tetrahydroisoquinolin-2-ylmethyl)-2-(2-furyl)-5-(2-methoxyethylamino) [1,2,4]triazolo[1,5-c] pyrimidine (Compound 155)

The subject compound (92%) was obtained as white crystals from 8-formyl-2-(2-furyl)-5-(2-methoxyethylamino)[1,2,4]triazolo[1,5-c]pyrimidine obtained in Reference Example 63 and 6,7-dimethoxy-1,2,3,4-tetrahydroisoquinoline in a manner similar to that in Example 1.
Melting point: 139-141°C
$^1$H NMR ($CDCl_3$, δ, ppm): 2.80-2.95 (m, 4H), 3.42 (s, 3H), 3.63-3.72 (m, 2H), 3.70 (s, 2H), 3.81 (s, 3H), 3.83 (s, 3H), 3.80-3.90 (m, 2H), 3.99 (s, 2H), 6.45 (t, J = 6.8 Hz, 1H), 6.50 (s, 1H), 6.58 (dd, J = 2.0, 3.6 Hz, 1H), 6.59 (s, 1H), 7.25 (d, J = 3.6 Hz, 1H), 7.62 (d, J = 2.0 Hz, 1H), 7.97 (s, 1H)

Example 156

2-(2-Furyl)-5-(4-phenylpiperazin-1-yl)-8-(4-phenylpiperazin-1-ylmethyl)[1,2,4]triazolo[1,5-c]pyrimidine (Compound 156)

The subject compound (37%) was obtained as white crystals from 8-formyl-2-(2-furyl)-5-(4-phenylpiperazin-1-yl) [1,2,4]triazolo[1,5-c]pyrimidine obtained in Reference Example 59 and 1-phenylpiperazine in a manner similar to that in Example 1.
Melting point: 182.5-183.1°C
$^1$H NMR ($CDCl_3$, δ , ppm): 2.72-2.84 (m, 4H), 3.18-3.29 (m, 4H), 3.36-3.46 (m, 4H), 3.95 (s, 2H), 4.22-4.31 (m, 4H), 6.58 (dd, J = 1.7, 3.4 Hz, 1H), 6.81-7.07 (m, 6H), 7.19-7.47 (m, 5H), 7.63 (d, J = 1.8 Hz, 1H), 7.99 (s, 1H)

Example 157

5-(3,4-Dimethoxybenzylamino)-2-(2-furyl)-8-(1,2,3,4-tetrahydrobenzofuro[2,3-c]pyridin-2-ylmethyl) [1,2,4]triazolo [1,5-c]pyrimidine (Compound 157)

The subject compound (quantitative) was obtained as a pale brown oily matter from 5-(3,4-dimethoxybenzylamino)-8-formyl-2-(2-furyl)[1,2,4]triazolo[1,5-c]pyrimidine and a known compound (WO00/20421), 1,2,3,4-tetrahydrobenzofuro[2,3-c]pyridine, in a manner similar to that in Example 1.
$^1$H NMR ($CDCl_3$, δ, ppm) : 2.78 (t, J = 5.7 Hz, 2H), 3.03 (t, J = 5.7 Hz, 2H), 3.84 (brs, 2H), 3.88 (s, 3H), 3.88 (s, 3H), 4.10 (s, 2H), 4.77 (d, J = 5.9 Hz, 2H), 6.44 (t, J = 5.9 Hz, 1H), 6.57 (dd, J = 1.6, 3.2 Hz, 1H), 6.85 (d, J = 7.8 Hz, 1H), 6.96 (s, 1H), 6.98 (d, J = 7.8 Hz, 1H), 7.15 -7.25 (m, 3H), 7.37-7.45 (m, 2H), 7.60 (d, J = 1.6 Hz, 1H), 8.00 (s, 1H)

Example 158

5-Amino-2-(2-furyl)-8-(1,2,3,4-tetrahydrobenzofuro[2,3-c]pyridin-2-ylmethyl) [1,2,4]triazolo[1,5-c]pyrimidine (Compound 158)

In a manner similar to that in Example 2, the subject compound (75%) was obtained as white crystals from 5-(3,4-dimethoxybenzylamino)-2-(2-furyl)-8-(1,2,3,4-tetrahydrobenzofuro[2,3-c]pyridin-2-ylmethyl) [1,2,4]triazolo [1,5-c]pyrimidine obtained in Example 157.
Melting point: 140-142°C
$^1$H NMR ($CDCl_3$, δ , ppm) : 2.73-2.86 (m, 2H), 2.98-3.08 (m, 2H), 3.84 (s, 2H), 4.11 (s, 2H), 5.85 (brs, 2H), 6.59 (dd, J = 1.6, 3.2 Hz, 1H), 7.15-7.31 (m, 3H), 7.36-7.47 (m, 2H), 7.63 (d, J = 1.6 Hz, 1H), 7.96 (s, 1H)

Example 159

5-(3,4-Dimethoxybenzylamino)-2-(2-furyl)-8-[4-(2-trifluoromethylphenyl)piperazin-1-ylmethyl][1,2,4]triazolo[1,5-c] pyrimidine (Compound 159)

The subject compound (quantitative) was obtained as a pale brown oily matter from 5-(3,4-dimethoxybenzylamino)-8-formyl-2-(2-furyl)[1,2,4]triazolo[1,5-c]pyrimidine and 1-(2-trifluoromethylphenyl)piperazine in a manner similar to that in Example 1.
$^1$H NMR ($CDCl_3$, δ, ppm): 2.71-2.79 (m, 4H), 2.92-3.00 (m, 4H), 3.88 (s, 3H), 3.88 (s, 3H), 3.90 (s, 2H), 4.76 (d, J =

5.6 Hz, 2H), 6.41 (t, J = 5.6 Hz, 1H), 6.57 (dd, J = 1.8, 3.5 Hz, 1H), 6.85 (d, J = 7.9 Hz, 1H), 6.96 (s, 1H), 6.99 (d, J = 7.9 Hz, 1H), 7.17-7.20 (m, 1H), 7.22 (d, J = 3.5 Hz, 1H), 7.35-7.38 (m, 1H), 7.47-7.52 (m, 1H), 7.60 (d, J = 1.8 Hz, 1H), 7.60-7.64 (m, 1H), 7.96 (s, 1H)

Example 160

5-Amino-2-(2-furyl)-8-[4-(2-trifluoromethylphenyl)piperazin-1-ylmethyl][1,2,4]triazolo[1,5-c]pyrimidine (Compound 160)

In a manner similar to that in Example 2, the subject compound (93%) was obtained as white crystals from 5-(3,4-dimethoxybenzylamino)-2-(2-furyl)-8-[4-(2-trifluoromethylphenyl)piperazin-1-ylmethyl] [1,2,4]triazolo[1,5-c]pyrimidine obtained in Example 159.
Melting point: 199-200°C
$^1$H NMR ($CDCl_3$, δ, ppm) : 2.74-2.76 (m, 4H), 2.97-2.99 (m, 4H), 3.91 (s, 2H), 5.86 (s, 2H), 6.59 (dd, J = 1.8, 3.5 Hz, 1H), 7.20 (dd, J = 7.5, 7.5 Hz, 1H), 7.27 (d, J = 3.5 Hz, 1H), 7.37 (d, J = 7.5 Hz, 1H), 7.50 (dd, J = 7.5, 7.5 Hz, 1H), 7.60 (d, J = 7.5 Hz, 1H), 7.63 (d, J = 1.8 Hz, 1H), 7.92 (s, 1H)

Example 161

5-(3,4-Dimethoxybenzylamino)-2-(2-furyl)-8-(1,2,3,4-tetrahydro-9H-pyrido[3,4-b]indol-2-ylmethyl) [1,2,4]triazolo [1,5-c]pyrimidine (Compound 161)

The subject compound (95%) was obtained as a pale brown oily matter from 5-(3,4-dimethoxybenzylamino)-8-formyl-2-(2-furyl)[1,2,4]triazolo[1,5-c]pyrimidine and 1,2,3,4-tetrahydro-9H-pyrido[3,4-b]indole in a manner similar to that in Example 1.
$^1$H NMR (DMSO-$d_6$, δ, ppm): 2.68-2.70 (m, 2H), 2.85-2.88 (m, 2H), 3.68 (s, 2H), 3.73 (s, 3H), 3.73 (s, 3H), 3.92 (s, 2H), 4.64 (d, J = 5.6 Hz, 2H), 6.72 (dd, J = 1.8, 3.5 Hz, 1H), 6.73-7.03 (m, 7H), 7.08 (d, J = 3.5 Hz, 1H), 7.22-7.25 (m, 1H), 7.93 (d, J = 1.8 Hz, 1H), 7.94 (s, 1H), 8.69 (t, J = 5.6 Hz, 1H), 10.63 (s, 1H)

Example 162

5-Amino-2-(2-furyl)-8-(1,2,3,4-tetrahydro-9H-pyrido[3,4-b]indol-2-ylmethyl) [1,2,4]triazolo[1,5-c]pyrimidine (Compound 162)

In a manner similar to that in Example 2, the subject compound (21%) was obtained as white crystals from 5-(3,4-dimethoxybenzylamino)-2-(2-furyl)-8-(1,2,3,4-tetrahydro-9H-pyrido[3,4-b]indol-2-ylmethyl) [1,2,4]triazolo [1,5-c]pyrimidine obtained in Example 161.
Melting point: 243-245°C
$^1$H NMR ($CDCl_3$, δ , ppm) : 2.86-2.88 (m, 2H) , 3.02-3.06 (m, 2H), 3.84 (s, 2H), 4.09 (s, 2H), 5.88 (s, 2H), 6.58 (dd, J = 1.8, 3.5 Hz, 1H), 7.08-7.13 (m, 2H), 7.24 (d, J = 3.5 Hz, 1H), 7.46-7.48 (m, 2H), 7.63 (d, J = 1.8 Hz, 1H), 7.71 (s, 1H), 7.97 (s, 1H)

Example 163

2-Benzyl-5-(3,4-dimethoxybenzylamino)-8-(4-phenylpiperazin-1-ylmethyl)[1,2,4]triazolo[1,5-c]pyrimidine (Compound 163)

The subject compound (95%) was obtained as a white powder from 2-benzyl-5-(3,4-dimethoxybenzylamino)-8-formyl[1,2,4]triazolo[1,5-c]pyrimidine obtained in Reference Example 39 and 1-phenylpiperazine in a manner similar to that in Example 1.
Melting point: 92-94°C
$^1$H NMR ($CDCl_3$, δ, ppm): 2.73 (t, J = 4.9 Hz, 4H), 3.21 (t, J = 4.9 Hz, 4H), 3.85 (s, 2H), 3.87 (s, 6H), 4.23 (s, 2H), 4.71 (d, J = 5.8 Hz, 2H), 6.28 (t, J = 5.8 Hz, 1H), 6.81-6.96 (m, 6H), 7.19-7.41 (m, 7H), 7.91 (s, 1H)

Example 164

5-Amino-2-benzyl-8-(4-phenylpiperazin-1-ylmethyl)[1,2,4]triazolo[1,5-c]pyrimidine (Compound 164)

In a manner similar to that in Example 2, the subject compound (88%) was obtained as white crystals from 2-benzyl-5-(3,4-dimethoxybenzylamino)-8-(4-phenylpiperazin-1-ylmethyl)[1,2,4]triazolo[1,5-c]pyrimidine obtained in Example 163.
Melting point: 153-154°C
$^1$H NMR ($CDCl_3$, δ , ppm): 2.71 (t, J = 4.9 Hz, 4H), 3.21 (t, J = 4.9 Hz, 4H), 3.83 (s, 2H), 4.26 (s, 2H), 6.02 (brs, 2H), 6.80-6.93 (m, 3H), 7.16-7.42 (m, 7H), 7.83 (s, 1H)

Example 165

2-Cyclohexyl-5-(3,4-dimethoxybenzylamino)-8-(4-phenylpiperazin-1-ylmethyl) [1,2,4]triazolo[1,5-c]pyrimidine (Compound 165)

The subject compound (95%) was obtained as a white powder from 2-cyclohexyl-5-(3,4-dimethoxybenzylamino)-8-formyl[1,2,4]triazolo[1,5-c]pyrimidine obtained in Reference Example 45 and 1-phenylpiperazine in a manner similar to that in Example 1.
$^1$H NMR ($CDCl_3$, δ, ppm): 1.22-1.48 (m, 3H), 1.55-1.87 (m, 5H), 2.02-2.12 (m, 2H), 2.74 (t, J = 4.9 Hz, 4H), 2.91 (tt, J = 3.6, 11.5 Hz, 1H), 3.21 (t, J = 4.9 Hz, 4H), 3.86 (s, 2H), 3.88 (s, 6H), 4.74 (d, J = 5.8 Hz, 2H), 6.28 (t, J = 5.8 Hz, 1H), 6.80-6.98 (m, 6H), 7.20-7.27 (m, 2H), 7.89 (s, 1H)

Example 166

5-Amino-2-cyclohexyl-8-(4-phenylpiperazin-1-ylmethyl)[1,2,4]triazolo[1,5-c]pyrimidine (Compound 166)

In a manner similar to that in Example 2, the subject compound (65%) was obtained as a white powder from 2-cyclohexyl-5-(3,4-dimethoxybenzylamino)-8-(4-phenylpiperazin-1-ylmethyl)[1,2,4]triazolo[1,5-c]pyrimidine obtained in Example 165.
Melting point: 164-165°C
$^1$H NMR ($CDCl_3$, δ , ppm) : 1.24-1.52 (m, 3H), 1.58-1.95 (m, 5H), 2.04-2.16 (m, 2H), 2.73 (t, J = 4.9 Hz, 4H), 2.94 (tt, J = 3.6, 11.5 Hz, 1H), 3.22 (t, J = 4.9 Hz, 4H), 3.85 (s, 2H), 5.98 (brs, 2H), 6.80-6.93 (m, 3H), 7.21-7.28 (m, 2H), 7.83 (s, 1H)

Example 167

2-Benzyl-5-(3,4-dimethoxybenzylamino)-8-(1,2,3,4-tetrahydroisoquinolin-2-ylmethyl) [1,2,4]triazolo[1,5-c]pyrimidine (Compound 167)

The subject compound (95%) was obtained as a white powder from 2-benzyl-5-(3,4-dimethoxybenzylamino)-8-formyl[1,2,4]triazolo[1,5-c]pyrimidine obtained in Reference Example 39 and 1,2,3,4-tetrahydroisoquinoline in a manner similar to that in Example 1.
Melting point: 126-127°C
$^1$H NMR ($CDCl_3$, δ, ppm) : 2.81-2.97 (m, 4H) , 3.76 (s, 2H) , 3.86 (s, 3H), 3.87 (s, 3H), 3.94 (s, 2H), 4.24 (s, 2H), 4.71 (d, J = 5.8 Hz, 2H), 6.28 (t, J = 5.8 Hz, 1H), 6.81-7.39 (m, 12H), 7.95 (s, 1H)

Example 168

5-Amino-2-benzyl-8-(1,2,3,4-tetrahydroisoquinolin-2-ylmethyl)[1,2,4]triazolo[1,5-c]pyrimidine (Compound 168)

In a manner similar to that in Example 2, the subject compound (73%) was obtained as a white powder from 2-benzyl-5-(3,4-dimethoxybenzylamino)-8-(1,2,3,4-tetrahydroisoquinolin-2-ylmethyl) [1,2,4]triazolo[1,5-c]pyrimidine obtained in Example 167.
Melting point: 167-168°C
$^1$H NMR ($CDCl_3$, δ , ppm): 2.80-2.92 (m, 4H), 3.74 (s, 2H), 3.93 (s, 2H), 4.26 (s, 2H), 6.11 (brs, 2H), 6.93-7.41 (m, 9H), 7.88 (s, 1H)

Example 169

2-Cyclohexyl-5-(3,4-dimethoxybenzylamino)-8-(1,2,3,4-tetrahydroisoquinolin-2-ylmethyl)[1,2,4]triazolo[1,5-c]pyrimidine (Compound 169)

The subject compound (quantitative) was obtained as a yellow amorphous matter from 2-cyclohexyl-5-(3,4-dimethoxybenzylamino)-8-formyl[1,2,4]triazolo[1,5-c]pyrimidine obtained in Reference Example 45 and 1,2,3,4-tetrahydroisoquinoline in a manner similar to that in Example 1.
$^1$H NMR ($CDCl_3$, δ, ppm): 1.22-1.46 (m, 3H), 1.60-1.83 (m, 5H), 2.02-2.12 (m, 2H), 2.86-2.92 (m, 5H), 3.77 (s, 2H), 3.87 (s, 6H), 3.96 (s, 2H), 4.74 (d, J = 5.8 Hz, 2H), 6.30 (t, J = 5.8 Hz, 1H), 6.83-7.11 (m, 7H), 7.94 (s, 1H)

Example 170

5-Amino-2-cyclohexyl-8-(1,2,3,4-tetrahydroisoquinolin-2-ylmethyl)[1,2,4]triazolo[1,5-c]pyrimidine (Compound 170)

In a manner similar to that in Example 2, the subject compound (56%) was obtained as a white powder from 2-cyclohexyl-5-(3,4-dimethoxybenzylamino)-8-(1,2,3,4-tetrahydroisoquinolin-2-ylmethyl)[1,2,4]triazolo[1,5-c]pyrimidine obtained in Example 169.
Melting point: 155-156°C
$^1$H NMR ($CDCl_3$, δ , ppm) : 1.28-1.50 (m, 3H), 1.58-1.89 (m, 5H), 2.01-2.20 (m, 2H), 2.84-3.01 (m, 5H), 3.76 (s, 2H), 3.95 (s, 2H), 6.26 (brs, 2H), 6.95-7.13 (m, 4H), 7.88 (s, 1H)

Example 171

5-(3,4-Dimethoxybenzylamino)-8-(6,7-dimethoxy-3-methyl-1,2,3,4-tetrahydroisoquinolin-2-ylmethyl)-2-(2-furyl)[1,2,4] triazolo[1,5-c]pyrimidine (Compound 171)

The subject compound (quantitative) was obtained as a pale brown oily matter from 5-(3,4-dimethoxybenzylamino)-8-formyl-2-(2-furyl)[1,2,4]triazolo[1,5-c]pyrimidine and 6,7-dimethoxy-3-methyl-1,2,3,4-tetrahydroisoquinoline in a manner similar to that in Example 1.
$^1$H NMR ($CDCl_3$, δ, ppm): 1.23-1.25 (m, 3H), 2.04-2.61 (m, 1H), 2.94-3.02 (m, 1H), 3.23-3.30 (m, 1H) , 3.76-3.77 (m, 2H), 3.80 (s, 3H), 3.85 (s, 3H), 3.88 (s, 3H), 3.88 (s, 3H), 3.97-3.99 (m, 2H), 4.76 (d, J = 5.6 Hz, 2H), 6.39 (t, J = 5.6 Hz, 1H), 6.48 (s, 1H), 6.56 (dd, J = 1.8, 3.5 Hz, 1H), 6.58 (s, 1H), 6.85 (d, J = 8.0 Hz, 1H), 6.95 (s, 1H), 6.97 (d, J = 8.0 Hz, 1H), 7.20 (d, J = 3.5 Hz, 1H), 7.59 (d, J = 1.8 Hz, 1H), 8.01 (s, 1H)

Example 172

5-Amino-8-(6,7-dimethoxy-3-methyl-1,2,3,4-tetrahydroisoquinolin-2-ylmethyl)-2-(2-furyl) [1,2,4]triazolo[1,5-c] pyrimidine (Compound 172)

In a manner similar to that in Example 2, the subject compound (97%) was obtained as white crystals from 5-(3,4-dimethoxybenzylamino)-8-(6,7-dimethoxy-3-methyl-1,2,3,4-tetrahydroisoquinolin-2-ylmethyl)-2-(2-furyl) [1,2,4] triazolo[1,5-c]pyrimidine obtained in Example 171.
Melting point: 204-206°C
$^1$H NMR ($CDCl_3$, δ , ppm): 1.23-1.25 (m, 3H), 2.53-2.61 (m, 1H), 2.94-3.02 (m, 1H), 3.20-3.29 (m, 1H) , 3.75-3.76 (m, 2H), 3.80 (s, 3H), 3.85 (s, 3H), 3.98-4.01 (m, 2H), 5.98 (s, 2H), 6.47 (s, 1H), 6.57 (dd, J = 1.8, 3.5 Hz, 1H), 6.59 (s, 1H), 7.23 (d, J = 3.5 Hz, 1H), 7.62 (d, J = 1.8 Hz, 1H), 7.96 (s, 1H)

Example 173

5-(3,4-Dimethoxybenzylamino)-2-(2-furyl)-8-(2-isoindolinylmethyl)[1,2,4]triazolo[1,5-c]pyrimidine (Compound 173)

The subject compound (quantitative) was obtained as a pale brown oily matter from 5-(3,4-dimethoxybenzylamino)-8-formyl-2-(2-furyl)[1,2,4]triazolo[1,5-c]pyrimidine and isoindoline in a manner similar to that in Example 1.
$^1$H NMR ($CDCl_3$, δ, ppm): 3.88 (s, 3H), 3.88 (s, 3H), 4.09 (s, 4H), 4.21 (s, 2H), 4.77 (d, J = 5.5 Hz, 2H), 6.41 (t, J = 5.5 Hz, 1H), 6.56 (dd, J = 1.7, 3.4 Hz, 1H), 6.86 (d, J = 7.8 Hz, 1H), 6.96 (s, 1H), 6.98 (d, J = 7.8 Hz, 1H), 7.18 -7.26 (m, 4H), 7.22 (d, J = 3.4 Hz, 1H), 7.59 (d, J = 1.7 Hz, 1H), 8.02 (s, 1H)

Example 174

5-Amino-2-(2-furyl)-8-(2-isoindolinylmethyl) [1,2,4]triazolo[1,5-c]pyrimidine (Compound 174)

In a manner similar to that in Example 2, the subject compound (80%) was obtained as white crystals from 5- (3,4-dimethoxybenzylamino) -2-(2-furyl) -8-(2-isoindolinylmethyl) [1,2,4]triazolo[1,5-c]pyrimidine obtained in Example 173.
Melting point: 167-170°C
$^1$H NMR ($CDCl_3$, δ, ppm): 4.09 (s, 4H), 4.22 (s, 2H), 5.89 (s, 2H), 6.59 (dd, J = 1.8, 3.5 Hz, 1H), 7.18 (s, 4H), 7.25 (d, J = 3.5 Hz, 1H), 7.63 (d, J = 1.8 Hz, 1H), 7.97 (s, 1H)

Example 175

5- (2-Benzyloxyethylamino) -2- (2-furyl) -8- (4-phenylpiperazin-1-ylmethyl)[1,2,4]triazolo[1,5-c]pyrimidine (Compound 175)

The subject compound (69%) was obtained as a pale brown oily matter from 5-(2-benzyloxyethylamino)-8-formyl-2-(2-furyl)[1,2,4]triazolo[1,5-c]pyrimidine obtained in Reference Example 66 and 1-phenylpiperazine in a manner similar to that in Example 1.
$^1$H NMR ($CDCl_3$, δ, ppm): 2.66-2.85 (m, 4H) , 3.11-3.36 (m, 4H), 3.67-4.02 (m, 4H), 3.89 (s, 2H), 4.58 (s, 2H), 6.51 (t, J = 3.4 Hz, 1H), 6.58 (dd, J = 1.8, 3.5 Hz, 1H), 6.77 -7.00 (m, 3H), 7.12-7.45 (m, 8H), 7.63 (d, J = 1.8 Hz, 1H), 7.90 (s, 1H)

Example 176

5-(2-Benzyloxyethylamino)-2-(2-furyl)-8-(1,2,3,4-tetrahydroisoquinolin-2-ylmethyl)[1,2,4]triazolo[1,5-c]pyrimidine (Compound 176)

The subject compound (quantitative) was obtained as a pale brown oily matter from 5-(2-benzyloxyethylamino)-8-formyl-2-(2-furyl) [1,2,4]triazolo[1,5-c]pyrimidine obtained in Reference Example 66 and 1,2,3,4-tetrahydroisoquinoline in a manner similar to that in Example 1.
$^1$H NMR ($CDCl_3$, δ , ppm) : 2.74-2.98 (m, 2H), 3.70-3.91 (m, 4H), 3.81 (brs, 2H), 4.03 (brs, 2H), 4.58 (brs, 2H), 6.50 (t, J = 3.5 Hz, 1H), 6.58 (dd, J = 1.7, 3.5 Hz, 1H), 6.95 -7.16 (m, 6H), 7.20-7.36 (m, 6H), 7.62 (d, J = 1.7 Hz, 1H), 7.97 (s, 1H)

Example 177

5-(3-Benzyloxypropylamino)-2-(2-furyl)-8-(4-phenylpiperazin-1-ylmethyl)[1,2,4]triazolo[1,5-c]pyrimidine (Compound 177)

The subject compound (85%) was obtained as a brown oily matter from 5-(3-benzyloxypropylamino)-8-formyl-2-(2-furyl) [1,2,4]triazolo[1,5-c]pyrimidine obtained in Reference Example 65 and 1-phenylpiperazine in a manner similar to that in Example 1.
$^1$H NMR ($CDCl_3$, δ, ppm): 1.92-2.21 (m, 2H), 2.60-2.97 (m, 4H), 3.11-3.39 (m, 4H), 3.79-4.05 (m, 4H), 3.90 (s, 2H), 4.56 (s, 2H), 6.57 (dd, J = 1.8, 3.5 Hz, 1H), 6.78-7.07 (m, 2H), 7.15 (d, J = 3.5 Hz, 1H), 7.09-7.53 (m, 8H), 7.61 (d, J = 1.8 Hz, 1H), 7.86 (s, 1H)

Example 178

5-(3-Benzyloxypropylamino)-8-(6,7-dimethoxy-1,2,3,4-tetrahydroisoquinolin-2-ylmethyl)-2-(2-furyl)[1,2,4]triazolo [1,5-c]pyrimidine (Compound 178)

The subject compound (85%) was obtained as a brown oily matter from 5-(3-benzyloxypropylamino)-8-formyl-2-(2-furyl)[1,2,4]triazolo[1,5-c]pyrimidine obtained in Reference Example 65 and 6,7-dimethoxy-1,2,3,4-tetrahydroisoquinoline in a manner similar to that in Example 1.
$^1$H NMR ($CDCl_3$, δ, ppm): 1.94-2.12 (m, 2H), 2.82-3.06 (m, 4H), 3.61-3.95 (m, 6H), 3.80 (s, 3H), 3.83 (s, 3H), 4.01 - 4.13 (m, 2H), 4.56 (brs, 2H), 6.44-6.77 (m, 2H), 6.48 (s, 1H), 7.11-7.43 (m, 7H), 7.60 (brs, 1H), 7.86 (s, 1H)

Example 179

2-(2-Furyl)-5-(2-hydroxyethylamino)-8-(4-phenylpiperazin-1-ylmethyl)[1,2,4]triazolo[1,5-c]pyrimidine (Compound 179)

5-(2-Benzyloxyethylamino)-2-(2-furyl)-8-(4-phenylpiperazin-1-ylmethyl) [1,2,4]triazolo[1,5-c]pyrimidine (1.40 g, 2.75 mmol) obtained in Example 175 was dissolved in dichloromethane (10 ml), and dimethyl sulfide (9 ml, large excess) and boron trifluoride-diethyl ether complex (3.49 ml, 27.5 mmol) were added thereto under ice-cooling, followed by stirring at room temperature for 12 hours. The reaction mixture was poured into a saturated aqueous sodium hydrogencarbonate solution under ice-cooling and subjected to extraction with chloroform. The organic layer was dried over anhydrous magnesium sulfate, and the solvent was distilled away under reduced pressure. The resulting residue was recrystallized from ethyl acetate to obtain the subject compound (35%) as white crystals.
Melting point: 168.7-169.2°C
$^1$H NMR ($CDCl_3$, δ, ppm): 2.73-2.84 (m, 4H), 3.18-3.29 (m, 4H), 3.75-3.84 (m, 2H), 3.88 (s, 2H), 3.89-3.98 (m, 2H), 6.58 (dd, J = 1.8, 3.5 Hz, 1H), 6.62-6.71 (m, 1H), 6.79-6.96 (m, 3H), 7.18-7.33 (m, 3H), 7.61 (d, J = 1.8 Hz, 1H), 7.88 (s, 1H)

Example 180

2-(2-Furyl)-5-(2-hydroxyethylamino)-8-(1,2,3,4-tetrahydroisoquinolin-2-ylmethyl)[1,2,4]triazolo[1,5-c]pyrimidine (Compound 180)

In a manner similar to that in Example 179, the subject compound (quantitative) was obtained as white crystals from 5-(2-benzyloxyethylamino)-2-(2-furyl)-8-(1,2,3,4-tetrahydroisoquinolin-2-ylmethyl)[1,2,4]triazolo[1,5-c]pyrimidine obtained in Example 176.
Melting point: 177.2-177.8°C
$^1$H NMR ($CDCl_3$, δ, ppm) : 2.83-3.09 (m, 4H) , 3.60-3.72 (m, 2H), 3.75-4.03 (m, 2H), 3.84 (s, 2H), 3.94 (s, 2H), 6.57 (dd, J = 1.6, 3.3 HZ, 1H), 6.69 (t, J = 5.6 Hz, 1H), 7.00 -7.16 (m, 4H), 7.20 (d, J = 3.3 Hz, 1H), 7.60 (d, J = 1.6 Hz, 1H), 7.85 (s, 1H)

Example 181

2-(2-Furyl)-5-(3-hydroxypropylamino)-8-(4-phenylpiperazin-1-ylmethyl)[1,2,4]triazolo[1,5-c]pyrimidine (Compound 181)

In a manner similar to that in Example 179, the subject compound (92%) was obtained as white crystals from 5-(3-benzyloxypropylamino)-2-(2-furyl)-8-(4-phenylpiperazin-1-ylmethyl)[1,2,4]triazolo[1,5-c]pyrimidine obtained in Example 177.
Melting point: 156.0-156.8°C
$^1$H NMR ($CDCl_3$, δ, ppm): 1.81-1.98 (m, 2H) , 2.70-2.83 (m, 4H), 3.17-3.28 (m, 4H), 3.65-3.76 (m, 3H), 3.77-3.92 (m, 2H), 3.89 (s, 2H), 6.42 (t, J = 6.3 Hz, 1H), 6.58 (dd, J = 1.8, 3.5 Hz, 1H), 6.79-6.96 (m, 3H), 7.18-7.30 (m, 2H), 7.23 (d, J = 3.5 Hz, 1H), 7.62 (d, J = 1.8 Hz, 1H), 7.90 (s, 1H)

Example 182

8-(6,7-Dimethoxy-1,2,3,4-tetrahydroisoquinolin-2-ylmethyl)-2-(2-furyl)-5-(3-hydroxypropylamino)[1,2,4]triazolo[1,5-c]pyrimidine (Compound 182)

In a manner similar to that in Example 179, the subject compound (69%) was obtained as white crystals from 5-(3-benzyloxypropylamino)-8-(6,7-dimethoxy-1,2,3,4-tetrahydroisoquinolin-2-ylmethyl)-2-(2-furyl)[1,2,4]triazolo[1,5-c]pyrimidine obtained in Example 178.
Melting point: 174.8-175.3°C
$^1$H NMR ($CDCl_3$, δ, ppm): 1.81-1.97 (m, 2H), 2.77-2.98 (m, 4H), 3.63-3.76 (m, 5H), 3.76-3.92 (m, 2H), 3.82 (s, 3H), 3.84 (s, 3H), 3.92-4.02 (m, 2H), 6.36-6.46 (m, 1H), 6.51 (s, 1H), 6.54-6.67 (m, 1H), 6.59 (s, 1H), 7.19-7.32 (m, 1H), 7.62 (s, 1H), 7.94 (s, 1H)

Example 183

5-(3,4-Dimethoxybenzylamino)-8-(6,7-dimethoxy-1-methyl-1,2,3,4-tetrahydroisoquinolin-2-ylmethyl)-2-(2-furyl) [1,2,4]triazolo[1,5-c]pyrimidine (Compound 183)

The subject compound (quantitative) was obtained as a pale brown oily matter from 5-(3,4-dimethoxybenzylamino)-8-formyl-2-(2-furyl) [1,2,4]triazolo[1,5-c]pyrimidine and 6,7-dimethoxy-1-methyl-1,2,3,4-tetrahydroisoquinoline in a manner similar to that in Example 1.
$^1$H NMR ($CDCl_3$, δ , ppm): 1.46 (d, J = 6. 8 Hz, 3H) , 2.63-2.69 (m, 2H), 3.16-3.25 (m, 2H), 3.84 (s, 3H), 3.85 (s, 3H), 3.88 (s, 3H), 3.88 (s, 3H), 3.93 (q, J = 6.8 Hz, 1H), 4.06 (d, J = 6.8 Hz, 2H), 4.76 (d, J = 5.6 Hz, 2H), 6.38 (t, J = 5.6 Hz, 1H), 6.55 (s, 1H), 6.57 (dd, J = 1.8, 3.5 Hz, 1H), 6.59 (s, 1H), 6.85 (d, J = 7.7 Hz, 1H), 6.96 (s, 1H), 6.97 (d, J = 7.7 Hz, 1H), 7.20 (dd, J = 0.8, 3.5 Hz, 1H), 7.59 (dd, J = 0.8, 1.8 Hz, 1H), 8.06 (s, 1H)

Example 184

5-Amino-8-(6,7-dimethoxy-1-methyl-1,2,3,4-tetrahydroisoquinolin-2-ylmethyl)-2-(2-furyl) [1,2,4]triazolo[1,5-c] pyrimidine (Compound 184)

In a manner similar to that in Example 2, the subject compound (quantitative) was obtained as white crystals from 5-(3,4-dimethoxybenzylamino)-8-(6,7-dimethoxy-1-methyl-2,2,3,4-tetrahydroisoquinolin-2-ylmethyl)-2-(2-furyl) [1,2,4]triazolo[1,5-c]pyrimidine obtained in Example 183.
Melting point: 218-219°C
$^1$H NMR ($CDCl_3$, δ, ppm): 1.45 (d, J = 6.6 Hz, 3H), 2.59-2.69 (m, 1H), 2.80-2.96 (m, 2H), 3.14-3.24 (m, 1H), 3.83 (s, 3H), 3.85 (s, 3H), 3.93 (q, J = 6.6 Hz, 1H), 4.06 (d, J = 6.6 Hz, 2H), 5.91 (s, 2H), 6.55 (s, 1H), 6.57 (dd, J = 1.8, 3.5 Hz, 1H), 6.59 (s, 1H), 7.23 (d, J = 3.5 Hz, 1H), 7.62 (d, J = 1.8 Hz, 1H), 8.01 (s, 1H)

Example 185

8-(6,7-Diethoxy-1,2,3,4-tetrahydroisoquinolin-2-ylmethyl)-5-(3,4-dimethoxybenzylamino)-2-(2-furyl)[1,2,4]triazolo [1,5-c]pyrimidine (Compound 185)

The subject compound (quantitative) was obtained as a pale brown oily matter from 5-(3,4-dimethoxybenzylamino)-8-formyl-2-(2-furyl)[1,2,4]triazolo[1,5-c]pyrimidine and 6,7-diethoxy-1,2,3,4-tetrahydroisoquinoline in a manner similar to that in Example 1.
$^1$H NMR ($CDCl_3$, δ, ppm) : 1.38-1.44 (m, 6H), 2.83-2.87 (m, 4H), 3.69 (s, 2H), 3.88 (s, 3H), 3.88 (s, 3H), 3.98 (s, 2H), 3.99-4.08 (m, 4H), 4.76 (d, J = 5.8 Hz, 2H), 6.41 (t, J = 5.8 Hz, 1H), 6.52 (s, 1H), 6.57 (dd, J = 1.8, 3.5 Hz, 1H), 6.61 (s, 1H), 6.85 (d, J = 7.9 Hz, 1H), 6.96 (s, 1H), 6.98 (d, J = 7.9 Hz, 1H), 7.23 (d, J = 3.5 Hz, 1H), 7.60 (d, J = 1.8 Hz, 1H), 8.01 (s, 1H)

Example 186

5-Amino-8-(6,7-diethoxy-1,2,3,4-tetrahydroisoquinolin-2-ylmethyl)-2-(2-furyl)[1,2,4]triazolo[1,5-c]pyrimidine (Compound 186)

In a manner similar to that in Example 2, the subject compound (29%) was obtained as white crystals from 8-(6,7-diethoxy-1,2,3,4-tetrahydroisoquinolin-2-ylmethyl)-5-(3,4-dimethoxybenzylamino)-2-(2-furyl)[1,2,4]triazolo [1,5-c]pyrimidine obtained in Example 185.
Melting point: 141-143°C
$^1$H NMR ($CDCl_3$, δ, ppm): 1.38-1.59 (m, 6H), 2.84-2.86 (m, 4H), 3.68 (s, 2H), 3.97-4.08 (m, 4H), 4.00 (s, 2H), 5.85 (s, 2H), 6.51 (s, 1H), 6.59 (dd, J = 1.8, 3.5 Hz, 1H), 6.61 (s, 1H), 7.25 (d, J = 3.5 Hz, 1H), 7.63 (d, J = 1.8 Hz, 1H), 7.97 (s, 1H)

Example 187

8-(7-Bromo-1,2,3,4-tetrahydroisoquinolin-2-ylmethyl)-5-(3,4-dimethoxybenzylamino)-2-(2-furyl)[1,2,4]triazolo[1,5-c] pyrimidine (Compound 187)

The subject compound (quantitative) was obtained as a pale brown oily matter from 5-(3,4-dimethoxybenzylamino)-8-formyl-2-(2-furyl)[1,2,4]triazolo[1,5-c]pyrimidine and 7-bromo-1,2,3,4-tetrahydroisoquinoline in a manner

similar to that in Example 1.
$^1$H NMR ($CDCl_3$, δ, ppm): 2.78-2.95 (m, 4H), 3.75 (s, 2H), 3.88 (s, 3H), 3.88 (s, 3H), 4.00 (s, 2H), 4.76 (d, J = 5.7 Hz, 2H), 6.43 (t, J = 5.7 Hz, 1H), 6.57 (dd, J = 1.6, 3.2 Hz, 1H), 6.83-7.30 (m, 7H), 7.60 (d, J = 1.6 Hz, 1H), 7.89 (s, 1H)

Example 188

5-Amino-8-(7-bromo-1,2,3,4-tetrahydroisoquinolin-2-ylmethyl)-2-(2-furyl)[1,2,4]triazolo[1,5-c]pyrimidine (Compound 188)

The subject compound (81%) was obtained as white crystals from a known compound (WO98/42711), 5-amino-8-formyl-2-(2-furyl)[1,2,4]triazolo[1,5-c]pyrimidine, and 7-bromo-1,2,3,4-tetrahydroisoquinoline in a manner similar to that in Example 1.
Melting point: 223-224°C
$^1$H NMR (DMSO-$d_6$, δ, ppm): 2.68-2.85 (m, 4H), 3.64 (s, 2H), 3.82 (s, 2H), 6.71 (dd, J = 1.6, 3.5 Hz, 1H), 7.06 (d, J = 7.8 Hz, 1H), 7.20 (dd, J = 0.5, 3.5 Hz, 1H), 7.26 (s, 1H), 7.28 (d, J = 7.8 Hz, 1H), 7.84 (s, 1H), 7.88 (brs, 2H), 7.92 (dd, J = 0.5, 1.6 Hz, 1H)

Example 189

5-Amino-8-(6-benzyloxy-7-methoxy-1,2,3,4-tetrahydroisoquinolin-2-ylmethyl) -2- (2-furyl) [1,2,4]triazolo[1,5-c] pyrimidine (Compound 189)

The subject compound (29%) was obtained as white crystals from 5-amino-8-formyl-2-(2-furyl)[1,2,4]triazolo [1,5-c]pyrimidine and 6-benzyloxy-7-methoxy-1,2,3,4-tetrahydroisoquinoline in a manner similar to that in Example 1.
Melting point: 194-195°C
$^1$H NMR (DMSO-$d_6$, δ, ppm): 2.65-2.80 (m, 4H), 3.53 (s, 2H), 3.68 (s, 3H), 3.80 (s, 2H), 5.00 (s, 2H), 6.63 (s, 1H), 6.71 (dd, J = 1.6, 3.2 Hz, 1H), 6.75 (s, 1H), 7.21 (d, J = 3.2 Hz, 1H), 7.26-7.45 (m, 5H), 7.83 (s, 1H), 7.88 (brs, 2H), 7.93 (d, J = 1.6 Hz, 1H)

Example 190

8-(6,7-Dihydroxy-1,2,3,4-tetrahydroisoquinolin-2-ylmethyl)-5-(3,4-dimethoxybenzylamino)-2-(2-furyl) [1,2,4]triazolo [1,5-c]pyrimidine (Compound 190)

The subject compound (33%) was obtained as a pale brown oily matter from 5-(3,4-dimethoxybenzylamino)-8-formyl-2-(2-furyl) [1,2,4]triazolo[1,5-c]pyrimidine and 6,7-dihydroxy-1,2,3,4-tetrahydroisoquinoline in a manner similar to that in Example 1.
$^1$H NMR (DMSO-$d_6$, δ, ppm): 2.55-2.70 (m, 4H), 3.45 (s, 2H), 3.70 (s, 3H), 3.73 (s, 3H), 3.77 (s, 2H), 4.63 (d, J = 6.5 Hz, 2H), 6.36 (s, 1H), 6.42 (s, 1H), 6.72 (dd, J = 1.9, 3.5 Hz, 1H), 6.87 (d, J = 8.4 Hz, 1H), 6.92 (dd, J = 1.6, 8.4 Hz, 1H), 7.08 (d, J = 1.6 Hz, 1H), 7.22 (dd, J = 0.5, 3.5 Hz, 1H), 7.87 (s, 1H), 7.93 (dd, J = 0.5, 1.9 Hz, 1H), 8.54 (s, 1H), 8.57 (s, 1H), 8.66 (t, J = 6.5 Hz, 1H)

Example 191

5-Amino-8-(6,7-dihydroxy-1,2,3,4-tetrahydroisoquinolin-2-ylmethyl)-2-(2-furyl)[1,2,4]triazolo[1,5-c]pyrimidine (Compound 191)

In a manner similar to that in Example 2, the subject compound (46%) was obtained as white crystals from 8-(6,7-dihydroxy-1,2,3,4-tetrahydroisoquinolin-2-ylmethyl)-5-(3,4-dimethoxybenzylamino)-2-(2-furyl) [1,2,4]triazolo [1,5-c]pyrimidine obtained in Example 190.
Melting point: 245-247°C
$^1$H NMR (DMSO-$d_6$, δ , ppm) : 2.70-2.92 (m, 4H), 3.30-3.40 (m, 4H), 6.45 (s, 1H), 6.51 (s, 1H), 6.73 (dd, J = 1.6, 3.2 Hz, 1H), 7.21 (d, J = 3.2 Hz, 1H), 7.95 (d, J = 1.6 Hz, 1H), 7.98 (brs, 1H), 8.15 (brs, 1H), 8.85 (brs, 1H)

Example 192

8-(7-Chloro-1,2,3,4-tetrahydroisoquinolin-2-ylmethyl)-5-(3,4-dimethoxybenzylamino)-2-(2-furyl)[1,2,4]triazolo[1,5-c]pyrimidine (Compound 192)

The subject compound (93%) was obtained as a pale brown oily matter from 5-(3,4-dimethoxybenzylamino)-8-formyl-2-(2-furyl)[1,2,4]triazolo[1,5-c]pyrimidine and 7-chloro-1,2,3,4-tetrahydroisoquinoline in a manner similar to that in Example 1.
$^1$H NMR ($CDCl_3$, δ, ppm) : 2.85-2.90 (m, 4H), 3.75 (s, 2H), 3.88 (s, 3H), 3.88 (s, 3H), 3.99 (s, 2H), 4.76 (d, J = 5.7 Hz, 2H), 6.42 (t, J = 5.7 Hz, 1H), 6.57 (dd, J = 1.9, 3.5 Hz, 1H), 6.83-7.12 (m, 6H), 7.23 (dd, J = 0.8, 3.5 Hz, 1H), 7.60 (dd, J = 0.8, 1.9 Hz, 1H), 7.99 (s, 1H)

Example 193

5-Amino-8-(7-chloro-1,2,3,4-tetrahydroisoquinolin-2-ylmethyl)-2-(2-furyl)[1,2,4]triazolo[1,5-c]pyrimidine (Compound 193)

In a manner similar to that in Example 2, the subject compound (60%) was obtained as white crystals from 8-(7-chloro-1,2,3,4-tetrahydroisoquinolin-2-ylmethyl)-5-(3,4-dimethoxybenzylamino)-2-(2-furyl)[1,2,4]triazolo[1,5-c]pyrimidine obtained in Example 192.
Melting point: 217-218°C
$^1$H NMR ($CDCl_3$, δ, ppm) : 2.80-2.95 (m, 4H), 3.74 (s, 2H), 4.00 (s, 2H), 5.88 (brs, 2H), 6.59 (dd, J = 1.9, 3.5 Hz, 1H), 6.97-7.15 (m, 3H) , 7.23-7.28 (m, 1H) , 7.63 (d, J = 1.9 Hz, 1H), 7.94 (s, 1H)

Example 194

5- (3,4-Dimethoxybenzylamino)-2- (2-furyl) -8- (7-methyl-1,2,3,4-tetrahydroisoquinolin-2-ylmethyl)[1,2,4]triazolo[1,5-c]pyrimidine (Compound 194)

The subject compound (31%) was obtained as a pale brown oily matter from 5-(3,4-dimethoxybenzylamino)-8-formyl-2-(2-furyl) [1,2,4]triazolo[1,5-c]pyrimidine and 7-methyl-1,2,3,4-tetrahydroisoquinoline in a manner similar to that in Example 1.
$^1$H NMR ($CDCl_3$, δ , ppm): 2.23 (s, 3H), 2.88 (s, 4H), 3.75 (s, 2H), 3.88 (s, 3H), 3.88 (s, 3H), 4.11 (s, 2H), 4.76 (d, J = 5.7 Hz, 2H), 6.40 (t, J = 5.7 Hz, 1H), 6.57 (dd, J = 1.6, 3.5 Hz, 1H), 6.80-7.03 (m, 6H), 7.22 (d, J = 3.5 Hz, 1H), 7.60 (d, J = 1.6 Hz, 1H) , 8.01 (s, 1H)

Example 195

5-Amino-2-(2-furyl)-8-(7-methyl-1,2,3,4-tetrahydroisoquinolin-2-ylmethyl) [1,2,4]triazolo[1,5-c]pyrimidine (Compound 195)

In a manner similar to that in Example 2, the subject compound (46%) was obtained as white crystals from 5-(3,4-dimethoxybenzylamino)-2-(2-furyl)-8-(7-methyl-1,2,3,4-tetrahydroisoquinolin-2-ylmethyl) [1,2,4]triazolo[1,5-c]pyrimidine obtained in Example 194.
Melting point: 178-179°C
$^1$H NMR ($CDCl_3$, δ , ppm): 2.27 (s, 3H), 2.88 (s, 4H), 3.75 (s, 2H), 4.00 (s, 2H), 6.02 (brs, 2H), 6.58 (dd, J = 2.2, 3.8 Hz, 1H), 6.82 (brs, 1H), 6.90-7.03 (m, 2H), 7.25 (d, J = 3.8 Hz, 1H), 7.62 (d, J = 2.2 Hz, 1H), 7.96 (s, 1H)

Example 196

5-Amino-8-(7-benzyloxy-6-methoxy-1,2,3,4-tetrahydroisoquinolin-2-ylmethyl)-2-(2-furyl)[1,2,4]triazolo[1,5-c]pyrimidine (Compound 196)

The subject compound (59%) was obtained as white crystals from 5-amino-8-formyl-2-(2-furyl)[1,2,4]triazolo[1,5-c]pyrimidine and 7-benzyloxy-6-methoxy-1,2,3,4-tetrahydroisoquinoline in a manner similar to that in Example 1.
Melting point: 176.5-177.0°C
$^1$H NMR (DMSO-$d_6$, δ, ppm): 2.60-2.85 (m, 4H), 3.52 (m, 2H), 3.71 (s, 3H), 3.81 (brs, 2H), 4.98 (s, 2H), 6.60-6.75 (m, 3H), 7.20 (d, J = 2.7 Hz, 1H), 7.25-7.40 (m, 5H), 7.89 (s, 1H), 7.93 (brs, 2H), 7.93 (s, 1H)

Example 197

5-Amino-8-(5,7-dichloro-1,2,3,4-tetrahydroisoquinolin-2-ylmethyl)-2-(2-furyl)[1,2,4]triazolo[1,5-c]pyrimidine (Compound 197)

The subject compound (54%) was obtained as white crystals from 5-amino-8-formyl-2-(2-furyl)[1,2,4]triazolo [1,5-c]pyrimidine and 5,7-dichloro-1,2,3,4-tetrahydroisoquinoline in a manner similar to that in Example 1.
Melting point: 233-234°C
$^1$H NMR (DMSO-$d_6$, δ, ppm): 2.65-2.88 (m, 4H), 3.64 (s, 2H), 3.83 (s, 2H), 6.71 (dd, J = 1.6, 3.2 Hz, 1H), 7.12-7.21 (m, 2H), 7.40 (d, J = 3.2 Hz, 1H), 7.83 (s, 1H), 7.90 (brs, 2H), 7.93 (d, J = 1.6 Hz, 1H)

Example 198

5-Amino-2-(2-furyl)-8-(5,6,7,8-tetrahydro-1,3-dioxolo[4,5-g]isoquinolin-6-ylmethyl)[1,2,4]triazolo[1,5-c]pyrimidine (Compound 198)

The subject compound (94%) was obtained as white crystals from 5-amino-8-formyl-2-(2-furyl)[1,2,4]triazolo [1,5-c]pyrimidine and 5,6,7,8-tetrahydro-1,3-dioxolo[4,5-g]isoquinoline in a manner similar to that in Example 1.
Melting point: 227-228°C
$^1$H NMR (DMSO-$d_6$, δ, ppm) : 2.70 (s, 4H), 3.52 (s, 2H), 3.79 (s, 2H), 5.90 (s, 2H), 6.59 (s, 1H), 6.63 (s, 1H), 6.71 (dd, J = 1.6, 3.2 Hz, 1H), 7.20 (d, J = 3.2 Hz, 1H), 7.83 (s, 1H), 7.87 (brs, 2H), 7.93 (d, J = 1.6 Hz, 1H)

Example 199

Mixture (2:3) of 5-amino-8-(7,8-dichloro-1,2,3,4-tetrahydroisoquinolin-2-ylmethyl)-2-(2-furyl) [1,2,4]triazolo[1,5-c] pyrimidine (Compound 199) and 5-amino-8-(6,7-dichloro-1,2,3,4-tetrahydroisoquinolin-2-ylmethyl)-2-(2-furyl)[1,2,4] triazolo[1,5-c]pyrimidine (Compound 200)

The subject compound (64%) was obtained as white crystals from 5-amino-8-formyl-2-(2-furyl)[1,2,4]triazolo [1,5-c]pyrimidine and a mixture (2:3) of 7,8-dichloro-1,2,3,4-tetrahydroisoquinoline and 6,7-dichloro-1,2,3,4-tetrahydroisoquinoline in a manner similar to that in Example 1.
Melting point: 202-203°C
$^1$H NMR (DMSO-$d_6$, δ, ppm) : 2.65-2.90 (m, 4H), 3.62 (s, 2Hx0.6), 3.72 (s, 2Hx0.4), 3.82 (s, 2Hx0.6), 3.91 (s, 2Hx0.4), 6.67-6.73 (m, 1H), 7.14 (d, J = 8.1 Hz, 1Hx0.4), 7.18-7.20 (m, 1H), 7.35 (s, 1Hx0.6), 7.38 (s, 1Hx0.6), 7.42 (d, J = 8.1 Hz, 1Hx0.4), 7.83 (s, 1Hx0.6), 7.86 (s, 1Hx0.4), 7.89 (brs, 2H), 7.90-7.93 (m, 1H)

Example 200

5-Amino-8-(7-fluoro-1,2,3,4-tetrahydroisoquinolin-2-ylmethyl)-2-(2-furyl)[1,2,4]triazolo[1,5-c]pyrimidine (Compound 201)

The subject compound (42%) was obtained as white crystals by preparing 5-(3,4-dimethoxybenzylamino)-8-(7-fluoro-1,2,3,4-tetrahydroisoquinolin-2-ylmethyl)-2-(2-furyl) [1,2,4]triazolo[1,5-c]pyrimidine from 5-(3,4-dimethoxybenzylamino)-8-formyl-2-(2-furyl)[1,2,4]triazolo[1,5-c]pyrimidine and 7-fluoro-1,2,3,4-tetrahydroisoquinoline in a manner similar to that in Example 1 and subsequently treating the obtained compound without purification in a manner similar to that in Example 2.
Melting point: 183-184°C
$^1$H NMR (DMSO-$d_6$, δ, ppm): 2.68-2.90 (m, 4H), 3.67 (s, 2H), 3.85 (s, 2H), 6.72 (dd, J = 1.6, 3.3 Hz, 1H), 6.82-7.15 (m, 3H), 7.20 (d, J = 3.3 Hz, 1H), 7.86 (s, 1H), 7.93 (brs, 2H), 7.93 (d, J = 1.6 Hz, 1H)

Example 201

5-(3,4-Dimethoxybenzylamino)-2-(2-furyl)-8-(4-phenylpiperidinomethyl)[1,2,4]triazolo[1,5-c]pyrimidine (Compound 202)

The subject compound (quantitative) was obtained as a pale brown oily matter from 5-(3,4-dimethoxybenzylamino)-8-formyl-2-(2-furyl)[1,2,4]triazolo[1,5-c]pyrimidine and 4-phenylpiperidine in a manner similar to that in Example 1.

$^1$H NMR ($CDCl_3$, δ, ppm) : 1.72-1.79 (m, 4H) , 2.18-2.32 (m, 2H), 2.40-2.58 (m, 1H), 3.08-3.20 (m, 2H), 3.86 (s, 3H), 3.86 (s, 3H), 3.86 (s, 2H), 4.74 (d, J = 5.7 Hz, 2H), 6.41 (t, J = 5.7 Hz, 1H), 6.55 (dd, J = 1.9, 3.5 Hz, 1H), 6.83 (d, J = 8.1 Hz, 1H), 6.94 (s, 1H), 6.95 (d, J = 8.1 Hz, 1H), 7.13-7.35 (m, 6H), 7.58 (d, J = 1.9 Hz, 1H), 7.96 (s, 1H)

Example 202

5-Amino-2-(2-furyl)-8-(4-phenylpiperidinomethyl)[1,2,4]triazolo[1,5-c]pyrimidine (Compound 203)

In a manner similar to that in Example 2, the subject compound (76%) was obtained as white crystals from 5-(3,4-dimethoxybenzylamino)-2-(2-furyl)-8-(4-phenylpiperidinomethyl) [1,2,4]triazolo[1,5-c]pyrimidine obtained in Example 201.
Melting point: 215.5-216.0°C
$^1$H NMR ($CDCl_3$, δ, ppm): 1.78-1.90 (m, 4H) , 2.20-2.35 (m, 2H), 2.42-2.60 (m, 1H), 3.08-3.20 (m, 2H), 3.89 (s, 2H), 5.83-5.88 (m, 2H), 6.59 (dd, J = 1.9, 3.5 Hz, 1H), 7.18-7.27 (m, 6H), 7.63 (dd, J = 0.8, 1.9 Hz, 1H), 7.93 (s, 1H)

Example 203

8-[4-(2,3-Dichlorophenyl)piperazin-1-ylmethyl]-5-(3,4-dimethoxybenzylamino)-2-(2-furyl)[1,2,4]triazolo[1,5-c]pyrimidine (Compound 204)

The subject compound (quantitative) was obtained as a pale brown oily matter from 5-(3,4-dimethoxybenzylamino)-8-formyl-2-(2-furyl)[1,2,4]triazolo[1,5-c]pyrimidine and 1-(2,3-dichlorophenyl)piperazine in a manner similar to that in Example 1.
$^1$H NMR ($CDCl_3$, δ, ppm): 2.75-2.85 (m, 4H) , 3.03-3.15 (m, 4H), 3.88 (s, 3H), 3.88 (s, 3H), 3.92 (s, 2H), 4.76 (d, J = 5.7 Hz, 2H), 6.44 (t, J = 5.7 Hz, 1H), 6.57 (dd, J = 1.9, 3.5 Hz, 1H), 6.83-7.17 (m, 6H), 7.23 (d, J = 3.5 Hz, 1H), 7.60 (d, J = 1.9 Hz, 1H), 7.96 (s, 1H)

Example 204

5-Amino-8-[4-(2,3-dichlorophenyl)piperazin-1-ylmethyl]-2-(2-furyl)[1,2,4]triazolo[1,5-c]pyrimidine (Compound 205)

In a manner similar to that in Example 2, the subject compound (52%) was obtained as white crystals from 8-[4-(2,3-dichlorophenyl)piperazin-1-ylmethyl]-5-(3,4-dimethoxybenzylamino)-2-(2-furyl)[1,2,4]triazolo[1,5-c]pyrimidine obtained in Example 203.
Melting point: 149.5-150.0°C
$^1$H NMR ($CDCl_3$, δ, ppm): 2.79 (t, J = 4.6 Hz, 4H), 3.10 (t, J = 4.6 Hz, 4H), 3.93 (s, 2H), 5.95 (brs, 2H), 6.59 (dd, J = 1.9, 3.5 Hz, 1H), 6.93-7.15 (m, 3H), 7.23-7.27 (m, 1H), 7.63 (dd, J = 0.8, 1.9 Hz, 1H), 7.90 (s, 1H)

Example 205

5-(3,4-Dimethoxybenzylamino)-8-(6,7-dimethoxy-1,2,3,4-tetrahydroisoquinolin-2-ylmethyl)-2-(2-furyl)[1,2,4]triazolo[1,5-c]pyrimidine (Compound 206)

The subject compound (quantitative) was obtained as a pale brown oily matter from 5-(3,4-dimethoxybenzylamino)-8-formyl-2-(2-furyl) [1,2,4]triazolo[1,5-c]pyrimidine and 6,7-dimethoxy-1,2,3,4-tetrahydroisoquinoline in a manner similar to that in Example 1.
$^1$H NMR ($CDCl_3$, δ , ppm): 2.87-2.88 (m, 4H), 3.71 (s, 2H), 3.81 (s, 3H), 3.84 (s, 3H), 3.88 (s, 3H), 3.88 (s, 3H), 3.99 (s, 2H), 4.76 (d, J = 5.6 Hz, 2H), 6.42 (t, J = 5.6 Hz, 1H), 6.50 (s, 1H), 6.57 (dd, J = 1.8, 3.5 Hz, 1H), 6.59 (s, 1H), 6.85 (d, J = 8.0 Hz, 1H), 6.94 (s, 1H), 6.99 (d, J = 8.0 Hz, 1H), 7.22 (d, J = 3.5 Hz, 1H), 7.60 (d, J = 1.8 Hz, 1H), 8.01 (s, 1H)

Example 206

5-Amino-8-(6,7-dimethoxy-1,2,3,4-tetrahydroisoquinolin-2-ylmethyl)-2-(2-furyl)[1,2,4]triazolo[1,5-c]pyrimidine (Compound 207)

In a manner similar to that in Example 2, the subject compound (77%) was obtained as white crystals from 5-(3,4-dimethoxybenzylamino)-8-(6,7-dimethoxy-1,2,3,4-tetrahydroisoquinolin-2-ylmethyl)-2-(2-furyl)[1,2,4]triazolo[1,5-c]pyrimidine obtained in Example 205.

Melting point: 220-222°C
$^1$H NMR ($CDCl_3$, δ, ppm): 2.86-2.88 (m, 4H) , 3.71 (s, 2H) , 3.81 (s, 3H), 3.84 (s, 3H), 4.01 (s, 2H), 5.93 (s, 2H), 6.50 (s, 1H), 6.59 (dd, J = 1.8, 3.5 Hz, 1H), 6.60 (s, 1H), 7.25 (d, J = 3.5 Hz, 1H), 7.63 (d, J = 1.8 Hz, 1H), 7.97 (s, 1H)

Example 207

5- (3,4-Dimethoxybenzylamino) -2-(2-furyl) -8-(5-methoxyisoindolin-2-ylmethyl)[1,2,4]triazolo[1,5-c]pyrimidine (Compound 208)

The subject compound (quantitative) was obtained as a pale brown oily matter from 5-(3,4-dimethoxybenzylamino)-8-formyl-2-(2-furyl) [1,2,4]triazolo[1,5-c]pyrimidine and 5-methoxyisoindoline in a manner similar to that in Example 1.
$^1$H NMR ($CDCl_3$, δ, ppm): 3.70 (s, 3H), 3.88 (s, 3H), 3.88 (s, 3H), 4.02 (s, 2H), 4.02 (s, 2H), 4.19 (s, 2H), 4.76 (d, J = 5.9 Hz, 2H), 6.46 (t, J = 5.9 Hz, 1H), 6.56 (dd, J = 1.9, 3.8 Hz, 1H), 6.72 (d, J = 8.9 Hz, 1H), 6.74 (s, 1H), 6.85 (d, J = 8.4 Hz, 1H), 6.95 (s, 1H), 6.97 (d, J = 8.4 Hz, 1H), 7.07 (d, J = 8.9 Hz, 1H), 7.22 (d, J = 3.8 Hz, 1H), 7.59 (d, J = 1.9 Hz, 1H), 8.00 (s, 1H)

Example 208

5-Amino-2-(2-furyl)-8-(5-methoxyisoindolin-2-ylmethyl)[1,2,4]triazolo[1,5-c]pyrimidine (Compound 209)

In a manner similar to that in Example 2, the subject compound (79%) was obtained as white crystals from 5-(3,4-dimethoxybenzylamino)-2-(2-furyl)-8-(5-methoxyisoindolin-2-ylmethyl)[1,2,4]triazolo[1,5-c]pyrimidine obtained in Example 207.
Melting point: 182-185°C
$^1$H NMR ($CDCl_3$, δ, ppm): 3.77 (s, 3H), 4.02 (s, 2H), 4.02 (s, 2H), 4.20 (s, 2H), 5.85-5.98 (m, 2H), 6.58 (dd, J = 1.6, 3.2 Hz, 1H), 6.73 (d, J = 9.2 Hz, 1H), 6.74 (s, 1H), 7.08 (d, J = 9.2 Hz, 1H), 7.25 (d, J = 3.2 Hz, 1H), 7.62 (d, J = 1.6 Hz, 1H), 7.97 (s, 1H)

Example 209

5-(3,4-Dimethoxybenzylamino)-2-(2-furyl)-8-(4-methoxyisoindolin-2-ylmethyl)[1,2,4]triazolo[1,5-c]pyrimidine (Compound 210)

The subject compound (99%) was obtained as a pale brown oily matter from 5-(3,4-dimethoxybenzylamino)-8-formyl-2-(2-furyl) [1,2,4]triazolo[1,5-c]pyrimidine and 4-methoxyisoindoline in a manner similar to that in Example 1.
$^1$H NMR ($CDCl_3$, δ, ppm): 3.79 (s, 3H), 3.88 (s, 3H), 3.88 (s, 3H), 4.06 (s, 2H), 4.11 (s, 2H), 4.20 (s, 2H), 4.76 (d, J = 5.9 Hz, 2H), 6.44 (t, J = 5.9 Hz, 1H), 6.56 (dd, J = 1.9, 3.5 Hz, 1H), 6.68 (d, J = 8.1 Hz, 1H), 6.79 (d, J = 7.6 Hz, 1H), 6.85 (d, J = 8.4 Hz, 1H), 6.96 (s, 1H), 6.97 (d, J = 8.4 Hz, 1H), 7.16 (dd, J = 7.6, 8.1 Hz, 1H), 7.22 (d, J = 3.5 Hz, 1H), 7.59 (d, J = 1.9 Hz, 1H), 8.02 (s, 1H)

Example 210

5-Amino-2-(2-furyl)-8-(4-methoxyisoindolin-2-ylmethyl)[1,2,4]triazolo[1,5-c]pyrimidine (Compound 211)

In a manner similar to that in Example 2, the subject compound (62%) was obtained as white crystals from 5-(3,4-dimethoxybenzylamino)-2-(2-furyl)-8-(4-methoxyisoindolin-2-ylmethyl)[1,2,4]triazolo[1,5-c]pyrimidine obtained in Example 209.
Melting point: 205-206°C
$^1$H NMR ($CDCl_3$, δ, ppm): 3.80 (s, 3H), 4.06 (s, 2H), 4.11 (s, 2H), 4.22 (s, 2H), 5.86 (brs, 2H), 6.58 (dd, J = 2.2, 3.8 Hz, 1H), 6.69 (d, J = 8.4 Hz, 1H), 6.80 (d, J = 7.3 Hz, 1H), 7.16 (dd, J = 7.3, 8.4 Hz, 1H), 7.25 (d, J = 3.8 Hz, 1H), 7.62 (d, J = 2.2 Hz, 1H), 7.98 (s, 1H)

Example 211

8-(1,4-Benzodioxan-2-ylmethylaminomethyl)-5-(3,4-dimethoxybenzylamino)-2-(2-furyl)[1,2,4]triazolo[1,5-c] pyrimidine (Compound 212)

The subject compound (quantitative) was obtained as a pale brown oily matter from 5-(3,4-dimethoxybenzylamino)-8-formyl-2-(2-furyl)[1,2,4]triazolo[1,5-c]pyrimidine and 1,4-benzodioxan-2-ylmethylamine in a manner similar to that in Example 1.
$^1$H NMR ($CDCl_3$, δ, ppm) : 2.85-3.02 (m, 2H), 3.88 (s, 3H), 3.88 (s, 3H), 4.00-4.10 (m, 1H), 4.08 (s, 2H), 4.20-4.40 (m, 2H), 4.74 (d, J = 5.9 Hz, 2H), 6.46 (t, J = 5.9 Hz, 1H), 6.57 (dd, J = 1.9, 3.5 Hz, 1H), 6.75-7.00 (m, 7H), 7.21 (dd, J = 0.8, 3.5 Hz, 1H), 7.60 (dd, J = 0.8, 1.9 Hz, 1H), 7.89 (s, 1H)

Example 212

5-Amino-8-(1,4-benzodioxan-2-ylmethylaminomethyl)-2-(2-furyl)[1,2,4]triazolo[1,5-c]pyrimidine (Compound 213)

In a manner similar to that in Example 2, the subject compound (52%) was obtained as a pale brown oily matter from 8-(1,4-benzodioxan-2-ylmethylaminomethyl)-5-(3,4-dimethoxybenzylamino)-2-(2-furyl)[1,2,4]triazolo [1,5-c]pyrimidine obtained in Example 211.
$^1$H NMR ($CDCl_3$, δ , ppm) : 2.79-3.05 (m, 2H) , 4.08 (s, 2H), 4.00-4.20 (m, 1H), 4.23-4.41 (m, 2H), 6.06 (brs, 2H), 6.60 (dd, J = 1.7, 3.1 Hz, 1H), 6.75-6.87 (m, 4H), 7.26 (d, J = 3.1 Hz, 1H), 7.63 (d, J = 1.7 Hz, 1H), 7.81 (s, 1H)

Example 213

5-(3,4-Dimethoxybenzylamino)-2-(2-furyl)-8-[7-(2-methoxyphenyl)-1,2,3,4-tetrahydroisoquinolin-2-ylmethyl] [1,2,4] triazolo[1,5-c]pyrimidine (Compound 214)

The subject compound (87%) was obtained as a pale brown oily matter from 5-(3,4-dimethoxybenzylamino)-8-formyl-2-(2-furyl)[1,2,4]triazolo[1,5-c]pyrimidine and 7-(2-methoxyphenyl)-1,2,3,4-tetrahydroisoquinoline in a manner similar to that in Example 1.
$^1$H NMR ($CDCl_3$, δ , ppm): 2.86-3.02 (m, 4H), 3.79 (s, 3H), 3.84 (s, 2H), 3.88 (s, 3H), 3.88 (s, 3H), 4.02 (s, 2H), 4.77 (d, J = 5.7 Hz, 2H), 6.43 (t, J = 5.7 Hz, 1H), 6.57 (dd, J = 1.8, 3.5 Hz, 1H), 6.83-7.05 (m, 5H), 7.10-7.35 (m, 5H), 7.23 (dd, J = 0.8, 3.5 Hz, 1H), 7.60 (dd, J = 0.8, 1.8 Hz, 1H), 8.03 (s, 1H)

Example 214

5-Amino-2-(2-furyl)-8-[7-(2-methoxyphenyl)-1,2,3,4-tetrahydroisoquinolin-2-ylmethyl][1,2,4]triazolo[1,5-c]pyrimidine (Compound 215)

In a manner similar to that in Example 2, the subject compound (80%) was obtained as white crystals from 5-(3,4-dimethoxybenzylamino) -2- (2-furyl) -8- [7-(2-methoxyphenyl)-1,2,3,4-tetrahydroisoquinolin-2-ylmethyl] [1,2,4] triazolo[1,5-c]pyrimidine obtained in Example 213.
Melting point: 203-205°C
$^1$H NMR ($CDCl_3$, δ, ppm) : 2.82-3.03 (m, 4H), 3.79 (s, 3H), 3.84 (s, 2H), 4.03 (s, 2H), 5.85-6.02 (m, 2H), 6.59 (dd, J = 1.8, 3.5 Hz, 1H), 6.90-7.05 (m, 2H), 7.11-7.18 (m, 2H), 7.24-7.33 (m, 4H), 7.63 (d, J = 1.8 Hz, 1H), 7.99 (s, 1H)

Example 215

5-(3,4-Dimethoxybenzylamino)-8-(6,7-dimethoxy-1,2,3,4-tetrahydroisoquinolin-2-ylmethyl)-2-phenyl[1,2,4]triazolo [1,5-c]pyrimidine (Compound 216)

The subject compound (quantitative) was obtained as a white powder from 5-(3,4-dimethoxybenzylamino)-8-formyl-2-phenyl[1,2,4]triazolo[1,5-c]pyrimidine obtained in Reference Example 7 and 6,7-dimethoxy-1,2,3,4-tetrahydroisoquinoline in a manner similar to that in Example 1.
$^1$H NMR (DMSO-$d_6$, δ, ppm): 2.84-2.90 (m, 4H), 3.71 (s, 2H), 3.81 (s, 3H), 3.84 (s, 3H), 3.89 (s, 6H), 3.99 (s, 2H), 4.78 (d, J = 5.4 Hz, 2H), 6.37 (t, J = 5.4 Hz, 1H), 6.51 (s, 1H), 6.60 (s, 1H), 6.84-7.02 (m, 3H), 7.14 (t, J = 7.3 Hz, 1H), 7.44 (d, J = 7.3 Hz, 2H), 7.90 (t, J = 7.3 Hz, 2H), 7.99 (s, 1H)

Example 216

5-Amino-8-(6,7-dimethoxy-1,2,3,4-tetrahydroisoquinolin-2-ylmethyl)-2-phenyl[1,2,4]triazolo[1,5-c]pyrimidine (Compound 217)

In a manner similar to that in Example 2, the subject compound (51%) was obtained as a white powder from 5-(3,4-dimethoxybenzylamino)-8-(6,7-dimethoxy-1,2,3,4-tetrahydroisoquinolin-2-ylmethyl)-2-phenyl[1,2,4]triazolo [1,5-c]pyrimidine obtained in Example 215.
Melting point: 173-174°C
$^1$H NMR (DMSO-$d_6$, δ , ppm): 2.72-2.76 (m, 4H), 3.55 (s, 2H), 3.66 (s, 3H), 3.69 (s, 3H), 3.85 (s, 2H), 6.60 (s, 1H), 6.65 (s, 1H), 7.51-7.56 (m, 3H), 7.83 (s, 1H), 7.84 (brs, 2H), 8.21-8.26 (m, 2H), 7.84 (s, 1H)

Example 217

5-(3,4-Dimethoxybenzylamino)-8-(6,7-dimethoxy-1,2,3,4-tetrahydroisoquinolin-2-ylmethyl)-2-(2-thienyl)[1,2,4]triazolo [1,5-c]pyrimidine (Compound 218)

The subject compound (86%) was obtained as a white powder from 5-(3,4-dimethoxybenzylamino)-8-formyl-2-(2-thienyl)[1,2,4]triazolo[1,5-c]pyrimidine obtained in Reference Example 12 and 6,7-dimethoxy-1,2,3,4-tetrahydroisoquinoline in a manner similar to that in Example 1.
$^1$H NMR ($CDCl_3$, δ, ppm): 2.85-2.90 (m, 4H), 3.72 (s, 2H), 3.82 (s, 3H), 3.84 (s, 3H), 3.89 (s, 6H), 3.99 (s, 2H), 4.78 (d, J = 5.4 Hz, 2H), 6.34 (t, J = 5.4Hz, 1H), 6.51 (s, 1H), 6.60 (s, 1H), 6.85-6.91 (m, 1H) , 6.97-7.03 (m, 2H), 7.12-7.16 (m, 1H), 7.43-7.46 (m, 1H), 7.89-7.91 (m, 1H) , 7.99 (s, 1H)

Example 218

5-Amino-8-(6,7-dimethoxy-1,2,3,4-tetrahydroisoquinolin-2-ylmethyl)-2-(2-thienyl)[1,2,4]triazolo[1,5-c]pyrimidine (Compound 219)

In a manner similar to that in Example 2, the subject compound (33%) was obtained as a white powder from 5-(3,4-dimethoxybenzylamino)-8-(6,7-dimethoxy-1,2,3,4-tetrahydroisoquinolin-2-ylmethyl)-2-(2-thienyl)[1,2,4]triazolo [1,5-c]pyrimidine obtained in Example 217.
Melting point: 243-244°C
$^1$H NMR (DMSO-$d_6$, δ, ppm) : 2.71-2.75 (m, 4H) , 3.38 (s, 2H) , 3.66 (s, 3H), 3.73 (s, 3H), 4.09 (s, 2H), 6.60 (s, 1H), 6.65 (s, 1H), 7.23 (dd, J = 3.6, 4.9 Hz, 1H), 7.77 (dd, J = 1.2, 4.9 Hz, 1H), 7.82-7.85 (m, 3H)

Example 219

5-(3,4-Dimethoxybenzylamino)-8-(6,7-dimethoxy-1,2,3,4-tetrahydroisoquinolin-2-ylmethyl)-2-(3-pyridyl) [1,2,4] triazolo[1,5-c]pyrimidine (Compound 220)

The subject compound (73%) was obtained as a white powder from 5-(3,4-dimethoxybenzylamino)-8-formyl-2-(3-pyridyl)[1,2,4]triazolo[1,5-c]pyrimidine obtained in Reference Example 23 and 6,7-dimethoxy-1,2,3,4-tetrahydroisoquinoline in a manner similar to that in Example 1.
$^1$H NMR ($CDCl_3$, δ, ppm) : 2.86-2.91 (m, 4H) , 3.73 (s, 2H) , 3.81 (s, 3H), 3.82 (s, 3H), 3.89 (s, 6H), 4.01 (s, 2H), 4.80 (d, J = 5.4 Hz, 2H), 6.45 (t, J = 5.4 Hz, 1H), 6.51 (s, 1H), 6.60 (s, 1H), 6.87-7.02 (m, 3H), 7.41 (ddd, J = 1.0, 5.0, 7.9 Hz, 1H), 8.01 (s, 1H), 8.54 (ddd, J = 2.0, 2.0, 7.9 Hz, 1H), 8.70 (dd, J = 2.0, 5.0 Hz, 1H), 9.48 (dd, J = 1.0, 2.0 Hz, 1H)

Example 220

5-Amino-8-(6,7-dimethoxy-1,2,3,4-tetrahydroisoquinolin-2-ylmethyl)-2-(3-pyridyl)[1,2,4]triazolo[1,5-c]pyrimidine (Compound 221)

In a manner similar to that in Example 2, the subject compound (24%) was obtained as a white powder from 5-(3,4-dimethoxybenzylamino)-8-(6,7-dimethoxy-1,2,3,4-tetrahydroisoquinolin-2-ylmethyl)-2-(3-pyridyl)[1,2,4]triazolo [1,5-c]pyrimidine obtained in Example 219.
Melting point: 206°C
$^1$H NMR (DMSO-$d_6$, δ , ppm) : 2.72-2.76 (m, 4H) , 3.58 (s, 2H), 3.66 (s, 3H), 3.69 (s, 3H), 3.88 (s, 2H), 6.61 (s, 1H),

6.65 (s, 1H), 7.60 (ddd, J = 1.0, 5.0, 7.9 Hz, 1H), 7.88 (s, 1H), 7.95 (brs, 2H), 8.52 (ddd, J = 2.0, 2.0, 7.9 Hz, 1H), 8.73 (dd, J = 2.0, 5.0 Hz, 1H), 9.38 (dd, J = 1.0, 2.0 Hz, 1H)

Example 221

5-(3,4-Dimethoxybenzylamino)-8-(6,7-dimethoxy-1,2,3,4-tetrahydroisoquinolin-2-ylmethyl)-2-(3-furyl)[1,2,4]triazolo [1,5-c]pyrimidine (Compound 222)

The subject compound (86%) was obtained as a white powder from 5-(3,4-dimethoxybenzylamino)-8-formyl-2-(3-furyl)[1,2,4]triazolo[1,5-c]pyrimidine obtained in Reference Example 19 and 6,7-dimethoxy-1,2,3,4-tetrahydroisoquinoline in a manner similar to that in Example 1.
$^1$H NMR (DMSO-$d_6$, $\delta$, ppm): 2.84-2.90 (m, 4H), 3.71 (s, 2H), 3.84 (s, 3H), 3.87 (s, 3H), 3.91 (s, 6H), 4.01 (s, 2H), 4.80 (d, J = 5.4 Hz, 2H), 6.40 (t, J = 5.4 Hz, 1H), 6.52 (s, 1H), 6.61 (s, 1H), 6.90-7.22 (m, 4H), 7.53 (dd, J = 1.7, 1.8 Hz, 1H), 8.20 (s, 1H), 8.22 (dd, J = 0.8, 1.7 Hz, 1H)

Example 222

5-Amino-8-(6,7-dimethoxy-1,2,3,4-tetrahydroisoquinolin-2-ylmethyl)-2-(3-furyl)[1,2,4]triazolo[1,5-c]pyrimidine (Compound 223)

In a manner similar to that in Example 2, the subject compound (8.3%) was obtained as a white powder from 5-(3,4-dimethoxybenzylamino)-8-(6,7-dimethoxy-1,2,3,4-tetrahydroisoquinolin-2-ylmethyl)-2-(3-furyl)[1,2,4]triazolo [1,5-c]pyrimidine obtained in Example 221.
Melting point: 219°C
$^1$H NMR (DMSO-$d_6$, $\delta$, ppm): 2.70-2.74 (m, 4H), 3.52 (s, 2H), 3.66 (s, 3H), 3.69 (s, 3H), 3.80 (s, 2H), 6.60 (s, 1H), 6.65 (s, 1H), 7.00 (dd, J = 0.7, 2.0 Hz, 1H), 7.80 (brs, 2H), 7.82 (s, 1H), 7.86 (dd, J = 1.6, 2.0 Hz, 1H), 8.38 (dd, J = 0.7, 1.6 Hz, 1H)

Example 223

2-Cyclohexyl-5-(3,4-dimethoxybenzylamino)-8-(6,7-dimethoxy-1,2,3,4-tetrahydroisoquinolin-2-ylmethyl)[1,2,4] triazolo[1,5-c]pyrimidine (Compound 224)

The subject compound (quantitative) was obtained as a brown amorphous matter from 2-cyclohexyl-5-(3,4-dimethoxybenzylamino)-8-formyl[1,2,4]triazolo[1,5-c]pyrimidine obtained in Reference Example 45 and 6,7-dimethoxy-1,2,3,4-tetrahydroisoquinoline in a manner similar to that in Example 1.
$^1$H NMR ($CDCl_3$, $\delta$, ppm): 1.23-2.18 (m, 11H), 2.80-2.84 (m, 4H), 3.67 (s, 2H), 3.79 (s, 3H), 3.81 (s, 3H), 3.86 (s, 6H), 3.92 (s, 2H), 4.72 (d, J = 5.4 Hz, 2H), 6.28 (t, J = 5.4 Hz, 1H), 6.47 (s, 1H), 6.57 (s, 1H), 6.80-6.96 (m, 3H), 7.92 (s, 1H)

Example 224

5-Amino-2-cyclohexyly-8-(6,7-dimethoxy-1,2,3,4-tetrahydroisoquinolin-2-ylmethyl)[1,2,4]triazolo[1,5-c]pyrimidine (Compound 225)

In a manner similar to that in Example 2, the subject compound (29%) was obtained as a white powder from 2-cyclohexyl-5-(3,4-dimethoxybenzylamino)-8-(6,7-dimethoxy-1,2,3,4-tetrahydroisoquinolin-2-ylmethyl)[1,2,4]triazolo [1,5-c]pyrimidine obtained in Example 223.
Melting point: 145-146°C
$^1$H NMR (DMSO-$d_6$, $\delta$ , ppm): 1.13-2.10 (m, 11H), 2.67-2.73 (m, 4H), 3.50 (s, 2H), 3.66 (s, 3H), 3.69 (s, 3H), 3.74 (s, 2H), 6.59 (s, 1H), 6.64 (s, 1H), 7.64 (brs, 2H), 7.75 (s, 1H)

Example 225

2-Benzyl-5-(3,4-dimethoxybenzylamino)-8-(6,7-dimethoxy-1,2,3,4-tetrahydroisoquinolin-2-ylmethyl)[1,2,4]triazolo [1,5-c]pyrimidine (Compound 226)

The subject compound (65%) was obtained as a white powder from 2-benzyl-5-(3,4-dimethoxybenzylamino)-8-formyl[1,2,4]triazolo[1,5-c]pyrimidine obtained in Reference Example 39 and 6,7-dimethoxy-1,2,3,4-tetrahydroisoquinoline in a manner similar to that in Example 1.
$^1H$ NMR (DMSO-$d_6$, δ, ppm): 2.65-2.70 (m, 4H), 3.48 (s, 2H), 3.65 (s, 3H), 3.68 (s, 3H), 3.70 (s, 3H), 3.72 (s, 3H), 3.78 (s, 2H), 4.21 (s, 2H), 4.60 (d, J = 5.4 Hz, 2H), 6.55 (s, 1H), 6.63 (s, 1H), 6.86-6.89 (m, 2H), 7.05 (s, 1H), 7.21-7.35 (m, 5H), 7.83 (s, 1H), 8.57 (s, 1H)

Example 226

5-Amino-2-benzyl-8-(6,7-dimethoxy-1,2,3,4-tetrahydroisoquinolin-2-ylmethyl)[1,2,4]triazolo[1,5-c]pyrimidine (Compound 227)

In a manner similar to that in Example 2, the subject compound (51%) was obtained as a white powder from 2-benzyl-5-(3,4-dimethoxybenzylamino)-8-(6,7-dimethoxy-1,2,3,4-tetrahydroisoquinolin-2-ylmethyl)[1,2,4]triazolo [1,5-c]pyrimidine obtained in Example 225.
Melting point: 160-161°C
$^1H$ NMR (DMSO-$d_6$, δ, ppm): 2.68-2.73 (m, 4H), 3.29 (s, 2H), 3.48 (s, 2H), 3.66 (s, 3H), 3.68 (s, 3H), 3.74 (s, 2H), 4.19 (s, 2H), 6.55 (s, 1H), 6.63 (s, 1H), 7.18-7.37 (m, 5H), 7.74 (brs, 2H), 7.77 (s, 1H)

Example 227

5-(3,4-Dimethoxybenzylamino)-8-(4-phenylpiperazin-1-ylmethyl)-2-(2-pyridyl)[1,2,4]triazolo[1,5-c]pyrimidine (Compound 228)

The subject compound (55%) was obtained as a white powder from 5-(3,4-dimethoxybenzylamino)-8-formyl-2-(2-pyridyl)[1,2,4]triazolo[1,5-c]pyrimidine obtained in Reference Example 71 and 1-phenylpiperazine in a manner similar to that in Example 1.
$^1H$ NMR ($CDCl_3$, δ, ppm): 2.78 (t, J = 5.0 Hz, 4H) , 3.23 (t, J = 5.0 Hz, 4H), 3.88 (s, 6H), 3.95 (s, 2H), 4.77 (d, J = 5.8 Hz, 2H), 6.73-7.05 (m, 7H), 7.25 (t, J = 7.4 Hz, 2H), 7.38 (ddd, J = 1.3, 4.8, 7.6 Hz, 1H), 7.86 (ddd, J = 1.8, 7.6, 7.9 Hz, 1H), 7.98 (s, 1H), 8.40 (ddd, J = 0.8, 1.3, 7.9 Hz, 1H), 8.78 (ddd, J = 0.8, 1.8, 4.8 Hz, 1H)

Example 228

5-Amino-8-(4-phenylpiperazin-1-ylmethyl)-2-(2-pyridyl)[1,2,4]triazolo[1,5-c]pyrimidine (Compound 229)

In a manner similar to that in Example 2, the subject compound (38%) was obtained as a white powder from 5-(3,4-dimethoxybenzylamino)-8-(4-phenylpiperazin-1-ylmethyl)-2-(2-pyridyl)[1,2,4]triazolo[1,5-c]pyrimidine obtained in Example 227.
Melting point: 234-235°C
$^1H$ NMR ($CDCl_3$, δ , ppm): 2.79 (t, J = 4.9 Hz, 4H), 3.24 (t, J = 4.9 Hz, 4H), 3.96 (s, 2H), 6.17 (brs, 2H), 6.84 (t, J = 7.3 Hz, 1H), 6.91 (d, J = 7.3 Hz, 2H), 7.25 (t, J = 7.3 Hz, 2H), 7.42 (ddd, J = 1.3, 4.8, 7.6 Hz, 1H), 7.88 (ddd, J = 1.8, 7.6, 7.9 Hz, 1H), 7.93 (s, 1H), 8.41 (ddd, J = 0.8, 1.3, 7.9 Hz, 1H), 8.82 (ddd, J = 0.8, 1.8, 4.8 Hz, 1H)

Example 229

5-(3,4-Dimethoxybenzylamino)-2-(2-pyridyl)-8-(1,2,3,4-tetrahydroisoquinolin-2-ylmethyl)[1,2,4]triazolo[1,5-c] pyrimidine (Compound 230)

The subject compound (quantitative) was obtained as a brown amorphous matter from 5-(3,4-dimethoxybenzylamino)-8-formyl-2-(2-pyridyl)[1,2,4]triazolo[1,5-c]pyrimidine obtained in Reference Example 71 and 1,2,3,4-tetrahydroisoquinoline in a manner similar to that in Example 1.
$^1H$ NMR ($CDCl_3$, δ, ppm) : 2.92-2.94 (m, 4H) , 3.81 (s, 2H), 3.88 (s, 6H), 4.04 (s, 2H), 4.76 (d, J = 5.8 Hz, 2H), 6.69 (t, J = 5.8 Hz, 1H), 6.82-7.14 (m, 7H), 7.39 (ddd, J = 1.3, 4.8, 7.6 Hz, 1H), 7.85 (ddd, J = 1.8, 7.6, 7.9 Hz, 1H), 8.03 (s, 1H), 8.41 (ddd, J = 0.8, 1.3, 7.9 Hz, 1H), 8.79 (ddd, J = 0.8, 1.8, 4.8 Hz, 1H)

Example 230

5-Amino-2-(2-pyridyl)-8-(1,2,3,4-tetrahydroisoquinolin-2-ylmethyl)[1,2,4]triazolo[1,5-c]pyrimidine (Compound 231)

In a manner similar to that in Example 2, the subject compound (20%) was obtained as a white powder from

5-(3,4-dimethoxybenzylamino)-2-(2-pyridyl)-8-(1,2,3,4-tetrahydroisoquinolin-2-ylmethyl)[1,2,4]triazolo[1,5-c]pyrimidine obtained in Example 229.
$^1$H NMR ($CDCl_3$, δ, ppm) : 2.76-2.84 (m, 4H) , 3.67 (s, 2H) , 3.88 (s, 2H), 6.93-7.18 (m, 4H), 7.54 (ddd, J = 1.3, 4.8, 7.6 Hz, 1H), 7.87 (s, 1H), 7.93 (brs, 2H), 8.00 (ddd, J = 1.8, 7.6, 7.9 Hz, 1H), 8.29 (ddd, J = 0.8, 1.3, 7.9 Hz, 1H), 8.74 (ddd, J = 0.8, 1.8, 4.8 Hz, 1H)

Example 231

5-(3,4-Dimethoxybenzylamino)-8-(6,7-dimethoxy-1,2,3,4-tetrahydroisoquinolin-2-ylmethyl)-2-(2-pyridyl)[1,2,4]triazolo [1,5-c]pyrimidine (Compound 232)

The subject compound (39%) was obtained as a white powder from 5-(3,4-dimethoxybenzylamino)-8-formyl-2-(2-pyridyl)[1,2,4]triazolo[1,5-c]pyrimidine obtained in Reference Example 71 and 6,7-dimethoxy-1,2,3,4-tetrahydroisoquinoline in a manner similar to that in Example 1.
$^1$H NMR ($CDCl_3$, δ, ppm) : 2.72-2.90 (m, 4H), 3.73 (s, 2H), 3.81 (s, 3H), 3.84 (s, 3H), 3.91 (s, 6H), 4.04 (s, 2H), 4.76 (d, J = 5.4 Hz, 2H), 6.51 (s, 1H), 6.60 (s, 1H), 6.67 (t, J = 5.4 Hz, 1H), 6.83-6.93 (m, 3H), 7.40 (ddd, J = 1.3, 4.8, 7.6 Hz, 1H), 7.86 (ddd, J = 1.8, 7.6, 7.9 Hz, 1H), 8.04 (s, 1H), 8.41 (ddd, J = 0.8, 1.3, 7.9 Hz, 1H), 8.80 (ddd, J = 0.8, 1.8, 4.8 Hz, 1H)

Example 232

5-Amino-8-(6,7-dimethoxy-1,2,3,4-tetrahydroisoquinolin-2-ylmethyl)-2-(2-pyridyl)[1,2,4]triazolo[1,5-c]pyrimidine (Compound 233)

In a manner similar to that in Example 2, the subject compound (58%) was obtained as a white powder from 5-(3,4-dimethoxybenzylamino)-8-(6,7-dimethoxy-1,2,3,4-tetrahydroisoquinolin-2-ylmethyl)-2-(2-pyridyl)[1,2,4]triazolo [1,5-c]pyrimidine obtained in Example 231.
Melting point: 300°C
$^1$H NMR (DMSO-$d_6$, δ , ppm) : 2.72-2.78 (m, 4H) , 3.55 (s, 2H) , 3.66 (s, 3H), 3.69 (s, 3H), 3.86 (s, 2H), 6.60 (s, 1H), 6.65 (s, 1H), 7.54 (ddd, J = 1.3, 4.8, 7.6 Hz, 1H), 7.86 (s, 1H), 7.95 (brs, 2H), 8.00 (ddd, J = 1.8, 7.6, 7.9 Hz, 1H), 8.29 (ddd, J = 0.8, 1.3, 7.9 Hz, 1H), 8.74 (ddd, J = 0.8, 1.8, 4.8 Hz, 1H)

Example 233

5-(3,4-Dimethoxybenzylamino)-8-(4-phenylpiperazin-1-ylmethyl)-2-(4-pyridyl) [1,2,4]triazolo[1,5-c]pyrimidine (Compound 234)

The subject compound (82%) was obtained as a white powder from 5-(3,4-dimethoxybenzylamino)-8-formyl-2-(4-pyridyl)[1,2,4]triazolo[1,5-c]pyrimidine obtained in Reference Example 75 and 1-phenylpiperazine in a manner similar to that in Example 1.
$^1$H NMR ($CDCl_3$, δ, ppm) : 2.78 (t, J = 5.0 Hz, 4H) , 3.23 (t, J = 5.0 Hz, 4H), 3.88 (s, 6H), 3.95 (s, 2H), 4.77 (d, J = 5.8 Hz, 2H), 6.70-7.02 (m, 7H), 7.20 (t, J = 7.4 Hz, 2H), 8.00 (s, 1H), 8.13 (d, J = 6.2 Hz, 2H), 8.75 (d, J = 6.2 Hz, 2H)

Example 234

5-Amino-8-(4-phenylpiperazin-1-ylmethyl)-2-(4-pyridyl)[1,2,4]triazolo[1,5-c]pyrimidine (Compound 235)

In a manner similar to that in Example 2, the subject compound (36%) was obtained as a white powder from 5-(3,4-dimethoxybenzylamino)-8-(4-phenylpiperazin-1-ylmethyl)-2-(4-pyridyl)[1,2,4]triazolo[1,5-c]pyrimidine obtained in Example 233.
Melting point: 137-138°C
$^1$H NMR (DMSO-$d_6$, δ, ppm) : 2.63 (t, J = 5.0 Hz, 4H), 3.13 (t, J = 5.0 Hz, 4H), 3.78 (s, 2H), 6.75 (t, J = 7.3 Hz, 1H), 6.90 (d, J = 7.3 Hz, 2H), 7.18 (t, J = 7.3 Hz, 2H), 7.87 (s, 1H), 7.97 (brs, 2H), 8.12 (d, J = 6.2 Hz, 2H), 8.79 (d, J = 6.2 Hz, 2H)

Example 235

5-(3,4-Dimethoxybenzylamino)-2-(4-pyridyl)-8-(1,2,3,4-tetrahydroisoquinolin-2-ylmethyl)[1,2,4]triazolo[1,5-c] pyrimidine (Compound 236)

The subject compound (93%) was obtained as a white powder from 5-(3,4-dimethoxybenzylamino)-8-formyl-2-(4-pyridyl)[1,2,4]triazolo[1,5-c]pyrimidine obtained in Reference Example 75 and 1,2,3,4-tetrahydroisoquinoline in a manner similar to that in Example 1.
$^{1}$H NMR ($CDCl_3$, δ, ppm) : 2.92-2.94 (m, 4H), 3.81 (s, 2H), 3.89 (s, 6H), 4.02 (s, 2H), 4.79 (d, J = 5.4 Hz, 2H), 6.44 (t, J = 5.4 Hz, 1H), 6.85-7.11 (m, 7H), 8.03 (s, 1H), 8.14 (d, J = 6.2 Hz, 2H), 8.75 (d, J = 6.2 Hz, 2H)

Example 236

5-Amino-2-(4-pyridyl)-8-(1,2,3,4-tetrahydroisoquinolin-2-ylmethyl)[1,2,4]triazolo[1,5-c]pyrimidine (Compound 237)

In a manner similar to that in Example 2, the subject compound (46%) was obtained as a white powder from 5-(3,4-dimethoxybenzylamino)-2-(4-pyridyl)-8-(1,2,3,4-tetrahydroisoquinolin-2-ylmethyl) [1,2,4]triazolo[1,5-c]pyrimidine obtained in Example 235.
Melting point: 190°C
$^{1}$H NMR ($CDCl_3$, δ, ppm) : 2.88-2.94 (m, 4H), 3.81 (s, 2H) , 4.02 (s, 2H), 5.95 (brs, 2H), 6.97-7.12 (m, 4H), 7.98 (s, 1H), 8.16 (d, J = 6.2 Hz, 2H), 8.77 (d, J = 6.2 Hz, 2H)

Example 237

5-(3,4-Dimethoxybenzylamino)-8-(6,7-dimethoxy-1,2,3,4-tetrahydroisoquinolin-2-ylmethyl)-2-(4-pyridyl)[1,2,4]triazolo [1,5-c]pyrimidine (Compound 238)

The subject compound (46%) was obtained as a white powder from 5-(3,4-dimethoxybenzylamino)-8-formyl-2-(4-pyridyl)[1,2,4]triazolo[1,5-c]pyrimidine obtained in Reference Example 75 and 6,7-dimethoxy-1,2,3,4-tetrahydroisoquinoline in a manner similar to that in Example 1.
$^{1}$H NMR ($CDCl_3$, δ, ppm) : 2.86-2.90 (m, 4H), 3.71 (s, 2H) , 3.78 (s, 3H), 3.82 (s, 3H), 3.88 (s, 6H), 4.00 (s, 2H), 4.78 (d, J = 5.4 Hz, 2H), 6.40 (t, J = 5.4 Hz, 1H), 6.49 (s, 1H), 6.58 (s, 1H), 6.84-7.00 (m, 3H), 8.01 (s, 1H), 8.11 (d, J = 6.2 Hz, 2H), 8.73 (d, J = 6.2 Hz, 2H)

Example 238

5-Amino-8-(6,7-dimethoxy-1,2,3,4-tetrahydroisoquinolin-2-ylmethyl)-2-(4-pyridyl)[1,2,4]triazolo[1,5-c]pyrimidine (Compound 239)

In a manner similar to that in Example 2, the subject compound (31%) was obtained as a white powder from 5-(3,4-dimethoxybenzylamino)-8-(6,7-dimethoxy-1,2,3,4-tetrahydroisoquinolin-2-ylmethyl)-2-(4-pyridyl)[1,2,4]triazolo [1,5-c]pyrimidine obtained in Example 237.
Melting point: 198-199°C
$^{1}$H NMR (DMSO-$d_6$, δ, ppm): 2.88-2.94 (m, 2H), 3.20-3.30 (m, 2H), 3.69 (s, 2H), 3.66 (s, 3H), 3.73 (s, 3H), 3.96-4.04 (m, 2H), 4.24-4.30 (m, 2H), 6.67 (s, 1H), 6.73 (s, 1H), 7.91 (brs, 2H), 8.01 (s, 1H), 8.11 (d, J = 6.2 Hz, 2H), 8.78 (d, J = 6.2 Hz, 2H)

Example 239

5-(3,4-Dimethoxybenzylamino)-2-(2-fluorophenyl)-8-(4-phenylpiperazin-1-ylmethyl)[1,2,4]triazolo[1,5-c]pyrimidine (Compound 240)

The subject compound (98%) was obtained as a white amorphous matter from 5-(3,4-dimethoxybenzylamino)-2-(2-fluorophenyl)-8-formyl[1,2,4]triazolo[1,5-c]pyrimidine obtained in Reference Example 80 and 1-phenylpiperazine in a manner similar to that in Example 1.
$^{1}$H NMR ($CDCl_3$, δ , ppm): 2.80 (t, J = 5.0 Hz, 4H), 3.23 (t, J = 5.0 Hz, 4H), 3.89 (s, 6H), 3.95 (s, 2H), 4.79 (d, J = 5.4 Hz, 2H), 6.49 (t, J = 5.4 Hz, 1H), 6.81-7.00 (m, 6H), 7.17-7.31 (m, 4H), 7.42-7.46 (m, 1H), 7.98 (s, 1H), 8.22 (dt, J = 1.4, 7.6 Hz, 1H)

Example 240

5-Amino-2-(2-fluorophenyl)-8-(4-phenylpiperazin-1-ylmethyl)[1,2,4]triazolo[1,5-c]pyrimidine (Compound 241)

In a manner similar to that in Example 2, the subject compound (68%) was obtained as a white powder from 5-(3,4-dimethoxybenzylamino)-2-(2-fluorophenyl)-8-(4-phenylpiperazin-1-ylmethyl)[1,2,4]triazolo[1,5-c]pyrimidine obtained in Example 239.
Melting point: 191-192°C
$^1$H NMR (DMSO-$d_6$, δ , ppm): 2.62 (t, J = 5.0 Hz, 4H), 3.12 (t, J = 5.0 Hz, 4H), 3.76 (s, 2H), 6.75 (t, J = 7.3 Hz, 1H), 6.90 (d, J = 7.3 Hz, 2H), 7.18 (t, J = 7.3 Hz, 2H), 7.36-7.44 (m, 2H), 7.56-7.59 (m, 1H), 7.80 (brs, 2H), 7.84 (s, 1H), 8.14 (dt, J = 1.4, 7.6 Hz, 1H)

Example 241

5-(3,4-Dimethoxybenzylamino)-2-(2-fluorophenyl)-8-(1,2,3,4-tetrahydroisoquinolin-2-ylmethyl)[1,2,4]triazolo[1,5-c]pyrimidine (Compound 242)

The subject compound (56%) was obtained as a white powder from 5-(3,4-dimethoxybenzylamino)-2-(2-fluorophenyl)-8-formyl[1,2,4]triazolo[1,5-c]pyrimidine obtained in Reference Example 80 and 1,2,3,4-tetrahydroisoquinoline in a manner similar to that in Example 1.
$^1$H NMR ($CDCl_3$, δ , ppm) : 2.92-2.96 (m, 4H), 3.83 (s, 2H), 3.88 (s, 6H), 4.05 (s, 2H), 4.79 (d, J = 5.4 Hz, 2H), 6.48 (t, J = 5.4 Hz, 1H), 6.84-7.30 (m, 9H), 7.42-7.46 (m, 1H), 8.03 (s, 1H), 8.22 (dt, J = 1.4, 7.6 Hz, 1H)

Example 242

5-Amino-2-(2-fluorophenyl)-8-(1,2,3,4-tetrahydroisoquinolin-2-ylmethyl)[1,2,4]triazolo[1,5-c]pyrimidine (Compound 243)

In a manner similar to that in Example 2, the subject compound (78%) was obtained as a white powder from 5-(3,4-dimethoxybenzylamino)-2-(2-fluorophenyl)-8-(1,2,3,4-tetrahydroisoquinolin-2-ylmethyl)[1,2,4]triazolo[1,5-c]pyrimidine obtained in Example 241.
Melting point: 165°C
$^1$H NMR (DMSO-$d_6$, δ, ppm) : 2.74-2.79 (m, 4H) , 3.66 (s, 2H) , 3.87 (s, 2H), 7.00-7.09 (m, 4H), 7.34-7.44 (m, 2H), 7.54 -7.62 (m, 1H), 7.83 (brs, 2H), 7.87 (s, 1H), 8.15 (dt, J = 1.4, 7.6 Hz, 1H)

Example 243

5-(3,4-Dimethoxybenzylamino)-8-(6,7-dimethoxy-1,2,3,4-tetrahydroisoquinolin-2-ylmethyl)-2-(2-fluorophenyl)[1,2,4]triazolo[1.5-c]pyrimidine (Compound 244)

The subject compound (18%) was obtained as a white powder from 5-(3,4-dimethoxybenzylamino)-2-(2-fluorophenyl)-8-formyl[1,2,4]triazolo[1,5-c]pyrimidine obtained in Reference Example 80 and 6,7-dimethoxy-1,2,3,4-tetrahydroisoquinoline in a manner similar to that in Example 1.
$^1$H NMR ($CDCl_3$, δ, ppm): 3.06-3.11 (m, 2H), 3.38-3.42 (m, 2H), 3.79 (s, 3H), 3.86 (s, 3H), 3.88 (s, 3H), 3.90 (s, 3H), 4.22 (s, 2H), 4.62 (s, 2H), 4.82 (d, J = 5.4 Hz, 2H), 6.49 (s, 1H), 6.64 (s, 1H), 6.72 (t, J = 5.4 Hz, 1H), 6.87 (d, J = 7.8 Hz, 1H) , 6.98-7.02 (m, 2H), 7.19-7.31 (m, 2H), 7.45-7.53 (m, 1H), 8.13 (dt, J = 1.4, 7.6 Hz, 1H), 8.63 (s, 1H)

Example 244

5-Amino-8-(6,7-dimethoxy-1,2,3,4-tetrahydroisoquinolin-2-ylmethyl)-2-(2-fluorophenyl) [1,2,4]triazolo[1,5-c]pyrimidine (Compound 245)

In a manner similar to that in Example 2, the subject compound (65%) was obtained as a white powder from 5-(3,4-dimethoxybenzylamino)-8-(6,7-dimethoxy-1,2,3,4-tetrahydroisoquinolin-2-ylmethyl)-2-(2-fluorophenyl) [1,2,4]triazolo[1,5-c]pyrimidine obtained in Example 243.
Melting point: 182-183°C
$^1$H NMR (DMSO-$d_6$, δ , ppm) : 2.70-2.78 (m, 4H) , 3.54 (s, 2H) , 3.66 (s, 3H), 3.69 (s, 3H), 3.84 (s, 2H), 6.60 (s, 1H), 6.64 (s, 1H), 7.36-7.43 (m, 2H), 7.54-7.63 (m, 1H), 7.84 (brs, 2H), 7.86 (s, 1H), 8.14 (dt, J = 1.4, 7.6 Hz, 1H)

Example 245

5-(3,4-Dimethoxybenzylamino)-2-(2-methoxyphenyl)-8-(4-phenylpiperazin-1-ylmethyl)[1,2,4]triazolo[1,5-c]pyrimidine (Compound 246)

The subject compound (80%) was obtained as a white amorphous matter from 5-(3,4-dimethoxybenzylamino)-8-formyl-2- (2-methoxyphenyl) [1,2,4]triazolo[1,5-c]pyrimidine obtained in Reference Example 85 and 1-phenylpiperazine in a manner similar to that in Example 1.
$^1$H NMR ($CDCl_3$, δ, ppm) : 2.79 (t, J = 5.0 Hz, 4H) , 3.24 (t, J = 5.0 Hz, 4H), 3.88 (s, 6H), 3.92 (s, 3H), 4.77 (d, J = 5.4 Hz, 2H), 6.65 (t, J = 5.4 Hz, 1H), 6.55-7.30 (m, 10H), 7.45 (ddd, J = 1.7, 7.6, 8.6 Hz, 1H), 7.93-7.95 (m, 2H)

Example 246

5-Amino-2-(2-methoxyphenyl)-8-(4-phenylpiperazin-1-ylmethyl)[1,2,4]triazolo[1,5-c]pyrimidine (Compound 247)

In a manner similar to that in Example 2, the subject compound (39%) was obtained as a white powder from 5-(3,4-dimethoxybenzylamino)-2-(2-methoxyphenyl)-8-(4-phenylpiperazin-1-ylmethyl)[1,2,4]triazolo[1,5-c]pyrimidine obtained in Example 245.
Melting point: 197-198°C
$^1$H NMR (DMSO-$d_6$, δ, ppm) : 2.62 (t, J = 5.0 Hz, 4H), 3.12 (t, J = 5.0 Hz, 4H), 3.74 (s, 2H), 3.83 (s, 3H), 6.75 (t, J = 7.3 Hz, 1H), 6.76 (d, J = 7.3 Hz, 2H), 7.09 (t, J = 7.3 Hz, 2H), 7.10-7.21 (m, 2H), 7.51 (ddd, J = 1.7, 7.6, 8.6 Hz, 1H), 7.75-7.79 (m, 1H), 7.77 (brs, 2H), 7.81 (s, 1H)

Example 247

5-(3,4-Dimethoxybenzylamino)-2-(2-methoxyphenyl)-8-(1,2,3,4-tetrahydroisoquinolin-2-ylmethyl)[1,2,4]triazolo[1,5-c]pyrimidine (Compound 248)

The subject compound (95%) was obtained as a brown amorphous matter from 5-(3,4-dimethoxybenzylamino)-8-formyl-2-(2-methoxyphenyl) [1,2,4]triazolo[1,5-c]pyrimidine obtained in Reference Example 85 and 1,2,3,4-tetrahydroisoquinoline in a manner similar to that in Example 1.
$^1$H NMR ($CDCl_3$, δ, ppm): 2.90-2.94 (m, 4H), 3.75 (s, 2H), 3.89 (s, 6H), 3.93 (s, 3H), 4.02 (s, 2H), 4.78 (d, J = 5.4 Hz, 2H), 6.51 (t, J = 5.4 Hz, 1H), 6.84-7.30 (m, 9H), 7.44 (ddd, J = 1.7, 7.6, 8.6 Hz, 1H), 7.95-7.99 (m, 2H)

Example 248

5-Amino-2-(2-methoxyphenyl)-8-(1,2,3,4-tetrahydroisoquinolin-2-ylmethyl) [1,2,4]triazolo[1,5-c]pyrimidine (Compound 249)

In a manner similar to that in Example 2, the subject compound (31%) was obtained as a white powder from 5-(3,4-dimethoxybenzylamino)-2-(2-methoxyphenyl)-8-(1,2,3,4-tetrahydroisoquinolin-2-ylmethyl)[1,2,4]triazolo[1,5-c]pyrimidine obtained in Example 247.
Melting point: 176-177°C
$^1$H NMR (DMSO-$d_6$, δ, ppm): 2.74-2.93 (m, 4H), 3.64 (s, 2H), 3.82 (s, 3H), 3.84 (s, 2H), 7.02-7.11 (m, 5H), 7.18 (d, J = 8.6 Hz, 1H), 7.50 (ddd, J = 1.7, 7.6, 8.6 Hz, 1H), 7.73 (dd, J = 1.7, 7.6 Hz, 1H), 7.76 (brs, 2H), 8.73 (s, 1H)

Example 249

5-(3,4-Dimethoxybenzylamino)-8-(6,7-dimethoxy-1,2,3,4-tetrahydroisoquinolin-2-ylmethyl)-2-(2-methoxyphenyl)[1,2,4]triazolo[1,5-c]pyrimidine (Compound 250)

The subject compound (84%) was obtained as a white amorphous matter from 5-(3,4-dimethoxybenzylamino)-8-formyl-2-(2-methoxyphenyl)[1,2,4]triazolo[1,5-c]pyrimidine obtained in Reference Example 85 and 6,7-dimethoxy-1,2,3,4-tetrahydroisoquinoline in a manner similar to that in Example 1.
$^1$H NMR ($CDCl_3$, δ, ppm): 2.86-2.90 (m, 4H), 3.72 (s, 2H), 3.81 (s, 3H), 3.84 (s, 3H), 3.89 (s, 6H), 3.91 (s, 3H), 4.02 (s, 2H), 4.77 (d, J = 5.4 Hz, 2H), 6.50 (s, 1H), 6.54 (t, J = 5.4 Hz, 1H), 6.59 (s, 1H), 6.84 (d, J = 7.8 Hz, 1H), 6.93-7.10 (m, 4H), 7.44 (ddd, J = 1.7, 7.6, 8.6 Hz, 1H), 7.95-8.00 (m, 2H)

Example 250

5-Amino-8-(6,7-dimethoxy-1,2,3,4-tetrahydroisoquinolin-2-ylmethyl)-2-(2-methoxyphenyl)[1,2,4]triazolo[1,5-c]pyrimidine (Compound 251)

In a manner similar to that in Example 2, the subject compound (45%) was obtained as a white powder from 5-(3,4-dimethoxybenzylamino)-8-(6,7-dimethoxy-1,2,3,4-tetrahydroisoquinolin-2-ylmethyl)-2-(2-methoxyphenyl)[1,2,4]triazolo[1,5-c]pyrimidine obtained in Example 249.
Melting point: 139-140°C
$^1$H NMR (DMSO-$d_6$, δ, ppm): 2.70-2.76 (m, 4H), 3.53 (s, 2H), 3.66 (s, 3H), 3.68 (s, 3H), 3.82 (s, 2H), 6.59 (s, 1H), 6.64 (s, 1H), 7.08 (t, J = 7.6 Hz, 1H), 7.18 (d, J = 8.6 Hz, 1H), 7.50 (ddd, J = 1.7, 7.6, 8.6 Hz, 1H), 7.55 (dd, J = 1.7, 7.6 Hz, 1H), 7.76 (brs, 2H), 7.82 (s, 1H)

Example 251

2-(2-Furyl)-5-(1-pyrrolidinyl)-8-(1,2,3,4-tetrahydroisoquinolin-2-ylmethyl)[1,2,4]triazolo[1,5-c]pyrimidine (Compound 252)

8-Formyl-2-(2-furyl)-5-methylthio[1,2,4]triazolo[1,5-c]pyrimidine (13.0 mg, 0.05 mmol) obtained in Reference Example 47 was suspended in 1,2-dimethoxyethane (300 µl), and pyrrolidine (8.4 µl, 0.1 mmol) was added thereto, followed by stirring at 80°C for 17 hours. The solvent was distilled away under reduced pressure, and the resulting residue was dissolved in chloroform (600 µl). To the solution were added benzoyl chloride polymer bound (41.0 mg, rolling ratio: up to 2.1 mmol/g resin) and poly(4-vinylpyridine) (22.1 mg), followed by stirring at room temperature for 20 hours. After filtration of the reaction mixture, the solvent was distilled away from the filtrate. The resulting residue was suspended in dichloroethane (600 µl), and 1,2,3,4-tetrahydroisoquinoline (12.5 µl, 0.1 mmol) and sodium triacetoxyborohydride (31.8 mg, 0.15 mmol) were added thereto, followed by stirring at room temperature for 16 hours. To the reaction mixture was added a 3 mol/l aqueous sodium hydroxide solution, and the mixture was subjected to extraction with dichloromethane. After the organic layer was dried over anhydrous magnesium sulfate, the solvent was distilled away under reduced pressure, and the resulting residue was dissolved in chloroform (640 µl) and methanol (160 µl). To the solution were added benzoyl chloride polymer bound (41.0 mg, rolling ratio: up to 2.1 mmol/g resin) and poly(4-vinylpyridine) (22.1 mg), followed by stirring at room temperature for 16 hours. After filtration of the reaction mixture, the solvent was distilled away from the filtrate, and the resulting residue was purified by silica gel column chromatography (eluted with chloroform/methanol = 100/3) to obtain the subject compound (4 mg, 20%) as a pale brown oily matter.
APCIMS m/z: $[M + H]^+$ 401

Example 252

8-(6,7-Dimethoxy-1,2,3,4-tetrahydroisoquinolin-2-ylmethyl)-2-(2-furyl)-5-(1-pyrrolidinyl)[1,2,4]triazolo[1,5-c]pyrimidine (Compound 253)

The subject compound (20%) was obtained as a pale brown oily matter from 8-formyl-2-(2-furyl)-5-methylthio[1,2,4]triazolo[1,5-c]pyrimidine, pyrrolidine and 6,7-dimethoxy-1,2,3,4-tetrahydroisoquinoline in a manner similar to that in Example 251.
APCIMS m/z: $[M + H]^+$ 461

Example 253

8-(4-Benzylpiperidinomethyl)-2-(2-furyl)-5-(1-pyrrolidinyl)[1,2,4]triazolo[1,5-c]pyrimidine (Compound 254)

The subject compound (23%) was obtained as a pale brown oily matter from 8-formyl-2-(2-furyl)-5-methylthio[1,2,4]triazolo[1,5-c]pyrimidine, pyrrolidine and 4-benzylpiperidine in a manner similar to that in Example 251.
APCIMS m/z: $[M + H]^+$ 443

Example 254

8-(4-Benzyl-4-hydroxypiperidinomethyl)-2-(2-furyl)-5-(1-pyrrolidinyl)[1,2,4]triazolo[1,5-c]pyrimidine (Compound 255)

The subject compound (12%) was obtained as a pale brown oily matter from 8-formyl-2-(2-furyl)-5-methylthio [1,2,4]triazolo[1,5-c]pyrimidine, pyrrolidine and 4-benzyl-4-hydroxypiperidine in a manner similar to that in Example 251.
APCIMS m/z: $[M + H]^+$ 459

Example 255

2-(2-Furyl)-8-(4-phenylpiperazin-1-ylmethyl)-5-(1-pyrrolidinyl)[1,2,4]triazolo[1,5-c]pyrimidine (Compound 256)

The subject compound (22%) was obtained as a pale brown oily matter from 8-formyl-2-(2-furyl)-5-methylthio [1,2,4]triazolo[1,5-c]pyrimidine, pyrrolidine and 1-phenylpiperazine in a manner similar to that in Example 251.
APCIMS m/z: $[M + H]^+$ 430

Example 256

2-(2-Furyl)-8-(4-phenyl-1,2,3,6-tetrahydropyridin-1-ylmethyl)-5-(1-pyrrolidinyl)[1,2,4]triazolo[1,5-c]pyrimidine (Compound 257)

The subject compound (24%) was obtained as a pale brown oily matter from 8-formyl-2-(2-furyl)-5-methylthio [1,2,4]triazolo[1,5-c]pyrimidine, pyrrolidine and 4-phenyl-1,2,3,6-tetrahydropyridine in a manner similar to that in Example 251.
APCIMS m/z: $[M + H]^+$ 427

Example 257

2-(2-Furyl)-8-[4-(2-pyrimidinyl)piperazin-1-ylmethyl]-5-(1-pyrrolidinyl)[2,2,4]triazolo[1,5-c]pyrimidine (Compound 258)

The subject compound (51%) was obtained as a pale brown oily matter from 8-formyl-2-(2-furyl)-5-methylthio [1,2,4]triazolo[1,5-c]pyrimidine, pyrrolidine and 1-(2-pyrimidinyl)piperazine in a manner similar to that in Example 251.
APCIMS m/z: $[M + H]^+$ 432

Example 258

8-[4-(2-Fluorophenyl)piperazin-1-ylmethyl]-2-(2-furyl)-5-(1-pyrrolidinyl)[1,2,4]triazolo[1,5-c]pyrimidine (Compound 259)

The subject compound (25%) was obtained as a pale brown oily matter from 8-formyl-2-(2-furyl)-5-methylthio [1,2,4]triazolo[1,5-clpyrimidine, pyrrolidine and 1-(2-fluorophenyl)piperazine in a manner similar to that in Example 251.
APCIMS m/z: $[M + H]^+$ 448

Example 259

2-(2-Furyl)-8-(1,2,3,4-tetrahydroisoquinolin-2-ylmethyl)-5-(1,2,3,6-tetrahydropyridin-1-yl)[1,2,4]triazolo[1,5-c] pyrimidine (Compound 260)

The subject compound (40%) was obtained as a pale brown oily matter from 8-formyl-2-(2-furyl)-5-methylthio [1,2,4]triazolo[1,5-c]pyrimidine, 1,2,3,6-tetrahydropyridine and 1,2,3,4-tetrahydroisoquinoline in a manner similar to that in Example 251.
APCIMS m/z: $[M + H]^+$ 413

Example 260

8-(6,7-Dimethoxy-1,2,3,4-tetrahydroisoquinolin-2-ylmethyl)-2-(2-furyl)-5-(1,2,3,6-tetrahydropyridin-1-yl) [1,2,4] triazolo[1,5-c]pyrimidine (Compound 261)

The subject compound (44%) was obtained as a pale brown oily matter from 8-formyl-2-(2-furyl)-5-methylthio [1,2,4]triazolo[1,5-c]pyrimidine, 1,2,3,6-tetrahydropyridine and 6,7-dimethoxy-1,2,3,4-tetrahydroisoquinoline in a manner similar to that in Example 251.
APCIMS m/z: $[M + H]^+$ 473

Example 261

8-(4-Benzylpiperidinomethyl)-2-(2-furyl)-5-(1,2,3,6-tetrahydropyridin-1-yl)[1,2,4]triazolo[1,5-c]pyrimidine (Compound 262)

The subject compound (53%) was obtained as a pale brown oily matter from 8-formyl-2-(2-furyl)-5-methylthio [1,2,4]triazolo[1,5-c]pyrimidine, 1,2,3,6-tetrahydropyridine and 4-benzylpiperidine in a manner similar to that in Example 251.
APCIMS m/z: $[M + H]^+$ 455

Example 262

8-(4-Benzyl-4-hydroxypiperidinomethyl)-2-(2-furyl)-5-(1,2,3,6-tetrahydropyridin-1-yl)[1,2,4]triazolo[1,5-c]pyrimidine (Compound 263)

The subject compound (54%) was obtained as a pale brown oily matter from 8-formyl-2-(2-furyl)-5-methylthio [1,2,4]triazolo[1,5-c]pyrimidine, 1,2,3,6-tetrahydropyridine and 4-benzyl-4-hydroxypiperidine in a manner similar to that in Example 251.
APCIMS m/z: $[M + H]^+$ 471

Example 263

2-(2-Furyl)-8-(4-phenylpiperazin-1-ylmethyl)-5-(1,2,3,6-tetrahydropyridin-1-yl)[1,2,4]triazolo[1,5-c]pyrimidine (Compound 264)

The subject compound (46%) was obtained as a pale brown oily matter from 8-formyl-2-(2-furyl)-5-methylthio [1,2,4]triazolo[1,5-c]pyrimidine, 1,2,3,6-tetrahydropyridine and 1-phenylpiperazine in a manner similar to that in Example 251.
APCIMS m/z: $[M + H]^+$ 442

Example 264

2-(2-Furyl)-8-(4-phenyl-1,2,3,6-tetrahydropyridin-1-ylmethyl)-5-(1,2,3,6-tetrahydropyridin-1-yl)[1,2,4]triazolo[1,5-c] pyrimidine (Compound 265)

The subject compound (44%) was obtained as a pale brown oily matter from 8-formyl-2-(2-furyl)-5-methylthio [1,2,4]triazolo[1,5-c]pyrimidine, 4-phenyl-1,2,3,6-tetrahydropyridine and 1,2,3,6-tetrahydropyridine in a manner similar to that in Example 251.
APCIMS m/z: $[M + H]^+$ 439

Example 265

2-(2-Furyl)-8-[4-(2-pyrimidinyl)piperazin-1-ylmethyl]-5-(1,2,3,6-tetrahydropyridin-1-yl)[1,2,4]triazolo[1,5-c]pyrimidine (Compound 266)

The subject compound (43%) was obtained as a pale brown oily matter from 8-formyl-2-(2-furyl)-5-methylthio [1,2,4]triazolo[1,5-c]pyrimidine, 1,2,3,6-tetrahydropyridine and 1-(2-pyrimidinyl)piperazine in a manner similar to that in Example 251.

APCIMS m/z: $[M + H]^+$ 444

Example 266

8-[4-(2-Fluorophenyl)piperazin-1-ylmethyl]-2-(2-furyl)-5-(1,2,3,6-tetrahydropyridin-1-yl)[1,2,4]triazolo[1,5-c]pyrimidine (Compound 267)

The subject compound (34%) was obtained as a pale brown oily matter from 8-formyl-2-(2-furyl)-5-methylthio [1,2,4]triazolo[1,5-c]pyrimidine, 1,2,3,6-tetrahydropyridine and 1-(2-fluorophenyl)piperazine in a manner similar to that in Example 251.
APCIMS m/z: $[M + H]^+$ 460

Example 267

2-(2-Furyl)-5-(4-methylpiperazin-1-yl)-8-(1,2,3,4-tetrahydroisoquinolin-2-ylmethyl)[1,2,4]triazolo[1,5-c]pyrimidine (Compound 268)

The subject compound (29%) was obtained as a pale brown oily matter from 8-formyl-2-(2-furyl)-5-methylthio [1,2,4]triazolo[1,5-e]pyrimidine, 1-methylpiperazine and 1,2,3,4-tetrahydroisoquinoline in a manner similar to that in Example 251.
APCIMS m/z: $[M + H]^+$ 430

Example 268

8-(6,7-Dimethoxy-1,2,3,4-tetrahydroisoquinolin-2-ylmethyl)-2-(2-furyl)-5-(4-methylpiperazin-1-yl)[1,2,4]triazolo[1,5-c] pyrimidine (Compound 269)

The subject compound (31%) was obtained as a pale brown oily matter from 8-formyl-2-(2-furyl)-5-methylthio [1,2,4]triazolo[1,5-c]pyrimidine, 1-methylpiperazine and 6,7-dimethoxy-1,2,3,4-tetrahydroisoquinoline in a manner similar to that in Example 251.
APCIMS m/z: $[M + H]^+$ 490

Example 269

8-(4-Benzylpiperidinomethyl)-2-(2-furyl)-5-(4-methylpiperazin-1-yl) [1,2,4]triazolo[1,5-c]pyrimidine (Compound 270)

The subject compound (31%) was obtained as a pale brown oily matter from 8-formyl-2-(2-furyl)-5-methylthio [1,2,4]triazolo[1,5-c]pyrimidine, 1-methylpiperazine and 4-benzylpiperidine in a manner similar to that in Example 251.
APCIMS m/z: $[M + H]^+$ 472

Example 270

8- (4-Benzyl-4-hydroxypiperidinomethyl) -2- (2-furyl) -5- (4-methylpiperazin-1-yl)[1,2,4]triazolo[1,5-c]pyrimidine (Compound 271)

The subject compound (27%) was obtained as a pale brown oily matter from 8-formyl-2-(2-furyl)-5-methylthio [1,2,4]triazolo[1,5-c]pyrimidine, 1-methylpiperazine and 4-benzyl-4-hydroxypiperidine in a manner similar to that in Example 251.
APCIMS m/z: $[M + H]^+$ 488

Example 271

2-(2-Furyl)-5-(4-methylpiperazin-1-yl)-8-(4-phenylpiperazin-1-ylmethyl)[1,2,4]triazolo[1,5-c]pyrimidine (Compound 272)

The subject compound (26%) was obtained as a pale brown oily matter from 8-formyl-2-(2-furyl)-5-methylthio [1,2,4]triazolo[1,5-c]pyrimidine, 1-methylpiperazine and 1-phenylpiperazine in a manner similar to that in Example 251.
APCIMS m/z: $[M + H]^+$ 459

Example 272

2-(2-Furyl)-5-(4-methylpiperazin-1-yl)-8-(4-phenyl-1,2,3,6-tetrahydropyridin-1-ylmethyl)[1,2,4]triazolo[1,5-c]pyrimidine (Compound 273)

The subject compound (27%) was obtained as a pale brown oily matter from 8-formyl-2-(2-furyl)-5-methylthio[1,2,4]triazolo[1,5-c]pyrimidine, 1-methylpiperazine and 4-phenyl-1,2,3,6-tetrahydropyridine in a manner similar to that in Example 251.
APCIMS m/z: $[M + H]^+$ 456

Example 273

2-(2-Furyl)-5-(4-methylpiperazin-1-yl)-8-[4-(2-pyrimidinyl)piperazin-1-ylmethyl][1,2,4]triazolo[1,5-c]pyrimidine (Compound 274)

The subject compound (32%) was obtained as a pale brown oily matter from 8-formyl-2-(2-furyl)-5-methylthio[1,2,4]triazolo[1,5-c]pyrimidine, 1-methylpiperazine and 1-(2-pyrimidinyl)piperazine in a manner similar to that in Example 251.
APCIMS m/z: $[M + H]^+$ 461

Example 274

8-[4-(2-Fluorophenyl)piperazin-1-ylmethyl]-2-(2-furyl)-5-(4-methylpiperazin-1-yl)[1,2,4]triazolo[1,5-c]pyrimidine (Compound 275)

The subject compound (32%) was obtained as a pale brown oily matter from 8-formyl-2-(2-furyl)-5-methylthio[1,2,4]triazolo[1,5-c]pyrimidine, 1-methylpiperazine and 1-(2-fluorophenyl)piperazine in a manner similar to that in Example 251.
APCIMS m/z: $[M + H]^+$ 477

Example 275

2-(2-Furyl)-5-(4-methylpiperidino)-8-(1,2,3,4-tetrahydroisoquinolin-2-ylmethyl)[1,2,4]triazolo[1,5-c]pyrimidine (Compound 276)

The subject compound (33%) was obtained as a pale brown oily matter from 8-formyl-2-(2-furyl)-5-methylthio[1,2,4]triazolo[1,5-c]pyrimidine, 4-methylpiperidine and 1,2,3,4-tetrahydroisoquinoline in a manner similar to that in Example 251.
APCIMS m/z: $[M + H]^+$ 429

Example 276

8-(6,7-Dimethoxy-1,2,3,4-tetrahydroisoquinolin-2-ylmethyl)-2-(2-furyl)-5-(4-methylpiperidino)[1,2,4]triazolo[1,5-c]pyrimidine(Compound 277)

The subject compound (28%) was obtained as a pale brown oily matter from 8-formyl-2-(2-furyl)-5-methylthio[1,2,4]triazolo[1,5-c]pyrimidine, 4-methylpiperidine and 6,7-dimethoxy-1,2,3,4-tetrahydroisoquinoline in a manner similar to that in Example 251.
APCIMS m/z: $[M + H]^+$ 489

Example 277

8-(4-Benzylpiperidinomethyl)-2-(2-furyl)-5-(4-methylpiperidino)[1,2,4]triazolo[1,5-c]pyrimidine (Compound 278)

The subject compound (34%) was obtained as a pale brown oily matter from 8-formyl-2-(2-furyl)-5-methylthio[1,2,4]triazolo[1,5-c]pyrimidine, 4-methylpiperidine and 4-benzylpiperidine in a manner similar to that in Example 251.
APCIMS m/z: $[M + H]^+$ 471

Example 278

8-(4-Benzyl-4-hydroxypiperidinomethyl)-2-(2-furyl)-5-(4-methylpiperidino)[1,2,4]triazolo[1,5-c]pyrimidine (Compound 279)

The subject compound (41%) was obtained as a pale brown oily matter from 8-formyl-2-(2-furyl)-5-methylthio [1,2,4]triazolo[1,5-c]pyrimidine, 4-methylpiperidine and 4-benzyl-4-hydroxypiperidine in a manner similar to that in Example 251.
APCIMS m/z: $[M + H]^+$ 487

Example 279

2-(2-Furyl)-5-(4-methylpiperidino)-8-(4-phenylpiperazin-1-ylmethyl)[1,2,4]triazolo[1,5-c]pyrimidine (Compound 280)

The subject compound (29%) was obtained as a pale brown oily matter from 8-formyl-2-(2-furyl)-5-methylthio [1,2,4]triazolo[1,5-c]pyrimidine, 4-methylpiperidine and 1-phenylpiperazine in a manner similar to that in Example 251.
APCIMS m/z: $[M + H]^+$ 458

Example 280

2-(2-Furyl)-5-(4-methylpiperidino)-8-(4-phenyl-1,2,3,6-tetrahydropyridin-1-ylmethyl)[1,2,4]triazolo[1,5-c]pyrimidine (Compound 281)

The subject compound (30%) was obtained as a pale brown oily matter from 8-formyl-2-(2-furyl)-5-methylthio [1,2,4]triazolo[1,5-c]pyrimidine, 4-methylpiperidine and 4-phenyl-1,2,3,6-tetrahydropyridine in a manner similar to that in Example 251.
APCIMS m/z: $[M + H]^+$ 455

Example 281

2-(2-Furyl)-5-(4-methylpiperidino)-8-[4-(2-pyrimidinyl)piperazin-1-ylmethyl][1,2,4]triazolo[1,5-c]pyrimidine (Compound 282)

The subject compound (37%) was obtained as a pale brown oily matter from 8-formyl-2-(2-furyl)-5-methylthio [1,2,4]triazolo[1,5-c]pyrimidine, 4-methylpiperidine and 1-(2-pyrimidinyl)piperazine in a manner similar to that in Example 251.
APCIMS m/z: $[M + H]^+$ 460

Example 282

8-[4-(2-Fluorophenyl)piperazin-1-ylmethyl]-2-(2-furyl)-5-(4-methylpiperidino)[1,2,4]triazolo[1,5-c]pyrimidine (Compound 283)

The subject compound (34%) was obtained as a pale brown oily matter from 8-formyl-2-(2-furyl)-5-methylthio [1,2,4]triazolo[1,5-c]pyrimidine, 4-methylpiperidine and 1-(2-fluorophenyl)piperazine in a manner similar to that in Example 251.
APCIMS m/z: $[M + H]^+$ 476

Example 283

2-(2-Furyl)-5-morpholino-8-(1,2,3,4-tetrahydroisoquinolin-2-ylmethyl)[1,2,4]triazolo[1,5-c]pyrimidine (Compound 284)

The subject compound (33%) was obtained as a pale brown oily matter from 8-formyl-2-(2-furyl)-5-methylthio [1,2,4]triazolo[1,5-c]pyrimidine, morpholine and 1,2,3,4-tetrahydroisoquinoline in a manner similar to that in Example 251.
APCIMS m/z: $[M + H]^+$ 417

Example 284

8-(6,7-Dimethoxy-1,2,3,4-tetrahydroisoquinolin-2-ylmethyl)-2-(2-furyl)-5-morpholino[1,2,4]triazolo[1,5-c]pyrimidine (Compound 285)

The subject compound (34%) was obtained as a pale brown oily matter from 8-formyl-2-(2-furyl)-5-methylthio [1,2,4]triazolo[1,5-c]pyrimidine, morpholine and 6,7-dimethoxy-1,2,3,4-tetrahydroisoquinoline in a manner similar to that in Example 251.
APCIMS m/z: $[M + H]^+$ 477

Example 285

8-(4-Benzylpiperidinomethyl)-2-(2-furyl)-5-morpholino[1,2,4]triazolo[1,5-c]pyrimidine (Compound 286)

The subject compound (37%) was obtained as a pale brown oily matter from 8-formyl-2-(2-furyl)-5-methylthio [1,2,4]triazolo[1,5-c]pyrimidine, morpholine and 4-benzylpiperidine in a manner similar to that in Example 251.
APCIMS m/z: $[M + H]^+$ 459

Example 286

8-(4-Benzyl-4-hydroxypiperidinomethyl)-2-(2-furyl)-5-morpholino[1,2,4]triazolo[1,5-c]pyrimidine (Compound 287)

The subject compound (29%) was obtained as a pale brown oily matter from 8-formyl-2-(2-furyl)-5-methylthio [1,2,4]triazolo[1,5-c]pyrimidine, morpholine and 4-benzyl-4-hydroxypiperidine in a manner similar to that in Example 251.
APCIMS m/z: $[M + H]^+$ 475

Example 287

2-(2-Furyl)-5-morpholino-8-(4-phenylpiperazin-1-ylmethyl) [1,2,4]triazolo[1,5-c]pyrimidine (Compound 288)

The subject compound (37%) was obtained as a pale brown oily matter from 8-formyl-2-(2-furyl)-5-methylthio [1,2,4]triazolo[1,5-c]pyrimidine, morpholine and 1-phenylpiperazine in a manner similar to that in Example 251.
APCIMS m/z: $[M + H]^+$ 446

Example 288

2-(2-Furyl)-5-morpholino-8-(4-phenyl-1,2,3,6-tetrahydropyridin-1-ylmethyl) [1,2,4]triazolo[1,5-c]pyrimidine (Compound 289)

The subject compound (32%) was obtained as a pale brown oily matter from 8-formyl-2-(2-furyl)-5-methylthio [1,2,4]triazolo[1,5-c]pyrimidine, morpholine and 4-phenyl-1,2,3,6-tetrahydropyridine in a manner similar to that in Example 251.
APCIMS m/z: $[M + H]^+$ 443

Example 289

2-(2-Furyl)-5-morpholino-8-[4- (2-pyrimidinyl)piperazin-1-ylmethyl] [1,2,4]triazolo[1,5-c]pyrimidine (Compound 290)

The subject compound (42%) was obtained as a pale brown oily matter from 8-formyl-2-(2-furyl)-5-methylthio [1,2,4]triazolo[1,5-c]pyrimidine, morpholine and 1-(2-pyrimidinyl)piperazine in a manner similar to that in Example 251.
APCIMS m/z: $[M + H]^+$ 448

Example 290

8-[4-(2-Fluorophenyl)piperazin-1-ylmethyl]-2- (2-furyl) -5-morpholino[1,2,4]triazolo[1,5-c]pyrimidine (Compound 291)

The subject compound (32%) was obtained as a pale brown oily matter from 8-formyl-2-(2-furyl)-5-methylthio[1,2,4]triazolo[1,5-c]pyrimidine, morpholine and 1-(2-fluorophenyl)piperazine in a manner similar to that in Example 251.
APCIMS m/z: $[M + H]^+$ 464

Example 291

5-(2,6-Dimethylmorpholino)-2-(2-furyl)-8-(1,2,3,4-tetrahydroisoquinolin-2-ylmethyl)[1,2,4]triazolo[1,5-c]pyrimidine (Compound 292)

The subject compound (37%) was obtained as a pale brown oily matter from 8-formyl-2-(2-furyl)-5-methylthio [1,2,4]triazolo[1,5-c]pyrimidine, 2,6-dimethylmorpholine and 1,2,3,4-tetrahydroisoquinoline in a manner similar to that in Example 251.
APCIMS m/z: $[M + H]^+$ 445

Example 292

2-(2-Furyl)-5-(2,6-dimethylmorpholino)-8-(6,7-dimethoxy-1,2,3,4-tetrahydroisoquinolin-2-ylmethyl)[1,2,4]triazolo [1,5-c]pyrimidine (Compound 293)

The subject compound (37%) was obtained as a pale brown oily matter from 8-formyl-2-(2-furyl)-5-methylthio [1,2,4]triazolo[1,5-c]pyrimidine, 2,6-dimethylmorpholine and 6,7-dimethoxy-1,2,3,4-tetrahydroisoquinoline in a manner similar to that in Example 251.
APCIMS m/z: $[M + H]^+$ 505

Examale 293

8-(4-Benzylpiperidinomethyl)-5-(2,6-dimethylmorpholino)-2-(2-furyl) [1,2,4]triazolo[1,5-c]pyrimidine (Compound 294)

The subject compound (34%) was obtained as a pale brown oily matter from 8-formyl-2-(2-furyl)-5-methylthio [1,2,4]triazolo[1,5-c]pyrimidine, 2,6-dimethylmorpholine and 4-benzylpiperidine in a manner similar to that in Example 251.
APCIMS m/z: $[M + H]^+$ 487

Example 294

8-(4-Benzyl-4-hydroxypiperidinomethyl)-5-(2,6-dimethylmorpholino)-2-(2-furyl)[1,2,4]triazolo[1,5-c]pyrimidine (Compound 295)

The subject compound (29%) was obtained as a pale brown oily matter from 8-formyl-2-(2-furyl)-5-methylthio [1,2,4]triazolo[1,5-c]pyrimidine, 2,6-dimethylmorpholine and 4-benzyl-4-hydroxypiperidine in a manner similar to that in Example 251.
APCIMS m/z: $[M + H]^+$ 503

Example 295

5-(2,6-Dimethylmorpholino)-2-(2-furyl)-8-(4-phenylpiperazin-1-ylmethyl)[1,2,4]triazolo[1,5-c]pyrimidine (Compound 296)

The subject compound (37%) was obtained as a pale brown oily matter from 8-formyl-2-(2-furyl)-5-methylthio [1,2,4]triazolo[1,5-c]pyrimidine, 2,6-dimethylmorpholine and 1-phenylpiperazine in a manner similar to that in Example 251.
APCIMS m/z: $[M + H]^+$ 474

Example 296

5-(2,6-Dimethylmorpholino)-2-(2-furyl)-8-(4-phenyl-1,2,3,6-tetrahydropyridin-1-ylmethyl)[1,2,4]triazolo[1,5-c] pyrimidine (Compound 297)

The subject compound (39%) was obtained as a pale brown oily matter from 8-formyl-2-(2-furyl)-5-methylthio [1,2,4]triazolo[1,5-c]pyrimidine, 2,6-dimethylmorpholine and 4-phenyl-1,2,3,6-tetrahydropyridine in a manner similar to that in Example 251.
APCIMS m/z: $[M + H]^+$ 471

Example 297

5-(2,6-Dimethylmorpholino)-2-(2-furyl)-8-[4-(2-pyrimidinyl)piperazin-1-ylmethyl] [1,2,4]triazolo[1,5-c]pyrimidine (Compound 298)

The subject compound (24%) was obtained as a pale brown oily matter from 8-formyl-2-(2-furyl)-5-methylthio [1,2,4]triazolo[1,5-c]pyrimidine, 2,6-dimethylmorpholine and 1-(2-pyrimidinyl)piperazine in a manner similar to that in Example 251.
APCIMS m/z: $[M + H]^+$ 476

Example 298

5-(2,6-Dimethylmorpholino)-8-[4-(2-fluorophenyl)piperazin-1-ylmethyl]-2-(2-furyl)[1,2,4]triazolo[1,5-c]pyrimidine (Compound 299)

The subject compound (35%) was obtained as a pale brown oily matter from 8-formyl-2-(2-furyl)-5-methylthio [1,2,4]triazolo[1,5-c]pyrimidine, 2,6-dimethylmorpholine and 1-(2-fluorophenyl)piperazine in a manner similar to that in Example 251.
APCIMS m/z: $[M + H]^+$ 492

Example 299

2-(2-Furyl)-5-(4-piperidinopiperidino)-8-(1,2,3,4-tetrahydroisoquinolin-2-ylmethyl)[1,2,4]triazolo[1,5-c]pyrimidine (Compound 300)

The subject compound (45%) was obtained as a pale brown oily matter from 8-formyl-2-(2-furyl)-5-methylthio [1,2,4]triazolo[1,5-c]pyrimidine, 4-piperidinopiperidine and 1,2,3,4-tetrahydroisoquinoline in a manner similar to that in Example 251.
APCIMS m/z: $[M + H]^+$ 498

Example 300

8-(6,7-Dimethoxy-1,2,3,4-tetrahydroisoquinolin-2-ylmethyl)-2-(2-furyl)-5-(4-piperidinopiperidino) [1,2,4]triazolo[1,5-c] pyrimidine (Compound 301)

The subject compound (47%) was obtained as a pale brown oily matter from 8-formyl-2-(2-furyl)-5-methylthio [1,2,4]triazolo[1,5-c]pyrimidine, 4-piperidinopiperidine and 6,7-dimethoxy-1,2,3,4-tetrahydroisoquinoline in a manner similar to that in Example 251.
APCIMS m/z: $[M + H]^+$ 558

Example 301

8- (4-Benzylpiperidinomethyl) -2- (2-furyl) -5- (4-piperidinopiperidino)[1,2,4]triazolo[1,5-c]pyrimidine (Compound 302)

The subject compound (34%) was obtained as a pale brown oily matter from 8-formyl-2-(2-furyl)-5-methylthio [1,2,4]triazolo[1,5-c]pyrimidine, 4-piperidinopiperidine and 4-benzylpiperidine in a manner similar to that in Example 251.
APCIMS m/z: $[M + H]^+$ 540

Example 302

8-(4-Benzyl-4-hydroxypiperidinomethyl)-2-(2-furyl)-5-(4-piperidinopiperidino) [1,2,4]triazolo[1,5-c]pyrimidine (Compound 303)

The subject compound (46%) was obtained as a pale brown oily matter from 8-formyl-2-(2-furyl)-5-methylthio [1,2,4]triazolo[1,5-c]pyrimidine, 4-piperidinopiperidine and 4-benzyl-4-hydroxypiperidine in a manner similar to that in Example 251.
APCIMS m/z: $[M + H]^+$ 556

Example 303

2-(2-Furyl)-8-(4-phenylpiperazin-1-ylmethyl)-5-(4-piperidinopiperidino)[1,2,4]triazolo[1,5-c]pyrimidine (Compound 304)

The subject compound (39%) was obtained as a pale brown oily matter from 8-formyl-2-(2-furyl)-5-methylthio [1,2,4]triazolo[1,5-c]pyrimidine, 4-piperidinopiperidine and 1-phenylpiperazine in a manner similar to that in Example 251.
APCIMS m/z: $[M + H]^+$ 527

Example 304

2-(2-Furyl)-8-(4-phenyl-1,2,3,6-tetrahydropyridin-1-ylmethyl)-5-(4-piperidinopiperidino)[1,2,4]triazolo[1,5-c]pyrimidine (Compound 305)

The subject compound (38%) was obtained as a pale brown oily matter from 8-formyl-2-(2-furyl)-5-methylthio [1,2,4]triazolo[1,5-c]pyrimidine, 4-piperidinopiperidine and 4-phenyl-1,2,3,6-tetrahydropyridine in a manner similar to that in Example 251.
APCIMS m/z: $[M + H]^+$ 523

Example 305

2-(2-Furyl)-5-(4-piperidinopiperidino)-8-[4-(2-pyrimidinyl)piperazin-1-ylmethyl][1,2,4]triazolo[1,5-c]pyrimidine (Compound 306)

The subject compound (48%) was obtained as a pale brown oily matter from 8-formyl-2-(2-furyl)-5-methylthio [1,2,4]triazolo[1,5-c]pyrimidine, 4-piperidinopiperidine and 1-(2-pyrimidinyl)piperazine in a manner similar to that in Example 251.
APCIMS m/z: $[M + H]^+$ 529

Example 306

8-[4-(2-Fluorophenyl)piperazin-1-ylmethyl]-2-(2-furyl)-5-(4-piperidinopiperidino) [1,2,4]triazolo[1,5-c]pyrimidine (Compound 307)

The subject compound (35%) was obtained as a pale brown oily matter from 8-formyl-2-(2-furyl)-5-methylthio [1,2,4]triazolo[1,5-c]pyrimidine, 4-piperidinopiperidine and 1-(2-fluorophenyl)piperazine in a manner similar to that in Example 251.
APCIMS m/z: $[M + H]^+$ 545

Example 307

5-(4-Benzylpiperidino)-2-(2-furyl)-8-(1,2,3,4-tetrahydroisoquinolin-2-ylmethyl) [1,2,4]triazolo[1,5-c]pyrimidine (Compound 308)

The subject compound (25%) was obtained as a pale brown oily matter from 8-formyl-2-(2-furyl)-5-methylthio [1,2,4]triazolo[1,5-c]pyrimidine, 4-benzylpiperidine and 1,2,3,4-tetrahydroisoquinoline in a manner similar to that in Example 251.

APCIMS m/z: $[M + H]^+$ 505

Example 308

5-(4-Benzylpiperidino)-8-(6,7-dimethoxy-1,2,3,4-tetrahydroisoquinolin-2-ylmethyl)-2-(2-furyl)[1,2,4]triazolo[1,5-c] pyrimidine (Compound 309)

The subject compound (25%) was obtained as a pale brown oily matter from 8-formyl-2-(2-furyl)-5-methylthio [1,2,4]triazolo[1,5-c]pyrimidine, 4-benzylpiperidine and 6,7-dimethoxy-1,2,3,4-tetrahydroisoquinoline in a manner similar to that in Example 251.
APCIMS m/z: $[M + H]^+$ 565

Example 309

5-(4-Benzylpiperidino)-8-(4-benzylpiperidinomethyl)-2-(2-furyl) [1,2,4]triazolo[1,5-c]pyrimidine (Compound 310)

The subject compound (25%) was obtained as a pale brown oily matter from 8-formyl-2-(2-furyl)-5-methylthio [1,2,4]triazolo[1,5-c]pyrimidine and 4-benzylpiperidine in a manner similar to that in Example 251.
APCIMS m/z: $[M + H]^+$ 547

Example 310

8-(4-Benzyl-4-hydroxypiperidinomethyl)-5-(4-benzylpiperidino)-2-(2-furyl)[1,2,4]triazolo[1,5-c]pyrimidine (Compound 311)

The subject compound (15%) was obtained as a pale brown oily matter from 8-formyl-2-(2-furyl)-5-methylthio [2,2,4]triazolo[1,5-c]pyrimidine, 4-benzylpiperidine and 4-benzyl-4-hydroxypiperidine in a manner similar to that in Example 251.
APCIMS m/z: $[M + H]^+$ 563

Example 311

5-(4-Benzylpiperidino)-2-(2-furyl)-8-(4-phenylpiperazin-1-ylmethyl)[1,2,4]triazolo[1,5-c]pyrimidine (Compound 312)

The subject compound (26%) was obtained as a pale brown oily matter from 8-formyl-2-(2-furyl)-5-methylthio [1,2,4]triazolo[1,5-c]pyrimidine, 4-benzylpiperidine and 1-phenylpiperazine in a manner similar to that in Example 251.
APCIMS m/z: $[M + H]^+$ 534

Example 312

5-(4-Benzylpiperidino)-2-(2-furyl)-8-(4-phenyl-1,2,3,6-tetrahydropyridin-1-ylmethyl) [1,2,4]triazolo[1,5-c]pyrimidine (Compound 313)

The subject compound (23%) was obtained as a pale brown oily matter from 8-formyl-2-(2-furyl)-5-methylthio [1,2,4]triazolo[1,5-c]pyrimidine, 4-benzylpiperidine and 4-phenyl-1,2,3,6-tetrahydropyridine in a manner similar to that in Example 251.
APCIMS m/z: $[M + H]^+$ 531

Example 313

5-(4-Benzylpiperidino)-2-(2-furyl)-8-[4-(2-pyrimidinyl)piperazin-1-ylmethyl][1,2,4]triazolo[1,5-c]pyrimidine (Compound 314)

The subject compound (33%) was obtained as a pale brown oily matter from 8-formyl-2-(2-furyl)-5-methylthio [1,2,4]triazolo[1,5-c]pyrimidine, 4-benzylpiperidine and 1-(2-pyrimidinyl)piperazine in a manner similar to that in Example 251.
APCIMS m/z: $[M + H]^+$ 536

Example 314

5-(4-Benzylpiperidino)-8-[4-(2-fluorophenyl)piperazin-1-ylmethyl]-2-(2-furyl)[1,2,4]triazolo[1,5-c]pyrimidine (Compound 315)

The subject compound (29%) was obtained as a pale brown oily matter from 8-formyl-2-(2-furyl)-5-methylthio [1,2,4]triazolo[1,5-c]pyrimidine, 4-benzylpiperidine and 1-(2-fluorophenyl)piperazine in a manner similar to that in Example 251.
APCIMS m/z: $[M + H]^+$ 552

Example 315

2-(2-Furyl)-5-propylamino-8-(1,2,3,4-tetrahydroisoquinolin-2-ylmethyl)[1,2,4]triazolo[1,5-c]pyrimidine (Compound 316)

8-Formyl-2-(2-furyl)-5-methylthio[1,2,4]triazolo[1,5-c]pyrimidine (13.0 mg, 0.05 mmol) obtained in Reference Example 47 was suspended in 1,2-dimethoxyethane (300 μl), and water (20 μl) and propylamine (6.2 μl, 0.075 mmol) were added thereto, followed by stirring at 80°C for 17 hours. The solvent was distilled away under reduced pressure, and the resulting residue was dissolved in chloroform (600 μl). To the solution were added benzoyl chloride polymer bound (41.0 mg, rolling ratio: up to 2.1 mmol/g resin) and poly(4-vinylpyridine) (22.1 mg), followed by stirring at room temperature for 20 hours. After filtration of the reaction mixture, the solvent was distilled away from the filtrate. The resulting residue was suspended in dichloroethane (600 μl), and 1,2,3,4-tetrahydroisoquinoline (12.5 μl, 0.1 mmol) and sodium triacetoxyborohydride (31.8 mg, 0.15 mmol) were added thereto, followed by stirring at room temperature for 16 hours. To the reaction mixture was added a 3 mol/l aqueous sodium hydroxide solution, and the mixture was subjected to extraction with dichloromethane. After the organic layer was dried over anhydrous magnesium sulfate, the solvent was distilled away under reduced pressure, and the resulting residue was dissolved in chloroform (640 μl) and methanol (160 μl). To the solution were added benzoyl chloride polymer bound (41.0 mg, rolling ratio: up to 2.1 mmol/g resin) and poly(4-vinylpyridine) (22.1 mg), followed by stirring at room temperature for 16 hours. After filtration of the reaction mixture, the solvent was distilled away from the filtrate, and the resulting residue was purified by silica gel column chromatography (eluted with chloroform/methanol = 100/3) to obtain the subject compound (3.1 mg, 16%) as a pale brown oily matter.
APCIMS m/z: $[M + H]^+$ 388

Example 316

8-(6,7-Dimethoxy-1,2,3,4-tetrahydroisoquinolin-2-ylmethyl)-2-(2-furyl)-5-propylamino[1,2,4]triazolo[1,5-c]pyrimidine (Compound 317)

The subject compound (23%) was obtained as a pale brown oily matter from 8-formyl-2-(2-furyl)-5-methylthio [1,2,4]triazolo[1,5-c]pyrimidine, propylamine and 6,7-dimethoxy-1,2,3,4-tetrahydroisoquinoline in a manner similar to that in Example 315.
APCIMS m/z: $[M + H]^+$ 449

Example 317

8-(4-Benzylpiperidinomethyl)-2-(2-furyl)-5-propylamino[1,2,4]triazolo[1,5-c]pyrimidine (Compound 318)

The subject compound (20%) was obtained as a pale brown oily matter from 8-formyl-2-(2-furyl)-5-methylthio [1,2,4]triazolo[1,5-c]pyrimidine, propylamine and 4-benzylpiperidine in a manner similar to that in Example 315.
APCIMS m/z: $[M + H]^+$ 431

Example 318

8-(4-Benzyl-4-hydroxypiperidinomethyl)-2-(2-furyl)-5-propylamino[1,2,4]triazolo[1,5-c]pyrimidine (Compound 319)

The subject compound (14%) was obtained as a pale brown oily matter from 8-formyl-2-(2-furyl)-5-methylthio [1,2,4]triazolo[1,5-c]pyrimidine, propylamine and 4-benzyl-4-hydroxypiperidine in a manner similar to that in Example 315.

APCIMS m/z: $[M + H]^+$ 447

Example 319

2-(2-Furyl)-8-(4-phenylpiperazin-1-ylmethyl)-5-propylamino[1,2,4]triazolo[1,5-c]pyrimidine (Compound 320)

The subject compound (19%) was obtained as a pale brown oily matter from 8-formyl-2-(2-furyl)-5-methylthio [1,2,4]triazolo[1,5-c]pyrimidine, propylamine and 1-phenylpiperazine in a manner similar to that in Example 315.
APCIMS m/z: $[M + H]^+$ 418

Example 320

2-(2-Furyl)-8-(4-phenyl-1,2,3,6-tetrahydropyridin-1-ylmethyl)-5-propylamino[1,2,4]triazolo[1,5-c]pyrimidine (Compound 321)

The subject compound (25%) was obtained as a pale brown oily matter from 8-formyl-2-(2-furyl)-5-methylthio [1,2,4]triazolo[1,5-c]pyrimidine, propylamine and 4-phenyl-1,2,3,6-tetrahydropyridine in a manner similar to that in Example 315.
APCIMS m/z: $[M + H]^+$ 415

Example 321

2-(2-Furyl)-5-propylamino-8-[4-(2-pyrimidinyl)piperazin-1-ylmethyl][1,2,4]triazolo[1,5-c]pyrimidine (Compound 322)

The subject compound (12%) was obtained as a pale brown oily matter from 8-formyl-2-(2-furyl)-5-methylthio [1,2,4]triazolo[1,5-c]pyrimidine, propylamine and 1-(2-pyrimidinyl)piperazine in a manner similar to that in Example 315.
APCIMS m/z: $[M + H]^+$ 420

Example 322

8-[4-(2-Fluorophenyl)piperazin-1-ylmethyl]-2-(2-furyl)-5-propylamino[1,2,4]triazolo[1,5-c]pyrimidine (Compound 323)

The subject compound (19%) was obtained as a pale brown oily matter from 8-formyl-2-(2-furyl)-5-methylthio [1,2,4]triazolo[1,5-c]pyrimidine, propylamine and 1-(2-fluorophenyl)piperazine in a manner similar to that in Example 315.
APCIMS m/z: $[M + H]^+$ 436

Example 323

2-(2-Furyl)-5-(3-methoxypropylamino)-8-(1,2,3,4-tetrahydroisoquinolin-2-ylmethyl)[1,2,4]triazolo[1,5-c]pyrimidine (Compound 324)

The subject compound (30%) was obtained as a pale brown oily matter from 8-formyl-2-(2-furyl)-5-methylthio [1,2,4]triazolo[1,5-c]pyrimidine, 3-methoxypropylamine and 1,2,3,4-tetrahydroisoquinoline in a manner similar to that in Example 315.
APCIMS m/z: $[M + H]^+$ 419

Example 324

8-(6,7-Dimethoxy-1,2,3,4-tetrahydroisoquinolin-2-ylmethyl)-2-(2-furyl)-5-(3-methoxypropylamino)[1,2,4]triazolo[1,5-c] pyrimidine (Compound 325)

The subject compound (16%) was obtained as a pale brown oily matter from 8-formyl-2-(2-furyl)-5-methylthio [1,2,4]triazolo[1,5-c]pyrimidine, 3-methoxypropylamine and 6,7-dimethoxy-1,2,3,4-tetrahydroisoquinoline in a manner similar to that in Example 315.
APCIMS m/z: $[M + H]^+$ 479

Example 325

8-(4-Benzylpiperidinomethyl)-2-(2-furyl)-5-(3-methoxypropylamino)[1,2,4]triazolo[1,5-c]pyrimidine (Compound 326)

The subject compound (12%) was obtained as a pale brown oily matter from 8-formyl-2-(2-furyl)-5-methylthio [1,2,4]triazolo[1,5-c]pyrimidine, 3-methoxypropylamine and 4-benzylpiperidine in a manner similar to that in Example 315.
APCIMS m/z: $[M + H]^+$ 461

Example 326

8-(4-Benzyl-4-hydroxypiperidinomethyl)-2-(2-furyl)-5-(3-methoxypropylamino) [1,2,4]triazolo[1,5-c]pyrimidine (Compound 327)

The subject compound (21%) was obtained as a pale brown oily matter from 8-formyl-2-(2-furyl)-5-methylthio [1,2,4]triazolo[1,5-c]pyrimidine, 3-methoxypropylamine and 4-benzyl-4-hydroxypiperidine in a manner similar to that in Example 315.
APCIMS m/z: $[M + H]^+$ 477

Example 327

2-(2-Furyl)-5-(3-methoxypropylamino)-8-(4-phenylpiperazin-1-ylmethyl)[1,2,4]triazolo[1,5-c]pyrimidine (Compound 328)

The subject compound (17%) was obtained as a pale brown oily matter from 8-formyl-2-(2-furyl)-5-methylthio [1,2,4]triazolo[1,5-c]pyrimidine, 3-methoxypropylamine and 1-phenylpiperazine in a manner similar to that in Example 315.
APCIMS m/z: $[M + H]^+$ 448

Example 328

2-(2-Furyl)-5-(3-methoxypropylamino)-8-(4-phenyl-1,2,3,6-tetrahydropyridin-1-ylmethyl)[1,2,4]triazolo[1,5-c] pyrimidine (Compound 329)

The subject compound (24%) was obtained as a pale brown oily matter from 8-formyl-2-(2-furyl)-5-methylthio [1,2,4]triazolo[1,5-c]pyrimidine, 3-methoxypropylamine and 4-phenyl-1,2,3,6-tetrahydropyridine in a manner similar to that in Example 315.
APCIMS m/z: $[M + H]^+$ 445

Example 329

2-(2-Furyl)-5-(3-methoxypropylamino)-8-[4-(2-pyrimidinyl)piperazin-1-ylmethyl] [1,2,4]triazolo[1,5-c]pyrimidine (Compound 330)

The subject compound (24%) was obtained as a pale brown oily matter from 8-formyl-2-(2-furyl)-5-methylthio [1,2,4]triazolo[1,5-c]pyrimidine, 3-methoxypropylamine and 1-(2-pyrimidinyl)piperazine in a manner similar to that in Example 315.
APCIMS m/z: $[M + H]^+$ 450

Example 330

8-[4-(2-Fluorophenyl)piperazin-1-ylmethyl]-2-(2-furyl)-5-(3-methoxypropylamino) [1,2,4]triazolo[1,5-c]pyrimidine (Compound 331)

The subject compound (21%) was obtained as a pale brown oily matter from 8-formyl-2-(2-furyl)-5-methylthio [1,2,4]triazolo[1,5-c]pyrimidine, 3-methoxypropylamine and 1-(2-fluorophenyl)piperazine in a manner similar to that in Example 315.
APCIMS m/z: $[M + H]^+$ 467

Example 331

5-[2-(Dimethylamino)ethylamino]-2-(2-furyl)-8-(1,2,3,4-tetrahydroisoquinolin-2-ylmethyl)[1,2,4]triazolo[1,5-c]pyrimidine (Compound 332)

The subject compound (22%) was obtained as a pale brown oily matter from 8-formyl-2-(2-furyl)-5-methylthio [1,2,4]triazolo[1,5-c]pyrimidine, N,N-dimethylethylenediamine and 1,2,3,4-tetrahydroisoquinoline in a manner similar to that in Example 315.
APCIMS m/z: $[M + H]^+$ 418

Example 332

8-(6,7-Dimethoxy-1,2,3,4-tetrahydroisoquinolin-2-ylmethyl)-5-[2-(dimethylamino)ethylamino]-2-(2-furyl)[1,2,4]triazolo [1,5-c]pyrimidine (Compound 333)

The subject compound (21%) was obtained as a pale brown oily matter from 8-formyl-2-(2-furyl)-5-methylthio [1,2,4]triazolo[1,5-c]pyrimidine, N,N-dimethylethylenediamine and 6,7-dimethoxy-1,2,3,4-tetrahydroisoquinoline in a manner similar to that in Example 315.
APCIMS m/z: $[M + H]^+$ 478

Example 333

8-(4-Benzylpiperidinomethyl)-5-[2-(dimethylamino)ethylamino]-2-(2-furyl)[1,2,4]triazolo [1,5-c]pyrimidine (Compound 334)

The subject compound (28%) was obtained as a pale brown oily matter from 8-formyl-2-(2-furyl)-5-methylthio [1,2,4]triazolo[1,5-c]pyrimidine, N,N-dimethylethylenediamine and 4-benzylpiperidine in a manner similar to that in Example 315.
APCIMS m/z: $[M + H]^+$ 460

Example 334

8-(4-Benzyl-4-hydroxypiperidinomethyl)-5-[2-(dimethylamino)ethylamino]-2-(2-furyl)[1,2,4]triazolo[1,5-c]pyrimidine (Compound 335)

The subject compound (15%) was obtained as a pale brown oily matter from 8-formyl-2-(2-furyl)-5-methylthio [1,2,4]triazolo[1,5-c]pyrimidine, N,N-dimethylethylenediamine and 4-benzyl-4-hydroxypiperidine in a manner similar to that in Example 315.
APCIMS m/z: $[M + H]^+$ 476

Example 335

5-[2-(Dimethylamino)ethylamino]-2-(2-furyl)-8-(4-phenylpiperazin-1-ylmethyl)[1,2,4]triazolo[1,5-c]pyrimidine (Compound 336)

The subject compound (25%) was obtained as a pale brown oily matter from 8-formyl-2-(2-furyl)-5-methylthio [1,2,4)triazolo[1,5-c]pyrimidine, N,N-dimethylethylenediamine and 1-phenylpiperazine in a manner similar to that in Example 315.
APCIMS m/z: $[M + H]^+$ 447

Example 336

5- [2- (Dimethylamino)ethylamino]-2- (2-furyl) -8- (4-phenyl-1,2,3,6-tetrahydropyridin-1-ylmethyl)[1,2,4]triazolo[1,5-c] pyrimidine (Compound 337)

The subject compound (19%) was obtained as a pale brown oily matter from 8-formyl-2-(2-furyl)-5-methylthio [1,2,4]triazolo[1,5-c]pyrimidine, N,N-dimethylethylenediamine and 4-phenyl-1,2,3,6-tetrahydropyridine in a manner similar to that in Example 315.

APCIMS m/z: $[M + H]^+$ 444

Example 337

5-[2-(Dimethylamino)ethylamino]-2-(2-furyl)-8-[4-(2-pyrimidinyl)piperazin-1-ylmethyl][1,2,4]triazolo[1,5-c]pyrimidine (Compound 338)

The subject compound (31%) was obtained as a pale brown oily matter from 8-formyl-2-(2-furyl)-5-methylthio [1,2,4]triazolo[1,5-c]pyrimidine, N,N-dimethylethylenediamine and 1-(2-pyrimidinyl)piperazine in a manner similar to that in Example 315.
APCIMS m/z: $[M + H]^+$ 449

Example 338

5-[2-(Dimethylamino)ethylamino]-8-[4-(2-fluorophenyl)piperazin-1-ylmethyl]-2-(2-furyl)[1,2,4]triazolo[1,5-c]pyrimidine (Compound 339)

The subject compound (21%) was obtained as a pale brown oily matter from 8-formyl-2-(2-furyl)-5-methylthio [1,2,4]triazolo[1,5-c]pyrimidine, N,N-dimethylethylenediamine and 1-(2-fluorophenyl)piperazine in a manner similar to that in Example 315.
APCIMS m/z: $[M + H]^+$ 465

Example 339

2-(2-Furyl)-5-isopentylamino-8-(1,2,3,4-tetrahydroisoquinolin-2-ylmethyl) [1,2,4]triazolo[1,5-c]pyrimidine (Compound 340)

The subject compound (28%) was obtained as a pale brown oily matter from 8-formyl-2-(2-furyl)-5-methylthio [1,2,4]triazolo[1,5-c]pyrimidine, isoamylamine and 1,2,3,4-tetrahydroisoquinoline in a manner similar to that in Example 315.
APCIMS m/z: $[M + H]^+$ 417

Example 340

8-(6,7-Dimethoxy-1,2,3,4-tetrahydroisoquinolin-2-ylmethyl)-2-(2-furyl)-5-isopentylamino[1,2,4]triazolo[1,5-c] pyrimidine (Compound 341)

The subject compound (20%) was obtained as a pale brown oily matter from 8-formyl-2-(2-furyl)-5-methylthio [1,2,4]triazolo[1,5-c]pyrimidine, isoamylamine and 6,7-dimethoxy-1,2,3,4-tetrahydroisoquinoline in a manner similar to that in Example 315.
APCIMS m/z: $[M + H]^+$ 477

Example 341

8-(4-Benzylpiperidinomethyl)-2-(2-furyl)-5-isopentylamino[1,2,4]triazolo[1,5-c]pyrimidine (Compound 342)

The subject compound (19%) was obtained as a pale brown oily matter from 8-formyl-2-(2-furyl)-5-methylthio [1,2,4]triazolo[1,5-c]pyrimidine, isoamylamine and 4-benzylpiperidine in a manner similar to that in Example 315.
APCIMS m/z: $[M + H]^+$ 459

Example 342

8-(4-Benzyl-4-hydroxypiperidinomethyl)-2-(2-furyl)-5-isopentylamino[1,2,4]triazolo[1,5-c]pyrimidine (Compound 343)

The subject compound (26%) was obtained as a pale brown oily matter from 8-formyl-2-(2-furyl)-5-methylthio [1,2,4]triazolo[1,5-c]pyrimidine, isoamylamine and 4-benzyl-4-hydroxypiperidine in a manner similar to that in Example 315.
APCIMS m/z: $[M + H]^+$ 475

Example 343

2-(2-Furyl)-5-isopentylamino-8-(4-phenylpiperazin-1-ylmethyl) [1,2,4]triazolo[1,5-c]pyrimidine (Compound 344)

The subject compound (22%) was obtained as a pale brown oily matter from 8-formyl-2-(2-furyl)-5-methylthio [1,2,4]triazolo[1,5-c]pyrimidine, isoamylamine and 1-phenylpiperazine in a manner similar to that in Example 315.
APCIMS m/z: $[M + H]^+$ 446

Example 344

2-(2-Furyl)-5-isopentylamino-8-(4-phenyl-1,2,3,6-tetrahydropyridin-1-ylmethyl) [1,2,4]triazolo[1,5-c]pyrimidine (Compound 345)

The subject compound (20%) was obtained as a pale brown oily matter from 8-formyl-2-(2-furyl)-5-methylthio [1,2,4]triazolo[1,5-c]pyrimidine, isoamylamine and 4-phenyl-1,2,3,6-tetrahydropyridine in a manner similar to that in Example 315.
APCIMS m/z: $[M + H]^+$ 443

Example 345

2-(2-Furyl)-5-isopentylamino-8-[4-(2-pyrimidinyl)piperazin-1-ylmethyl][1,2,4]triazolo[1,5-c]pyrimidine (Compound 346)

The subject compound (28%) was obtained as a pale brown oily matter from 8-formyl-2-(2-furyl)-5-methylthio [1,2,4]triazolo[1,5-c]pyrimidine, isoamylamine and 1-(2-pyrimidinyl)piperazine in a manner similar to that in Example 315.
APCIMS m/z: $[M + H]^+$ 448

Example 346

8-[4-(2-Fluorophenyl)piperazin-1-ylmethyl]-2-(2-furyl)-5-isopentylamino[1,2,4]triazolo[1,5-c]pyrimidine (Compound 347)

The subject compound (16%) was obtained as a pale brown oily matter from 8-formyl-2-(2-furyl)-5-methylthio [1,2,4]triazolo[1,5-c]pyrimidine, isoamylamine and 1-(2-fluorophenyl)piperazine in a manner similar to that in Example 315.
APCIMS m/z: $[M + H]^+$ 464

Example 347

2-(2-Furyl)-5-(N-methylphenethylamino)-8-(1,2,3,4-tetrahydroisoquinolin-2-ylmethyl)[1,2,4]triazolo[1,5-c]pyrimidine (Compound 348)

The subject compound (36%) was obtained as a pale brown oily matter from 8-formyl-2-(2-furyl)-5-methylthio [1,2,4]triazolo[1,5-c]pyrimidine, N-methylphenethylamine and 1,2,3,4-tetrahydroisoquinoline in a manner similar to that in Example 251.
APCIMS m/z: $[M + H]^+$ 465

Example 348

8-(6,7-Dimethoxy-1,2,3,4-tetrahydroisoquinolin-2-ylmethyl)-2-(2-furyl)-5-(N-methylphenethylamino)[1,2,4]triazolo [1,5-c]pyrimidine (Compound 349)

The subject compound (45%) was obtained as a pale brown oily matter from 8-formyl-2-(2-furyl)-5-methylthio [1,2,4]triazolo[1,5-c]pyrimidine, N-methylphenethylamine and 6,7-dimethoxy-1,2,3,4-tetrahydroisoquinoline in a manner similar to that in Example 251.
APCIMS m/z: $[M + H]^+$ 525

Example 349

2-(2-Furyl)-8-(7-methoxy-1,2,3,4-tetrahydroisoquinolin-2-ylmethyl)-5-(N-methylphenethylamino)[1,2,4]triazolo[1,5-c] pyrimidine (Compound 350)

The subject compound (48%) was obtained as a pale brown oily matter from 8-formyl-2-(2-furyl)-5-methylthio [1,2,4]triazolo[1,5-c]pyrimidine, N-methylphenethylamine and 7-methoxy-1,2,3,4-tetrahydroisoquinoline in a manner similar to that in Example 251.
APCIMS m/z: $[M + H]^+$ 495

Example 350

2-(2-Furyl)-5-(N-methylphenethylamino)-8-(4-phenyl-1,2,3,6-tetrahydropyridin-1-ylmethyl)[1,2,4]triazolo[1,5-c] pyrimidine (Compound 351)

The subject compound (41%) was obtained as a pale brown oily matter from 8-formyl-2-(2-furyl)-5-methylthio [1,2,4]triazolo[1,5-c]pyrimidine, N-methylphenethylamine and 4-phenyl-1,2,3,6-tetrahydropyridine in a manner similar to that in Example 251.
APCIMS m/z: $[M + H]^+$ 491

Example 351

8-[4-(4-Fluorophenyl)-1,2,3,6-tetrahydropyridin-1-ylmethyl]-2-(2-furyl)-5-(N-methylphenethylamino)[1,2,4]triazolo [1,5-c]pyrimidine (Compound 352)

The subject compound (52%) was obtained as a pale brown oily matter from 8-formyl-2-(2-furyl)-5-methylthio [1,2,4]triazolo[1,5-c]pyrimidine, N-methylphenethylamine and 4-(4-fluorophenyl)-1,2,3,6-tetrahydropyridine in a manner similar to that in Example 251.
APCIMS m/z: $[M + H]^+$ 509

Example 352

2-(2-Furyl)-5-(N-methylphenethylamino)-8-(4-phenylpiperazin-1-ylmethyl) [1,2,4]triazolo[1,5-c]pyrimidine (Compound 353)

The subject compound (52%) was obtained as a pale brown oily matter from 8-formyl-2-(2-furyl)-5-methylthio [1,2,4]triazolo[1,5-c]pyrimidine, N-methylphenethylamine and 1-phenylpiperazine in a manner similar to that in Example 251.
APCIMS m/z: $[M + H]^+$ 494

Example 353

8-[4-(2-Fluorophenyl)piperazin-1-ylmethyl]-2-(2-furyl)-5-(N-methylphenethylamino)[1,2,4]triazolo[1,5-clpyrimidine (Compound 354)

The subject compound (48%) was obtained as a pale brown oily matter from 8-formyl-2-(2-furyl)-5-methylthio [1,2,4]triazolo[1,5-c]pyrimidine, N-methylphenethylamine and 1-(2-fluorophenyl)piperazine in a manner similar to that in Example 251.
APCIMS m/z: $[M + H]^+$ 512

Example 354

2-(2-Furyl)-5-(N-methylphenethylamino)-8-(4-phenylpiperidinomethyl)[1,2,4]triazolo[1,5-c]pyrimidine (Compound 355)

The subject compound (43%) was obtained as a pale brown oily matter from 8-formyl-2-(2-furyl)-5-methylthio [1,2,4]triazolo[1,5-c]pyrimidine, N-methylphenethylamine and 4-phenylpiperidine in a manner similar to that in Example 251.

APCIMS m/z: $[M + H]^+$ 493

Example 355

2-(2-Furyl)-5-(N-methylbutylamino)-8-(1,2,3,4-tetrahydroisoquinolin-2-ylmethyl)[1,2,4]triazolo[1,5-c]pyrimidine (Compound 356)

The subject compound (37%) was obtained as a pale brown oily matter from 8-formyl-2-(2-furyl)-5-methylthio [1,2,4]triazolo[1,5-c]pyrimidine, N-methylbutylamine and 1,2,3,4-tetrahydroisoquinoline in a manner similar to that in Example 251.
APCIMS m/z: $[M + H]^+$ 417

Example 356

8-(6,7-Dimethoxy-1,2,3,4-tetrahydroisoquinolin-2-ylmethyl)-2-(2-furyl)-5-(N-methylbutylamino)[1,2,4]triazolo[1,5-c] pyrimidine (Compound 357)

The subject compound (49%) was obtained as a pale brown oily matter from 8-formyl-2-(2-furyl)-5-methylthio [1,2,4]triazolo[1,5-c]pyrimidine, N-methylbutylamine and 6,7-dimethoxy-1,2,3,4-tetrahydroisoquinoline in a manner similar to that in Example 251.
APCIMS m/z: $[M + H]^+$ 477

Example 357

2-(2-Furyl)-8-(7-methoxy-1,2,3,4-tetrahydroisoquinolin-2-ylmethyl)-5-(N-methylbutylamino)[1,2,4]triazolo[1,5-c] pyrimidine (Compound 358)

The subject compound (44%) was obtained as a pale brown oily matter from 8-formyl-2-(2-furyl)-5-methylthio [1,2,4]triazolo[1,5-c]pyrimidine, N-methylbutylamine and 7-methoxy-1,2,3,4-tetrahydroisoquinoline in a manner similar to that in Example 251.
APCIMS m/z: $[M + H]^+$ 447

Example 358

2-(2-Furyl)-5-(N-methylbutylamino)-8-(4-phenyl-1,2,3,6-tetrahydropyridin-1-ylmethyl)[1,2,4]triazolo[1,5-c]pyrimidine (Compound 359)

The subject compound (40%) was obtained as a pale brown oily matter from 8-formyl-2-(2-furyl)-5-methylthio [1,2,4]triazolo[1,5-c]pyrimidine, N-methylbutylamine and 4-phenyl-1,2,3,6-tetrahydropyridine in a manner similar to that in Example 251.
APCIMS m/z: $[M + H]^+$ 443

Example 359

8-[4-(4-Fluorophenyl)-1,2,3,6-tetrahydropyridin-1-ylmethyl]-2-(2-furyl)-5-(N-methylbutylamino)[1,2,4]triazolo[1,5-c] pyrimidine (Compound 360)

The subject compound (39%) was obtained as a pale brown oily matter from 8-formyl-2-(2-furyl)-5-methylthio [1,2,4]triazolo[1,5-c]pyrimidine, N-methylbutylamine and 4-(4-fluorophenyl)-1,2,3,6-tetrahydropyridine in a manner similar to that in Example 251.
APCIMS m/z: $[M + H]^+$ 461

Example 360

2-(2-Furyl)-5-(N-methylbutylamino)-8-(4-phenylpiperazin-1-ylmethyl)[1,2,4]triazolo[1,5-c]pyrimidine (Compound 361)

The subject compound (52%) was obtained as a pale brown oily matter from 8-formyl-2-(2-furyl)-5-methylthio [1,2,4]triazolo[1,5-c]pyrimidine, N-methylbutylamine and 1-phenylpiperazine in a manner similar to that in Example 251.

APCIMS m/z: $[M + H]^+$ 446

Example 361

8-[4-(2-Fluorophenyl)piperazin-1-ylmethyl]-2-(2-furyl)-5-(N-methylbutylamino) [1,2,4]triazolo[1,5-c]pyrimidine (Compound 362)

The subject compound (48%) was obtained as a pale brown oily matter from 8-formyl-2- (2-furyl) -5-methylthio [1,2,4]triazolo[1,5-c]pyrimidine, N-methylbutylamine and 1-(2-fluorophenyl)piperazine in a manner similar to that in Example 251.
APCIMS m/z: $[M + H]^+$ 464

Example 362

2-(2-Furyl)-5-(N-methylbutylamino)-8-(4-phenylpiperidinomethyl)[1,2,4]triazolo[1,5-c]pyrimidine (Compound 363)

The subject compound (45%) was obtained as a pale brown oily matter from 8-formyl-2-(2-furyl)-5-methylthio [1,2,4]triazolo[1,5-c]pyrimidine, N-methylbutylamine and 4-phenylpiperidine in a manner similar to that in Example 251.
APCIMS m/z: $[M + H]^+$ 445

Example 363

2-(2-Furyl)-5-(N-methylbenzylamino)-8-(1,2,3,4-tetrahydroisoquinolin-2-ylmethyl)[1,2,4]triazolo[1,5-c]pyrimidine (Compound 364)

The subject compound (55%) was obtained as a pale brown oily matter from 8-formyl-2-(2-furyl)-5-methylthio [1,2,4]triazolo[1,5-c]pyrimidine, N-methylbenzylamine and 1,2,3,4-tetrahydroisoquinoline in a manner similar to that in Example 251.
APCIMS m/z: $[M + H]^+$ 451

Example 364

8-(6,7-Dimethoxy-1,2,3,4-tetrahydroisoquinolin-2-ylmethyl)-2-(2-furyl)-5-(N-methylbenzylamino)[1,2,4]triazolo[1,5-c] pyrimidine (Compound 365)

The subject compound (43%) was obtained as a pale brown oily matter from 8-formyl-2-(2-furyl)-5-methylthio [1,2,4]triazolo[1,5-c]pyrimidine, N-methylbenzylamine and 6,7-dimethoxy-1,2,3,4-tetrahydroisoquinoline in a manner similar to that in Example 251.
APCIMS m/z: $[M + H]^+$ 511

Example 365

2-(2-Furyl)-8-(7-methoxy-1,2,3,4-tetrahydroisoquinolin-2-ylmethyl)-5-(N-methylbenzylamino)[1,2,4]triazolo[1,5-c] pyrimidine (Compound 366)

The subject compound (41%) was obtained as a pale brown oily matter from 8-formyl-2-(2-furyl)-5-methylthio [1,2,4]triazolo[1,5-c]pyrimidine, N-methylbenzylamine and 7-methoxy-1,2,3,4-tetrahydroisoquinoline in a manner similar to that in Example 251.
APCIMS m/z: $[M + H]^+$ 481

Example 366

2-(2-Furyl)-5-(N-methylbenzylamino)-8-(4-phenyl-1,2,3,6-tetrahydropyridin-1-ylmethyl)[1,2,4]triazolo[1,5-c]pyrimidine (Compound 367)

The subject compound (38%) was obtained as a pale brown oily matter from 8-formyl-2-(2-furyl)-5-methylthio [1,2,4]triazolo[1,5-c]pyrimidine, N-methylbenzylamine and 4-phenyl-1,2,3,6-tetrahydropyridine in a manner similar to that in Example 251.

APCIMS m/z: $[M + H]^+$ 477

Example 367

8-[4-(4-Fluorophenyl)-1,2,3,6-tetrahydropyridin-1-ylmethyl]-2-(2-furyl)-5-(N-methylbenzylamino)[1,2,4]triazolo[1,5-c] pyrimidine (Compound 368)

The subject compound (44%) was obtained as a pale brown oily matter from 8-formyl-2-(2-furyl)-5-methylthio [1,2,4]triazolo[1,5-c]pyrimidine, N-methylbenzylamine and 4-(4-fluorophenyl)-1,2,3,6-tetrahydropyridine in a manner similar to that in Example 251.
APCIMS m/z: $[M + H]^+$ 495

Example 368

2-(2-Furyl)-5-(N-methylbenzylamino)-8-(4-phenylpiperazin-1-ylmethyl)[1,2,4]triazolo[1,5-c]pyrimidine (Compound 369)

The subject compound (46%) was obtained as a pale brown oily matter from 8-formyl-2-(2-furyl)-5-methylthio [1,2,4]triazolo[1,5-c]pyrimidine, N-methylbenzylamine and 1-phenylpiperazine in a manner similar to that in Example 251.
APCIMS m/z: $[M + H]^+$ 480

Example 369

8-[4-(2-Fluorophenyl)piperazin-1-ylmethyl]-2-(2-furyl)-5-(N-methylbenzylamino)[1,2,4]triazolo[1,5-c]pyrimidine (Compound 370)

The subject compound (51%) was obtained as a pale brown oily matter from 8-formyl-2-(2-furyl)-5-methylthio [1,2,4]triazolo[1,5-c]pyrimidine, N-methylbenzylamine and 1-(2-fluorophenyl)piperazine in a manner similar to that in Example 251.
APCIMS m/z: $[M + H]^+$ 498

Example 370

2-(2-Furyl)-5-(N-methylbenzylamino)-8-(4-phenylpiperidinomethyl) [1,2,4]triazolo[1,5-c]pyrimidine (Compound 371)

The subject compound (38%) was obtained as a pale brown oily matter from 8-formyl-2-(2-furyl)-5-methylthio [1,2,4]triazolo[1,5-c]pyrimidine, N-methylbenzylamine and 4-phenylpiperidine in a manner similar to that in Example 251.
APCIMS m/z: $[M + H]^+$ 479

Example 371

2-(2-Furyl)-5-[N-(2-methoxyethyl)methylamino]-8-(1,2,3,4-tetrahydroisoquinolin-2-ylmethyl)[1,2,4]triazolo[1,5-c] pyrimidine (Compound 372)

The subject compound (60%) was obtained as a pale brown oily matter from 8-formyl-2-(2-furyl)-5-methylthio [1,2,4]triazolo[1,5-c]pyrimidine, N-(2-methoxyethyl)methylamine and 1,2,3,4-tetrahydroisoquinoline in a manner similar to that in Example 251.
APCIMS m/z: $[M + H]^+$ 419

Example 372

8-(6,7-Dimethoxy-1,2,3,4-tetrahydroisoquinolin-2-ylmethyl)-2-(2-furyl)-5-[N-(2-methoxyethyl) methylamino] [1,2,4] triazolo[1,5-c]pyrimidine (Compound 373)

The subject compound (58%) was obtained as a pale brown oily matter from 8-formyl-2-(2-furyl)-5-methylthio [1,2,4]triazolo[1,5-c]pyrimidine, N-(2-methoxyethyl)methylamine and 6,7-dimethoxy-1,2,3,4-tetrahydroisoquinoline in

a manner similar to that in Example 251.
APCIMS m/z: $[M + H]^+$ 479

Example 373

2-(2-Furyl)-5-[N-(2-methoxyethyl)methylamino]-8-(7-methoxy-1,2,3,4-tetrahydroisoquinolin-2-ylmethyl)[1,2,4]triazolo[1,5-c]pyrimidine (Compound 374)

The subject compound (55%) was obtained as a pale brown oily matter from 8-formyl-2-(2-furyl)-5-methylthio[1,2,4]triazolo[1,5-c]pyrimidine, N-(2-methoxyethyl)methylamine and 7-methoxy-1,2,3,4-tetrahydroisoquinoline in a manner similar to that in Example 251.
APCIMS m/z: $[M + H]^+$ 449

Example 374

2-(2-Furyl)-5-[N-(2-methoxyethyl)methylamino]-8-(4-phenyl-1,2,3,6-tetrahydropyridin-1-ylmethyl) [1,2,4]triazolo[1,5-c]pyrimidine (Compound 375)

The subject compound (47%) was obtained as a pale brown oily matter from 8-formyl-2-(2-furyl)-5-methylthio[1,2,4]triazolo[1,5-c]pyrimidine, N-(2-methoxyethyl)methylamine and 4-phenyl-1,2,3,6-tetrahydropyridine in a manner similar to that in Example 251.
APCIMS m/z: $[M + H]^+$ 445

Example 375

8-[4-(4-Fluorophenyl)-1,2,3,6-tetrahydropyridin-1-ylmethyl]-2-(2-furyl)-5-[N-(2-methoxyethyl)methylamino][1,2,4]triazolo[1,5-c]pyrimidine (Compound 376)

The subject compound (46%) was obtained as a pale brown oily matter from 8-formyl-2-(2-furyl)-5-methylthio[1,2,4]triazolo[1,5-c]pyrimidine, N-(2-methoxyethyl)methylamine and 4-(4-fluorophenyl)-1,2,3,6-tetrahydropyridine in a manner similar to that in Example 251.
APCIMS m/z: $[M + H]^+$ 463

Example 376

2-(2-Furyl)-5-[N-(2-methoxyethyl)methylamino]-8-(4-phenylpiperazin-1-ylmethyl)[1,2,4]triazolo[1,5-c]pyrimidine (Compound 377)

The subject compound (63%) was obtained as a pale brown oily matter from 8-formyl-2-(2-furyl)-5-methylthio[1,2,4]triazolo[1,5-c]pyrimidine, N-(2-methoxyethyl)methylamine and 1-phenylpiperazine in a manner similar to that in Example 251.
APCIMS m/z: $[M + H]^+$ 448

Example 377

8-[4-(2-Fluorophenyl)piperazin-1-ylmethyl]-2-(2-furyl)-5-[N-(2-methoxyethyl)methylamino][1,2,4]triazolo[1,5-c]pyrimidine (Compound 378)

The subject compound (54%) was obtained as a pale brown oily matter from 8-formyl-2-(2-furyl)-5-methylthio[1,2,4]triazolo[1,5-c]pyrimidine, N-(2-methoxyethyl)methylamine and 1-(2-fluorophenyl)piperazine in a manner similar to that in Example 251.
APCIMS m/z: $[M + H]^+$ 466

Example 378

2-(2-Furyl)-5-[N-(2-methoxyethyl)methylamino]-8-(4-phenylpiperidinomethyl)[1,2,4]triazolo[1,5-c]pyrimidine (Compound 379)

The subject compound (52%) was obtained as a pale brown oily matter from 8-formyl-2-(2-furyl)-5-methylthio [1,2,4]triazolo[1,5-c]pyrimidine, N-(2-methoxyethyl)methylamine and 4-phenylpiperidine in a manner similar to that in Example 251.
APCIMS m/z: $[M + H]^+$ 447

Example 379

2-(2-Furyl)-5-(N-methylcyclohexylamino)-8-(1,2,3,4-tetrahydroisoquinolin-2-ylmethyl) [1,2,4]triazolo[1,5-c]pyrimidine (Compound 380)

The subject compound (48%) was obtained as a pale brown oily matter from 8-formyl-2-(2-furyl)-5-methylthio [1,2,4]triazolo[1,5-c]pyrimidine, N-methylcyclohexylamine and 1,2,3,4-tetrahydroisoquinoline in a manner similar to that in Example 251.
APCIMS m/z: $[M + H]^+$ 443

Example 380

8-(6,7-Dimethoxy-1,2,3,4-tetrahydroisoquinolin-2-ylmethyl)-2-(2-furyl)-5-(N-methylcyclohexylamino)[1,2,4]triazolo [1,5-c]pyrimidine (Compound 381)

The subject compound (44%) was obtained as a pale brown oily matter from 8-formyl-2-(2-furyl)-5-methylthio [1,2,4]triazolo[1,5-c]pyrimidine, N-methylcyclohexylamine and 6,7-dimethoxy-1,2,3,4-tetrahydroisoquinoline in a manner similar to that in Example 251.
APCIMS m/z: $[M + H]^+$ 503

Example 381

2-(2-Furyl)-8-(7-methoxy-1,2,3,4-tetrahydroisoquinolin-2-ylmethyl)-5-(N-methylcyclohexylamino)[1,2,4]triazolo[1,5-c] pyrimidine (Compound 382)

The subject compound (46%) was obtained as a pale brown oily matter from 8-formyl-2-(2-furyl)-5-methylthio [1,2,4]triazolo[1,5-c]pyrimidine, N-methylcyclohexylamine and 7-methoxy-1,2,3,4-tetrahydroisoquinoline in a manner similar to that in Example 251.
APCIMS m/z: $[M + H]^+$ 473

Example 382

2-(2-Furyl)-5-(N-methylcyclohexylamino)-8-(4-phenyl-1,2,3,6-tetrahydropyridin-1-ylmethyl)[1,2,4]triazolo[1,5-c] pyrimidine (Compound 383)

The subject compound (44%) was obtained as a pale brown oily matter from 8-formyl-2-(2-furyl)-5-methylthio [1,2,4]triazolo[1,5-c]pyrimidine, N-methylcyclohexylamine and 4-phenyl-1,2,3,6-tetrahydropyridine in a manner similar to that in Example 251.
APCIMS m/z: $[M + H]^+$ 469

Example 383

8-[4-(4-Fluorophenyl)-1,2,3,6-tetrahydropyridin-1-ylmethyl]-2-(2-furyl)-5-(N-methylcyclohexylamino) [1,2,4] triazolo [1,5-c]pyrimidine (Compound 384)

The subject compound (42%) was obtained as a pale brown oily matter from 8-formyl-2-(2-furyl)-5-methylthio [1,2,4]triazolo[1,5-c]pyrimidine, N-methylcyclohexylamine and 4-(4-fluorophenyl)-1,2,3,6-tetrahydropyridine in a manner similar to that in Example 251.

APCIMS m/z: $[M + H]^+$ 487

Example 384

2-(2-Furyl)-5-(N-methylcyclohexylamino)-8-(4-phenylpiperazin-1-ylmethyl)[1,2,4]triazolo[1,5-c]pyrimidine (Compound 385)

The subject compound (45%) was obtained as a pale brown oily matter from 8-formyl-2-(2-furyl)-5-methylthio [1,2,4]triazolo[1,5-c]pyrimidine, N-methylcyclohexylamine and 1-phenylpiperazine in a manner similar to that in Example 251.
APCIMS m/z: $[M + H]^+$ 472

Example 385

8-[4-(2-Fluorophenyl)piperazin-1-ylmethyl]-2-(2-furyl)-5-(N-methylcyclohexylamino)[1,2,4]triazolo[1,5-c]pyrimidine (Compound 386)

The subject compound (47%) was obtained as a pale brown oily matter from 8-formyl-2-(2-furyl)-5-methylthio [1,2,4]triazolo[1,5-c]pyrimidine, N-methylcyclohexylamine and 1-(2-fluorophenyl)piperazine in a manner similar to that in Example 251.
APCIMS m/z: $[M + H]^+$ 490

Example 386

2-(2-Furyl)-5-(N-methylcyclohexylamino)-8-(4-phenylpiperidinomethyl) [1,2,4]triazolo[1,5-c]pyrimidine (Compound 387)

The subject compound (34%) was obtained as a pale brown oily matter from 8-formyl-2-(2-furyl)-5-methylthio [1,2,4]triazolo[1,5-c]pyrimidine, N-methylcyclohexylamine and 4-phenylpiperidine in a manner similar to that in Example 251.
APCIMS m/z: $[M + H]^+$ 471

Example 387

2-(2-Furyl)-5-(perhydroazepin-1-yl)-8-(1,2,3,4-tetrahydroisoquinolin-2-ylmethyl)[1,2,4]triazolo[1,5-c]pyrimidine (Compound 388)

The subject compound (30%) was obtained as a pale brown oily matter from 8-formyl-2-(2-furyl)-5-methylthio [1,2,4]triazolo[1,5-c]pyrimidine, hexamethyleneimine and 1,2,3,4-tetrahydroisoquinoline in a manner similar to that in Example 251.
APCIMS m/z: $[M + H]^+$ 429

Example 388

8-(6,7-Dimethoxy-1,2,3,4-tetrahydroisoquinolin-2-ylmethyl)-2-(2-furyl)-5-(perhydroazepin-1-yl)[1,2,4]triazolo[1,5-c] pyrimidine (Compound 389)

The subject compound (34%) was obtained as a pale brown oily matter from 8-formyl-2-(2-furyl)-5-methylthio [1,2,4]triazolo[1,5-c]pyrimidine, hexamethyleneimine and 6,7-dimethoxy-1,2,3,4-tetrahydroisoquinoline in a manner similar to that in Example 251.
APCIMS m/z: $[M + H]^+$ 489

Example 389

2-(2-Furyl)-8-(7-methoxy-1,2,3,4-tetrahydroisoquinolin-2-ylmethyl)-5-(perhydroazepin-1-yl)[1,2,4]triazolo[1,5-c] pyrimidine (Compound 390)

The subject compound (31%) was obtained as a pale brown oily matter from 8-formyl-2-(2-furyl)-5-methylthio[1,2,4]triazolo[1,5-c]pyrimidine, hexamethyleneimine and 7-methoxy-1,2,3,4-tetrahydroisoquinoline in a manner similar to that in Example 251.
APCIMS m/z: $[M + H]^+$ 459

Example 390

2-(2-Furyl)-5-(perhydroazepin-1-yl)-8-(4-phenyl-1,2,3,6-tetrahydropyridin-1-ylmethyl)[1,2,4]triazolo[1,5-c]pyrimidine (Compound 391)

The subject compound (33%) was obtained as a pale brown oily matter from 8-formyl-2-(2-furyl)-5-methylthio [1,2,4]triazolo[1,5-c]pyrimidine, hexamethyleneimine and 4-phenyl-1,2,3,6-tetrahydropyridine in a manner similar to that in Example 251.
APCIMS m/z: $[M + H]^+$ 455

Example 391

8-[4-(4-Fluorophenyl)-1,2,3,6-tetrahydropyridin-1-ylmethyl]-2-(2-furyl)-5-(perhydroazepin-1-yl)[1,2,4]triazolo[1, 5-c] pyrimidine (Compound 392)

The subject compound (31%) was obtained as a pale brown oily matter from 8-formyl-2-(2-furyl)-5-methylthio [1,2,4]triazolo[1,5-c]pyrimidine, hexamethyleneimine and 4-(4-fluorophenyl)-1,2,3,6-tetrahydropyridine in a manner similar to that in Example 251.
APCIMS m/z: $[M + H]^+$ 473

Example 392

2-(2-Furyl)-5-(perhydroazepin-1-yl)-8-(4-phenylpiperazin-1-ylmethyl)[1,2,4]triazolo[1,5-c]pyrimidine (Compound 393)

The subject compound (34%) was obtained as a pale brown oily matter from 8-formyl-2-(2-furyl)-5-methylthio [1,2,4]triazolo[1,5-c]pyrimidine, hexamethyleneimine and 1-phenylpiperazine in a manner similar to that in Example 251.
APCIMS m/z: $[M + H]^+$ 458

Example 393

8-[4-(2-Fluorophenyl)piperazin-1-ylmethyl]-2-(2-furyl)-5-(perhydroazepin-1-yl)[1,2,4]triazolo[1,5-c]pyrimidine (Compound 394)

The subject compound (34%) was obtained as a pale brown oily matter from 8-formyl-2-(2-furyl)-5-methylthio [1,2,4]triazolo[1,5-c]pyrimidine, hexamethyleneimine and 1-(2-fluorophenyl)piperazine in a manner similar to that in Example 251.
APCIMS m/z: $[M + H]^+$ 476

Example 394

2-(2-Furyl)-5-(perhydroazepin-1-yl)-8-(4-phenylpiperidinomethyl)[1,2,4]triazolo[1,5-c]pyrimidine (Compound 395)

The subject compound (35%) was obtained as a pale brown oily matter from 8-formyl-2-(2-furyl)-5-methylthio [1,2,4]triazolo[1,5-c]pyrimidine, hexamethyleneimine and 4-phenylpiperidine in a manner similar to that in Example 251.
APCIMS m/z: $[M + H]^+$ 457

Example 395

2-(2-Furyl)-5-(2-methoxyethylamino)-8-(7-methoxy-1,2,3,4-tetrahydroisoquinolin-2-ylmethyl)[1,2,4]triazolo[1,5-c] pyrimidine (Compound 396)

The subject compound (52%) was obtained as a pale brown oily matter from 8-formyl-2-(2-furyl)-5-methylthio [1,2,4]triazolo[1,5-c]pyrimidine, 2-methoxyethylamine and 7-methoxy-1,2,3,4-tetrahydroisoquinoline in a manner similar to that in Example 315.
APCIMS m/z: $[M + H]^+$ 435

Example 396

2-(2-Furyl)-5-(2-methoxyethylamino)-8-(4-phenyl-1,2,3,6-tetrahydropyridin-1-ylmethyl)[1,2,4]triazolo[1,5-c]pyrimidine (Compound 397)

The subject compound (43%) was obtained as a pale brown oily matter from 8-formyl-2-(2-furyl)-5-methylthio [1,2,4]triazolo[1,5-c]pyrimidine, 2-methoxyethylamine and 4-phenyl-1,2,3,6-tetrahydropyridine in a manner similar to that in Example 315.
APCIMS m/z: $[M + H]^+$ 431

Example 397

8-[4-(4-Fluorophenyl)-1,2,3,6-tetrahydropyridin-1-ylmethyl]-2-(2-furyl)-5-(2-methoxyethylamino)[1,2,4]triazolo[1,5-c] pyrimidine (Compound 398)

The subject compound (40%) was obtained as a pale brown oily matter from 8-formyl-2-(2-furyl)-5-methylthio [1,2,4]triazolo[1,5-c]pyrimidine, 2-methoxyethylamine and 4-(4-fluorophenyl)-1,2,3,6-tetrahydropyridine in a manner similar to that in Example 315.
APCIMS m/z: $[M + H]^+$ 449

Example 398

2-(2-Furyl)-5-(2-methoxyethylamino)-8-(4-phenylpiperazin-1-ylmethyl)[1,2,4]triazolo[1,5-c]pyrimidine (Compound 399)

The subject compound (35%) was obtained as a pale brown oily matter from 8-formyl-2-(2-furyl)-5-methylthio [1,2,4]triazolo[1,5-c]pyrimidine, 2-methoxyethylamine and 1-phenylpiperazine in a manner similar to that in Example 315.
APCIMS m/z: $[M + H]^+$ 434

Example 399

8-[4-(2-Fluorophenyl)piperazin-1-ylmethyl]-2-(2-furyl)-5-(2-methoxyethylamino)[1,2,4]triazolo[1,5-c]pyrimidine (Compound 400)

The subject compound (46%) was obtained as a pale brown oily matter from 8-formyl-2-(2-furyl)-5-methylthio [1,2,4]triazolo[1,5-c]pyrimidine, 2-methoxyethylamine and 1-(2-fluorophenyl)piperazine in a manner similar to that in Example 315.
APCIMS m/z: $[M + H]^+$ 452

Example 400

2-(2-Furyl)-5-(2-methoxyethylamino)-8-(4-phenylpiperidinomethyl)[1,2,4]triazolo[1,5-c]pyrimidine (Compound 401)

The subject compound (51%) was obtained as a pale brown oily matter from 8-formyl-2-(2-furyl)-5-methylthio [1,2,4]triazolo[1,5-c]pyrimidine, 2-methoxyethylamine and 4-phenylpiperidine in a manner similar to that in Example 315.
APCIMS m/z: $[M + H]^+$ 433

Example 401

2-(2-Furyl)-5-[2-(1-pyrrolidinyl)ethylamino]-8-(1,2,3,4-tetrahydroisoquinolin-2-ylmethyl)[1,2,4]triazolo[1,5-c]pyrimidine (Compound 402)

The subject compound (33%) was obtained as a pale brown oily matter from 8-formyl-2-(2-furyl)-5-methylthio[1,2,4]triazolo[1,5-c]pyrimidine, N-(2-aminoethyl)pyrrolidine and 1,2,3,4-tetrahydroisoquinoline in a manner similar to that in Example 315.
APCIMS m/ z : $[M + H]^+$ 444

Example 402

8-(6,7-Dimethoxy-1,2,3,4-tetrahydroisoquinolin-2-ylmethyl)-2-(2-furyl)-5-[2-(1-pyrrolidinyl) ethylamino] [1,2,4]triazolo [1,5-c]pyrimidine (Compound 403)

The subject compound (41%) was obtained as a pale brown oily matter from 8-formyl-2-(2-furyl)-5-methylthio [1,2,4]triazolo[1,5-c]pyrimidine, N- (2-aminoethyl)pyrrolidine and 6,7-dimethoxy-1,2,3,4-tetrahydroisoquinoline in a manner similar to that in Example 315.
APCIMS m/z: $[M + H]^+$ 504

Example 403

2-(2-Furyl)-8-(7-methoxy-1,2,3,4-tetrahydroisoquinolin-2-ylmethyl)-5-[2-(1-pyrrolidinyl)ethylamino][1,2,4]triazolo [1,5-c]pyrimidine (Compound 404)

The subject compound (34%) was obtained as a pale brown oily matter from 8-formyl-2-(2-furyl)-5-methylthio [1,2,4]triazolo[1,5-c]pyrimidine, N- (2-aminoethyl)pyrrolidine and 7-methoxy-1,2,3,4-tetrahydroisoquinoline in a manner similar to that in Example 315.
APCIMS m/z: $[M + H]^+$ 474

Example 404

2-(2-Furyl)-8-(4-phenyl-1,2,3,6-tetrahydropyridin-1-ylmethyl)-5-[2-(1-pyrrolidinyl)ethylamino][1,2,4]triazolo[1,5-c] pyrimidine (Compound 405)

The subject compound (33%) was obtained as a pale brown oily matter from 8-formyl-2-(2-furyl)-5-methylthio [1,2,4]triazolo[1,5-c]pyrimidine, N-(2-aminoethyl)pyrrolidine and 4-phenyl-1,2,3,6-tetrahydropyridine in a manner similar to that in Example 315.
$^1H$ NMR ($CDCl_3$, $\delta$, ppm): 1.73-1.92 (m, 4H) , 2.52-2.70 (m, 6H), 2.78-2.92 (m, 4H), 3.28-3.35 (m, 2H), 3.70-3.80 (m, 2H), 3.95 (s, 2H), 6.05-6.10 (m, 1H), 6.57 (dd, J = 1.9, 3.5 Hz, 1H), 7.13-7.42 (m, 6H), 7.62 (dd, J = 0.8, 1.9 Hz, 1H), 7.94 (s, 1H)

Example 405

8-[4-(4-Fluorophenyl)-1,2,3,6-tetrahydropyridin-1-ylmethyl]-2-(2-furyl)-5-[2-(1-pyrrolidinyl)ethylamino][1,2,4]triazolo [1,5-c]pyrimidine (Compound 406)

The subject compound (36%) was obtained as a pale brown oily matter from 8-formyl-2-(2-furyl)-5-methylthio [1,2,4]triazolo[1,5-c]pyrimidine, N-(2-aminoethyl)pyrrolidine and 4-(4-fluorophenyl)-1,2,3,6-tetrahydropyridine in a manner similar to that in Example 315.
$^1H$ NMR ($CDCl_3$, $\delta$, ppm): 1.73-1.90 (m, 4H) , 2.50-2.63 (m, 2H), 2.60-2.72 (m, 4H), 2.75-2.90 (m, 4H), 3.25-3.36 (m, 2H), 3.70-3.82 (m, 2H), 3.95 (s, 2H), 5.97-6.03 (m, 1H), 6.57 (dd, J = 1.9, 3.5 Hz, 1H), 6.93-7.02 (m, 1H), 7.22-7.36 (m, 2H), 7.58-7.63 (m, 1H), 7.93 (s, 1H)

Example 406

2-(2-Furyl)-8-(4-phenylpiperazin-1-ylmethyl)-5-[2-(1-pyrrolidinyl)ethylamino][1,2,4]triazolo[1,5-c]pyrimidine (Compound 407)

The subject compound (39%) was obtained as a pale brown oily matter from 8-formyl-2-(2-furyl)-5-methylthio [1,2,4]triazolo[1,5-c]pyrimidine, N-(2-aminoethyl)pyrrolidine and 1-phenylpiperazine in a manner similar to that in Example 315.
APCIMS m/z: $[M + H]^+$ 473

Example 407

8-[4-(2-Fluorophenyl)piperazin-1-ylmethyl]-2-(2-furyl)-5-[2-(1-pyrrolidinyl)ethylamino][1,2,4]triazolo[1,5-c]pyrimidine (Compound 408)

The subject compound (43%) was obtained as a pale brown oily matter from 8-formyl-2-(2-furyl)-5-methylthio [1,2,4]triazolo[1,5-c]pyrimidine, N-(2-aminoethyl)pyrrolidine and 1-(2-fluorophenyl)piperazine in a manner similar to that in Example 315.
APCIMS m/z: $[M + H]^+$ 491

Example 408

2-(2-Furyl)-8-(4-phenylpiperidinomethyl)-5-[2-(1-pyrrolidinyl)ethylamino)[1,2,4]triazolo[1,5-c]pyrimidine (Compound 409)

The subject compound (34%) was obtained as a pale brown oily matter from 8-formyl-2-(2-furyl)-5-methylthio [1,2,4]triazolo[1,5-c]pyrimidine, N-(2-aminoethyl)pyrrolidine and 4-phenylpiperidine in a manner similar to that in Example 315.
$^1$H NMR ($CDCl_3$, δ, ppm): 1.70-1.93 (m, 8H), 2.18-2.33 (m, 2H), 2.41-2.50 (m, 1H), 2.58-2.70 (m, 4H), 2.82 (t, J = 6.2 Hz, 2H), 3.10-3.20 (m, 2H), 3.70-3.81 (m, 2H), 3.88 (s, 2H), 6.57 (dd, J = 1.9, 3.5 Hz, 1H), 7.10-7.40 (m, 6H), 7.62 (dd, J = 0.8, 1.9 Hz, 1H), 7.94 (s, 1H)

Example 409

5-(2-Ethoxyethylamino)-2-(2-furyl)-8-(1,2,3,4-tetrahydroisoquinolin-2-ylmethyl)[1,2,4]triazolo[1,5-c]pyrimidine (Compound 410)

The subject compound (59%) was obtained as a pale brown oily matter from 8-formyl-2-(2-furyl)-5-methylthio [1,2,4]triazolo[1,5-c]pyrimidine, 2-ethoxyethylamine and 1,2,3,4-tetrahydroisoquinoline in a manner similar to that in Example 315.
APCIMS m/z: $[M + H]^+$ 419

Example 410

8-(6,7-Dimethoxy-1,2,3,4-tetrahydroisoquinolin-2-ylmethyl)-5-(2-ethoxyethylamino)-2-(2-furyl)[1,2,4]triazolo[1,5-c] pyrimidine (Compound 411)

The subject compound (51%) was obtained as a pale brown oily matter from 8-formyl-2-(2-furyl)-5-methylthio [1,2,4]triazolo[1,5-c]pyrimidine, 2-ethoxyethylamine and 6,7-dimethoxy-1,2,3,4-tetrahydroisoquinoline in a manner similar to that in Example 315.
APCIMS m/z: $[M + H]^+$ 479

Example 411

5-(2-Ethoxyethylamino)-2-(2-furyl)-8-(7-methoxy-1,2,3,4-tetrahydroisoquinolin-2-ylmethyl)[1,2,4]triazolo[1,5-c] pyrimidine (Compound 412)

The subject compound (58%) was obtained as a pale brown oily matter from 8-formyl-2-(2-furyl)-5-methylthio [1,2,4]triazolo[1,5-c]pyrimidine, 2-ethoxyethylamine and 7-methoxy-1,2,3,4-tetrahydroisoquinoline in a manner similar to that in Example 315.
APCIMS m/z: $[M + H]^+$ 449

Example 412

5-(2-Ethoxyethylamino)-2-(2-furyl)-8-(4-phenyl-1,2,3,6-tetrahydropyridin-1-ylmethyl)[1,2,4]triazolo[1,5-c]pyrimidine (Compound 413)

The subject compound (51%) was obtained as a pale brown oily matter from 8-formyl-2-(2-furyl)-5-methylthio[1,2,4]triazolo[1,5-c]pyrimidine, 2-ethoxyethylamine and 4-phenyl-1,2,3,6-tetrahydropyridine in a manner similar to that in Example 315.
APCIMS m/z: $[M + H]^+$ 445

Example 413

5-(2-Ethoxyethylamino)-8-[4-(4-fluorophenyl)-1,2,3,6-tetrahydropyridin-1-ylmethyl]-2-(2-furyl)[1,2,4]triazolo[1,5-c]pyrimidine (Compound 414)

The subject compound (57%) was obtained as a pale brown oily matter from 8-formyl-2-(2-furyl)-5-methylthio[1,2,4]triazolo[1,5-c]pyrimidine, 2-ethoxyethylamine and 4-(4-fluorophenyl)-1,2,3,6-tetrahydropyridine in a manner similar to that in Example 315.
APCIMS m/z: $[M + H]^+$ 463

Example 414

5-(2-Ethoxyethylamino)-2-(2-furyl)-8-(4-phenylpiperazin-1-ylmethyl)[1,2,4]triazolo[1,5-c]pyrimidine (Compound 415)

The subject compound (57%) was obtained as a pale brown oily matter from 8-formyl-2-(2-furyl)-5-methylthio[1,2,4]triazolo[1,5-c]pyrimidine, 2-ethoxyethylamine and 1-phenylpiperazine in a manner similar to that in Example 315.
APCIMS m/z: $[M + H]^+$ 448

Example 415

5-(2-Ethoxyethylamino)-8-[4-(2-fluorophenyl)piperazin-1-ylmethyl]-2-(2-furyl)[1,2,4]triazolo[1,5-c]pyrimidine (Compound 416)

The subject compound (49%) was obtained as a pale brown oily matter from 8-formyl-2-(2-furyl)-5-methylthio[1,2,4]triazolo[1,5-c]pyrimidine, 2-ethoxyethylamine and 1-(2-fluorophenyl)piperazine in a manner similar to that in Example 315.
APCIMS m/z: $[M + H]^+$ 466

Example 416

5-(2-Ethoxyethylamino)-2-(2-furyl)-8-(4-phenylpiperidinomethyl) [1,2,4]triazolo[1,5-c]pyrimidine (Compound 417)

The subject compound (53%) was obtained as a pale brown oily matter from 8-formyl-2-(2-furyl)-5-methylthio[1,2,4]triazolo[1,5-c]pyrimidine, 2-ethoxyethylamine and 4-phenylpiperidine in a manner similar to that in Example 315.
APCIMS m/z: $[M + H]^+$ 447

Example 417

2-(2-Furyl)-8-(1,2,3,4-tetrahydroisoquinolin-2-ylmethyl)-5-(2-tetrahydrofurfurylamino)[1,2,4]triazolo[1,5-c]pyrimidine (Compound 418)

The subject compound (59%) was obtained as a pale brown oily matter from 8-formyl-2-(2-furyl)-5-methylthio[1,2,4]triazolo[1,5-c]pyrimidine, 2-tetrahydrofurfurylamine and 1,2,3,4-tetrahydroisoquinoline in a manner similar to that in Example 315.
APCIMS m/z: $[M + H]^+$ 431

Example 418

8-(6,7-Dimethoxy-1,2,3,4-tetrahydroisoquinolin-2-ylmethyl)-2-(2-furyl)-5-(2-tetrahydrofurfurylamino)[1,2,4]triazolo[1,5-c]pyrimidine (Compound 419)

The subject compound (52%) was obtained as a pale brown oily matter from 8-formyl-2-(2-furyl)-5-methylthio[1,2,4]triazolo[1,5-c]pyrimidine, 2-tetrahydrofurfurylamine and 6,7-dimethoxy-1,2,3,4-tetrahydroisoquinoline in a manner similar to that in Example 315.

APCIMS m/z: $[M + H]^+$ 491

Example 419

2-(2-Furyl)-8-(7-methoxy-1,2,3,4-tetrahydroisoquinolin-2-ylmethyl)-5-(2-tetrahydrofurfurylamino)[1,2,4]triazolo[1,5-c] pyrimidine (Compound 420)

The subject compound (53%) was obtained as a pale brown oily matter from 8-formyl-2-(2-furyl)-5-methylthio [1,2,4]triazolo[1,5-c]pyrimidine, 2-tetrahydrofurfurylamine and 7-methoxy-1,2,3,4-tetrahydroisoquinoline in a manner similar to that in Example 315.
APCIMS m/z: $[M + H]^+$ 461

Example 420

2-(2-Furyl)-8-(4-phenyl-1,2,3,6-tetrahydropyridin-1-ylmethyl)-5-(2-tetrahydrofurfurylamino)[1,2,4]triazolo[1,5-c] pyrimidine (Compound 421)

The subject compound (44%) was obtained as a pale brown oily matter from 8-formyl-2-(2-furyl)-5-methylthio [1,2,4]triazolo[1,5-c]pyrimidine, 2-tetrahydrofurfurylamine and 4-phenyl-1,2,3,6-tetrahydropyridine in a manner similar to that in Example 315.
APCIMS m/z: $[M + H]^+$ 457

Example 421

8-[4-(4-Fluorophenyl)-1,2,3,6-tetrahydropyridin-1-ylmethyl]-2-(2-furyl)-5-(2-tetrahydrofurfurylamino)[1,2,4]triazolo [1,5-c]pyrimidine (Compound 422)

The subject compound (52%) was obtained as a pale brown oily matter from 8-formyl-2-(2-furyl)-5-methylthio [1,2,4]triazolo[1,5-c]pyrimidine, 2-tetrahydrofurfurylamine and 4-(4-fluorophenyl)-1,2,3,6-tetrahydropyridine in a manner similar to that in Example 315.
APCIMS m/z: $[M + H]^+$ 475

Example 422

2-(2-Furyl)-8-(4-phenylpiperazin-1-ylmethyl)-5-(2-tetrahydrofurfurylamino) [1,2,4]triazolo[1,5-c]pyrimidine (Compound 423)

The subject compound (61%) was obtained as a pale brown oily matter from 8-formyl-2-(2-furyl)-5-methylthio [1,2,4]triazolo[1,5-c]pyrimidine, 2-tetrahydrofurfurylamine and 1-phenylpiperazine in a manner similar to that in Example 315.
APCIMS m/z: $[M + H]^+$ 460

Example 423

8-[4-(2-Fluorophenyl)piperazin-1-ylmethyl]-2-(2-furyl)-5-(2-tetrahydrofurfurylamino)[1,2,4]triazolo[1,5-c]pyrimidine (Compound 424)

The subject compound (55%) was obtained as a pale brown oily matter from 8-formyl-2-(2-furyl)-5-methylthio [1,2,4]triazolo[1,5-c]pyrimidine, 2-tetrahydrofurfurylamine and 1-(2-fluorophenyl)piperazine in a manner similar to that in Example 315.
APCIMS m/z: $[M + H]^+$ 478

Example 424

2-(2-Furyl)-8-(4-phenylpiperidinomethyl)-5-(2-tetrahydrofurfurylamino)[1,2,4]triazolo[1,5-c]pyrimidine (Compound 425)

The subject compound (55%) was obtained as a pale brown oily matter from 8-formyl-2-(2-furyl)-5-methylthio[1,2,4]triazolo[1,5-c]pyrimidine, 2-tetrahydrofurfurylamine and 4-phenylpiperidine in a manner similar to that in Example 315.
APCIMS m/z: $[M + H]^+$ 459

Example 425

5-Cyclohexylmethylamino-2-(2-furyl)-8-(1,2,3,4-tetrahydroisoquinolin-2-ylmethyl)[1,2,4]triazolo[1,5-c]pyrimidine (Compound 426)

The subject compound (28%) was obtained as a pale brown oily matter from 8-formyl-2-(2-furyl)-5-methylthio [1,2,4]triazolo[1,5-c]pyrimidine, cyclohexanemethylamine and 1,2,3,4-tetrahydroisoquinoline in a manner similar to that in Example 315.
APCIMS m/z: $[M + H]^+$ 443

Example 426

5-Cyclohexylmethylamino-8-(6,7-dimethoxy-1,2,3,4-tetrahydroisoquinolin-2-ylmethyl)-2-(2-furyl) [1,2,4]triazolo[1,5-c] pyrimidine (Compound 427)

The subject compound (28%) was obtained as a pale brown oily matter from 8-formyl-2-(2-furyl)-5-methylthio [1,2,4]triazolo[1,5-c]pyrimidine, cyclohexanemethylamine and 6,7-dimethoxy-1,2,3,4-tetrahydroisoquinoline in a manner similar to that in Example 315.
APCIMS m/z: $[M + H]^+$ 503

Example 427

5-Cyclohexylmethylamino-2-(2-furyl)-8-(7-methoxy-1,2,3,4-tetrahydroisoquinolin-2-ylmethyl)[1,2,4]triazolo[1,5-c] pyrimidine (Compound 428)

The subject compound (31%) was obtained as a pale brown oily matter from 8-formyl-2-(2-furyl)-5-methylthio [1,2,4]triazolo[1,5-c]pyrimidine, cyclohexanemethylamine and 7-methoxy-1,2,3,4-tetrahydroisoquinoline in a manner similar to that in Example 315.
APCIMS m/z: $[M + H]^+$ 473

Example 428

5-Cyclohexylmethylamino-2-(2-furyl)-8-(4-phenyl-1,2,3,6-tetrahydropyridin-1-ylmethyl)[1,2,4]triazolo[1,5-c]pyrimidine (Compound 429)

The subject compound (25%) was obtained as a pale brown oily matter from 8-formyl-2-(2-furyl)-5-methylthio [1,2,4]triazolo[1,5-c]pyrimidine, cyclohexanemethylamine and 4-phenyl-1,2,3,6-tetrahydropyridine in a manner similar to that in Example 315.
APCIMS m/z: $[M + H]^+$ 469

Example 429

5-Cyclohexylmethylamino-8-[4-(4-fluorophenyl)-1,2,3,6-tetrahydropyridin-1-ylmethyl]-2-(2-furyl)[1,2,4]triazolo[1,5-c] pyrimidine (Compound 430)

The subject compound (32%) was obtained as a pale brown oily matter from 8-formyl-2-(2-furyl)-5-methylthio [1,2,4]triazolo[1,5-c]pyrimidine, cyclohexanemethylamine and 4-(4-fluorophenyl)-1,2,3,6-tetrahydropyridine in a manner similar to that in Example 315.
APCIMS m/z: $[M + H]^+$ 487

Example 430

5-Cyclohexylmethylamino-2-(2-furyl)-8-(4-phenylpiperazin-1-ylmethyl)[1,2,4]triazolo[1,5-c]pyrimidine (Compound 431)

The subject compound (28%) was obtained as a pale brown oily matter from 8-formyl-2-(2-furyl)-5-methylthio [1,2,4]triazolo[1,5-c]pyrimidine, cyclohexanemethylamine and 1-phenylpiperazine in a manner similar to that in Example 315.
APCIMS m/z: $[M + H]^+$ 472

Example 431

5-Cyclohexylmethylamino-8-[4-(2-fluorophenyl)piperazin-1-ylmethyl]-2-(2-furyl)[1,2,4]triazolo[1,5-c]pyrimidine (Compound 432)

The subject compound (36%) was obtained as a pale brown oily matter from 8-formyl-2-(2-furyl)-5-methylthio [1,2,4]triazolo[1,5-c]pyrimidine, cyclohexanemethylamine and 1-(2-fluorophenyl)piperazine in a manner similar to that in Example 315.
APCIMS m/z: $[M + H]^+$ 490

Example 432

5-Cyclohexylmethylamino-2-(2-furyl)-8-(4-phenylpiperidinomethyl) [1,2,4]triazolo[1,5-c]pyrimidine (Compound 433)

The subject compound (25%) was obtained as a pale brown oily matter from 8-formyl-2- (2-furyl) -5-methylthio [1,2,4]triazolo[1,5-c]pyrimidine, cyclohexanemethylamine and 4-phenylpiperidine in a manner similar to that in Example 315.
APCIMS m/z: $[M + H]^+$ 471

Example 433

2-(2-Furyl)-5-(2-morpholinoethylamino)-8-(1,2,3,4-tetrahydroisoquinolin-2-ylmethyl)[1,2,4]triazolo[1,5-c]pyrimidine (Compound 434)

The subject compound (29%) was obtained as a pale brown oily matter from 8-formyl-2-(2-furyl)-5-methylthio [1,2,4]triazolo[1,5-c]pyrimidine, N-(2-aminoethyl)morpholine and 1,2,3,4-tetrahydroisoquinoline in a manner similar to that in Example 315.
APCIMS m/z: $[M + H]^+$ 460

Example 434

8-(6,7-Dimethoxy-1,2,3,4-tetrahydroisoquinolin-2-ylmethyl)-2-(2-furyl)-5-(2-morpholinoethylamino)[1,2,4]triazolo [1,5-c]pyrimidine (Compound 435)

The subject compound (23%) was obtained as a pale brown oily matter from 8-formyl-2-(2-furyl)-5-methylthio [1,2,4]triazolo[1,5-c]pyrimidine, N-(2-aminoethyl)morpholine and 6,7-dimethoxy-1,2,3,4-tetrahydroisoquinoline in a manner similar to that in Example 315.
APCIMS m/z: $[M + H]^+$ 520

Example 435

2-(2-Furyl)-8-(7-methoxy-1,2,3,4-tetrahydroisoquinolin-2-ylmethyl)-5-(2-morpholinoethylamino)[1,2,4]triazolo[1,5-c] pyrimidine (Compound 436)

The subject compound (24%) was obtained as a pale brown oily matter from 8-formyl-2-(2-furyl)-5-methylthio [1,2,4]triazolo[1,5-c]pyrimidine, N- (2-aminoethyl)morpholine and 7-methoxy-1,2,3,4-tetrahydroisoquinoline in a manner similar to that in Example 315.
APCIMS m/z: $[M + H]^+$ 490

Example 436

2-(2-Furyl)-5-(2-morpholinoethylamino)-8-(4-phenyl-1,2,3,6-tetrahydropyridin-1-ylmethyl)[1,2,4]triazolo[1,5-c] pyrimidine (Compound 437)

The subject compound (18%) was obtained as a pale brown oily matter from 8-formyl-2-(2-furyl)-5-methylthio [1,2,4]triazolo[1,5-c]pyrimidine, N-(2-aminoethyl)morpholine and 4-phenyl-1,2,3,6-tetrahydropyridine in a manner similar to that in Example 315.
APCIMS m/z: $[M + H]^+$ 486

Example 437

8-[4-(4-Fluorophenyl)-1,2,3,6-tetrahydropyridin-1-ylmethyl]-2-(2-furyl)-5-(2-morpholinoethylamino)[1,2,4]triazolo [1,5-c]pyrimidine (Compound 438)

The subject compound (17%) was obtained as a pale brown oily matter from 8-formyl-2-(2-furyl)-5-methylthio [1,2,4]triazolo[1,5-c]pyrimidine, N-(2-aminoethyl)morpholine and 4-(4-fluorophenyl)-1,2,3,6-tetrahydropyridine in a manner similar to that in Example 315.
APCIMS m/z: $[M + H]^+$ 504

Example 438

2- (2-Furyl) -5- (2-morpholinoethylamino) -8- (4-phenylpiperazin-1-ylmethyl)[1,2,4]triazolo[1,5-c]pyrimidine (Compound 439)

The subject compound (19%) was obtained as a pale brown oily matter from 8-formyl-2-(2-furyl)-5-methylthio [1,2,4]triazolo[1,5-c]pyrimidine, N- (2-aminoethyl)morpholine and 1-phenylpiperazine in a manner similar to that in Example 315.
APCIMS m/z: $[M + H]^+$ 489

Example 439

8-[4-(2-Fluorophenyl)piperazin-1-ylmethyl]-2-(2-furyl)-5-(2-morpholinoethylamino)[1,2,4]triazolo[1,5-c]pyrimidine (Compound 440)

The subject compound (18%) was obtained as a pale brown oily matter from 8-formyl-2-(2-furyl)-5-methylthio [1,2,4]triazolo[1,5-c]pyrimidine, N- (2-aminoethyl)morpholine and 1-(2-fluorophenyl)piperazine in a manner similar to that in Example 315.
APCIMS m/z: $[M + H]^+$ 507

Example 440

2-(2-Furyl)-5-(2-morpholinoethylamino)-8-(4-phenylpiperidinomethyl)[1,2,4]triazolo[1,5-c]pyrimidine (Compound 441)

The subject compound (25%) was obtained as a pale brown oily matter from 8-formyl-2-(2-furyl)-5-methylthio [1,2,4]triazolo[1,5-c]pyrimidine, N-(2-aminoethyl)morpholine and 4-phenylpiperidine in a manner similar to that in Example 315.
APCIMS m/z: $[M + H]^+$ 488

Example 441

8-(7-Fluoro-1,2,3,4-tetrahydroisoquinolin-2-ylmethyl)-2-(2-furyl)-5-propylamino[1,2,4]triazolo[1,5-c]pyrimidine (Compound 442)

The subject compound (43%) was obtained as white crystals from 8-formyl-2-(2-furyl)-5-methylthio[1,2,4]triazolo[1,5-c]pyrimidine, propylamine and 7-fluoro-1,2,3,4-tetrahydroisoquinoline in a manner similar to that in Example 315.
Melting point: 158.0-159.0°C

$^{1}H$ NMR ($CDCl_3$, δ, ppm): 1.05 (t, J = 7.6 Hz, 3H), 1.77 (tq, J = 7.3, 7.6 Hz, 2H), 2.88 (s, 4H), 3.61 (dt, J = 5.9, 7.3 Hz, 2H), 3.75 (s, 2H), 3.98 (s, 2H), 6.18 (t, J = 5.9 Hz, 1H), 6.58 (dd, J = 1.6, 3.2 Hz, 1H), 6.70 (dd, J = 3.0, 9.5 Hz, 1H), 6.81 (dt, J = 3.0, 8.4 Hz, 1H), 7.03 (dd, J = 8.4, 9.5 Hz, 1H), 7.24 (dd, J = 0.5, 3.2 Hz, 1H), 7.62 (dd, J = 0.5, 1.6 Hz, 1H), 7.95 (s, 1H)

Example 442

8-(6,7-Dimethoxy-1,2,3,4-tetrahydroisoquinolin-2-ylmethyl)-5-ethylamino-2-(2-furyl)[1,2,4]triazolo[1,5-c]pyrimidine (Compound 443)

The subject compound (quantitative) was obtained as white crystals from 8-formyl-2-(2-furyl)-5-methylthio [1,2,4]triazolo[1,5-c]pyrimidine, ethylamine and 6,7-dimethoxy-1,2,3,4-tetrahydroisoquinoline in a manner similar to that in Example 315.
Melting point: 114-115°C
$^{1}H$ NMR ($CDCl_3$, δ, ppm) : 1.38 (t, J = 7.3 Hz, 3H) , 2.80-2.92 (m, 4H), 3.64-3.76 (m, 2H), 3.70 (s, 2H), 3.81 (s, 3H), 3.83 (s, 3H), 3.98 (s, 2H), 6.13 (t, J = 5.4 Hz, 1H), 6.50 (s, 1H), 6.58 (dd, J = 1.6, 3.5 Hz, 1H), 6.59 (s, 1H), 7.24 (dd, J = 0.8, 3.5 Hz, 1H), 7.62 (dd, J = 0.8, 1.6 Hz, 1H), 7.98 (s, 1H)

Example 443

8-(6,7-Dimethoxy-1,2,3,4-tetrahydroisoquinolin-2-ylmethyl) -2- (2-furyl) -5-methylamino[1,2,4]triazolo[1,5-c]pyrimidine (Compound 444)

The subject compound (90%) was obtained as white crystals from 8-formyl-2-(2-furyl)-5-methylthio[1,2,4]triazolo[1,5-c]pyrimidine, methylamine and 6,7-dimethoxy-1,2,3,4-tetrahydroisoquinoline in a manner similar to that in Example 315.
Melting point: 174-175°C
$^{1}H$ NMR ($CDCl_3$, δ, ppm): 2.79-2.91 (m, 4H), 3.20-3.27 (m, 3H), 3.70 (s, 2H), 3.81 (s, 3H), 3.84 (s, 3H), 3.99 (s, 2H), 6.10-6.20 (m, 1H), 6.50 (s, 1H), 6.58 (dd, J = 1.9, 3.5 Hz, 1H), 6.59 (s, 1H), 7.23 (d, J = 3.5 Hz, 1H), 7.62 (d, J = 1.9 Hz, 1H), 8.00 (s, 1H)

Example 444

5-Butylamino-8-(6,7-dimethoxy-1,2,3,4-tetrahydroisoquinolin-2-ylmethyl)-2-(2-furyl) [1,2,4]triazolo[1,5-c]pyrimidine (Compound 445)

The subject compound (84%) was obtained as white crystals from 8-formyl-2-(2-furyl)-5-methylthio[1,2,4]triazolo[1,5-c]gyrimidine, butylamine and 6,7-dimethoxy-1,2,3,4-tetrahydroisoquinoline in a manner similar to that in Example 315.
Melting point: 168-170°C
$^{1}H$ NMR ($CDCl_3$, δ, ppm): 0.99 (t, J = 7.3 Hz, 3H), 1.40-1.56 (m, 2H), 1.66-1.88 (m, 2H), 2.80-2.92 (m, 4H), 3.60 -3.72 (m, 2H), 3.70 (s, 2H), 3.81 (s, 3H), 3.84 (s, 3H), 3.98 (s, 2H), 6.10-6.18 (m, 1H), 6.50 (s, 1H), 6.58 (dd, J = 1.9, 3.2 Hz, 1H), 6.59 (s, 1H), 7.24 (d, J = 3.2 Hz, 1H), 7.62 (d, J = 1.9 Hz, 1H), 7.97 (s, 1H)

Example 445

8-(6,7-Dimethoxy-1,2,3,4-tetrahydroisoquinolin-2-ylmethyl)-2-(2-furyl)-5-(N-methylpropylamino)[1,2,4]triazolo[1,5-c] pyrimidine (Compound 446)

The subject compound (96%) was obtained as white crystals from 8-formyl-2-(2-furyl)-5-methylthio[1,2,4]triazolo[1,5-c] pyrimidine, N-methylpropylamine and 6,7-dimethoxy-1,2,3,4-tetrahydroisoquinoline in a manner similar to that in Example 251.
Melting point: 130-132°C
$^{1}H$ NMR ($CDCl_3$, δ, ppm): 0.98 (t, J = 7.5 Hz, 3H) , 1.70-1.90 (m, 2H), 2.78-2.92 (m, 4H), 3.43 (s, 3H), 3.70 (s, 2H), 3.82 (s, 3H), 3.84 (s, 3H), 3.93-4.03 (m, 2H), 4.00 (s, 2H), 6.51 (s, 1H), 6.56 (dd, J = 1.7, 3.3 Hz, 1H), 6.59 (s, 1H), 7.18 (dd, J = 0.7, 3.3 Hz, 1H), 7.61 (dd, J = 0.7, 1.7 Hz, 1H), 7.95 (s, 1H)

Example 446

8-(6,7-Dimethoxy-1,2,3,4-tetrahydroisoquinolin-2-ylmethyl)-5-dimethylamino-2-(2-furyl)[1,2,4]triazolo[1,5-c]pyrimidine (Compound 447)

The subject compound (quantitative) was obtained as white crystals from 8-formyl-2-(2-furyl)-5-methylthio[1,2,4]triazolo[1,5-c]pyrimidine, dimethylamine and 6,7-dimethoxy-1,2,3,4-tetrahydroisoquinoline in a manner similar to that in Example 251.
Melting point: 170-172°C
$^1$H NMR ($CDCl_3$, δ, ppm) : 2.80-2.93 (m, 4H) , 3.49 (s, 6H), 3.71 (s, 2H), 3.82 (s, 3H), 3.84 (s, 3H), 4.01 (s, 2H), 6.50 (s, 1H), 6.57 (dd, J = 1.8, 3.5 Hz, 1H), 6.60 (s, 1H), 7.21 (dd, J = 0.8, 3.3 Hz, 1H), 7.62 (dd, J = 0.8, 1.8 Hz, 1H), 7.98 (s, 1H)

Example 447

8-(6,7-Dimethoxy-1,2,3,4-tetrahydroisoquinolin-2-ylmethyl)-2-(2-furyl)-5-piperidino[1,2,4]triazolo[1,5-c]pyrimidine (Compound 448)

The subject compound (41%) was obtained as white crystals from 8-formyl-2-(2-furyl)-5-methylthio[1,2,4]triazolo[1,5-c]pyrimidine, piperidine and 6,7-dimethoxy-1,2,3,4-tetrahydroisoquinoline in a manner similar to that in Example 251.
Melting point: 200-202°C
$^1$H NMR ($CDCl_3$, δ, ppm): 1.68-1.85 (m, 6H), 2.79-2.92 (m, 4H), 3.71 (s, 2H), 3.82 (s, 3H), 3.84 (s, 3H), 3.95-4.06 (m, 4H), 4.02 (s, 2H), 6.51 (s, 1H), 6.57 (dd, J = 1.8, 3.5 Hz, 1H), 6.60 (s, 1H), 7.22 (dd, J = 0.7, 3.5 Hz, 1H), 7.62 (dd, J = 0.7, 1.8 Hz, 1H), 8.00 (s, 1H)

Example 448

8-(6,7-Dimethoxy-1,2,3,4-tetrahydroisoquinolin-2-ylmethyl)-2-(2-furyl)-5-(perhydroazocin-1-yl)[1,2,4]triazolo[1,5-c]pyrimidine (Compound 449)

The subject compound (59%) was obtained as white crystals from 8-formyl-2- (2-furyl) -5-methylthio[1,2,4]triazolo[1,5-c]pyrimidine, heptamethyleneimine and 6,7-dimethoxy-1,2,3,4-tetrahydroisoquinoline in a manner similar to that in Example 251.
Melting point: 163-165°C
$^1$H NMR ($CDCl_3$, δ, ppm) : 1.50-1.61 (m, 6H) , 1.83-2.00 (m, 4H), 2.80-2.94 (m, 4H), 3.71 (s, 2H), 3.82 (s, 3H), 3.84 (s, 3H), 3.99 (s, 2H), 4.07-4.16 (m, 4H), 6.51 (s, 1H), 6.55 (dd, J = 1.8, 3.1 Hz, 1H), 6.59 (s, 1H), 7.16 (dd, J = 0.8, 3.1 Hz, 1H), 7.61 (dd, J = 0.8, 1.8 Hz, 1H), 7.93 (s, 1H)

Example 449

8-(6,7-Dimethoxy-1,2,3,4-tetrahydroisoquinolin-2-ylmethyl)-5-(N-ethylmethylamino)-2-(2-furyl)[1,2,4]triazolo[1,5-c]pyrimidine (Compound 450)

The subject compound (quantitative) was obtained as white crystals from 8-formyl-2-(2-furyl)-5-methylthio[1,2,4]triazolo[1,5-c]pyrimidine, N-ethylmethylamine and 6,7-dimethoxy-1,2,3,4-tetrahydroisoquinoline in a manner similar to that in Example 251.
Melting point: 141-143°C
$^1$H NMR ($CDCl_3$, δ, ppm) : 1.34 (t, J = 7. 0 Hz, 3H) , 2.80-2.93 (m, 4H), 3.42 (s, 3H), 3.71 (s, 2H), 3.81 (s, 3H), 3.84 (s, 3H), 4.00 (s, 2H), 4.03 (q, J = 7.0 Hz, 2H), 6.51 (s, 1H), 6.56 (dd, J = 1.7, 3.3 Hz, 1H), 6.59 (s, 1H), 7.19 (dd, J = 0.7, 3.3 Hz, 1H), 7.61 (dd, J = 0.7, 1.7 Hz, 1H), 7.96 (s, 1H)

Example 450

2-(2-Furyl)-5-methylthio-8-(4-phenylpiperazin-1-ylmethyl)[1,2,4]triazolo[1,5-c]pyrimidine (Compound 451)

The subject compound (52%) was obtained as white crystals from 8-formyl-2-(2-furyl)-5-methylthio[1,2,4]triazolo[1,5-c]pyrimidine obtained in Reference Example 47 and 1-phenylpiperazine in a manner similar to that in Example

1.
Melting point: 194.0-194.3°C
$^1$H NMR ($CDCl_3$, δ, ppm): 2.73-2.86 (m, 4H), 2.76 (s, 3H), 3.19-3.30 (m, 4H), 4.01 (s, 2H), 6.58 (dd, J = 1.8, 3.5 Hz, 1H), 6.82-6.97 (m, 3H), 7.20-7.35 (m, 2H), 7.30 (d, J = 3.5 Hz, 1H), 7.64 (d, J = 1.8 Hz, 1H), 8.22 (s, 1H)

Example 451

2-(2-Furyl)-5-methylthio-8-(1,2,3,4-tetrahydroisoquinolin-2-ylmethyl)[1,2,4]triazolo[1,5-c]pyrimidine (Compound 452)

The subject compound (37%) was obtained as white crystals from 8-formyl-2-(2-furyl)-5-methylthio[1,2,4]triazolo[1,5-c]pyrimidine obtained in Reference Example 47 and 1,2,3,4-tetrahydroisoquinoline in a manner similar to that in Example 1.
Melting point: 193.4-194.0°C
$^1$H NMR (DMSO-$d_6$, δ, ppm) : 2.74 (s, 3H), 2.76-2.88 (m, 4H), 3.70 (s, 2H), 3.98 (s, 2H), 6.74 (dd, J = 1.8, 3.6 Hz, 1H), 6.98-7.13 (m, 4H), 7.30 (d, J = 3.6 Hz, 1H) , 7.97 (d, J = 1.8 Hz, 1H), 8.26 (s, 1H)

Example 452

8-(6,7-Dimethoxy-1,2,3,4-tetrahydroisoquinolin-2-ylmethyl)-2-(2-furyl)-5-methylthio[1,2,4]triazolo[1,5-c]pyrimidine (Compound 453)

The subject compound (63%) was obtained as white crystals from 8-formyl-2-(2-furyl)-5-methylthio[1,2,4]triazolo[1,5-c]pyrimidine obtained in Reference Example 47 and 6,7-dimethoxy-1,2,3,4-tetrahydroisoquinoline in a manner similar to that in Example 1.
Melting point: 209.5-210.0°C
$^1$H NMR (DMSO-$d_6$, δ, ppm): 2.66-2.76 (m, 4H), 2.74 (s, 3H), 3.58 (s, 2H), 3.66 (s, 3H), 3.69 (s, 3H), 3.96 (s, 2H), 6.59 (s, 1H), 6.66 (s, 1H), 6.74 (dd, J = 1.8, 3.5 Hz, 1H), 7.30 (d, J = 3.5 Hz, 1H), 7.97 (d, J = 1.8 Hz, 1H), 8.25 (s, 1H)

Example 453

5-(3,4-Dimethoxybenzylamino)-8-(7,8-dimethoxy-1,2,4,5-tetrahydro-3H-benzo[d]azepin-3-ylmethyl)-2-(2-furyl)[1,2,4]triazolo[1,5-c]pyrimidine (Compound 454)

The subject compound (quantitative) was obtained as a pale brown oily matter from 5-(3,4-dimethoxybenzylamino)-8-formyl-2-(2-furyl)[1,2,4]triazolo[1,5-c]pyrimidine and 7,8-dimethoxy-1,2,4,5-tetrahydro-3H-benzo[d]azepine in a manner similar to that in Example 1.
$^1$H NMR ($CDCl_3$, δ, ppm) : 2.68-3.00 (m, 8H), 3.84 (s, 6H), 3.88 (s, 3H), 3.88 (s, 3H), 4.01 (s, 2H), 4.75 (d, J = 5.9 Hz, 2H), 6.40 (t, J = 5.9 Hz, 1H), 6.57 (dd, J = 1.8, 3.5 Hz, 1H), 6.63 (s, 2H), 6.85 (d, J = 7.9 Hz, 1H), 6.95 (s, 1H), 6.97 (d, J = 7.9 Hz, 1H), 7.21 (dd, J = 0.9, 3.5 Hz, 1H), 7.60 (dd, J = 0.9, 1.8 Hz, 1H), 7.99 (s, 1H)

Example 454

5-Amino-8-(7,8-dimethoxy-1,2,4,5-tetrahydro-3H-benzo[d]azepin-3-ylmethyl)-2-(2-furyl)[1,2,4]triazolo[1,5-c]pyrimidine (Compound 455)

In a manner similar to that in Example 2, the subject compound (66%) was obtained as white crystals from 5-(3,4-dimethoxybenzylamino)-8-(7,8-dimethoxy-1,2,4,5-tetrahydro-3H-benzo[d]azepin-3-ylmethyl)-2-(2-furyl)[1,2,4]triazolo[1,5-c]pyrimidine obtained in Example 453.
Melting point: 225-226°C
$^1$H NMR ($CDCl_3$, δ , ppm) : 2.71-2.82 (m, 4H) , 2.84-2.95 (m, 4H), 3.84 (s, 6H), 4.01 (s, 2H), 5.89 (s, 2H), 6.59 (dd, J = 1.8, 3.4 Hz, 1H), 6.63 (s, 2H), 7.22-7.26 (m, 1H), 7.62 -7.65 (m, 1H), 7.94 (s, 1H)

Example 455

5-(3,4-Dimethoxybenzylamino)-2-(2-furyl)-8-(1,2,4,5-tetrahydro-3H-benzo[d]azepin-3-ylmethyl)[1,2,4]triazolo[1,5-c]pyrimidine (Compound 456)

The subject compound (quantitative) was obtained as a pale brown oily matter from 5-(3,4-dimethoxybenzylamino)-8-formyl-2-(2-furyl)[1,2,4]triazolo[1,5-c]pyrimidine and 1,2,4,5-tetrahydro-3H-benzo[d]azepine in a manner similar to that in Example 1.
$^1$H NMR ($CDCl_3$, δ, ppm): 2.72-2.85 (m, 4H), 2.92-3.05 (m, 4H), 3.88 (s, 3H), 3.88 (s, 3H), 4.01 (s, 2H), 4.75 (d, J = 5.6 Hz, 2H), 6.42 (t, J = 5.6 Hz, 1H), 6.56 (dd, J = 1.7, 3.1 Hz, 1H), 6.85 (d, J = 7.9 Hz, 1H), 6.95 (s, 1H), 6.96 (d, J = 7.9 Hz, 1H), 7.03-7.14 (m, 4H), 7.21 (d, J = 3.1 Hz, 1H) , 7.59 (d, J = 1.7 Hz, 1H), 7.99 (s, 1H)

Example 456

5-Amino-2-(2-furyl)-8-(1,2,4,5-tetrahydro-3H-benzo[d]azepin-3-ylmethyl)[1,2,4]triazolo[1,5-c]pyrimidine (Compound 457)

In a manner similar to that in Example 2, the subject compound (71%) was obtained as white crystals from 5-(3,4-dimethoxybenzylamino) -2- (2-furyl) -8- (1,2,4,5-tetrahydro-3H-benzo[d]azepin-3-ylmethyl)[1,2,4]triazolo[1,5-c]pyrimidine obtained in Example 455.
Melting point: 183-184°C
$^1$H NMR ($CDCl_3$, δ, ppm): 2.70-2.83 (m, 4H), 2.92-3.03 (m, 4H), 4.01 (s, 2H), 5.82 (s, 2H), 6.59 (dd, J = 1.5, 3.3 Hz, 1H), 7.03-7.13 (m, 4H), 7.24 (dd, J = 0.7, 3.5 Hz, 1H), 7.63 (dd, J = 0.7, 1.5 Hz, 1H), 7.94 (s, 1H)

Example 457

5-(3,4-Dimethoxybenzylamino)-8-[3-(6,7-dimethoxy-1,2,3,4-tetrahydroisoquinolin-2-yl)piperidinomethyl]-2-(2-furyl)[1,2,4]triazolo[1,5-c]pyrimidine (Compound 458)

The subject compound (98%) was obtained as a pale brown oily matter from 5-(3,4-dimethoxybenzylamino)-8-formyl-2-(2-furyl)[1,2,4]triazolo[1,5-c]pyrimidine and 6,7-dimethoxy-2-(3-piperidinyl)-1,2,3,4-tetrahydroisoquinoline in a manner similar to that in Example 1.
$^1$H NMR ($CDCl_3$, δ, ppm): 1.22-1.40 (m, 1H), 1.55-1.85 (m, 2H), 1.95-2.20 (m, 3H), 2.67-2.90 (m, 5H), 2.92-3.03 (m, 1H), 3.20-3.30 (m, 1H), 3.65-3.77 (m, 2H), 3.81 (s, 3H), 3.82 (s, 3H), 3.88 (s, 3H), 3.88 (s, 3H), 3.88 (s, 2H), 4.76 (d, J = 5.7 Hz, 2H), 6.40 (t, J = 5.7 Hz, 1H), 6.49 (s, 1H), 6.56 (s, 1H), 6.57 (dd, J = 1.7, 3.3 Hz, 1H), 6.85 (d, J = 8.1 Hz, 1H), 6.96 (s, 1H), 6.96 (d, J = 8.1 Hz, 1H), 7.21 (d, J = 3.3 Hz, 1H), 7.59 (d, J = 1.7 Hz, 1H), 7.95 (s, 1H)

Example 458

5-Amino-8-[3-(6,7-dimethoxy-1,2,3,4-tetrahydroisoquinolin-2-yl)piperidinomethyl]-2-(2-furyl)[1,2,4]triazolo[1,5-c]pyrimidine (Compound 459)

In a manner similar to that in Example 2, the subject compound (74%) was obtained as white crystals from 5-(3,4-dimethoxybenzylamino)-8-[3-(6,7-dimethoxy-1,2,3,4-tetrahydroisoquinolin-2-yl)piperidinomethyl]-2-(2-furyl)[1,2,4]triazolo[1,5-c]pyrimidine obtained in Example 457.
Melting point: 143-145°C
$^1$H NMR ($CDCl_3$, δ, ppm): 1.22-1.41 (m, 1H), 1.58-1.85 (m, 2H), 1.93-2.22 (m, 3H), 2.65-2.90 (m, 5H), 2.91-3.02 (m, 1H), 3.16-3.30 (m, 1H), 3.72 (s, 2H), 3.81 (s, 3H), 3.82 (s, 3H), 3.88 (s, 2H), 5.85 (s, 2H), 6.49 (s, 1H), 6.55 (s, 1H), 6.58 (dd, J = 1.7, 3.3 Hz, 1H), 7.24 (d, J = 3.3 Hz, 1H), 7.63 (d, J = 1.7 Hz, 1H), 7.90 (s, 1H)

Example 459

5-(3,4-Dimethoxybenzylamino)-8-[4-(6,7-dimethoxy-1,2,3,4-tetrahydroisoquinolin-2-yl)piperidinomethyl]-2-(2-furyl)[1,2,4]triazolo[1,5-c]pyrimidine (Compound 460)

The subject compound (84%) was obtained as a pale brown oily matter from 5-(3,4-dimethoxybenzylamino)-2-(2-furyl)-8-(4-oxopiperidinomethyl)[1,2,4]triazolo[1,5-c]pyrimidine obtained in Reference Example 67 and 6,7-dimethoxy-1,2,3,4-tetrahydroisoquinoline in a manner similar to that in Example 1.
$^1$H NMR ($CDCl_3$, δ, ppm): 1.63-1.82 (m, 2H), 1.84-1.96 (m, 2H), 2.10-2.25 (m, 2H), 2.40-2.55 (m, 1H), 2.81 (s, 4H), 3.05-3.16 (m, 2H), 3.61 (s, 2H), 3.82 (s, 3H), 3.83 (s, 3H), 3.85 (s, 2H), 3.88 (s, 3H), 3.88 (s, 3H), 4.76 (d, J = 6.0 Hz, 2H), 6.42 (t, J = 6.0 Hz, 1H), 6.51 (s, 1H), 6.56 (dd, J = 1.6, 3.2 Hz, 1H), 6.58 (s, 1H), 6.85 (d, J = 7.8 Hz, 1H), 6.95 (s, 1H), 6.97 (d, J = 7.8 Hz, 1H), 7.21 (dd, J = 0.5, 3.2 Hz, 1H), 7.60 (dd, J = 0.5, 1.7 Hz, 1H), 7.95 (s, 1H)

Example 460

5-Amino-8-[4-(6,7-dimethoxy-1,2,3,4-tetrahydroisoquinolin-2-yl)piperidinomethyl]-2-(2-furyl)[1,2,4]triazolo[1,5-c]pyrimidine (Compound 461)

In a manner similar to that in Example 2, the subject compound (67%) was obtained as pale yellow crystals from 5-(3,4-dimethoxybenzylamino)-8-[4-(6,7-dimethoxy-1,2,3,4-tetrahydroisoquinolin-2-yl)piperidinomethyl]-2-(2-furyl)[1,2,4]triazolo[1,5-c]pyrimidine obtained in Example 459.
Melting point: 159-160°C
$^1$H NMR ($CDCl_3$, δ, ppm): 1.75-2.10 (m, 4H), 2.25-2.45 (m, 2H), 2.65-3.20 (m, 5H) , 3.14-3.30 (m, 2H), 3.83 (s, 3H), 3.84 (s, 3H), 3.89 (s, 2H), 3.95 (s, 2H), 6.10 (s, 2H), 6.52 (s, 1H), 6.55-6.64 (m, 1H), 6.59 (s, 1H), 7.25 (d, J = 3.3 Hz, 1H), 7.64 (s, 1H), 7.94 (s, 1H)

Example 461

5-(3,4-Dimethoxybenzylamino)-2-(2-furyl)-8-[4-(4-phenylpiperazin-1-yl)piperidinomethyl][1,2,4]triazolo[1,5-c]pyrimidine (Compound 462)

The subject compound (81%) was obtained as a pale brown oily matter from 5-(3,4-dimethoxybenzylamino)-2-(2-furyl)-8-(4-oxopiperidinomethyl)[1,2,4]triazolo[1,5-c]pyrimidine obtained in Reference Example 67 and 1-phenylpiperazine in a manner similar to that in Example 1.
$^1$H NMR ($CDCl_3$, δ, ppm): 1.55-1.75 (m, 2H), 1.79-1.92 (m, 2H), 2.07-2.23 (m, 2H), 2.24-2.40 (m, 1H), 2.72 (t, J = 5.1 Hz, 4H), 3.02-3.18 (m, 2H), 3.20 (t, J = 5.1 Hz, 4H), 3.83 (s, 2H), 3.88 (s, 3H), 3.88 (s, 3H), 4.76 (d, J = 5.7 Hz, 2H), 6.40 (t, J = 5.7 Hz, 1H), 6.56 (dd, J = 1.6, 3.2 Hz, 1H), 6.80-7.03 (m, 6H), 7.18-7.31 (m, 3H), 7.58-7.62 (m, 1H), 7.95 (s, 1H)

Example 462

5-Amino-2-(2-furyl)-8-[4-(4-phenylpiperazin-1-yl)piperidinomethyl][1,2,4]triazolo[1,5-c]pyrimidine (Compound 463)

In a manner similar to that in Example 2, the subject compound (48%) was obtained as white crystals from 5-(3,4-dimethoxybenzylamino)-2-(2-furyl)-8-[4-(4-phenylpiperazin-1-yl)piperidinomethyl][1,2,4]triazolo[1,5-c]pyrimidine obtained in Example 461.
Melting point: 213-214°C
$^1$H NMR ($CDCl_3$, δ, ppm): 1.58-1.79 (m, 2H), 1.80-1.95 (m, 2H), 2.11-2.30 (m, 2H), 2.31-2.45 (m, 1H), 2.76 (t, J = 4.8 Hz, 4H), 3.06-3.18 (m, 2H), 3.22 (t, J = 4.8 Hz, 4H), 3.88 (s, 2H), 5.94 (s, 2H), 6.54-6.64 (m, 1H), 6.86 (t, J = 7.1 Hz, 1H), 6.93 (d, J = 8.2 Hz, 2H), 7.22-7.30 (m, 3H), 7.63 (s, 1H), 7.91 (s, 1H)

Example 463

5-Amino-8-[3-(6,7-dimethoxy-1,2,3,4-tetrahydroisoquinolin-2-yl)pyrrolidinylmethyl]-2-(2-furyl)[1,2,4]triazolo[1,5-c]pyrimidine (Compound 464)

5-(3,4-Dimethoxybenzylamino)-8-[3-(6,7-dimethoxy-1,2,3,4-tetrahydroisoquinolin-2-yl)pyrrolidinylmethyl]-2-(2-furyl)[1,2,4]triazolo[1,5-c]pyrimidine was obtained as a pale brown oily matter from 5-(3,4-dimethoxybenzylamino)-8-formyl-2-(2-furyl)[1,2,4]triazolo[1,5-c]pyrimidine and 6,7-dimethoxy-2-(3-pyrrolidinyl)-1,2,3,4-tetrahydroisoquinoline in a manner similar to that in Example 1. The obtained compound was subsequently treated in a manner similar to that in Example 2 without purification to obtain the subject compound (quantitative) as white crystals.
APCIMS m/z: $[M + H]^+$ 476

Example 464

8-(6,7-Dimethoxy-1,2,3,4-tetrahydroisoquinolin-2-ylmethyl)-5-dipropylamino-2-(2-furyl)[1,2,4]triazolo[1,5-c]pyrimidine (Compound 465)

The subject compound (44%) was obtained as white crystals from 8-formyl-2-(2-furyl)-5-methylthio[1,2,4]triazolo[1,5-c]pyrimidine, dipropylamine and 6,7-dimethoxy-1,2,3,4-tetrahydroisoquinoline in a manner similar to that in Example 251.

Melting point: 95-96°C
$^1$H NMR ($CDCl_3$, δ, ppm): 0.98 (t, J = 7.3 Hz, 6H), 1.68-1.85 (m, 4H), 2.80-2.93 (m, 4H), 3.70 (s, 2H), 3.82 (s, 3H), 3.84 (s, 3H), 3.83-3.93 (m, 4H), 4.00 (s, 2H), 6.51 (s, 1H), 6.53-6.59 (m, 1H) , 6.59 (s, 1H), 7.15 (d, J = 3.3 Hz, 1H), 7.61 (s, 1H), 7.92 (s, 1H)

Example 465

8-(6,7-Dimethoxy-1,2,3,4-tetrahydroisoquinolin-2-ylmethyl)-2-(2-furyl)-5-propargylamino[1,2,4]triazolo[1,5-c]pyrimidine (Compound 466)

The subject compound (54%) was obtained as white crystals from 8-formyl-2-(2-furyl)-5-methylthio[1,2,4]triazolo[1,5-c]pyrimidine, propargylamine and 6,7-dimethoxy-1,2,3,4-tetrahydroisoquinoline in a manner similar to that in Example 251.
Melting point: 193-194°C
$^1$H NMR ($CDCl_3$, δ, ppm): 2.33 (t, J = 2.6 Hz, 1H), 2.78-2.94 (m, 4H), 3.70 (s, 2H), 3.82 (s, 3H), 3.84 (s, 3H), 4.00 (s, 2H), 4.46 (dd, J = 2.4, 5.7 Hz, 2H), 6.35 (t, J = 5.7 Hz, 1H), 6.50 (s, 1H), 6.56-6.65 (m, 1H), 6.60 (s, 1H), 7.23-7.26 (m, 1H), 7.60-7.67 (m, 1H), 8.03 (s, 1H)

Example 466

5-Allylamino-8-(6,7-dimethoxy-1,2,3,4-tetrahydroisoquinolin-2-ylmethyl)-2-(2-furyl)[1,2,4]triazolo[1,5-c]pyrimidine (Compound 467)

The subject compound (46%) was obtained as white crystals from 8-formyl-2-(2-furyl)-5-methylthio[1,2,4]triazolo[1,5-c]pyrimidine, allylamine and 6,7-dimethoxy-1,2,3,4-tetrahydroisoquinoline in a manner similar to that in Example 251.
Melting point: 170-171°C
$^1$H NMR ($CDCl_3$, δ, ppm) : 2.87 (s, 4H), 3.72 (s, 2H) , 3.81 (s, 3H), 3.84 (s, 3H), 4.01 (s, 2H), 4.30 (dd, J = 5.8, 5.8 Hz, 2H), 5.25 (d, J = 10.3 Hz, 1H), 5.35 (d, J = 16.1 Hz, 1H), 6.02 (ddt, J = 5.8, 10.3, 16.1 Hz, 1H), 6.26 (t, J = 5.8 Hz, 1H), 6.50 (s, 1H), 6.55-6.62 (m, 1H), 6.60 (s, 1H), 7.24 (d, J = 3.5 Hz, 1H), 7.63 (s, 1H), 8.00 (s, 1H)

Example 467

8-(6,7-Dimethoxy-1,2,3,4-tetrahydroisoquinolin-2-ylmethyl)-5-(N-ethylpropylamino)-2-(2-furyl)[1,2,4]triazolo[1,5-c]pyrimidine (Compound 468)

The subject compound (57%) was obtained as white crystals from 8-formyl-2-(2-furyl)-5-methylthio[1,2,4]triazolo[1,5-c]pyrimidine, N-ethylpropylamine and 6,7-dimethoxy-1,2,3,4-tetrahydroisoquinoline in a manner similar to that in Example 251.
Melting point: 80-81°C
$^1$H NMR ($CDCl_3$, δ, ppm): 0.99 (t, J = 7.3 Hz, 3H), 1.33 (t, J = 6.1 Hz, 3H), 1.71-1.90 (m, 2H), 2.87 (s, 4H), 3.71 (s, 2H), 3.81 (s, 3H), 3.84 (s, 3H), 3.83-4.05 (m, 6H), 6.51 (s, 1H), 6.56 (dd, J = 1.8, 3.5 Hz, 1H), 6.59 (s, 1H), 7.16 (d, J = 3.5 Hz, 1H), 7.61 (d, J = 1.8 Hz, 1H), 7.94 (s, 1H)

Example 468

2-(2-Furyl)-8-(4-phenylpiperazin-1-ylmethyl)-5-propionylamino[1,2,4]triazolo[1,5-c]pyrimidine (Compound 469)

5-Amino-2-(2-furyl)-8-(4-phenylpiperazin-1-ylmethyl)[1,2,4]triazolo[1,5-c]pyrimidine (105.8 mg, 0.28 mmol) obtained in Example 2 was dissolved in DMF (13.5 ml), and propionic anhydride (0.1 ml, 0.80 mmol), 4-dimethylaminopyridine (7.5 mg, 0.06 mmol) and triethylamine (0.56 ml, 4.1 mmol) were added thereto, followed by stirring at room temperature for 24 hours. After the completion of reaction, the solvent was distilled away from the reaction mixture under reduced pressure, and the resulting residue was purified by silica gel column chromatography (eluted with 1% methanol-chloroform) to obtain the subject compound (121.1 mg, quantitative) as white crystals.
Melting point: 155-156°C
$^1$H NMR ($CDCl_3$, δ, ppm) : 1.31 (t, J = 7.3 Hz, 3H), 2.77 (t, J = 4.7 Hz, 4H), 2.96 (q, J = 7.3 Hz, 2H), 3.23 (t, J = 4.7 Hz, 4H), 3.98 (s, 2H), 6.60 (dd, J = 1.6, 3.5 Hz, 1H), 6.85 (t, J = 7.1 Hz, 1H), 6.92 (d, J = 7.9 Hz, 2H), 7.22-7.31 (m, 3H), 7.65 (dd, J = 0.7, 1.6 Hz, 1H), 8.17 (s, 1H), 8.93 (s, 1H)

Example 469

5-Acetylamino-2-(2-furyl)-8-(4-phenylpiperazin-1-ylmethyl)[1,2,4]triazolo[1,5-c]pyrimidine (Compound 470)

The subject compound (69%) was obtained as white crystals from 5-amino-2-(2-furyl)-8-(4-phenylpiperazin-1-ylmethyl)[1,2,4]triazolo[1,5-c]pyrimidine obtained in Example 2 and acetic anhydride in a manner similar to that in Example 468.
Melting point: 163-164°C
$^1$H NMR ($CDCl_3$, δ, ppm): 2.65 (s, 3H) , 2.78 (t, J = 5.0 Hz, 4H), 3.24 (t, J = 5.0 Hz, 4H), 3.98 (s, 2H), 6.62 (dd, J = 1.7, 3.5 Hz, 1H), 6.86 (t, J = 7.3 Hz, 1H), 6.93 (d, J = 7.9 Hz, 2H), 7.20-7.35 (m, 3H), 7.66 (d, J = 1.7 Hz, 1H), 8.17 (s, 1H), 8.93 (s, 1H)

Example 470

5-(3,4-Dimethoxybenzylamino)-8-[2-(6,7-dimethoxy-1,2,3,4-tetrahydroisoquinolin-2-yl)ethyl]-2-(2-furyl)[1,2,4]triazolo[1,5-c]pyrimidine (Compound 471)

The subject compound (43%) was obtained as a pale brown oily matter from 5-(3,4-dimethoxybenzylamino)-8-formylmethyl-2-(2-furyl) [1,2,4]triazolo[1,5-c]pyrimidine obtained in Reference Example 89 and 6,7-dimethoxy-1,2,3,4-tetrahydroisoquinoline in a manner similar to that in Example 1.
$^1$H NMR ($CDCl_3$, δ, ppm): 2.84 (s, 4H) , 2.95 (t, J = 7.8 Hz, 2H), 3.14 (t, J = 7.8 Hz, 2H), 3.68 (s, 2H), 3.83 (s, 3H), 3.84 (s, 3H), 3.87 (s, 3H), 3.88 (s, 3H), 4.72 (d, J = 5.9 Hz, 2H), 6.34 (t, J = 5.9 Hz, 1H), 6.54 (s, 1H), 6.57 (dd, J = 1.9, 3.8 Hz, 1H), 6.59 (s, 1H), 6.84 (d, J = 8.1 Hz, 1H), 6.94 (s, 1H), 6.95 (d, J = 8.1 Hz, 1H), 7.22 (d, J = 3.8 Hz, 1H), 7.60 (d, J = 1.9 Hz, 1H), 7.83 (s, 1H)

Example 471

5-Amino-8-[2-(6,7-dimethoxy-1,2,3,4-tetrahydroisoquinolin-2-yl)ethyl]-2-(2-furyl)[1,2,4]triazolo[1,5-c]pyrimidine (Compound 472)

In a manner similar to that in Example 2, the subject compound (68%) was obtained as white crystals from 5-(3,4-dimethoxybenzylamino)-8-[2-(6,7-dimethoxy-1,2,3,4-tetrahydroisoquinolin-2-yl)ethyl]-2-(2-furyl)[1,2,4]triazolo[1,5-c]pyrimidine obtained in Example 470.
Melting point: 175-177°C
$^1$H NMR ($CDCl_3$, δ, ppm): 2.84 (s, 4H), 2.96 (t, J = 7.0 Hz, 2H), 3.15 (t, J = 7.0 Hz, 2H), 3.69 (s, 2H), 3.83 (s, 3H), 3.84 (s, 3H), 5.77 (s, 2H), 6.53 (s, 1H), 6.59 (s, 1H), 6.55-6.60 (m, 1H), 7.23-7.25 (m, 1H), 7.61-7.65 (m, 1H), 7.76 (s, 1H)

Example 472

5-(3,4-Dimethoxybenzylamino)-2-(2-furyl)-8-[2-(1,2,3,4-tetrahydroisoquinolin-2-yl)ethyl][1,2,4]triazolo[1,5-c]pyrimidine (Compound 473)

The subject compound (37%) was obtained as a pale brown oily matter from 5-(3,4-dimethoxybenzylamino)-8-formylmethyl-2-(2-furyl)[1,2,4]triazolo[1,5-c]pyrimidine obtained in Reference Example 89 and 1,2,3,4-tetrahydroisoquinoline in a manner similar to that in Example 1.
$^1$H NMR ($CDCl_3$, δ, ppm) : 2.82-3.02 (m, 6H), 3.15 (t, J = 7.6 Hz, 2H), 3.75 (s, 2H), 3.86 (s, 3H), 3.87 (s, 3H), 4.72 (d, J = 5.9 Hz, 2H), 6.34 (t, J = 5.9 Hz, 1H), 6.57 (dd, J = 2.2, 3.8 Hz, 1H), 6.84 (d, J = 7.6 Hz, 1H), 6.94 (s, 1H), 6.96 (d, J = 7.6 Hz, 1H), 6.98-7.15 (m, 4H), 7.22 (dd, J = 1.1, 3.8 Hz, 1H), 7.60 (dd, J = 1.1, 2.2 Hz, 1H), 7.83 (s, 1H)

Example 473

5-Amino-2-(2-furyl)-8-[2-(1,2,3,4-tetrahydroisoquinolin-2-yl)ethyl][1,2,4]triazolo[1,5-c]pyrimidine (Compound 474)

In a manner similar to that in Example 2, the subject compound (75%) was obtained as white crystals from 5-(3,4-dimethoxybenzylamino)-2-(2-furyl)-8-[2-(1,2,3,4-tetrahydroisoquinolin-2-yl)ethyl][1,2,4]triazolo[1,5-c]pyrimidine obtained in Example 472.
Melting point: 186-188°C
$^1$H NMR ($CDCl_3$, δ , ppm) : 2.83-3.05 (m, 6H) , 3.16 (t, J = 7.8 Hz, 2H), 3.77 (s, 2H), 5.85 (s, 2H), 6.59 (dd, J = 2.2, 3.8

Hz, 1H), 6.99-7.18 (m, 4H), 7.22-7.25 (m, 1H), 7.63 (d, J = 2.2 Hz, 1H), 7.76 (s, 1H)

Example 474

5-(3,4-Dimethoxybenzylamino)-2-(2-furyl)-8-[2-(4-phenylpiperazin-1-yl)ethyl][1,2,4]triazolo[1,5-c]pyrimidine (Compound 475)

The subject compound (58%) was obtained as a pale brown oily matter from 5-(3,4-dimethoxybenzylamino)-8-formylmethyl-2-(2-furyl)[1,2,4]triazolo[1,5-c]pyrimidine obtained in Reference Example 89 and 1-phenylpiperazine in a manner similar to that in Example 1.
$^1$H NMR ($CDCl_3$, δ, ppm): 2.73 (t, J = 5.1 Hz, 4H), 2.83 (t, J = 7.3 Hz, 2H), 3.08 (t, J = 7.3 Hz, 2H), 3.22 (t, J = 5.1 Hz, 4H), 3.88 (s, 3H), 3.88 (s, 3H), 4.73 (d, J = 5.7 Hz, 2H), 6.38 (t, J = 5.7 Hz, 1H), 6.57 (dd, J = 2.2, 3.5 Hz, 1H), 6.88-7.00 (m, 6H), 7.21 (dd, J = 0.8, 3.5 Hz, 1H), 7.22-7.30 (m, 2H), 7.60 (dd, J = 0.8, 2.2 Hz, 1H), 7.82 (s, 1H)

Example 475

5-Amino-2-(2-furyl)-8-[2-(4-phenylpiperazin-1-yl) ethyl] [1,2,4]triazolo[1,5-c]pyrimidine (Compound 476)

In a manner similar to that in Example 2, the subject compound (67%) was obtained as white crystals from 5-(3,4-dimethoxybenzylamino)-2-(2-furyl)-8-[2-(4-phenylpiperazin-1-yl)ethyl][1,2,4]triazolo[1,5-c]pyrimidine obtained in Example 474.
Melting point: 178-180°C
$^1$H NMR ($CDCl_3$, δ, ppm): 2.75 (t, J = 4.3 Hz, 4H), 2.85 (t, J = 7.3 Hz, 2H), 3.10 (t, J = 7.3 Hz, 2H), 3.23 (t, J = 4.3 Hz, 4H), 5.82 (s, 2H), 6.59 (dd, J = 1.9, 3.5 Hz, 1H), 6.85 (t, J = 7.0 Hz, 1H), 6.93 (d, J = 8.4 Hz, 2H), 7.20-7.32 (m, 3H), 7.63 (dd, J = 1.1, 1.9 Hz, 1H), 7.75 (s, 1H)

Example 476

5-(3,4-Dimethoxybenzylamino)-8-[3-(6,7-dimethoxy-1,2,3,4-tetrahydroisoquinolin-2-yl)propyl]-2-(2-furyl)[1,2,4] triazolo[1,5-c]pyrimidine (Compound 477)

The subject compound (64%) was obtained as a pale brown oily matter from 5-(3,4-dimethoxybenzylamino)-8-(2-formylethyl)-2-(2-furyl)[1,2,4]triazolo[1,5-c] pyrimidne obtained in Reference Example 95 and 6,7-dimethoxy-1,2,3,4-tetrahydroisoquinoline in a manner similar to that in Example 1.
$^1$H NMR ($CDCl_3$, δ, ppm): 2.00-2.15 (m, 2H), 2.61 (t, J = 7.8 Hz, 2H), 2.68-2.88 (m, 4H), 2.93 (t, J = 7.6 Hz, 2H), 3.60 (s, 2H), 3.82 (s, 3H), 3.84 (s, 3H), 4.73 (d, J = 5.7 Hz, 2H), 6.36 (t, J = 5.7 Hz, 1H), 6.51 (s, 1H), 6.57 (dd, J = 2.2, 3.8 Hz, 1H), 6.59 (s, 1H), 6.84 (d, J = 8.6 Hz, 1H), 6.95 (s, 1H), 6.97 (d, J = 8.6 Hz, 1H), 7.21 (d, J = 3.8 Hz, 1H), 7.60 (d, J = 2.2 Hz, 1H), 7.76 (s, 1H)

Example 477

5-Amino-8-[3-(6,7-dimethoxy-1,2,3,4-tetrahydroisoquinolin-2-yl)propyl]-2-(2-furyl) [1,2,4]triazolo[1,5-c]pyrimidine (Compound 478)

In a manner similar to that in Example 2, the subject compound (75%) was obtained as white crystals from 5-(3,4-dimethoxybenzylamino)-8-[3-(6,7-dimethoxy-1,2,3,4-tetrahydroisoquinolin-2-yl)propyl]-2-(2-furyl) [1,2,4]triazolo[1,5-c]pyrimidine obtained in Example 476.
Melting point (hydrochloride): 170-172°C
$^1$H NMR ($CDCl_3$, δ, ppm): 2.03-2.17 (m, 2H), 2.62 (t, J = 7.6 Hz, 2H), 2.72-2.88 (m, 4H), 2.94 (t, J = 7.6 Hz, 2H), 3.59 (s, 2H), 3.82 (s, 3H), 3.84 (s, 3H), 6.02 (s, 2H), 6.51 (s, 1H), 6.57-6.60 (m, 1H), 6.59 (s, 1H), 7.24 (dd, J = 0.8, 3.2 Hz, 1H), 7.63 (dd, J = 0.8, 1.9 Hz, 1H), 7.69 (s, 1H)

Example 478

5-(3,4-Dimethoxybenzylamino)-2-(2-furyl)-8-[3-(1,2,3,4-tetrahydroisoquinolin-2-yl)propyl][1,2,4]triazolo[1,5-c] pyrimidine (Compound 479)

The subject compound (85%) was obtained as a pale brown oily matter from 5-(3,4-dimethoxybenzylamino)-8-(2-formylethyl)-2-(2-furyl)[1,2,4]triazolo[1,5-c]pyrimidne obtained in Reference Example 95 and 1,2,3,4-tetrahydroisoquinoline in a manner similar to that in Example 1.
$^1$H NMR ($CDCl_3$, δ, ppm): 2.03-2.17 (m, 2H), 2.61 (t, J = 7.6 Hz, 2H), 2.75 (t, J = 6.2 Hz, 2H), 2.85-2.98 (m, 4H), 3.64 (s, 2H), 3.88 (s, 3H), 3.88 (s, 3H), 4.73 (d, J = 5.9 Hz, 2H), 6.36 (t, J = 5.9 Hz, 1H), 6.56 (dd, J =1.9, 3.5 Hz, 1H), 6.84 (d, J = 8.6 Hz, 1H), 6.95 (s, 1H), 6.97 (d, J = 8.6 Hz, 1H), 6.98-7.15 (m, 4H), 7.21 (dd, J = 0.8, 3.5 Hz, 1H), 7.59 (dd, J = 0.8, 1.9 Hz, 1H), 7.75 (s, 1H)

Example 479

5-Amino-2-(2-furyl)-8-[3-(1,2,3,4-tetrahydroisoquinolin-2-yl)propyl][1,2,4]triazolo[1,5-c]pyrimidine (Compound 480)

In a manner similar to that in Example 2, the subject compound (51%) was obtained as white crystals from 5-(3,4-dimethoxybenzylamino)-2-(2-furyl)-8-[3-(1,2,3,4-tetrahydroisoquinolin-2-yl)propyl][1,2,4]triazolo[1,5-c]pyrimidine obtained in Example 478.
Melting point (hydrochloride): 177-178°C
$^1$H NMR ($CDCl_3$, δ, ppm): 2.02-2.18 (m, 2H) , 2.61 (t, J = 7.8 Hz, 2H), 2.75 (t, J = 5.9 Hz, 2H), 2.87-3.00 (m, 4H), 3.65 (s, 2H), 5.87-6.00 (m, 2H), 6.59 (dd, J = 1.6, 3.2 Hz, 1H), 6.97-7.14 (m, 4H), 7.24 (dd, J =0.8, 3.2 Hz, 1H), 7.63 (dd, J = 0.8, 1.6 Hz, 1H), 7.69 (s, 1H)

Example 480

5-(3,4-Dimethoxybenzylamino)-2-(2-furyl)-8-[3-(4-phenylpiperazin-1-yl)propyl][1,2,4]triazolo[1,5-c]pyrimidine (Compound 481)

The subject compound (97%) was obtained as a pale brown oily matter from 5-(3,4-dimethoxybenzylamino)-8-(2-formylethyl)-2-(2-furyl)[1,2,4]triazolo[1,5-c]pyrimidne obtained in Reference Example 95 and 1-phenylpiperazine in a manner similar to that in Example 1.
$^1$H NMR ($CDCl_3$, δ, ppm): 1.95-2.08 (m, 2H) , 2.50 (t, J = 7.6 Hz, 2H), 2.62 (t, J = 4.9 Hz, 4H), 2.91 (t, J = 7.6 Hz, 2H), 3.20 (t, J = 4.9 Hz, 4H), 3.88 (s, 3H), 3.88 (s, 3H), 4.73 (d, J = 5.9 Hz, 2H), 6.37 (t, J = 5.9 Hz, 1H), 6.56 (dd, J = 1.6, 3.2 Hz, 1H), 6.79-7.00 (m, 6H), 7.18-7.30 (m, 3H), 7.60 (d, J = 1.6 Hz, 1H), 7.74 (s, 1H)

Example 481

5-Amino-2-(2-furyl)-8-[3-(4-phenylpiperazin-1-yl)propyl] [1,2,4]triazolo[1,5-c]pyrimidine (Compound 482)

In a manner similar to that in Example 2, the subject compound (56%) was obtained as white crystals from 5-(3,4-dimethoxybenzylamino)-2-(2-furyl)-8-[3-(4-phenylpiperazin-1-yl)propyl][1,2,4]triazolo[1,5-c]pyrimidine obtained in Example 480.
Melting point (hydrochloride): 264-266°C
$^1$H NMR ($CDCl_3$, δ, ppm): 1.95-2.10 (m, 2H), 2.50 (t, J = 7.6 Hz, 2H), 2.62 (t, J = 4.9 Hz, 4H), 2.92 (t, J = 7.6 Hz, 2H), 3.20 (t, J = 4.9 Hz, 4H), 5.92-6.05 (m, 2H), 6.59 (dd, J = 1.9, 3.5 Hz, 1H), 6.84 (t, J = 7.3 Hz, 1H), 6.92 (d, J = 7.8 Hz, 2H), 7.20-7.30 (m, 3H), 7.63 (dd, J = 0.8, 1.9 Hz, 1H), 7.68 (s, 1H)

Example 482

2-(2-Furyl)-8-(4-phenylpiperazin-1-ylmethyl)-6H-[1,2,4]triazolo[1,5-c]pyrimidin-5-one (Compound 483)

2-(2-Furyl)-5-methylthio-8-(4-phenylpiperazin-1-ylmethyl)[1,2,4]triazolo[1,5-c]pyrimidine (200 mg, 0.49 mmol) obtained in Example 450 was suspended in a mixed solvent of ethanol (3 ml) and water (0.1 ml). Lithium hydroxide monohydrate (205 mg, 4.90 mmol) was added thereto, and the mixture was refluxed for 3 hours. After cooling the reaction mixture to room temperature, a 10% aqueous hydrochloric acid solution was added thereto to adjust the pH to 4, followed by stirring at room temperature for one hour. The deposited crystals were recovered by filtration and washed with water, and the resulting crystals were dried under reduced pressure to obtain the subject compound (88.0 mg, 48%) as white crystals.
$^1$H NMR ($DMSO-d_6$, δ, ppm): 2.43-2.61 (m, 4H), 3.01-3.18 (m, 4H), 3.55 (s, 2H), 6.59-6.64 (m, 1H), 6.69-6.79 (m, 1H), 6.84-6.94 (m, 2H), 6.97-7.02 (m, 1H), 7.11-7.23 (m, 2H), 7.50 (s, 1H), 7.81 (s, 1H)

Example 483

2-(2-Furyl)-6-methyl-8-(4-phenylpiperazin-1-ylmethyl)-6H-[1,2,4]triazolo[1,5-c]pyrimidin-5-one (Compound 484)

2-(2-Furyl)-8-(4-phenylpiperazin-1-ylmethyl)-6H-[1,2,4]triazolo[1,5-c]pyrimidin-5-one (50 mg, 0.13 mmol) obtained in Example 482 was dissolved in THF (1.3 ml). Methanol (8.1 µl, 0.20 mmol), triphenylphosphine (52.5 mg, 0.20 mmol) and diethyl azodicarboxylate (36 µl, 0.20 mmol) were added successively thereto under ice-cooling, followed by stirring at room temperature for 2 hours. The solvent was distilled away under reduced pressure, and the resulting residue was purified by thin layer chromatography (chloroform/methanol/triethylamine = 10/1/0.5) to obtain the subject compound (12.9 mg, 56%) as white crystals.
$^1$H NMR (DMSO-$d_6$, δ, ppm): 2.58-2.70 (m, 4H), 3.07-3.20 (m, 4H), 3.60 (s, 3H), 3.63 (s, 2H), 6.67-6.80 (m, 2H), 6.88 -6.99 (m, 2H), 7.12-7.26 (m, 3H), 7.80 (s, 1H), 7.93 (d, J = 1.8 Hz, 1H)

Example 484

2-(2-Furyl)-6-(2-methoxyethyl)-8-(4-phenylpiperazin-1-ylmethyl)-6H-[1,2,4]triazolo[1,5-c]pyrimidin-5-one (Compound 485)

The subject compound (22%) was obtained from 2-(2-furyl)-8-(4-phenylpiperazin-1-ylmethyl)-6H-[1,2,4]triazolo[1,5-c]pyrimidin-5-one obtained in Example 482 and 2-methoxyethanol in a manner similar to that in Example 483.
$^1$H NMR (DMSO-$d_6$, δ , ppm) : 1.56-1.73 (m, 4H), 3.07-3.23 (m, 4H), 3.27 (s, 3H), 3.58-3.73 (m, 4H), 4.12-4.29 (m, 2H), 6.71 (dd, J = 1.7, 3.4 Hz, 1H), 6.65-6.82 (m, 1H), 6.85-6.99 (m, 2H), 7.11-7.27 (m, 3H), 7.74 (s, 1H), 7.93 (d, J = 1.7 Hz, 1H)

Example 485

6-(2-Fluoroethyl)-2-(2-furyl)-8-(4-phenylpiperazin-1-ylmethyl)-6H-[1,2,4]triazolo[1,5-c]pyrimidin-5-one (Compound 486)

The subject compound (36%) was obtained as white crystals from 2-(2-furyl)-8-(4-phenylpiperazin-1-ylmethyl)-6H-[1,2,4]triazolo[1,5-c]pyrimidin-5-one obtained in Example 482 and 2-fluoroethanol in a manner similar to that in Example 483.
Melting point: 175.5-175.8°C
$^1$H NMR ($CDCl_3$, δ, ppm) : 2.69-2.83 (m, 4H), 3.19-3.30 (m, 4H), 3.82 (s, 2H), 4.38 (dt, J = 4.5, 26.7 Hz, 2H), 4.80 (dt, J = 4.5, 46.8 Hz, 2H), 6.57 (dd, J = 1.6, 3.5 Hz, 1H), 6.81-6.96 (m, 3H), 7.21-7.33 (m, 3H), 7.45 (s, 1H) , 7.62 (d, J = 1.7 Hz, 1H)

Example 486

6-Cyclohexylmethyl-2-(2-furyl)-8-(4-phenylpiperazin-1-ylmethyl)-6H-[1,2,4]triazolo[1,5-c]pyrimidin-5-one (Compound 487)

The subject compound (15%) was obtained as white crystals from 2-(2-furyl)-8-(4-phenylpiperazin-1-ylmethyl)-6H-[1,2,4]triazolo[1,5-c]pyrimidin-5-one obtained in Example 482 and cyclohexylmethanol in a manner similar to that in Example 483.
$^1$H NMR (DMSO-$d_6$, δ, ppm): 0.92-1.34 (m, 5H), 1.54-1.95 (m, 6H), 1.56-1.78 (m, 4H), 3.08-3.25 (m, 4H), 3.66 (s, 2H), 3.87 (d, J = 7.4 Hz, 2H), 6.71 (dd, J = 1.8, 3.6 Hz, 1H), 6.65-6.82 (m, 1H), 6.87-7.02 (m, 2H), 7.13-7.28 (m, 3H), 7.75 (s, 1H), 7.93 (d, J = 1.8 Hz, 1H)

Example 487

6-Benzyl-2-(2-furyl)-8-(4-phenylpiperazin-1-ylmethyl)-6H-[1,2,4]triazolo[1,5-c]pyrimidin-5-one (Compound 488)

The subject compound (44%) was obtained as white crystals from 2-(2-furyl)-8-(4-phenylpiperazin-1-ylmethyl)-6H-[1,2,4]triazolo[1,5-c]pyrimidin-5-one obtained in Example 482 and benzyl alcohol in a manner similar to that in Example 483.
$^1$H NMR (DMSO-$d_6$, δ, ppm) : 2.56-2.70 (m, 4H), 3.06-3.20 (m, 4H), 3.66 (s, 2H), 5.26 (s, 2H), 6.66-6.81 (m, 2H), 6.87 -6.96 (m, 2H), 7.13-7.50 (m, 8H), 7.87-7.98 (m, 2H)

Example 488

2-(2-Furyl)-8-(4-phenylpiperazin-1-ylmethyl)-6-(3-phenylpropyl)-6H-[1,2,4]triazolo[1,5-c]pyrimidin-5-one (Compound 489)

The subject compound (28%) was obtained as white crystals from 2-(2-furyl)-8-(4-phenylpiperazin-1-ylmethyl)-6H-[1,2,4]triazolo[1,5-c]pyrimidin-5-one obtained in Example 482 and 3-phenylpropanol in a manner similar to that in Example 483.
$^1$H NMR ($CDCl_3$, δ, ppm): 2.12-2.27 (m, 2H), 2.67-2.80 (m, 6H), 3.17-3.29 (m, 4H), 3.78 (s, 2H), 4.03-4.15 (m, 2H), 6.54-6.59 (m, 1H), 6.82-6.98 (m, 3H), 7.12-7.77 (m, 10H)

Example 489

2-(2-Furyl)-4-(4-phenylpiperazin-1-ylmethyl)-6,7-dihydro-1,3,5a,8,8b-pentaaza-as-indacene (Compound 490)

2-(2-Furyl)-5-(2-hydroxyethylamino)-8-(4-phenylpiperazin-1-ylmethyl)[1,2,4]triazolo[1,5-c]pyrimidine (45 mg, 0.11 mmol) obtained in Example 179 was dissolved in dichloromethane (1.1 ml). Triethylamine (46.0 μl), potassium carbonate (45.6 mg, 0.33 mmol) and methanesulfonic acid chloride (11.0 μl) were added thereto under ice-cooling. After stirring the mixture at room temperature for 2 hours, the temperature was elevated, and the resulting mixture was refluxed for 20 hours. After cooling to room temperature, the reaction mixture was diluted with chloroform, washed with water and saturated saline and then dried over anhydrous magnesium sulfate. The solvent was distilled away under reduced pressure, and the resulting residue was purified by thin layer chromatography (chloroform/methanol = 15/1) to obtain the subject compound (26.5 mg, 66%) as white crystals.
$^1$H NMR (DMSO-$d_6$, δ, ppm): 2.57-2.70 (m, 4H) , 3.03-3.22 (m, 4H), 3.52 (s, 2H), 3.96 (t, J = 9.2 Hz, 2H), 4.21 (t, J = 9.2 Hz, 2H), 6.69 (dd, J = 1.8, 3.5 Hz, 1H), 6.72-6.80 (m, 1H), 6.86-6.99 (m, 2H), 7.13 (dd, J = 0.7, 3.5 Hz, 1H), 7.15-7.29 (m, 2H), 7.65 (s, 1H), 7.91 (dd, J = 0.7, 1.8 Hz, 1H)

Example 490

2-(2-Furyl)-4-(1,2,3,4-tetrahydroisoquinolin-2-ylmethyl)-6,7-dihydro-1,3,5a,8,8b-pentaaza-as-indacene (Compound 491)

In a manner similar to that in Example 489, the subject compound (16%) was obtained as white crystals from 2-(2-furyl)-5-(2-hydroxyethylamino)-8-(1,2,3,4-tetrahydroisoquinolin-2-ylmethyl) [1,2,4]triazolo[1,5-c]pyrimidine obtained in Example 180.
Melting point: 199°C
$^1$H NMR (DMSO-$d_6$, δ, ppm): 2.73-2.91 (m, 4H), 3.56-3.73 (m, 4H), 3.89-4.05 (m, 2H), 4.14-4.29 (m, 2H), 6.68 (dd, J = 1.6, 3.5 Hz, 1H), 6.95-7.16 (m, 5H), 7.67 (s, 1H), 7.89 (d, J = 1.7 Hz, 1H)

Example 491

2-(2-Furyl)-4-(4-phenylpiperazin-1-ylmethyl)-7,8-dihydro-6H-1,3,5a,9,9b-pentaazacyclopenta[a]naphthalene (Compound 492)

In a manner similar to that in Example 489, the subject compound (99%) was obtained as white crystals from 2-(2-furyl)-5-(3-hydroxypropylamino)-8-(4-phenylpiperazin-1-ylmethyl) [1,2,4]triazolo[1,5-c]pyrimidine obtained in Example 181.
$^1$H NMR (DMSO-$d_6$, δ, ppm) : 1.81-2.04 (m, 2H) , 2.53-2.73 (m, 4H), 3.04-3.22 (m, 4H), 3.40-3.62 (m, 4H), 3.87-4.10 (m, 2H), 6.67 (dd, J = 1.8, 3.5 Hz, 1H), 6.70-6.80 (m, 1H), 6.82-6.98 (m, 2H), 7.08 (d, J = 3.5 Hz, 1H), 7.11-7.25 (m, 2H), 7.28 (s, 1H), 7.88 (d, J = 1.8 Hz, 1H)

Example 492

2-(2-Furyl)-4-(6,7-dimethoxy-1,2,3,4-tetrahydroisoquinolin-2-ylmethyl)-7,8-dihydro-6H-1,3,5a,9,9b-pentaazacyclopenta[a]naphthalene (Compound 493)

In a manner similar to that in Example 489, the subject compound (5%) was obtained as white crystals from 8-(6,7-dimethoxy-1,2,3,4-tetrahydroisoquinolin-2-ylmethyl)-2-(2-furyl)-5-(3-hydroxypropylamino) [1,2,4]triazolo[1,5-c]pyrimidine obtained in Example 182.
$^1H$ NMR ($DMSO-d_6$, δ, ppm): 1.81-2.04 (m, 2H) , 2.72 (s, 2H), 2.72 (s, 2H), 3.47-3.63 (m, 4H), 3.62 (s, 2H), 3.69 (s, 3H), 3.70 (s, 3H), 3.90-4.13 (m, 2H), 6.62 (s, 1H), 6.68 (s, 1H), 6.69 (dd, J = 0.8, 1.6 Hz, 1H), 7.08 (d, J = 1.6 Hz, 1H), 7.31 (s, 1H), 7.88 (d, J = 0.8 Hz, 1H)

Example 493

Tablets

Tablets having the following composition are prepared according to a conventional method.

| | | |
|---|---|---|
| Formula | Compound 2 | 20 mg |
| | Lactose | 143.4 mg |
| | Potato starch | 30 mg |
| | Hydroxypropyl cellulose | 6 mg |
| | Magnesium stearate | 0.6 mg |

Example 494

Injections

Injections having the following composition are prepared according to a conventional method.

| | | |
|---|---|---|
| Formula | Compound 203 | 2 mg |
| | Purified soybean oil | 200 mg |
| | Purified egg yolk lecithin | 24 mg |
| | Glycerol for injection | 50 mg |
| | Distilled water for injection | 1.72 ml |

Industrial Applicability

The present invention provides condensed-ring-pyrimidine derivatives or pharmaceutically acceptable salts thereof which show $\alpha_2$-adrenoceptor antagonism, particularly $\alpha_{2c}$-adrenoceptor antagonism and are useful for treating and/or preventing various diseases induced by hyperactivity of $\alpha_{2c}$-adrenoceptor (for example, Parkinson's disease, L-DOPA-induced dyskinesia, tardive dyskinesia, depression, hypertension, diabetes, obesity and erectile dysfunction), $\alpha_{2c}$-adrenoceptor antagonists comprising a condensed-ring-pyrimidine derivative or a pharmaceutically acceptable salt thereof as an active ingredient, etc.

**Claims**

**1.** An $\alpha_{2c}$-adrenoceptor antagonist comprising, as an active ingredient, a condensed-ring-pyrimidine derivative represented by general formula (I):

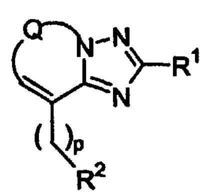

(I)

<wherein p represents an integer of 1 to 3;
$R^1$ represents a hydrogen atom, substituted or unsubstituted lower alkyl, substituted or unsubstituted cycloalkyl, substituted or unsubstituted aryl, substituted or unsubstituted aralkyl, a substituted or unsubstituted heterocyclic

group, or substituted or unsubstituted heterocycle-lower alkyl;
$R^2$ represents -N(-$R^3$)(-$R^4$) (wherein $R^3$ and $R^4$ are the same or different, and each represents a hydrogen atom, substituted or unsubstituted lower alkyl, substituted or unsubstituted cycloalkyl, substituted or unsubstituted aryl, substituted or unsubstituted aralkyl, a substituted or unsubstituted heterocyclic group, or substituted or unsubstituted heterocycle-lower alkyl, or $R^3$ and $R^4$ form a substituted or unsubstituted heterocyclic group together with the adjacent nitrogen atom) or general formula (A) :

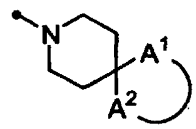

(A)

{wherein -$A^1$-$A^2$- represents -$Y^1$-C(=O)-$Y^2$-$CH_2$-$CH_2$- [wherein $Y^1$ and $Y^2$ are the same or different, and each represents an oxygen atom or -N(-$R^5$)- (wherein $R^5$ represents a hydrogen atom, substituted or unsubstituted lower alkyl, substituted or unsubstituted aryl, substituted or unsubstituted aralkyl, a substituted or unsubstituted heterocyclic group, or substituted or unsubstituted heterocycle-lower alkyl)] or -$Y^3$-$CH_2$-$Y^4$-C(=O)- (wherein $Y^3$ and $Y^4$ have the same meanings as the above-described $Y^1$ and $Y^2$, respectively)}; and
- Q- represents (a) -N=C(-$R^7$)- [wherein $R^7$ represents -O(-$R^8$) (wherein $R^8$ represents a hydrogen atom, substituted or unsubstituted lower alkyl, substituted or unsubstituted lower alkanoyl, substituted or unsubstituted aroyl, substituted or unsubstituted aryl, substituted or unsubstituted aralkyl, a substituted or unsubstituted heterocyclic group, or substituted or unsubstituted heterocycle-lower alkyl), -N(-$R^9$) (-$R^{10}$) (wherein $R^9$ and $R^{10}$ are the same or different, and each represents a hydrogen atom, substituted or unsubstituted lower alkyl,
substituted or unsubstituted cycloalkyl, substituted or unsubstituted lower alkenyl, substituted or unsubstituted lower alkynyl, substituted or unsubstituted aryl,
substituted or unsubstituted lower alkanoyl, substituted or unsubstituted aroyl, substituted or unsubstituted aralkyl, a substituted or unsubstituted heterocyclic group, or substituted or unsubstituted heterocycle-lower alkyl,
or $R^9$ and $R^{10}$ form a substituted or unsubstituted heterocyclic group together with the adjacent nitrogen atom) or -S(-$R^{11}$) (wherein $R^{11}$ represents a hydrogen atom, substituted or unsubstituted lower alkyl, substituted or unsubstituted aralkyl, or substituted or unsubstituted heterocycle-lower alkyl)],
(b) -N(-$R^{12}$)-C(=O)- (wherein $R^{12}$ represents a hydrogen atom, substituted or unsubstituted lower alkyl, substituted or unsubstituted aralkyl, or substituted or unsubstituted heterocycle-lower alkyl) or
(c) general formula (B):

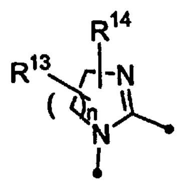

(B)

(wherein n represents an integer of 1 to 3; and $R^{13}$ and $R^{14}$ are the same or different, and each represents a hydrogen atom, substituted or unsubstituted lower alkyl, substituted or unsubstituted aryl, substituted or unsubstituted aralkyl, a substituted or unsubstituted heterocyclic group, or substituted or unsubstituted heterocycle-lower alkyl)> or a pharmaceutically acceptable salt thereof.

**2.** The $\alpha_{2c}$-adrenoceptor antagonist according to claim 1, wherein $R^2$ is -N(-$R^3$)(-$R^4$) in which $R^3$ and $R^4$ form a substituted or unsubstituted heterocyclic group together with the adjacent nitrogen atom.

**3.** The $\alpha_{2c}$-adrenoceptor antagonist according to claim 1, wherein $R^2$ is substituted or unsubstituted piperazinyl, substituted or unsubstituted piperidino, or substituted or unsubstituted tetrahydroisoquinolyl.

**4.** The $\alpha_{2c}$-adrenoceptor antagonist according to any one of claims 1 to 3, wherein -Q- is -N=C(-$R^7$)- in which $R^7$ is

$-N(-R^9)(-R^{10})$.

**5.** The $\alpha_{2c}$-adrenoceptor antagonist according to claim 4, wherein $R^9$ and $R^{10}$ each are a hydrogen atom.

**6.** The $\alpha_{2c}$-adrenoceptor antagonist according to any one of claims 1 to 3, wherein -Q- is $-N=C(-R^7)-$ in which $R^7$ is $-S(-R^{11})$.

**7.** The $\alpha_{2c}$-adrenoceptor antagonist according to any one of claims 1 to 3, wherein -Q- is $-N(-R^{12})-C(=O)-$.

**8.** The $\alpha_{2c}$-adrenoceptor antagonist according to any one of claims 1 to 3, wherein -Q- is general formula (B).

**9.** The $\alpha_{2c}$-adrenoceptor antagonist according to any one of claims 1 to 8, wherein $R^1$ is furyl.

**10.** An agent for preventing and/or treating dyskinesia comprising, as an active ingredient, a condensed-ring-pyrimidine derivative represented by general formula (I):

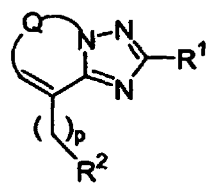

(I)

(wherein p, $R^1$, $R^2$ and -Q- each have the same meanings as defined above) or a pharmaceutically acceptable salt thereof.

**11.** The agent for preventing and/or treating dyskinesia according to claim 10, wherein $R^2$ is $-N(-R^3)(-R^4)$ in which $R^3$ and $R^4$ form a substituted or unsubstituted heterocyclic group together with the adjacent nitrogen atom.

**12.** The agent for preventing and/or treating dyskinesia according to claim 10, wherein $R^2$ is substituted or unsubstituted piperazinyl, substituted or unsubstituted piperidino, or substituted or unsubstituted tetrahydroisoquinolyl.

**13.** The agent for preventing and/or treating dyskinesia according to any one of claims 10 to 12, wherein -Q- is $-N=C(-R^7)-$ in which $R^7$ is $-N(-R^9)(-R^{10})$.

**14.** The agent for preventing and/or treating dyskinesia according to claim 13, wherein $R^9$ and $R^{10}$ each are a hydrogen atom.

**15.** The agent for preventing and/or treating dyskinesia according to any one of claims 10 to 12, wherein -Q- is $-N=C(-R^7)-$ in which $R^7$ is $-S(-R^{11})$.

**16.** The agent for preventing and/or treating dyskinesia according to any one of claims 10 to 12, wherein -Q- is $-N(-R^{12})-C(=O)-$.

**17.** The agent for preventing and/or treating dyskinesia according to any one of claims 10 to 12, wherein -Q- is general formula (B).

**18.** The agent for preventing and/or treating dyskinesia according to any one of claims 10 to 17, wherein $R^1$ is furyl.

**19.** The agent for preventing and/or treating dyskinesia according to any one of claims 10 to 18, wherein dyskinesia is L-DOPA-induced dyskinesia.

**20.** A condensed-ring-pyrimidine derivative represented by general formula (II) :

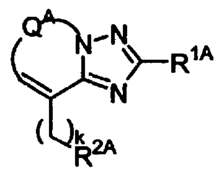

(II)

<wherein k represents an integer of 1 to 3;

$R^{1A}$ represents a hydrogen atom, substituted or unsubstituted lower alkyl, substituted or unsubstituted cycloalkyl, substituted or unsubstituted aryl, substituted or unsubstituted aralkyl, a substituted or unsubstituted heterocyclic group, or substituted or unsubstituted heterocycle-lower alkyl;

$R^{2A}$ represents $-N(-R^{3A})(-R^{4A})$ (wherein $R^{3A}$ and $R^{4A}$ are the same or different, and each represents a hydrogen atom, substituted or unsubstituted lower alkyl, substituted or unsubstituted cycloalkyl, substituted or unsubstituted aryl, substituted or unsubstituted aralkyl, a substituted or unsubstituted heterocyclic group, or substituted or unsubstituted heterocycle-lower alkyl, or $R^{3A}$ and $R^{4A}$ form a substituted or unsubstituted heterocyclic group together with the adjacent nitrogen atom) or general formula (C):

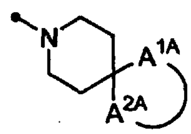

(C)

{wherein $-A^{1A}-A^{2A}-$ represents $-Y^{1A}-C(=O)-Y^{2A}-CH_2-CH_2-$ [wherein $Y^{1A}$ and $Y^{2A}$ are the same or different, and each represents an oxygen atom or $-N(-R^{5A})-$ (wherein $R^{5A}$ represents a hydrogen atom, substituted or unsubstituted lower alkyl, substituted or unsubstituted aryl, substituted or unsubstituted aralkyl, a substituted or unsubstituted heterocyclic group, or substituted or unsubstituted heterocycle-lower alkyl)] or $-Y^{3A}-CH_2-Y^{4A}-C(=O)-$ (wherein $Y^{3A}$ and $Y^{4A}$ have the same meanings as the above-described $Y^{1A}$ and $Y^{2A}$, respectively)}; and

$-Q^{A}-$ represents (d) $-N=C(-R^{7A})-$ [wherein $R^{7A}$ represents $-O(-R^{8A})$ (wherein $R^{8A}$ represents a hydrogen atom, substituted or unsubstituted lower alkyl, substituted or unsubstituted lower alkanoyl, substituted or unsubstituted aroyl, substituted or unsubstituted aryl, substituted or unsubstituted aralkyl, a substituted or unsubstituted heterocyclic group, or substituted or unsubstituted heterocycle-lower alkyl), $-N(-R^{9A})(-R^{10A})$ (wherein $R^{9A}$ and $R^{10A}$ are the same or different, and each represents a hydrogen atom, substituted or unsubstituted lower alkyl, substituted or unsubstituted cycloalkyl, substituted or unsubstituted lower alkenyl, substituted or unsubstituted lower alkynyl, substituted or unsubstituted aryl,
substituted or unsubstituted lower alkanoyl, substituted or unsubstituted aroyl, substituted or unsubstituted aralkyl, a substituted or unsubstituted heterocyclic group, or substituted or unsubstituted heterocycle-lower alkyl,
or $R^{9A}$ and $R^{10A}$ form a substituted or unsubstituted heterocyclic group together with the adjacent nitrogen atom) or $-S(-R^{11A})$ (wherein $R^{11A}$ represents a hydrogen atom, substituted or unsubstituted lower alkyl, substituted or unsubstituted aralkyl, or substituted or unsubstituted heterocycle-lower alkyl)],
(e) $-N(-R^{12A})-C(=O)-$ (wherein $R^{12A}$ represents a hydrogen atom, substituted or unsubstituted lower alkyl, substituted or unsubstituted aralkyl, or substituted or unsubstituted heterocycle-lower alkyl) or
(f) general formula (D):

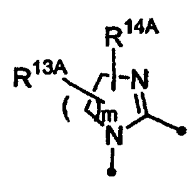

(D)

(wherein m represents an integer of 1 to 3; and $R^{13A}$ and $R^{14A}$ are the same or different, and each represents a hydrogen atom, substituted or unsubstituted lower alkyl, substituted or unsubstituted aryl, substituted or unsubstituted aralkyl, a substituted or unsubstituted heterocyclic group, or substituted or unsubstituted heterocycle-lower alkyl), provided that when $R^{1A}$ is furyl and $-Q^{A}-$ is $-N=C(-R^{7A})-$ in which $R^{7A}$ is amino or 3,4-dimethoxybenzylamino, $R^{2A}$ is not morpholino, 1-piperazinyl, substituted 1-piperazinyl in which the 4-position is substituted by lower alkyl or aryl, phenylamino or substituted phenylamino in which the 4-position is substituted by halogen> or a pharmaceutically acceptable salt thereof.

**21.** The condensed-ring-pyrimidine derivative according to claim 20, wherein the heterocyclic group in the substituted or unsubstituted heterocyclic group formed by $R^{3A}$ and $R^{4A}$ together with the adjacent nitrogen atom is not 1-piperazinyl, morpholino, thiomorpholino, piperidino or 1-homopiperazinyl when $R^{1A}$ is substituted or unsubstituted aryl, or a substituted or unsubstituted aromatic heterocyclic group, and $-Q^{A}-$ is $-N=C(-R^{7A})-$ in which $R^{7A}$ is amino or 3,4-dimethoxyamino, or a pharmaceutically acceptable salt thereof.

**22.** The condensed-ring-pyrimidine derivative according to claim 20 or 21, wherein $R^{1A}$ is substituted or unsubstituted lower alkyl, substituted or unsubstituted cycloalkyl, substituted or unsubstituted aralkyl, a substituted or unsubstituted alicyclic heterocyclic group, or substituted or unsubstituted heterocycle-lower alkyl, or a pharmaceutically acceptable salt thereof.

**23.** The condensed-ring-pyrimidine derivative according to claim 20 or 21, wherein $R^{7A}$ is not amino or 3,4-dimethoxybenzylamino when $R^{1A}$ is substituted or unsubstituted aryl, or a substituted or unsubstituted aromatic heterocyclic group, or a pharmaceutically acceptable salt thereof.

**24.** The condensed-ring-pyrimidine derivative according to any one of claims 20 to 23, wherein $-Q^{A}-$ is $-N=C(-R^{7A})-$ in which $R^{7A}$ is $-N(-R^{9A})$ $(-R^{10A})$, or a pharmaceutically acceptable salt thereof.

**25.** The condensed-ring-pyrimidine derivative according to any one of claims 20 to 23, wherein $-Q^{A}-$ is $-N=C(-R^{7A})-$ in which $R^{7A}$ is $-S(-R^{11A})$, or a pharmaceutically acceptable salt thereof.

**26.** The condensed-ring-pyrimidine derivative according to any one of claims 20 to 23, wherein $-Q^{A}-$ is $-N(-R^{12A})-C(=O)-$, or a pharmaceutically acceptable salt thereof.

**27.** The condensed-ring-pyrimidine derivative according to any one of claims 20 to 23, wherein $-Q^{A}-$ is general formula (D), or a pharmaceutically acceptable salt thereof.

**28.** The condensed-ring-pyrimidine derivative according to any one of claims 20 to 27, wherein $R^{2A}$ is a group selected from the group consisting of pyridyl, tetrahydropyridyl, indolinyl, isoindolinyl, pyrrolidinyl, thiazolidinyl, oxazolidinyl, piperidino, homopiperidino, piperazinyl, homopiperazinyl, morpholino, thiomorpholino, tetrahydroquinolyl, tetrahydroisoquinolyl, octahydroquinolyl, benzimidazolyl, indazolyl, indolyl, isoindolyl, purinyl, dihydroindolyl, pyrrolyl, pyrazolyl, triazolyl, tetrazolyl, imidazolyl, octahydropyrido[1,2-a]pyrazinyl, octahydropyrrolo[1,2-a]pyrazinyl, 2-ketopiperazinyl, 1,8-diaza-4-oxabicyclo[4.4.0]decanyl, 2,5-diazabicyclo[2.2.1]heptyl, 2,3-dihydro-1H-benzo[de]isoquinolyl, 1,2,3,4-tetrahydrobenzofuro[2,3-c]pyridyl, 1,2,3,4-tetrahydro-9H-pyrido[3,4-b]indolyl, 5,6,7,8-tetrahydro-1,3-dioxolo[4,5-g]isoquinolyl and 1,2,4,5-tetrahydro-3H-benzo[d]azepinyl, or a pharmaceutically acceptable salt thereof.

**29.** A pharmaceutical composition comprising the condensed-ring-pyrimidine derivative according to any one of claims 20 to 28 or a pharmaceutically acceptable salt thereof as an active ingredient.

**30.** An $\alpha_{2c}$-adrenoceptor antagonist comprising the condensed-ring-pyrimidine derivative according to any one of claims 20 to 28 or a pharmaceutically acceptable salt thereof as an active ingredient.

**31.** An agent for preventing and/or treating Parkinson's disease comprising the condensed-ring-pyrimidine derivative according to any one of claims 20 to 28 or a pharmaceutically acceptable salt thereof as an active ingredient.

**32.** An agent for preventing and/or treating dyskinesia comprising the condensed-ring-pyrimidine derivative according to any one of claims 20 to 28 or a pharmaceutically acceptable salt thereof as an active ingredient.

**33.** An agent for preventing and/or treating dyskinesia comprising a compound having $\alpha_{2c}$-adrenoceptor antagonism

or a pharmaceutically acceptable salt thereof as an active ingredient.

**34.** The agent for preventing and/or treating dyskinesia according to claim 32 or 33, wherein dyskinesia is L-DOPA-induced dyskinesia.

**35.** Use of the condensed-ring-pyrimidine derivative according to any one of claims 20 to 28 or a pharmaceutically acceptable salt thereof for the manufacture of an agent for preventing and/or treating diseases induced by hyperactivity of $\alpha_{2c}$-adrenoceptor.

**36.** Use of the condensed-ring-pyrimidine derivative according to any one of claims 20 to 28 or a pharmaceutically acceptable salt thereof for the manufacture of an agent for preventing and/or treating Parkinson's disease.

**37.** Use of the condensed-ring-pyrimidine derivative according to any one of claims 20 to 28 or a pharmaceutically acceptable salt thereof for the manufacture of an agent for preventing and/or treating dyskinesia.

**38.** Use of a compound having $\alpha_{2c}$-adrenoceptor antagonism or a pharmaceutically acceptable salt thereof for the manufacture of an agent for preventing and/or treating dyskinesia.

**39.** The use according to claim 27 or 28, wherein dyskinesia is L-DOPA-induced dyskinesia.

**40.** A method for preventing and/or treating diseases induced by hyperactivity of $\alpha_{2c}$-adrenoceptor, which comprises administering an effective amount of the condensed-ring-pyrimidine derivative according to any one of claims 20 to 28 or a pharmaceutically acceptable salt thereof.

**41.** A method for preventing and/or treating Parkinson's disease, which comprises administering an effective amount of the condensed-ring-pyrimidine derivative according to any one of claims 20 to 28 or a pharmaceutically acceptable salt thereof.

**42.** A method for preventing and/or treating dyskinesia, which comprises administering an effective dose of the condensed-ring-pyrimidine derivative according to any one of claims 20 to 28 or a pharmaceutically acceptable salt thereof.

**43.** A method for preventing and/or treating dyskinesia, which comprises administering an effective amount of a compound having $\alpha_{2c}$-adrenoceptor antagonism or a pharmaceutically acceptable salt thereof.

**44.** The method for preventing and/or treating dyskinesia according to claim 42 or 43, wherein dyskinesia is L-DOPA-induced dyskinesia.

## INTERNATIONAL SEARCH REPORT

International application No.
PCT/JP02/09911

**A. CLASSIFICATION OF SUBJECT MATTER**

Int.Cl[7] A61K31/519, 31/5377, 31/537, 31/5365, 31/527, 31/5383, 45/00, A61P3/04, 3/10, 9/12, 15/10, 25/00, 25/14, 25/16, 25/24, 43/00, C07D487/04, 487/14, 519/00, 455/04

According to International Patent Classification (IPC) or to both national classification and IPC

**B. FIELDS SEARCHED**

Minimum documentation searched (classification system followed by classification symbols)

Int.Cl[7] A61K31/519, 31/5377, 31/537, 31/5365, 31/527, 31/5383, 45/00, A61P3/04, 3/10, 9/12, 15/10, 25/00, 25/14, 25/16, 25/24, 43/00, C07D487/04, 487/14, 519/00, 455/04

Documentation searched other than minimum documentation to the extent that such documents are included in the fields searched

Electronic data base consulted during the international search (name of data base and, where practicable, search terms used)

CAPLUS(STN), CAOLD(STN), REGISTRY(STN), WPI/L(DIALOG)

**C. DOCUMENTS CONSIDERED TO BE RELEVANT**

| Category* | Citation of document, with indication, where appropriate, of the relevant passages | Relevant to claim No. |
|---|---|---|
| X<br><br>Y | EP 976753 A1 (Kyowa Hakko Kogyo Co., Ltd.), 02 February, 2000 (02.02.00), Compounds 25 to 28, 56 to 59; text example 5; Claims & WO 98/42711 A1 & US 6222035 B1 | 20,24,28-29, 31,36<br>10-15,18-19, 32,34,37-39 |
| Y | Chemical Abstracts, 1998, Vol.129, abstract No. 49522 & Tomotuki KANDA, et al., Adenosine A2A antagonist: a novel antiperkinsonian agent that does not provoke dyskinesia in parkinsonian monkeys, Annals of Neulorogy, 1998, Vol.43, No.4, pages 507 to 513 | 10-15,18-19, 32,34,37-39 |
| P,X<br>P,A | WO 02/39991 A2 (Orion Corp.), 23 May, 2002 (23.05.02), Claims; examples (Family: none) | 33-34,38<br>1-32,35-37, 39 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>24 December, 2002 (24.12.02) | Date of mailing of the international search report<br>14 January, 2003 (14.01.03) |
| Name and mailing address of the ISA/<br>Japanese Patent Office<br>Facsimile No. | Authorized officer<br><br>Telephone No. |

Form PCT/ISA/210 (second sheet) (July 1998)

**INTERNATIONAL SEARCH REPORT**

International application No.
PCT/JP02/09911

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | Haapalinna A. et al., Evaluation of the effects of a specific alpha 2-adrenoceptor antagonist, atipamezole, on alpha 1- and alpha 2-adrenoceptor subtype binding, brain neurochemistry and behavior in comparison with yohimbine, Naunyn-Schmiedeberg's Arch Pharmacol., 1997, Vol.356, No.5, pages 570 to 582; page 574, table 1 | 33-34,38<br>1-32,35-37, 39 |
| Y<br>A | WO 01/19371 A1 (REED RITCHER), 22 March, 2001 (22.03.01), Claims & US 6281207 B1 | 33-34,38<br>1-32,35-37, 39 |
| Y<br>A | Richard GRODIN, et al., Noradrenoceptor antagonism with idazoxan improves L-dopa-induced dyskinesias in MPTP monkeys, Naunyn-Schmiedeberg's Arch Pharmacol., 2000, Vol.361, No.2, pages 181 to 186; all pages | 33-34,38,<br>1-32,35-37, 39 |

Form PCT/ISA/210 (continuation of second sheet) (July 1998)

**INTERNATIONAL SEARCH REPORT**

International application No.
PCT/JP02/09911

**Box I Observations where certain claims were found unsearchable (Continuation of item 2 of first sheet)**

This international search report has not been established in respect of certain claims under Article 17(2)(a) for the following reasons:

1. [X] Claims Nos.: 40 to 44
because they relate to subject matter not required to be searched by this Authority, namely:
Claims 40 to 44 pertain to a methods for treatment of the human body by therapy and thus relate to a subject matter which this International Searching Authority is not required, under the provisions of Article 17(2)(a)(i) of (continued to extra sheet)

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[ ] No protest accompanied the payment of additional search fees.

Form PCT/ISA/210 (continuation of first sheet (1)) (July 1998)

**INTERNATIONAL SEARCH REPORT**

International application No.
PCT/JP02/09911

Continuation of Box No.I-1 of continuation of first sheet(1)

the PCT and Rule 39.1(iv) of the Regulations under the PCT, to make an international search.

Form PCT/ISA/210 (extra sheet) (July 1998)